# EUROPEAN PATENT SPECIFICATION

(11) **EP 4 523 692 B1**
(45) Date of publication and mention of the grant of the patent: **14.10.2026**
(21) Application number: 24213541.6
(22) Date of filing: 07.05.2020
(51) Int. Cl.: A61P 5/14, C07D 401/14, C07D 403/12, C07D 403/14, A61K 31/53

(54) **MODULATORS OF THR-SS AND METHODS OF USE THEREOF**
MODULATOREN VON THR-SS UND VERFAHREN ZUR VERWENDUNG DAVON
MODULATEURS DE THR-SS ET LEURS PROCÉDÉS D'UTILISATION

(30) Priority: 08.05.2019 US 201962845252 P; 05.12.2019 US 201962944052 P; 06.04.2020 US 202063005661 P
(43) Date of publication of application: 19.03.2025
(62) Document number(s) of the earlier application(s) in accordance with Art. 76 EPC: 20729390.3 / 3 965 884
(73) Proprietor: Aligos Therapeutics, Inc., South San Francisco, CA 94080 (US)
(72) Inventor: Vandyck, Koen, 3583 Beringen (BE); Raboisson, Pierre Jean-Marie Bernard, 3001 Leuven (Heverlee) (BE); McGowan, David, 1150 Brussels (BE); Deval, Jerome, El Granada, California, 94018 (US)
(74) Representative: Hoffmann Eitle

(56) References cited:
- WO-A1-2007/009913
- WO-A1-2009/037172
- WO-A1-2014/043706
- WO-A1-2020/073974
- MARTHA J. KELLY ET AL: "Discovery of 2-[3,5-Dichloro-4-(5-isopropyl-6-oxo-1,6-dihydropyridazin-3-yloxy)phenyl]-3,5-dioxo-2,3,4,5-tetrahydro[1,2,4]triazine-6-carbonitrile (MGL-3196), a Highly Selective Thyroid Hormone Receptor &bgr; Agonist in Clinical Trials for the Treatment of Dyslipidemia", JOURNAL OF MEDICINAL CHEMISTRY, vol. 57, no. 10, 8 April 2014 (2014-04-08), US, pages 3912 - 3923, XP055436939, ISSN: 0022-2623, DOI: 10.1021/jm4019299

## Description

### CROSS-REFERENCE TO RELATED APPLICATIONS

This application claims the benefit of priority to U.S. Provisional Patent Application Serial No. 63/005,661, filed on April 6, 2020, U.S. Provisional Patent Application Serial No. 62/944,052, filed on December 5, 2019, and U.S. Provisional Patent Application Serial No. 62/845,252, filed on May 8, 2019.

### FIELD OF THE INVENTION

The present invention is in the field of pharmaceutical compounds and preparations and method of their use in the treatment of disease. In particular, the present invention is in the field of THR-β modulators and their use.

### BACKGROUND OF THE DISCLOSURE

In parallel with the global increase in obesity, nonalcoholic fatty liver disease (NAFLD) is becoming the leading cause of chronic liver disease and liver transplantation worldwide [1,2]. NAFLD is believed to affect 30% of the adult population and 70-80% of individuals who are obese and diabetic. NAFLD is defined as excess liver fat accumulation greater than 5% induced by causes other than alcohol intake. NAFLD progresses to liver inflammation (nonalcoholic steatohepatitis, NASH) and fibrosis in a variable proportion of individuals, ultimately leading to liver failure and hepatocellular carcinoma (HCC) in susceptible individuals [3].

In the United States alone, NASH is the third most common indication for liver transplantation and is on a trajectory to become the most common [4]. The most important medical need in patients with NAFLD and NASH is an effective treatment to halt the progression and possibly reverse fibrosis, which is the main predictor of liver disease evolution [5,6].

Thyroid hormone (TH) is essential for normal development, growth and metabolism of all vertebrates. Its effects are mediated principally through triiodothyronine (T3), which acts as a ligand for the TH receptors (TRs, or THRs) β1, β2 and α1 [7]. In the absence of ligand, TR first binds as a heterodimer or homodimer on TH response elements (TRE) located in the promoter regions of target genes, where it interacts with corepressors. Upon ligand binding, the TR homodimers are dissociated in favor of heterodimer formation with the retinoid-X receptor (RXR), resulting in release of the corepressors and recruitment of coactivators. This new complex attracts a large number of proteins which engage the RNA polymerase II in the transcription of the targeted genes.

Two different genetic loci, denoted THRA and THRB, are responsible for encoding multiple interrelated TR isoforms that have distinct tissue distributions and biological functions. The two major isoforms with the broadest level of tissue expression are TRα1 and TRβ1 [8]. While TRα1 is expressed first during fetal development and is widely expressed in adult tissues, TRβ1 appears later in development and displays highest expression in the adult liver, kidney, and lung [9]. TRα1 is a key regulator of cardiac output, whereas TRβ1 helps in the control of metabolism in the liver. Importantly, the natural thyroid hormone T3 activates both TRα1 and TRβ1 without any significant selectivity.

Design of thyromimetic small molecule agents led to the identification of TR (or THR) agonists with varying levels of TRβ selectivity despite high structural similarity between the ligand-binding domains for TRβ and TRα. TRβ selectivity achieved by some of these compounds resulted in an improved therapeutic index for lipid lowering relative to cardiac effects such as heart rate, cardiac hypertrophy, and contractility [10-12].

Another strategy to avoid activation of TRα in cardiac tissue is to design prodrugs of phosphonate-containing TR agonists that are specifically converted to the active agonist in the liver but remain stable as an inactive prodrug in blood and extrahepatic tissues, including the heart [13]. TRα and TRβ agonists are also used in indications other than liver-related disorders, as has been known in the art. WO 2007/009913 A1 describes pyridazinone derivatives as thyroid hormone receptor agonists. WO 2009/037172 A1 describes prodrugs of pyridazinone compounds as thyroid hormone receptor agonists. WO 2014/043706 A1 describes methods of large-scale preparation of pyridazinone compounds as well as characterization of certain polymorphs. Kelly et al. in "Discovery of 2-[3,5-dichloro-4-(5-isopropyl-6-oxo-1,6- dihydropyridazin-3-yloxy)phenyl]-3,5-dioxo-2,3,4,5-tetrahydro[1,2,4] triazine-6-carbonitrile (MGL-3196), a Highly Selective Thyroid Hormone Receptor β Agonist in Clinical Trial for the Treatment of Dyslipidemia", Journal of Medicinal Chemistry, 2014, 57, 3912-3923, discloses the discovery of 2-[3,5-dichloro-4-(5-isopropyl-6-oxo-1,6-dihydropyridazin-3-yloxy)phenyl]-3,5-dioxo-2,3,4,5-tetrahydro[1,2,4]triazine-6-carbonitrile (MGL-3196).

### SUMMARY

Disclosed herein are compounds of Formula I':

(I') TL-Lₐ-CE-HD

or a pharmaceutically acceptable salt thereof, where i) TL is a moiety of Formula IIIb; ii) Lₐ is independently a bond; -(C(Rₐ)₂)ₙ-; oxygen; sulfur; -NRₐ- iii) CE is a moiety of Formula IV; iv) HD is a moiety of Formula V; where the substituents are as defined in the appendent claims. Disclosed are also pharmaceutical compositions comprising the above compounds, and compounds for use in treating disease by administering or contact a patient with one or more of the above compounds.

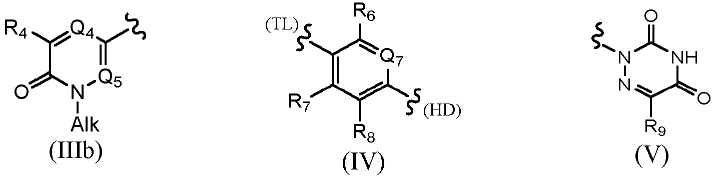

### BRIEF DESCRIPTION OF THE DRAWINGS

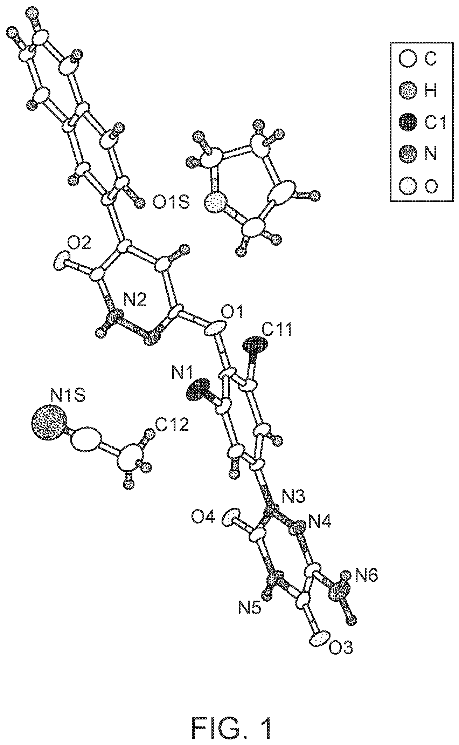

Figure 1 depicts the chemical structure of Compound 67 as confirmed by x-ray crystallography.

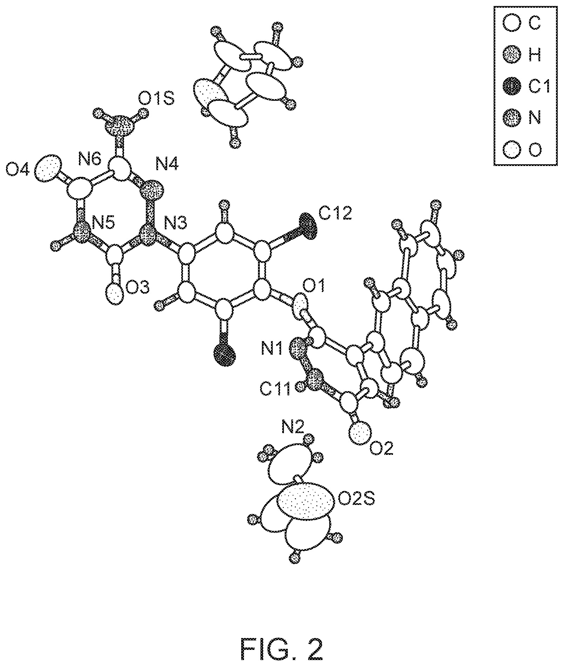

Figure 2 depicts the chemical structure of Compound 67-A as confirmed by x-ray crystallography.

### DETAILED DESCRIPTION OF THE EMBODIMENTS

Disclosed herein are novel compounds that are effective modulators of THR-β activity that can be used for the treatment of various THR-β related disorders. The compounds and the methods of their use are discussed in detail below. Certain of the compounds disclosed herein are agonists, while others are antagonists, of TRα and/or TRβ receptors and are used to treat liver-related disorders and other indications known in the art that are mediated by TRα and/or TRβ receptors.

### DEFINITIONS

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The use of the terms "a" and "an" and "the" and similar referents in the context of describing the elements (especially in the context of the following claims and embodiments) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.,* "such as") provided herein, is intended merely to better illuminate the embodiments and does not pose a limitation on the scope of the claims unless otherwise stated. No language in the specification should be construed as indicating any non-claimed element as essential.

In the definition of chemical substituents, each of Rₓ and R_{y} is independently hydrogen, alkyl, carbocyclic ring, heterocyclic ring, aryl, or heteroaryl, all of which, except hydrogen, are optionally substituted.

Unless otherwise indicated, the abbreviations "TR" and "THR" refer to thyroid hormone receptors.

As used herein, "pharmaceutically acceptable salt" refers to a salt of a compound that does not cause significant irritation to a patient to which it is administered and does not abrogate the biological activity and properties of the compound. Pharmaceutical salts can be obtained by reaction of a compound disclosed herein with an acid or base. Base-formed salts include, without limitation, ammonium salt (NH₄⁺); alkali metal, such as, without limitation, sodium or potassium, salts; alkaline earth, such as, without limitation, calcium or magnesium, salts; salts of organic bases such as, without limitation, dicyclohexylamine, N-methyl-D-glucamine, tris(hydroxymethyl)methylamine; and salts with the amino group of amino acids such as, without limitation, arginine and lysine. Useful acid-based salts include, without limitation, hydrochlorides, hydrobromides, sulfates, nitrates, phosphates, methanesulfonates, ethanesulfonates, p-toluenesulfonates and salicylates.

As used herein, "pharmaceutically acceptable ester" refers to an ester of a compound that does not cause significant irritation to a patient to which it is administered. The ester is metabolized in the body to result in the parent compound, e.g., the claimed compound. Accordingly, the ester does not abrogate the biological activity and properties of the compound. Pharmaceutical esters can be obtained by reaction of a compound disclosed herein with an alcohol. Methyl, ethyl, and isopropyl esters are some of the common esters to be prepared. Other esters suitable are well-known to those skilled in the art (see, for example Wuts, P.G.M., Greene's Protective Groups in Organic Synthesis, 5th Ed., John Wiley & Sons, New York, N.Y., 2014).

Where the compounds disclosed herein have at least one chiral center, they may exist as a racemate or as individual enantiomers. It should be noted that all such isomers and mixtures thereof are included in the scope of the present disclosure. Thus, the illustration of a chiral center without a designation of R or S signifies that the scope of the disclosure includes the R isomer, the S isomer, the racemic mixture of the isomers, or mixtures where one isomer is present in greater abundance than the other.

Where the processes for the preparation of the compounds disclosed herein give rise to mixtures of stereoisomers, such isomers may be separated by conventional techniques such as preparative chiral chromatography. The compounds may be prepared in racemic form or individual enantiomers may be prepared by stereoselective synthesis or by resolution. The compounds may be resolved into their component enantiomers by standard techniques, such as the formation of diastereomeric pairs by salt formation with an optically active acid, such as (-)-di-p-toluoyl-d-tartaric acid and/or (+)-di-p-toluoyl-l-tartaric acid, followed by fractional crystallization and regeneration of the free base. The compounds may also be resolved by formation of diastereomeric esters or amides followed by chromatographic separation and removal of the chiral auxiliary.

Unless otherwise indicated, when a substituent is deemed to be "optionally substituted" it is meant that the substituent is a group that may be substituted with one or more group(s) individually and independently selected, without limitation, from alkyl, alkenyl, alkynyl, cycloalkyl, aryl, heteroaryl, heteroalicyclic, hydroxy, alkoxy, aryloxy, mercapto, alkylthio, arylthio, cyano, halo, carbonyl, thiocarbonyl, O-carbamoyl, N-carbamoyl, O-thiocarbamoyl, N-thiocarbamoyl, C-amido, N-amido, S-sulfonamido, N-sulfonamido, C-carboxy, O-carboxy, is O-cyanato, thiocyanato, isothiocyanato, nitro, silyl, trihalomethanesulfonyl, and amino, including mono- and di-substituted amino groups, and the protected derivatives thereof. The protecting groups that may form the protective derivatives of the above substituents are known to those of skill in the art and may be found in references such as Wuts, above.

As used herein, a "carbocyclic ring" is a ring structure in which all the atoms in the ring are carbon atoms. If any of the atoms in the ring is anything other than a carbon atom, then the ring is a "heterocyclic ring." Examples of atoms that are within a ring include sulfur, oxygen, and nitrogen. A carbocyclic ring or a heterocyclic ring may be polycyclic, e.g., a fused ring system, a spirocyclic ring system, or a bridged ring system. These polycyclic rings include, for example, adamantyl, norbornyl (*i.e.,* bicyclo[2.2.1 ]heptanyl), norbornenyl, decalinyl, 7,7-dimethyl-bicyclo[2.2.1]heptanyl, and the like. Additional non-limiting examples include:

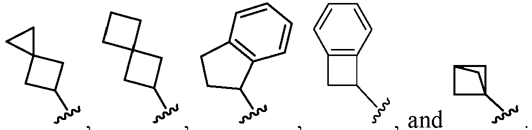

As used herein, "aryl" refers to a carbocyclic (all carbon) ring that has a fully delocalized pi-electron system. The "aryl" group can be made up of two or more fused rings (rings that share two adjacent carbon atoms). When the aryl is a fused ring system, then the ring that is connected to the rest of the molecule has a fully delocalized pi-electron system. The other ring(s) in the fused ring system may or may not have a fully delocalized pi-electron system. Examples of aryl groups include, without limitation, the radicals of benzene, naphthalene and azulene. Additional non-limiting examples include:

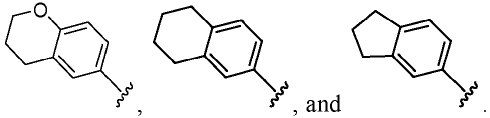

As used herein, "heteroaryl" refers to a ring that has a fully delocalized pi-electron system and contains one or more heteroatoms selected from the group consisting of nitrogen, oxygen, and sulfur in the ring. The "heteroaryl" group can be made up of two or more fused rings (rings that share two adjacent carbon atoms). When the heteroaryl is a fused ring system, then the ring that is connected to the rest of the molecule has a fully delocalized pi-electron system. The other ring(s) in the fused ring system may or may not have a fully delocalized pi-electron system. Examples of heteroaryl rings include, without limitation, furan, thiophene, phthalazinone, pyrrole, oxazole, thiazole, imidazole, pyrazole, isoxazole, isothiazole, triazole, thiadiazole, pyridine, pyridazine, pyrimidine, pyrazine and triazine.

Wherever "hetero" is used it is intended to mean a group as specified, such as an alkyl or an aryl group, where at least one carbon atom has been replaced with a heteroatom selected from nitrogen, oxygen and sulfur.

As used herein, "alkyl" refers to a straight or branched chain fully saturated (no double or triple bonds) hydrocarbon group. An alkyl group of the presently disclosed compounds may comprise from 1 to 20 carbon atoms. An alkyl group herein may also be of medium size having 1 to 10 carbon atoms. An alkyl group herein may also be a lower alkyl having 1 to 5 carbon atoms. Examples of alkyl groups include, without limitation, methyl, ethyl, *n*-propyl, isopropyl, *n*-butyl, *i*-butyl, *sec*-butyl, *t*-butyl, amyl, *t*-amyl, hexyl, heptyl, octyl, nonyl, decyl, undecyl and dodecyl.

An alkyl group of the presently disclosed compounds may be substituted or unsubstituted. When substituted, the substituent group(s) can be one or more group(s) independently selected from cycloalkyl, aryl, heteroaryl, heteroalicyclyl, hydroxy, protected hydroxy, alkoxy, aryloxy, mercapto, alkylthio, arylthio, cyano, halogen, carbonyl, thiocarbonyl, O-carbamoyl, N-carbamoyl, O-thiocarbamoyl, N-thiocarbamoyl, C-amido, N-amido, S-sulfonamido, N-sulfonamido, C-carboxy, protected C-carboxy, O-carboxy, isocyanato, thiocyanato, isothiocyanato, nitro, silyl, trihalomethanesulfonyl, -NRₓR_{y} and protected amino.

As used herein, "alkenyl" refers to an alkyl group that contains in the straight or branched hydrocarbon chain one or more double bonds. An alkenyl group of the presently disclosed compounds may be unsubstituted or substituted. When substituted, the substituent(s) may be selected from the same groups disclosed above regarding alkyl group substitution, or with regard to optional substitution.

As used herein, "alkynyl" refers to an alkyl group that contains in the straight or branched hydrocarbon chain one or more triple bonds. An alkynyl group of the presently disclosed compounds may be unsubstituted or substituted. When substituted, the substituent(s) may be selected from the same groups disclosed above regarding alkyl group substitution, or with regard to optional substitution.

As used herein, "acyl" refers to an "RₓC(=O)-" group.

As used herein, "cycloalkyl" refers to a completely saturated (no double bonds) hydrocarbon ring. Cycloalkyl groups of the presently disclosed compounds may range from C₃ to C₈. A cycloalkyl group may be unsubstituted or substituted. If substituted, the substituent(s) may be selected from those indicated above regarding substitution of an alkyl group. The "cycloalkyl" group can be made up of two or more fused rings (rings that share two adjacent carbon atoms). When the cycloalkyl is a fused ring system, then the ring that is connected to the rest of the molecule is a cycloalkyl as defined above. The other ring(s) in the fused ring system may be a cycloalkyl, a cycloalkenyl, an aryl, a heteroaryl, or a heteroalicyclic.

As used herein, "cycloalkenyl" refers to a cycloalkyl group that contains one or more double bonds in the ring although, if there is more than one, they cannot form a fully delocalized pi-electron system in the ring (otherwise the group would be "aryl," as defined herein). A cycloalkenyl group of the presently disclosed compounds may unsubstituted or substituted. When substituted, the substituent(s) may be selected from the same groups disclosed above regarding alkyl group substitution. The "cycloalkenyl" group can be made up of two or more fused rings (rings that share two adjacent carbon atoms). When the cycloalkenyl is a fused ring system, then the ring that is connected to the rest of the molecule is a cycloalkenyl as defined above. The other ring(s) in the fused ring system may be a cycloalkyl, a cycloalkenyl, an aryl, a heteroaryl, or a heteroalicyclic.

The term "alkylene" refers to an alkyl group, as defined herein, which is a biradical and is connected to two other moieties. Thus, methylene (-CH₂-), ethylene (-CH₂CH₂-), propylene (-CH₂CH₂CH₂-), isopropylene (IUPAC: (methyl)ethylene) (-CH₂-CH(CH₃)-), and isobutylene (IUPAC: 2-(methyl)propylene) (-CH₂-CH(CH₃)-CH₂-) are examples, without limitation, of an alkylene group. Similarly, the term "cycloalkylene" refers to a cycloalkyl group, as defined here, which binds in an analogous way to two other moieties. If the alkyl and cycloalkyl groups contain unsaturated carbons, the terms "alkenylene" and "cycloalkenylene" are used.

As used herein, "heterocycloalkyl," "heteroalicyclic," or "heteroali-cyclyl" refers to a ring having in the ring system one or more heteroatoms independently selected from nitrogen, oxygen and sulfur. The ring may also contain one or more double bonds provided that they do not form a fully delocalized pi-electron system in the rings. The ring defined herein can be a stable 3- to 18-membered ring that consists of carbon atoms and from one to five heteroatoms selected from the group consisting of nitrogen, oxygen, and sulfur. Heteroalicyclyl groups of the presently disclosed compounds may be unsubstituted or substituted. When substituted, the substituent(s) may be one or more groups independently selected from the group consisting of halogen, hydroxy, protected hydroxy, cyano, nitro, alkyl, alkoxy, acyl, acyloxy, carboxy, protected carboxy, amino, protected amino, carboxamide, protected carboxamide, alkylsulfonamido and trifluoromethane-sulfonamido. The "heterocycloalkyl" group can be made up of two or more fused rings (rings that share two adjacent carbon atoms). When the heterocycloalkyl is a fused ring system, then the ring that is connected to the rest of the molecule is a heterocycloalkyl as defined above. The other ring(s) in the fused ring system may be a cycloalkyl, a cycloalkenyl, an aryl, a heteroaryl, or a heteroalicyclic.

As used herein, "aralkyl" refers to an alkylene substituted with an aryl group.

As used herein, "carbocyclic alkyl" or "(carbocyclic)alkyl" refers to an alkylene substituted with a carbocyclic group.

As used herein, "heterocyclicalkyl" or (heterocyclic)alkyl" refers to an alkylene substituted with a heterocyclic group. Similarly, "(heterocycloalkyl)alkyl" refers to an alkylene substituted with a heterocycloalkyl group.

As used herein, "heteroarylalkyl" or "(heteroaryl)alkyl" refers to an alkylene substituted with a heteroaryl group.

An "O-carboxy" group refers to a "RₓC](=O)O-" group.

A "C-carboxy" group refers to a "-C(=O)R" group.

An "acetyl" group refers to a CH₃C(=O)- group.

A "C-amido" group refers to a "-C(=O)NRₓR_{y}" group.

An "N-amido" group refers to a "RC(=O)NRₓ-" group.

The term "perhaloalkyl" refers to an alkyl group in which all the hydrogen atoms are replaced by halogen atoms.

Any unsubstituted or monosubstituted amine group on a compound herein can be converted to an amide, any hydroxy group can be converted to an ester and any carboxyl group can be converted to either an amide or ester using techniques well-known to those skilled in the art (see, for example Wuts, above).

It is understood that, in any compound of the presently disclosed compounds having one or more chiral centers, if an absolute stereochemistry is not expressly indicated, then each center may independently be R or S or a mixture thereof. In addition, it is understood that, in any compound of the presently disclosed compounds having one or more double bond(s) generating geometrical isomers that can be defined as E or Z each double bond may independently be E or Z, or a mixture thereof.

It is understood that the disclosure of a compound herein inherently includes the disclosure of a tautomer thereof, if applicable. For instance, the disclosure of: 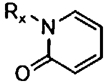
also includes the disclosure of: 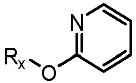
and vice versa, even if only one of the two structures is disclosed.

Throughout the present disclosure, when a compound is illustrated or named, it is understood that the isotopically enriched analogs of the compound are also contemplated. For example, a compound may have a deuterium incorporated instead of a hydrogen, or a carbon-13 instead of carbon with natural isotopic distribution. The isotopic enrichment may be in one location on the compound, i.e., only one hydrogen is replaced by a deuterium, or in more than one location. The present disclosure also encompasses compounds where all the similar atoms are replaced by their less common isotope, for example, a perdeutero compound where all the hydrogen atoms are replaced by a deuterium. The isotopically enriched compounds are useful when obtaining NMR spectra or when making use of an isotope effect in managing the kinetics of the reaction the compound undergoing.

The term "pharmaceutical composition" refers to a mixture of one or more compounds disclosed herein with other chemical components, such as diluents or carriers. The pharmaceutical composition facilitates administration of the compound to an organism. Multiple techniques of administering a compound exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example, dimethyl sulfoxide (DMSO) is a commonly utilized carrier as it facilitates the uptake of many organic compounds into the cells or tissues of an organism.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

In certain embodiments, the same substance can act as a carrier, diluent, or excipient, or have any of the two roles, or have all three roles. Thus, a single additive to the pharmaceutical composition can have multiple functions.

The term "physiologically acceptable" defines a carrier or diluent that does not abrogate the biological activity and properties of the compound.

### COMPOUNDS

A compound according to the present invention is as defined in the claims. In the following, aspects and embodiments are described that are useful for further understanding the invention. However, only aspects and embodiments relating to formula (I'), wherein TL is (IIIb), Lₐ is as defined in the claims, CE is formula (IV), and HD is formula (V), as well as specific examples falling within these general formulas are according to the invention. In one aspect, disclosed herein are compounds of Formula I (according to the invention if TL is (IIIb), Lₐ is as defined in claim 1, CE is formula (IV), and HD is formula (V)):

(I) TL-Lₐ-CE-HD

or a pharmaceutically acceptable salt, prodrug, amide or ester thereof, where:
- i) TL is a moiety of Formula IIa, IIb, IIIa, IIIb, IIIc, or IIId;: 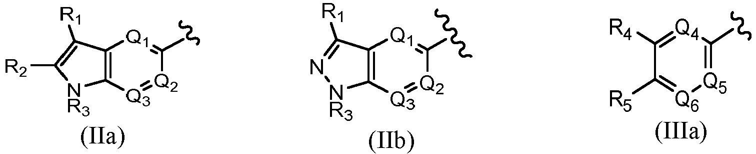 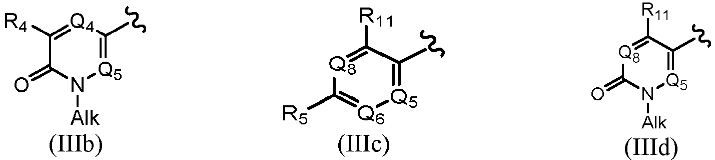 - where:
   - each of Q₁, Q₂, Q₃, Q₄, Q₅, Q₆ and Q₈, is independently nitrogen or -CR_{b}-, wherein each R_{b} is independently hydrogen, halogen, or lower alkyl;
   - R₁ is hydrogen, an optionally substituted alkyl, an optionally substituted carbocyclic group, an optionally substituted aryl group, an optionally substituted heterocyclic group, an optionally substituted heteroaryl group, an optionally substituted carbocyclic alkyl group, an optionally substituted aralkyl group, an optionally substituted heterocyclicalkyl group, an optionally substituted heteroarylalkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   - R₂ is hydrogen, halogen, optionally substituted alkyl, optionally substituted cycloalkyl, or -CN;
   - R₃ is hydrogen or lower alkyl;
   - R₄ is an optionally substituted alkyl, an optionally substituted carbocyclic group, an optionally substituted aryl group, an optionally substituted heterocyclic group, an optionally substituted heteroaryl group, an optionally substituted carbocyclic alkyl group, an optionally substituted aralkyl group, an optionally substituted heterocyclicalkyl group, an optionally substituted heteroarylalkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
      - R₅ is hydroxy, NH₂, alkylamino, alkanoylamino, or alkylsulfonylamino;
   - or R₄ and R₅ taken together along with the carbon atoms to which they are attached form a five- or six-membered optionally substituted carbocyclic group, optionally substituted aryl group, optionally substituted heterocyclic group, or optionally substituted heteroaryl group;
   - or R₄ and R₅ taken together along with the carbon atoms to which they are attached form a seven to eleven membered, optionally substituted spirocyclic ring or a seven to eleven membered, optionally substituted spiro-heterocyclic ring; and
   - Alk is hydrogen or an optionally substituted alkyl;
ii) CE is a moiety of Formula IV 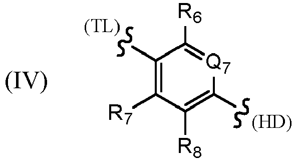 wherein:
   - each of R₆ and R₇ is independently selected from halogen, -CN, optionally substituted lower alkyl, optionally substituted lower alkoxy, optionally substituted lower alkenyl, or cyclopropyl;
   - R₈ is selected from hydrogen, optionally substituted lower alkyl, optionally substituted lower alkoxy, cyano, or halogen;
   - optionally R₇ and R₈ taken together, along with the carbon atoms to which they are attached, form a 4, 5- or 6-membered carbocyclic, heterocyclic, aryl, or heteroaryl ring
   - Q₇ is nitrogen or -CR_{c}-, wherein R_{c} is hydrogen, halogen, or lower alkyl;
   - (TL) denotes the point where the moiety of Formula IV connects to TL-Lₐ-; and
   - (HD) denotes the point where the moiety of Formula IV connects to -HD;
iii) HD is a moiety of Formula V or VI: 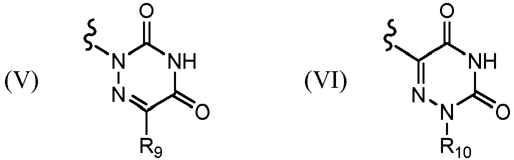 wherein:
   - R₉ is selected from hydrogen, -(C(R_{d})₂)ₙ-C(R_{d})₃, -(C(R_{d})₂)ₙ-OR_{d}, -(C(R_{d})₂)ₙ-N(R_{d})₂, -(C(R_{d})₂)ₙ-S(=O)_{q}R_{d}, -(C(R_{d})₂)ₙ-CN, -(C(R_{d})₂)ₙ-C≡C-R_{d}, -(C(R_{d})₂)ₙ-C(=O)-OR_{d}, -(C(R_{d})₂)ₙ-HeAr, or -(C(R_{d})₂)ₙ-C(=O)-N(R_{d})₂; wherein
      ∘ each R_{d} is independently hydrogen or optionally substituted lower alkyl;
      ∘ each q is independently selected from 0, 1, or 2;
      ∘ each n is independently selected from 0, 1, 2, 3, 4, or 5; and
      ∘ HeAr is a 5- or 6- membered heteroaryl.
   - R₁₀ is hydrogen, -C(Rₑ)₃, wherein each Rₑ is independently hydrogen, halogen, or optionally substituted lower alkyl; and
   - R₁₁ is an aryl group, optionally substituted with lower alkyl, halogen, cycloalkyl; or a bicyclic ring system containing either aromatic or saturated rings; or a bicyclic heterocyclic containing either aromatic or saturated ring systems
iv) Lₐ is independently a bond; -(C(Rₐ)₂)ₙ-; oxygen; sulfur; -NRₐ-; wherein:
   - each Rₐ is independently a hydrogen or lower alkyl; and
   - n is 0, 1, 2, 3, 4 or 5.

In some embodiments, R₁ is an optionally substituted alkyl, an optionally substituted carbocyclic group, an optionally substituted aryl group, and an optionally substituted C-carboxy or O-carboxy group. In some of these embodiments, the alkyl is selected from the group consisting of methyl, ethyl, *n*-propyl, *i*-propyl, *n*-butyl, *i*-butyl, and *t*-butyl. In certain embodiments, the carbocyclic group is cyclohexane or cyclopentane. In various embodiments, the aryl group is phenyl. In some embodiments, the C-carboxy group is a moiety of formula -C(=O)-O-R and the O-carboxy group a moiety of formula -O-C(=O)-R, where R is selected from the group consisting of methyl, ethyl, *n*-propyl, *i*-propyl, *n*-butyl, *i*-butyl, and *t*-butyl.

In some embodiments, R₂ is selected from the group consisting of hydrogen, methyl, ethyl, *n*-propyl, *i*-propyl, *n*-butyl, *i*-butyl, and *t*-butyl.

In some embodiments, Q₁, Q₂, Q₃, and Q₄ are -CR_{b}-, where each R_{b} is independently selected from the group consisting of hydrogen, methyl, ethyl, *n*-propyl, *i-*propyl, *n*-butyl, *i*-butyl, and *t*-butyl.

In some embodiments, R₄ is selected from the group consisting of methyl, ethyl, *n*-propyl, *i*-propyl, *n*-butyl, *i*-butyl, and *t*-butyl.

In some embodiments, R₅ is hydroxy.

In some embodiments, TL is a moiety selected from: 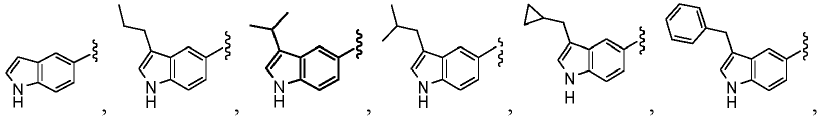 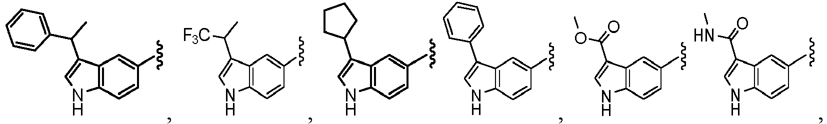 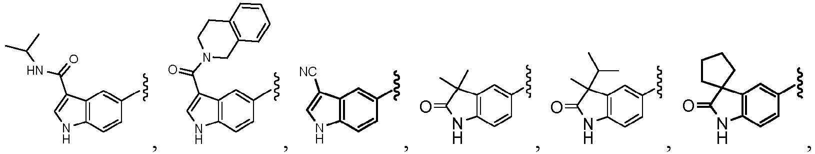 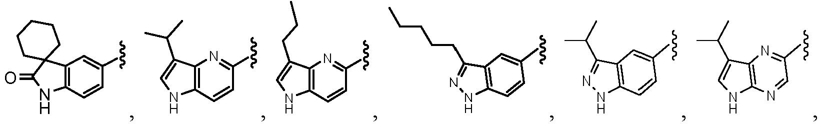 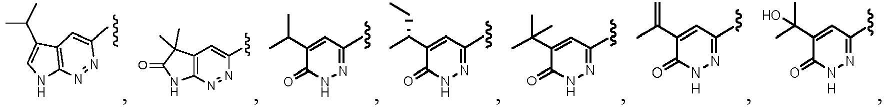 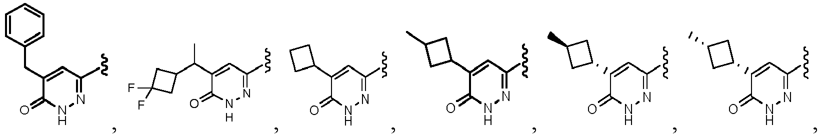 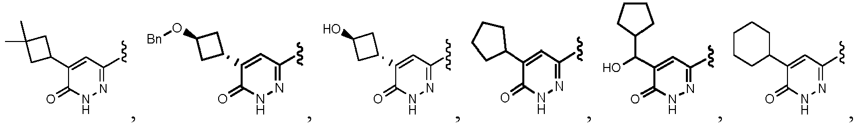 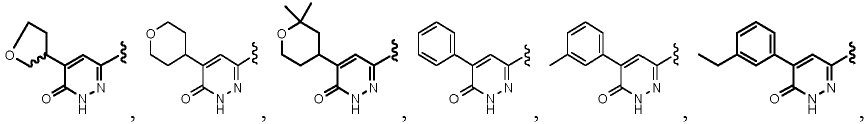 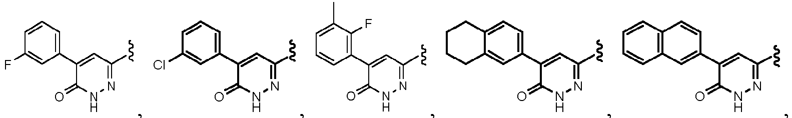 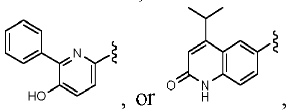 wherein the moieties falling within formula IIIb are according to the invention.

In some embodiments, TL is a moiety selected from: 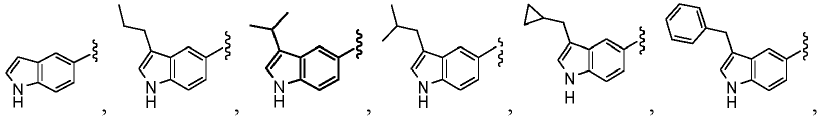 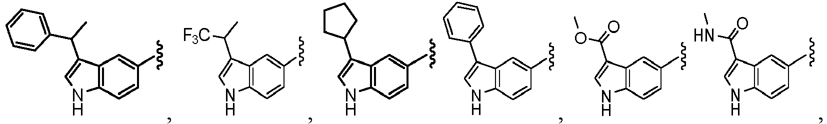 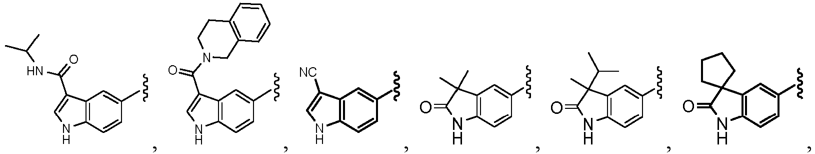 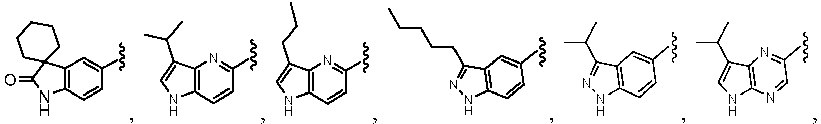 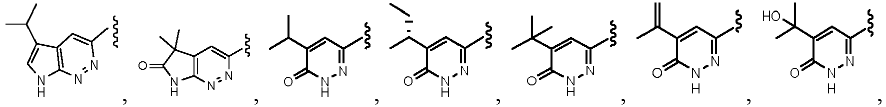 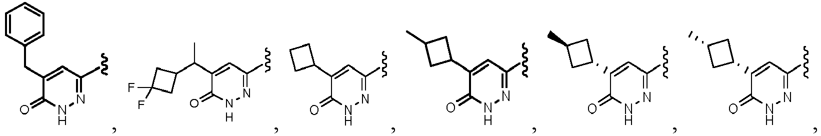 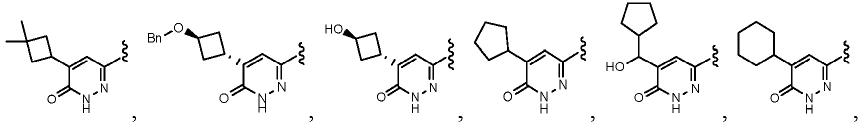 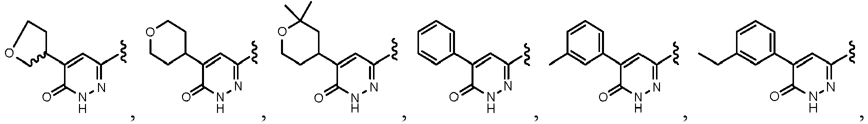 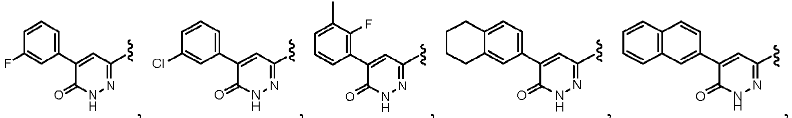 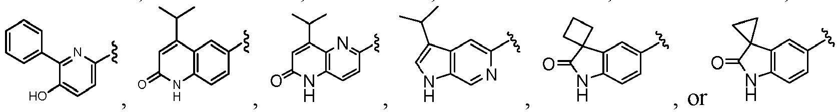 wherein the moieties falling within formula IIIb are according to the invention.

In some embodiments of the compound of Formula I, each of R₆ and R₇ is independently chlorine, bromine, and iodine. In other embodiments, each of R₆ and R₇ is independently -CN, an optionally substituted lower alkyl or an optionally substituted lower alkoxy, where the lower alkyl and the alkyl group of the lower alkoxy is each independently selected from methyl, ethyl, *n*-propyl, *i*-propyl, *n*-butyl, s-butyl, and *t*-butyl. In some embodiments, R₆ and R₇ are the same. In certain embodiments, each of R₆ and R₇ is independently chlorine or methyl.

In some embodiments of the compound of Formula I, R₈ is hydrogen.

In some embodiments, R_{c} is hydrogen or methyl.

In some embodiments, disclosed herein are compounds of Formula I (according to the invention if TL is (IIIb), Lₐ is as defined in claim 1, CE is formula (IV), and HD is formula (V)), where:
- TL is a moiety of Formula IIa, IIb, IIIa, IIIb, IIIc, or IIId; 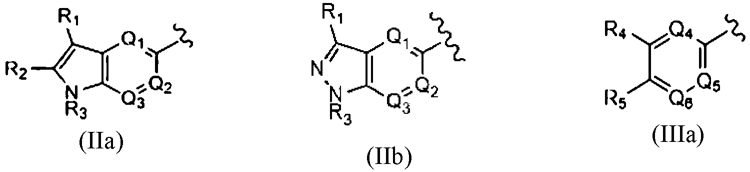 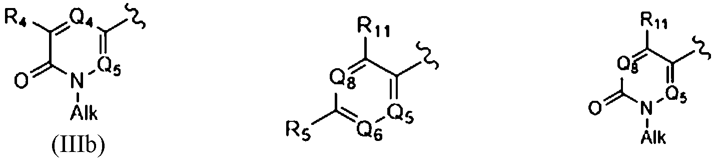 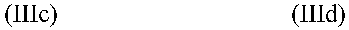 where:
   ∘ each of Q₁, Q₂, Q₃, Q₄, Q₅, Q₆ and Q₈, is independently nitrogen or -CR_{b}-, wherein each R_{b} is independently hydrogen, halogen, or lower alkyl;
   ∘ R₁ is an optionally substituted alkyl, an optionally substituted carbocyclic group, an optionally substituted aryl group, an optionally substituted heterocyclic group, an optionally substituted heteroaryl group, an optionally substituted carbocyclic alkyl group, an optionally substituted aralkyl group, an optionally substituted heterocyclicalkyl group, an optionally substituted heteroarylalkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   ∘ R₂ is halogen, optionally substituted alkyl, optionally substituted cycloalkyl, or -CN;
   ∘ R₃ is hydrogen
   ∘ R₄ is an optionally substituted alkyl, an optionally substituted carbocyclic group, an optionally substituted aryl group, an optionally substituted heterocyclic group, an optionally substituted heteroaryl group, an optionally substituted carbocyclic alkyl group, an optionally substituted aralkyl group, an optionally substituted heterocyclicalkyl group, an optionally substituted heteroarylalkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
   ∘ R₅ is hydroxy, NH₂, alkylamino, alkanoylamino, or alkylsulfonylamino;
   ∘ or R₄ and R₅ taken together along with the carbon atoms to which they are attached form a five- or six-membered optionally substituted carbocyclic group, optionally substituted aryl group, optionally substituted heterocyclic group, or optionally substituted heteroaryl group;
   ∘ or R₄ and R₅ taken together along with the carbon atoms to which they are attached form a seven to eleven membered, optionally substituted spirocyclic ring or a seven to eleven membered, optionally substituted spiro-heterocyclic ring;
   ∘ or when Q₆ is nitrogen and R₅ is hydroxy, then the tautomer of the moiety of Formula III; and
   ∘ Alk is hydrogen or an optionally substituted alkyl;
- CE is a moiety of Formula IV 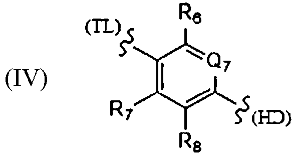 where:
   ∘ each of R₆ and R₇ is independently selected from halogen, -CN, optionally substituted lower alkyl, optionally substituted lower alkoxy, optionally substituted lower alkenyl, or cyclopropyl;
   ∘ R₈ is selected from hydrogen, optionally substituted lower alkyl, optionally substituted lower alkoxy, cyano, or halogen;
   ∘ Q₇ is nitrogen or -CR_{c}-, where R_{c} is hydrogen, halogen, or lower alkyl;
   ∘ (TL) denotes the point where the moiety of Formula IV connects to TL-Lₐ-; and
   ∘ (HD) denotes the point where the moiety of Formula IV connects to -HD;
- HD is a moiety of Formula V or VI: 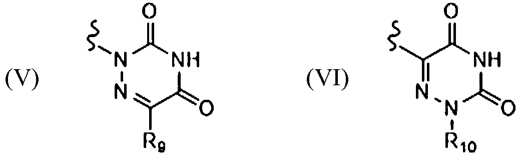 where
   ∘ R₉ is -NH₂ and R₁₀ is -CH₃; and
   ∘ Lₐ is oxygen.

In other embodiments not according to the invention, disclosed herein are compounds of Formula I, where:
- TL is a moiety of Formula II: 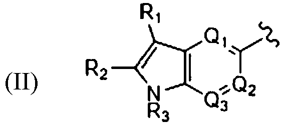 where:
   ∘ each of Q₁, Q₂, and Q₃ is independently nitrogen or -CR_{b}-, where each R_{b} is independently hydrogen, halogen, or lower alkyl;
   o R₁ is an optionally substituted alkyl, an optionally substituted carbocyclic group, an optionally substituted aryl group, an optionally substituted heterocyclic group, an optionally substituted heteroaryl group, an optionally substituted carbocyclic alkyl group, an optionally substituted aralkyl group, an optionally substituted heterocyclicalkyl group, an optionally substituted heteroarylalkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   ∘ R₂ is halogen, optionally substituted alkyl, optionally substituted cycloalkyl, or -CN;
   ∘ R₃ is hydrogen;
- CE is a moiety of Formula IV 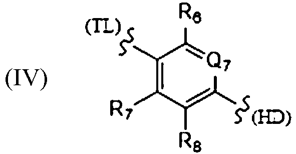 where:
   ∘ each of R₆ and R₇ is independently selected from halogen, -CN, optionally substituted lower alkyl, optionally substituted lower alkoxy, optionally substituted lower alkenyl, or cyclopropyl;
   ∘ R₈ is selected from hydrogen, optionally substituted lower alkyl, optionally substituted lower alkoxy, cyano, or halogen;
   ∘ Q₇ is nitrogen or -CR_{c}-, where R_{c} is hydrogen, halogen, or lower alkyl;
   ∘ (TL) denotes the point where the moiety of Formula IV connects to TL-Lₐ-; and
   ∘ (HD) denotes the point where the moiety of Formula IV connects to -HD;
- HD is a moiety of Formula V: 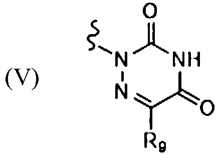 where
   ∘ R₉ is hydrogen, -CN or -C≡C-R_{d}, where R_{d} is hydrogen or lower alkyl; and
   ∘ Lₐ is oxygen.

In some embodiments, La is a combination of two or more of a bond; -(C(Rₐ)₂)ₙ-; oxygen; sulfur; or -NRₐ-.

In another aspect, disclosed herein are compounds of Formula I' (according to the invention if TL is (IIIb), Lₐ is as defined in claim 1, CE is formula (IV), and HD is formula (V)):

(I') TL-Lₐ-CE-HD

or a stereoisomer or a tautomer thereof, or a pharmaceutically acceptable salt, prodrug, amide or ester thereof, wherein:
i) TL is a moiety of Formula IIa, IIb, IIIa, IIIb, IIIc, or IIId: 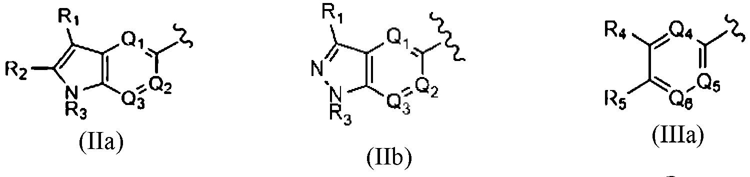 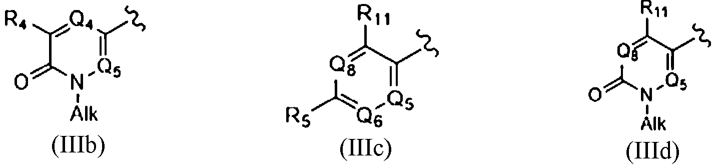 wherein:
   each of Q₁, Q₂, Q₃, Q₄, Q₅, Q₆, and Q₈, is independently nitrogen or -CR_{b}-, wherein each R_{b} is independently hydrogen, halogen, or lower alkyl;
   R₁ is hydrogen, an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   R₂ is hydrogen, halogen, optionally substituted alkyl, optionally substituted cycloalkyl, or -CN;
   R₃ is hydrogen or lower alkyl;
   R₄ is an optionally substituted alkyl, an optionally substituted alkenyl, an optionally substituted non-aromatic carbocyclic group, an optionally substituted aryl group, an optionally substituted heterocyclic group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
   R₅ is hydroxy, NH₂, alkylamino, alkanoylamino, or alkylsulfonylamino;
   or R₄ and R₅ taken together along with the carbon atoms to which they are attached form a five- or six-membered optionally substituted non-aromatic carbocyclic group, optionally substituted aryl group, optionally substituted heterocyclic group, or optionally substituted heteroaryl group;
   or R₄ and R₅ taken together along with the carbon atoms to which they are attached form a seven to eleven membered, optionally substituted spirocyclic ring or a seven to eleven membered, optionally substituted spiro-heterocyclic ring;
   Alk is hydrogen or an optionally substituted alkyl; and
   R₁₁ is an aryl group optionally substituted with one to five substituents independently selected from lower alkyl, alkoxy, haloalkoxy, halogen, and cycloalkyl; or a heteroaryl group optionally substituted with one to five substituents independently selected from lower alkyl, alkoxy, haloalkoxy, halogen, and cycloalkyl; or a bicyclic ring system; or a bicyclic heterocyclic ring system;
ii) CE is a moiety of Formula IV 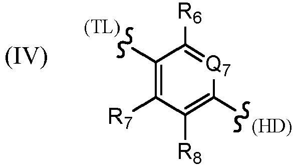 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   optionally R₇ and R₈ taken together, along with the carbon atoms to which they are attached, form a 4-, 5- or 6-membered non-aromatic carbocyclic, heterocycloalkyl, aryl, or heteroaryl ring
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
iii) HD is a moiety of Formula V or VI: 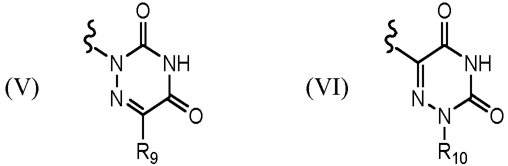 wherein:
   R₉ is selected from hydrogen, -(C(R_{d})₂)ₙ-C(R_{d})₃, -(C(R_{d})₂)ₙ-OR_{d}, -(C(R_{d})₂)ₙ-N(R_{d})₂, -(C(R_{d})₂)ₙ-S(=O)_{q}R_{d}, -(C(R_{d})₂)ₙ-CN, -(C(R_{d})₂)ₙ-C≡C-R_{d}, -(C(R_{d})₂)ₙ-C(=O)-OR_{d}, -(C(R_{d})₂)ₙ-HeAr, or -(C(R_{d})₂)ₙ-C(=O)-N(R_{d})₂; wherein
   each R_{d} is independently hydrogen or optionally substituted lower alkyl;
   each q is independently selected from 0, 1, or 2;
   each n is independently selected from 0, 1, 2, 3, 4, or 5;
   HeAr is a 5- or 6- membered heteroaryl; and
   R₁₀ is hydrogen or -C(Rₑ)₃, wherein each Rₑ is independently hydrogen, halogen, or optionally substituted lower alkyl; and
   Lₐ is independently a bond; -(C(Rₐ)₂)_{z}-; oxygen; sulfur; or -NRₐ-; wherein:
   each Rₐ is independently a hydrogen or lower alkyl; and
   z is 0, 1, 2, 3, 4 or 5;
   provided that:
      (1) when TL is a moiety of Formula IIIa, wherein Q₄, Q₅, and Q₆ are -CH-, and R₄ is an optionally substituted C₁-C₃alkyl group, an optionally substituted sulfamoyl group, or an optionally substituted carbamoyl group; HD is a moiety of Formula V, wherein R₉ is H or - CN; and Q₇ is -CH-, then R₅ cannot be hydroxy;
      (2) when TL is a moiety of Formula IIa, wherein Q₁, Q₂, and Q₃ are -CH-, R₁ is -CH₃, and R₂ is hydrogen; HD is a moiety of Formula V, wherein R₉ is hydrogen; Q₇ is -CH-; R₆ is halogen or methyl; and R₇ and R₈ taken together, along with the carbon atoms to which they are attached, form a 5-membered non-aromatic carbocyclic ring, then R₄ and R₅ taken together along with the carbon atoms to which they are attached cannot form a five-membered heteroaryl group;
      (3) when TL is a moiety of Formula IIIa, wherein Q₄ is nitrogen, Q₅ and Q₆ are -CH-, and R₅ is -OH; HD is a moiety of Formula V, wherein R₉ is -CN; Q₇ is -CH-; Lₐ is -CH₂-, then R₄ cannot be cyclohexyl, cycloheptyl, isopropyl, or optionally substituted benzene;
      (4) when TL is a moiety of Formula IIIb, wherein Q₄ is -CH- and Q₅ is nitrogen; HD is a moiety of Formula V, wherein R₉ is hydrogen, -CN, or -CO₂H; Q₇ is -CH-; and R₆ and R₇ are independently halogen or methyl, or R₇ and R₈ taken together, along with the carbon atoms to which they are attached, form a 5-membered non-aromatic carbocyclic ring, then R₄ cannot be isopropyl or 2-hydroxy-1-methyl-ethyl;
      (5) when TL is a moiety of Formula IIIb, wherein Q₄ is -CR_{c}- and Q₅ is nitrogen; HD is a moiety of Formula V, wherein R₉ is -CN; Q₇ is -CH-; and Lₐ is -O-, then
         (a) none of R₆, R₇, and R₈ can be deuterium;
         (b) Q₄ cannot be -CD-, wherein D is deuterium; and
         (c) R₄ cannot be a three- to five-membered cycloalkyl ring optionally substituted with one or more halogens; non-aromatic (carbocyclic)alkyl optionally substituted with methyl or hydroxy; (1H-pyrazol-4-yl)methyl; (3-methylisoxazol-5-yl)methyl; phenyl; benzyl optionally substituted with methyl, halogen, hydroxy, or methoxy; phenethyl optionally substituted with halogen; C₁-C₄ alkyl optionally substituted with one to six substituents selected from halogen, hydroxyl, and deuterium; (tetrahydro-2H-pyran-4-yl)methyl; or a three- to five-membered heterocycloalkyl optionally substituted with benzyl;
      (6) when TL is a moiety of Formula IIIb, wherein (i) Q₄ and Q₅ are nitrogen, (ii) Q₄ and Q₅ are -CR_{b}-, or (iii) Q₄ is nitrogen and Q₅ is -CR_{c}-; HD is a moiety of Formula V, wherein R₉ is -CN; and Q₇ is -CH-; then R₄ cannot be -CH(CH₃)₂ or -CH(CD₃)₂;
      (7) when TL is a moiety of Formula IIIa, wherein Q₄ is -CH-, Q₅ and Q₆ are nitrogen, and R₄ is isopropyl; HD is a moiety of Formula V, wherein R₉ is -CN; and Q₇ is -CH-; then R₅ cannot be -NH₂ or -NHCH₃;
      (8) when TL is a moiety of Formula IIIb, wherein Q₄ is -CH- and Q₅ is nitrogen; Lₐ is -O-; Q₇ is -CH-; and HD is a moiety of Formula V, then R₉ cannot be methyl;
      (9) when TL is a moiety of Formula IIIb, wherein Q₄ is -CH-, Q₅ is nitrogen, and R₄ is isopropyl; Lₐ is -O-; Q₇ is -CH-; and HD is a moiety of Formula V, then R₉ cannot be isopropyl;
      (10) when TL is a moiety of Formula IIa, wherein Q₁, Q₂, and Q₃ are -CH-; Lₐ is -O-; Q₇ is -CH-; R₆ and R₇ are independently chlorine or trifluoromethyl; and HD is a moiety of Formula V, wherein R₉ is -CN or methyl, then R₁ cannot be isopropyl, 4-tetrahydropyranyl, or -C(O)NH₂;
      (11) when TL is a moiety of Formula IIa, wherein Q₁ and Q₂ are -CH-, and Q₃ is nitrogen; Lₐ is -O-; Q₇ is -CH-; R₆ and R₇ are chlorine; and HD is a moiety of Formula V, wherein R₉ is -CN, then R₁ cannot be isopropyl;
      (12) when TL is a moiety of Formula IIb, wherein Q₁, Q₂, and Q₃ are -CH-; Lₐ is -O-; Q₇ is -CH-; R₆ and R₇ are chlorine; and HD is a moiety of Formula V, wherein R₉ is -CN, then R₁ cannot be isopropyl;
      (13) when TL is a moiety of Formula IIIb, wherein Q₄ is -CH- and Q₅ is nitrogen; Lₐ is -O-; Q₇ is -CH-; R₆ and R₇ are chlorine; and HD is a moiety of Formula VI, wherein R₁₀ is H, Me, Et, isopropyl, -CH₂CF₃, or -CH₂CHF₂, then R₄ cannot be C₂-C₅ alkyl or C₁-C₃ hydroxyalkyl;
      (14) when TL is a moiety of Formula IIIb, wherein Q₄ is -CH- and Q₅ is nitrogen; Lₐ is -O-; Q₇ is -CH-; R₆ and R₇ are chlorine; and HD is a moiety of Formula VI, wherein R₁₀ is H or Me, then R₄ cannot be cyclopropyl; and
      (15) when TL is a moiety of Formula IIIb, wherein Q₄ is -CH- and Q₅ is nitrogen; Lₐ is -CH₂-; Q₇ is -CH-; R₆ and R₇ are both chlorine or both methyl; and HD is a moiety of Formula VI, wherein R₁₀ is H or Me, then R₄ cannot be isopropyl.
In some embodiments, when TL is a moiety of Formula IIIb, wherein Q₄ is -CH- and Q₅ is nitrogen; HD is a moiety of Formula V, wherein R₉ is -CH₂CN or -C≡CH; Q₇ is -CH-; and R₆ and R₇ are chlorine, then R₄ cannot be isopropyl.

In another aspect, disclosed herein are compounds of Formula I' (according to the invention if TL is (IIIb), Lₐ is as defined in claim 1, CE is formula (IV), and HD is formula (V)):

(I') TL-Lₐ-CE-HD

or a stereoisomer or a tautomer thereof, or a pharmaceutically acceptable salt, prodrug, amide or ester thereof, wherein:
i) TL is a moiety of Formula IIa, IIb, IIIa, IIIb, IIIc, or IIId: 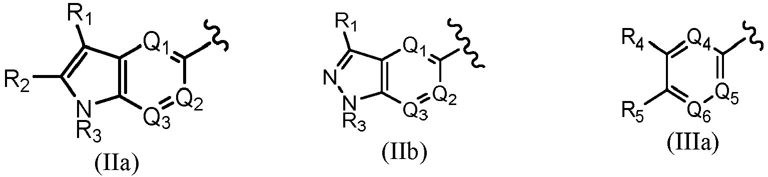 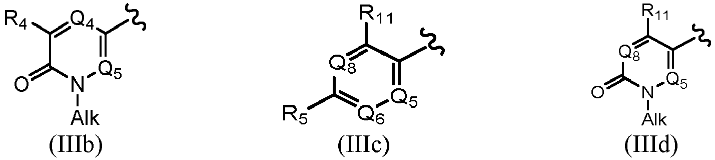 wherein:
   each of Q₁, Q₂, Q₃, Q₄, Q₅, Q₆, and Q₈, is independently nitrogen or -CR_{b}-, wherein each R_{b} is independently hydrogen, halogen, or lower alkyl;
   R₁ is hydrogen, an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   R₂ is hydrogen, halogen, optionally substituted alkyl, optionally substituted cycloalkyl, or -CN;
   R₃ is hydrogen or lower alkyl;
   R₄ is an optionally substituted alkyl, an optionally substituted alkenyl, an optionally substituted non-aromatic carbocyclic group, an optionally substituted aryl group, an optionally substituted heterocyclic group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
   R₅ is hydroxy, NH₂, alkylamino, alkanoylamino, or alkylsulfonylamino;
   or R₄ and R₅ taken together along with the carbon atoms to which they are attached form a five- or six-membered optionally substituted non-aromatic carbocyclic group, optionally substituted aryl group, optionally substituted heterocyclic group, or optionally substituted heteroaryl group;
   or R₄ and R₅ taken together along with the carbon atoms to which they are attached form a seven to eleven membered, optionally substituted spirocyclic ring or a seven to eleven membered, optionally substituted spiro-heterocyclic ring;
   Alk is hydrogen or an optionally substituted alkyl; and
   R₁₁ is an aryl group optionally substituted with one to five substituents independently selected from lower alkyl, alkoxy, haloalkoxy, halogen, and cycloalkyl; or a heteroaryl group optionally substituted with one to five substituents independently selected from lower alkyl, alkoxy, haloalkoxy, halogen, and cycloalkyl; or a bicyclic ring system; or a bicyclic heterocyclic ring system;
ii) CE is a moiety of Formula IV 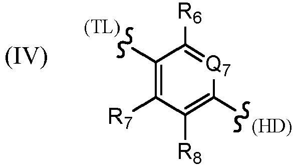 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   optionally R₇ and R₈ taken together, along with the carbon atoms to which they are attached, form a 4-, 5- or 6-membered non-aromatic carbocyclic, heterocycloalkyl, aryl, or heteroaryl ring
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
iii) HD is a moiety of Formula V or VI: 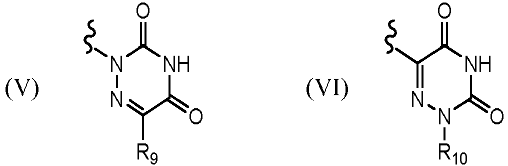 wherein:
   R₉ is selected from hydrogen, -(C(R_{d})₂)ₙ-C(R_{d})₃, -(C(R_{d})₂)ₙ-OR_{d}, -(C(R_{d})₂)ₙ-N(R_{d})₂, -(C(R_{d})₂)ₙ-S(=O)_{q}R_{d}, -(C(R_{d})₂)ₙ-CN, -(C(R_{d})₂)ₙ-C≡C-R_{d}, -(C(R_{d})₂)ₙ-C(=O)-OR_{d}, -(C(R_{d})₂)ₙ-HeAr, or -(C(R_{d})₂)ₙ-C(=O)-N(R_{d})₂; wherein
   each R_{d} is independently hydrogen or optionally substituted lower alkyl;
   each q is independently selected from 0, 1, or 2;
   each n is independently selected from 0, 1, 2, 3, 4, or 5;
   HeAr is a 5- or 6- membered heteroaryl; and
   R₁₀ is hydrogen or -C(Rₑ)₃, wherein each Rₑ is independently hydrogen, halogen, or optionally substituted lower alkyl; and
(iv) Lₐ is independently a bond; -(C(Rₐ)₂)_{z}-; oxygen; sulfur; or -NRₐ-; wherein:
   each Rₐ is independently a hydrogen or lower alkyl; and
   z is 0, 1, 2, 3, 4 or 5;
   provided that:
      (1) when TL is a moiety of Formula IIIa, wherein Q₄, Q₅, and Q₆ are -CH-, and R₄ is an optionally substituted C₁-C₃alkyl group, an optionally substituted sulfamoyl group, or an optionally substituted carbamoyl group; HD is a moiety of Formula V, wherein R₉ is H or - CN; and Q₇ is -CH-, then R₅ cannot be hydroxy;
      (2) when TL is a moiety of Formula IIa, wherein Q₁, Q₂, and Q₃ are -CH-, R₁ is -CH₃, and R₂ is hydrogen; HD is a moiety of Formula V, wherein R₉ is hydrogen; Q₇ is -CH-; R₆ is halogen or methyl; and R₇ and R₈ taken together, along with the carbon atoms to which they are attached, form a 5-membered non-aromatic carbocyclic ring, then R₄ and R₅ taken together along with the carbon atoms to which they are attached cannot form a five-membered heteroaryl group;
      (3) when TL is a moiety of Formula IIIa, wherein Q₄ is nitrogen, Q₅ and Q₆ are -CH-, and R₅ is -OH; HD is a moiety of Formula V, wherein R₉ is -CN; Q₇ is -CH-; Lₐ is -CH₂-, then R₄ cannot be cyclohexyl, cycloheptyl, isopropyl, or optionally substituted benzene;
      (4) when TL is a moiety of Formula IIIb, wherein Q₄ is -CH- and Q₅ is nitrogen; HD is a moiety of Formula V, wherein R₉ is hydrogen, -CN, or -CO₂H; Q₇ is -CH-; and R₆ and R₇ are independently halogen or methyl, or R₇ and R₈ taken together, along with the carbon atoms to which they are attached, form a 5-membered non-aromatic carbocyclic ring, then R₄ cannot be isopropyl or 2-hydroxy-1-methyl-ethyl;
      (5) when TL is a moiety of Formula IIIb, wherein Q₄ is -CR_{c}- and Q₅ is nitrogen; HD is a moiety of Formula V, wherein R₉ is -CN; Q₇ is -CH-; and Lₐ is -O-, then
         (a) none of R₆, R₇, and R₈ can be deuterium;
         (b) Q₄ cannot be -CD-, wherein D is deuterium; and
         (c) R₄ cannot be a three- to five-membered cycloalkyl ring optionally substituted with one or more halogens; non-aromatic (carbocyclic)alkyl optionally substituted with methyl or hydroxy; (1H-pyrazol-4-yl)methyl; (3-methylisoxazol-5-yl)methyl; phenyl; benzyl optionally substituted with methyl, halogen, hydroxy, or methoxy; phenethyl optionally substituted with halogen; C₁-C₄ alkyl optionally substituted with one to six substituents selected from halogen, hydroxyl, and deuterium; (tetrahydro-2H-pyran-4-yl)methyl; or a three- to five-membered heterocycloalkyl optionally substituted with benzyl;
      (6) when TL is a moiety of Formula IIIb, wherein (i) Q₄ and Q₅ are nitrogen, (ii) Q₄ and Q₅ are -CR_{b}-, or (iii) Q₄ is nitrogen and Q₅ is -CR_{c}-; HD is a moiety of Formula V, wherein R₉ is -CN; and Q₇ is -CH-; then R₄ cannot be -CH(CH₃)₂ or -CH(CD₃)₂;
      (7) when TL is a moiety of Formula IIIa, wherein Q₄ is -CH-, Q₅ and Q₆ are nitrogen, and R₄ is isopropyl; HD is a moiety of Formula V, wherein R₉ is -CN; and Q₇ is -CH-; then R₅ cannot be -NH₂ or -NHCH₃;
      (8) when TL is a moiety of Formula IIIb, wherein Q₄ is -CH- and Q₅ is nitrogen; Lₐ is -O-; Q₇ is -CH-; and HD is a moiety of Formula V, then R₉ cannot be methyl;
      (9) when TL is a moiety of Formula IIIb, wherein Q₄ is -CH-, Q₅ is nitrogen, and R₄ is isopropyl; Lₐ is -O-; Q₇ is -CH-; and HD is a moiety of Formula V, then R₉ cannot be isopropyl;
      (10) when TL is a moiety of Formula IIa, wherein Q₁, Q₂, and Q₃ are -CH-; Lₐ is -O-; Q₇ is -CH-; R₆ and R₇ are independently chlorine or trifluoromethyl; and HD is a moiety of Formula V, wherein R₉ is -CN or methyl, then R₁ cannot be isopropyl, 4-tetrahydropyranyl, or -C(O)NH₂;
      (11) when TL is a moiety of Formula IIa, wherein Q₁ and Q₂ are -CH-, and Q₃ is nitrogen; Lₐ is -O-; Q₇ is -CH-; R₆ and R₇ are chlorine; and HD is a moiety of Formula V, wherein R₉ is -CN, then R₁ cannot be isopropyl;
      (12) when TL is a moiety of Formula IIb, wherein Q₁, Q₂, and Q₃ are -CH-; Lₐ is -O-; Q₇ is -CH-; R₆ and R₇ are chlorine; and HD is a moiety of Formula V, wherein R₉ is -CN, then R₁ cannot be isopropyl;
      (13) when TL is a moiety of Formula IIIb, wherein Q₄ is -CH- and Q₅ is nitrogen; Lₐ is -O-; Q₇ is -CH-; R₆ and R₇ are chlorine; and HD is a moiety of Formula VI, wherein R₁₀ is H, Me, Et, isopropyl, -CH₂CF₃, or -CH₂CHF₂, then R₄ cannot be C₂-C₅ alkyl or C₁-C₃ hydroxyalkyl;
      (14) when TL is a moiety of Formula IIIb, wherein Q₄ is -CH- and Q₅ is nitrogen; Lₐ is -O-; Q₇ is -CH-; R₆ and R₇ are chlorine; and HD is a moiety of Formula VI, wherein R₁₀ is H or Me, then R₄ cannot be cyclopropyl;
      (15) when TL is a moiety of Formula IIIb, wherein Q₄ is -CH- and Q₅ is nitrogen; Lₐ is -CH₂-; Q₇ is -CH-; R₆ and R₇ are both chlorine or both methyl; and HD is a moiety of Formula VI, wherein R₁₀ is H or Me, then R₄ cannot be isopropyl; and
      (16) when TL is a moiety of Formula IIIb, wherein Q₄ is -CH- and Q₅ is nitrogen; HD is a moiety of Formula V, wherein R₉ is -CH₂CN or -C≡CH; Q₇ is -CH-; and R₆ and R₇ are chlorine, then R₄ cannot be isopropyl.

In another aspect, disclosed herein are compounds of Formula I' (according to the invention if TL is (IIIb), Lₐ is as defined in claim 1, CE is formula (IV), and HD is formula (V)):

(I') TL-Lₐ-CE-HD

or a stereoisomer or a tautomer thereof, or a pharmaceutically acceptable salt, prodrug, amide or ester thereof, wherein:
i) TL is a moiety of Formula IIa, IIb, IIIa, IIIb, IIIc, or IIId: 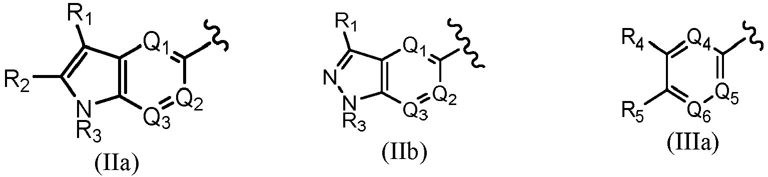 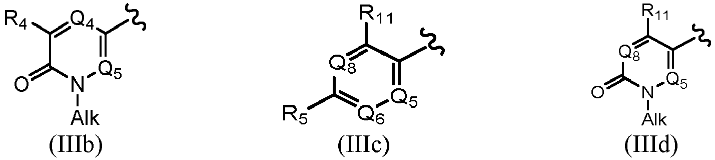 wherein:
   each of Q₁, Q₂, Q₃, Q₄, Q₅, Q₆, and Q₈, is independently nitrogen or -CR_{b}-, wherein each R_{b} is independently hydrogen, halogen, or lower alkyl;
   R₁ is hydrogen, an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   R₂ is hydrogen, halogen, optionally substituted alkyl, optionally substituted cycloalkyl, or -CN;
   R₃ is hydrogen or lower alkyl;
   R₄ is an optionally substituted alkyl, an optionally substituted alkenyl, an optionally substituted non-aromatic carbocyclic group, an optionally substituted aryl group, an optionally substituted heterocyclic group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
   R₅ is hydroxy, NH₂, alkylamino, alkanoylamino, or alkylsulfonylamino;
   or R₄ and R₅ taken together along with the carbon atoms to which they are attached form a five- or six-membered optionally substituted non-aromatic carbocyclic group, optionally substituted aryl group, optionally substituted heterocyclic group, or optionally substituted heteroaryl group;
   or R₄ and R₅ taken together along with the carbon atoms to which they are attached form a seven to eleven membered, optionally substituted spirocyclic ring or a seven to eleven membered, optionally substituted spiro-heterocyclic ring;
   Alk is hydrogen or an optionally substituted alkyl; and
   R₁₁ is an aryl group optionally substituted with one to five substituents independently selected from lower alkyl, halogen, and cycloalkyl; or a bicyclic ring system containing either aromatic or saturated rings; or a bicyclic heterocyclic containing either aromatic or saturated ring systems;
ii) CE is a moiety of Formula IV 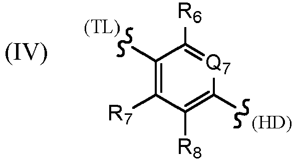 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   optionally R₇ and R₈ taken together, along with the carbon atoms to which they are attached, form a 4-, 5- or 6-membered non-aromatic carbocyclic, heterocycloalkyl, aryl, or heteroaryl ring
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
iii) HD is a moiety of Formula V or VI: 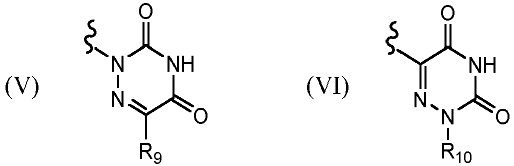 wherein:
   R₉ is selected from hydrogen, -(C(R_{d})₂)ₙ-C(R_{d})₃, -(C(R_{d})₂)ₙ-OR_{d}, -(C(R_{d})₂)ₙ-N(R_{d})₂, -(C(R_{d})₂)ₙ-S(=O)_{q}R_{d}, -(C(R_{d})₂)ₙ-CN, -(C(R_{d})₂)ₙ-C≡C-R_{d}, -(C(R_{d})₂)ₙ-C(=O)-OR_{d}, -(C(R_{d})₂)ₙ-HeAr, or -(C(R_{d})₂)ₙ-C(=O)-N(R_{d})₂; wherein
   each R_{d} is independently hydrogen or optionally substituted lower alkyl;
   each q is independently selected from 0, 1, or 2;
   each n is independently selected from 0, 1, 2, 3, 4, or 5;
   HeAr is a 5- or 6- membered heteroaryl; and
   R₁₀ is hydrogen or -C(Rₑ)₃, wherein each Rₑ is independently hydrogen, halogen, or optionally substituted lower alkyl; and
   Lₐ is independently a bond; -(C(Rₐ)₂)_{z}-; oxygen; sulfur; or -NRₐ-; wherein:
   each Rₐ is independently a hydrogen or lower alkyl; and
   z is 0, 1, 2, 3, 4 or 5;
   provided that:
      (1) when TL is a moiety of Formula IIIa, wherein Q₄, Q₅, and Q₆ are -CH-, and R₄ is an optionally substituted C₁-C₃alkyl group, an optionally substituted sulfamoyl group, or an optionally substituted carbamoyl group; HD is a moiety of Formula V, wherein R₉ is H or - CN; and Q₇ is -CH-, then R₅ cannot be hydroxy;
      (2) when TL is a moiety of Formula IIa, wherein Q₁, Q₂, and Q₃ are -CH-, R₁ is -CH₃, and R₂ is hydrogen; HD is a moiety of Formula V, wherein R₉ is hydrogen; Q₇ is -CH-; R₆ is halogen or methyl; and R₇ and R₈ taken together, along with the carbon atoms to which they are attached, form a 5-membered non-aromatic carbocyclic ring, then R₄ and R₅ taken together along with the carbon atoms to which they are attached cannot form a five-membered heteroaryl group;
      (3) when TL is a moiety of Formula IIIa, wherein Q₄ is nitrogen, Q₅ and Q₆ are -CH-, and R₅ is -OH; HD is a moiety of Formula V, wherein R₉ is -CN; Q₇ is -CH-; Lₐ is -CH₂-, then R₄ cannot be cyclohexyl, cycloheptyl, isopropyl, or optionally substituted benzene;
      (4) when TL is a moiety of Formula IIIb, wherein Q₄ is -CH- and Q₅ is nitrogen; HD is a moiety of Formula V, wherein R₉ is hydrogen, -CN, or -CO₂H; Q₇ is -CH-; and R₆ and R₇ are independently halogen or methyl, or R₇ and R₈ taken together, along with the carbon atoms to which they are attached, form a 5-membered non-aromatic carbocyclic ring, then R₄ cannot be isopropyl or 2-hydroxy-1-methyl-ethyl;
      (5) when TL is a moiety of Formula IIIb, wherein Q₄ is -CR_{c}- and Q₅ is nitrogen; HD is a moiety of Formula V, wherein R₉ is -CN; Q₇ is -CH-; and Lₐ is -O-, then
         (a) none of R₆, R₇, and R₈ can be deuterium;
         (b) Q₄ cannot be -CD-, wherein D is deuterium; and
         (c) R₄ cannot be a three- to five-membered cycloalkyl ring optionally substituted with one or more halogens; C₁-C₄ alkyl optionally substituted with one to six substituents selected from halogen, hydroxyl, and deuterium; or a three- to five-membered heterocycloalkyl;
      (6) when TL is a moiety of Formula IIIb, wherein (i) Q₄ and Q₅ are nitrogen, (ii) Q₄ and Q₅ are -CR_{b}-, or (iii) Q₄ is nitrogen and Q₅ is -CR_{c}-; HD is a moiety of Formula V, wherein R₉ is -CN; and Q₇ is -CH-; then R₄ cannot be -CH(CH₃)₂ or -CH(CD₃)₂; and
      (7) when TL is a moiety of Formula IIIa, wherein Q₄ is -CH-, Q₅ and Q₆ are nitrogen, and R₄ is isopropyl; HD is a moiety of Formula V, wherein R₉ is -CN; and Q₇ is -CH-; then R₅ cannot be -NH₂ or -NHCH₃.
In some embodiments, when TL is a moiety of Formula IIIb, wherein Q₄ is -CH- and Q₅ is nitrogen; HD is a moiety of Formula V, wherein R₉ is -CH₂CN or -C≡CH; Q₇ is -CH-; and R₆ and R₇ are chlorine, then R₄ cannot be isopropyl.

In another aspect, disclosed herein are compounds of Formula I' (according to the invention if TL is (IIIb), Lₐ is as defined in claim 1, CE is formula (IV), and HD is formula (V)):

(I') TL-Lₐ-CE-HD

or a stereoisomer or a tautomer thereof, or a pharmaceutically acceptable salt, prodrug, amide or ester thereof, wherein:
i) TL is a moiety of Formula IIa, IIb, IIIa, IIIb, IIIc, or IIId: 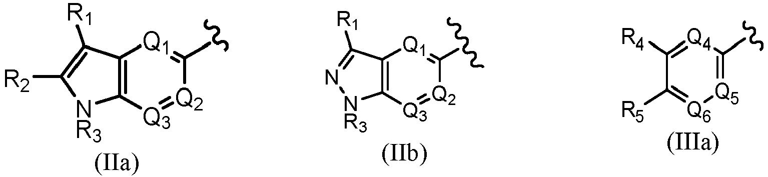 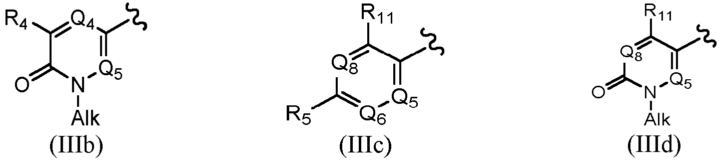 wherein:
   each of Q₁, Q₂, Q₃, Q₄, Q₅, Q₆, and Q₈, is independently nitrogen or -CR_{b}-, wherein each R_{b} is independently hydrogen, halogen, or lower alkyl;
   R₁ is hydrogen, an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
   R₂ is hydrogen, halogen, optionally substituted alkyl, optionally substituted cycloalkyl, or -CN;
   R₃ is hydrogen or lower alkyl;
   R₄ is an optionally substituted alkyl, an optionally substituted alkenyl, an optionally substituted non-aromatic carbocyclic group, an optionally substituted aryl group, an optionally substituted heterocyclic group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
   R₅ is hydroxy, NH₂, alkylamino, alkanoylamino, or alkylsulfonylamino;
   or R₄ and R₅ taken together along with the carbon atoms to which they are attached form a five- or six-membered optionally substituted non-aromatic carbocyclic group, optionally substituted aryl group, optionally substituted heterocyclic group, or optionally substituted heteroaryl group;
   or R₄ and R₅ taken together along with the carbon atoms to which they are attached form a seven to eleven membered, optionally substituted spirocyclic ring or a seven to eleven membered, optionally substituted spiro-heterocyclic ring;
   Alk is hydrogen or an optionally substituted alkyl; and
   R₁₁ is an aryl group optionally substituted with one to five substituents independently selected from lower alkyl, halogen, and cycloalkyl; or a bicyclic ring system containing either aromatic or saturated rings; or a bicyclic heterocyclic containing either aromatic or saturated ring systems;
ii) CE is a moiety of Formula IV 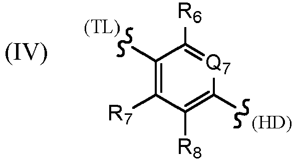 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   optionally R₇ and R₈ taken together, along with the carbon atoms to which they are attached, form a 4-, 5- or 6-membered non-aromatic carbocyclic, heterocycloalkyl, aryl, or heteroaryl ring
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
iii) HD is a moiety of Formula V or VI: 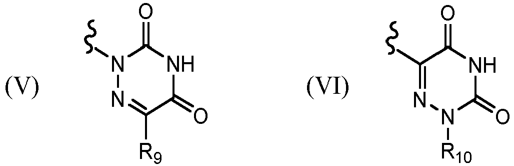 wherein:
   R₉ is selected from hydrogen, -(C(R_{d})₂)ₙ-C(R_{d})₃, -(C(R_{d})₂)ₙ-OR_{d}, -(C(R_{d})₂)ₙ-N(R_{d})₂, -(C(R_{d})₂)ₙ-S(=O)_{q}R_{d}, -(C(R_{d})₂)ₙ-CN, -(C(R_{d})₂)ₙ-C≡C-R_{d}, -(C(R_{d})₂)ₙ-C(=O)-OR_{d}, -(C(R_{d})₂)ₙ-HeAr, or -(C(R_{d})₂)ₙ-C(=O)-N(R_{d})₂; wherein
   each R_{d} is independently hydrogen or optionally substituted lower alkyl;
   each q is independently selected from 0, 1, or 2;
   each n is independently selected from 0, 1, 2, 3, 4, or 5;
   HeAr is a 5- or 6- membered heteroaryl; and
   R₁₀ is hydrogen or -C(Rₑ)₃, wherein each Rₑ is independently hydrogen, halogen, or optionally substituted lower alkyl; and
(iv) Lₐ is independently a bond; -(C(Rₐ)₂)_{z}-; oxygen; sulfur; or -NRₐ-; wherein:
   each Rₐ is independently a hydrogen or lower alkyl; and
   z is 0, 1, 2, 3, 4 or 5;
   provided that:
      (1) when TL is a moiety of Formula IIIa, wherein Q₄, Q₅, and Q₆ are -CH-, and R₄ is an optionally substituted C₁-C₃alkyl group, an optionally substituted sulfamoyl group, or an optionally substituted carbamoyl group; HD is a moiety of Formula V, wherein R₉ is H or - CN; and Q₇ is -CH-, then R₅ cannot be hydroxy;
      (2) when TL is a moiety of Formula IIa, wherein Q₁, Q₂, and Q₃ are -CH-, R₁ is -CH₃, and R₂ is hydrogen; HD is a moiety of Formula V, wherein R₉ is hydrogen; Q₇ is -CH-; R₆ is halogen or methyl; and R₇ and R₈ taken together, along with the carbon atoms to which they are attached, form a 5-membered non-aromatic carbocyclic ring, then R₄ and R₅ taken together along with the carbon atoms to which they are attached cannot form a five-membered heteroaryl group;
      (3) when TL is a moiety of Formula IIIa, wherein Q₄ is nitrogen, Q₅ and Q₆ are -CH-, and R₅ is -OH; HD is a moiety of Formula V, wherein R₉ is -CN; Q₇ is -CH-; Lₐ is -CH₂-, then R₄ cannot be cyclohexyl, cycloheptyl, isopropyl, or optionally substituted benzene;
      (4) when TL is a moiety of Formula IIIb, wherein Q₄ is -CH- and Q₅ is nitrogen; HD is a moiety of Formula V, wherein R₉ is hydrogen, -CN, -CH₂CN, -C≡CH, or -CO₂H; Q₇ is - CH-; and R₆ and R₇ are independently halogen or methyl, or R₇ and R₈ taken together, along with the carbon atoms to which they are attached, form a 5-membered non-aromatic carbocyclic ring, then R₄ cannot be isopropyl or 2-hydroxy-1-methyl-ethyl;
      (5) when TL is a moiety of Formula IIIb, wherein Q₄ is -CR_{c}- and Q₅ is nitrogen; HD is a moiety of Formula V, wherein R₉ is -CN; Q₇ is -CH-; and Lₐ is -O-, then
         (a) none of R₆, R₇, and R₈ can be deuterium;
         (b) Q₄ cannot be -CD-, wherein D is deuterium; and
         (c) R₄ cannot be a three- to five-membered cycloalkyl ring optionally substituted with one or more halogens; C₁-C₄ alkyl optionally substituted with one to six substituents selected from halogen, hydroxyl, and deuterium; or a three- to five-membered heterocycloalkyl;
      (6) when TL is a moiety of Formula IIIb, wherein (i) Q₄ and Q₅ are nitrogen, (ii) Q₄ and Q₅ are -CR_{b}-, or (iii) Q₄ is nitrogen and Q₅ is -CR_{c}-; HD is a moiety of Formula V, wherein R₉ is -CN; and Q₇ is -CH-; then R₄ cannot be -CH(CH₃)₂ or -CH(CD₃)₂; and
      (7) when TL is a moiety of Formula IIIa, wherein Q₄ is -CH-, Q₅ and Q₆ are nitrogen, and R₄ is isopropyl; HD is a moiety of Formula V, wherein R₉ is -CN; and Q₇ is -CH-; then R₅ cannot be -NH₂ or -NHCH₃.

In some embodiments of the compound of Formula I' (according to the invention if TL is (IIIb), Lₐ is as defined in claim 1, CE is formula (IV), and HD is formula (V)), TL is a moiety of Formula IIa, IIb, IIIa, IIIb, IIIc, or IIId; 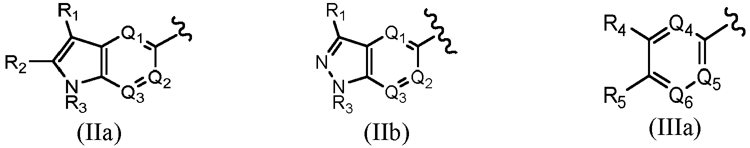 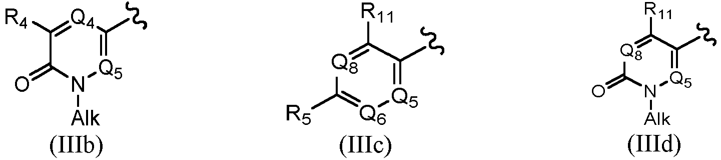 wherein:
each of Q₁, Q₂, Q₃, Q₄, Q₅, Q₆ and Q₈, is independently nitrogen or -CR_{b}-, wherein each R_{b} is independently hydrogen, halogen, or lower alkyl;
R₁ is an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
R₂ is halogen, optionally substituted alkyl, optionally substituted cycloalkyl, or -CN;
R₃ is hydrogen;
R₄ is an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
R₅ is hydroxy, NH₂, alkylamino, alkanoylamino, or alkylsulfonylamino;
or R₄ and R₅ taken together along with the carbon atoms to which they are attached form a five- or six-membered optionally substituted non-aromatic carbocyclic group, optionally substituted aryl group, optionally substituted heterocycloalkyl group, or optionally substituted heteroaryl group;
or R₄ and R₅ taken together along with the carbon atoms to which they are attached form a seven to eleven membered, optionally substituted spirocyclic ring or a seven to eleven membered, optionally substituted spiro-heterocyclic ring; and
Alk is hydrogen or an optionally substituted alkyl;
   CE is a moiety of Formula IV 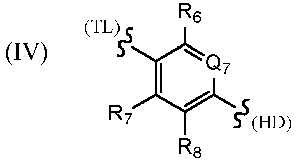 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
      HD is a moiety of Formula V or VI: 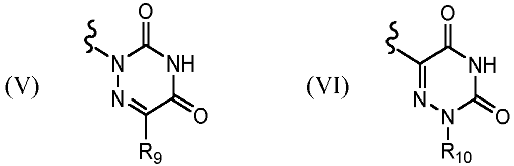 wherein:
      R₉ is selected from -NH₂, -CN, -CH₂-S-CH₃, or -CH₂-S(=O)₂-CH₃;
      R₁₀ is -CH₃; and
      Lₐ is oxygen or -CH₂-.

In some embodiments of the compound of Formula I' not according to the invention,
TL is a moiety of Formula IIa: 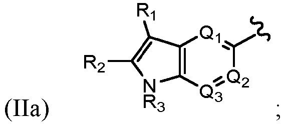 and
HD is a moiety of Formula V:

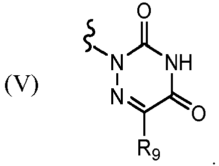

In some embodiments of the compound of Formula I' not according to the invention:
TL is a moiety of Formula IIa: 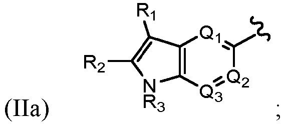 and
HD is a moiety of Formula VI:

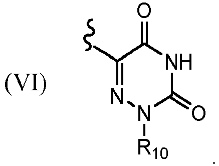

In some embodiments of the compound of Formula I' not according to the invention:
TL is a moiety of Formula IIb: 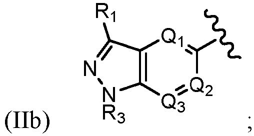 and
HD is a moiety of Formula V:

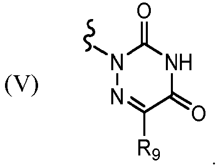

In some embodiments of the compound of Formula I' not according to the invention:
TL is a moiety of Formula IIb: 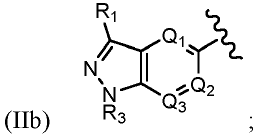 and
HD is a moiety of Formula VI:

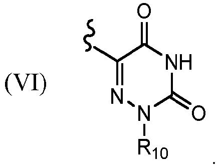

In some embodiments of the compound of Formula I' not according to the invention,
TL is a moiety of Formula IIIa: 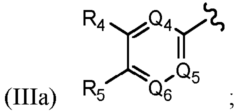 and
HD is a moiety of Formula V:

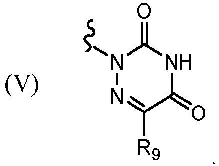

In some embodiments of the compound of Formula I' not according to the invention,
TL is a moiety of Formula IIIa: 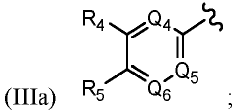 and
HD is a moiety of Formula VI:

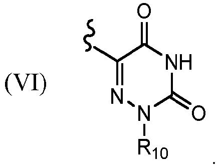

In the compound of Formula I' according to the invention,
TL is a moiety of Formula IIIb: 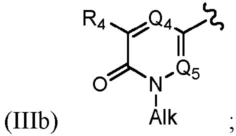 and
HD is a moiety of Formula V: 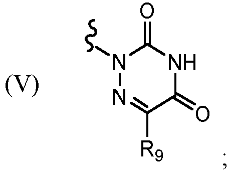
wherein:
R₉ is -NH₂.

In some embodiments of the compound of Formula I' not according to the invention,
TL is a moiety of Formula IIIb: 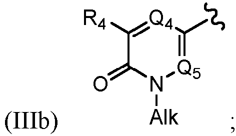 and
HD is a moiety of Formula VI:

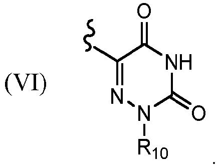

In some embodiments of the compound of Formula I' not according to the invention,
TL is a moiety of Formula IIIc: 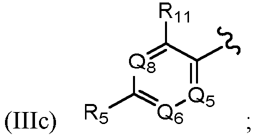 and
HD is a moiety of Formula V:

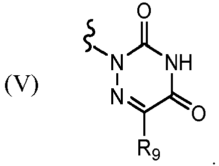

In some embodiments of the compound of Formula I' not according to the invention,
TL is a moiety of Formula IIIc: 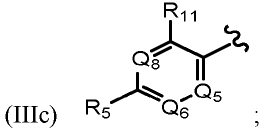 and
HD is a moiety of Formula VI:

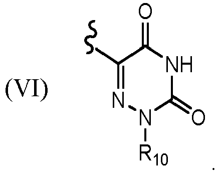

In some embodiments of the compound of Formula I' not according to the invention,
TL is a moiety of Formula IIId: 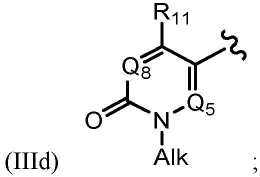 and
HD is a moiety of Formula V:

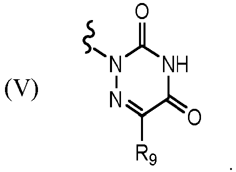

In some embodiments of the compound of Formula I' not according to the invention,
TL is a moiety of Formula IIId: 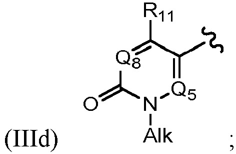 and
HD is a moiety of Formula VI:

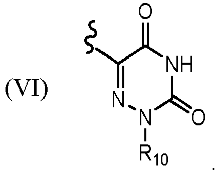

In some embodiments of the compound of Formula I' not according to the invention,
TL is a moiety of Formula II: 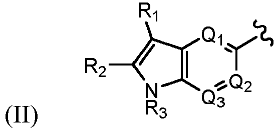 wherein:
each of Q₁, Q₂, and Q₃ is independently nitrogen or -CR_{b}-, wherein each R_{b} is independently hydrogen, halogen, or lower alkyl;
R₁ is an optionally substituted alkyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted C-carboxy or O-carboxy group, -CN, an optionally substituted carbamoyl group, or an optionally substituted carbamoyl alkyl group, where the nitrogen of the carbamoyl or carbamoyl alkyl group is optionally a heteroatom in a ring structure;
R₂ is halogen, optionally substituted alkyl, optionally substituted cycloalkyl, or -CN;
R₃ is hydrogen;

CE is a moiety of Formula IV 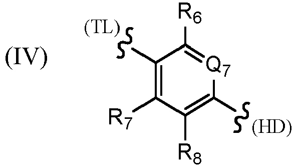 wherein:
   each of R₆ and R₇ is independently selected from halogen, -CN, optionally substituted lower alkyl, optionally substituted lower alkoxy, optionally substituted lower alkenyl, or cyclopropyl;
   R₈ is selected from hydrogen, optionally substituted lower alkyl, optionally substituted lower alkoxy, cyano, or halogen;
   Q₇ is nitrogen or -CR_{c}-, wherein R_{c} is hydrogen, halogen, or lower alkyl;
   (TL) denotes the point where the moiety of Formula IV connects to TL-Lₐ-; and
   (HD) denotes the point where the moiety of Formula IV connects to -HD;
HD is a moiety of Formula V: 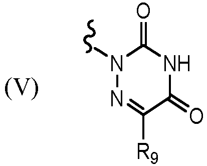 wherein
   R₉ is hydrogen, -CN, -NH₂, -C(R_{d})₂-S-R_{d}, -C(R_{d})₂-S(=O)₂R_{d}, or -C≡C-R_{d}, wherein each R_{d} is independently hydrogen or lower alkyl; and
   Lₐ is oxygen or -CH₂-.

In some embodiments of the compound of Formula I', Q₁, Q₂, Q₃, and Q₄ are -CR_{b}-, where each R_{b} is independently selected from the group consisting of hydrogen, methyl, ethyl, *n*-propyl, *i*-propyl, *n*-butyl, *i*-butyl, and *t*-butyl. In some embodiments of the compound of Formula I', Q₁, Q₂, and Q₃ are -CH-.

In some embodiments of the compound of Formula I', Q₁ is -CH-, and Q₂ and Q₃ are nitrogen. In some embodiments of the compound of Formula I', Q₂ is -CH-, and Q₁ and Q₃ are nitrogen. In some embodiments of the compound of Formula I', Q₃ is -CH-, and Q₁ and Q₂ are nitrogen.

In some embodiments of the compound of Formula I', Q₁ is nitrogen, and Q₂ and Q₃ are -CH-. In some embodiments of the compound of Formula I', Q₂ is nitrogen, and Q₁ and Q₃ are -CH-. In some embodiments of the compound of Formula I', Q₃ is nitrogen, and Q₁ and Q₂ are -CH-.

In some embodiments of the compound of Formula I', Q₄, Q₅, and Q₆ are - CR_{b}-, where each R_{b} is independently selected from the group consisting of hydrogen, methyl, ethyl, *n*-propyl, *i*-propyl, *n*-butyl, *i*-butyl, and *t*-butyl. In some embodiments of the compound of Formula I', Q₄, Q₅, and Q₆ are -CH-.

In some embodiments of the compound of Formula I', Q₄ is -CH-, and Q₅ and Q₆ are nitrogen. In some embodiments of the compound of Formula I', Q₅ is -CH-, and Q₄ and Q₆ are nitrogen. In some embodiments of the compound of Formula I', Q₆ is -CH-, and Q₄ and Q₅ are nitrogen.

In some embodiments of the compound of Formula I', Q₄ is nitrogen, and Q₅ and Q₆ are -CH-. In some embodiments of the compound of Formula I', Q₅ is nitrogen, and Q₄ and Q₆ are -CH-. In some embodiments of the compound of Formula I', Q₆ is nitrogen, and Q₄ and Q₅ are -CH-.

In the compound of Formula I' according to the invention, Q₄ is -CH- and Q₅ is nitrogen.

In some embodiments of the compound of Formula I', Q₅ and Q₆ are nitrogen.

In some embodiments of the compound of Formula I', Q₅ is nitrogen and Q₈ is -CH-.

In some embodiments of the compound of Formula I', Q₅, Q₆, and Q₈ are - CR_{b}-, where each R_{b} is independently selected from the group consisting of hydrogen, methyl, ethyl, *n*-propyl, *i*-propyl, *n*-butyl, *i*-butyl, and *t*-butyl. In some embodiments of the compound of Formula I', Q₅, Q₆, and Q₈ are -CH-.

In some embodiments of the compound of Formula I', Q₅ is -CH-, and Q₆ and Q₈ are nitrogen. In some embodiments of the compound of Formula I', Q₆ is -CH-, and Q₅ and Q₈ are nitrogen. In some embodiments of the compound of Formula I', Q₈ is -CH-, and Q₅ and Q₆ are nitrogen.

In some embodiments of the compound of Formula I', R₁ is hydrogen, an optionally substituted alkyl, an optionally substituted non-aromatic carbocyclic group, an optionally substituted aryl group, and an optionally substituted C-carboxy group. In some embodiments of the compound of Formula I', the alkyl is selected from the group consisting of methyl, ethyl, *n*-propyl, *i*-propyl, *n*-butyl, *i*-butyl, and *t*-butyl. In some embodiments of the compound of Formula I', at least one carbon atom of the listed alkyl moieties is perfluorinated. In some embodiments of the compound of Formula I', the alkyl is substituted with cycloalkyl or aryl. In some embodiments of the compound of Formula I', the cycloalkyl is selected from the group consisting of cyclopropyl, cyclopentyl, and cyclohexyl. In some embodiments of the compound of Formula I', the aryl is optionally substituted phenyl. In some embodiments of the compound of Formula I', the carbocyclic group is cyclohexane or cyclopentane. In some embodiments of the compound of Formula I', the aryl group is phenyl. In some embodiments of the compound of Formula I', the C-carboxy group is a moiety of formula -C(=O)-O-R, where R is selected from the group consisting of methyl, ethyl, *n-*propyl, *i*-propyl, *n*-butyl, *i*-butyl, and *t*-butyl.

In some embodiments of the compound of Formula I', R₁ is hydrogen, C₁₋C₆ alkyl, a non-aromatic C₃-C₁₂ carbocyclic group, a C₆-C₁₀ aryl group, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, a (carbocyclic)alkyl group, an aralkyl group, a (heterocycloalkyl)alkyl group, a (heteroaryl)alkyl group, an amino group, a C-carboxy or O-carboxy group, -CN, or a carbamoyl group; and R₁ is optionally substituted with one to five Rₖ independently selected from the group consisting of hydroxy, halogen, -CN, amino, oxo, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is hydrogen. In some embodiments of the compound of Formula I', R₁ is -CN.

In some embodiments of the compound of Formula I', R₁ is an optionally substituted C₁₋C₆ alkyl. In some embodiments of the compound of Formula I', R₁ is C₁₋C₆ alkyl optionally substituted with one to five Rₖ independently selected from the group consisting of hydroxy, halogen, -CN, amino, O-carboxy, C-carboxy, C-amido, N-amido, C₁-C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₁ is C₁₋C₆ alkyl.

In some embodiments of the compound of Formula I', R₁ is an optionally substituted non-aromatic carbocyclic group. In some embodiments of the compound of Formula I', R₁ is a non-aromatic C₃-C₁₂ carbocyclic group optionally substituted with one to five Rₖ independently selected from the group consisting of hydroxy, oxo, halogen, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted aryl group. In some embodiments of the compound of Formula I', R₁ is a C₆-C₁₀ aryl group optionally substituted with one to five Rₖ independently selected from the group consisting of hydroxy, halogen, -CN, O-carboxy, C-carboxy, C-amido, N-amido, amino, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted heterocycloalkyl group. In some embodiments of the compound of Formula I', R₁ is a 3- to 6-membered heterocycloalkyl ring containing one to four ring heteroatoms independently selected from oxygen, sulfur, or nitrogen, wherein the heterocycloalkyl ring is optionally substituted with one to five Rₖ independently selected from the group consisting of hydroxy, oxo, halogen, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted heteroaryl group. In some embodiments of the compound of Formula I', R₁ is a five- to ten-membered heteroaryl group containing one to four ring heteroatoms independently selected from oxygen, sulfur, or nitrogen, wherein the heteroaryl group is optionally substituted with one to five Rₖ independently selected from the group consisting of hydroxy, halogen, -CN, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted (carbocyclic)alkyl group. In some embodiments of the compound of Formula I', R₁ is (cyclopentyl)C₁₋C₆ alkyl, (cyclobutyl)C₁₋C₆ alkyl, (cyclopentyl)C₁₋C₆ alkyl, (cyclohexyl)C₁₋C₆ alkyl, (cycloheptyl)C₁₋C₆ alkyl, (cyclooctyl)C₁₋C₆ alkyl, or (cyclononyl)C₁₋C₆ alkyl, and R₁ is optionally substituted with one to five Rₖ independently selected from the group consisting of hydroxy, halogen, -CN, amino, O-carboxy, C-carboxy, C-amido, N-amido, oxo, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted aralkyl group. In some embodiments of the compound of Formula I', R₁ is a benzyl group optionally substituted with one to five Rₖ independently selected from the group consisting of hydroxy, halogen, -CN, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted (heterocycloalkyl)alkyl group. In some embodiments of the compound of Formula I', R₁ is a (heterocycloalkyl)alkyl group optionally substituted with one to five Rₖ independently selected from the group consisting of hydroxy, oxo, halogen, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted (heteroaryl)alkyl group. In some embodiments of the compound of Formula I', R₁ is a (heteroaryl)alkyl group optionally substituted with one to five Rₖ independently selected from the group consisting of hydroxy, halogen, -CN, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3-to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted amino group. In some embodiments of the compound of Formula I', R₁ is an amino group optionally substituted with one or two substituents independently selected from the group consisting of C₁₋C₆ alkyl, C₁₋C₆ haloalkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, C₆-C₁₀ aryl, C-carboxy, C-amido, -(SO₂)(C₁₋C₆ alkyl), -C(O)O(C₁₋C₆ alkyl), -C(O)O(C₁₋C₆ haloalkyl), - C(O)O(C₃₋C₉ cycloalkyl), -C(O)O(3- to 6-membered heterocycloalkyl), -C(O)O(five- to ten-membered heteroaryl), and -C(O)O(C₆-C₁₀ aryl). In some embodiments of the compound of Formula I', R₁ is -NRₘRₙ, wherein Rₘ and Rₙ are independently selected from the group consisting of hydrogen, C₁₋C₆ alkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, C₆-C₁₀ aryl, C-carboxy, C-amido, - (SO₂)(C₁₋C₆ alkyl), -C(O)O(C₁₋C₆ alkyl), -C(O)O(C₃₋C₉ cycloalkyl), -C(O)O(3- to 6-membered heterocycloalkyl), -C(O)O(five- to ten-membered heteroaryl), and -C(O)O(C₆-C₁₀ aryl); or Rₘ and Rₙ together with the nitrogen to which they are attached form a 3- to 18-membered heterocycloalkyl ring; and R₁ is optionally substituted with one to five Rₖ independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted C-carboxy group. In some embodiments of the compound of Formula I', R₁ is - C(O)(C₁₋C₆ alkyl), -C(O)(C₃₋C₉ cycloalkyl), -C(O)(3- to 6-membered heterocycloalkyl), - C(O)(five- to ten-membered heteroaryl), and -C(O)(C₆-C₁₀ aryl); and R₁ is optionally substituted with one to five Rₖ independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted O-carboxy group. In some embodiments of the compound of Formula I', R₁ is - OC(O)(C₁₋C₆ alkyl), -OC(O)(C₃₋C₉ cycloalkyl), -OC(O)(3- to 6-membered heterocycloalkyl), -OC(O)(five- to ten-membered heteroaryl), and -OC(O)(C₆-C₁₀ aryl); and R₁ is optionally substituted with one to five Rₖ independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is an optionally substituted carbamoyl group. In some embodiments of the compound of Formula I', R₁ is - C(O)NRₘRₙ, wherein Rₘ and Rₙ are independently selected from the group consisting of hydrogen, C₁₋C₆ alkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl; or Rₘ and Rₙ together with the nitrogen to which they are attached form a 3- to 18-membered heterocycloalkyl ring; and R₁ is optionally substituted with one to five Rₖ independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₁ is hydrogen, an optionally substituted C₁₋C₆ alkyl, an optionally substituted non-aromatic C₃-C₁₂ carbocyclic group, an optionally substituted C₆-C₁₀ aryl group, and an optionally substituted C-carboxy group.

In some embodiments of the compound of Formula I', R₁ is hydrogen, an optionally substituted C₁₋C₆ alkyl, an optionally substituted non-aromatic C₃-C₁₂ carbocyclic group, an optionally substituted C₆-C₁₀ aryl group, an optionally substituted C-carboxy group, and an optionally substituted carbamoyl group.

In some embodiments of the compound of Formula I', R₁ is hydrogen; -CN; C₁₋C₆ alkyl; a non-aromatic C₃-C₁₂ carbocyclic ring; a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; or -C(O)-Rⱼ; wherein Rⱼ is -NRₘRₙ or -ORₘ; Rₘ is hydrogen or C₁₋C₆ alkyl; Rₙ is C₁₋C₆ alkyl; or Rₘ and Rₙ, together with the nitrogen to which they are attached, form a ring structure; and R₁ is optionally substituted with one to five Rₖ independently selected from hydroxy, halogen, C₁-C₆ alkyl, C₁₋C₆ haloalkyl, C₁₋C₆ alkoxy, and C₆-C₁₀ aralkoxy.

In some embodiments of the compound of Formula I', R₁ is hydrogen, -CN, C₁₋C₆ alkyl; a non-aromatic C₃-C₁₂ carbocyclic ring; a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; or -C(O)-Rⱼ; wherein Rⱼ is -NRₘRₙ or -ORₘ; Rₘ is hydrogen or C₁₋C₆ alkyl; Rₙ is C₁₋C₆ alkyl; or Rₘ and Rₙ, together with the nitrogen to which they are attached, form a ring structure; and R₁ is optionally substituted with one to three Rₖ independently selected from phenyl and haloalkyl.

In some embodiments of the compound of Formula I', R₁ is hydrogen, -CN, C₁₋C₆ alkyl, cyclopentyl, phenyl, (cyclopropyl)alkyl, benzyl, or -C(O)-Rⱼ; wherein Rⱼ is - NRₘRₙ or -ORₘ; Rₘ is hydrogen or C₁₋C₆ alkyl; Rₙ is C₁₋C₆ alkyl; or Rₘ and Rₙ, together with the nitrogen to which they are attached, form 1,2,3,4-tetrahydroisoquinoline; and R₁ is optionally substituted with one to three Rₖ independently selected from phenyl and haloalkyl.

In some embodiments of the compound of Formula I', R₁ is hydrogen, -CN, C₁₋C₆ alkyl, cyclopentyl, phenyl, (cyclopropyl)alkyl, benzyl, isopropylamino, or -C(O)-Rⱼ; wherein Rⱼ is -NRₘRₙ or -ORₘ; Rₘ is hydrogen or C₁₋C₆ alkyl; Rₙ is C₁₋C₆ alkyl; or Rₘ and Rₙ, together with the nitrogen to which they are attached, form 1,2,3,4-tetrahydroisoquinoline; and R₁ is optionally substituted with one to three Rₖ independently selected from phenyl and haloalkyl.

In some embodiments of the compound of Formula I', R₂ is hydrogen, halogen, C₁₋C₆ alkyl, C₃₋C₉ cycloalkyl, or -CN; and R₂ is optionally substituted with one to five substituents independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₂ is hydrogen. In some embodiments of the compound of Formula I', R₂ is halogen. In some embodiments of the compound of Formula I', R₂ is -CN.

In some embodiments of the compound of Formula I', R₂ is optionally substituted C₁₋C₆ alkyl. In some embodiments of the compound of Formula I', R₂ is C₁₋C₆ alkyl optionally substituted with one to five substituents independently selected from the group consisting of hydroxy, halogen, -CN, amino, O-carboxy, C-carboxy, C-amido, N-amido, C₁₋C₆ alkoxy, C₆-C₁₉ aralkoxy, C₃₋C₈ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₂ is optionally substituted C₃₋C₉ cycloalkyl. In some embodiments of the compound of Formula I', R₂ is C₃₋Cs cycloalkyl optionally substituted with one to five substituents independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₂ is C₃-C₉ cycloalkyl optionally substituted with one to ten substituents independently selected from the group consisting of hydroxy, halogen, and C₁₋C₆ alkoxy.

In some embodiments of the compound of Formula I', R₂ is hydrogen or optionally substituted C₁₋C₆ alkyl. In some embodiments of the compound of Formula I', R₂ is selected from the group consisting of hydrogen, methyl, ethyl, n-propyl, i-propyl, n-butyl, i-butyl, and t-butyl.

In some embodiments of the compound of Formula I', R₂ is hydrogen; halogen; C₁₋C₆ alkyl optionally substituted with one to five substituents independently selected from the group consisting of hydroxy, halogen, and C₁₋C₆ alkoxy; C₃-C₉ cycloalkyl optionally substituted with one to ten substituents independently selected from the group consisting of hydroxy, halogen, and C₁₋C₆ alkoxy; or -CN.

In some embodiments of the compound of Formula I', R₃ is hydrogen. In some embodiments of the compound of Formula I', R₃ is lower alkyl.

In some embodiments of the compound of Formula I', R₄ is an optionally substituted alkyl, an optionally substituted alkenyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, or an optionally substituted (heterocycloalkyl)alkyl group.

In some embodiments of the compound of Formula I', R₄ is C₁₋C₆ alkyl; C₂-C₁₀ alkenyl; a non-aromatic C₃-C₁₂ carbocyclic ring; a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; or a (heterocycloalkyl)alkyl group; and R₄ is optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

In some embodiments of the compound of Formula I', R₄ is C₁₋C₆ alkyl; C₂-C₁₀ alkenyl; a non-aromatic C₃-C₁₂ carbocyclic ring; a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; or a (heterocycloalkyl)alkyl group; and R₄ is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy, or two R_{g} together with the atoms to which they are attached form a ring.

In some embodiments of the compound of Formula I', R₄ is C₁₋C₆ alkyl; C₂-C₁₀ alkenyl; a non-aromatic C₃-C₁₂ carbocyclic ring; a 3- to 6-membered heterocycloalkyl ring containing one to four ring heteroatoms independently selected from oxygen, sulfur, or nitrogen; a (carbocyclic)alkyl group; an aralkyl group; or a (heterocycloalkyl)alkyl group; and R₄ is optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

In some embodiments of the compound of Formula I', R₄ is C₁₋C₆ alkyl, C₂-C₁₀ alkenyl, a non-aromatic C₃-C₁₂ carbocyclic ring, a 3- to 6-membered heterocycloalkyl ring containing one to four ring heteroatoms independently selected from oxygen, sulfur, or nitrogen, a (carbocyclic)alkyl group, an aralkyl group, or a (heterocycloalkyl)alkyl group; and R₄ is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is C₁₋C₆ alkyl, C₂-C₁₀ alkenyl, cyclopropyl, cyclobutyl, cyclopentyl, cyclohexyl, tetrahydrofuranyl, tetrahydropyranyl, azetidinyl, pyridazin-3(2H)-one, phenyl, naphthyl, pyridinyl, cinnolinyl, isoquinolinyl, quinolinyl, pyrazolo[1,5-a]pyridinyl, imidazo[1,5-a]pyridinyl, benzo[b]thiophenyl, a (cyclobutyl)alkyl group, a (cyclopentyl)alkyl group, a benzyl group, a (tetrahydrofuranyl)alkyl group, or a (tetrahydropyranyl)alkyl group; and R₄ is optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

In some embodiments of the compound of Formula I', R₄ is C₁₋C₆ alkyl, C₂-C₁₀ alkenyl, cyclopropyl, cyclobutyl, cyclopentyl, cyclohexyl, tetrahydrofuranyl, tetrahydropyranyl, a (cyclobutyl)alkyl group, a (cyclopentyl)alkyl group, a benzyl group, a (tetrahydrofuranyl)alkyl group, or a (tetrahydropyranyl)alkyl group; and R₄ is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted alkyl. In some embodiments of the compound of Formula I', R₄ is an optionally substituted C₁₋C₆ alkyl. In some embodiments of the compound of Formula I', R₄ is C₁₋C₆ alkyl optionally substituted with one to five R_{g} independently selected from the group consisting of hydroxy, halogen, -CN, amino, O-carboxy, C-carboxy, C-amido, N-amido, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is C₁₋C₆ alkyl optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy. In some embodiments of the compound of Formula I', R₄ is selected from the group consisting of methyl, ethyl, n-propyl, i-propyl, n-butyl, i-butyl, and t-butyl. In some embodiments of the compound of Formula I', R₄ is C₁₋C₆ alkyl. In some embodiments of the compound of Formula I', R₄ is C₁-C₃ alkyl. In some embodiments of the compound of Formula I', R₄ is C₅-C₆ alkyl.

In some embodiments of the compound of Formula I', R₄ is an optionally substituted alkenyl. In some embodiments of the compound of Formula I', R₄ is an optionally substituted C₂-C₁₀ alkenyl. In some embodiments of the compound of Formula I', R₄ is C₂-C₁₀ alkenyl optionally substituted with one to five R_{g} independently selected from the group consisting of hydroxy, halogen, -CN, amino, O-carboxy, C-carboxy, C-amido, N-amido, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is C₂-C₁₀ alkenyl optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted non-aromatic carbocyclic group. In some embodiments of the compound of Formula I', R₄ is an optionally substituted non-aromatic C₃-C₁₂ carbocyclic group. In some embodiments of the compound of Formula I', R₄ is a non-aromatic C₃-C₁₂ carbocyclic group optionally substituted with one to five R_{g} independently selected from the group consisting of hydroxy, oxo, halogen, CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a non-aromatic C₃-C₁₂ carbocyclic group optionally substituted with one to five R_{g} independently selected from the group consisting of hydroxy, oxo, halogen, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is a non-aromatic C₃-C₁₂ carbocyclic group optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a non-aromatic C₃-C₁₂ carbocyclic group optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted heterocycloalkyl group. In some embodiments of the compound of Formula I', R₄ is an optionally substituted 3- to 6-membered heterocycloalkyl ring containing one to four ring heteroatoms independently selected from oxygen, sulfur, or nitrogen; wherein the heterocycloalkyl ring is optionally substituted with one to five R_{g} independently selected from the group consisting of hydroxy, oxo, halogen, CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is an optionally substituted 3- to 6-membered heterocycloalkyl ring containing one to four ring heteroatoms independently selected from oxygen, sulfur, or nitrogen; wherein the heterocycloalkyl ring is optionally substituted with one to five R_{g} independently selected from the group consisting of hydroxy, oxo, halogen, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is an optionally substituted 3- to 6-membered heterocycloalkyl ring containing one to four ring heteroatoms independently selected from oxygen, sulfur, or nitrogen; wherein the heterocycloalkyl ring is optionally substituted with one to five R_{g} independently selected from the group consisting of hydroxy, halogen, CN, oxo, C₁₋C₆ alkyl, C₆-C₁₀ aryl, C₁₋C₆ alkoxy, C₁₋C₆ haloalkoxy, and C₆-C₁₀ aralkoxy. In some embodiments of the compound of Formula I', R₄ is an optionally substituted 3- to 6-membered heterocycloalkyl ring containing one to four ring heteroatoms independently selected from oxygen, sulfur, or nitrogen; wherein the heterocycloalkyl ring is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted (carbocyclic)alkyl group. In some embodiments of the compound of Formula I', R₄ is (cyclopropyl)C₁₋C₆ alkyl, (cyclobutyl)C₁₋C₆ alkyl, (cyclopentyl)C₁₋C₆ alkyl, (cyclohexyl)C₁₋C₆ alkyl, (cycloheptyl)C₁₋C₆ alkyl, (cyclooctyl)C₁₋C₆ alkyl, or (cyclononyl)C₁₋C₆ alkyl, and R₄ is optionally substituted with one to five R_{g} independently selected from the group consisting of hydroxy, halogen, -CN, amino, O-carboxy, C-carboxy, C-amido, N-amido, oxo, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is (cyclopropyl)C₁₋C₆ alkyl, (cyclobutyl)C₁₋C₆ alkyl, (cyclopentyl)C₁₋C₆ alkyl, (cyclohexyl)C₁₋C₆ alkyl, (cycloheptyl)C₁₋C₆ alkyl, (cyclooctyl)C₁₋C₆ alkyl, or (cyclononyl)C₁₋C₆ alkyl, and R₄ is optionally substituted with one to five R_{g} independently selected from the group consisting of hydroxy, halogen, -CN, amino, O-carboxy, C-carboxy, C-amido, N-amido, oxo, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is (cyclopropyl)C₁₋C₆ alkyl, (cyclobutyl)C₁₋C₆ alkyl, (cyclopentyl)C₁₋C₆ alkyl, (cyclohexyl)C₁₋C₆ alkyl, (cycloheptyl)C₁₋C₆ alkyl, (cyclooctyl)C₁₋C₆ alkyl, or (cyclononyl)C₁₋C₆ alkyl, and R₄ is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted aralkyl group. In some embodiments of the compound of Formula I', R₄ is a benzyl group optionally substituted with one to five R_{g} independently selected from the group consisting of hydroxy, halogen, -CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a benzyl group optionally substituted with one to five R_{g} independently selected from the group consisting of hydroxy, halogen, -CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is a benzyl group optionally substituted with one to five R_{g} independently selected from the group consisting of hydroxy, halogen, CN, C₁-C₆ alkyl, C₆-C₁₀ aryl, C₁₋C₆ alkoxy, C₁₋C₆ haloalkoxy, and C₆-C₁₀ aralkoxy. In some embodiments of the compound of Formula I', R₄ is a benzyl group optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted (heterocycloalkyl)alkyl group. In some embodiments of the compound of Formula I', R₄ is a (heterocycloalkyl)alkyl group optionally substituted with one to five R_{g} independently selected from the group consisting of hydroxy, oxo, halogen, amino, CN, O-carboxy, C-carboxy, C-amido, N-amido, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a (heterocycloalkyl)alkyl group optionally substituted with one to five R_{g} independently selected from the group consisting of hydroxy, oxo, halogen, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is a (heterocycloalkyl)alkyl group optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted (heteroaryl)alkyl group. In some embodiments of the compound of Formula I', R₄ is a (heteroaryl)alkyl group optionally substituted with one to five R_{g} independently selected from the group consisting of hydroxy, halogen, -CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a (heteroaryl)alkyl group optionally substituted with one to five R_{g} independently selected from the group consisting of hydroxy, halogen, -CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is a (heteroaryl)alkyl group optionally substituted with one to five R_{g} independently selected from the group consisting of hydroxy, halogen, CN, C₁₋C₆ alkyl, C₆-C₁₉ aryl, C₁₋C₆ alkoxy, C₁₋C₆ haloalkoxy, and C₆-C₁₀ aralkoxy. In some embodiments of the compound of Formula I', R₄ is a (heteroaryl)alkyl group optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is a C₆-C₁₀ aryl group or a five- to ten-membered heteroaryl ring containing one to four ring heteroatoms independently selected from oxygen, sulfur, or nitrogen; and R₄ is optionally substituted with one to five R_{g} independently selected from the group consisting of hydroxy, halogen, CN, C₁-C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a C₆-C₁₀ aryl group or a five- to ten-membered heteroaryl ring containing one to four ring heteroatoms independently selected from oxygen, sulfur, or nitrogen; and R₄ is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted C₆-C₁₀ aryl group. In some embodiments of the compound of Formula I', R₄ is a C₆-C₁₀ aryl group optionally substituted with one to five R_{g} independently selected from the group consisting of hydroxy, halogen, -CN, O-carboxy, C-carboxy, C-amido, N-amido, amino, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a C₆-C₁₀ aryl group optionally substituted with one to five R_{g} independently selected from the group consisting of hydroxy, halogen, -CN, O-carboxy, C-carboxy, C-amido, N-amido, amino, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is a C₆-C₁₀ aryl group optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is a C₆-C₁₀ aryl group optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is benzene optionally substituted with one to five R_{g} independently selected from the group consisting of hydroxy, halogen, CN, C₁₋C₆ alkyl, C₁₋C₆ alkoxy, and C₁₋C₆ haloalkoxy, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is benzene optionally substituted with one to five R_{g} independently selected from the group consisting of hydroxy, halogen, C₁₋C₆ alkyl, and C₁₋C₆ alkoxy, or two R_{g} together with the atoms to which they are attached form a ring.

In some embodiments of the compound of Formula I', R₄ is naphthalene optionally substituted with one to five R_{g} independently selected from the group consisting of hydroxy, halogen, C₁₋C₆ alkyl, and C₁₋C₆ alkoxy.

In some embodiments of the compound of Formula I', R₄ is an optionally substituted heteroaryl group. In some embodiments of the compound of Formula I', R₄ is five- to ten-membered heteroaryl ring containing one to four ring heteroatoms independently selected from oxygen, sulfur, or nitrogen, wherein the heteroaryl ring is optionally substituted with one to five R_{g} independently selected from the group consisting of hydroxy, halogen, - CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₁₋C₆ haloalkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl, or two R_{g} together with the atoms to which they are attached form an aromatic or non-aromatic 3- to 6-membered ring, optionally containing one or two ring heteroatoms independently selected from oxygen, sulfur, or nitrogen. In some embodiments of the compound of Formula I', R₄ is five- to ten-membered heteroaryl ring containing one to four ring heteroatoms independently selected from oxygen, sulfur, or nitrogen, wherein the heteroaryl ring is optionally substituted with one to five R_{g} independently selected from the group consisting of hydroxy, halogen, -CN, amino, O-carboxy, C-carboxy, C-amido, N-amido, C₁₋C₆ alkyl, C₁₋C₆ haloalkyl, C₆-C₁₀ aryl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₄ is five-to ten-membered heteroaryl ring containing one to four ring heteroatoms independently selected from oxygen, sulfur, or nitrogen, wherein the heteroaryl ring is optionally substituted with one to five R_{g} independently selected from the group consisting of hydroxy, halogen, CN, C₁₋C₆ alkyl, C₆-C₁₀ aryl, C₁₋C₆ alkoxy, C₁₋C₆ haloalkoxy, and C₆-C₁₀ aralkoxy. In some embodiments of the compound of Formula I', R₄ is five- to ten-membered heteroaryl ring containing one to four ring heteroatoms independently selected from oxygen, sulfur, or nitrogen, wherein the heteroaryl ring is optionally substituted with one to five R_{g} independently selected from the group consisting of hydroxy, halogen, C₁₋C₆ alkyl, C₆-C₁₀ aryl, C₁₋C₆ alkoxy, and C₆-C₁₀ aralkoxy.

In some embodiments of the compound of Formula I', R₄ is an optionally substituted amino group. In some embodiments of the compound of Formula I', R₄ is an amino group optionally substituted with one or two substituents independently selected from the group consisting of C₁₋C₆ alkyl, C₁₋C₆ haloalkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, C₆-C₁₀ aryl, C-carboxy, C-amido, -(SO₂)(C₁₋C₆ alkyl), -C(O)O(C₁₋C₆ alkyl), -C(O)O(C₁₋C₆ haloalkyl), - C(O)O(C₃₋C₉ cycloalkyl), -C(O)O(3- to 6-membered heterocycloalkyl), -C(O)O(five- to ten-membered heteroaryl), and -C(O)O(C₆-C₁₀ aryl). In some embodiments of the compound of Formula I', R₄ is -NRₘRₙ, wherein Rₘ and Rₙ are independently selected from the group consisting of hydrogen, C₁₋C₆ alkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, C₆-C₁₀ aryl, C-carboxy, C-amido, - (SO₂)(C₁₋C₆ alkyl), -C(O)O(C₁₋C₆ alkyl), -C(O)O(C₃₋C₉ cycloalkyl), -C(O)O(3- to 6-membered heterocycloalkyl), -C(O)O(five- to ten-membered heteroaryl), and -C(O)O(C₆-C₁₀ aryl); or Rₘ and Rₙ together with the nitrogen to which they are attached form a 3- to 18-membered heterocycloalkyl ring; and R₁ is optionally substituted with one to five R_{g} independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₄ is an optionally substituted sulfamoyl group. In some embodiments of the compound of Formula I', R₄ is - SO₂NRₘRₙ, wherein Rₘ and Rₙ are independently selected from the group consisting of hydrogen, C₁₋C₆ alkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl; or Rₘ and Rₙ together with the nitrogen to which they are attached form a 3- to 18-membered heterocycloalkyl ring; and R₁ is optionally substituted with one to five R_{g} independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₄ is an optionally substituted carbamoyl group. In some embodiments of the compound of Formula I', R₄ is - C(O)NRₘRₙ, wherein Rₘ and Rₙ are independently selected from the group consisting of hydrogen, C₁₋C₆ alkyl, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl; or Rₘ and Rₙ together with the nitrogen to which they are attached form a 3- to 18-membered heterocycloalkyl ring; and R₁ is optionally substituted with one to five R_{g} independently selected from the group consisting of hydroxy, halogen, -CN, amino, C₁₋C₆ alkyl, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₅ is hydroxy. In some embodiments of the compound of Formula I', R₅ is NH₂.

In some embodiments of the compound of Formula I', R₅ is alkylamino. In some embodiments of the compound of Formula I', R₅ is -NRᵤRᵥ, wherein Rᵤ is hydrogen or C₁₋C₆ alkyl and Rᵥ is C₁₋C₁₂ alkyl optionally substituted with one to five substituents independently selected from the group consisting of hydroxy, halogen, -CN, amino, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₅ is alkanoylamino. In some embodiments of the compound of Formula I', R₅ is -NRᵤC(O)Rᵥ, wherein Rᵤ is hydrogen or C₁₋C₆ alkyl and Rᵥ is C₁₋C₁₂ alkyl optionally substituted with one to five substituents independently selected from the group consisting of hydroxy, halogen, -CN, amino, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₅ is alkylsulfonylamino. In some embodiments of the compound of Formula I', R₅ is -NRᵤSO₂Rᵥ, wherein Rᵤ is hydrogen or C₁₋C₆ alkyl and Rᵥ is C₁₋C₁₂ alkyl optionally substituted with one to five substituents independently selected from the group consisting of hydroxy, halogen, - CN, amino, O-carboxy, C-carboxy, C-amido, N-amido, C₁₋C₆ alkoxy, C₆-C₁₀ aralkoxy, C₃-C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₅ is hydroxy or NH₂. In some embodiments of the compound of Formula I', R₅ is hydroxy, NH₂, C₁₋C₆ alkylamino, C₁₋C₆ alkanoylamino, or C₁₋C₆ alkylsulfonylamino.

In some embodiments of the compound of Formula I', R₄ and R₅ taken together along with the carbon atoms to which they are attached form a five- or six-membered optionally substituted non-aromatic C₃-C₁₂ carbocyclic group, optionally substituted C₆-C₁₀ aryl group, optionally substituted heterocyclic group, or optionally substituted heteroaryl group. In some embodiments of the compound of Formula I', R₄ and R₅ taken together along with the carbon atoms to which they are attached form a five- or six-membered non-aromatic C₃-C₁₂ carbocyclic group, C₆-C₁₀ aryl group, 4- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, wherein the carbocyclic group, the aryl group, the heterocycloalkyl group, and the heteroaryl group are optionally substituted with one to five substituents independently selected from the group consisting of hydroxy, halogen, -CN, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₄ and R₅ taken together along with the carbon atoms to which they are attached form a seven to eleven membered, optionally substituted spirocyclic ring or a seven to eleven membered, optionally substituted spiro-heterocyclic ring. In some embodiments of the compound of Formula I', R₄ and R₅ taken together along with the carbon atoms to which they are attached form a seven to eleven membered, spirocyclic ring or a seven to eleven membered spiro-heterocyclic ring; and the spirocyclic ring and spiro-heterocyclic ring are optionally substituted with one to five substituents independently selected from the group consisting of hydroxy, halogen, -CN, amino, O-carboxy, C-carboxy, C-amido, N-amido, C₁₋C₆ alkyl, C₁₋C₆ haloalkyl, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', R₄ and R₅ taken together along with the carbon atoms to which they are attached form a five- or six-membered optionally substituted non-aromatic carbocyclic group, optionally substituted aryl group, optionally substituted heterocycloalkyl group, or optionally substituted heteroaryl group.

In some embodiments of the compound of Formula I', R₄ and R₅ taken together along with the carbon atoms to which they are attached form a five- or six-membered non-aromatic carbocyclic group; a C₆-C₁₀ aryl group; a 3- to 6-membered heterocycloalkyl ring containing one to four ring heteroatoms independently selected from oxygen, sulfur, or nitrogen; or a five- to ten-membered heteroaryl ring containing one to four ring heteroatoms independently selected from oxygen, sulfur, or nitrogen; and wherein the carbocyclic group, the aryl group, the heterocycloalkyl ring, and the heteroaryl ring are optionally substituted with one to five R_{g} independently selected from the group consisting of hydroxy, halogen, C₁₋C₆ alkyl, C₁₋C₆ alkoxy, and oxo, and/or optionally two R_{g} together with the atom(s) to which they are attached form a 3- to 6-membered carbocyclic group.

In some embodiments of the compound of Formula I', R₄ and R₅ taken together along with the carbon atoms to which they are attached form a 3- to 6-membered heterocycloalkyl ring containing one to four ring heteroatoms independently selected from oxygen, sulfur, or nitrogen; and wherein the heterocycloalkyl ring is optionally substituted with one to five R_{g} independently selected from the group consisting of C₁₋C₆ alkyl and oxo, and/or optionally two R_{g} together with the atom(s) to which they are attached form a 3- to 6-membered non-aromatic carbocyclic group.

In some embodiments of the compound of Formula I', R₄ and R₅ taken together along with the carbon atoms to which they are attached form a pyrrolidine optionally substituted with one to five R_{g} independently selected from the group consisting of C₁₋C₆ alkyl and oxo, and/or optionally two R_{g} together with the atom(s) to which they are attached form a 3- to 6-membered non-aromatic carbocyclic group.

In some embodiments of the compound of Formula I' not according to the invention, TL is a moiety of Formula IIIaa: 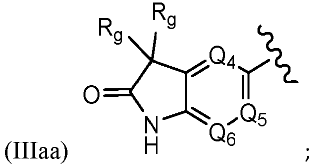 wherein each R_{g} is independently C₁₋C₆ alkyl; or the two R_{g} together with the atom to which they are attached form an alkene optionally substituted with C₁₋C₆ alkyl; or the two R_{g} together with the atom to which they are attached form a 3- to 6-membered non-aromatic carbocyclic group. In some embodiments of the compound of Formula I', the two R_{g} together with the atom to which they are attached form a cyclopropyl group, a cyclobutyl group, a cyclopentyl group, or a cyclohexyl group.

In some embodiments of the compound of Formula I' not according to the invention, TL is a moiety of Formula IIIaa: 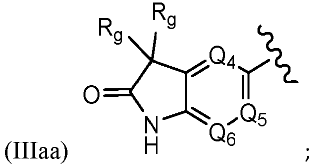 wherein each R_{g} is independently C₁₋C₆ alkyl or the two R_{g} together with the atom(s) to which they are attached form a 3- to 6-membered non-aromatic carbocyclic group. In some embodiments of the compound of Formula I', the two R_{g} together with the atom to which they are attached form a cyclopentyl group or a cyclohexyl group.

In some embodiments of the compound of Formula I' not according to the invention, TL is a moiety of Formula IIIab: 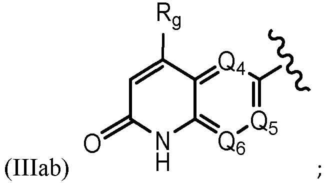 wherein R_{g} is C₁₋C₆ alkyl. In some embodiments of the compound of Formula I', R_{g} is isopropyl.

In some embodiments of the compound of Formula I' not according to the invention, TL is a moiety of Formula IIIac: 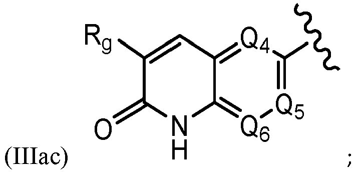 wherein R_{g} is C₁₋C₆ alkyl. In some embodiments of the compound of Formula I', R_{g} is methyl.

In some embodiments of the compound of Formula I' not according to the invention, TL is a moiety of Formula IIIad: 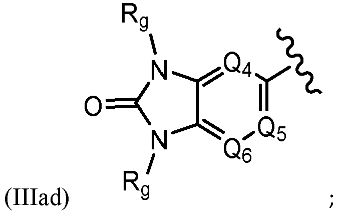 wherein each R_{g} is independently hydrogen or C₁₋C₆ alkyl.

In some embodiments of the compound of Formula I', R₄ is an optionally substituted alkyl, an optionally substituted alkenyl, an optionally substituted non-aromatic carbocyclic group, an optionally substituted aryl group, an optionally substituted heterocycloalkyl group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, or an optionally substituted carbamoyl group; and R₅ is hydroxy, NH₂, alkylamino, alkanoylamino, or alkylsulfonylamino.

In some embodiments of the compound of Formula I', R₄ is C₁₋C₆ alkyl; C₂-C₁₀ alkenyl; a non-aromatic C₃-C₁₂ carbocyclic ring, a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; a (heteroaryl)alkyl group; or -C(O)NRₘRₙ;
Rₘ and Rₙ are independently selected from the group consisting of hydrogen, C₁₋C₆ alkyl, C₁₋C₆ haloalkyl, C₂-C₆ alkoxy, a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; or a (heteroaryl)alkyl group; or Rₘ and Rₙ together with the nitrogen to which they are attached form a 3- to 18-membered heterocycloalkyl ring;
and R₄ is optionally substituted with one to five R_{g} independently selected from the group consisting of hydroxy, halogen, C₁₋C₆ alkyl, C₁₋C₆ haloalkyl, C₆-C₁₀ aryl, C₁₋C₆ alkoxy, and C₆-C₁₀ aralkoxy; and
R₅ is hydroxy, NH₂, alkylamino, alkanoylamino, or alkylsulfonylamino.

In some embodiments of the compound of Formula I', R₄ is C₁₋C₆ alkyl; C₂-C₁₀ alkenyl; a non-aromatic C₃-C₁₂ carbocyclic ring, a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; a (heteroaryl)alkyl group; or -C(O)NRₘRₙ;
Rₘ and Rₙ are independently selected from the group consisting of hydrogen, C₁₋C₆ alkyl, C₁₋C₆ haloalkyl, C₂-C₆ alkoxy, a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; a (heterocycloalkyl)alkyl group; or a (heteroaryl)alkyl group; or Rₘ and Rₙ together with the nitrogen to which they are attached form a 3- to 18-membered heterocycloalkyl ring;
and R₄ is optionally substituted with one to five R_{g} independently selected from the group consisting of hydroxy, halogen, C₁₋C₆ alkyl, C₁₋C₆ haloalkyl, C₆-C₁₀ aryl, C₁₋C₆ alkoxy, and C₆-C₁₀ aralkoxy; and
R₅ is hydroxy.

In the compound of Formula I', Alk is hydrogen or optionally substituted C₁₋C₆ alkyl. In some embodiments of the compound of Formula I', Alk is hydrogen or C₁₋C₆ alkyl optionally substituted with one to five substituents independently selected from the group consisting of hydroxy, halogen, -CN, amino, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', Alk is hydrogen. In some embodiments of the compound of Formula I', Alk is C₁₋C₆ alkyl optionally substituted with one to five substituents independently selected from the group consisting of hydroxy, halogen, -CN, amino, O-carboxy, C-carboxy, C-amido, N-amido, C₁₋C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', Alk is C₁₋C₆ alkyl optionally substituted with one to five substituents independently selected from the group consisting of hydroxy, halogen, and C₁₋C₆ alkoxy.

In some embodiments of the compound of Formula I', R₁₁ is an aryl group optionally substituted with one to five substituents independently selected from lower alkyl, alkoxy, haloalkoxy, halogen, and C₃-C₉ cycloalkyl; or a heteroaryl group optionally substituted with one to five substituents independently selected from lower alkyl, alkoxy, haloalkoxy, halogen, and cycloalkyl; or a bicyclic ring system containing either aromatic or saturated rings; or a bicyclic heterocyclic containing either aromatic or saturated ring systems. In some embodiments of the compound of Formula I', R₁₁ is an aryl group optionally substituted with one to five substituents independently selected from lower alkyl, halogen, and C₃-C₉ cycloalkyl; or a bicyclic ring system containing either aromatic or saturated rings; or a bicyclic heterocyclic containing either aromatic or saturated ring systems. In some embodiments of the compound of Formula I', R₁₁ is a C₆-C₁₀ aryl group optionally substituted with one to five substituents independently selected from lower alkyl, alkoxy, haloalkoxy, halogen, and C₃-C₉ cycloalkyl; or a five- to ten-membered heteroaryl group containing one to four ring heteroatoms independently selected from oxygen, sulfur, or nitrogen, wherein the heteroaryl group is optionally substituted with one to five substituents independently selected from lower alkyl, alkoxy, haloalkoxy, halogen, and C₃-C₉ cycloalkyl; or a 7- to 12-membered bicyclic ring system containing either aromatic or saturated rings; or a 7- to 12-membered bicyclic heterocyclic containing either aromatic or saturated ring systems. In some embodiments of the compound of Formula I', R₁₁ is a C₆-C₁₀ aryl group optionally substituted with one to five substituents independently selected from lower alkyl, halogen, and C₃-C₉ cycloalkyl; or a 7- to 12-membered bicyclic ring system containing either aromatic or saturated rings; or a 7- to 12-membered bicyclic heterocyclic containing either aromatic or saturated ring systems. In some embodiments of the compound of Formula I', R₁₁ is a C₆-C₁₀ aryl group optionally substituted with one to five substituents independently selected from lower alkyl, alkoxy, haloalkoxy, halogen, and C₃-C₉ cycloalkyl. In some embodiments of the compound of Formula I', R₁₁ is a C₆-C₁₀ aryl group optionally substituted with one to five substituents independently selected from lower alkyl, halogen, and C₃-C₉ cycloalkyl. In some embodiments of the compound of Formula I', R₁₁ a five- to ten-membered heteroaryl group containing one to four ring heteroatoms independently selected from oxygen, sulfur, or nitrogen, wherein the heteroaryl group is optionally substituted with one to five substituents independently selected from lower alkyl, alkoxy, haloalkoxy, halogen, and C₃-C₉ cycloalkyl. In some embodiments of the compound of Formula I', R₁₁ is a 7- to 12-membered bicyclic ring system containing either aromatic or saturated rings. In some embodiments of the compound of Formula I', R₁₁ is a 7- to 12-membered bicyclic heterocyclic containing either aromatic or saturated ring systems. In some embodiments of the compound of Formula I', R₁₁ is a benzene optionally substituted with one to five substituents independently selected from C₁-C₅ alkyl, C₁-C₅ alkoxy, C₁-C₅ haloalkoxy, halogen, and C₃-C₉ cycloalkyl; pyridine optionally substituted with C₁-C₅ alkyl; cinnoline; isoquinoline; quinoline; pyrazolo[1,5-a]pyridine; imidazo[1,5-a]pyridine; benzo[b]thiophene; chromane; 1,2,3,4-tetrahydronaphthalene; or naphthalene. In some embodiments of the compound of Formula I', R₁₁ is a benzene optionally substituted with one to five substituents independently selected from C₁-C₅ alkyl, halogen, and C₃-C₉ cycloalkyl; 1,2,3,4-tetrahydronaphthalene; or naphthalene.

In some embodiments of the compound of Formula I', each of R₆ and R₇ is independently halogen or C₁-C₅ alkyl optionally substituted with one to five substituents independently selected from hydroxy, halogen, and C₁-C₆ alkoxy; and R₈ is hydrogen; or R₆ is halogen or C₁-C₅ alkyl optionally substituted with one to five substituents independently selected from hydroxy, halogen, and C₁-C₆ alkoxy; and R₇ and R₈ taken together, along with the carbon atoms to which they are attached, form a 4-, 5- or 6-membered carbocyclic ring.

In some embodiments of the compound of Formula I', each of R₆ and R₇ is independently chlorine, bromine, and iodine. In some embodiments of the compound of Formula I', each of R₆ and R₇ is independently -CN, an optionally substituted lower alkyl or an optionally substituted lower alkoxy, where the lower alkyl and the alkyl group of the lower alkoxy is each independently selected from methyl, ethyl, *n*-propyl, *i*-propyl, *n*-butyl, s-butyl, and *t*-butyl. In some embodiments of the compound of Formula I', R₆ and R₇ are the same. In some embodiments of the compound of Formula I', each of R₆ and R₇ is independently chlorine or methyl. In some embodiments of the compound of Formula I', R₆ is Cl, R₇ is Cl, and R₈ is hydrogen. In some embodiments of the compound of Formula I', R₆ is Cl, R₇ is Cl, and R₈ is methyl. In some embodiments of the compound of Formula I', R₆ is halogen and R₇ and R₈ taken together, along with the carbon atoms to which they are attached, form a 4-membered carbocyclic ring.

In some embodiments of the compound of Formula I', R₈ is hydrogen. In some embodiments of the compound of Formula I', R₈ is optionally substituted lower alkyl. In some embodiments of the compound of Formula I', R₈ is lower alkyl optionally substituted with one to five substituents selected from the group consisting of hydroxy, halogen, amino, -CN, O-carboxy, C-carboxy, C-amido, N-amido, C₁-C₆ alkoxy, C₃₋C₅ cycloalkyl, a 3- to 5-membered heterocycloalkyl group containing one heteroatom independently selected from oxygen, sulfur, or nitrogen. In some embodiments of the compound of Formula I', R₈ is optionally substituted lower alkoxy. In some embodiments of the compound of Formula I', R₈ is lower alkoxy optionally substituted with one to five substituents selected from the group consisting of hydroxy, halogen, amino, -CN, O-carboxy, C-carboxy, C-amido, N-amido, C₁-C₆ alkoxy, C₃₋C₅ cycloalkyl, a 3- to 5-membered heterocycloalkyl group containing one heteroatom independently selected from oxygen, sulfur, or nitrogen. In some embodiments of the compound of Formula I', R₈ is cyano. In some embodiments of the compound of Formula I', R₈ is halogen.

In some embodiments of the compound of Formula I', R₇ and R₈ taken together, along with the carbon atoms to which they are attached, form a 4-, 5- or 6-membered non-aromatic carbocyclic, a 3- to 5-membered heterocycloalkyl, a C₆-C₁₀ aryl, or a five- to ten-membered heteroaryl ring.

In some embodiments of the compound of Formula I', Q₇ is nitrogen. In some embodiments of the compound of Formula I', Q₇ is -CR_{c}-. In some embodiments of the compound of Formula I', R_{c} is hydrogen. In some embodiments of the compound of Formula I', R_{c} is halogen. In some embodiments of the compound of Formula I', R_{c} is lower alkyl. In some embodiments of the compound of Formula I', R_{c} is hydrogen or methyl. In some embodiments of the compound of Formula I', Q₇ is -CH-.

In some embodiments of the compound of Formula I', R₉ is hydrogen, - (C(R_{d})₂)ₙ-N(R_{d})₂, -(C(R_{d})₂)ₙ-CN, or -(C(R_{d})₂)ₙ-C≡C-R_{d}. In some embodiments of the compound of Formula I', R₉ is hydrogen, -(CH₂)ₙ-N(R_{d})₂, -(CH₂)ₙ-CN, or -(CH₂)ₙ-C≡C-R_{d}. In some embodiments of the compound of Formula I', R₉ is selected from hydrogen, -N(R_{d})₂, -CN, or -C≡C-R_{d}. In the compound of Formula I' according to the invention, R₉ is -NH₂.

In some embodiments of the compound of Formula I', R₉ is hydrogen. In some embodiments of the compound of Formula I', R₉ is -(C(Rₐ)₂)ₙ-C(Rₐ)₃. In some embodiments of the compound of Formula I', R₉ is -(C(R₄)₂)ₙ-OR_{d}. In some embodiments of the compound of Formula I', R₉ is -(C(R_{d})₂)ₙ-N(R_{d})₂. In the compound of Formula I' according to the invention, R₉ is -NH₂. In some embodiments of the compound of Formula I', R₉ is -(C(R_{d})₂)ₙ-S(=O)_{q}R_{d}. In some embodiments of the compound of Formula I', R₉ is -(C(R_{d})₂)ₙ-CN. In some embodiments of the compound of Formula I', R₉ is -CN. In some embodiments of the compound of Formula I', R₉ is -(C(R_{d})₂)ₙ-C≡C-R_{d}. In some embodiments of the compound of Formula I', R₉ is -C≡CH. In some embodiments of the compound of Formula I', R₉ is -(C(R_{d})₂)ₙ-C(=O)-OR_{d}. In some embodiments of the compound of Formula I', R₉ is -(C(R_{d})₂)ₙ-HeAr. In some embodiments of the compound of Formula I', R₉ is -(C(R_{d})₂)ₙ-C(=O)-N(R_{d})₂.

In some embodiments of the compound of Formula I', each R_{d} is independently hydrogen or lower alkyl optionally substituted with one to five substituents independently selected from the group consisting of hydroxy, halogen, -CN, amino, O-carboxy, C-carboxy, C-amido, N-amido, C₁-C₆ alkoxy, C₆-C₁₀ aralkoxy, C₃-C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', each R_{d} is hydrogen. In some embodiments of the compound of Formula I', each R_{d} is independently lower alkyl optionally substituted with one to five substituents independently selected from the group consisting of hydroxy, halogen, -CN, amino, O-carboxy, C-carboxy, C-amido, N-amido, C₁-C₆ alkoxy, C₆-C₁₀ aralkoxy, C₃-C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl.

In some embodiments of the compound of Formula I', q is 0. In some embodiments of the compound of Formula I', q is 1. In some embodiments of the compound of Formula I', q is 2.

In some embodiments of the compound of Formula I', n is 0. In some embodiments of the compound of Formula I', n is 1. In some embodiments of the compound of Formula I', n is 2. In some embodiments of the compound of Formula I', n is 3. In some embodiments of the compound of Formula I', n is 4. In some embodiments of the compound of Formula I', n is 5.

In some embodiments of the compound of Formula I', HeAr is a 5- or 6-membered heteroaryl group containing one to three ring heteroatoms independently selected from oxygen, sulfur, or nitrogen.

In some embodiments of the compound of Formula I', R₁₀ is hydrogen or - C(Rₑ)₃, wherein each Rₑ is independently hydrogen, halogen, or lower alkyl optionally substituted with one to five substituents independently selected from the group consisting of hydroxy, halogen, -CN, amino, O-carboxy, C-carboxy, C-amido, N-amido, C₁-C₆ alkoxy, C₆-C₁₀ aralkoxy, C₃₋C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₁₀ is hydrogen. In some embodiments of the compound of Formula I', R₁₀ is - C(Rₑ)₃, wherein each Rₑ is independently hydrogen, halogen, or lower alkyl optionally substituted with one to five substituents independently selected from the group consisting of hydroxy, halogen, -CN, amino, O-carboxy, C-carboxy, C-amido, N-amido, C₁-C₆ alkoxy, C₆-C₁₀ aralkoxy, C₃-C₉ cycloalkyl, a 3- to 6-membered heterocycloalkyl group containing one to four ring heteroatoms independently selected from oxygen, sulfur, or nitrogen, a five- to ten-membered heteroaryl group containing one to four ring heteroatoms independently selected from oxygen, sulfur, or nitrogen, and C₆-C₁₀ aryl. In some embodiments of the compound of Formula I', R₁₀ is -C(Rₑ)₃, wherein each Rₑ is independently hydrogen, halogen, or lower alkyl. In some embodiments of the compound of Formula I', R₁₀ is hydrogen, -CHF₂, -CH₃, or ethyl.

In some embodiments of the compound of Formula I', Lₐ is a bond; - (C(Rₐ)₂)_{z}-; oxygen; sulfur; or -NRₐ-. In some embodiments of the compound of Formula I', Lₐ is a bond. In some embodiments of the compound of Formula I', Lₐ is -(C(Rₐ)₂)_{z}-. In some embodiments of the compound of Formula I', Lₐ is -CH₂-. In some embodiments of the compound of Formula I', Lₐ is oxygen. In some embodiments of the compound of Formula I', Lₐ is sulfur. In some embodiments of the compound of Formula I', Lₐ is -NRₐ-. In some embodiments of the compound of Formula I', each Rₐ is hydrogen. In some embodiments of the compound of Formula I', each Rₐ is independently lower alkyl. In some embodiments of the compound of Formula I', each Rₐ is independently a hydrogen or lower alkyl.

In some embodiments of the compound of Formula I', z is 0. In some embodiments of the compound of Formula I', z is 1. In some embodiments of the compound of Formula I', z is 2. In some embodiments of the compound of Formula I', z is 3. In some embodiments of the compound of Formula I', z is 4. In some embodiments of the compound of Formula I', z is 5.

In another aspect, disclosed herein is a compound selected from the group consisting of: 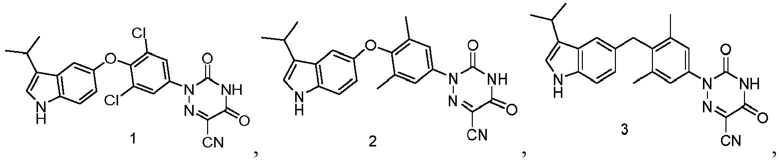 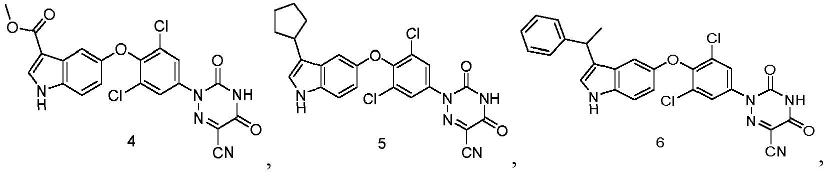 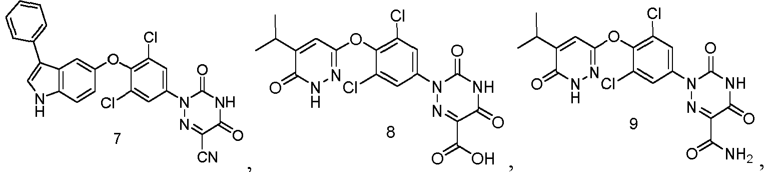 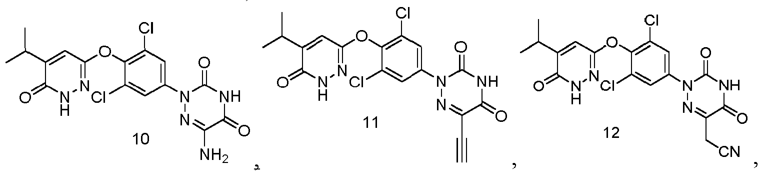 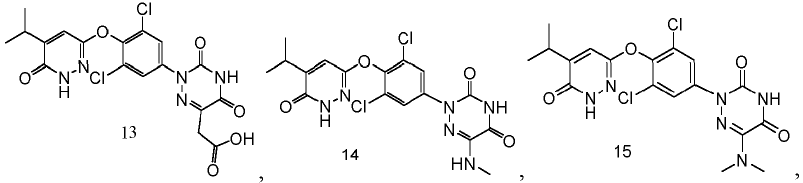 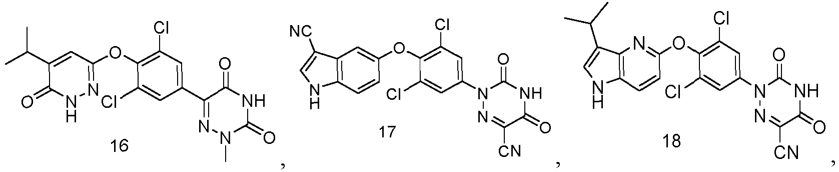 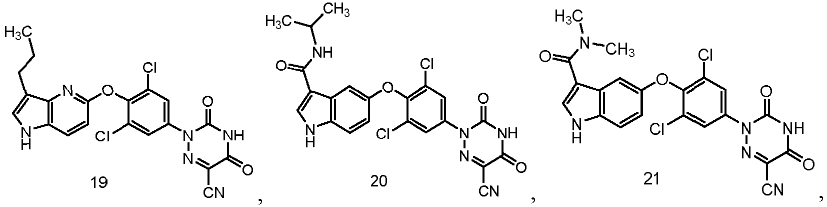 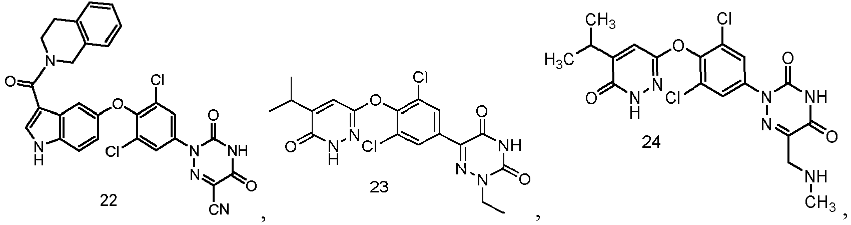 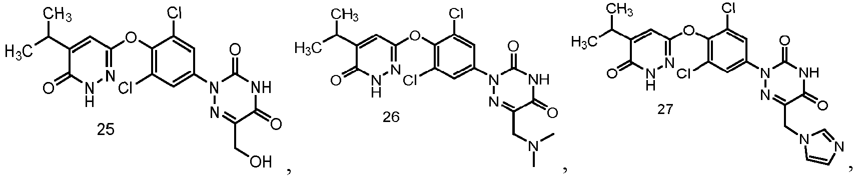 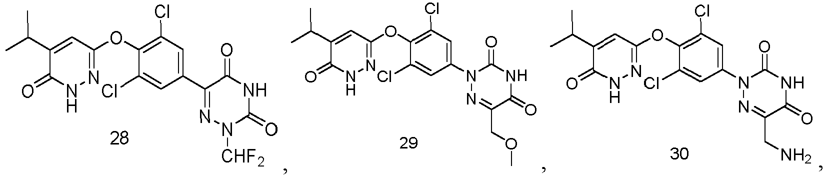 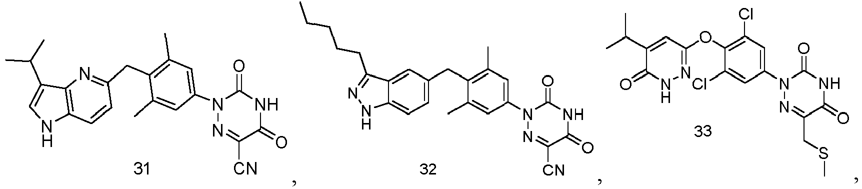 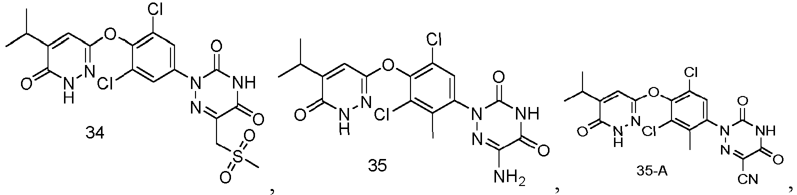 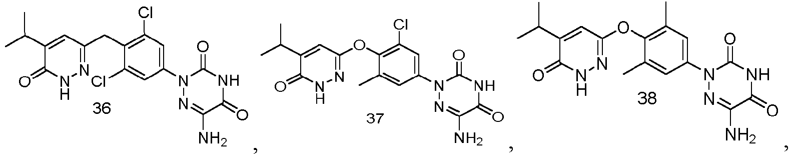 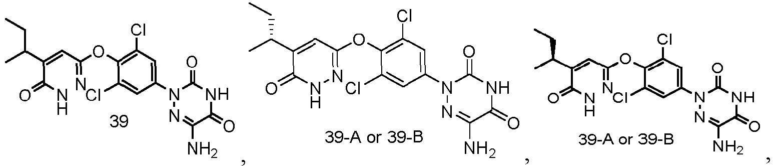 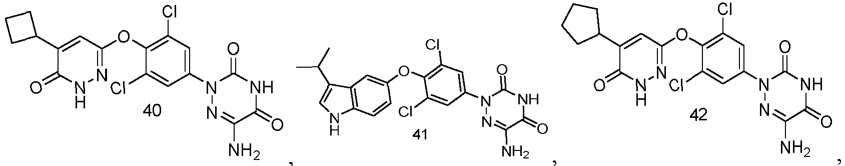 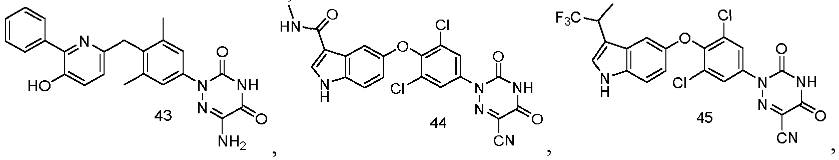 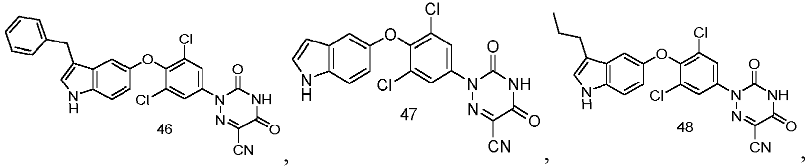 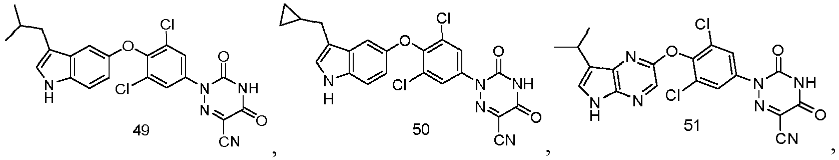 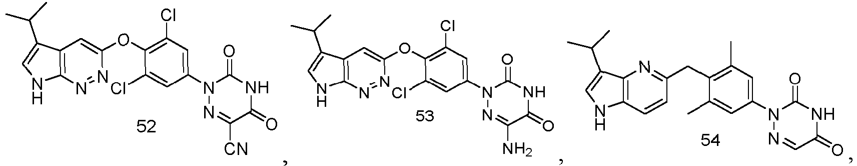 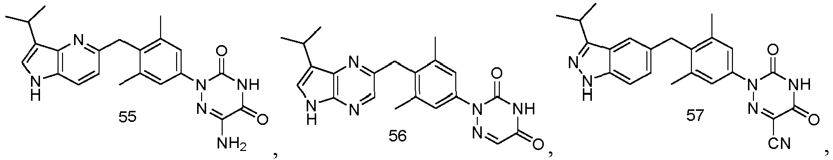 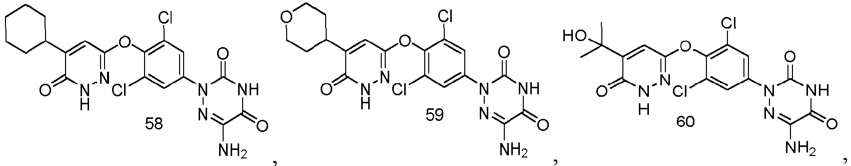 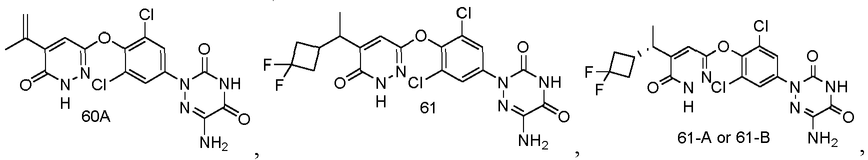 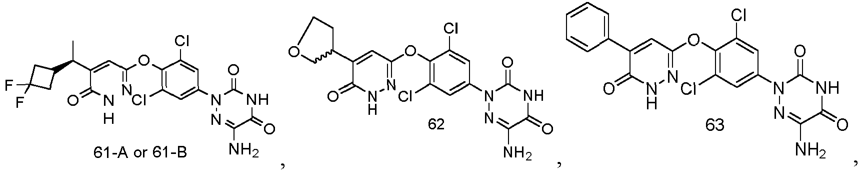 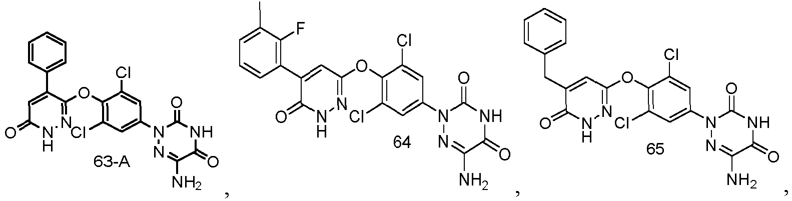 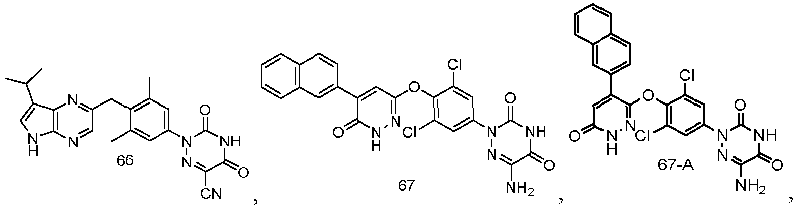 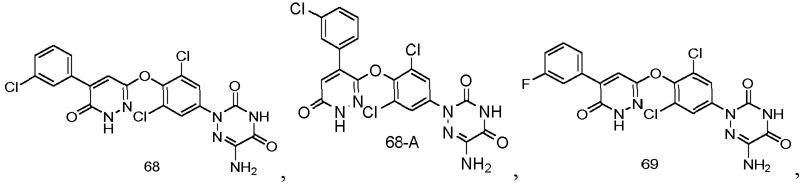 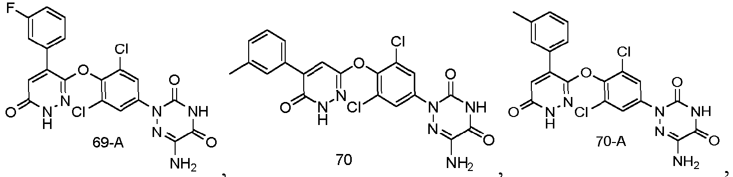 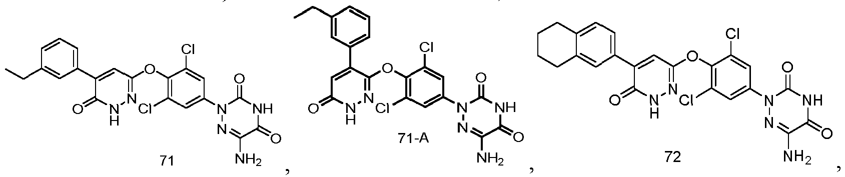 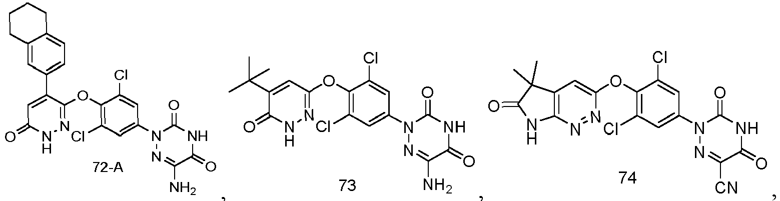 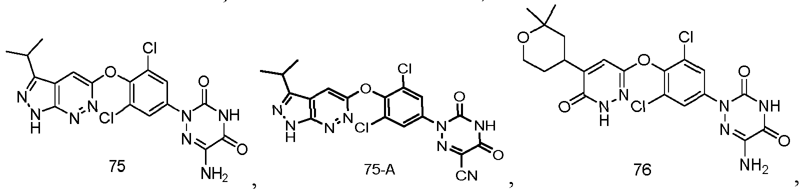 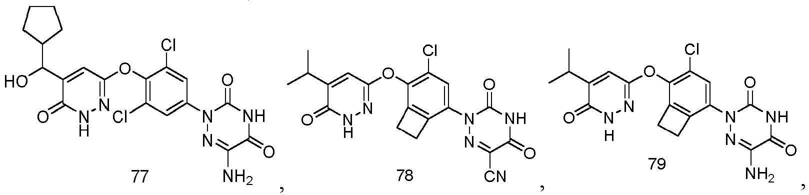 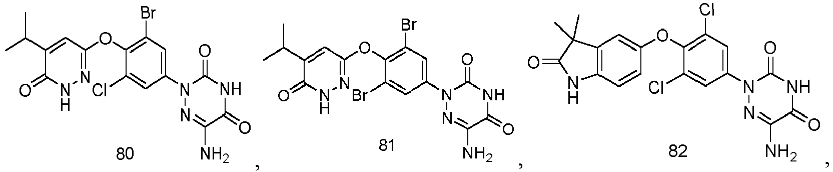 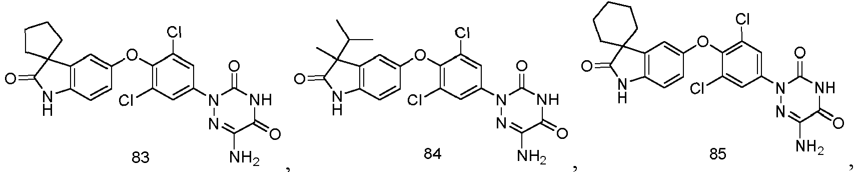 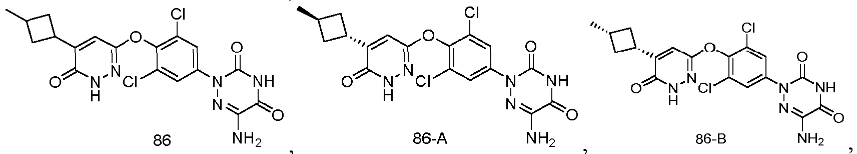 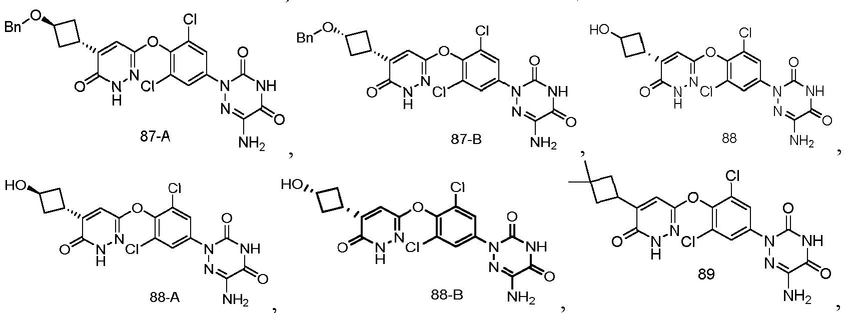 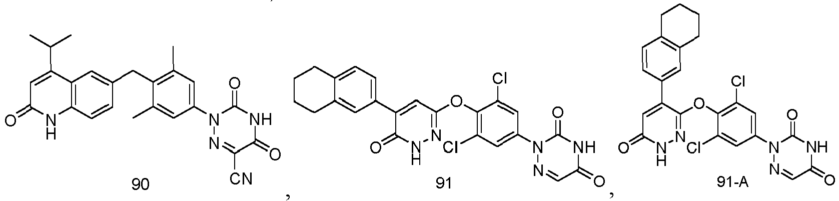 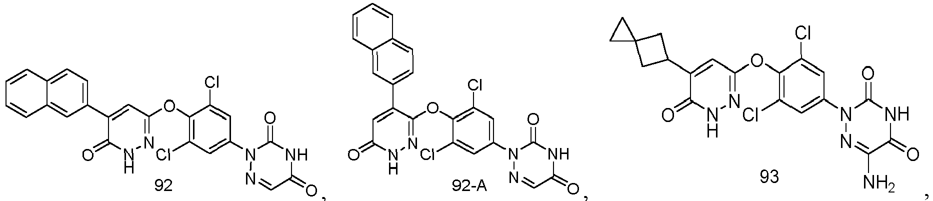 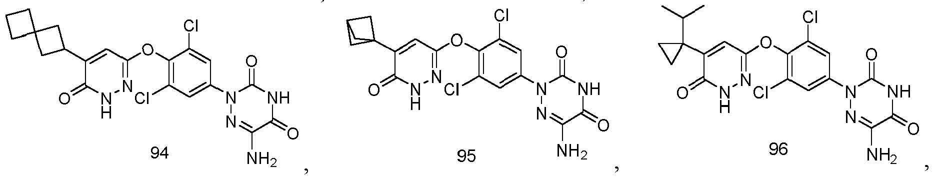 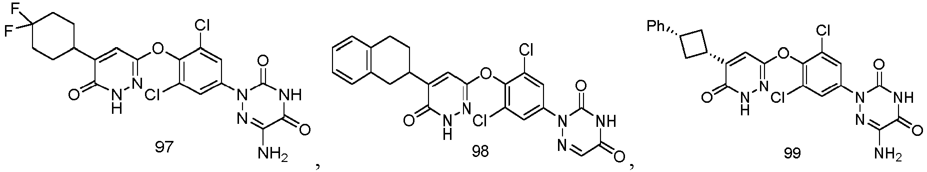 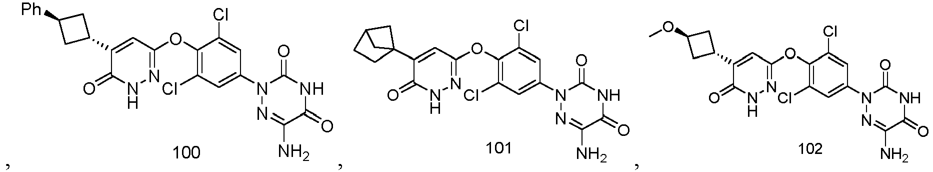 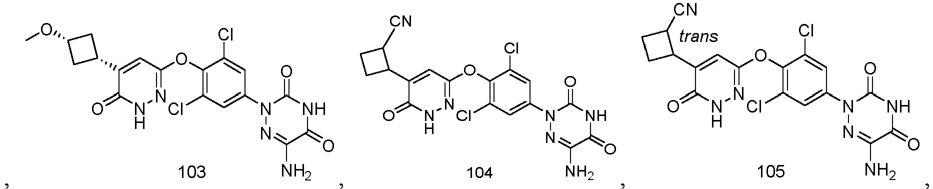 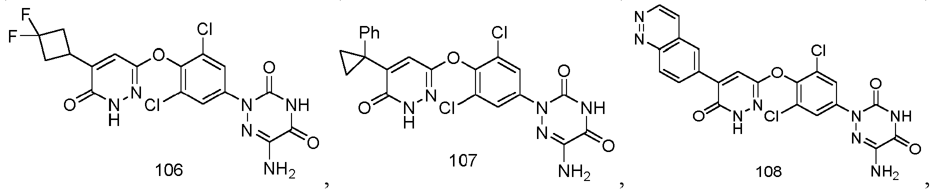 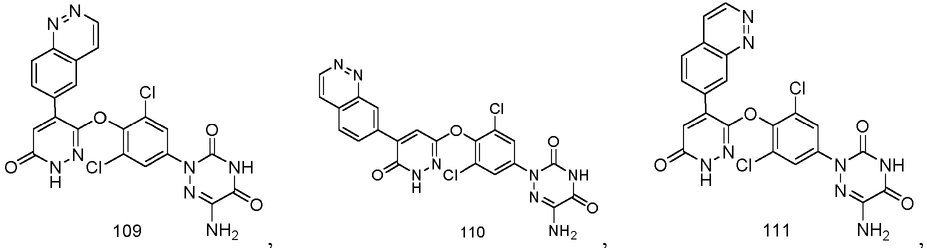 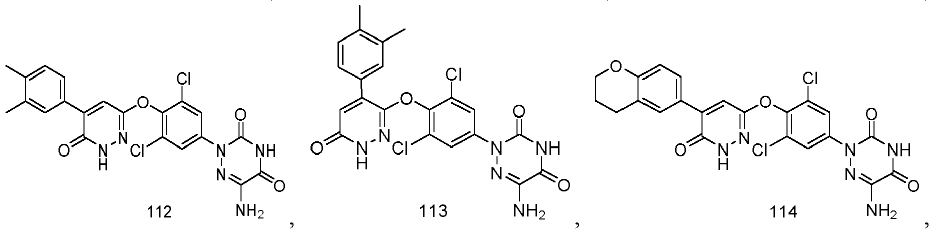 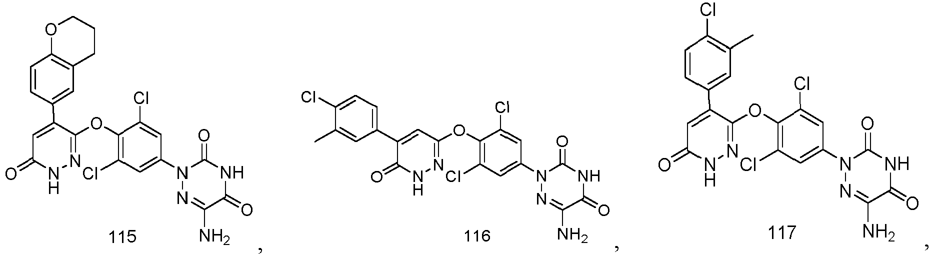 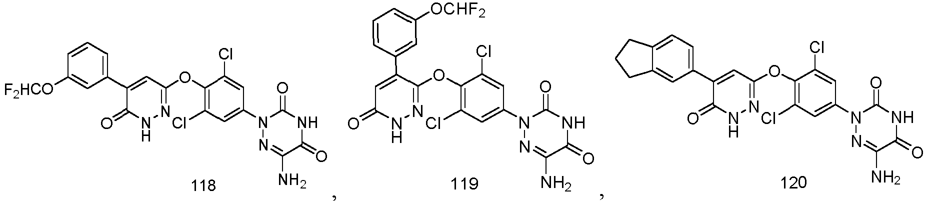 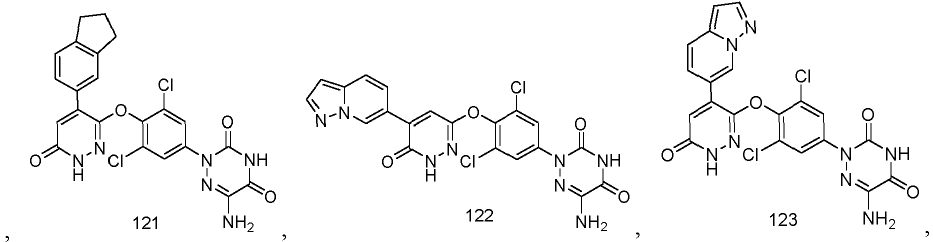 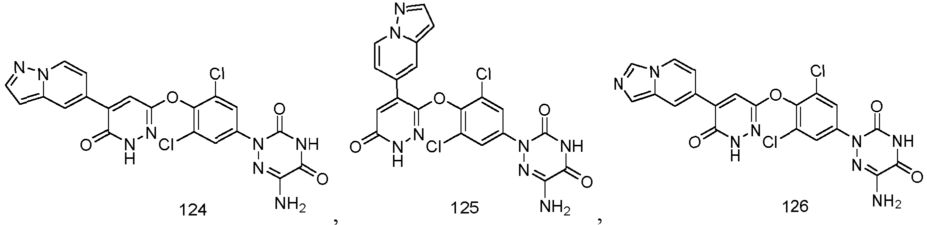 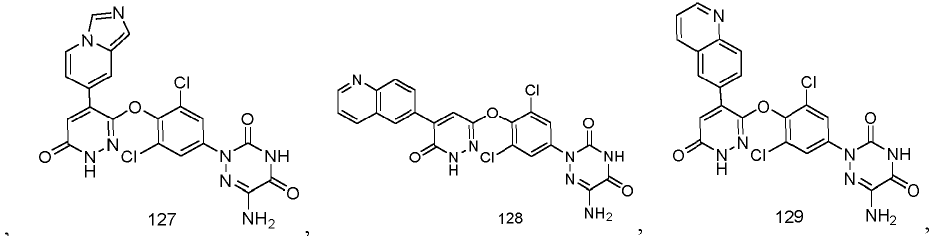 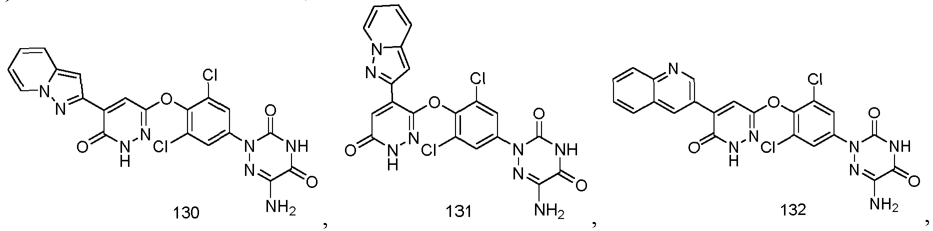 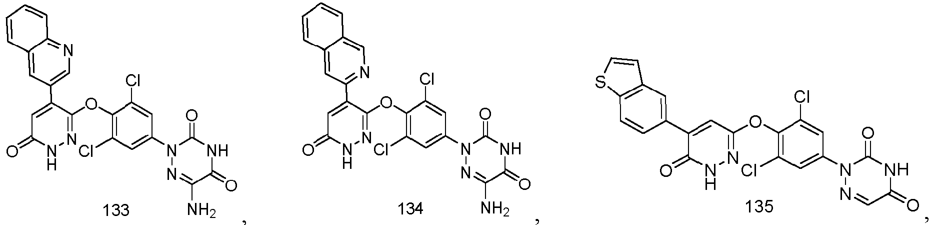 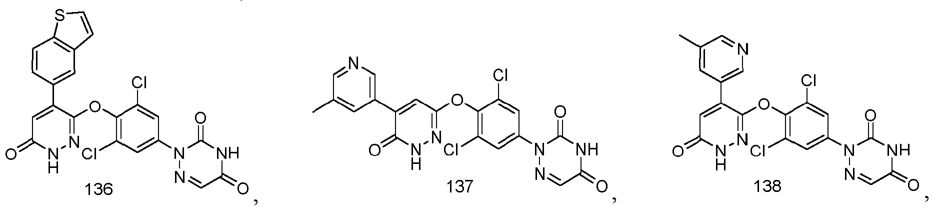 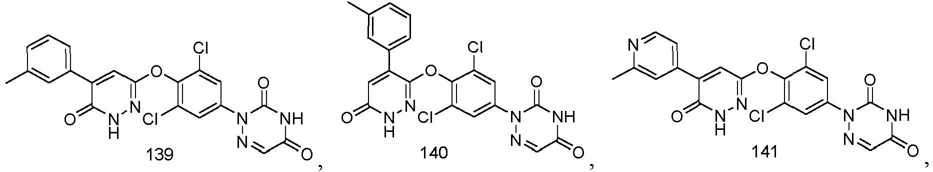 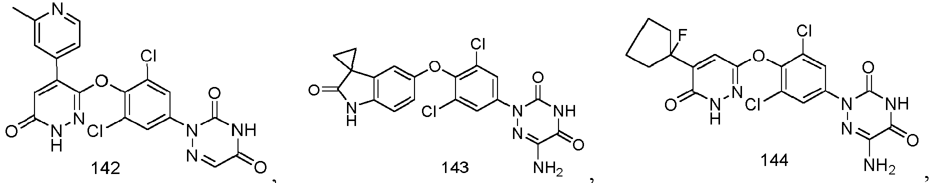 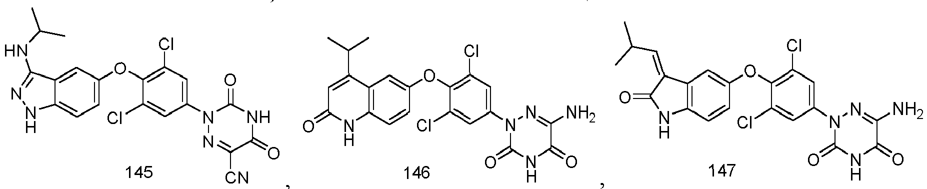 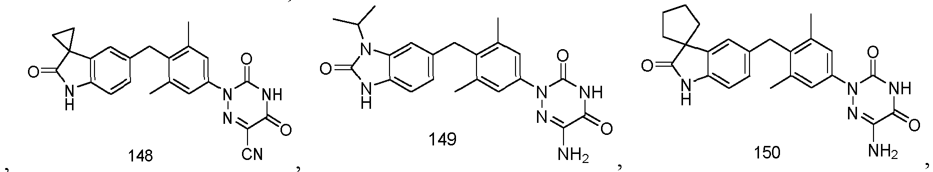 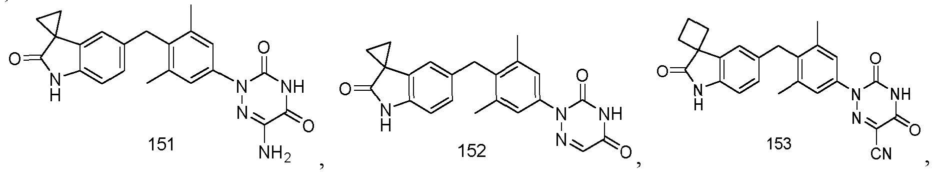 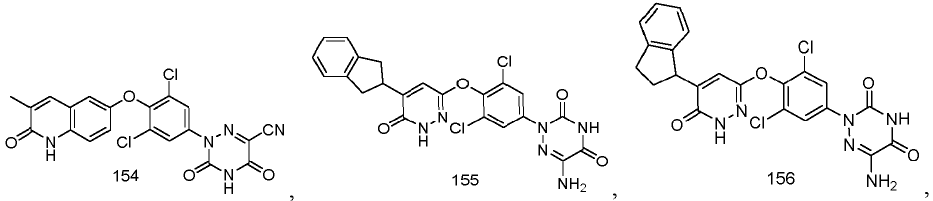 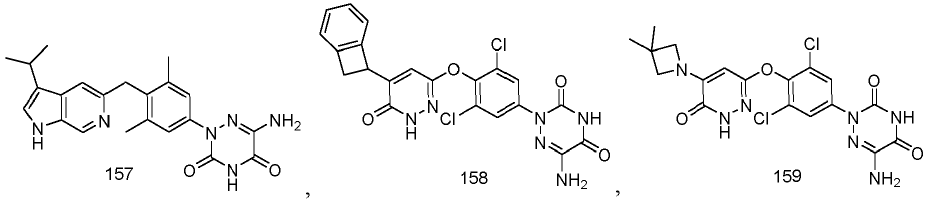 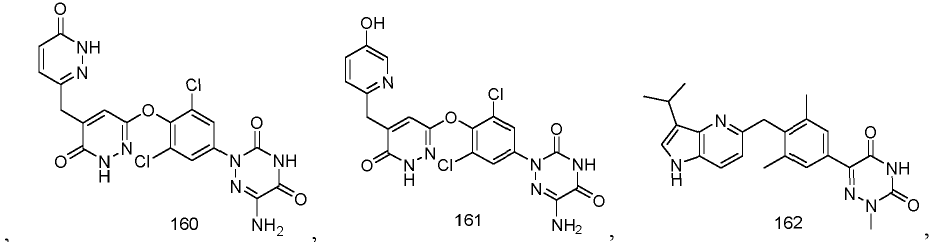 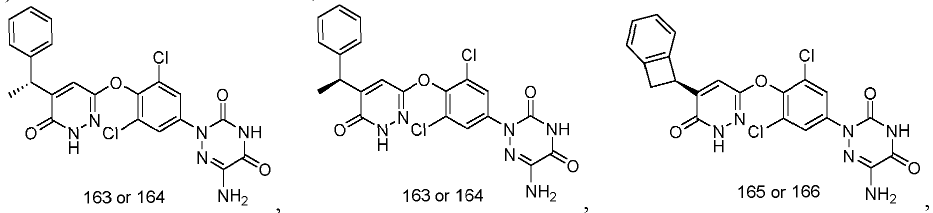 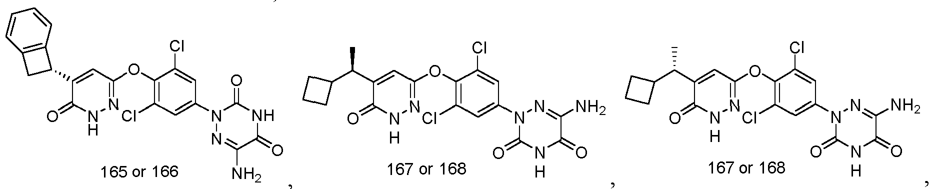 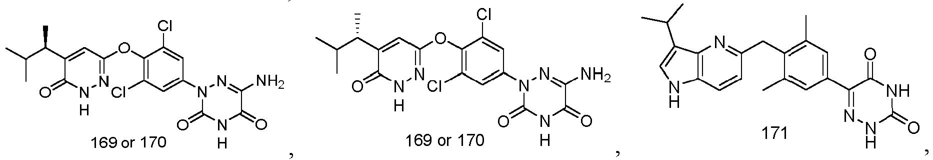 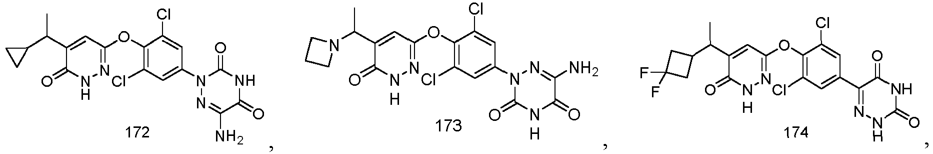 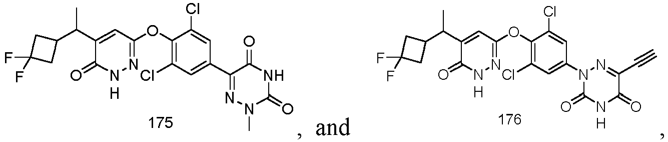 wherein the compounds falling within the definition of claim 1 are according to the invention.

### SYNTHESIS OF THE COMPOUNDS

The presently disclosed compounds were synthesized using the general synthetic procedures set forth in Schemes 1-10 below. The carrying out of each individual illustrated step is within the skill of an ordinary artisan, who also knows how to modify the synthetic procedures of the below schemes to synthesize the full scope of the compounds disclosed herein. The synthetic procedure for individual compounds is provided in the Examples section, below. The compounds disclosed below are encompassed by the claims, unless they clearly do not fall within the scope of the claims. For example, compounds 10, 35-39, 39-A, 39-B, 40, 42, 58-60, 60A, 61, 61-A, 61-B, 62-65, 67-73, 76, 77, 79-81, 86, 86A, 86B, 87A, 87B, 88, 88A, 88B, 89, 93-97, 99-108, 110, 112, 114, 116, 118, 120, 122, 124, 126, 128, 130, 132, 144, 155, 156, 158-161, 163-170, 172, and 173 are according to the invention.

As described in Scheme 1, an aromatic amine compound of Formula S-II is transformed to an aza-uracil compound of Formula S-III, first by generating the corresponding diazonium salt, followed by reaction with an N-(2-cyanoacetyl)-carbamate, and finally cyclization, resulting in the formation of a compound of Formula S-III. Next the nitrile of Formula S-III is hydrolyzed to a carboxylic acid compound of Formula S-IV. The compound of Formula S-IV is then reacted with diphenylphosphoryl azide (DPPA), resulting in the formation of a compound of Formula S-V. Finally, the compound of Formula S-V is deprotected.

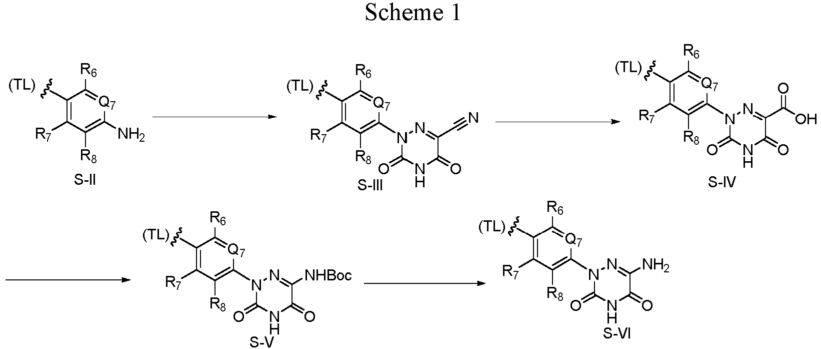

As described in Scheme 2, the aromatic amine compound of Formula S-II can be converted to a boronic acid compound of Formula S-VII, first by generation of the diazonium salt, followed by reaction with tetrahydroxydiborane. The corresponding boronic acid is then coupled with a suitably protected (with protecting group 'PG') bromo-azauracil compound of Formula Int-I. The resulting bromide compound of Formula S-VIII is then further transformed, either by a substitution reaction, or by transition metal catalyzed transformations as exemplified in the Examples section, below. Removal of the protecting group 'PG' results in a compound of Formula S-X.

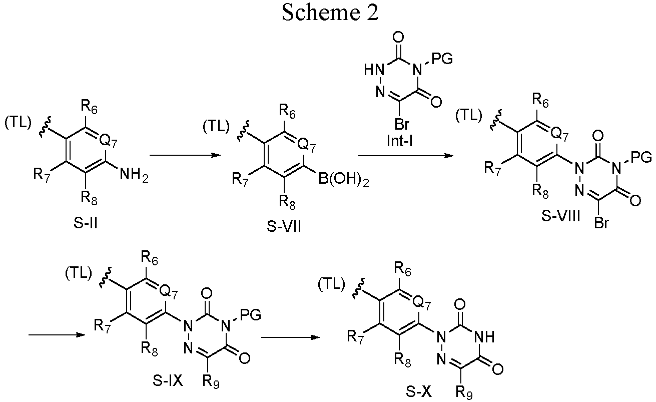

The synthesis of an aromatic amine compound of Formula S-XIV is described in Scheme 3. A compound of Formula S-XI is reacted with a compound of Formula S-XII, ('X' represents a halogen like F or Cl), followed by protection with a protecting group 'PG', resulting in a compound of Formula S-XIII. Reduction of the nitro functionality of the compound of Formula S-XIII results in the formation of an aromatic amine compound of Formula S-XIV.

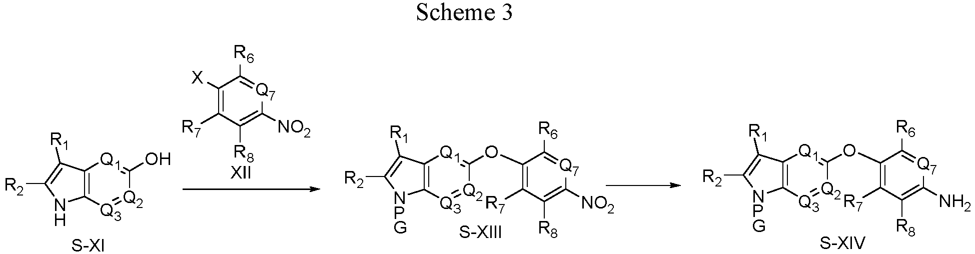

Scheme 4 describes the synthesis of a compound of Formula S-XIX. A transmetalation reaction of a compound of Formula S-XVI ('X' in the structure represents a halogen, e.g., Br or I) is followed by an addition to the aldehyde of general Formula S-XV affording the alcohol compound of Formula S-XVII, which is then reduced to a compound of Formula S-XVIII. Deprotection of PG2 of the compound of Formula S-XVIII results in the formation of a compound of Formula S-XIX.

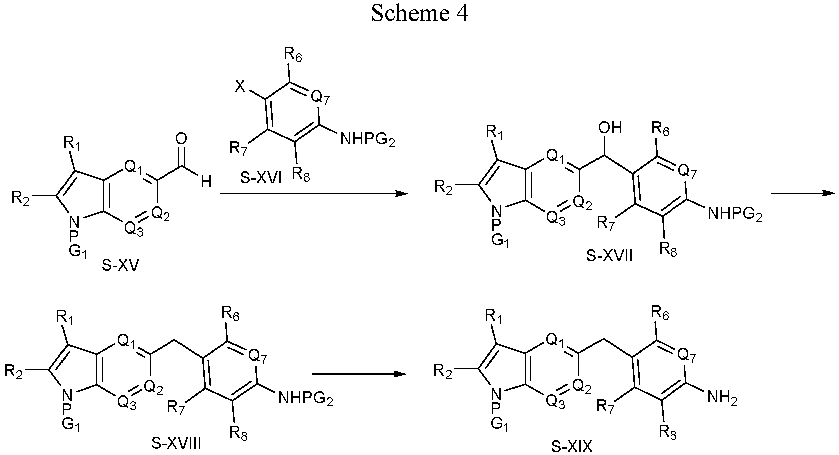

As described in Scheme 5, a compound of Formula S-XX is obtained by coupling a compound of Formula S-VII with a bromide compound of Formula Int-II.

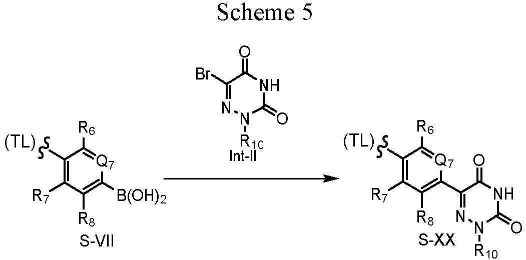

Scheme 6a depicts an alternative synthesis of the compounds of the general Formula S-XIX. A compound of Formula S-XXI (wherein the structure represents a halogen, e.g., Cl, Br or I) is coupled with a compound of Formula S-XXII in a Suzuki type coupling, resulting in the formation of a compound of Formula S-XXIII. The protecting group PG₃, for example a benzyl moiety, can then be removed resulting in a compound of Formula S-XXIV. The resulting phenol functionality can then be replaced with a triflate group, resulting in a compound of general Formula S-XXV. The -OTf group can then be replaced with a -NH₂ moiety via a transition metal catalyzed reaction, such as a Buchwald coupling, for example with t-Butyl carbamate or benzophenone imine, followed by deprotection, resulting in a compound of general Formula S-XIX. The method described in Scheme 6a could also apply when an indazole halogenide is used as the starting material instead of S-XXI. Alternatively, the indazole halogenide may undergo lithium-halogen exchange. The resulting aryl lithium species could react with an aldehyde of type S-XXVIII to form compounds of formula S-XXIX. Further elaboration of structures of formula S-XXIX are described in Scheme 7.

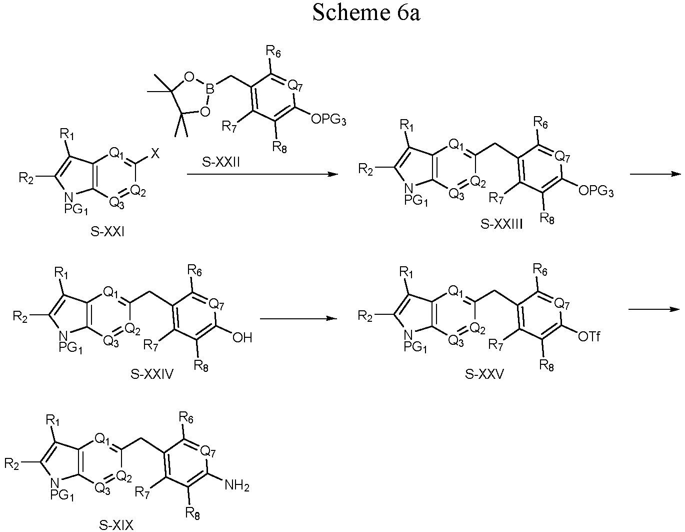

Scheme 6b depicts the synthesis of a compound of formula S-XXII from a compound of formula S-XXVI in a Suzuki reaction with 4,4,5,5-tetramethyl-2-[(4,4,5,5-tetramethyl-1,3,2-dioxaborolan-2-yl)methyl]-1,3,2-dioxaborolane.

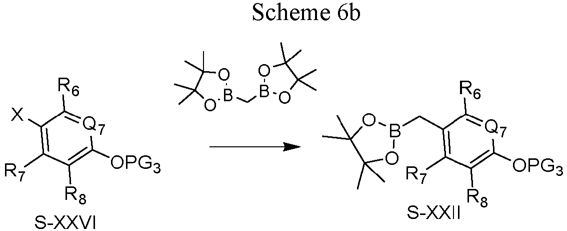

Scheme 7 describes the synthesis of a compound of Formula S-XXXI. A transmetalation reaction of a compound of Formula S-XXVII ('X' in the structure represents a halogen, e.g., Br or I) is followed by an addition to the aldehyde of general formula S-XXVIII affording the alcohol compound of Formula S-XXIX, which is then reduced to a compound of Formula S-XXX. Deprotection of PG2 of the compound of Formula S-XXX results in the formation of a compound of Formula S-XXXI.

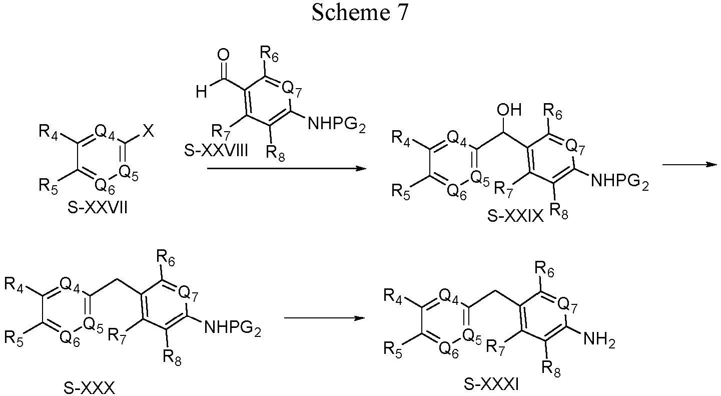

Scheme 8 describes the general synthesis of compounds of Formula S-XXI and products thereof. When R₁, for example, is hydrogen, and Y is hydrogen, then R₁ can be transformed to the corresponding acyl group via the acid chloride and a Lewis acid (e.g. InBr₃) in a non-polar, aprotic solvent (e.g. dichloroethane). The newly formed ketone group can be partially reduced to the alcohol or fully reduced to the corresponding alkane. Further transformations of a ketone group are evident to those skilled in the art. In a second example, where R₁ is hydrogen, and Y is an appropriate protecting group (e.g. tosyl) then R₁ can be converted to iodide via an iodinating agent (e.g. NIS) using available literature procedures.

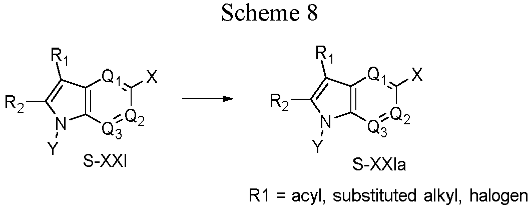

Scheme 9 describes the general synthesis of compounds of Formula S-XXXIII. 4-bromo-6-chloropyridazin-3-amine can be converted to the corresponding 4-aryl-6-chloropyridazin-3-amine (S-XXXII) using, for example, an ArB(OH)₂ (e.g. phenylboronic acid) and a palladium catalyst (e.g. PdCl₂(PPh₃)₂) in typical Suzuki-Miyaura conditions. Subsequent transformation of the amino group to a Cl via typical Sandmeyer reaction conditions (e.g. CuCl₂, t-Bu-ONO, acetonitrile, heat) affords compounds of type S-XXXIII.

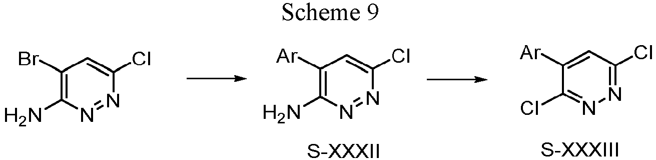

Scheme 10 describes the general synthesis of compounds of Formula S-XXXVI. Compounds of formula S-XXXIV may be coupled with the phenol HO-CE-HD under a Cu(I) mediated coupling reaction in DMSO with base (e.g. K₂CO₃) at elevated temperature to afford intermediates of type S-XXXV. Subsequent hydrolysis of the chloropyridazine via acetic acid and an acetate salt (e.g. NaOAc) affords products of formula S-XXXVI where the desired regioisomer can be isolated. In the context of scheme 10, the group HD may contain a general protecting group that can be cleaved at the stage of the intermediates S-XXXV or at the end of the synthesis to afford compounds of formula S-XXXVI.

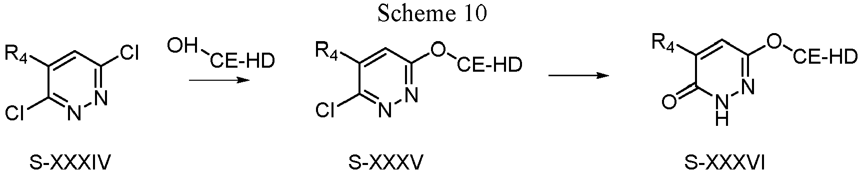

The synthesis of compounds of formula S-XXXVII can be performed using literature procedures. For example, an alkynylester can be combined with a protected aniline using a Ru catalyst under the conditions described in Org. Lett. 2014, 16, 3568-3571 to afford 6-bromoquinolones. Alternatively, the compounds of formula S-XXXVII can be formed by other procedures reported in the literature, including but not limited to the following examples: (a) Kadnikov, D. V.; et al. J. Org. Chem. 2004, 69, 6772. (b) Manley, P. J.; et al. Org. Lett. 2004, 6, 2433. (c) Jia, C.; Piao, D.; et al. J. Org. Chem. 2000, 65, 7516. (d) Inamoto, K.; et al. J. Org. Chem. 2010, 75, 3900. (e) Ferguson, J.; et al. Org. Lett. 2013, 15, 1998. (f) Fan, H; Org. Lett. 2018, 20, 7929-7932.

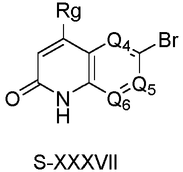

Alternatively, Scheme 11 depicts the synthesis of compounds of formula S-XXXVII that can be made starting from a dihalogenated aminoaryl (S-XXXVII-a). The amine is then protected (e.g. trimethylacetamide) to afford an amide intermediate (S-XXXVII-b). Under strongly basic conditions (e.g. n-buLi, THF) S-XXXVII-b reacts with an aldehyde containing the desired Rg substitution to afford the alcohol product S-XXXVI-c, which is oxidized (via a common oxidizing agent; e.g. Dess-Martin reagent) in a subsequent step to afford the ketone S-XXXVII-d. The ketone undergoes an aldol type reaction with a protected ester (e.g. t-butyl) to afford intermediate S-XXXVII-e. In the final step, an intramolecular cyclization can occur to afford the compounds of formula S-XXXVII.

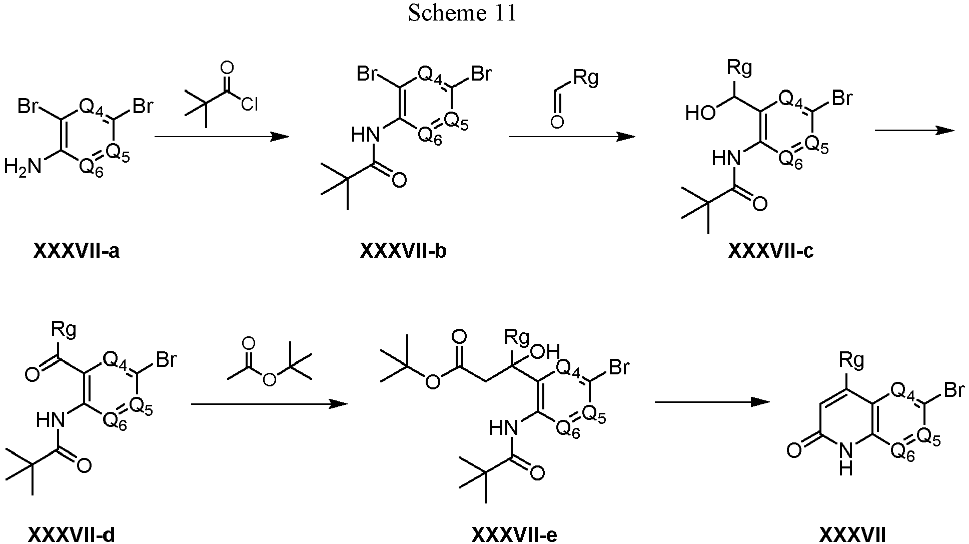

Incorporation of the compounds of formula S-XXXVII to form final products can occur using analogous methods as described in Schemes 6a and 6b.

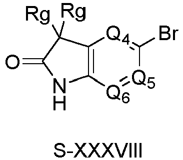

Several methods exist to access compounds of formula XXXVIII and they include but are not limited to the methods found in the following references or referenced therein: (a) Hajra,S; et al. Org. Lett. 2018, 20, 4540-4544. (b) Zaytsev, S. et al. Journal of Organic Chemistry (2018), 83(15), 8695-8709. (c) Wu, C; et al. Organic Letters (2014), 16(7), 1960-1963. (d) Ye, N; et al. ACS Infect Dis. 2016, 2(6), 382-392. Incorporation of the compounds of formula S-XXXVIII to form final products can occur using analogous methods as described in Schemes 6a and 6b.

As described in Scheme 12, a compound of Formula S-XXXIX is obtained by coupling a compound of Formula S-VII with an azauracil compound of Formula Int-A. The benzyloxymethyl acetal can then be deprotected using a variety of methods described in the literature.

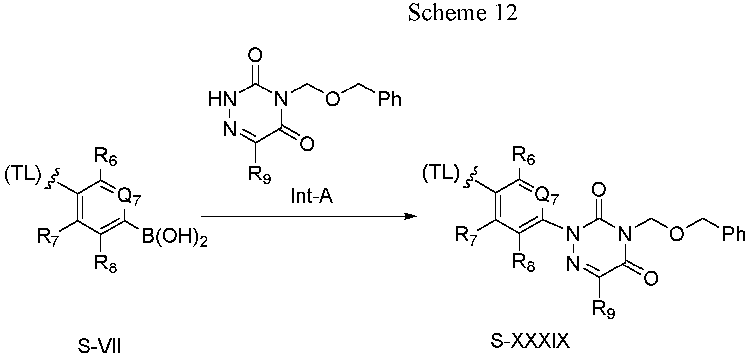

### PHARMACEUTICAL COMPOSITIONS

In another aspect, disclosed herein are pharmaceutical compositions comprising, consisting essentially of, or consisting of a compound as described herein, and at least one pharmaceutically acceptable excipient.

In another aspect, disclosed herein are pharmaceutical compositions comprising a compound of Formula I, as described herein, and a pharmaceutically acceptable diluent, excipient, or carrier. In some embodiments, disclosed herein are pharmaceutical compositions comprising, consisting essentially of, or consisting of a compound of Formula I, as described herein, and at least one pharmaceutically acceptable diluent, excipient, or carrier.

In another aspect, disclosed herein are pharmaceutical compositions comprising, consisting essentially of, or consisting of a compound of Formula I', as described herein, and at least one pharmaceutically acceptable excipient.

The pharmaceutical composition disclosed herein may comprise a pharmaceutically acceptable carrier, such as diluents, disintegrants, sweetening agents, glidants, or flavoring agents and may be formulated into an oral dosage form such as tablets, capsules, powders, granules, suspensions, emulsions, or syrups; or a parenteral dosage form such as liquids for external use, suspensions for external use, emulsions for external use, gels (ointments or the like), inhaling agents, spraying agents, injections, etc. Said dosage forms may be formulated in various forms, e.g., a dosage form for single administration or for multiple administrations.

The pharmaceutical composition disclosed herein may include excipients such as lactose, corn starch, or the like, glidants such as magnesium stearate, etc., emulsifying agents, suspending agents, stabilizers, and isotonic agents, etc. If desired, a sweetening agent and/or a flavoring agent may be added. Exemplary excipients include, without limitation, polyethylene glycol (PEG), hydrogenated castor oil (HCO), cremophors, carbohydrates, starches (e.g., corn starch), inorganic salts, antimicrobial agents, antioxidants, binders/fillers, surfactants, lubricants (e.g., calcium or magnesium stearate), glidants such as talc, disintegrants, diluents, buffers, acids, bases, film coats, combinations thereof, and the like.

Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like.

Inorganic salt or buffers include, but are not limited to, citric acid, sodium chloride, potassium chloride, sodium sulfate, potassium nitrate, sodium phosphate monobasic, sodium phosphate dibasic, and combinations thereof.

Suitable antioxidants for use in the present disclosure include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

Additional exemplary excipients include surfactants such as polysorbates, e.g., "Tween 20" and "Tween 80," and pluronics such as F68 and F88 (both of which are available from BASF, Mount Olive, N.J.), sorbitan esters, lipids (e.g., phospholipids such as lecithin and other phosphatidylcholines, and phosphatidylethanolamines), fatty acids and fatty esters, steroids such as cholesterol, and chelating agents, such as EDTA, zinc and other such suitable cations.

Further, a composition disclosed herein may optionally include one or more acids or bases. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Non-limiting examples of suitable bases include bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of any individual excipient in the composition will vary depending on the role of the excipient, the dosage requirements of the active agent components, and particular needs of the composition. Generally, however, the excipient will be present in the composition in an amount of about 1% to about 99% by weight, preferably from about 5% to about 98% by weight, more preferably from about 15 to about 95% by weight of the excipient. In general, the amount of excipient present in a composition of the disclosure is selected from the following: at least about 2%, 5%, 10%, 15%, 20%, 25%, 30%, 35%, 40%, 45%, 50%, 55%, 60%, 65%, 70%, 75%, 80%, 85%, 90%, or even 95% by weight.

The pharmaceutical compositions described herein can be administered to a human patient per se, or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, Pa., 18th edition, 1990.

Suitable routes of administration may, for example, include oral, transdermal, rectal, transmucosal, or intestinal administration; parenteral delivery, including intramuscular, subcutaneous, intravenous, intramedullary injections, as well as inhalation, intrathecal, direct intraventricular, intraperitoneal, intranasal, or intraocular injections.

The pharmaceutical compositions disclosed herein may be manufactured in a manner that is itself known, e.g., by means of conventional mixing, dissolving, granulating, dragee-making, levigating, emulsifying, encapsulating, entrapping or tableting processes. These pharmaceutical compositions, then, may be formulated in a conventional manner using one or more known physiologically acceptable carriers comprising excipients and/or auxiliaries, which facilitate processing of the active compounds into preparations that can be used pharmaceutically. Any of the well-known techniques, carriers, and excipients may be used as suitable and as understood in the art; e.g., in Remington's Pharmaceutical Sciences, above.

Pharmaceutical compositions suitable for use in the presently disclosed formulations include compositions where the active ingredients are contained in an amount effective to achieve its intended purpose. More specifically, a therapeutically effective amount means an amount of compound effective to prevent, alleviate or ameliorate symptoms of disease or prolong the survival of the subject being treated. In some embodiments, a therapeutically effective amount means an amount of compound effective to alleviate or ameliorate symptoms of disease or prolong the survival of the subject being treated.

Although the exact dosage can be determined on a drug-by-drug basis, in most cases, some generalizations regarding the dosage can be made. The daily dosage regimen for an adult human patient may be, for example, an oral dose of between 0.001 mg and 1000 mg of each ingredient, preferably between 0.01 mg and 500 mg, for example 1 to 200 mg or each active ingredient of the pharmaceutical compositions disclosed herein or a pharmaceutically acceptable salt thereof calculated as the free base or free acid, the composition being administered 1 to 4 times per day or per week. Alternatively, the compositions disclosed herein may be administered by continuous such as sustained, delayed, or extended release, preferably at a dose of each ingredient up to 500 mg per day. Thus, the total daily dosage by oral administration of each ingredient will typically be in the range 0.1 mg to 2000 mg.

### METHODS OF TREATMENT

In another aspect, disclosed herein are methods of treating a thyroid hormone receptor related disorder in a patient, the method comprising, consisting essentially of, or consisting of the steps of identifying a patient in need of treatment for the thyroid hormone receptor related disorder, and administering to the patient, or contacting the patient with, a compound as described herein.

In another aspect, disclosed herein are methods of treating a thyroid hormone receptor related disorder in a patient, the method comprising the steps of identifying a patient in need of treatment for the thyroid hormone receptor related disorder, and administering to the patient, or contacting the patient with, a compound of Formula **I,** as described herein. In some embodiments, the method of treating a thyroid hormone receptor related disorder in a patient consists essentially of or consists of the steps of identifying a patient in need of treatment for the thyroid hormone receptor related disorder, and administering to the patient, or contacting the patient with, a compound of Formula I, as described herein.

In another aspect, disclosed herein are methods of treating a thyroid hormone receptor related disorder in a patient, the method comprising, consisting essentially of, or consisting of the steps of identifying a patient in need of treatment for the thyroid hormone receptor related disorder, and administering to the patient, or contacting the patient with, a compound of Formula I', as described herein.

In some embodiments, a health care professional, such as a physician, physician's assistant, nurse practitioner, or the like, identifies an individual as being in need of treatment for the thyroid hormone receptor related disorder, and/or a candidate for treatment with a compound disclosed herein. The identification may be based on medical test results, non-responsiveness to other, first-line therapies, the specific nature of the particular liver disorder, or the like.

In some embodiments, the thyroid hormone receptor related disorder is selected from non-alcoholic steatohepatitis (NASH), obesity, hyperlipidemia, hypercholesterolemia, diabetes, liver steatosis, atherosclerosis, cardiovascular diseases, hypothyroidism, and thyroid cancer.

In another aspect, disclosed herein are methods of treating a disorder or disease in a subject in need thereof, the method comprising, consisting essentially of, or consisting of administering to the subject a therapeutically effective amount of a compound or composition disclosed herein, wherein the disorder or disease is selected from non-alcoholic steatohepatitis (NASH), obesity, hyperlipidemia, hypercholesterolemia, diabetes, liver steatosis, atherosclerosis, cardiovascular diseases, hypothyroidism, and thyroid cancer.

In another aspect, disclosed herein are methods of treating NASH in a subject in need thereof, the method comprising, consisting essentially of, or consisting of administering to the subject a therapeutically effective amount of a compound or composition disclosed herein.

In another aspect, disclosed herein are methods of treating obesity in a subject in need thereof, the method comprising, consisting essentially of, or consisting of administering to the subject a therapeutically effective amount of a compound or composition disclosed herein.

In another aspect, disclosed herein are methods of treating hyperlipidemia in a subject in need thereof, the method comprising, consisting essentially of, or consisting of administering to the subject a therapeutically effective amount of a compound or composition disclosed herein.

In another aspect, disclosed herein are methods of treating hypercholesterolemia in a subject in need thereof, the method comprising, consisting essentially of, or consisting of administering to the subject a therapeutically effective amount of a compound or composition disclosed herein.

In another aspect, disclosed herein are methods of treating diabetes in a subject in need thereof, the method comprising, consisting essentially of, or consisting of administering to the subject a therapeutically effective amount of a compound or composition disclosed herein.

In another aspect, disclosed herein are methods of treating liver steatosis in a subject in need thereof, the method comprising, consisting essentially of, or consisting of administering to the subject a therapeutically effective amount of a compound or composition disclosed herein.

In another aspect, disclosed herein are methods of selectively modulating the activity of a thyroid hormone receptor beta (THR-β) comprising, consisting essentially of, or consisting of contacting a compound as described herein, with a thyroid hormone receptor. In some embodiments, the contacting is *in vitro* or *ex vivo,* whereas in other embodiments, the contacting is *in vivo.*

In another aspect, disclosed herein are methods of selectively modulating the activity of a thyroid hormone receptor beta (THR-β) comprising contacting a compound of Formula I, as described herein, with a thyroid hormone receptor. In some embodiments, the contacting is *in vitro* or *ex vivo,* whereas in other embodiments, the contacting is *in vivo.* In some embodiments, the method of selectively modulating the activity of a thyroid hormone receptor beta (THR-β) consists essentially of or consists of contacting a compound of Formula I, as described herein, with a thyroid hormone receptor.

In another aspect, disclosed herein are methods of selectively modulating the activity of a thyroid hormone receptor beta (THR-β) comprising, consisting essentially of, or consisting of contacting a compound of Formula I', as described herein, with a thyroid hormone receptor. In some embodiments, the contacting is *in vitro* or *ex vivo,* whereas in other embodiments, the contacting is *in vivo.*

In another aspect, disclosed herein are methods of selectively modulating the activity of a thyroid hormone receptor beta (THR-β) comprising, consisting essentially of, or consisting of contacting a composition described herein, with a thyroid hormone receptor. In some embodiments, the contacting is *in vitro* or *ex vivo,* whereas in other embodiments, the contacting is *in vivo.*

### EXAMPLES

The following exemplify aspects of the present invention and is not limiting of its scope. Conditions for the preparation of several of the compounds disclosed herein are presented. Procedures for the synthesis of common intermediates are provided only once. The chemical names were generated using Marvin 17.28.0 or Chemdraw 18.1.

### Table of Abbreviations:

The following abbreviations are used in the present disclosure:

| | |
|---|---|
| Ac | Acetate |
| ACN | Acetonitrile |
| anhyd. | Anhydrous |
| aq. | Aqueous |
| Bu | Butyl |
| CAN | Ceric ammonium nitrate |
| conc. | Concentrated |
| DCM | Dichloromethane |
| DIPEA | N,N-Diisopropylethylamine |
| DMA | N,N-dimethylacetamide |
| DMF | N,N-Dimethylformamide |
| DMSO | Dimethyl sulfoxide |
| DPPA | Diphenylphosphoryl azide |
| dppf | 1,1'-Bis(diphenylphosphino)ferrocene |
| EA=EtOAc | Ethyl acetate |
| ECF | Ethyl chloroformate |
| Et | Ethyl |
| EtOH | Ethanol |
| FA | Formic acid |
| g | Gram(s) |
| h | Hour(s) |
| Me | Methyl |
| MeOH | Methanol |
| min | Minute(s) |
| NIS | N-Iodosuccinimide |
| PE | Petroleum ether |
| rt | Room temperature |
| sat. | Saturated |
| Selectfluor^{™} | 1-Chloromethyl-4-fluoro-1,4-diazoniabicyclo[2.2.2]octane bis(tetrafluoroborate) |
| TBAF | Tetra-*n*-butylammonium fluoride |
| TBSCL | *t*-Butyldimethylsilyl chloride |
| TFA | Trifluoroacetic acid |
| THF | Tetrahydrofuran |

### SYNTHESIS OF BUILDING BLOCKS

### 1. Synthesis of 3-isopropyl-1H-indol-5-ol

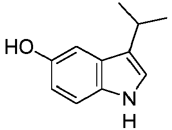

To a stirred mixture of (4-methoxyphenyl)hydrazine (10.00 g, 72.375 mmol, 1.0 eq) in100 mL of AcOH was added isovaleraldehyde (6.23 g, 0.072 mmol, 1 eq) dropwise at 80 °C. The resulting mixture was stirred for 2 h at 120 °C. The resulting mixture was concentrated under vacuum. The resulting mixture was extracted with EA (3x100 mL). The combined organic layers were washed with brine (1x100 mL), dried over anhydr. Na₂SO₄. After filtration, the filtrate was concentrated under reduced pressure. The residue was purified by silica gel column chromatography to afford 3-isopropyl-5-methoxy-1*H*-indole (2.6 g, 19%) as a brown solid.

To a solution of 3-isopropyl-5-methoxy-1*H*-indole (9.03 g, 47.713 mmol, 1.0 eq) in DCM (100.00 mL) was added boron tribromide (35.88 g, 143.217 mmol, 3 eq) dropwise for 1 h at -78 °C. The resulting mixture was stirred for additional 3 h at rt. The reaction was quenched by the addition of H₂O at 0 °C. The resulting mixture was extracted with EA. The combined organic layers were washed with brine, dried over anhydrous Na₂SO₄. After filtration, the filtrate was concentrated under reduced pressure. The residue was purified by silica gel column chromatography to afford 3-isopropyl-1*H*-indol-5-ol (5.86 g, 56%) as a black oil.

### 2a. Synthesis of 5-bromo-3-isopropyl-1H-indole

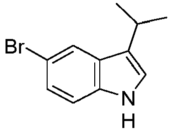

To a stirred solution of 4-bromophenyl-hydrazine (50.00 g, 267.32 mmol, 1.00 eq) in AcOH (500 mL) was added isovaleraldehyde (23.03 g, 267.37 mmol, 1.00 eq) dropwise at 80 °C. The resulting mixture was stirred for 3 h at 120 °C. The resulting mixture was concentrated under vacuum. The resulting mixture was extracted with EA (3x500 mL). The combined organic layers were washed with brine (1x300 mL), dried over anhydrous Na₂SO₄. After filtration, the filtrate was concentrated under reduced pressure. The residue was purified by silica gel column chromatography to afford 5-bromo-3-isopropyl-1*H*-indole (28 g, 44%) as a brown solid.

### 2b. Synthesis of 5-bromo-3-isopropyl-1-(p-tolylsulfonyl)pyrrolo[3,2-b]pyridine:

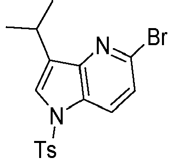

To a stirred solution of 5-bromo-3-isopropyl-1*H*-pyrrolo[3,2-b]pyridine (2.0 g, 8.36 mmol, 1 eq), DMAP (20.44 mg, 167.29 µmol, 0.02 eq) and DIPEA (2.38 g, 18.40 mmol, 2.2 eq) in DCM (60 mL) was added TosCl (1.91 g, 10.04 mmol, 1.2 eq) at 20 °C. Then the resulting mixture was stirred at 20 °C for 12 h. TLC (Petroleum ether/Ethyl acetate=5/1, UV) showed the starting material was consumed completely. The mixture was diluted with H₂O (100 mL) and extracted with DCM (100 mLx3). The combined organic layers were washed with brine (200 mL), dried over anhydrous Na₂SO₄, filtered and concentrated to give a residue. The residue was purified by flash silica gel chromatography (Ethyl acetate in Petroleum ether = 0~10%) to give 5-bromo-3-isopropyl-1-(p-tolylsulfonyl)pyrrolo[3,2-b]pyridine (2.6 g, 5.61 mmol, 67.0% yield) as an off-white solid.

### 2c. Synthesis of 5-bromo-3-pentyl-1H-indazole

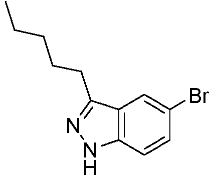

To a solution of 5-bromo-2-fluoro-benzaldehyde (25 g, 123.15 mmol, 1 eq) in THF (100 mL) was added bromo(pentyl)magnesium (1 M, 184.73 mL, 1.5 eq) at 0 °C. The mixture was stirred at 20 °C for 1 hr. The reaction mixture was quenched by addition sat. aq. NH₄Cl (50 mL) at 0 °C, and then diluted with H₂O (50 mL) and extracted with EA (100 mL * 2). The combined organic layers were washed with brine (50 mL), dried over anhydrous Na₂SO₄, filtered and concentrated under reduced pressure to give a residue, which was purified by flash silica gel chromatography (Eluent of 0~30% Ethyl acetate/Petroleum ether) to give 1-(5-bromo-2-fluoro-phenyl)hexan-1-ol (9.5 g, 34.53 mmol, 28 % yield) as colorless oil.

To a solution of 1-(5-bromo-2-fluoro-phenyl)hexan-1-ol (9.5 g, 34.53 mmol, 1 eq) in DCM (100 mL) was added 4A MS (10 g) and PDC (25.98 g, 69.05 mmol, 2 eq) at 20 °C. The mixture was stirred at 20 °C for 12 h. The mixture was filtered and concentrated under reduced pressure to give a residue, which was purified by flash silica gel chromatography (Eluent of 0~30% Ethyl acetate/Petroleum ether) to give 1-(5-bromo-2-fluoro-phenyl)hexan-1-one (8.2 g, 30.02 mmol, 87 % yield) as a pale yellow solid.

To a solution of 1-(5-bromo-2-fluoro-phenyl)hexan-1-one (5 g, 18.31 mmol, 1 eq) in NMP (2 mL) was added hydrazine hydrate (2.16 g, 36.61 mmol, 2.09 mL, 85% purity, 2 eq) at 20 °C. The mixture was stirred at 100 °C for 12 h. The reaction mixture was added to ice water (50 mL) and extracted with EA (50 mL * 2). The combined organic layers were washed with brine (10 mL), dried over anhydrous Na₂SO₄, filtered and concentrated under reduced pressure to give a residue, which was purified by flash silica gel chromatography (Eluent of 0~20% Ethyl acetate/Petroleum ether) to give 5-bromo-3-pentyl-1*H*-indazole (2 g, 7.49 mmol, 41% yield) as a white solid.

### 3. Synthesis of 3-isopropyl-1-(4-methylbenzenesulfonyl)indole-5-carbaldehyde

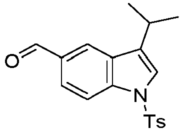

To a stirred solution of 5-bromo-3-isopropyl-1*H*-indole (3.0 g, 12.6 mmol, 1.00 eq) in toluene (50 mL) was added Bu₄NHSO₄ (0.43 g, 1.27 mmol, 0.10 eq) in portions at 0 °C. TsCl (2.90 g, 15.26 mmol, 1.2 eq) was added dropwise at 0 °C. The resulting mixture was stirred overnight at rt. The resulting mixture was concentrated under vacuum. The resulting mixture was extracted with EA (3x100 mL). The combined organic layers were washed with brine (1x200 mL), dried over anhydrous Na₂SO₄. After filtration, the filtrate was concentrated under reduced pressure. The residue was purified by silica gel column chromatography to afford 5-bromo-3-isopropyl-1-(4-methylbenzenesulfonyl)indole (4.0 g, 67%) as a light yellow solid.

To a stirred solution of 5-bromo-3-isopropyl-1-(4-methylbenzene-sulfonyl)indole (4 g, 10.2 mmol, 1.00 eq) in THF were added *n*-BuLi (31.2 ml, 51.0 mmol, 5.0 eq, 1.6 M in hexane) dropwise at -78 °C under nitrogen atmosphere. The resulting mixture was stirred for 40min at -78 °C under nitrogen atmosphere. DMF (3.70 g, 0.051 mmol, 5.0 eq) was added at -78 °C under nitrogen atmosphere. The resulting mixture was stirred for 1.5 h at -78 °C under nitrogen atmosphere. The reaction was quenched with water (50 mL). The resulting mixture was extracted with EA (3x100 mL). The combined organic layers were washed with brine (1x200 mL), dried over anhydrous Na₂SO₄. After filtration, the filtrate was concentrated under reduced pressure. The residue was purified by silica gel column chromatography to afford 3-isopropyl-1-(4-methylbenzenesulfonyl)indole-5-carbaldehyde (1.1g, 31%) as a light yellow oil.

### 4. Synthesis of 4-[(benzyloxy)methyl]-6-bromo-2H-1,2,4-triazine-3,5-dione

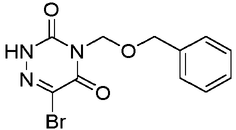

6-bromo-2,4-dihydro-1,2,4-triazine-3,5-dione (10.00 g, 52.091 mmol, 1.00 eq) was placed in acetic anhydride (50 mL) under reflux (140 °C) for 5 h. After dry concentration of the reaction medium, a precipitate is isolated and then recrystallized from ether to get the desired product. The desired product was isolated as a light yellow solid (11.6 g, 95% pure, 90% yield).

NaH (2.19 g, 54.755 mmol, 1.10 eq, 60%) was placed in DMF (50 mL) under nitrogen. A solution of 2-acetyl-6-bromo-4*H*-1,2,4-triazine-3,5-dione (11.60 g, 49.571 mmol, 1.0 eq) in DMF (150 mL) was poured dropwise. The reaction medium was stirred for 1 h at rt and then [(chloromethoxy)methyl]benzene (8.54 g, 54.53 mmol, 1.1 eq) was added and stirred then continues for 18h at rt. After dry concentration the obtained residue was taken up with H₂O (200 mL) and extracted with ethyl acetate (EA) (3x500 mL). After drying on Na₂SO₄, the organic phase are evaporated and the obtained clear oil was purified and 15 g of crystals were isolated. Crystals were placed in EtOH (400 mL) in the presence of TsOH (100.0 mg, 0.581 mmol, 0.01 eq). This mixture was heated with reflux for 4 h and then dry concentrated. The residue was taken up with H₂O and then extracted with EA. After drying and evaporation of the organic phases, the desired product was obtained as a yellow oil (9.5 g, yellow oil, 90% pure, 61% yield).

### 5a. Synthesis of 6-bromo-2-methyl-4H-1,2,4-triazine-3,5-dione

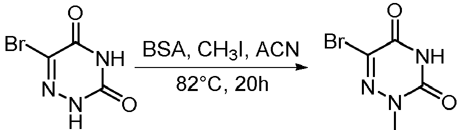

To a stirred solution of 6-bromo-2,4-dihydro-1,2,4-triazine-3,5-dione (200.0 mg, 1.04 mmol, 1.0 eq) in ACN (5 mL) were added BSA (529.8 mg, 2.61 mmol, 2.5 eq) dropwise at 0 °C under argon atmosphere. The resulting mixture was stirred for 3 h at 82 °C under argon atmosphere, and then added CH₃I (251.4 mg, 1.77 mmol, 1.7 eq) dropwise at 82 °C, continued stirred 20 h at 82 °C. The mixture was allowed to cool down to rt, concentrated under reduced pressure, and then dissolved in DCM, washed with water and brine, dried over anhydrous Na₂SO₄. After filtration, the filtrate was concentrated under reduced pressure. The residue was purified by silica gel column chromatography to afford 6-bromo-2-methyl-4*H-*1,2,4-triazine-3,5-dione (870 mg, 69%) as a yellow solid.

### 5b. Synthesis of 6-bromo-2-ethyl-4H-1,2,4-triazine-3,5-dione

6-bromo-2-ethyl-4*H*-1,2,4-triazine-3,5-dione was prepared similarly as described for 6-bromo-2-methyl-4*H*-1,2,4-triazine-3,5-dione using 2.5 eq of iodoethane instead of 1.7 eq CH₃I.

### 6. Synthesis of 5-(2,6-dichloro-4-nitrophenoxy)-3-isopropyl-1H-indole

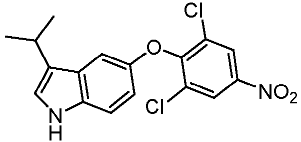

To a stirred solution of 3-isopropyl-1*H*-indol-5-ol (1.00 g, 5.71 mmol, 1.00 eq) in THF was added tert-butoxypotassium (0.64 g, 5.70 mmol, 1.0 eq) dropwise at 0 °C. The resulting mixture was stirred for 30 min at rt. After completion of reaction, the resulting mixture was concentrated under vacuum and re-dissolved by DMF. The second reaction flask: To a stirred solution of 1,2,3-trichloro-5-nitrobenzene (1.29 g, 5.70 mmol, 1.0 eq) in DMF was added the first reaction flask at 0 °C. The resulting mixture was stirred for 5 min at 0 °C, and then warmed to 100 °C and stirred for 1 h. The resulting mixture was extracted with EA. The combined organic layers were washed with brine and dried over anhydrous Na₂SO₄. The residue was purified by silica gel column chromatography to afford 5-(2,6-dichloro-4-nitrophenoxy)-3-isopropyl-1H-indole (1.3 g, 55%) as a yellow solid.

### 7. Synthesis of 5-(2,6-dimethyl-4-nitrophenoxy)-3-isopropyl-1H-indole

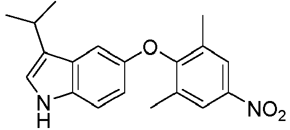

To a stirred mixture of 3-isopropyl-1*H*-indol-5-ol (5.10 g, 29.10 mmol, 1.0 eq) in 50 mL of DMSO were added K₂CO₃ (4.42 g, 32.0 mmol, 1.1 eq) and 2-fluoro-1,3-dimethyl-5-nitrobenzene (4.92 g, 29.1 mmol, 1.0 eq) in portions at rt. The resulting mixture was stirred for 2 h at 100 °C. The resulting mixture was extracted with EA (3x100 mL). The combined organic layers were washed with brine (3x50 mL), dried over anhydrous Na₂SO₄. After filtration, the filtrate was concentrated under reduced pressure. The residue was purified by silica gel column chromatography to afford 5-(2,6-dimethyl-4-nitrophenoxy)-3-isopropyl-1H-indole (6 g, 64%) as a light yellow solid.

### 8. Synthesis of 5-(2,6-dichloro-4-nitrophenoxy)-1H-indole

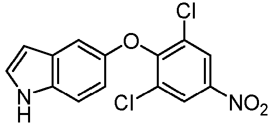

To a solution of 5-hydroxyindol (16.0 g, 120.2 mmol, 1.0 eq) in acetonitrile (320 mL) was added potassium tert-butoxide (13.48 g, 120.2 mmol, 1.0 eq) at 0 °C. After 20 mins, the mixture was concentrated to remove acetonitrile and dissolved in N,N-dimethylformamide (320 mL). Then 1,2,3-trichloro-5-nitrobenzene (27.21 g, 120.2 mmol, 1.00 eq) was added at 0 °C. The resulting solution was stirred at 100 °C overnight and then quenched with water (300 mL). The resulting solution was extracted with ethyl acetate (3x500 mL) and the organic layers were combined, washed with brine (2x300 mL), dried over anhydrous sodium sulfate, filtered and concentrated under reduced pressure. The residue was purified to provide 28 g (68% yield) of 5-(2,6-dichloro-4-nitrophenoxy)-1*H*-indole as yellow solid.

### 9. Synthesis of 5-dichloro-4-[[3-isopropyl-1-(4-methy-lbenzenesu-lfonyl)indol-5-yl]oxy]aniline

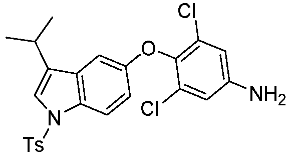

To a stirred solution of 5-(2,6-dichloro-4-nitrophenoxy)-3-isopropyl-1*H-*indole (1.29 g, 3.532 mmol, 1.0 eq) in toluene (50.00 mL) was added TsCl (0.81 g, 4.239 mmol, 1.2 eq) in toluene (10 mL), KOH (50%) (21.4 mL), Bu₄NHSO₄ (0.12 g, 0.353 mmol, 0.1 eq) dropwise at 0 °C. The resulting mixture was stirred for 6 h at rt. After completion of reaction, the resulting mixture was extracted with EA (3x50 mL). The combined organic layers were washed with brine, dried over anhydrous Na₂SO₄. After filtration, the filtrate was concentrated under reduced pressure. The residue was purified by silica gel column chromatography to afford 5-(2,6-dichloro-4-nitrophenoxy)-3-isopropyl-1-(4-methylbenzenesulfonyl)indole (1.40 g, 75%) as a yellow solid.

To a stirred solution of 5-(2,6-dichloro-4-nitrophenoxy)-3-isopropyl-1-(4-methylben-zenesul-fonyl)indole (1.40 g, 2.73 mmol, 1.0 eq) and NH₄Cl (1.16 g, 21.62 mmol, 8.0 eq) in EtOH (5 0mL) and H₂O (25 mL) was added Fe powder(0.75 g, 13.514 mmol, 5.00 eq) in portions at rt under nitrogen atmosphere. The resulting mixture was stirred for 2 h at 50 °C under nitrogen atmosphere. The resulting mixture was filtered, the filter cake was washed with EA. The filtrate was concentrated under reduced pressure. The aqueous layer was extracted with EA, dried over anhydrous Na₂SO₄. After filtration, the filtrate was concentrated under reduced pressure. The residue was purified by silica gel column chromatography to afford 3,5-dichloro-4-[[3-isopropyl-1-(4-methy-lbenzenesulfonyl)-indol-5-yl]oxy]aniline (1.11 g, 78%) as a white solid.

### 10. Synthesis of 4-[[3-isopropyl-1-(4-methylbenzene-sulfonyl)indol-5-yl]oxy]-3,5-dimethylaniline

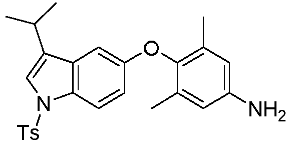

To a stirred mixture of 5-(2,6-dimethyl-4-nitrophenoxy)-3-isopropyl-1H-indole(5.00 g, 15.414 mmol, 1.00 eq) in 50 mL of toluene were added TsCl (3.53 g, 0.018 mmol, 1.2 eq), Bu₄NHSO₄ (0.52 g, 0.002 mmol, 0.1 eq), KOH (50 mL) dropwise at 0 °C. The mixture was stirred overnight at rt and quenched with water (50 mL). The resulting mixture was extracted with EA (3x200 mL). The combined organic layers were washed with brine (1x100 mL), dried over anhydrous Na₂SO₄. After filtration, the filtrate was concentrated under reduced pressure. The residue was purified by silica gel column chromatography in hexane to afford 5-(2,6-dimethyl-4-nitrophenoxy)-3-isopropyl-1-(4-methylbenzenesulfonyl)indole (5.8g, 79%) as a light yellow solid.

To a stirred mixture of 5-(2,6-dimethyl-4-nitrophenoxy)-3-isopropyl-1-(4-methylbenzene-sulfonyl)indole (5.80 g, 12.120 mmol, 1.0 eq) in MeOH (70 mL) were added Pd/C(1.74 g) in portions. The resulting mixture was stirred overnight at rt under hydrogen atmosphere. The resulting mixture was filtered, the filter cake was washed with MeOH (1x500 mL). The filtrate was concentrated under reduced pressure to get a residue, which was purified by silica gel column chromatography to afford 4-[[3-isopropyl-1-(4-methylbenzene-sulfonyl)indol-5-yl]oxy]-3,5-dimethyl-aniline (3.2 g, 59%) as a light yellow solid.

### 11. Synthesis of 4-[[3-isopropyl-1-(4-methyl-benzenesulfonyl)indol-5-yl]methyl]-3,5-dimethylaniline

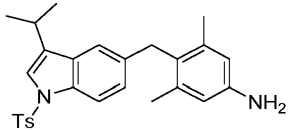

To a stirred solution of N-(4-bromo-3,5-dimethylphenyl)-2,2,2-trifluoroacetamide (260.16 mg, 0.879 mmol, 1.0 eq) in THF (5 mL) under nitrogen was added MeLi-LiBr (1.7 mL, 1.76 mmol, 2.0 eq, 1 M in ether) at -78 °C. Then t-BuLi (1.6 mL, 2.64 mmol, 3.00 eq, 1.6M in hexane) was added dropwise at -78 °C. The mixture was stirred for 20 min at -78 °C. 3-isopropyl-1-(4-methylbenzene-sulfonyl)indole-5-carbaldehyde (300 mg, 0.879 mmol, 1.0 eq) was added at -78 °C. The resulting mixture was stirred for 1 h at rt. The reaction was quenched with water (10 mL) at rt. The resulting mixture was extracted with EA (3x50 mL). The combined organic layers were washed with brine (2x100 mL), dried over anhydrous Na₂SO₄. After filtration, the filtrate was concentrated under reduced pressure. The residue was purified to afford 2,2,2-trifluoro-N-(4-[hydroxy[3-isopropyl-1-(4-methyl-benzenesulfonyl)indol-5-yl]methyl]-3,5-dimethylphenyl)acetamide (300 mg, 61%) as a white solid.

A solution of 2,2,2-trifluoro-N-(4-[hydroxy[3-isopropyl-1-(4-methyl-benzenesulfonyl)indol-5-yl]methyl]-3,5-dimethylphenyl)acetamide (300.0 mg, 0.537 mmol, 1.00 eq) in DCM (5 mL) was stirred under nitrogen at 0 °C. Then a solution of Et₃SiH (374.7 mg, 3.22 mmol, 6.0 eq) in DCM (15 mL) and TMSOTf (7.16 mg, 0.032 mmol, 0.06 eq) in DCM (5mL) was added dropwise to the reaction mixture. The reaction mixture was stirred for 30 min at 0 °C and 1.5 h at rt. The reaction mixture was quenched with saturated NaHCO₃ solution (20 mL). The resulting solution was extracted with dichloromethane (3x50 mL) and the organic layers were combined, washed with brine (2x30 mL), dried over anhydrous sodium sulfate, filtered and concentrated under reduced pressure to afford crude product. The crude product containing 2,2,2-trifluoro-N-(4-[[3-isopropyl-1-(4-methylbenzene-sulfonyl)indol-5-yl]methyl]-3,5-dimethylphenyl)acetamide was isolated as a yellow solid (260 mg, 85% pure, 76% yield).

To a solution of 2,2,2-trifluoro-N-(4-[[3-isopropyl-1-(4-methyl-benzenesulfonyl)indol-5-yl]methyl]-3,5-dimethylphenyl)acetamide (260.0 mg, 0.479 mmol, 1.0 eq) in MeOH (10 mL) and H₂O (2 mL) stirred under nitrogen at rt was added NaOH (76.66 mg, 1.917 mmol, 4.0 eq). The reaction mixture was stirred at 60 °C overnight. The reaction mixture was quenched with water (20 mL). The resulting solution was extracted with dichloromethane (3x50 mL) and the organic layers were combined, washed with brine (2x30 mL), dried over anhydrous sodium sulfate, filtered and concentrated under reduced pressure to afford crude product. The crude product was isolated as a yellow solid containing 4-[[3-isopropyl-1-(4-methyl-benzenesulfonyl)indol-5-yl]methyl]-3,5-dimethylaniline (230 mg, 90% pure, 97% yield).

### 12. Synthesis of 3,5-dichloro-4-[[1-(4-methylbenzenesulfonyl)indol-5-yl]oxy]aniline

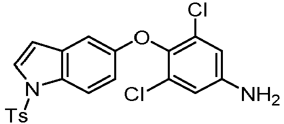

To a solution of 5-(2,6-dichloro-4-nitrophenoxy)-1*H*-indole (28.0 g, 86.65 mmol, 1.0 eq) in toluene (720 mL) was added tetrabutylammonium hydrogen sulfate (2.94 g, 8.665 mmol, 0.10 eq) and potassium hydroxide (120.00 g, 2138.8 mmol, 25.0 eq) in water (120 mL) at 0 °C. 4-Toluene sulfonyl chloride (19.82 g, 103.98 mmol, 1.20 eq) in toluene (720 mL) was added dropwise at 0 °C. Then the reaction was stirred at rt for 3 h and quenched with water (200 mL). The mixture was extracted with ethyl acetate (3x500 mL) and the organic layers were combined, concentrated under reduced pressure. The residue was purified to provide 5-(2,6-dichloro-4-nitrophenoxy)-1-(4-methylbenzenesulfonyl)indole (34 g, 78% yield) as yellow solid.

To a solution of 5-(2,6-dichloro-4-nitrophenoxy)-1-(4-methylbenzene-sulfonyl)-indole(26.00 g, 54.47 mmol, 1.0 eq) in ethanol (500 mL) and water (250 mL) was added iron dust (15.21 g, 272.36 mmol, 5.0 eq) and ammonium chloride (23.31 g, 435.78 mmol, 8.0 eq). The resulting solution was stirred for 2 h at 50 °C. The resulting mixture was filtered, the filter cake was washed with dichloromethane (6x100 mL). The filtrate was extracted with dichloromethane (3x500mL). The combined organic layers were combined, washed with brine (300 mL), dried over anhydrous sodium sulfate and concentrated under reduced pressure to provide 3,5-dichloro-4-[[1-(4-methylbenzenesulfonyl)indol-5-yl]oxy]aniline (23g, 88% yield) of as a light yellow solid.

### 13a. Synthesis of 6-(4-amino-2,6-dichlorophenoxy)-4-isopropyl-2H-pyridazin-3-one

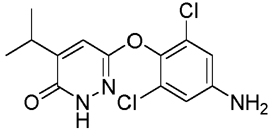

To a solution of 3,6-dichloropyridazine (50.00 g, 335.64 mmol, 1.0 eq) in H₂O (1.20 L) was added AgNO₃ (57.02 g, 335.64 mmol, 1.0 eq), isobutyric acid (29.57 g, 335.64 mmol, 1.0 eq) in portions at rt under air atmosphere and then stirred at 50 °C under nitrogen atmosphere. H₂SO₄ (98.76 g, 1006.9 mmol, 3.0 eq) was added in portions at 50 °C and stirred at 60 °C. Then (NH₄)₂S₂O₈ (229.78 g, 1006.914 mmol, 3.0 eq) was added and stirred for 30 min at 70 °C under nitrogen atmosphere. The mixture was neutralized to pH 9 with NaOH solution. The resulting mixture was extracted with EA (3x1 L). The combined organic layers were washed with brine (2x1 L), dried over anhydrous Na₂SO₄. After filtration, the filtrate was concentrated. The residue was purified by silica gel column chromatography to afford 3,6-dichloro-4-isopropylpyridazine (30 g, 47%) as a light yellow oil.

To a solution of 3,6-dichloro-4-isopropylpyridazine (20.00 g, 104.679 mmol, 1.0 eq) in DMSO (200 mL) were added phenol, 4-amino-2,6-dichloro- (18.63 g, 104.68 mmol, 1.0 eq), K₂CO₃ (58.16 g, 420.81 mmol, 4.02 eq) and CuI (11.96 g, 62.81mmol, 0.60 eq) in portions. The resulting mixture was stirred overnight at 90 °C under nitrogen atmosphere. The mixture was acidified to pH 8 with conc. HCl. The resulting mixture was extracted with EA (3x500 mL). The combined organic layers were washed with brine (3x300 mL), dried over anhydrous Na₂SO₄. After filtration, the filtrate was concentrated. The residue was purified by silica gel column chromatography to afford 3,5-dichloro-4-[(6-chloro-5-isopropyl-pyridazin-3-yl)oxy]-aniline (17 g, 49%) as a green solid.

To a solution of 3,5-dichloro-4-[(6-chloro-5-isopropylpyridazin-3-yl)oxy]aniline (17.00 g, 51.111 mmol, 1.0 eq) in HOAc (200 mL) was added NaOAc (24.33 g, 178.89 mmol, 3.50 eq) in portions. The mixture was stirred overnight at 100 °C under nitrogen atmosphere. The mixture was added NaOH (12.27 g, 306.77 mmol, 6.0 eq), and MeOH (200 mL) in portions. The resulting mixture was stirred overnight at 120 °C under nitrogen atmosphere. The resulting mixture was extracted with EA (3x500 mL). The combined organic layers were washed with brine (2x200 mL), dried over anhydrous Na₂SO₄. After filtration, the filtrate was concentrated. The residue was purified by silica gel column chromatography to afford 6-(4-amino-2,6-dichlorophenoxy)-4-isopropyl-2H-pyridazin-3-one (11 g, 69%) as a off-white solid.

### 13b. Synthesis of 3,5-dichloro-4-[(5-isopropyl-6-methoxypyridazin-3-yl)oxy]aniline

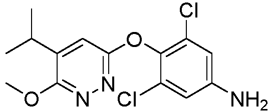

A mixture of 3,6-dichloro-4-isopropylpyridazine (80.0 g, 419 mmol, 1.00 eq), 4-amino-2,6-dichlorophenol (74.5 g, 419 mmol, 1.0 eq), K₂CO₃ (232 g, 1675 mmol, 4.0 eq) and CuI (47.9 g, 251 mmol, 0.6 eq) in DMSO (800 mL) was stirred overnight at 60 °C. After cooled down to 25 °C, the solution was then poured onto ice water (3 L) and the pH of the mixture was adjusted to 8 with hydrochloric acid (3 N). The resulting mixture was extracted with EA (3x1 L). The combined organic layers were washed with brine (3x1 L), dried over anhydrous Na₂SO₄. After filtration, the filtrate was concentrated under reduced pressure. The residue was purified by silica gel column chromatography (eluting with petroleum ether to ethyl acetate ratio (PE:EA) of 5:1) to afford 3,5-dichloro-4-[(6-chloro-5-isopropylpyridazin-3-yl)oxy]aniline (35.0 g, 24% yield) as an off-white solid. LCMS (ESI, *m*/*z):* 332 [M+H]⁺.

A mixture of 3,5-dichloro-4-[(6-chloro-5-isopropylpyridazin-3-yl)oxy]-aniline (10.0 g, 30.1 mmol, 1.00 eq) and potassium methoxide (21.1 g, 301 mmol, 10.0 eq) in MeOH (50 mL) was stirred for 48 h at 65°C. After cooled down to 25°C, the PH value of the mixture was adjusted to 6-7 with hydrochloric acid (1 N). The resulting mixture was extracted with EA (3x500 mL). The combined organic layers were dried over anhydrous Na₂SO₄, filtered and concentrated under reduced pressure. The residue was purified by silica gel column chromatography (eluting with PE:EA of 5:1) to afford 3,5-dichloro-4-[(5-isopropyl-6-methoxypyridazin-3-yl)oxy]aniline (6.80 g, 65% yield) as a white solid.

LCMS (ESI, *m*/*z):* 328 [M+H]⁺.

### 14a. Synthesis of 3,5-dichloro-4-[(5-isopropyl-6-oxo-1H-pyridazin-3-yl)oxy]phenylboronic acid

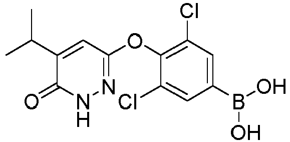

A 1000 mL round-bottom flask was charged with 6-(4-amino-2,6-dichlorophenoxy)-4-isopropyl-2H-pyridazin-3-one (14.13 g, 44.98 mmol, 1.0 eq) in MeOH (200 mL). HCl (4.10 mL, 112.439 mmol, 3 eq) was added dropwise at 0 °C. H₂O (100 mL) was added at 0 °C. The mixture was stirred for 10 min, then NaNO₂ (3.72 g, 53.971 mmol, 1.20 eq) was added, and was stirred for an additional 30 min at 0 °C. Tetrahydroxydiborane (40.32 g, 449.749 mmol, 10.0 eq) was added. The reaction was stirred for 1 h at 60 °C. The resulting solution was quenched with water (50 mL). The resulting solution was extracted with dichloromethane (3x400 mL) and the organic layers were combined, dried over anhydrous sodium sulfate, filtered and concentrated under reduced pressure. Purification resulted in 3,5-dichloro-4-[(5-isopropyl-6-oxo-1*H*-pyridazin-3-yl)oxy]phenylboronic acid (6.3 g, 37%)as a white solid.

### 14b. Synthesis of 3,5-dichloro-4-[(5-isopropyl-6-methoxypyridazin-3-yl)oxy]phenylboronic acid

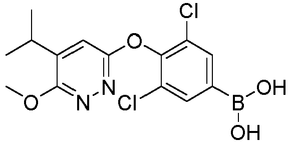

To a stirred mixture of 3,5-dichloro-4-[(5-isopropyl-6-methoxy-pyridazin-3-yl)oxy]aniline (5.80 g, 17.7 mmol, 1.00 eq) and CuBr₂ (7.89 g, 35.3 mmol, 2.00 eq) in ACN (40 mL) were added tert-butyl nitrite (4.24 mL, 35.4 mmol, 2.0 eq) dropwise at 0°C. The resulting mixture was then warmed up to room temperature and stirred for an additional 3 h. The resulting mixture was concentrated under reduced pressure. The residue was purified by silica gel column chromatography (eluting with PE:EA of 10:1) to afford 6-(4-bromo-2,6-dichlorophenoxy)-4-isopropyl-3-methoxypyridazine (3.50 g, 48% yield) as a yellow solid.

LCMS (ESI, *m*/*z):* 391[M+H]⁺.

A mixture of 6-(4-bromo-2,6-dichlorophenoxy)-4-isopropyl-3-meth-oxypyridazine (1.00 g, 2.55 mmol, 1.00 eq), bis(pinacolato)diboron (972 mg, 3.83 mmol, 1.50 eq), Pd(dppf)Cl₂ (187 mg, 0.256 mmol, 0.10 eq) and KOAc (750 mg, 7.64 mmol, 3.00 eq) in 1,4-dioxane (10 mL) was stirred for 2 h at 90 °C. After cooled down to 25 °C, the solids were filtered out and the filter cake was washed with EA (3x20 mL). The filtrate was concentrated under reduced pressure. The residue was purified by reverse phase chromatography (Column: C18 silica gel; Mobile phase, A: water (containing 0.5% TFA) and B: ACN (20% to 95% over 20 min) to afford 3,5-dichloro-4-[(5-isopropyl-6-methoxypyridazin-3-yl)oxy]phenylboronic acid (610 mg, 64% yield) as a brown solid.

LCMS (ESI, *m*/*z):* 359 [M+H]⁺.

### 15. Synthesis of 2-(3,5-dichloro-4-[[3-iodo-1-(4-methylbenzenesulfonyl)-indol-5-yl]oxy]phenyl)-3,5-dioxo-4H-1,2,4-triazine-6-carbonitrile

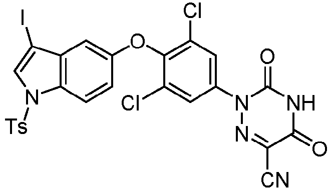

To a solution of 3,5-dichloro-4-[[1-(4-methylbenzenesulfonyl)indol-5-yl]oxy]aniline(15.00 g, 33.532 mmol, 1.0 eq) in water (700 mL), concentrated hydrochloric acid (336 mL) and acetic acid (942 mL) was added sodium nitrite (4.86 g, 70.418 mmol, 2.1 eq) in water (50 mL) dropwise at 0 °C. After the addition, the reaction was stirred at 0 °C for 45 min. Then the reaction mixture was poured into a solution of ethyl N-(2-cyanoacetyl)carbamate (7.85 g, 50.30 mmol, 1.5 eq) in water (600 mL) and pyridine (336 mL) at 0 °C quickly. The resulting mixture was stirred at 0 °C for 30 min and filtered. The filter cake was washed with water (100 mL) and petroleum ether (200 mL), dried under reduced pressure to provide ethyl (Z)-(2-cyano-2-(2-(3,5-dichloro-4-((1-tosyl-1H-indol-5-yl)oxy)phenyl)-hydrazineylidene)-acetyl) carbamate (18.4g, 80% yield) as red solid.

To a solution of ethyl (Z)-(2-cyano-2-(2-(3,5-dichloro-4-((1-tosyl-1*H*-indol-5-yl)oxy)phenyl)hydrazineylidene)acetyl)carbamate (18.40 g, 29.95 mmol, 1.0 eq) in N,N-dimethylacetamide (300 mL) was added potassium acetate (11.76 g, 119.782 mmol, 4.0 eq). The reaction was stirred at 120 °C for 2 h and quenched with water (100 mL). The mixture was extracted with ethyl acetate (3x100 mL) and the organic layers were combined, washed with brine (100 mL), dried over anhydrous sodium sulfate and concentrated under reduced pressure. The residue was purified to provide 2-(3,5-dichloro-4-[[1-(4-methylbenzenesulfonyl)indol-5-yl]oxy] phenyl)-3,5-dioxo-4*H*-1,2,4-triazine-6-carbonitrile (14g, 80% yield) as brown solid.

To a stirred solution of 2-(3,5-dichloro-4-[[1-(4-methylbenzene-sulfonyl)indol-5-yl]oxy]phenyl)-3,5-dioxo-4*H*-1,2,4-triazine-6-carbonitrile (7.0 g, 12.32 mmol, 1.0 eq) and p-toluenesulfonic acid (0.210 g, 1.232 mmol, 0.1 eq) in dichloromethane (300 mL ) was added N-iodosuccinimide (4.16 g, 18.47 mmol, 1.5 eq) at 0 °C under nitrogen atmosphere. The reaction mixture was stirred for 6 h at 0 °C and quenched with water (200 mL). The resulting mixture was extracted with dichloromethane (3x200 mL) and the combined organic layers were combined, washed with saturated sodium thiosulfate solution (2x500mL), dried over anhydrous sodium sulfate and concentrated under reduced pressure. The residue was purified to provide 2-(3,5-dichloro-4-[[3-iodo-1-(4-methylbenzene-sulfonyl)indol-5-yl]oxy]phenyl)-3,5-dioxo-4*H*-1,2,4-triazine-6-carbonitrile (5.30 g, 43% yield) as a brown solid.

### 16. Synthesis of 2-[3,5-dichloro-4-[(5-isopropyl-6-oxo-1H-pyridazin-3-yl)oxy]phenyl]-3,5-dioxo-4H-1,2,4-triazine-6-carbonitrile

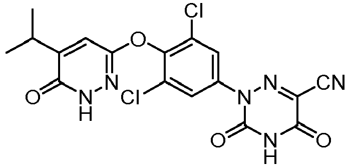

To a solution of 6-(4-amino-2,6-dichlorophenoxy)-4-isopropyl-2*H*-pyridazin-3-one (500 mg, 1.591 mmol, 1.0 eq) in HOAc (43.8 mL, 764.03 mmol) and HCl (15.3 mL, 503.55 mmol) were added NaNO₂ (230.59 mg, 3.34 mmol, 2.1 eq) in H₂O (33.4 mL) dropwise at 0 °C. The resulting mixture was stirred for 30 min at 0 °C under nitrogen atmosphere. The above solution was quickly poured into the second reaction flask which was placed ethyl N-(2-cyanoacetyl)carbamate (372.75 mg, 2.387 mmol, 1.5 eq) and H₂O (40.9 mL) in pyridine (20 mL) at 0 °C. The resulting mixture was for 30 min at 0 °C. The precipitated solids were collected by filtration and washed with PE and H₂O (3x300 mL). to afford ethyl N-[(Z)-cyano(2-[3,5-dichloro-4-[(5-isopropyl-6-oxo-1H-pyridazin-3-yl)oxy]phenyl]hydrazin-1-ylidene)-carbonyl]-carbamate, which can also be named ethyl (Z)-(2-cyano-2-(2-(3,5-dichloro-4-((5-isopropyl-6-oxo-1,6-dihydropyridazin-3-yl)oxy)phenyl)-hydrazineylidene)acetyl)-carbamate (name generated by Chemdraw) (640 mg, 84%) as an orange solid.

To a solution of ethyl N-[(Z)-cyano(2-[3,5-dichloro-4-[(5-isopropyl-6-oxo-1*H*-pyridazin-3-yl)oxy]phenyl]hydrazin-1-ylidene)carbonyl]carbamate, which can also be named ethyl (*Z*)-(2-cyano-2-(2-(3,5-dichloro-4-((5-isopropyl-6-oxo-1,6-dihydropyridazin-3-yl)oxy)phenyl)hydrazineylidene)acetyl)carbamate (640.00 mg, 1.330 mmol, 1.00 eq) in DMA (13.36 mL) was added KOAc (261.01 mg, 2.660 mmol, 2.00 eq) in portions at rt. The mixture was stirred for 3 h at 120 °C under nitrogen atmosphere. The reaction was quenched with Water. The resulting mixture was extracted with EA (3x100 mL). The combined organic layers were washed with brine (4x100 mL), dried over anhydrous Na₂SO₄. After filtration, the filtrate was concentrated. This resulted in 2-[3,5-dichloro-4-[(5-isopropyl-6-oxo-1*H-*pyridazin-3-yl)oxy]phenyl]-3,5-dioxo-4*H*-1,2,4-triazine-6-carbonitrile (590 mg, 102%) as a dark orange solid.

### 17. Synthesis of 4-[(benzyloxy)methyl]-6-bromo-2-[3,5-dichloro-4-[(5-isopropyl-6-oxo-1H-pyridazin-3-yl)oxy]-phenyl]-1,2,4-triazine-3,5-dione

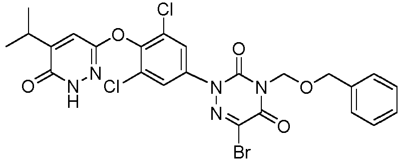

To a solution of 3,5-dichloro-4-[(5-isopropyl-6-oxo-1*H*-pyridazin-3-yl)oxy]phenylboronic acid (5.00 g, 11.663 mmol, 1.0 eq) in DCM (200 ml) stirred at rt was added 4-[(benzyloxy)methyl]-6-bromo-2*H*-1,2,4-triazine-3,5-dione (4.37 g, 13.995 mmol, 1.2 eq), Cu(OAc)₂ (4.24 g, 23.326 mmol, 2 eq) and pyridine (1.85 g, 23.326 mmol, 2 eq). The reaction mixture was stirred for 2 days at rt under oxygen, and was then concentrated under reduced pressure to afford the crude product. Purification resulted in 4-[(benzyloxy)methyl]-6-bromo-2-[3,5-dichloro-4-[(5-isopropyl-6-oxo-1*H*-pyridazin-3-yl)oxy]-phenyl]-1,2,4-triazine-3,5-dione (2.2g, yield 28%) as an off-white solid.

### 18. Synthesis of (E)-N'-[3,5-dichloro-4-([3-iodo-1H-pyrrolo[3,2-b]pyridin-5-yl]oxy)phenyl]-N,N-dimethylmethanimidamide

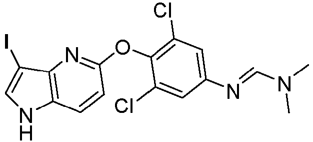

A 8 mL vial was charged with 6-chloro-2-methyl-3-nitropyridine (20.00 g, 115.895 mmol, 1.0 eq), phenol, 4-amino-2,6-dichloro- (24.76 g, 139.07 mmol, 1.2 eq), K₂CO₃ (48.05 g, 347.69 mmol, 3.0 eq) and DMF (200 mL). The resulting mixture was stirred overnight at 60 °C. The reaction mixture was quenched with water (400 mL). The resulting mixture was extracted with EA (3x500 mL) and the organic layers were combined, washed with brine (2x400 mL), dried over anhydrous sodium sulfate, filtered and concentrated under reduced pressure to afford crude product. The crude product was purified by silica gel column chromatography and eluted with EA:PE (0% ~ 40% over 30 min) to provide the desired product 3,5-dichloro-4-[(6-methyl-5-nitropyridin-2-yl)oxy]aniline (36.4 g, 91%) as a yellow solid.

LCMS (ESI, *m*/*z):* 314 [M+H]⁺.

To a solution of 3,5-dichloro-4-[(6-methyl-5-nitropyridin-2-yl)oxy]-aniline (36.40 g, 115.88 mmol, 1.0 eq) and (dimethoxymethyl)dimethylamine (33.14 g, 278.1 mmol, 2.4 eq) in dimethylformamide (400 mL) was added TEA (11.73 g, 115.9 mmol, 1.0 eq). The resulting mixture was stirred for 4 h at 100 °C. The mixture was cooled to 60 °C and concentrated under reduced pressure to remove half of DMF. The resulting solution was passed to the next step without further treatment.

A solution of (E)-N'-(3,5-dichloro-4-((6-((E)-2-(dimethylamino)vinyl)-5-nitropyridin-2-yl)oxy)phenyl)-N,N-dimethylformimidamide (49.17 g, 115.89 mmol, 1.0 eq) in DMF (200 mL) and MeOH (400 mL) was added Pd/C (10.0 g, 93.967 mmol, 0.81 eq) and NaOAc (9.51 g, 115.89 mmol, 1.0 eq) under hydrogen. The resulting mixture was stirred overnight at room temperature. The mixture was filtered through a celite pad and washed with MeOH (50 mL). The filtrate was concentrated under reduced pressure to afford crude product. The crude product was purified by silica gel column chromatography and eluted with EA:PE (0% ~ 70% over 30 min) to provide the desired product (E)-N'-(3,5-dichloro-4-[1*H-*pyrrolo[3,2-b]pyridin-5-yloxy]phenyl)-N,N-dimethylmethanimidamide (21.5 g, 45%) as a brown solid.

LCMS (ESI, *m*/*z):* 349 [M+H]⁺.

A solution of (E)-N'-(3,5-dichloro-4-[1*H*-pyrrolo[3,2-b]pyridin-5-yloxy]phenyl)-N,N-dimethylmethanimidamide (5.00 g, 14.32 mmol, 1.0 eq) and I₂ (4.00 g, 15.75 mmol, 1.1 eq) in dimethylformamide (50 mL) was added KOH (3.21 g, 57.27 mmol, 4.0 eq). The resulting mixture was stirred overnight at room temperature and quenched with water (80 mL). The resulting mixture was extracted with EA (3x100 mL) and the organic layers were combined, washed with brine (2x80 mL), dried over anhydrous sodium sulfate, filtered and concentrated under reduced pressure to afford crude product. The crude product was triturated with PE:EA of 5:1 to provide the desired product (E)-N'-[3,5-dichloro-4-([3-iodo-1*H*-pyrrolo[3,2-b]pyridin-5-yl]oxy)phenyl]-N,N-dimethylmethanimidamide (5.9 g, 78%) as a yellow solid.

LCMS (ESI, *m*/*z):* 475 [M+H]⁺.

### 19. Synthesis of 3,5-dichloro-4-([3-isopropyl-1H-pyrrolo[3,2-b]pyridin-5-yl]oxy)aniline

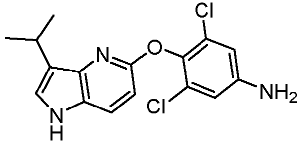

To a solution of (E)-N'-[3,5-dichloro-4-([3-iodo-1*H*-pyrrolo[3,2-b]pyridin-5-yl]oxy)phenyl]-N,N-dimethylmethanimidamide (3.0 g, 6.31 mmol, 1.0 eq), K₃PO₄ (2.01 g, 9.471 mmol, 1.5 eq) and 1,1'-Bis (di-t-butylphosphino)ferrocene palladium dichloride (0.410 g, 0.631 mmol, 0.10 eq) in dioxane (75 mL) and H₂O (15 mL) was added 4,4,5,5-tetramethyl-2-(prop-1-en-2-yl)-1,3,2-dioxaborolane (4.24 g, 25.26 mmol, 4.0 eq) under nitrogen. The resulting mixture was stirred overnight at 60 °C and quenched with water (100 mL). The resulting mixture was extracted with EA (3x150 mL) and the organic layers were combined, washed with brine (2x100 mL), dried over anhydrous sodium sulfate, filtered and concentrated under reduced pressure to afford crude product. The crude product was purified by column and eluted with EA:PE (0% ~ 50% over 30 min) to provide the desired product (E)-N'-(3,5-dichloro-4-[[3-(prop-1-en-2-yl)-1*H*-pyrrolo[3,2-b]pyridin-5-yl]oxy]phenyl)-N,N-dimethylmethanimidamide (430 mg, 14%) as a yellow solid.

LCMS (ESI, *m*/*z):* 389 [M+H]⁺.

To a solution of (E)-N'-(3,5-dichloro-4-[[3-(prop-1-en-2-yl)-1*H*-pyrrolo[3,2-b]pyridin-5-yl]oxy]phenyl)-N,N-dimethylmethanimidamide (400.0 mg, 1.028 mmol, 1.0 eq) in MeOH (25mL) was added Pd/C (250.0 mg, 2.349 mmol, 2.3 eq). The resulting mixture was stirred for 1 h at room temperature under hydrogen atmosphere. The mixture was filtered through a celite pad and washed with MeOH (20 mL). The combined organic layers were concentrated under reduced pressure to afford crude product (E)-N'-[3,5-dichloro-4-([3-isopropyl-1*H*-pyrrolo[3,2-b]pyridin-5-yl]-oxy)phenyl]-N,N-dimethylmethanimidamide (380 mg, crude) as a yellow solid.

LCMS (ESI, *m*/*z):* 391 [M+H]⁺.

To a solution of (E)-N'-[3,5-dichloro-4-([3-isopropyl-1*H*-pyrrolo[3,2-b]pyridin-5-yl]oxy)phenyl]-N,N-dimethylmethanimidamide (380.0 mg, 0.97 mmol, 1.0 eq) in ethyl alcohol (25 mL) was added ethylenediamine (262.6 mg, 4.37 mmol, 4.5 eq). The resulting mixture was stirred refluxed overnight. The reaction mixture was concentrated under reduced pressure to afford the crude product 3,5-dichloro-4-([3-isopropyl-1*H-*pyrrolo[3,2-b]pyridin-5-yl]oxy)aniline (350 mg, crude) as a yellow-semi solid.

LCMS (ESI, *m*/*z):* 336 [M+H]⁺.

### 20. Synthesis of 4-[(benzyloxy)methyl]-6-[3,5-dichloro-4-[(5-isopropyl-6-methoxypyridazin-3-yl)oxy]phenyl]-2H-1,2,4-triazine-3,5-dione

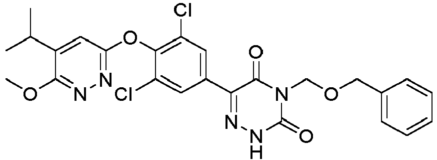

A mixture of 3,5-dichloro-4-[(5-isopropyl-6-methoxypyridazin-3-yl)oxy]phenylboronic acid (400 mg, 1.12 mmol, 1.0 eq), 4-[(benzyloxy)methyl]-6-bromo-2*H*-1,2,4-triazine-3,5-dione (398 mg, 1.28 mmol, 1.15 eq), PdAmphos(Cl)₂ (79.3 mg, 0.112 mmol, 0.1 eq) and K₃PO₄ (713 mg, 3.36 mmol, 3.0 eq) in 1,4-dioxane (8 mL)/water (0.8 mL) was stirred for 2 h at 90 °C. After cooled down to 25 °C, the solids were filtered out and the filter cake was washed with EA (3x15 mL). The filtrate was concentrated under reduced pressure. The residue was purified by silica gel column chromatography (eluting with PE:EA of 1:1) to afford 4-[(benzyl-oxy)methyl]-6-[3,5-dichloro-4-[(5-isopropyl-6-methoxypyridazin-3-yl)oxy]-phenyl]-2*H*-1,2,4-triazine-3,5-dione (190 mg, 30% yield) as a light yellow solid.

LCMS (ESI, *m*/*z):* 544 [M+H]⁺.

### 21. Procedures for the synthesis of 2-(4-(benzyloxy)-2,6-dimethylbenzyl)-4,4,5,5-tetramethyl-1,3,2-dioxaborolane:

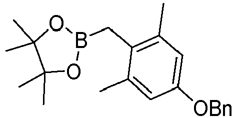

To a solution of 5-benzyloxy-2-bromo-1,3-dimethyl-benzene (1 g, 3.43 mmol) and 4,4,5,5-tetramethyl-2-[(4,4,5,5-tetramethyl-1,3,2-dioxaborolan-2-yl)meth-yl]-1,3,2-dioxaborolane (1.84 g, 6.87 mmol) in dioxane (10 mL) was added 8 N aq. KOH (858.57 µL, 6.86 mmol), followed by addition of Pd[P(*t*-Bu)₃]₂ (87.75 mg, 171.71 µmol) at rt (~15 °C) under N₂ protection. Then the mixture was stirred at 30°C for 18 h. TLC (PE:EA of 10:1, stained by I₂) showed the bromide was consumed completely and two new spots formed. The mixture was diluted with water (40 mL), extracted with ethyl acetate (50x2 mL). The combined organic phases were dried over anhydrous Na₂SO₄, filtered and concentrated in vacuo to give crude product, which was purified by flash silica gel chromatography (Eluent of 0~2% Ethyl acetate/Petroleum ether) to give desired 2-(4-(benzyloxy)-2,6-dimethylbenzyl)-4,4,5,5-tetramethyl-1,3,2-dioxa-borolane (870 mg, 72% yield) as a white solid.

### 22. Synthesis of [4-[[3-isopropyl-1-(p-tolylsulfonyl)pyrrolo[3,2-b]pyridin-5-yl]methyl]-3,5-dimethyl-phenyl] trifluoromethanesulfonate

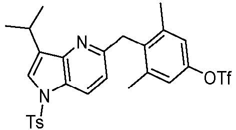

To a solution of 5-bromo-3-isopropyl-1-(p-tolylsulfonyl)pyrrolo[3,2-b]pyridine (1.0 g, 2.54 mmol, 1 eq) and 2-[(4-benzyloxy-2,6-dimethyl-phenyl)methyl]-4,4,5,5-tetramethyl-1,3,2-dioxaborolane (1.79 g, 5.09 mmol, 2 eq) in dioxane (30 mL) and H₂O (6 mL) was added dichloropalladium;tris-o-tolylphosphane (299.79 mg, 381.39 µmol, 0.15 eq) and K₃PO₄ (1.62 g, 7.63 mmol, 3 eq) at 20 °C under N₂ protection. Then the resulting mixture was stirred at 100 °C for 15 h under N₂ atmosphere. The combined mixture (combined with 300 mg batch) was diluted with H₂O (100 mL) and extracted with EA (100 mLx3). The combined organic layers were washed with brine (100 mL), dried over anhydrous Na₂SO₄, filtered and concentrated under reduced pressure to give a residue, which was purified by flash silica gel chromatography (Ethyl acetate in Petroleum ether = 0~10%) to give 5-[(4-benzyloxy-2,6-dimethyl-phenyl)methyl]-3-isopropyl-1-(p-tolylsulfonyl)pyrrolo[3,2-b]pyridine (1.36 g, 2.44 mmol, 74% yield, 96.5% purity) as a yellow solid.

To a solution of 5-[(4-benzyloxy-2,6-dimethyl-phenyl)methyl]-3-isopropyl-1-(p-tolylsulfonyl)pyrrolo[3,2-b]pyridine (1.36 g, 2.52 mmol, 1 eq) in MeOH (20 mL) and THF (10 mL) was added Pd-C (10%, 1.07 g) under N₂. The suspension was degassed under vacuum and purged with H₂ several times. The mixture was stirred under H₂ (50 psi) at 35°C for 18 hours. The mixture was filtered and the filtrate was concentrated, purified by flash silica gel chromatography (0~100% Ethyl acetate in Petroleum ether) to give 4-[[3-isopropyl-1-(p-tolylsulfonyl)pyrrolo[3,2-b]pyridin-5-yl]methyl]-3,5-dimethyl-phenol (1 g, 2.23 mmol, 88% yield) as a white solid.

To a solution of 4-[[3-isopropyl-1-(p-tolylsulfonyl)pyrrolo[3,2-b]pyridin-5-yl]methyl]-3,5-dimethyl-phenol (1 g, 2.23 mmol, 1 eq) and pyridine (440.84 mg, 5.57 mmol, 2.5 eq) in DCM (25 mL) at 0 °C was added dropwise Tf₂O (754.76 mg, 2.68 mmol, 441.38 µL, 1.2 eq) slowly. After the addition, the mixture was stirred at 0 °C for 1 h. The reaction mixture was partitioned between H₂O (30 mL) and DCM (30 mL). The organic phase was separated, washed with brine (10 mL * 3), dried over anhydrous MgSO₄, filtered and concentrated to give crude product, which was purified by flash silica gel chromatography (0~10% Ethyl acetate in Petroleum ether) to give [4-[[3-isopropyl-1-(p-tolylsulfonyl)pyrrolo[3,2-b]pyridin-5-yl]methyl]-3,5-dimethyl-phenyl] trifluoromethanesulfonate (1.25 g, 2.15 mmol, 97% yield) as a colorless gum.

### 23. Synthesis of 4-[[3-isopropyl-1-(p-tolylsulfonyl)pyrrolo[3,2-b]pyridin-5-yl]methyl]-3,5-dimethyl-aniline:

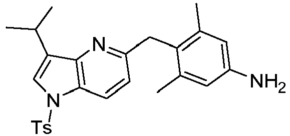

To a solution of [4-[[3-isopropyl-1-(p-tolylsulfonyl)pyrrolo[3,2-b]pyridin-5-yl]methyl]-3,5-dimethyl-phenyl] trifluoromethanesulfonate (300 mg, 516.67 µmol, 1 eq) and tert-butyl carbamate (121.05 mg, 1.03 mmol, 2 eq) in dioxane (8 mL) was added Pd₂(dba)₃ (47.31 mg, 51.67 µmol, 0.1 eq), (5-diphenylphosphanyl-9,9-dimethyl-xanthen-4-yl)-diphenyl-phosphane (59.79 mg, 103.33 µmol, 0.2 eq) and Cs₂CO₃ (505.03 mg, 1.55 mmol, 3 eq) at 20 °C under N₂ protection. Then the resulting mixture was stirred at 100 °C for 15 h under N₂. LCMS showed the reaction was complete. The mixture was diluted with H₂O (50 mL) and extracted with EA (50x3 mL). The combined organic layers were washed with brine (100 mL), dried over anhydrous Na₂SO₄, filtered and concentrated under reduced pressure to give a residue. The residue was purified by flash silica gel chromatography (Ethyl acetate in Petroleum ether = 0~10%) to give tert-butyl N-[4-[[3-isopropyl-1-(p-tolylsulfonyl)pyrrolo[3,2-b]pyridin-5-yl]methyl]-3,5-dimethyl-phenyl] carbamate (200 mg, 350.19 µmol, 68% yield, 95.9% purity) as a light yellow solid.

To a solution of tert-butyl N-[4-[[3-isopropyl-1-(p-tolylsulfonyl)-pyrrolo[3,2-b]pyridin-5-yl]methyl]-3,5-dimethyl-phenyl] carbamate (170 mg, 310.39 µmol, 1 eq) in MeOH (3 mL) was added 4 M HCl gas in MeOH (8 mL) at 20 °C. Then the resulting mixture was stirred at 20 °C for 12 h. MeOH was removed under reduced pressure and the residue was partitioned between EA (50 mL) and H₂O (50 mL). The EA phase was washed with H₂O (50 mL) again. The combined aqueous layers were adjusted to pH 7~8 with sat. aq. NaHCO₃, then extracted with EA (50 mLx2). The combined organic layers were washed with brine (50 mL), dried over anhydrous Na₂SO₄, filtered and concentrated to give 4-[[3-isopropyl-1-(p-tolylsulfonyl)-pyrrolo[3,2-b]pyridin-5-yl]methyl]-3,5-dimethyl-aniline (120 mg, 261.40 µmol, 84% yield, 97.5% purity) as a light yellow solid.

### 24. Synthesis of 2,2,2-trifluoro-N-(4-formyl-3,5-dimethylphenyl)acetamide

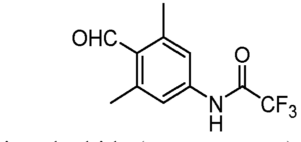

Trifluoroacetic anhydride (2 eq, 5.63 mL) was added to a solution of 4-iodo-3,5-dimethylaniline (1 eq, 5 g, 20.24 mmol) in anhydrous DCM (150 mL) under N₂ at 0 °C. Then, the reaction mixture was stirred at 25 °C for 2.5 hours. To the reaction mixture was added water (200 mL), the layers were separated, and the aqueous layer was extracted with DCM (2x150 mL). The combined organic layers were dried over MgSO₄, filtered and evaporated to dryness. The crude mixture was purified by flash chromatography on silica gel (DCM) to give 2,2,2-trifluoro-N-(4-iodo-3,5-dimethylphenyl)acetamide (6.35 g, 91%) as a white solid.

Anhydrous THF (35 mL) was added to a mixture of 2,2,2-trifluoro-N-(4-iodo-3,5-dimethylphenyl)acetamide (1 eq, 2.40 g, 7 mmol) and NaH 60% (1.5 eq, 420 mg) under N₂ at 0 °C. The reaction mixture was stirred at 25 °C for 1.5 h, then cooled to -78 °C and t-BuLi (2.4 eq, 9.88 mL) was added dropwise. The reaction mixture was stirred at -78 °C for 40 min. Then, anhydrous DMF (5 eq, 2.71 mL) was added and the reaction was further stirred at -78 °C for 1 h. The reaction mixture was hydrolyzed with sat. aq. NH₄Cl (5 mL), poured in DCM (250 mL) and washed 4 times with brine. The combined organic layers were dried over MgSO₄, filtered and evaporated to dryness. The crude mixture was recrystallized from hot EtOH to give 2,2,2-trifluoro-N-(4-formyl-3,5-dimethylphenyl)acetamide (317 mg, 19%) as a yellow solid. The supernatant was recrystallized from hot toluene to give more 2,2,2-trifluoro-*N*-(4-formyl-3,5-dimethylphenyl)acetamide (909 mg, 53%) as a light yellow solid.

### 25. Synthesis of 3,5-dimethyl-4-((3-pentyl-1H-indazol-5-yl)methyl)aniline

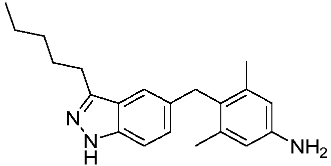

Anhydrous THF (8.3 mL) was added to a mixture of 5-bromo-3-pentyl-1*H-*indazole (1 eq, 668 mg, 2.5 mmol) and NaH 60% (1.5 eq, 150 mg) at 0 °C, under N₂. The resulting reaction mixture was stirred at 25 °C for 1 h. Then, the mixture was cooled to -78 °C and *n*-BuLi (1.5 eq, 2.34 mL) was added dropwise and the reaction mixture was stirred at -78 °C for 2 h. Then, a solution of 2,2,2-trifluoro-*N*-(4-formyl-3,5-dimethylphenyl)acetamide (1 eq, 613 mg) in anhydrous THF (8.3 mL) was added dropwise to the reaction mixture at - 78 °C. The reaction was further stirred at this temperature for 1.75 h and finally hydrolyzed with sat. aq. NH₄Cl (5 mL), poured in DCM (100 mL) and washed 3 times with water. The organic phase was dried over MgSO₄, filtered and evaporated to dryness. The crude mixture was purified by flash chromatography on silica gel (20% to 40% EA in Cyclohexane) to give 2,2,2-trifluoro-N-(4-(hydroxy(3-pentyl-1*H*-indazol-5-yl)methyl)-3,5-dimethylphenyl)acetamide (317 mg, 29%) as a white solid.

A solution of Et₃SiH (6 eq, 0.71 mL) in anhydrous DCM (20 mL) followed by a solution of TMSOTf (0.075 eq, 0.01 mL) in anhydrous DCM (7 mL) were added dropwise to a solution of 2,2,2-trifluoro-N-(4-(hydroxy(3-pentyl-1*H*-indazol-5-yl)methyl)-3,5-dimethylphenyl)acetamide (1 eq, 317 mg, 0.73 mmol) in anhydrous DCM (7 mL) at 0 °C under N₂. The reaction was stirred at 0 °C for 1h, at which point the ice bath was removed and the reaction was stirred at 25 °C. After 19h, extra Et₃SiH (6 eq, 0.71 mL) and TMSOTf (0.075 eq, 0.01 mL) were added and the reaction mixture was further stirred at 25 °C for 5h. Then, the reaction was quenched with sat. aq. NaHCO₃ (20 mL) and the aqueous phase was extracted 3 times with DCM (3x50 mL). The combined organic layers were washed with brine (150 mL), dried over MgSO₄, filtered and evaporated to dryness. The crude mixture was purified by flash chromatography on silica gel (0% to 30% EA in Cyclohexane) to give N-(3,5-dimethyl-4-((3-pentyl-1*H*-indazol-5-yl)methyl)phenyl)-2,2,2-trifluoroacetamide (144 mg, 47%) as a white solid.

NaOH (4 eq, 54 mg) was added to a solution of N-(3,5-di-methyl-4-((3-pentyl-1H-indazol-5-yl)methyl)phenyl)-2,2,2-trifluoroacetamide (1 eq, 141 mg, 0.34 mmol) in MeOH (7 mL) and water (1.4 mL) under N₂. The reaction mixture was stirred at 60 °C for 90 h. Then, the reaction mixture was quenched with water (30 mL). The resulting solution was extracted with DCM (3x20 mL) and the combined organic layers were washed with brine (2x20 mL), dried over MgSO₄, filtered and evaporated to dryness to give 3,5-dimethyl-4-((3-pentyl-1H-indazol-5-yl)methyl)aniline (105 mg, 97%) as a white solid, which was used without further purification.

### Example 1: Synthesis of compound 1

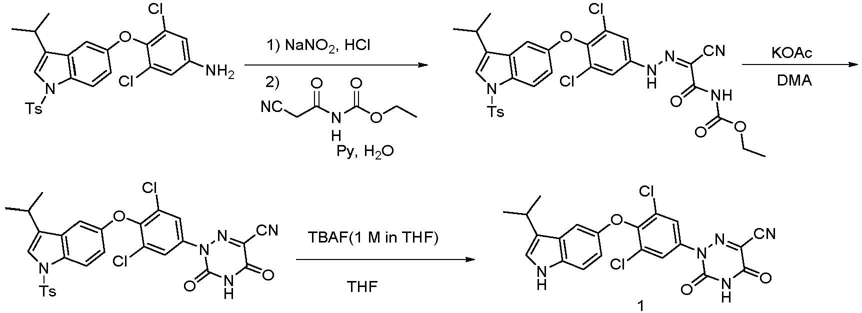

To a stirred solution of 3,5-dichloro-4-[[3-isopropyl-1-(4-methylbenzene-sulfonyl)indol-5-yl]methyl]aniline (1.11 g, 2.277 mmol, 1.00 eq) in HCl (21.90 mL), HOAc (62.50 mL) and H₂O (47.68 mL) was added NaNO₂ (0.32 g, 4.566 mmol, 2 eq) in H₂O (4.2 mL) dropwise for 1 h at 0 °C. The second reaction flask was placed ethyl N-(2-cyanoacetyl)carbamate (0.53 g, 3.424 mmol, 1.5 eq) in H₂O (58.41 mL) and Pyridine (22.35 mL) for 10 min at 0 °C. The above solution was quickly poured into the second reaction flask for 30 min at 0 °C. The precipitated solids were collected by filtration and washed with water and PE. The resulting mixture was concentrated under vacuum. This resulted in ethyl N-[(Z)-cyano[2-(3,5-dichloro-4-[[3-isopropyl-1-(4-methylbenzene-sulfonyl)indol-5-yl]methyl]phenyl)-hydrazin-1-ylidene]carbonyl]carbamate, which can also be named ethyl (*Z*)-(2-cyano-2-(2-(3,5-dichloro-4-((3-isopropyl-1-tosyl-1H-indol-5-yl)oxy)phenyl)hydrazineylidene)acetyl)carbamate as a red solid.

To a stirred solution of ethyl N-[(Z)-cyano[2-(3,5-dichloro-4-[[3-isopropyl-1-(4-methylbenzenesulfonyl)indol-5-yl]-oxy]phenyl)hydrazin-1-ylidene]-carbonyl]carbamate, which can also be named ethyl (Z)-(2-cyano-2-(2-(3,5-dichloro-4-((3-isopropyl-1-tosyl-1*H-*indol-5-yl)oxy)phenyl)hydrazineylidene)acetyl)carbamate (1.16 g, 1.773 mmol, 1.00 eq) in DMA (20.00 mL) was added KOAc (0.35 g, 3.546 mmol, 2 eq) in portions at rt. The resulting mixture was stirred for 2 h at 120 °C under nitrogen atmosphere. The reaction was quenched by the addition of water (10 mL) at rt. The precipitated solids were collected by filtration and washed with water (3x10 mL). The solid was dried overnight at rt to afford 2-(3,5-dichloro-4-[[3-isopropyl-1-(4-methylbenzenesulfonyl)indol-5-yl]oxy]-phenyl)-3,5-dioxo-4*H*-1,2,4-triazine-6-carbonitrile (521.2 mg, 45%) as a red solid.

To a stirred solution of 2-(3,5-dichloro-4-[[3-isopropyl-1-(4-methyl-benzenesulfonyl)indol-5-yl]oxy]phenyl)-3,5-dioxo-4*H*-1,2,4-triazine-6-carbonitrile (521.20 mg, 0.854 mmol, 1.00 eq) in THF (20.00 mL) was added TBAF (20 mL) in portions at rt under nitrogen atmosphere. The resulting mixture was stirred for 20 h at 65 °C under nitrogen atmosphere. The reaction was quenched with H₂O.The resulting mixture was extracted with EA, washed with brine, dried over anhydrous Na₂SO₄.After filtration, the filtrate was concentrated under reduced pressure. The residue was purified by Prep-TLC (DCM:MeOH 16:1) to afford the crude product (200 mg). The crude product (200 mg) was purified by prep-HPLC to afford 2-[3,5-dichloro-4-[(3-isopropyl-1*H*-indol-5-yl)oxy]-phenyl]-3,5-dioxo-4*H-*1,2,4-tri-azine-6-carbonitrile (11.5 mg, 3%, compound 1) as a yellow solid.

¹H-NMR: (300 MHz, DMSO-*d*₆) δ ppm: 10.67 - 10.60 (m, 1H), 7.77 (s, 2H), 7.30 (d, J = 8.7 Hz, 1H), 7.07 (s, 1H), 6.82 (d, J = 2.5 Hz, 1H), 6.67 (dd, J = 8.9, 2.5 Hz, 1H), 2.94 (p, J = 7.0 Hz, 1H), 1.18 (d, J = 6.8 Hz, 6H).

### Example 2: Synthesis of compound 2

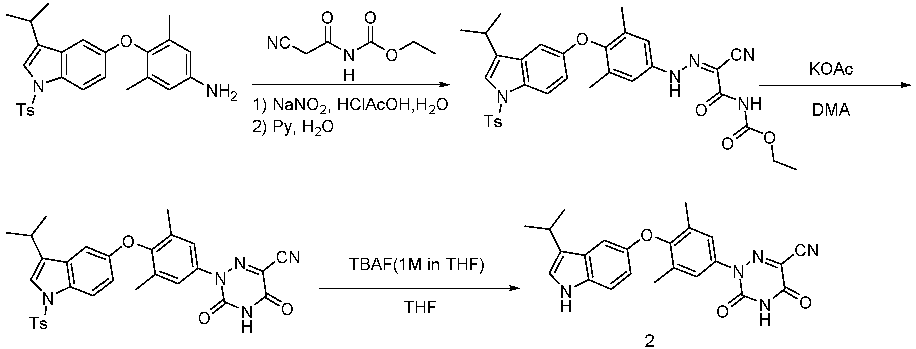

A 100 mL 3-necked round-bottom flask, maintained with an inert atmosphere of nitrogen, was charged with 4-[[3-isopropyl-1-(4-methylbenzene-sulfonyl)indol-5-yl]oxy]-3,5-dimethyl-aniline (450.00 mg, 1.003 mmol, 1.00 eq) in con. HCl (9.60 mL), AcOH (27.40 mL) and H₂O (20.9 mL).To a stirred solution was added NaNO₂ (145.35 mg, 2.107 mmol, 2.1 eq) in H₂O (1.8mL) and dropwise for 45 min at 0 °C. The second reaction flask was placed methyl N-(2-cyanoacetyl)carbamate (213.85 mg, 1.505 mmol, 1.50 eq) in H₂O (25.6 mL) and pyridine (9.80 mL) for 10 min at 0 °C. The above solution was quickly poured into the second reaction flask for 30 min at 0 °^{C}. The precipitated solids were collected by filtration and washed with water and PE. The resulting mixture was concentrated under vacuum. This resulted in ethyl N-[(Z)-cyano[2-(4-[[3-isopropyl-1-(4-methylbenzenesulfonyl)indol-5-yl]oxy]-3,5-dimethylphenyl)hydrazin-1-ylidene]-carbonyl]carbamate, which can also be named ethyl (Z)-(2-cyano-2-(2-(4-((3-isopropyl-1-tosyl-1*H*-indol-5-yl)oxy)-3,5-dimethylphenyl)hydrazineylidene)acetyl)carbamate as a red solid.

To a stirred solution of ethyl N-[(Z)-cyano[2-(4-[[3-isopropyl-1-(4-methyl-benzenesulfonyl)indol-5-yl]oxy]-3,5-dimethylphenyl)hydrazin-1-ylidene]-carbonyl]carbamate, which can also be named ethyl (Z)-(2-cyano-2-(2-(4-((3-isopropyl-1-tosyl-1*H*-indol-5-yl)oxy)-3,5-dimethylphenyl)hydrazineylidene)acetyl)carbamate (name generated by Chemdraw) (560.0 mg, 0.910 mmol, 1.00 eq) in DMA (10.00 mL) was added KOAc (178.52 mg, 1.819 mmol, 2.0 eq) in portions at rt. The resulting mixture was stirred for 2 h at 120 °C under nitrogen atmosphere. The reaction was quenched by the addition of water (10 mL) at rt. The resulting mixture was extracted with EA (3x100 mL). The combined organic layers were washed with brine (100 mL), dried over anhydrous Na₂SO₄. After filtration, the filtrate was concentrated under reduced pressure. The residue was purified by silica gel column chromatography to afford 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile (350 mg, 56%) as a red solid.

To a stirred solution of 2-(4-[[3-isopropyl-1-(4-methylbenzene-sulfonyl)-indol-5-yl]oxy]-3,5-dimethylphenyl)-3,5-dioxo-4*H*-1,2,4-tri-azine-6-carbo-nitrile (330.00 mg, 1 eq) in THF was added TBAF(1 M in THF) (8.00 mL) at rt. The resulting mixture was stirred for 48 h at 65 °C. The resulting mixture was diluted with water (100 mL).The resulting mixture was extracted with EA (3x100 mL). The combined organic layers were washed with brine (100 mL), dried over anhydrous Na₂SO₄. After filtration, the filtrate was concentrated under reduced pressure. Purification afforded 2-[4-[(3-isopropyl-1*H*-indol-5-yl)oxy]-3,5-dimethyl-phenyl]-3,5-dioxo-4*H*-1,2,4- triazine-6-carbonitrile (132.3 mg, 54%) as a yellow solid.

¹H-NMR: (300 MHz, DMSO-d₆) δ ppm: 10.66 (s, 1H), 7.29 - 7.20 (m, 3H), 7.04 (d, *J* = 2.2 Hz, 1H), 6.77 (d, *J* = 2.4 Hz, 1H), 6.60 (dd, *J* = 8.8, 2.5 Hz, 1H), 2.94 (p, *J* = 6.9 Hz, 1H), 2.10 (s, 6H), 1.19 (d, *J* = 6.8 Hz, 6H).

### Example 3: Synthesis of compound 3

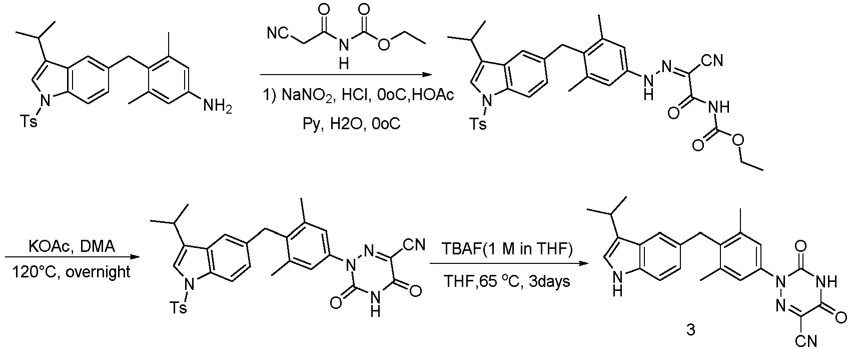

The first reaction flask, to a solution of 4-[[3-isopropyl-1-(4-methyl-benzenesulfonyl)indol-5-yl]methyl]-3,5-dimethylaniline (480.00 mg, 1.075 mmol, 1.00 eq) in concentrated HCl (9.60 mL), HOAc (28.80 mL) and H₂O (22.4mL) stirred under nitrogen at 0 °C was added NaNO₂ (155.72 mg, 2.257 mmol, 2.1 eq) in H₂O (22.4 mL). The reaction mixture was stirred for 45 min at 0 °C. The second reaction flask, a solution of ethyl N-(2-cyanoacetyl)carbamate (251.72 mg, 1.612 mmol, 1.50 eq) in H₂O (27.2mL) and pyridine (9.60 mL) was stirred at 0 °C for 10 min. The first reaction flask was quickly poured into the second reaction flask, the reaction mixture stirred at 0 °C for 30 min. The precipitated solids were collected by filtration and washed with water (3x50 mL) and PE (3x50 mL). The resulting mixture was concentrated under vacuum. The crude product was isolated as a yellow solid, 520 mg, 80% pure, 63% yield.

To a solution of ethyl N-[(Z)-cyano[2-(4-[[3-isopropyl-1-(4-methyl-benzenesulfonyl)indol-5-yl]methyl]-3, 5-dimethylphenyl)hydrazin-1-ylidene]-carbonyl]carbamate, which can also be named ethyl (Z)-(2-cyano-2-(2-(4-((3-isopropyl-1-tosyl-1*H-*indol-5-yl)methyl)-3,5-dimethylphenyl)hydrazineylidene)acetyl)carbamate (350.00 mg, 0.570 mmol, 1.0 eq) in DMA (8.0 mL) stirred at rt was added KOAc (111.94 mg, 1.141 mmol, 2.0 eq). The reaction mixture was stirred at 120 °C for 3 h. The reaction was quenched by addition of water (10 mL) at rt. The precipitation solids were collected by filtration and washed with water (3x10 mL). the solids were dried in a vacuum over 2 h at 60 °C to afford 2-(4-[[3-isopropyl-1-(4-methylbenzene-sulfonyl)indol-5-yl]methyl]-3,5-dimethylphenyl)-3,5-dioxo-4*H*-1,2,4-triazine-6-carbonitrile. The desired product was isolated as a dark orange solid, 250 mg, 80% pure, 62% yield.

To a solution of 2-(4-[[3-isopropyl-1-(4-methylbenzenesulfonyl)indol-5-yl]methyl]-3,5-dimethylphenyl)-3,5-dioxo-4*H*-1,2,4-triazine-6-carbonitrile (200.00 mg, 0.352 mmol, 1.00 eq) in THF (5.00 mL) stirred under nitrogen at 0 °C was added TBAF (10.57 mL, 10.570 mmol, 30.00 eq, 1 M in THF). The reaction mixture was stirred at 65 °C for 3 days. The reaction mixture was concentrated under reduced pressure to afford the crude product. The sample was purified to afford 2-(4-((3-isopropyl-1*H*-indol-5-yl)methyl)-3,5-dimethylphenyl)-3,5-dioxo-2,3,4,5-tetrahydro-1,2,4-triazine-6-carbo-nitrile. as a yellow solid (compound 3, 29.7 mg, 99% pure, 20% yield).

¹H NMR (400 MHz, DMSO-d₆) δ ppm: 13.00 (br, 1H), 10.63 (s, 1H), 7.19 - 7.22 (m, 2H), 7.14 (s, 2H), 7.01 (d, *J* = 2.0 Hz, 1H), 6.68 - 6.71 (m, 1H), 4.11 (s, 2H), 3.00 - 3.04 (m, 1H), 2.27 (s, 6H), 1.24 - 1.25 (m, 6H).

### Example 4: Synthesis of compound 4

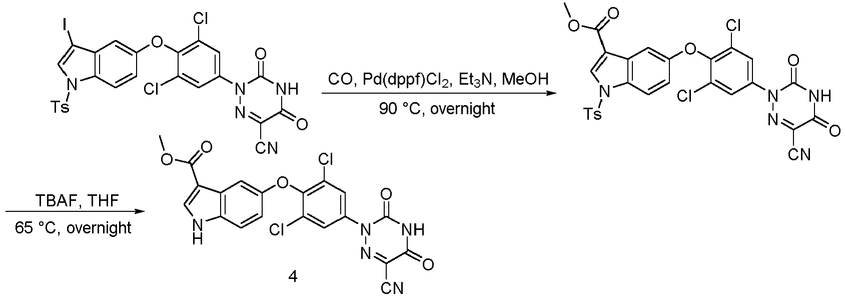

A 50 mL autoclave was charged with 2-(3,5-dichloro-4-[[3-iodo-1-(4-methylbenzenesulfonyl)indol-5-yl]oxy]phenyl)-3,5-dioxo-4*H*-1,2,4-triazine-6-carbo-nitrile (1.00 g, 1.44 mmol, 1.00 eq), dichloro[1,1'-bis(diphenylphosphino)-ferrocene]-palladium(II) (0.11 g, 0.144 mmol, 0.10 eq), triethylamine (0.44 g, 4.32 mmol, 3.00 eq), methanol (10 mL). The contents of the autoclave were placed under an atmosphere of carbon monoxide (30 atm). The reaction was stirred overnight at 90 °C. The catalysts were filtered out. The filtrate was concentrated under reduced pressure. The residue was purified to provide 500 mg (yield 67%) of methyl 5-[2,6-dichloro-4-(6-cyano-3,5-dioxo-4*H*-1,2,4-triazin-2-yl)phenoxy]-1-(4-methylbenzene-sulfonyl)-indole-3-carboxylate as a brown solid.

A 40 mL vial was charged with methyl 5-[2,6-dichloro-4-(6-cyano-3,5-dioxo-4*H*- 1,22,4-triazin-2-yl)phenoxy]-1-(4-methylbenzenesulfonyl)indole-3-carboxyl-ate (400 mg, 0.639 mmol, 1.00 eq), tetrahydrofuran (5 mL), tetrabutylammonium fluoride (6.39 mL, 1 M in tetrahydrofuran, 6.39 mmol, 10.0 eq). The reaction was stirred overnight at 65 °C and quenched with water (10 mL). The resulting solution was extracted with dichloromethane (3x20 mL) and the organic layers were combined, washed with brine (2x10 mL), dried over anhydrous sodium sulfate, filtered and concentrated under reduced pressure. The residue was purified to provide 30.4 mg (yield 15%) of methyl 5-[2,6-dichloro-4-(6-cyano-3,5-dioxo-4*H-*1,2,4-triazin-2-yl)phenoxy]-1*H*-indole-3-carboxylate as a yellow solid.

¹H NMR (300 MHz, Methanol-d₄) δ ppm: 7.97 (s, 1H), 7.80 (s, 2H), 7.41 - 7.44 (m, 2H), 6.88 - 6.92 (m, 1H), 3.84 (s, 3H).

### Example 5: Synthesis of compound 5

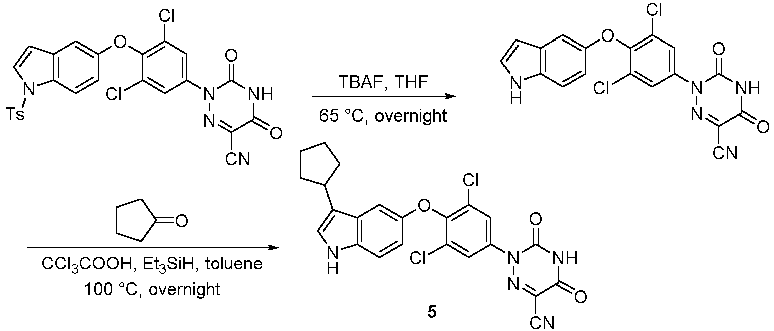

A 250mL round-bottom was charged with 2-(3,5-dichloro-4-[[1-(4-methylbenzenesulfonyl)indol-5-yl]oxy]phenyl)-3,5-dioxo-4*H*-1,2,4-triazine-6-carbo-nitrile (1.00 g, 1.759 mmol, 1.00 eq), tetrahydrofuran (20 mL), tetrabutylammonium fluoride (52.78 mL, 1 M in tetrahydrofuran, 52.78 mmol, 30.00 eq). The resulting solution stirred overnight at 65 °C and then quenched with water (150 mL). The resulting mixture was extracted with ethyl acetate (3x200 mL) and the organic layers were combined, washed with brine (3x200 mL), dried over anhydrous sodium sulfate, filtered and concentrated under reduced pressure. The residue was purified to provide 500 mg (69% yield) of 2-[3,5-dichloro-4-(1*H*-indol-5-yloxy)phenyl]-3,5-dioxo-4*H*-1,2,4-triazine-6-carbonitrile as a yellow solid.

To a solution of 2-[3,5-dichloro-4-(1*H*-indol-5-yloxy)phenyl]-3,5-dioxo-4*H-*1,2,4-triazine-6-carbonitrile (300 mg, 0.724 mmol, 1.00 eq), trichloroacetic acid (355 mg, 2.17 mmol, 3.00 eq) and triethylsilane (337 mg, 2.90 mmol, 4.0 eq) in toluene (10 mL) stirred under nitrogen at 100 °C was added cyclopentanone (366 mg, 4.35 mmol, 6.00 eq). The reaction mixture stirred overnight at 100 °C and concentrated under reduced pressure. Purification resulting in 20.2 mg (6% yield) of 2-[3,5-dichloro-4-[(3-cyclopentyl-1*H*-indol-5-yl)oxy]phenyl]-3,5-dioxo-4*H*-1,2,4-tri-azine-6-carbonitrile as a yellow solid.

¹H NMR (300 MHz, DMSO-d₆) δ ppm: 10.77 (s, 1H), 7.81 (s, 2H), 7.30 - 7.34 (m, 1H), 7.13 (d, *J* = 1.8 Hz, 1H), 6.90 (d, *J* = 2.4 Hz, 1H), 6.64 - 6.68 (m, 1H), 3.06 - 3.14 (m, 1H), 1.99 - 2.03 (m, 2H), 1.60 - 1.78 (m, 6H).

### Example 6: Synthesis of compound 6

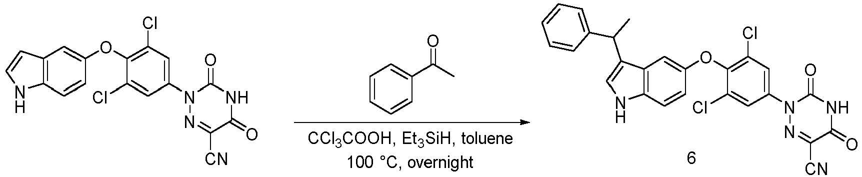

A 50 mL round-bottom flask was charged with 2-[3,5-dichloro-4-(1*H*-indol-5-yloxy)phenyl]-3,5-dioxo-4*H*-1,2,4-triazine-6-carbonitrile (300 mg, 0.724 mmol, 1.00 eq), trichloroacetic acid (355 mg, 2.17 mmol, 3.00 eq), toluene (10.00 mL), triethylsilane (504.10 mg, 4.35 mmol, 6.00 eq) under nitrogen. acetophenone (261 mg, 2.17 mmol, 3.00 eq) was added at 100 °C. The resulting solution was stirred overnight at 100 °C and concentrated under reduced pressure. The residue was dissolved in water (20 mL) and extracted with dichloromethane (3x50 mL). The organic layers were combined, washed with brine (2x10 mL), dried over anhydrous sodium sulfate, filtered and concentrated under reduced pressure. Purification resulted in 63.2 mg (17% yield) of 2-(3,5-dichloro-4-[[3-(1-phenylethyl)-1*H-*indol-5-yl]oxy]phenyl)-3,5-dioxo-4*H*-1,2,4-triazine-6-carbonitrile as a yellow solid.

¹H NMR (300 MHz, DMSO-d₆) δ ppm: 10.88 (s, 1H), 7.75 (s, 2H), 7.21 - 7.29 (m, 2H), 7.13 - 7.21 (m, 4H), 7.06 - 7.11 (m, 1H), 6.63 - 6.67 (m, 1H), 6.38 - 6.39 (m, 1H), 4.11 - 4.18 (m, 1H), 1.56 - 1.59 (m, 3H).

### Example 7: Synthesis of compound 7

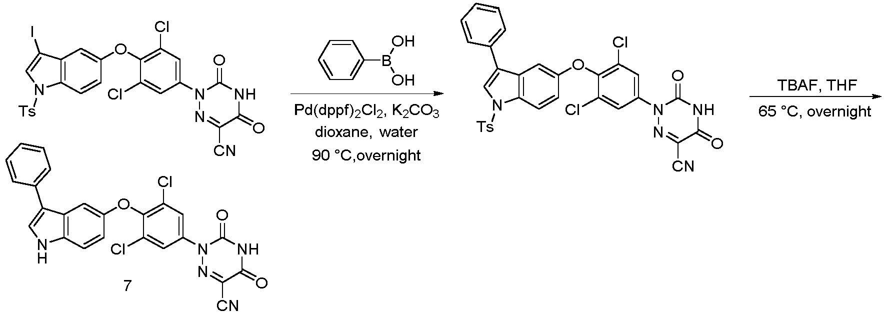

To a stirred solution of 2-(3,5-dichloro-4-[[3-iodo-1-(4-methylbenzene-sulfonyl)indol-5-yl]oxy]phenyl)-3,5-dioxo-4*H*-1,2,4-triazine-6-carbo-nitrile (400 mg, 0.576 mmol, 1.00 eq) and phenyl boronic acid (91.3 mg, 0.749 mmol, 1.30 eq) in dioxane (4 mL) and water (0.8 mL) was added dichloro[1,1'-bis(diphenylphosphino)-ferrocene]palladium(II) (42.2 mg, 0.0580 mmol, 0.10 eq) and potassium carbonate (238.88 mg, 1.728 mmol, 3.00 eq) at rt under nitrogen atmosphere. The reaction mixture was stirred overnight at 90 °C and concentrated under reduced pressure. The residue was purified to provide 170 mg (42% yield) of 2-(3,5-dichloro-4-[[1-(4-methyl-benzene-sulfonyl)-3-phenylindol-5-yl]oxy]phenyl)-3,5-dioxo-4H-1,2,4-triazine-6-carbonitrile as a light brown solid.

To a stirred solution of 2-(3,5-dichloro-4-[[1-(4-methylbenzenesulfon-yl)-3-phenylindol-5-yl]oxy]phenyl)-3,5-dioxo-4*H*-1,2,4-triazine-6-carbonitrile (160 mg, 0.248 mmol, 1.00 eq) in tetrahydrofuran (4 mL) was added t-butyl-ammonium fluoride (4.96 mL, 1 M in tetrahydrofuran, 4.96 mmol, 20.00 eq) at rt under nitrogen atmosphere. Then the reaction mixture was stirred overnight at 65 °C and quenched with water (10 mL).The resulting mixture was extracted with ethyl acetate (3x10 mL) and the combined organic layers were combined, washed with brine (3x20 mL), dried over anhydrous sodium sulfate and concentrated under reduced pressure. Purification resulted in of 2-[3,5-dichloro-4-[(3-phenyl-1*H*-indol-5-yl)oxy]phenyl]-3,5-dioxo-4*H*-1,2,4-triazine-6-carbonitrile (compound 7, 39 mg, 32%) as a light orange solid.

¹H NMR (300 MHz, Methanol-*d*₄) δ ppm: 7.78 (s, 2H), 7.50 - 7.56 (m, 2H), 7.49 (s, 1H), 7.35 - 7.41(m, 3H), 7.18 - 7.23 (m, 2H), 681 - 6.84(m, 1H).

### Example 8: Synthesis of compound 8

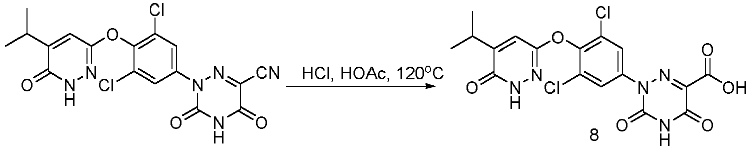

To a solution of 2-[3,5-dichloro-4-[(5-isopropyl-6-oxo-1*H*-pyridazin-3-yl)oxy]phenyl]-3,5-dioxo-4*H*-1,2,4-triazine-6-carbonitrile (200.00 mg, 0.460 mmol, 1.00 eq) in HOAc (4.43 mL, 0.074 mmol, 0.16 eq) was added HCl (2.00 mL, 0.055 mmol, 0.12 eq) in portions at rt. The resulting mixture was stirred overnight at 120 °C under nitrogen atmosphere. The crude product (160 mg) was purified to afford 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid (78.3 mg, 38%) as an off-white solid.

¹H NMR (400 MHz, DMSO-d₆): δ ppm: 13.82 (s, 1H), 12.70 (s, 1H), 12.19 (s, 1H), 7.82 (s, 2H), 7.44 (s, 1H), 3.05 (p, J = 6.8 Hz, 1H), 1.20 (d, J = 6.9 Hz, 6H).

### Example 9: Synthesis of compound 9

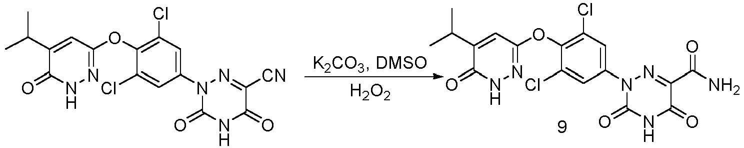

To a solution of 2-[3,5-dichloro-4-[(5-isopropyl-6-oxo-1*H*-pyridazin-3-yl)oxy]phenyl]-3,5-dioxo-4*H*-1,2,4-triazine-6-carbonitrile (200.00 mg, 0.460 mmol, 1.00 eq) in DMSO (5 mL) was added K₂CO₃ (190.53 mg, 1.379 mmol, 3.00 eq) and H₂O₂ (2 mL, 30% in water) in portions at 0 °C. The resulting mixture was stirred for 5 h at rt. The reaction was quenched with saturated Na₂S₂O₃ solution at rt and concentrated under reduced pressure. The residue was purified by reverse flash chromatography with the following conditions (column, C18; mobile phase, ACN in water, 10% to 80% gradient in 30 min) to provide the crude product (150 mg), which was then purified by Prep-HPLC (Column: XBridge Prep OBD C18 Column, Mobile Phase A: Water (10 mM NH₄HCO₃), Mobile Phase B: ACN; Flow rate: 60 mL/min; Gradient: 20% B to 33% B in 9 min;) to afford 2-[3,5-dichloro-4-[(5-isopropyl-6-oxo-1*H*-pyridazin-3-yl)oxy]phenyl]-3,5-dioxo-4*H*-1,2,4-triazine-6-carboxamide (112.1 mg, 54%) as a white solid.

¹H NMR (400 MHz, DMSO-d₆) δ ppm: 12.76(s, 1H), 12.20 (s, 1H), 8.14(br, 1H), 7.85-7.90(m, 3H), 7.44 (s, 1H), 3.05 (p, J =6.8 Hz, 1H), 1.20 (d, J = 6.8Hz, 6H).

### Example 10: Synthesis of compound 10

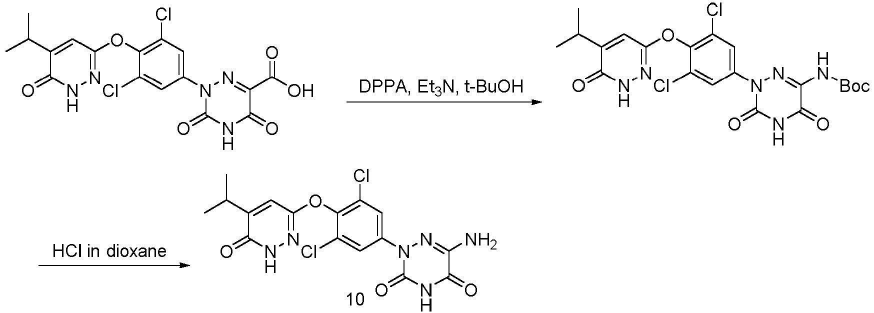

To a solution of 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid (200.00 mg, 0.440 mmol, 1.00 eq) in t-BuOH (20.00 mL, 210.465 mmol) were added DPPA (375.64 mg, 1.365 mmol, 3.10 eq) and Et₃N (138.12 mg, 1.365 mmol, 3.10 eq) in portions at rt. The resulting mixture was stirred for 24 h at 85 °C under nitrogen atmosphere. The resulting mixture was concentrated. The residue was dissolved in CH₂Cl₂ (100 mL), and was then washed with NH₄Cl (4x200 mL), NaHCO₃ and brine. The combined organic layers were dried over anhydrous Na₂SO₄. After filtration, the filtrate was concentrated. The residue was purified by silica gel column chromatography, eluted with CH₂Cl₂:MeOH (50:1), to afford tert-butyl N-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate (110 mg, 48%) as a white solid.

LCMS (ESI, *m*/*z):* 525 [M+H]⁺.

A solution of t-butyl N-(2-[3,5-dichloro-4-[(5-isopropyl-6-oxo-1*H*-pyridazin-3-yl)oxy]phenyl]-3,5-dioxo-4*H*-1,2,4-triazin-6-yl)carbamate (90.0 mg, 1 eq) in 4 N HCl in 1,4-dioxane (5.00 mL) was stirred overnight at rt. The resulting mixture was concentrated. The crude product (70 mg) was purified by Prep-HPLC (Column: XBridge Prep OBD C18, Mobile Phase A:Water (10 mmol/L NH₄HCO₃+0.1%NH₃.H₂O), Mobile Phase B:ACN; Flow rate:60 mL/min; Gradient:10 B to 40 B in 8 min) to afford 6-amino-2-[3,5-dichloro-4-[(5-isopropyl-6-oxo-1*H*-pyridazin-3-yl)oxy]phenyl]-4*H*-1,2,4-tri-azine-3,5-dione (18.8 mg, 26%) as a white solid.

¹H NMR (400 MHz, DMSO-d₆) δ ppm: 12.26 (s, 1H), 12.18 (s, 1H), 7.85 (s, 2H), 7.41 (s, 1H), 6.45 (s, 2H), 2.95-3.10 (m, 1H), 1.19 (d, J = 6.9 Hz, 6H).

### Example 11: Synthesis of compound 11

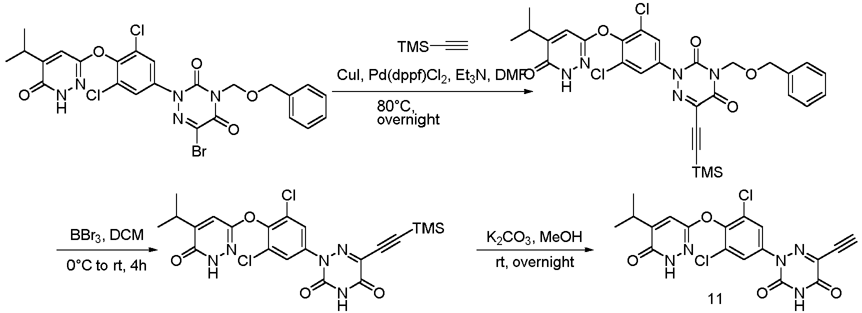

To a stirred solution of 4-[(benzyloxy)methyl]-6-bromo-2-[3,5-dichloro-4-[(5-isopropyl-6-oxo-1*H*-pyridazin-3-yl)oxy]phenyl]-1,2,4-triazine-3,5-di-one (430.00 mg, 0.706 mmol, 1.00 eq) and trimethylsilylacetylene (207.96 mg, 2.117 mmol, 3.0 eq) in DMF (10 mL) were added CuI (26.88 mg, 0.141 mmol, 0.20 eq), Et₃N (214.25 mg, 2.117 mmol, 3.00 eq) and Pd(dppf)Cl₂ (103.28 mg, 0.141 mmol, 0.20 eq) in portions at rt under nitrogen atmosphere. The reaction mixture was stirred overnight at 80 °C and quenched with water (10 mL). The resulting mixture was extracted with EA (3x40 mL). The combined organic layers were washed with water (1x100 mL), dried over anhydrous Na₂SO₄. After filtration, the filtrate was concentrated under reduced pressure. The residue was purified by silica gel column chromatography to afford 4-[(benzyloxy)methyl]-2-[3,5-dichloro-4-[(5-isopropyl-6-oxo-1*H*-pyridazin-3-yl)oxy]phenyl]-6-[2-(trimethylsilyl)ethynyl]-1,2,4-triazine-3,5-dione (260 mg, 52%) as a off-white solid.

To a stirred solution of 4-[(benzyloxy)methyl]-2-[3,5-dichloro-4-[(5-isopropyl-6-oxo-1*H*-pyridazin-3-yl)oxy]phenyl]-6-[2-(trimethylsilyl)ethynyl]-1,2,4-triazine-3,5-dione (240.00 mg, 0.383 mmol, 1.00 eq) in DCM (5 mL) was added BBr3 (383.84 mg, 1.532 mmol, 4.00 eq) dropwise at 0 °C under nitrogen atmosphere. The reaction was stirred for 4 h at rt and quenched with MeOH (5 mL).The resulting mixture was concentrated under reduced pressure to provide 100mg (crude) of 2-[3,5-dichloro-4-[(5-isopropyl-6-oxo-1*H-*pyridazin-3-yl)oxy]phenyl]-6-[2-(trimethylsilyl)-ethynyl]-4*H*-1,2,4-triazine-3,5-dione as light brown solid.

To a solution of 2-[3,5-dichloro-4-[(5-isopropyl-6-oxo-1*H*-pyridazin-3-yl)oxy]phenyl]-6-[2-(trimethylsilyl)ethynyl]-4*H-*1,2,4-triazine-3,5-dione (100.00 mg, 0.197 mmol, 1.00 eq) in MeOH (10 mL) was added K₂CO₃ (109.16 mg, 0.790 mmol, 4.00 eq) stirred at rt. The reaction mixture was stirred overnight at rt and concentrated under reduced pressure to afford the residue. Purification resulted in 19.4 mg (yield 22%) of 2-[3,5-dichloro-4-[(5-isopropyl-6-oxo-1*H*-pyridazin-3-yl)oxy]phenyl]-6-ethynyl-4*H*-1,2,4-triazine-3,5-dione as a white solid.

¹H NMR (400 MHz, DMSO-*d*₆) δ ppm: 12.75 (s, 1H), 12.23 (s, 1H), 7.77 (s, 2H), 7.44 (s, 1H), 4.70 (s, 1H) ,3.03 - 3.10 (m, 1H), 1.20 (d, J = 6.8 Hz, 6H)

### Example 12: Synthesis of compound 12

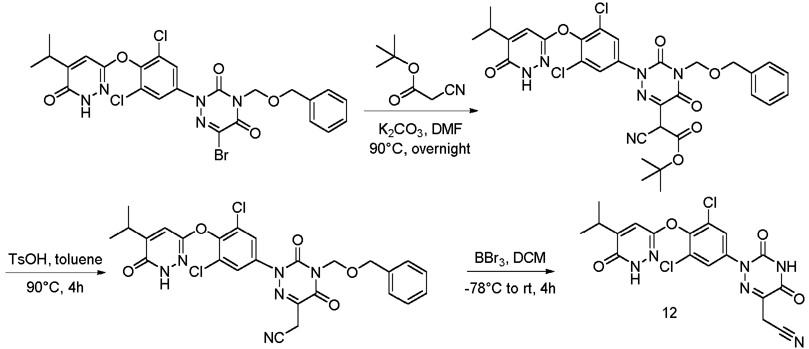

To a solution of 4-[(benzyloxy)methyl]-6-bromo-2-[3,5-dichloro-4-[(5-isopropyl-6-oxo-1*H*-pyridazin-3-yl)oxy]phenyl]-1,2,4-triazine-3,5-dione (300.00 mg, 0.492 mmol, 1.00 eq) and tert-butyl 2-cyanoacetate (208.54 mg, 1.477 mmol, 3.0 eq) in DMF (5.00 mg) stirred at rt was added K₂CO₃ (272.21 mg, 1.970 mmol, 4.0 eq). The reaction mixture was stirred overnight at 90 °C. The reaction mixture was quenched with water (30 mL). The resulting mixture was extracted with dichloromethane (3x50 mL) and the organic layers were combined, washed with brine (2x50 mL), dried over anhydrous sodium sulfate, filtered and concentrated under reduced pressure. The residue was purified to provide 190mg(95% pure) tert-butyl2-[4-[(benzyloxy)methyl]-2-[3,5-dichloro-4-[(5-isopropyl-6-oxo-1*H*-pyridazin-3-yl)oxy]phenyl]-3,5-dioxo-1,2,4-triazin-6-yl]-2-cyanoacetate (190 mg, 58%) as a yellow solid.

A 50 mL round-bottom flask was charged with tert-butyl 2-[4-[(benzyloxy)methyl]-2-[3,5-dichloro-4-[(5-isopropyl-6-oxo-1*H*-pyridazin-3-yl)oxy]-phenyl]-3,5-dioxo-1,2,4-triazin-6-yl]-2-cyanoacetate (190.00 mg, 0.284 mmol, 1.00 eq), TsOH (24.43 mg, 0.142 mmol, 0.5 eq) and toluene (5 mL). The resulting solution was stirred for 4 h at 90 °C and quenched with water (10 mL). The resulting mixture was extracted with dichloromethane (3x50 mL) and the organic layers were combined, washed with brine (2x30 mL), dried over anhydrous sodium sulfate, filtered and concentrated under reduced pressure to provide 190 mg of crude (70% pure) 2-[4-[(benzyloxy)methyl]-2-[3,5-dichloro-4-[(5-isopropyl-6-oxo-1*H*-pyridazin-3-yl)oxy]-phenyl]-3,5-dioxo-1,2,4-triazin-6-yl]acetonitrile (190 mg, 82% yield) as a yellow oil.

To a solution of 2-[4-[(benzyloxy)methyl]-2-[3,5-dichloro-4-[(5-isopropyl-6-oxo-1*H*-pyridazin-3-yl)oxy]phenyl]-3,5-dioxo-1,2,4-triazin-6-yl]aceto-nitrile (190.00 mg, 0.334 mmol, 1.00 eq) in DCM (3 mL) stirred under nitrogen at -78°C was added BBr₃ (501.57 mg, 2.002 mmol, 6.0 eq). The reaction mixture was stirred at rt for 4 h. The reaction mixture was quenched with water (10 mL). The resulting solution was extracted with dichloromethane (3x20 mL) and the organic layers were combined, washed with brine (2x10 mL), dried over anhydrous sodium sulfate, filtered and concentrated under reduced pressure to afford the crude product. Purification resulted in 24.9 mg (16% yield) of 2-(2-[3,5-dichloro-4-[(5-isopropyl-6-oxo-1*H*-pyridazin-3-yl)oxy]phenyl]-3,5-dioxo-4*H*-1,2,4-triazin-6-yl)acetonitrile as a white solid.

¹H NMR (400 MHz, DMSO-*d*₆) δ ppm: 12.71 (s, 1H), 12.20 (s, 1H), 7.81 (s, 2H), 7.46 (s, 1H), 4.05 (s, 2H), 3.03 - 3.07 (m, 1H), 1.11 - 1.31 (m, 6H).

### Example 13: Synthesis of compound 13

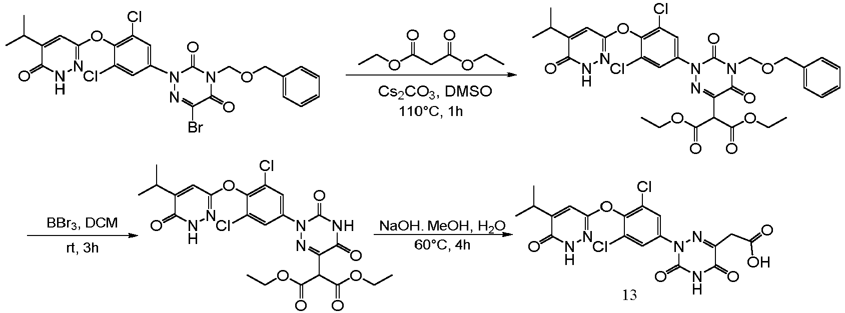

To a solution of 4-[(benzyloxy)methyl]-6-bromo-2-[3,5-dichloro-4-[(5-isopropyl-6-oxo-1*H*-pyridazin-3-yl)oxy]phenyl]-1,2,4-triazine-3,5-dione (350.00 mg, 0.574 mmol, 1.00 eq) and diethyl malonate (184.02 mg, 1.149 mmol, 2.00 eq) in DMSO (5 mL) stirred under nitrogen at rt was added Cs₂CO₃ (561.52 mg, 1.723 mmol, 3.0 eq). The reaction mixture was stirred at 110 °C for 1 h. The reaction mixture was quenched with water (20 mL).The resulting solution was extracted with dichloromethane (3x50 mL) and the organic layers were combined, washed with brine (2x30 mL), dried over anhydrous sodium sulfate, filtered and concentrated under reduced pressure to afford crude product. The crude product was diluted with dichloromethane (50mL) and made the slurry with 100 ~ 200 silica gel mesh (2 g). The sample was purified and the desired product was isolated as a yellow oil. 270 mg, 90% pure, 61% yield.

To a solution of 1,3-diethyl 2-[4-[(benzyloxy)methyl]-2-[3,5-dichloro-4-[(5-isopropyl-6-oxo-1*H*-pyridazin-3-yl)oxy]phenyl]-3,5-dioxo-1,2,4-triazin-6-yl]-propanedioate (250.00 mg, 0.363 mmol, 1.00 eq) in DCM (5.00 mL) stirred under nitrogen at 0 °C was added BBr₃ (363.86 mg, 1.452 mmol, 4.00 eq). The reaction mixture was stirred at rt for 3 h. The reaction mixture was quenched with water (15 mL). The resulting solution was extracted with dichloromethane (3x30 mL) and the organic layers were combined, washed with brine (2x15 mL), dried over anhydrous sodium sulfate, filtered and concentrated under reduced pressure to afford crude product. No further purification was carried out on this material. The crude product was isolated as a yellow solid, 210 mg, 70% pure, 71% yield.

To a solution of 1,3-diethyl 2-(2-[3,5-dichloro-4-[(5-isopropyl-6-oxo-1*H-*pyridazin-3-yl)oxy]phenyl]-3,5-dioxo-4*H*-1,2,4-triazin-6-yl)propanedioate (210.00 mg, 0.369 mmol, 1.00 eq) in MeOH (5.00 mL) and H₂O (1.00 mL) stirred at rt was added NaOH (147.78 mg, 3.695 mmol, 10.00 eq). The reaction mixture was stirred at 60 °C for 4 h. The pH value of the mixture was adjusted to ~5-6 with hydrochloric acid (1 M). The resulting solution was extracted with dichloromethane (3x30 mL) and the organic layers were combined, washed with brine (2x10 mL) dried over anhydrous sodium sulfate, filtered and concentrated under reduced pressure to afford crude product. The crude product was purified to provide 2-(2-(3,5-dichloro-4-((5-isopropyl-6-oxo-1,6-dihydropyridazin-3-yl)oxy)phenyl)-3,5-dioxo-2,3,4,5-tetra-hydro-1,2,4-triazin-6-yl)acetic acid. The desired product was isolated as a white solid (54.0 mg, 99.5% pure, 31% yield)

¹H NMR (300 MHz, DMSO-*d*₆) δ ppm: 7.82 (s, 1H), 7.44 (s, 1H), 3.44 (s, 2H), 3.01 - 3.10 (m, 1H), 1.19 - 1.24 (m, 6H).

### Example 14: Synthesis of compound 14

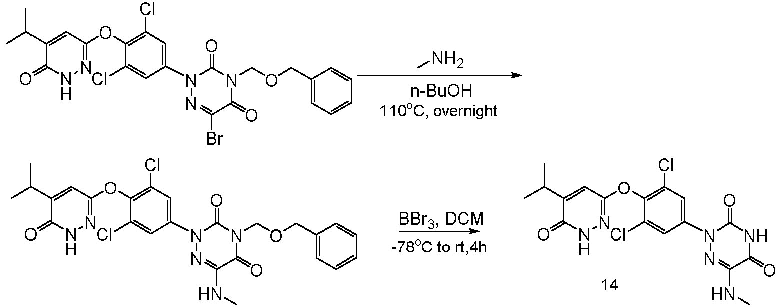

To a stirred solution of 4-[(benzyloxy)methyl]-6-bromo-2-[3,5-dichloro-4-[(5-isopropyl-6-oxo-1*H*-pyridazin-3-yl)oxy]phenyl]-1,2,4-triazine-3,5-di-one (400.00 mg, 0.657 mmol, 1.00 eq) in n-BuOH (5 mL) was added methylamine (3.3 mL, 6.565 mmol, 10.00 eq, 2 M in THF) at rt. The reaction mixture was stirred overnight at 110 °C. The reaction mixture was concentrated under reduced pressure to afford the residue. The residue was purified to provide 220mg (yield 53%) of4-[(benzyloxy)methyl]-2-[3,5-dichloro-4-[(5-isopropyl-6-oxo-1*H*-pyridazin-3-yl)oxy]-phenyl]-6-(dimethylamino)-1,2,4-triazine-3,5-dione as a light yield solid.

To a stirred solution of 4-[(benzyloxy)methyl]-2-[3,5-dichloro-4-[(5-isopropyl-6-oxo-1*H*-pyridazin-3-yl)oxy]phenyl]-6-(methylamino)-1,2,4-triazine-3,5-dione (400.00 mg, 0.715 mmol, 1.00 eq) in DCM was added BBr₃ (716.55 mg, 2.860 mmol, 4.00 eq) at -78 °C. The resulting solution was stirred for 4 h at rt. The reaction mixture was concentrated under reduced pressure to afford the crude product. Purification resulted in 86.9 mg (yield 54%) 2-[3,5-dichloro-4-[(5-isopropyl-6-oxo-1*H*-pyridazin-3-yl)oxy]phenyl]-6-(methylamino)-4H-1,2,4-triazine-3,5-dione as a white solid.

¹H NMR (300 MHz, DMSO-*d*₆) δ ppm: 12.99 (s, 1H), 12.23 (s, 1H), 8.82 (d, J = 4.8 Hz, 1H), 8.16 (s, 2H), 7.42 (s, 1H), 3.03 - 3.08 (m, 1H), 2.79 (d, J = 4.8 Hz, 3H), 1.20 (d, J = 6.9 Hz, 6H).

### Example 15: Synthesis compound 15

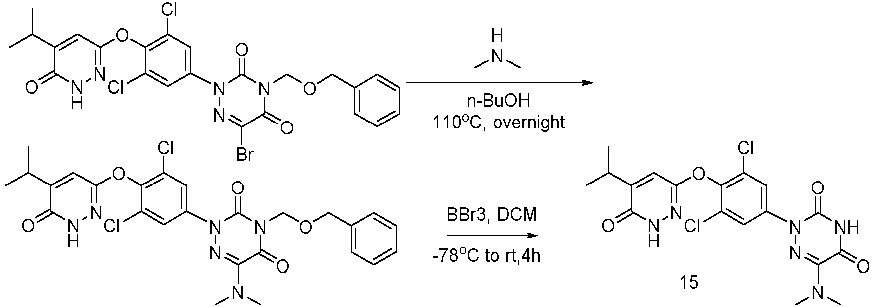

To a 50 mL round-bottom flask were added 4-[(benzyloxy)methyl]-6-bromo-2-[3,5-dichloro-4-[(5-isopropyl-6-oxo-1*H*-pyridazin-3-yl)oxy]phenyl]-1,2,4-triazine-3,5-dione (400.00 mg, 0.657 mmol, 1.00 eq) in n-BuOH (8 mL) and dimethylamine (3.3 mL, 6.565 mmol, 10.00 eq, 2 M in THF) at rt. The reaction mixture was stirred overnight at 110 °C. The reaction mixture was concentrated under reduced pressure to afford the residue. The residue was purified to provide 170 mg (yield 41%) of 4-[(benzyloxy)methyl]-2-[3,5-dichloro-4-[(5-isopropyl-6-oxo-1*H*-pyridazin-3-yl)-oxy]phenyl]-6-(dimethylamino)-1,2,4-triazine-3,5-dione as a light pink solid.

A 50 mL round bottom flask was charged with 4-[(benzyloxy)methyl]-2-[3,5-dichloro-4-[(5-isopropyl-6-oxo-1*H*-pyridazin-3-yl)oxy]phenyl]-6-(dimethyl-amino)-1,2,4-triazine-3,5-dione (160.00 mg, 0.279 mmol, 1.00 eq), DCM (10.00 mL). BBr₃ (279.61 mg, 1.116 mmol, 4.00 eq) was added at -78°C. The resulting solution was stirred 4 h at rt. The reaction mixture was concentrated under reduced pressure to afford the crude product. Purification resulted in 54.3mg (yield 42%) of 2-[3,5-dichloro-4-[(5-isopropyl-6-oxo-1*H-*pyridazin-3-yl)oxy]phenyl]-6-(dimethylamino)-4*H*-1,2,4-triazine-3,5-dione.

¹HNMR (300 MHz, DMSO-*d*₆) δ ppm: 7.91 (s, 2H), 7.43 (s, 1H), 3.03 - 3.10 (m, 1H), 2.99 (d, J = 8.1Hz, 6H), 1.20 (d, J = 6.9 Hz,6H).

### Example 16: Synthesis of compounds 16 and 23

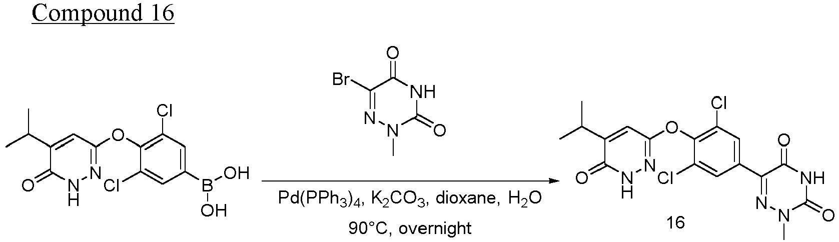

To a stirred mixture of 3,5-dichloro-4-[(5-isopropyl-6-oxo-1*H*-pyridazin-3-yl)oxy]phenylboronic acid (250.00 mg, 0.729 mmol, 1.00 eq), 6-bromo-2-methyl-4*H*-1,2,4-triazine-3,5-dione (180.19 mg, 0.875 mmol, 1.20 eq), K₂CO₃ (201.48 mg, 1.458 mmol, 2.00 eq) in dioxane (10 mL) and H₂O (1 mL) was added Pd(PPh₃)₄ (168.46 mg, 0.146 mmol, 0.20 eq) under nitrogen atmosphere. The resulting mixture was stirred overnight at 90 °C under nitrogen atmosphere. The reaction was quenched with water (15 mL) and extracted with EA (3x15 mL). The combined organic layers were washed with brine (2x15 mL), dried over anhydrous sodium sulfate filtered and concentrated under reduced pressure. The crude product was triturated with EA:PE of 1:5 and MeCN (5 mL), washed with ether (5 mL) to provide the desired product 6-[3,5-dichloro-4-[(5-isopropyl-6-oxo-1*H*-pyridazin-3-yl)oxy]phenyl]-2-methyl-4*H*-1,2,4-triazine-3,5-dione (103.8 mg, 34%) as a white solid.

¹H NMR (300 MHz, DMSO-*d*₆) δ ppm: 12.45 (br, 1H), 12.21 (s, 1H), 8.10 (s, 2H), 7.43 (s, 1H), 3.58 (s, 3H), 3.11 - 2.97 (m, 1H), 1.20 (d, *J =* 6.9 Hz, 6H).

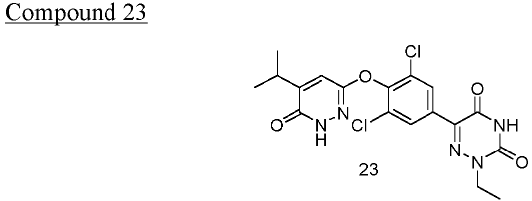

Compound 23 was prepared similarly as described for compound 16, using 6-bromo-2-ethyl-4*H*-1,2,4-triazine-3,5-dione instead of 6-bromo-2-methyl-4*H*-1,2,4-triazine-3,5-dione.

¹H NMR (300 MHz, DMSO-*d*₆) δ: 12.39 (s, 1H), 12.18 (s, 1H), 8.09 (s, 2H), 7.40 -7.41 (m, 1H), 3.95 - 4.03 (m, 2H), 3.00 - 3.07 (m, 1H), 1.28 (t, *J* = 7.0 Hz, 3H), 1.28 (t, *J* = 6.9 Hz, 6H). LCMS (ESI, *m*/*z):* 438 [M+H]⁺.

### Example 17: Synthesis of compound 17

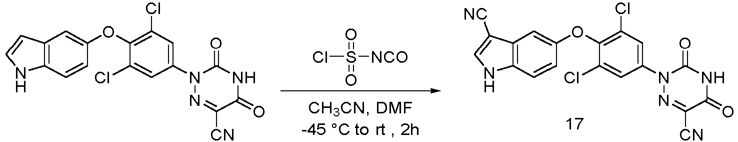

To a solution of 2-[3,5-dichloro-4-(1*H*-indol-5-yloxy)phenyl]-3,5-dioxo-4*H-*1,2,4-triazine-6-carbonitrile (250.0 mg, 0.604 mmol, 1.00 eq) in acetonitrile (4 mL) was added dropwise chlorosulfonyl isocyanate (102.5 mg, 0.724 mmol, 1.2 eq) in acetonitrile (0.4 mL) at -45 °C under nitrogen. Over the course of the addition a fine precipitate formed. The mixture was stirred at -45 °C under nitrogen for 10min. N,N-dimethylformamide (4 mL) was then slowly added, and the mixture was allowed to warm to rt and stirred for 2 h. The reaction was quenched by the addition of water/ice (10 mL) at 0 °C. The resulting mixture was extracted with ethyl acetate (3x15 mL). The combined organic layers were washed with water (3x10 mL), saturated NaHCO₃, and brine, dried anhydrous sodium sulfate, filtered and evaporated. The residue was purified by Prep-TLC with dichloromethane:methanol (12:1) to afford crude product (160 mg).The crude product (160mg) was purified by Prep-HPLC with the following conditions (Column: Kinetex EVO C 18 Column, 30*150, 5 µm; Mobile Phase A: Water (0.05% TFA), Mobile Phase B: ACN; Flow rate: 60 mL/min; Gradient: 50% B to 50% B in 8 min) to provide 5-[2,6-dichloro-4-(6-cyano-3,5-dioxo-4*H*-1,2,4-triazin-2-yl)phenoxy]-1*H*-indole-3-carbonitrile (75.9 mg, 29 % yield) of as a light yellow solid.

¹H NMR (300MHz, DMSO-*d*₆) δ 13.30 (s, 1H), 12.28 (s, 1H), 8.28 (d, J = 3.0 Hz, 1H), 7.84 (s, 2H), 7.58 (d, J = 9.0 Hz, 1H), 6.97 - 7.01 (m, 1H), 6.86 (d, J = 2.4 Hz, 1H).

### Example 18: Synthesis of compounds 18 and 19

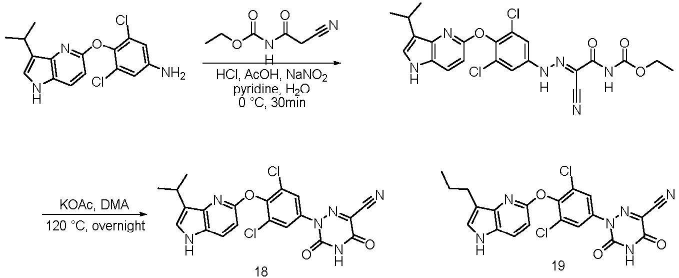

NaNO₂ (150.8 mg, 2.19 mmol, 2.1 eq) in H₂O (12 mL) was added dropwise to a solution of 3,5-dichloro-4-([3-isopropyl-1*H*-pyrrolo[3,2-b]pyridin-5-yl]oxy)aniline (350.0 mg, 1.04 mmol, 1.0 eq) in HCl (5.5 mL, conc.), AcOH (16 mL) and H₂O (12 mL). The mixture was stirred for 30 min at 0 °C. Then the reaction mixture was poured into a solution of ethyl N-(2-cyanoacetyl)carbamate (243.8 mg, 1.56 mmol, 1.5 eq) in H₂O (16 mL) and pyridine (5.5 mL) at 0 °C quickly. The resulting mixture was stirred at 0 °C for 30 min and filtered. The filter cake was washed with water (2x15 mL) and petroleum ether (2x15 mL) dried under reduced pressure to provide (350 mg, crude) ethyl N-[(E)-cyano([2-[3,5-dichloro-4-([3-isopropyl-1*H*-pyrrolo[3,2-b]pyridin-5-yl]oxy)phenyl]hydrazin-1-ylidene])carbonyl]carbamate as a yellow solid.

LCMS (ESI, *m*/*z):* 503 [M+H]⁺.

To a solution of ethyl N-[(E)-cyano([2-[3,5-dichloro-4-([3-isopropyl-1*H-*pyrrolo[3,2-b]pyridin-5-yl]oxy)phenyl]hydrazin-1-ylidene])carbonyl]carbamate (350.00 mg, 0.695 mmol, 1.00 eq) in DMA (20 mL) was added KOAc (341.22 mg, 3.477 mmol, 5.00 eq). The resulting mixture was stirred overnight at 120 °C and quenched with water (30 mL). The resulting mixture was extracted with EA (3x40 mL) and the organic layers were combined, washed with brine (2x30 mL), dried over anhydrous sodium sulfate, filtered and concentrated under reduced pressure to afford crude product. The crude product was purified by TLC (Mobile phase: MeOH/DCM=1: 7; Rf = 0.4; detection: UV) to provide the crude product (180 mg). The product was separated by Prep-Chiral-HPLC Column: CHIRALPAK IE, 2*25 cm, 5 µm; Mobile Phase A: Hex (0.1% FA)--HPLC, Mobile Phase B: EtOH--HPLC; Flow rate: 20 mL/min; Gradient: 10% B to 10% B in 21 min. Purification resulted in desired product 2-[3,5-dichloro-4-([3-isopropyl-1*H*-pyrrolo[3,2-b]pyridin-5-yl]oxy)phenyl]-3,5-dioxo-4*H-*1,2,4-triazine-6-carbonitrile (86.3 mg, 26%) as a yellow solid.

¹H NMR (300 MHz, DMSO-*d*₆) δ 13.25 (br, 1H), 11.00 (s, 1H), 7.82 (d, *J* = 8.7 Hz, 1H), 7.76 (s, 2H), 7.28 (d, *J* = 2.4 Hz,1H), 6.89 (d, *J =* 8.7 Hz, 1H), 2.78 - 2.95 (m, 1H), 1.13 (d, *J =* 6.6 Hz, 6H).

LCMS (ESI, *m*/*z):* 457.0 [M+H]⁺.

The reaction also produces the by-product compound 2-[3,5-dichloro-4-([3-propyl-1*H*-pyrrolo[3,2-b]-pyridin-5-yl]oxy)phenyl]-3,5-dioxo-4*H*-1,2,4-triazine-6-carbonitrile (20.8 mg, 6%) as a yellow solid.

¹H NMR (300 MHz, DMSO-*d*₆) δ 13.26 (br, 1H), 11.03 (s, 1H), 7.82 (d, *J =* 8.4 Hz, 1H), 7.76 (s, 2H), 7.33 (d, *J =* 2.4 Hz,1H), 6.87 (d, *J =* 8.7 Hz, 1H), 2.42 (t, *J =* 7.5 Hz, 2H), 1.45 - 1.58 (m, 2H), 0.77 (t, *J =* 7.5 Hz, 3H). LCMS (ESI, *m*/*z):* 457.0 [M+H]⁺.

### Example 19: Synthesis of compounds 20, 21 and 22

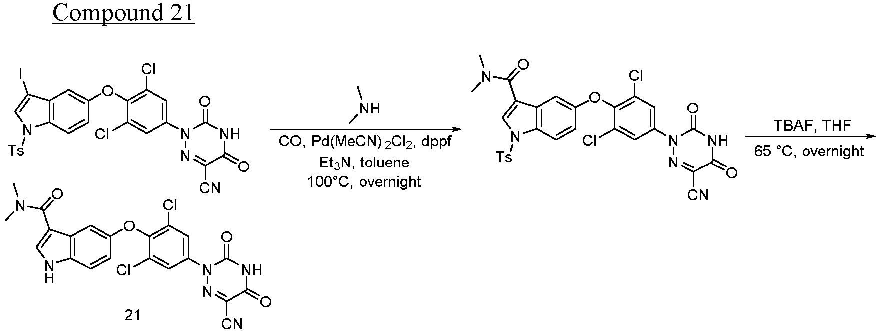

A 20 mL autoclave was charged with 2-(3,5-dichloro-4-[[3-iodo-1-(4-methylbenzenesulfonyl)indol-5-yl]oxy]phenyl)-3,5-dioxo-4*H*-1,2,4-triazine-6-carbo-nitrile (600.00 mg, 0.864 mmol, 1.00 eq), dimethylamine (2.59 mL, 2 M in tetrahydrofuran, 5.185 mmol, 6.00 eq), 1,1'-bis(diphenylphosphino)ferrocene (95.47 mg, 0.173 mmol, 0.20 eq), bis(acetonitrile)palladium(II) chloride (22.42 mg, 0.0864 mmol, 0.10 eq), triethylamine (262.35 mg, 2.593 mmol, 3.00 eq), toluene (15 mL). The contents of the autoclave were placed under an atmosphere of carbon monoxide (20 atm). The reaction was stirred overnight at 100 °C and quenched with water (10 mL). The resulting mixture was extracted with ethyl acetate (3x15 mL) and the organic layers were combined, washed with brine (3x15 mL), dried over anhydrous sodium sulfate. After filtration, the filtrate was concentrated under reduced pressure. The residue was chromatographed on a C18 column chromatography with CH₃CN:Water (3:2) to provide 300 mg (49% yield) of 5-[2,6-dichloro-4-(6-cyano-3,5-dioxo-4*H*-1,2,4-triazin-2-yl)phenoxy]-N,N-dimethyl-1-(4-methylbenzenesulfonyl)indole-3-carboxamide as a light brown solid.

LCMS (ESI, *m*/*z):* 637 [M-H]⁻.

To a stirred solution of 5-[2,6-dichloro-4-(6-cyano-3,5-dioxo-4*H*-1,2,4-triazin-2-yl)phenoxy]-N,N-dimethyl-1-(4-methylbenzenesulfonyl)indole-3-carbox-amide (200.00 mg, 0.313 mmol, 1.00 eq) in tetrahydrofuran (10 mL) was added tetrabutylammonium fluoride (1.25 mL, 1 M in tetrahydrofuran, 1.251 mmol, 4.00 eq) at room temperature under nitrogen atmosphere. The reaction mixture was stirred for overnight at 65 °C and quenched with water (5 mL). The resulting mixture was extracted with dichloromethane (3x20 mL) and the organic layers were combined, washed with brine (5x10 mL) and hydrochloric acid (1 M), dried over anhydrous sodium sulfate. After filtration, the filtrate was concentrated under reduced pressure. The crude product (150 mg) was purified by Prep-HPLC with the following conditions (Column: XBridge Shield RP18 OBD Column, 19*250 mm,10 µm; Mobile Phase A: Water (10 mM NH₄HCO₃), Mobile Phase B: ACN; Flow rate: 25 mL/min; Gradient: 27% B to 47% B in 7 min) to provide 52 mg (34% yield) of 5-[2,6-dichloro-4-(6-cyano-3,5-dioxo-4*H*-1,2,4-triazin-2-yl)phenoxy]-N,N-dimethyl-1*H-*indole-3-carboxamide as a light yellow solid.

¹H NMR (300 MHz, Methanol-*d*₄) δ 7.76 (s, 1H),7.68 (s, 2H), 7.41 (d, J = 9.0 Hz, 1H), 7.08 (d, J = 2.1 Hz, 1H), 6.90 - 6.94 (m, 1H), 3.13 (s, 6H).

LCMS (ESI, *m*/*z):* 485 [M+H]⁺.

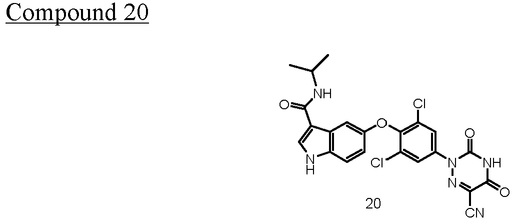

Compound 20 was prepared similarly as described for compound 21, using 2 eq of isopropylamine instead of 6 eq dimethylamine and reacting overnight at 80°C.

¹H NMR (300 MHz, Methanol-*d*₄) δ 7.89 (s, 1H),7.77 (s, 2H), 7.50 (d, J = 2.4 Hz, 1H), 7.38 (d, J = 9.3 Hz, 1H), 6.87 - 6.91(m, 1H),4.12 - 4.16 (m, 1H),1.21(d, J = 6.6 Hz, 6H).

LCMS (ESI, *m*/*z):* 499 [M+H]⁺.

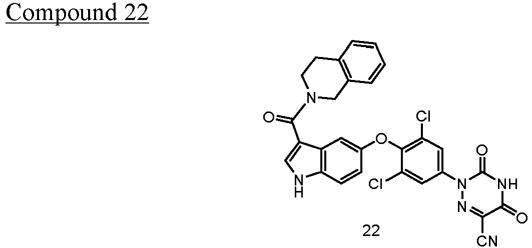

Compound 22 was prepared similarly as described for compound 21, using 1.5 eq tetrahydroisoquinoline instead of 6 eq dimethylamine.

¹H NMR (400 MHz, Methanol-*d*₄) δ 7.70 (d, *J* = 8.4 Hz, 3H), 7.45 (d, *J* = 8.8 Hz, 1H), 7.15 - 7.17 (m, 3H), 7.07 (br, 1H), 6.93 - 6.99 (m, 1H), 6.91 - 6.92 (m, 1H), 4.78 (s, 2H), 3.86 (t, *J* = 6.0 Hz, 2H), 2.87 (t, *J =* 5.8 Hz, 2H).

LCMS: 573 [M+H]⁺.

### Example 20: Synthesis of compounds 24, 25 and 26

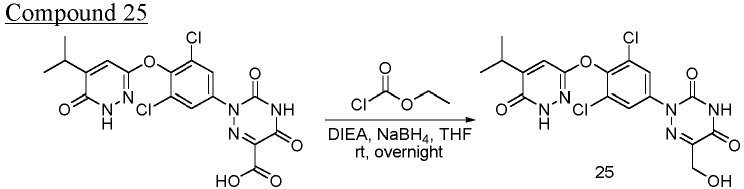

To a solution of 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid (500 mg, 1.101 mmol, 1.00 eq) and N,N-diisopropylethylamine (298.77 mg, 2.312 mmol, 2.10 eq) in tetrahydrofuran (30 mL) at 0 °C under nitrogen was added ethyl chloroformate (126.62 mg, 1.167 mmol, 1.06 eq) dropwise. The mixture was stirred for 1 h at room temperature and treated dropwise with a solution of sodium borohydride (124.94 mg, 3.302 mmol, 3.00 eq) in water (2 mL) at 0 °C. The resulting solution was stirred overnight at room temperature and then quenched slowly with saturated NaHCO₃ solution (10 mL). The resulting mixture was extracted with chloroform/isopropanol (3/1) (5x30 mL) and the organic layers were combined, dried over anhydrous sodium sulfate, filtered and concentrated under reduced pressure to provide the crude product. The crude product (400 mg) was purified by preparative HPLC using the following gradient conditions: Column: XSelect CSH Prep C18 OBD Column, 5 µm, 19*150 mm; Mobile Phase A: Water (10 mM NH₄HCO₃ + 0.1% NH₃.H₂O), Mobile Phase B: ACN; Flow rate: 25 mL/min; Gradient: 19% B to 31% B in 8 min), resulting in of 2-[3,5-dichloro-4-[(5-isopropyl-6-oxo-1*H*-pyridazin-3-yl)oxy]phenyl]-6-(hydroxymeth-yl)-4*H*-1,2,4-triazine-3,5-dione (100.6 mg, 21% yield) as a white solid.

¹H NMR (300 MHz, DMSO-*d*₆) δ 12.48 (br, 1H), 12.22 (br, 1H), 7.86 (s, 2H), 7.44 (s, 1H), 5.30 (t, J = 6.0 Hz, 1H), 4.40 (d, J = 6.0 Hz, 2H), 3.02 - 3.11 (m, 1H), 1.20 (d, J = 6.9 Hz, 6H).

LCMS (ESI, *m*/*z):* 440 [M+H]⁺.

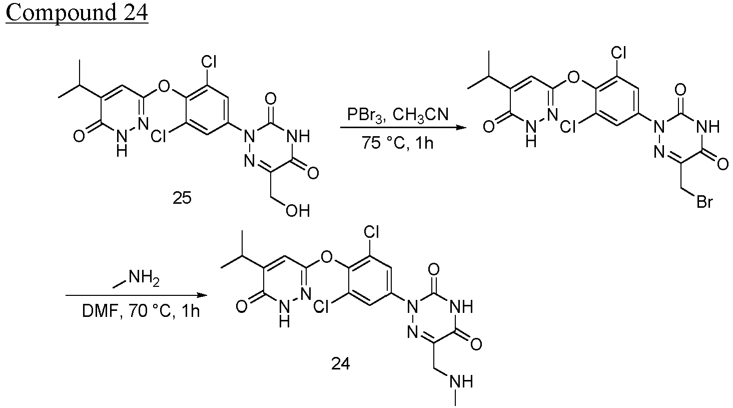

A 50-mL round-bottom flask was charged with 2-[3,5-dichloro-4-[(5-isopropyl-6-oxo-1*H*-pyridazin-3-yl)oxy]phenyl]-6-(hydroxymethyl)-4*H*-1,2,4-triazine-3,5-dione (1.60 g, 3.634 mmol, 1.00 eq), acetonitrile (15 mL). Phosphorus tribromide (0.3 mL) was added at 0 °C under nitrogen. The resulting solution was stirred for 1 h at 75 °C. The mixture was cooled to room temperature and then quenched with saturated NaHCO₃ aqueous (10 mL). The resulting mixture was extracted with ethyl acetate (3x15 mL) and the organic layers were combined, washed with brine (1x30 mL), dried over anhydrous sodium sulfate, filtered and concentrated under reduced pressure to provide 1.1 g (39 % yield) of 6-(bromomethyl)-2-[3,5-dichloro-4-[(5-isopropyl-6-oxo-1*H*-pyridazin-3-yl)oxy]phenyl]-4*H-*1,2,4-triazine-3,5-dione as a yellow solid.

LCMS (ESI, *m*/*z):* 502 [M+H]⁺.

A 50-mL round-bottom flask was charged with 6-(bromomethyl)-2-[3,5-dichloro-4-[(5-isopropyl-6-oxo-1*H*-pyridazin-3-yl)oxy]phenyl]-4*H*-1,2,4-tri-azine-3,5-dione (400 mg, 0.795 mmol, 1.00 eq), methylamine (1.2 mL, 2 M in tetrahydrofuran, 2.385 mmol, 3.00 eq) and N,N-dimethylformamide (8 mL). The mixture was stirred for 1 h at 70 °C. The mixture was cooled to room temperature and diluted with water (5 mL). At this time, it was acidified by the addition of 1 N hydrochloric acid (5 mL) and was extracted with ethyl acetate (3x10 mL). The organic layer was discarded. The aqueous layer was made basic by the addition saturated NaHCO₃ solution (15 mL) and extracted with a mixture solution of chloroform:isopropanol of 3:1 (5x30 mL) and the organic layers were combined, dried over anhydrous sodium sulfate, filtered and concentrated under reduced pressure to provide the crude product. The crude product (300 mg) was purified by preparative HPLC using the following gradient conditions: Column: XBridge Prep OBD C18 Column, 30×150 mm 5 µm; Mobile Phase A: Water (10 mM NH₄HCO₃ + 0.1% NH₃.H₂O), Mobile Phase B: ACN; Flow rate: 60 mL/min; Gradient: 17% B to 35% B in 8 min. Purification resulted in of 2-[3,5-dichloro-4-[(5-isopropyl-6-oxo-1*H*-pyridazin-3-yl)oxy]phenyl]-6-[(methylamino)methyl]-4*H*-1,2,4-triazine-3,5-dione (64.8 mg, 18% yield) as a white solid. ¹H NMR (300 MHz, DMSO-*d*₆) δ 12.23 (br, 1H), 7.88 (s, 2H), 7.43 (s, 1H), 3.83 (s, 2H), 3.01 - 3.19 (m, 1H), 2.51 (s, 3H), 1.20 (d, J = 6.9 Hz, 6H). LCMS (ESI, *m*/*z):* 453 [M+H]⁺.

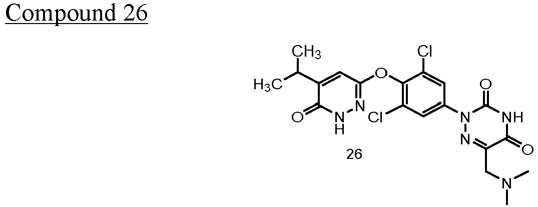

Compound 26 was prepared similarly as described for compound 24, using dimethylamine instead methylamine and reacting at 60°C for 1 h. ¹H NMR (300 MHz, DMSO-*d*₆) δ 12.21 (br, 1H), 7.80 (s, 2H), 7.45 (s, 1H), 3.37 (br, 2H), 3.03 - 3.08 (m, 1H), 2.25 (s, 6H), 1.20 (d, J = 6.9 Hz, 6H). LCMS (ESI, *m*/*z):* 467 [M+H]⁺.

### Example 21: Synthesis of compound 27

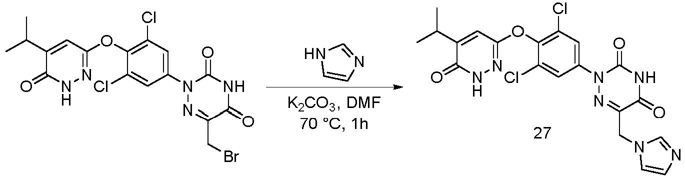

A 100-mL round-bottom flask was charged with 6-(bromomethyl)-2-[3,5-dichloro-4-[(5-isopropyl-6-oxo-1*H*-pyridazin-3-yl)oxy]phenyl]-4*H*-1,2,4-triazine-3,5-dione (400.00 mg, 0.795 mmol, 1.00 eq), imidazole (64.95 mg, 0.954 mmol, 1.20 eq), potassium carbonate (219.75 mg, 1.590 mmol, 2.00 eq) and N,N-dimethylformamide (8 mL). The mixture was stirred for 1 h at 70 °C and was subsequently cooled to room temperature and diluted with water (5 mL). At this time, it was acidified by the addition of 1 N hydrochloric acid (5 mL) and was extracted with ethyl acetate (3x10 mL). The organic layer was discarded. The aqueous layer was made basic by the addition saturated NaHCO₃ solution (15 mL) and extracted with a mixture of chloroform:isopropanol of 3:1 (5x30 mL) and the organic layers were combined, dried over anhydrous sodium sulfate, filtered and concentrated under reduced pressure to provide the crude product. The crude product (150 mg) was purified by preparative HPLC using the following gradient conditions: Column: XBridge Prep OBD C18 Column, 30×150 mm, 5 µm; Mobile Phase A: Water (10 mM NH₄HCO₃ + 0.1% NH₃.H₂O), Mobile Phase B: ACN; Flow rate: 60 mL/min; Gradient: 19% B to 37% B in 8 min; Purification resulted in of 2-[3,5-dichloro-4-[(5-isopropyl-6-oxo-1H-pyridazin-3-yl)oxy]phenyl]-6-(imidazol-1-ylmethyl)-4H-1,2,4-triazine-3,5-dione (14.5 mg, 4% yield) as a white solid.

¹H NMR (300 MHz, DMSO-*d*₆) δ 12.19 (s, 1H), 7.74 (s, 2H), 7.69 (s, 1H), 7.43 (s, 1H), 7.21 (s, 1H), 6.90 (s, 1H), 5.15 (s, 2H), 3.02 - 3.09 (m, 1H), 1.19 (d, J = 6.9 Hz, 6H).

LCMS (ESI, *m*/*z):* 490 [M+H]⁺.

### Example 22. Synthesis of compound 28

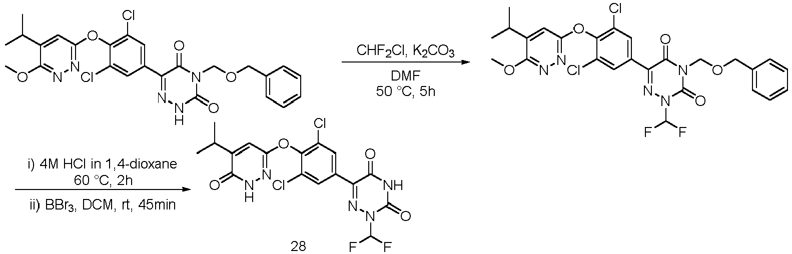

To a stirred mixture of 4-[(benzyloxy)methyl]-6-[3,5-dichloro-4-[(5-isopropyl-6-methoxypyridazin-3-yl)oxy]phenyl]-2*H*-1,2,4-triazine-3,5-dione (310 mg, 0.569 mmol, 1.00 eq) and K₂CO₃ (630 mg, 4.56 mmol, 8.00 eq) in DMF (20 mL) was bubbled with difluorochloromethane for 4 h and the mixture was stirred for 5 h at 50 °C. The reaction was quenched by the addition of water (50 mL). The resulting mixture was extracted with EA (3x30 mL). The combined organic layers were washed with water (150 mL), dried over anhydrous Na₂SO₄, filtered and concentrated under reduced pressure. The residue was purified by silica gel column chromatography (eluting with PE:EA of 2:1) to afford 230 mg (65% yield) of 4-[(benzyloxy)methyl]-6-[3,5-dichloro-4-[(5-isopropyl-6-methoxypyridazin-3-yl)oxy]phenyl]-2-(difluoromethyl)-1,2,4-triazine-3,5-dione as a light yellow solid.

LCMS (ESI, *m*/*z):* 594 [M+H]⁺.

4-[(Benzyloxy)methyl]-6-[3,5-dichloro-4-[(5-isopropyl-6-methoxy-pyridazin-3-yl)oxy]phenyl]-2-(difluoromethyl)-1,2,4-triazine-3,5-dione (230 mg, 0.387 mmol, 1.00 eq) in a solution of hydrogen chloride (4 mL, 4M in 1,4-dioxane) was stirred for 2 h at 60°C. After cooled down to 25°C, the resulting mixture was concentrated under vacuum. The residue was then diluted with DCM (5 mL) and added a solution of BBr₃ (1.5 mL, 1 M in DCM) dropwise at 25°C. The final reaction mixture was stirred for 45 min at 25°C. The reaction was quenched by the addition of water (1 mL). The resulting mixture was concentrated under reduced pressure. The residue was purified by reverse phase chromatography (column: C18 silica gel; Mobile phase, A: water (containing 10 mM NH₄HCO₃) and B: ACN (0% to 50% over 15 min); Detector: UV 220/254 nm). The product fractions were lyophilized to afford 113.4 mg (63% yield) of 6-[3,5-dichloro-4-[(5-isopropyl-6-oxo-1*H*-pyridazin-3-yl)oxy]phenyl]-2-(difluoromethyl)-4*H*-1,2,4-triazine-3,5-dione as a white solid.

¹H NMR (400 MHz, DMSO-*d*₆) δ ppm: 12.78 (br s, 1H), 12.22 (br s, 1H), 7.99 (s, 2H), 7.83 (t, *J =* 57.2 Hz, 1H), 7.45 (s, 1H), 3.13 - 2.98 (m, 1H), 1.20 (d, *J =* 7.2 Hz, 6H). LCMS (ESI, *m*/*z):* 460 [M+H]⁺.

### Example 23 Synthesis of compound 29

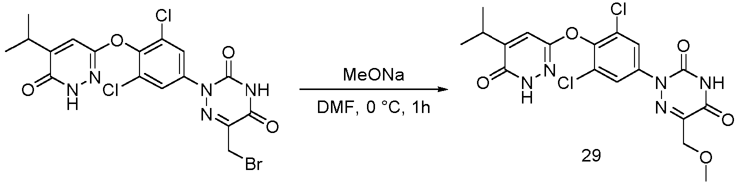

A 50-mL round-bottom flask was charged with 6-(bromomethyl)-2-[3,5-dichloro-4-[(5-isopropyl-6-oxo-1*H*-pyridazin-3-yl)oxy]phenyl]-4*H*-1,2,4-triazine-3,5-dione (400 mg, 0.795 mmol, 1.00 eq), N,N-dimethylformamide (8 mL) under nitrogen. Sodium methylate (0.50 mL, 30% w/w in methanol) was added at 0 °C. The mixture was stirred for 1 h at 0 °C. The mixture was acidified by the addition of 1 N hydrochloric acid (5 mL) and was extracted with ethyl acetate (3x15 mL). The organic layer was washed with brine (2x30 mL), dried over anhydrous sodium sulfate, filtered and concentrated under reduced pressure. The residue was chromatographed on a silica gel column with dichloromethane/methanol (10/1) to provide the crude product (150mg) and then was purified by preparative HPLC using the following gradient conditions: Column: XSelect CSH Prep C18 OBD Column,, 5 µm,19*150 mm; Mobile Phase A: Water (0.1%FA), Mobile Phase B: ACN; Flow rate: 25 mL/min; Gradient: 31% B to 51% B in 8 min; Purification resulted in 2-[3,5-dichloro-4-[(5-isopropyl-6-oxo-1*H*-pyridazin-3-yl)oxy]phenyl]-6-(methoxymethyl)-4*H*-1,2,4-triazine-3,5-dione (26.2 mg, 7% yield) of as a white solid.

¹H NMR (300 MHz, DMSO-*d*₆) δ 12.53 (s, 1H), 12.22 (s, 1H), 7.80 (s, 2H), 7.45 (s, 1H), 4.32 (s, 2H), 3.03 - 3.07 (m, 1H), 2.48 (s, 3H), 1.20 (d, J = 6.9 Hz, 6H).

LCMS (ESI, *m*/*z):* 454 [M+H]⁺.

### Example 24 Synthesis of compound 30

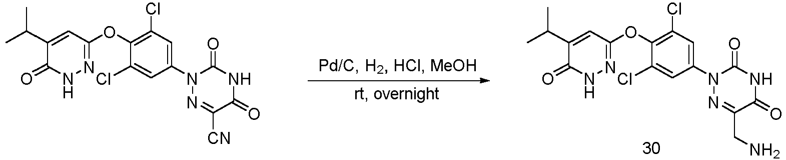

A 100-mL round-bottom flask was charged with 2-[3,5-dichloro-4-[(5-isopropyl-6-oxo-1*H*-pyridazin-3-yl)oxy]phenyl]-3,5-dioxo-4*H*-1,2,4-triazine-6-carbo-nitrile (300.00 mg, 0.689 mmol, 1.00 eq), concentrated hydrochloric acid (2 mL), Pd/C (30.00 mg, 0.282 mmol, 0.41 eq), methanol (30.00 mL, 0.936 mmol, 1.36 eq) under hydrogen. The resulting solution was stirred overnight at room temperature and then filtered through celite. The celite pad was washed with methanol (5x30 mL), the filtrate was collected and concentrated under reduced pressure. The residue was diluted with saturated NaHCO₃ solution(10ml) and extracted with chloroform:isopropanol (3:1) (5x15 mL) and the organic layers were combined, dried over anhydrous sodium sulfate, filtered and concentrated under reduced pressure. The crude product (300 mg) was purified by preparative HPLC using the following gradient conditions: Column: Kinetex EVO C18 Column, 30*150, 5 µm; Mobile Phase A: Water (10 mM NH₄HCO₃ + 0.1% NH₃.H₂O), Mobile Phase B: ACN; Flow rate: 25 mL/min; Gradient: 5% B to 30% B in 9 min;. Purification resulted in 6-(aminomethyl)-2-[3,5-dichloro-4-[(5-isopropyl-6-oxo-1*H*-pyridazin-3-yl)oxy]phenyl]-4*H*-1,2,4-triazine-3,5-dione (29.6 mg, 10% yield) of a white solid. ¹H NMR (300 MHz, DMSO-*d*₆) δ 7.93 (s, 2H), 7.42 (s, 1H), 3.84 (br, 2H), 3.02 - 3.07 (m, 1H), 1.20 (d, J = 6.9 Hz, 6H). LCMS (ESI, *m*/*z):* 439 [M+H]⁺.

### Example 25. Synthesis of compound 31

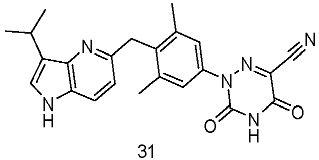

To a mixture of 4-[[3-isopropyl-1-(p-tolylsulfonyl)pyrrolo[3,2-b]pyridin-5-yl]methyl]-3,5-dimethyl-aniline (70 mg, 156.39 µmol, 1 eq), con. HCl (87.97 µL, 6.75 eq) in H₂O (1 mL) was added dropwise a solution of sodium nitrite (14.84 mg, 215.04 µmol, 1.38 eq) in H₂O (1 mL) while maintaining the temperature below 0 °C. After the completion of addition, the reaction mixture was stirred for 0.5 h. A mixture of ethyl N-(2-cyanoacetyl)carbamate (27.47 mg, 175.94 µmol, 1.13 eq) and NaOAc (43.30 mg, 527.83 µmol, 3.38 eq) in EtOH (3 mL) was added drop-wise to the resulting diazonium salt solution below 0 °C and stirred for a further 2 h. The reaction mixture (combined with another 50 mg batch) was diluted with H₂O (10 mL), adjusted to pH 7~8 by sat. aq. NaHCO₃, then extracted with EA (50 mLx3). The combined organic layers were washed with brine (100 mL), dried over anhydrous Na₂SO₄, filtered and concentrated to give a residue The residue was purified by flash silica gel chromatography (Ethyl acetate in Petroleum ether = 0~35%) to give ethyl N-[(2E)-2-cyano-2-[[4-[[3-isopropyl-1-(p-tolylsulfonyl)pyrrolo[3,2-b]pyridin-5-yl]methyl]-3,5-dimethyl-phenyl]hydrazono]acetyl]carbamate (75 mg, 31% yield) as a yellow solid.

A mixture of ethyl N-[(2E)-2-cyano-2-[[4-[[3-isopropyl-1-(p-tolylsulfonyl)pyrrolo[3,2-b]pyridin-5-yl]methyl]-3,5-dimethyl-phenyl]hydrazono]-acetyl]carbamate (55 mg, 89.47 µmol, 1 eq) and NaOAc (36.70 mg, 447.36 µmol, 5 eq) in AcOH (5 mL) was stirred at 130 °C for 5 h. AcOH was removed under reduced pressure, the residue was diluted with H₂O (50 mL), adjusted to pH 7~8 by sat. aq. NaHCO₃, then extracted with EA (50 mLx3). The combined organic layers were washed with brine (50 mL), dried over anhydrous Na₂SO₄, filtered and concentrated under reduced pressure to give 2-[4-[[3-isopropyl-1-(p-tolylsulfonyl)pyrrolo[3,2-b]pyridin-5-yl]methyl]-3,5-dimethyl-phenyl]-3,5-dioxo-1,2,4-triazine-6-carbonitrile (65 mg) as a yellow solid, which was directly used in the next step without further purification.

To a solution of 2-[4-[[3-isopropyl-1-(p-tolylsulfonyl)pyrrolo[3,2-b]pyridin-5-yl]methyl]-3,5-dimethyl-phenyl]-3,5-dioxo-1,2,4-triazine-6-carbonitrile (60 mg, 105.51 µmol, 1 eq) in THF (5 mL) was added TBAF (1 M in THF, 2.53 mL, 24 eq) in one portion at 20 °C. Then the resulting mixture was stirred at 65 °C for 8 h under N₂. LCMS showed the reaction was complete. The mixture was diluted with H₂O (100 mL) and extracted with EA (50 mLx3). The combined organic layers were washed with sat. aq. NH₄Cl (100 mLx3), dried over anhydrous Na₂SO₄, filtered and concentrated under reduced pressure to give crude product, which was purified by prep. HPLC [Column: Welch Xtimate C18 150*30mm, 5 µm; Mobile phase: from 15% ACN in water (0.225%FA) to 45% ACN in water (0.225%FA)] to give 2-[4-[(3-isopropyl-1*H*-pyrrolo[3,2-b]pyridin-5-yl)methyl]-3,5-dimethyl-phenyl]-3,5-dioxo-1,2,4-triazine-6-carbonitrile (25 mg, 56 % yield, 98.3 % purity) as a light yellow solid.

¹H NMR (400MHz, DMSO-*d*₆) δ 13.11 - 12.66 (m, 1H), 10.83 (br s, 1H), 7.57 (d, J=8.4 Hz, 1H), 7.30 (d, J=2.1 Hz, 1H), 7.15 (s, 2H), 6.70 (d, J=8.4 Hz, 1H), 4.24 (s, 2H), 3.20 (td, J=6.8, 13.7 Hz, 1H), 2.39 (s, 6H), 1.33 (d, J=6.9 Hz, 6H).

### Example 26. Synthesis of compound 32

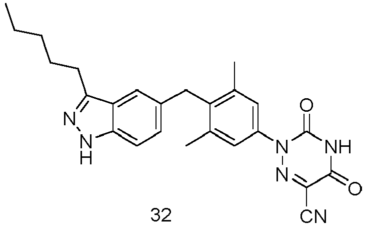

A solution of NaNO₂ (2.1 eq, 45 mg) in water (6.5 mL) was added to a solution of 3,5-dimethyl-4-((3-pentyl-1*H*-indazol-5-yl)methyl)aniline (1 eq, 100 mg, 0.31 mmol) in HCl 37% (106 eq, 2.7 mL), acetic acid (8.3 mL) and water (6.5 mL) at 0 °C under N₂. The reaction mixture was stirred at 0 °C for 1 h. In parallel, a solution of ethyl N-(2-cyanoacetyl)carbamate (1.5 eq, 73 mg) in water (7.8 mL) and pyridine (2.7 mL) was stirred at 0 °C for 15 min. The first reaction mixture was quickly added to the second one. The resulting reaction mixture was stirred at 0 °C for 2 h.

The precipitate was filtered and washed with water and petroleum ether to give ethyl-(2-cyano-2-(2-(3,5-dimethyl-4-((3-pentyl-1H-indazol-5-yl)methyl)-phenyl)hydrazineylidene)acetyl)carbamate (13 mg) as a yellow solid. The filtrate was extracted with DCM (3x50 mL) and the combined organic phases were dried over MgSO₄, filtered and evaporated to dryness to give ethyl-(2-cyano-2-(2-(3,5-dimethyl-4-((3-pentyl-1*H*-indazol-5-yl)methyl)phenyl)hydrazineylidene)acetyl)-carbamate (225 mg) as a yellow solid. The crude was used as such in the next step.

Sodium acetate (4 eq, 102 mg) was added to a solution of ethyl-(2-cyano-2-(2-(3,5-dimethyl-4-((3-pentyl-1*H*-indazol-5-yl)methyl)phenyl)hydrazineylidene)acetyl)carbamate (1 eq, 152 mg, 0.31 mmol) in acetic acid (5 mL) under N₂. The reaction mixture was heated to reflux for 4h and then cooled to 0 °C, water (10 mL) was added and the mixture was stirred for 30 min. Then, the precipitate was filtered, washed with water and petroleum ether and purified by flash chromatography on silica gel (0 to 10% MeOH in DCM) to give 40 mg of a red solid which was further triturated in Et₂O and *i*Pr₂O to give compound 32 (20 mg, 15%) as an orange solid. ¹H-NMR (DMSO, 400 MHz): 0.83 (t, *J* = 2.5 Hz, 3H); 1.27 (br, s, 4H); 1.63-1.70 (quint, *J* = 7.7 Hz, 2H); 2.28 (s, 6H) ; 2.80 (t, *J* = 7.2 Hz, 2H); 4.15 (s, 2H); 7.00 (d, *J* = 8.8 Hz, 1H); 7.18 (s, 2H); 7.27 (s, 1H); 7.35 (d, *J* = 8.8 Hz, 1H); 12.51 (s, 1H); 12.99 (s, 1H)

### Example 27. Synthesis of compound 33

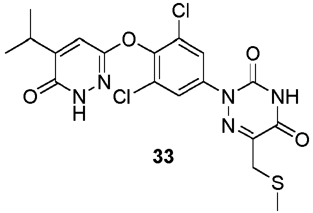

A 50 mL round-bottom flask was charged with 2-[3,5-dichloro-4-[(5-isopropyl-6-oxo-1*H*-pyridazin-3-yl)oxy]phenyl]-6-(hydroxymethyl)-4*H*-1,2,4-triazine-3,5-dione (1.60 g, 3.63 mmol), CH₃CN (15 mL). PBr₃ (0.3 mL) was added at 0 °C under N₂. The resulting solution was stirred for 1 h at 75 °C. The mixture was cooled to rt and then quenched with NaHCO₃ (sat., aq., 10 mL). The resulting mixture was extracted with EA (3 x 15 mL) and the organic layers were combined, washed with brine (1 x 30 mL), dried over anhyd. Na₂SO₄, filtered and concentrated under reduced pressure to provide 6-(bromomethyl)-2-[3,5-dichloro-4-[(5-isopropyl-6-oxo-1*H*-pyridazin-3-yl)oxy]phenyl]-4*H*-1,2,4-triazine-3,5-dione as a yellow solid (1.1 g, 39%).

A 50 mL round-bottom flask was charged with 6-(bromomethyl)-2-[3,5-dichloro-4-[(5-isopropyl-6-oxo-1*H*-pyridazin-3-yl)oxy]phenyl]-4*H*-1,2,4-triazine-3,5-dione (300 mg, 0.60 mmol), DMF (8 mL). NaSCH₃ (125 mg, 1.79 mmol) was added at 0 °C. The mixture was acidified by the addition of 1 N aq. HCl (5 mL) and was extracted with EA (3 x 15 mL). The organic layers were combined, washed with brine (2 x 50 mL), dried over anhyd. Na₂SO₄, filtered and concentrated under reduced pressure. The residue was purified on a silica gel column with PE:EA (1:3) to provide the crude product (150 mg) and then was purified by preparative HPLC. Purification resulted in 2-[3,5-dichloro-4-[(5-isopropyl-6-oxo-1*H-*pyridazin-3-yl)oxy]phenyl]-6-[(methylsulfanyl)-methyl]-4*H*-1,2,4-triazine-3,5-dione as an off-white solid (32.9 mg, 12%). ¹H NMR (300 MHz, DMSO-*d*₆) δ 12.55 (br, 1H), 12.21 (s, 1H), 7.81 (s, 2H), 7.45 (s, 1H), 3.54 (s, 2H), 3.01-3.10 (m, 1H), 2.11 (s, 3H), 1.20 (d, J=6.6 Hz, 6H). LCMS (ESI, *m*/*z*): 470 [M+H]⁺.

### Example 28. Synthesis of compound 34

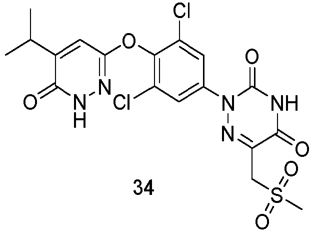

A 50 mL round-bottom flask was charged with 6-(bromomethyl)-2-[3,5-dichloro-4-[(5-isopropyl-6-oxo-1*H*-pyridazin-3-yl)oxy]phenyl]-4*H*-1,2,4-triazine-3,5-dione (480 mg, 0.954 mmol), DMF (8 mL), CH₃SO₂Na (292 mg, 2.86 mmol). The mixture was stirred for 1 h at 60 °C. The mixture was acidified by the addition of HCl (aq., 1N, 5 mL) and was extracted with EA (3 x 15 mL). The organic layers were combined, washed with brine (2 x 50 mL), dried over anhyd. Na₂SO₄, filtered and concentrated under reduced pressure. The residue was purified on a silica gel column with DCM:MeOH (10:1) to provide the crude product and then was purified by preparative HPLC to afford 2-[3,5-dichloro-4-[(5-isopropyl-6-oxo-1*H*-pyridazin-3-yl)oxy]-phenyl]-6-(methanesulfonylmethyl)-4*H*-1,2,4-triazine-3,5-dione as a white solid (70.8 mg, 15%). ¹H NMR (300 MHz, DMSO-*d*₆) δ 12.74 (br, 1H), 12.22 (s, 1H), 7.84 (s, 2H), 7.45 (s, 1H), 4.48 (s, 2H), 3.01-3.11 (m, 4H), 1.20 (d, *J*=6.9 Hz, 6H). LCMS (ESI, *m*/*z):* 502 [M+H]⁺.

### Example 29. Synthesis of compound 35

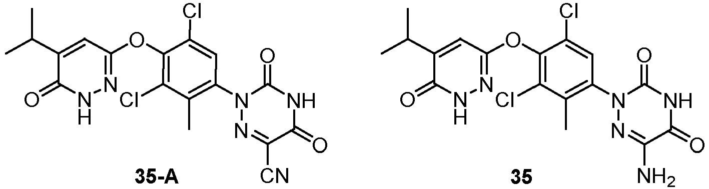

A 500 mL round-bottom was charged with 3-methyl-4-nitrophenol (4.50 g, 29.4 mmol), benzyltrimethylammonium tetrachloroiodate (24.6 g, 58.8 mmol), AcOH (600 mL). The reaction was stirred 18 h at 70 °C. The solids were removed by filtration and washed with AcOH (300 mL). The organic layers were concentrated under reduced pressure. The residue was dissolved in EA:water (500 mL:250 mL). The organic layer was separated, washed with brine (2 x 200 mL), dried over anhyd. Na₂SO₄, filtered and concentrated under reduced pressure. The crude product was purified on a silica gel column with EA:PE (4:94) to provide 2,6-dichloro-3-methyl-4-nitrophenol as a brown solid (4.9 g, 64%).

A 250 mL vial was charged with 2,6-dichloro-3-methyl-4-nitrophenol (5.00 g, 22.5 mmol), Fe powder (6.29 g, 113 mmol), NH₄Cl (9.64 g, 180 mmol), EtOH (50 mL), and water (25 mL). The reaction was stirred overnight at 50 °C. The solids were removed by filtration, and the filtrate was concentrated under reduced pressure. The residue was purified on a silica gel with EA:PE (2:5) to provide 4-amino-2,6-dichloro-3-methylphenol as a light brown solid (3.45 g, 59%).

3,5-Dichloro-4-[(6-chloro-5-isopropylpyridazin-3-yl)oxy]-2-methylaniline was prepared similarly as described for 3,5-dichloro-4-[(6-chloro-5-isopropyl-pyridazin-3-yl)oxy]-aniline to afford a brown solid (3.9 g, 73%), starting from 4-amino-2,6-dichloro-3-methylphenol.

6-(4-Amino-2,6-dichloro-3-methylphenoxy)-4-isopropyl-2*H*-pyridazin-3-one was prepared similarly as described for 6-(4-amino-2,6-dichlorophenoxy)-4-isopropyl-2*H-*pyridazin-3-one, starting from 4-amino-2,6-dichloro-3-methylphenol, to afford an off-white solid (2.5 g, 56%).

Ethyl (*E*)-(2-cyano-2-(2-(3,5-dichloro-4-((5-isopropyl-6-oxo-1,6-dihydropyridazin-3-yl)oxy)phenyl)hydrazineylidene)acetyl)carbamate was prepared similarly as described for ethyl (*E*)-(2-cyano-2-(2-(3,5-dichloro-4-((3-isopropyl-1*H-*pyrrolo[3,2-*b*]pyridin-5-yl)oxy)phenyl)hydrazineylidene)acetyl)carbamate to afford an orange solid (140 mg, 72%).

2-[3,5-Dichloro-4-[(5-isopropyl-6-oxo-1*H*-pyridazin-3-yl)oxy]-2-methylphenyl]-3,5-dioxo-4*H*-1,2,4-triazine-6-carbonitrile (compound 35-A) was prepared similarly as described for 2-[4-[[3-isopropyl-1-(*p*-tolylsulfonyl)pyrrolo[3,2-*b*]pyridin-5-yl]methyl]-3,5-dimethyl-phenyl]-3,5-dioxo-1,2,4-triazine-6-carbonitrile, to afford a brown solid (2.1 g, 64%).

2-[3,5-Dichloro-4-[(5-isopropyl-6-oxo-1*H*-pyridazin-3-yl)oxy]-2-methylphenyl]-3,5-dioxo-4*H*-1,2,4-triazine-6-carboxylic acid was prepared similarly as described for 2-[3,5-dichloro-4-[(5-isopropyl-6-oxo-1*H*-pyridazin-3-yl)oxy]-phenyl]-3,5-dioxo-4*H*-1,2,4-triazine-6-carboxylic acid to afford a brown solid (1.0 g, 52%).

*t*-Butyl-*N*-(2-[3,5-dichloro-4-[(5-isopropyl-6-oxo-1*H*-pyridazin-3-yl)oxy]-2-methylphenyl]-3,5-dioxo-4*H*-1,2,4-triazin-6-yl)carbamate was prepared similarly as described for *t*-butyl N-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate to afford a brown solid (600 mg, 71%).

A 100 mL round-bottom flask was charged with *t*-butyl-*N*-(2-[3,5-dichloro-4-[(5-isopropyl-6-oxo-1*H*-pyridazin-3-yl)oxy]-2-methylphenyl]-3,5-dioxo-4*H*-1,2,4-triazin-6-yl)carbamate (600 mg, 1.11 mmol), DCM (15.0 mL). TFA (5 mL) was added dropwise at 0 °C. The reaction was stirred overnight at rt and concentrated under reduced pressure. The residue was dissolved with EA (50 mL). The pH of the solution was adjusted to 8 with NaHCO₃ (sat., aq.), then extracted with EA (3x20 mL), the organic layers were combined, washed with brine (2x10 mL), dried over anhyd. Na₂SO₄, the solids were removed by filtration and the filtrate was concentrated under reduced pressure. The crude product was purified by preparative HPLC to provide 6-amino-2-[3,5-dichloro-4-[(5-isopropyl-6-oxo-1*H-pyridazin-3-yl)oxy]-2-methyl-phenyl]-4H-1,2,4-triazine-3,5-dione* (compound 35) as a white solid (105 mg, 39%). ¹H NMR (300 MHz, DMSO-*d*₆) δ 12.18 (br, 2H), 7.72 (s, 1H), 7.45 (s, 1H), 6.39 (br, 2H), 3.01-3.10 (m, 1H), 2.20 (s, 3H), 1.20 (d, *J*=6.9 Hz, 6H). LCMS (ESI, *m*/*z):* 439 [M+H]⁺.

### Example 30. Synthesis of compound 36

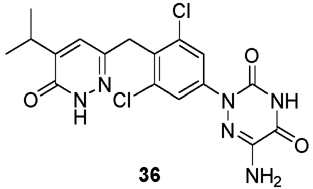

6-[(4-Amino-2,6-dichlorophenyl)methyl]-4-isopropyl-2*H*-pyridazin-3-one was prepared according to the literature procedure (J. Med. Chem. 2014, 57, 3912-3923) to afford a yellow solid (1.9 g, 50%).

Ethyl (2-cyano-2-(2-(3,5-dichloro-4-((5-isopropyl-6-oxo-1,6-dihydropyridazin-3-yl)methyl)phenyl)hydrazineylidene)acetyl)carbamate was prepared similarly as described for ethyl (2-cyano-2-(2-(4-((3-isopropyl-1-tosyl-1*H*-indol-5-yl)methyl)-3,5-dimethylphenyl)hydrazineylidene)acetyl)carbamate to afford a yellow solid (659 mg, 86%).

2-[3,5-Dichloro-4-[(5-isopropyl-6-oxo-1*H*-pyridazin-3-yl)methyl]phenyl]-3,5-dioxo-4*H*-1,2,4-triazine-6-carbonitrile was prepared similarly as described for 2-(4-[[3-isopropyl-1-(4-methylbenzene-sulfonyl)indol-5-yl]oxy]-3,5-dimethylphenyl)-3,5-dioxo-4*H-*1,2,4-triazine-6-carbonitrile to afford off-white solid (1.3 g, 86%).

2-[3,5-Dichloro-4-[(5-isopropyl-6-oxo-1*H*-pyridazin-3-yl)methyl]phenyl]-3,5-dioxo-4*H*-1,2,4-triazine-6-carboxylic acid was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid to afford a yellow oil (800 mg, 48%).

*t*-Butyl-N-(2-[3,5-dichloro-4-[(5-isopropyl-6-oxo-1*H*-pyridazin-3-yl)methyl]phenyl]-3,5-dioxo-4*H*-1,2,4-triazin-6-yl)-carbamate was prepared similarly as described for *t*-butyl *N*-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate to afford a yellow solid (600 mg, 65%).

6-Amino-2-[3,5-dichloro-4-[(5-isopropyl-6-oxo-1*H*-pyridazin-3-yl)methyl]-phenyl]-4*H*-1,2,4-triazine-3,5-dione was prepared similarly as described for 6-amino-2-[3,5-dichloro-4-[(5-isopropyl-6-oxo-1*H*-pyridazin-3-yl)oxy]-2-methylphenyl]-4*H*-1,2,4-triazine-3,5-dione to afford a white solid (227 mg, 49%). ¹H NMR (300 MHz, DMSO-*d*₆) δ 7.76 (s, 2H), 7.30 (s, 1H), 6.52 (br, 2H), 4.24 (s, 2H), 2.98-3.02 (m, 1H), 1.15 (d, *J*=6.9 Hz, 6H). LCMS (ESI, *m*/*z):* 423 [M+H]⁺.

### Example 31. Synthesis of compound 37

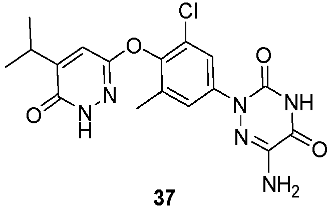

4-Amino-2-chloro-6-methylphenol was prepared similarly as described in WO2004014382 to afford a brown solid (10.8 g, 88%).

3-Chloro-4-[(6-chloro-5-isopropylpyridazin-3-yl)oxy]-5-methylaniline was prepared similarly as described for 3,5-dichloro-4-[(6-chloro-5-isopropyl-pyridazin-3-yl)oxy]-aniline to afford a brown semi-solid (9.5 g, 68%).

6-(4-Amino-2-chloro-6-methylphenoxy)-4-isopropyl-2*H*-pyridazin-3-one was prepared similarly as described for 6-(4-amino-2,6-dichlorophenoxy)-4-isopropyl-2*H-*pyridazin-3-one to afford a pink solid (6.5 g, 68%).

Ethyl (2-(2-(3-chloro-4-((5-isopropyl-6-oxo-1,6-dihydropyridazin-3-yl)oxy)-5-methylphenyl)hydrazineylidene)-2-cyanoacetyl)carbamate was prepared similarly as described for ethyl (2-cyano-2-(2-(4-((3-isopropyl-1-tosyl-1*H*-indol-5-yl)methyl)-3,5-dimethylphenyl)hydrazineylidene)acetyl)carbamate to afford a yellow solid (4.56 g, 75%).

2-[3-Chloro-4-[(5-isopropyl-6-oxo-1*H*-pyridazin-3-yl)oxy]-5-methylphenyl]-3,5-dioxo-4*H*-1,2,4-triazine-6-carbonitrile was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carbonitrile to afford a light yellow solid (2.3 g, 53%).

2-[3-Chloro-4-[(5-isopropyl-6-oxo-1*H*-pyridazin-3-yl)oxy]-5-methylphenyl]-3,5-dioxo-4*H*-1,2,4-triazine-6-carboxylic acid was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid to afford a white solid (880 mg, 75%).

*t*-Butyl-*N*-(2-[3-chloro-4-[(5-isopropyl-6-oxo-1*H*-pyridazin-3-yl)oxy]-5-methylphenyl]-3,5-dioxo-4*H*-1,2,4-triazin-6-yl)-carbamate was prepared similarly as described for *t*-butyl *N*-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate to afford a white solid (790 mg, 70%).

6-Amino-2-[3-chloro-4-[(5-isopropyl-6-oxo-1*H*-pyridazin-3-yl)oxy]-5-methyl-phenyl]-4*H*-1,2,4-triazine-3,5-dione was prepared similarly as described for 6-amino-2-[3,5-dichloro-4-[(5-isopropyl-6-oxo-1*H*-pyridazin-3-yl)oxy]-2-methylphenyl]-4*H*-1,2,4-triazine-3,5-dione to afford a white solid (100 mg, 50%). ¹H NMR (400 MHz, DMSO-*d*₆) δ 12.18 (br, 1H), 12.10 (s, 1H), 7.58-7.61 (m, 1H), 7.51-7.48 (m, 1H), 7.34-7.37 (m, 1H), 6.41 (s, 2H), 3.08-2.99 (m, 1H), 2.18 (s, 3H), 1.19 (d, *J*=6.8 Hz, 6H). LCMS (ESI, *m*/*z*): 405.0 [M+H]⁺.

### Example 32. Synthesis of compound 38

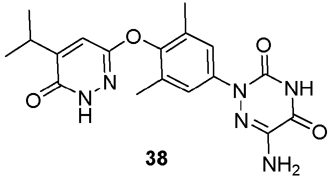

6-Amino-2-[4-[(5-isopropyl-6-oxo-1*H*-pyridazin-3-yl)oxy]-3,5-dimethylphenyl]-4*H*-1,2,4-triazine-3,5-dione was prepared similarly as described for 6-amino-2-(3,5-dichloro-4-((5-isopropyl-6-oxo-1,6-dihydropyridazin-3-yl)oxy)phenyl)-1,2,4-triazine-3,5(2*H*,4*H*)-dione to afford a white solid (238 mg, 54%). ¹H NMR (300 MHz, DMSO-*d*₆) δ 12.10 (br, 1H), 12.00 (br, 1H), 7.30 (s, 1H), 7.25 (s, 2H), 6.32 (br, 2H), 3.01-3.09 (m, 1H), 2.10 (s, 6H), 1.20 (d, *J*=6.6 Hz, 6H). LCMS (ESI, *m*/*z):* 385 [M+H]⁺.

### Example 33. Synthesis of compounds 39-A, and 39-B

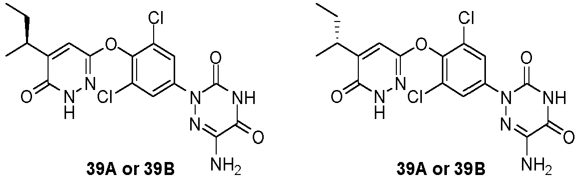

3,6-Dichloro-4-(*sec*-butyl)pyridazine was prepared similarly as described for 3,6-dichloro-4-isopropylpyridazine (see also Samaritoni, J. G. Homolytic alkylation of 3,6-dichloropyridazine. Org. Prep. Proced. Int. 1988, 20, 117-121) to afford a yellow oil (23.3 g, 79%).

To a mixture of 3,6-dichloro-4-(*sec*-butyl)pyridazine (7.00 g, 34.1 mmol) and 4-amino-2,6-dichlorophenol (6.68 g, 37.6 mmol) in DMSO (70 mL) was added K₂CO₃ (14.2 g, 103 mmol) and CuI (1.95 g, 10.2 mmol). The resulting mixture was stirred overnight at 90 °C under N₂. The solids were removed by filtration and the filtrate was quenched with NH₄Cl (sat. aq.,150 mL). The resulting mixture was extracted with EA (3 x 200 mL) and the organic layers were combined, washed with brine (2 x 150 mL), dried over anhyd. Na₂SO₄, the solids were removed by filtration and the filtrate was concentrated under reduced pressure to afford crude product. The sample was purified by silica column chromatography, eluted with EA:PE (0~40% over 20 min). Fractions were collected, combined, and concentrated under reduced pressure to provide 3,5-dichloro-4-[[6-chloro-5-(*sec*-butyl)pyridazin-3-yl]oxy]aniline as a brown semi-solid (10 g, 72%).

A mixture of 3,5-dichloro-4-[[6-chloro-5-(*sec*-butyl)pyridazin-3-yl]oxy]-aniline (10 g, 28.9 mmol) and NaOAc (8.28 g, 101 mmol) in AcOH (100 mL) was stirred overnight at 100 °C. The mixture was concentrated under reduced pressure to remove AcOH. The residue was diluted with water (100 mL) and the pH was adjusted to 8 with NaOH (aq., 1 M). The mixture was extracted with EA (3 x 150 mL) and the organic layers were combined, dried over anhyd. Na₂SO₄, filtered and concentrated under reduced pressure. The residue was dissolved in MeOH (100 mL) and NaOH (100 mL, 1 M aq.) and the resulting mixture was stirred overnight at 100 °C. The mixture was concentrated under reduced pressure to remove MeOH. The resulting mixture was extracted with EA (3 x 150 mL) and the organic layers were combined, washed with brine (2 x 100 mL), dried over anhyd. Na₂SO₄, filtered and concentrated under reduced pressure to afford crude product. The sample was purified by silica column chromatography (EA:PE=0~70% over 20 min). Fractions were collected, combined, and concentrated under reduced pressure to provide 6-(4-amino-2,6-dichlorophenoxy)-4-(*sec*-butyl)-2*H*-pyridazin-3-one as a light yellow solid (5.95 g, 60%).

Ethyl (2-(2-(4-((5-(*sec*-butyl)-6-oxo-1,6-dihydropyridazin-3-yl)oxy)-3,5-dichlorophenyl)hydrazineylidene)-2-cyanoacetyl)carbamate, a yellow solid (4.63 g, 77%), was prepared similarly as described for ethyl (2-cyano-2-(2-(3,5-dichloro-4-((1-tosyl-1*H-*indol-5-yl)oxy)phenyl)-hydrazineylidene)-acetyl) carbamate.

A mixture of ethyl (2-(2-(4-((5-(*sec*-butyl)-6-oxo-1,6-dihydropyridazin-3-yl)oxy)-3,5-dichlorophenyl)hydrazineylidene)-2-cyanoacetyl)carbamate (4.40 g, 8.88 mmol) and NaOAc (3.64 g, 44.4 mmol) in AcOH (50 mL) was stirred for 2 h at 120 °C. The mixture was poured into water (100 mL) and the crude product was obtained by filtration. The sample was purified by column chromatography (CH₃OH: DCM=0~15% over 20 min). Fractions were collected, combined, and concentrated under reduced pressure to provide 2-(3,5-dichloro-4-[[6-oxo-5-(*sec*-butyl)-1*H*-pyridazin-3-yl]oxy]phenyl)-3,5-dioxo-4*H*-1,2,4-triazine-6-carbonitrile as a yellow solid (2.27 g, 52%).

A mixture of 2-(3,5-dichloro-4-[[6-oxo-5-(*sec*-butyl)-1H-pyridazin-3-yl]oxy]phenyl)-3,5-dioxo-4*H*-1,2,4-triazine-6-carbonitrile (1.00 g, 2.23 mmol) in conc. HCl (5 mL) and AcOH (10 mL) was stirred for 2 h at 120 °C. The mixture was poured into water (20 mL) and the desired product 2-(3,5-dichloro-4-[[6-oxo-5-(*sec*-butyl)-1*H*-pyridazin-3-yl]oxy]phenyl)-3,5-dioxo-4*H*-1,2,4-triazine-6-carboxylic acid was obtained by filtration as a white solid (860 mg, 78%).

To a mixture of 2-(3,5-dichloro-4-[[6-oxo-5-(*sec*-butyl)-1*H*-pyridazin-3-yl]oxy]phenyl)-3,5-dioxo-4*H*-1,2,4-triazine-6-carboxylic acid (860 mg, 1.84 mmol) in tBuOH (15.0 mL) was added DPPA (1.52 g, 5.52 mmol) and NEt₃ (743 mg, 7.35 mmol). The resulting mixture was stirred overnight at 85 °C under N₂, then concentrated under reduced pressure. The residue was diluted with DCM (50 mL), washed with brine (30 mL), dried over anhyd. Na₂SO₄, the solids were removed by filtration and the filtrate was concentrated under reduced pressure to afford crude product that was purified by column chromatography and eluted with MeOH:DCM (0~7% over 20 min). Fractions were concentrated under reduced pressure to provide racemic *t*-butyl-*N*-[2-(3,5-dichloro-4-[[6-oxo-5-(*sec*-butyl)-1*H-*pyridazin-3-yl]oxy]-phenyl)-3,5-dioxo-4*H*-1,2,4-triazin-6-yl]carbamate as a white solid (700 mg, 67%).

*t*-Butyl-*N*-[2-(3,5-dichloro-4-[[6-oxo-5-(*sec*-butyl)-1*H*-pyridazin-3-yl]-oxy]phenyl)-3,5-dioxo-4*H*-1,2,4-triazin-6-yl]carbamate (500 mg, 0.927 mmol) was further purified by preparatory SFC-HPLC (Column: Reg-AD, 30 x 250mm, 5 µm; Mobile Phase A: CO₂, Mobile Phase B: EtOH (8 mmol/L NH₃.MeOH)-HPLC; Flow rate: 50 mL/min; Gradient: 50% B; 220 nm; Injection Volume: 4 mL; Number Of Runs:11). Purification resulted in enantiomer 1 as white solid (130 mg, 26%, Rt: 5.79 min), and enantiomer 2 as a white solid (Rt: 6.97 min, 190 mg, 37%).

The boc group of each enantiomer was separately deprotected in DCM (10 mL) and TFA (3 mL). The resulting mixtures were stirred for 4 h at rt and concentrated under reduced pressure. The solvent was removed under reduced pressure, the mixtures were diluted with EA (30 mL), washed with NaHCO₃ (sat., aq., 20 mL), dried over anhyd. Na₂SO₄, the solids were removed by filtration and the filtrate was concentrated under reduced pressure to afford the crude product.

Compound 39-A (corresponding to enantiomer 1) was purified by preparative HPLC (Column: Xselect CSH OBD Column 30 x 150 mm 5 µm; Mobile Phase A:Water (10 mmol/L NH₄HCO₃+0.1%NH₃.H₂O), Mobile Phase B: ACN; Flow rate:60 mL/min; Gradient: 23 B to 43 B in 7 min; 220 nm; RT: 5.32 min) to afford a white solid (49.6 mg, 46%). ¹H NMR (300 MHz, DMSO-*d*₆) δ 11.95 - 12.52 (m, 2H), 7.86 (s, 2H), 7.42 (s, 1H), 6.52 (s, 2H), 2.86 - 2.94 (m, 1H), 1.64 - 1.74 (m, 1H), 1.45 - 1.56 (m, 1H), 1.17 (d, *J* = 6.9 Hz, 3H), 0.86 (t, *J =* 7.5 Hz, 3H). LCMS (ESI, *m*/*z):* 439.0 [M+H]⁺.

Compound 39-B (corresponding to enantiomer 2) was triturated with EA:MeOH (5 mL:1 mL) to provide the desired product as an off-white solid (67.5 mg, 42%). ¹H NMR (300 MHz, DMSO-*d*₆) δ 12.19-12.28 (m, 2H), 7.86 (s, 2H), 7.42 (s, 1H), 6.53 (s, 2H), 2.83-2.94 (m, 1H), 1.61-1.77 (m, 1H), 1.43-1.58 (m, 1H), 1.18 (d, *J*=6.9 Hz, 3H), 0.86 (t, *J*=7.4 Hz, 3H). LCMS (ESI, *m*/*z*): 439.0 [M+H]⁺.

SFC Analysis on compounds 39-A, and 39-B: Column name: Enantiocel C3-3, 4.6 x 100 mm, 3 µm. Co-Solvent: 20% EtOH (0.1%DEA). Flow (mL/min): 4. Temperature: 35°C. Detector: 220nm. 39-A: Rt = 2.947 min. 39-B: Rt = 3.269 min.

### Example 34. Synthesis of compounds 40-A and 40

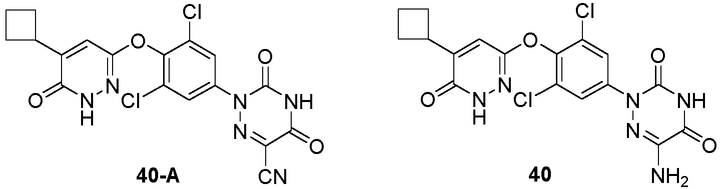

3,6-dichloro-4-cyclobutylpyridazine was prepared similarly as described for 3,6-dichloro-4-isopropylpyridazine to afford a colorless oil (12 g, 38%).

3,5-dichloro-4-[(6-chloro-5-cyclobutylpyridazin-3-yl)oxy]aniline was prepared similarly as described for 3,5-dichloro-4-[[6-chloro-5-(*sec*-butyl)pyridazin-3-yl]oxy]aniline to afford a yellow solid (3.07 g, 34%).

A stirred mixture of 3,5-dichloro-4-[(6-chloro-5-cyclobutylpyridazin-3-yl)oxy]aniline (2.52 g, 7.31 mmol), NaOAc (2.10 g, 25.6 mmol) in AcOH (25 mL) was stirred overnight at 100 °C. The resulting mixture was concentrated under reduced pressure to remove AcOH. The residue was diluted with water (30 mL) and the pH was adjusted to 8 with NaOH (aq., 1 M). The mixture was extracted with EA (3 x 60 mL) and the organic layers were combined, dried over anhyd. Na₂SO₄, the solids were removed by filtration and the filtrate was concentrated under reduced pressure. The residue was dissolved in MeOH (25 mL) and NaOH solution (25 mL, 1 M aq.) and the resulting mixture was stirred overnight at 120 °C. The mixture was concentrated under reduced pressure. The resulting mixture was extracted with EA (3 x 60 mL), the organic layers were combined, washed with brine (2 x 40 mL), dried over anhyd. Na₂SO₄, the solids were removed by filtration and the filtrate was concentrated under reduced pressure to afford 6-(4-amino-2,6-dichlorophenoxy)-4-cyclobutyl-2*H*-pyridazin-3-one (2.27 g, crude) as a brown solid.

Ethyl (2-cyano-2-(2-(3,5-dichloro-4-((5-cyclobutyl-6-oxo-1,6-dihydro-pyridazin-3-yl)oxy)phenyl)hydrazineylidene)acetyl)carbamate (2.61 g, crude yellow solid) was prepared similarly as described for ethyl (2-cyano-2-(2-(3,5-dichloro-4-((1-tosyl-1*H-*indol-5-yl)oxy)phenyl)-hydrazineylidene)-acetyl) carbamate.

To a stirred mixture of ethyl (2-cyano-2-(2-(3,5-dichloro-4-((5-cyclobutyl-6-oxo-1,6-dihydropyridazin-3-yl)oxy)phenyl)hydrazineylidene)acetyl)carbamate (2.50 g, 5.07 mmol) in DMA (25 mL) was added KOAc (1.99 g, 20.3 mmol) in portions at rt. The resulting mixture was stirred for 5 h at 120 °C under N₂. The reaction was quenched with water (200 mL) and extracted with EA (3 x 150 mL), and the organic layers were combined, dried over anhyd. Na₂SO₄, the solids were removed by filtration and the filtrate was concentrated under reduced pressure. The crude product was purified by silica gel column chromatography, eluted with DCM:MeOH (9:1) to afford 2-[3,5-dichloro-4-[(5-cyclobutyl-6-oxo-1*H-*pyridazin-3-yl)oxy]phenyl]-3,5-dioxo-4*H*-1,2,4-triazine-6-carbonitrile as a brown solid (compound 40-A, 2.1 g, 84%). ¹H NMR (300 MHz, DMSO-*d*₆) δ 13.27 (br, 1H), 12.19 (s, 1H), 7.79 (s, 2H), 7.49 - 7.51 (m, 1H), 3.50 - 3.62 (m, 1H), 1.93-2.34(m, 5H), 1.75 - 1.88 (m, 1H).

To a stirred mixture of 2-[3,5-dichloro-4-[(5-cyclobutyl-6-oxo-1*H*-pyridazin-3-yl)oxy]phenyl]-3,5-dioxo-4*H*-1,2,4-triazine-6-carbonitrile (1.30 g, 2.91 mmol) in AcOH (12 mL) was added HCl (6 mL) dropwise at rt. The resulting mixture was stirred for 2 h at 120 °C. The resulting mixture was concentrated under reduced pressure to remove AcOH. The pH of the mixture was adjusted to 8 with Na₂CO₃ (sat., aq.). The resulting mixture was extracted with EA (3x50 mL). The pH of the mixture was adjusted to 5 with HCl (1 M). The resulting mixture was extracted with EA (3 x 50 mL) and the organic layers were combined, dried over anhyd. Na₂SO₄, the solids were removed by filtration and the filtrate was concentrated under reduced pressure to afford 2-[3,5-dichloro-4-[(5-cyclobutyl-6-oxo-1*H*-pyridazin-3-yl)oxy]-phenyl]-3,5-dioxo-4*H*-1,2,4-triazine-6-carboxylic acid (1.0 g, crude) as a yellow solid. The crude product was used in the next step without further purification.

To a stirred solution of 2-[3,5-dichloro-4-[(5-cyclobutyl-6-oxo-1*H*-pyridazin-3-yl)oxy]phenyl]-3,5-dioxo-4*H*-1,2,4-triazine-6-carboxylic acid (800 mg, 1.72 mmol) in *t-*BuOH (30 mL) was added DPPA (1.42 g, 5.15 mmol) and NEt₃ (694 mg, 6.86 mmol) dropwise at rt. The resulting mixture was stirred overnight at 85 °C and concentrated under reduced pressure. The residue was diluted with EA (50 mL) and washed with brine (40 mL). The organic layers were combined was dried over anhyd. Na₂SO₄, filtered and concentrated under reduced pressure. The residue was purified by silica gel column chromatography with PE:EA (4:1) to afford *t*-butyl-*N*-(2-[3,5-dichloro-4-[(5-cyclobutyl-6-oxo-1*H*-pyridazin-3-yl)oxy]phenyl]-3,5-dioxo-4*H*-1,2,4-triazin-6-yl)-carbamate as a yellow solid (518 mg, 53%).

To a stirred solution of *t*-butyl-*N*-(2-[3,5-dichloro-4-[(5-cyclobutyl-6-oxo-1*H-*pyridazin-3-yl)oxy]phenyl]-3,5-dioxo-4*H*-1,2,4-triazin-6-yl)carbamate (450 mg, 0.837 mmol) in DCM (5 mL) was added TFA (2.50 mL) dropwise at 0 °C. The resulting mixture was stirred for 3 h at rt and concentrated under reduced pressure. The residue was diluted with DCM (30 mL), washed with NaHCO₃ (sat., aq., 40 mL), dried over anhyd. Na₂SO₄, the solids were removed by filtration and the filtrate was concentrated under reduced pressure. The crude product was purified by preparative HPLC to afford 6-amino-2-[3,5-dichloro-4-[(5-cyclobutyl-6-oxo-1*H*-pyridazin-3-yl)oxy]phenyl]-4*H*-1,2,4-triazine-3,5-dione as a white solid (89.4 mg, 24%). ¹H NMR (300 MHz, DMSO-*d*₆) δ 12.27 (s, 1H), 12.15 (s, 1H), 7.85 (s, 2H), 7.46-7.47 (m, 1H), 6.52 (s, 2H), 3.50-3.62 (m, 1H), 1.96-2.34 (m, 5H), 1.80-1.87 (m, 1H). LCMS (ESI, *m*/*z):* 459 [M+Na]⁺ .

### Example 35. Synthesis of compound 41

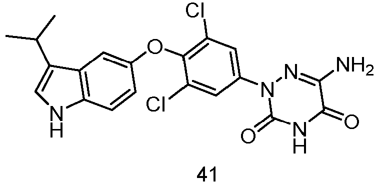

2-[3,5-dichloro-4-[(3-isopropyl-1*H*-indol-5-yl)oxy]-phenyl]-3,5-dioxo-4*H-*1,2,4-triazine-6-carboxylic acid was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid, with the exception that mixture was stirred for 2 h at 100 °C to afford a brown solid (200 mg, crude).

*t*-butyl-*N*-(2-[3,5-dichloro-4-[(3-isopropyl-1*H*-indol-5-yl)oxy]phenyl]-3,5-dioxo-4*H*-1,2,4-triazin-6-yl)carbamate was prepared, from 2-(3,5-dichloro-4-((3-isopropyl-1H-indol-5-yl)oxy)phenyl)-3,5-dioxo-2,3,4,5-tetrahydro-1,2,4-triazine-6-carboxylic acid, similarly as described for *t*-butyl *N*-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate (from 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carboxylic acid) to afford a yellow solid (117 mg, crude).

6-amino-2-[3,5-dichloro-4-[(3-isopropyl-1*H*-indol-5-yl)oxy]phenyl]-4*H*-1,2,4-triazine-3,5-dione (compound 41) was prepared similarly as described for 6-amino-2-[3,5-dichloro-4-[(5-isopropyl-6-oxo-1*H*-pyridazin-3-yl)oxy]-2-methylphenyl]-4*H*-1,2,4-triazine-3,5-dione to afford a white solid (13 mg, 17%). ¹H NMR (300 MHz, MeOH-*d*₄) 7.88 (s, 2H), 7.25-7.29 (m, 1H), 7.01 (s, 1H), 6.85-6.86 (m, 1H), 6.73-6.77 (m, 1H), 2.98-3.08 (m, 1H), 1.28-1.36 (m, 6H).

### Example 36. Synthesis of compound 42

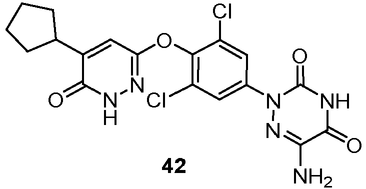

6-amino-2-(3,5-dichloro-4-((5-cyclopentyl-6-oxo-1,6-dihydropyridazin-3-yl)oxy)phenyl)-1,2,4-triazine-3,5(2*H*,4*H*)-dione was prepared similarly as described for 6-amino-2-(3,5-dichloro-4-((5-cyclobutyl-6-oxo-1,6-dihydropyridazin-3-yl)oxy)-phenyl)-1,2,4-triazine-3,5(2*H*,4*H*)-dione to afford a white solid (101 mg, 51%).

¹H NMR (300 MHz, DMSO-*d*₆) δ 12.21 (br, 1H), 12.17 (br, 1H), 7.85 (s, 2H), 7.44 (s, 1H), 6.52 (br,2H), 3.03-3.33 (m, 1H), 1.90-1.98 (m, 2H), 1.50-1.78 (m, 6H). LCMS (ESI, *m*/*z):* 451 [M+H]⁺.

### Example 37. Synthesis of compound 43

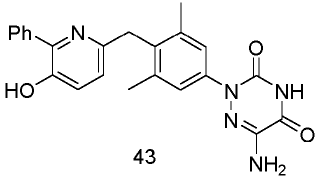

6-(4-amino-2,6-dimethylbenzyl)-2-phenylpyridin-3-ol was prepared similarly as described in WO 2010122980, and JP 2012106996.

6-amino-2-[4-[(5-hydroxy-6-phenylpyridin-2-yl)-methyl]-3,5-dimethylphenyl]-4*H*-1,2,4-triazine-3,5-dione was prepared from 6-(4-amino-2,6-dimethylbenzyl)-2-phenylpyridin-3-ol similarly as described in the conversion of 6-(4-amino-2,6-dichlorophenoxy)-4-isopropylpyridazin-3(2H)-one to prepare 6-amino-2-(3,5-dichloro-4-((5-isopropyl-6-oxo-1,6-dihydropyridazin-3-yl)oxy)phenyl)-1,2,4-triazine-3,5(2H,4H)-dione to afford a white solid (43.8 mg, 49%). ¹H NMR (300 MHz, DMSO-*d*₆) δ 9.32-9.89 (m, 2H), 7.99 (d, *J*=7.2 Hz, 2H), 7.31-7.43 (m, 3H), 7.19-7.24 (m, 3H), 6.76 (d, *J*=8.1 Hz, 1H), 6.21 (s, 2H), 4.09 (s, 2H), 2.11 (s, 6H). LCMS (ESI, *m*/*z):* 416 [M+H]⁺.

### Example 38. Synthesis of compound 44

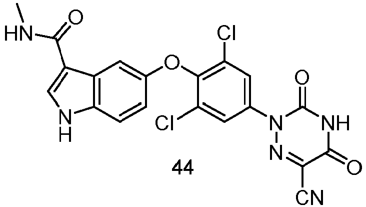

5-[2,6-dichloro-4-(6-cyano-3,5-dioxo-4*H*-1,2,4-triazin-2-yl)phenoxy]-*N-*methyl-1-(4-methylbenzenesulfonyl)indole-3-carboxamide was prepared similarly as described for 5-[2,6-dichloro-4-(6-cyano-3,5-dioxo-4*H*-1,2,4-triazin-2-yl)phenoxy]-*N,N-*dimethyl-1-(4-methylbenzenesulfonyl)indole-3-carboxamide to afford a light brown solid (200 mg, 50%).

Subsequent tosyl group deprotection was performed similarly as described for the formation of 5-[2,6-dichloro-4-(6-cyano-3,5-dioxo-4*H*-1,2,4-triazin-2-yl)phenoxy]-*N,N-*dimethyl-1*H*-indole-3-carboxamide to afford 5-[2,6-dichloro-4-(6-cyano-3,5-dioxo-4*H-*1,2,4-triazin-2-yl)phenoxy]-*N*-methyl-1*H*-indole-3-carboxamide as a yellow solid (21.5 mg, 14%). ¹H NMR (300 MHz, MeOH-*d*₄) δ 7.85 (s, 1H), 7.78 (s, 2H), 7.52 (d, *J*=2.4 Hz, 1H), 7.41 (d, *J*=8.7 Hz, 1H), 6.91-6.94 (m, 1H), 2.87 (s, 3H). LCMS (ESI, *m*/*z):* 471 [M+H]⁺.

### Example 39. Synthesis of compound 45

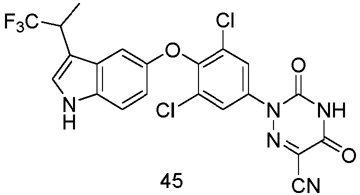

A 50 mL round-bottom flask was charged with 5-(2,6-dichloro-4-nitrophenoxy)-1-(4-methylbenzenesulfonyl)indole (1.00 g, 2.10 mmol), NIS (0.71 g, 3.14 mmol), p-toluenesulfonic acid (0.05 g, 0.314 mmol), DCM (30 mL). The reaction stirred for 5 h at rt and then quenched with water (150 mL). The resulting mixture was extracted with DCM (3 x 200 mL) and the organic layers were combined, washed with brine (1 x 200 mL), dried over anhyd. Na₂SO₄, filtered and concentrated under reduced pressure. The residue was purified on a silica gel column with EA:PE (1:4) to provide 5-(2,6-dichloro-4-nitrophenoxy)-3-iodo-1-(4-methylbenzenesulfonyl)indole as a yellow solid (580 mg, 41%).

A 50 mL round-bottom was charged with 5-(2,6-dichloro-4-nitrophenoxy)-3-iodo-1-(4-methylbenzenesulfonyl)indole (350 mg, 0.580 mmol), 4,4,6-trimethyl-2-(3,3,3-trifluoroprop-1-en-2-yl)-1,3,2-dioxaborinane (155 mg, 0.696 mmol), PdCl₂(dppf) (42.5 mg, 0.058 mmol), K₂CO₃ (241 mg, 1.74 mmol), ethylene glycol dimethyl ether (10 mL), and water (2 mL). The resulting solution stirred overnight under N₂ at 80 °C and was quenched with water (10 mL), then extracted with EA (3 x 30 mL) and the organic layers were combined, washed with brine (2 x 10 mL), dried over anhyd. Na₂SO₄, the solids were removed by filtration and the filtrate was concentrated under reduced pressure. The residue was purified on a silica gel column with EA:PE (1:4) to provide of 5-(2,6-dichloro-4-nitrophenoxy)-1-(4-methylbenzene-sulfonyl)-3-(3,3,3-trifluoroprop-1-en-2-yl)indole as a white solid (220 mg, 53%).

A 100 mL round-bottom flask was charged with 5-(2,6-dichloro-4-nitrophenoxy)-1-(4-methylbenzenesulfonyl)-3-(3,3,3-trifluoroprop-1-en-2-yl)indole (210 mg, 0.368 mmol), Pd/C (200 mg), and EA (20 mL) under hydrogen. The mixture was stirred for 3 h at rt. The reaction mixture was diluted with EA (60 mL) and filtered through celite, the celite pad was washed with EA (2 x 10 mL), the filtrate was concentrated under reduced pressure to afford the 3,5-dichloro-4-[[1-(4-methylbenzene-sulfonyl)-3-(1,1,1-trifluoropropan-2-yl)indol-5-yl]oxy]aniline as a yellow solid (155 mg crude).

Ethyl (2-cyano-2-(2-(3,5-dichloro-4-((1-tosyl-3-(1,1,1-trifluoropropan-2-yl)-1*H*-indol-5-yl)oxy)phenyl)hydrazineylidene)acetyl)carbamate, an orange solid (110 mg, 54%) was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3, 5-dichloro-4-[[1-(4-methylbenzenesulfonyl)-3-(1,1,1-trifluoropropan-2-yl)indol-5-yl]oxy]phenyl)-3,5-dioxo-4*H*-1,2,4-triazine-6-carbonitrile, a yellow solid (80 mg, 54%) was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile with the exception that the reaction duration was 5h.

Subsequent tosyl group deprotection via TBAF to afford 2-(3,5-dichloro-4-[[3-(1,1,1-trifluoropropan-2-yl)-1*H*-indol-5-yl]oxy]phenyl)-3,5-dioxo-4*H*-1,2,4-triazine-6-carbonitrile as a light-yellow solid (18.9 mg, 26%) was performed similarly as described for 2-[4-[(3-isopropyl-1*H*-pyrrolo[3,2-*b*]pyridin-5-yl)methyl]-3,5-dimethyl-phenyl]-3,5-dioxo-1,2,4-triazine-6-carbonitrile. ¹H NMR (300 MHz, DMSO-*d*₆) δ 11.20 (br, 1H), 7.81 (s, 2H), 7.34-7.42 (m, 2H), 7.04 (s, 1H), 6.69-6.72 (m, 1H), 3.92-3.97 (m, 1H), 1.46 (d, J=7.2 Hz, 3H). LCMS (ESI, *m*/*z*): 508[M-H]⁻.

### Example 40. Synthesis of compound 46

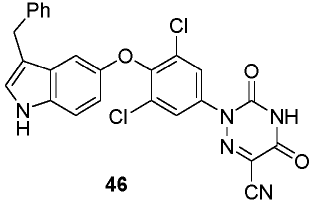

To a solution of 5-(2,6-dichloro-4-nitrophenoxy)-1*H*-indole (3.00 g, 9.29 mmol) in DCM (25 mL) was added diethyl aluminum chloride (15 mL, 0.9 M in toluene, 13.5 mmol) dropwise at 0 °C. The mixture was stirred at 0 °C for 30 min, then benzoyl chloride (1.96 g, 13.9 mmol) was added at 0 °C. The reaction mixture was stirred at 0 °C for 3 h and quenched with water (25 mL). The resulting mixture was extracted with DCM (3 x 20 mL). The organic layers were combined, washed with brine (3 x 20 mL), dried over anhyd. Na₂SO₄, the solids were removed by filtration and the filtrate was concentrated under reduced pressure. The residue was purified on a silica gel column with PE:EA (4:1) to afford 3-benzoyl-5-(2,6-dichloro-4-nitro-phenoxy)-1*H*-indole as a light brown solid (1.3 g, 26%).

To a solution of 3-benzoyl-5-(2,6-dichloro-4-nitrophenoxy)-1*H*-indole (1.3 g, 3.04 mmol) in THF (30 mL) was added LiAlH₄ (462 mg, 12.2 mmol) at 0 °C under N₂. The reaction mixture was stirred overnight at 60 °C and quenched with water (30 mL) at 0 °C. To the resulting solution was added NaOH (1N, 30 mL) and followed by water (30 mL). The solids were removed by filtration, and the filter cake was washed with water (3 x 10 mL). The filtrate was concentrated under reduced pressure. The residue was purified by preparatory TLC with PE:EA (1:1) to afford 4-[(3-benzyl-1*H*-indol-5-yl)oxy]-3,5-dichloroaniline as a light yellow solid (250 mg, 21%).

Ethyl (2-(2-(4-((3-benzyl-1*H*-indol-5-yl)oxy)-3,5-dichlorophenyl)-hydrazineylidene)-2-cyanoacetyl)carbamate (300 mg, 52%) was prepared similarly as described for ethyl (2-cyano-2-(2-(3,5-dichloro-4-((1-tosyl-1*H*-indol-5-yl)oxy)-phenyl)-hydrazineylidene)-acetyl) carbamate.

2-[4-[(3-benzyl-1*H*-indol-5-yl)oxy]-3,5-dichlorophenyl]-3,5-dioxo-4*H*-1,2,4-triazine-6-carbonitrile, a light brown solid was prepared similarly as described for 2-(4-[[3-isopropyl-1-(4-methylbenzene-sulfonyl)indol-5-yl]oxy]-3,5-dimethyl-phenyl)-3,5-dioxo-4H-1,2,4-triazine-6-carbonitrile with the exception that the reaction duration was 5h instead of 2h (13.3 mg, 7%). ¹HNMR (300 MHz, MeOH-*d*₄) δ 7.72 (s, 2H), 7.28 (d, *J*=9.0 Hz, 1H), 7.10-7.23 (m, 5H), 7.03 (s, 1H), 6.77-6.80 (m, 1H), 6.54 (d, *J*=2.4 Hz, 1H), 3.98 (s, 2H). LCMS (ESI, *m*/*z*):502 [M-H]⁻.

### Example 41. Synthesis of compound 47

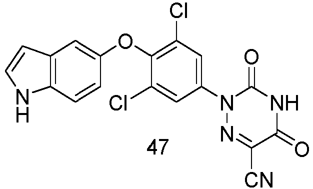

2-[3,5-dichloro-4-(1*H*-indol-5-yloxy)phenyl]-3,5-dioxo-4*H*-1,2,4-triazine-6-carbonitrile, a yellow solid (120 mg, 32%). ¹H NMR (300 MHz, MeOH-*d*₄) δ 7.77 (s, 2 h), 7.33-7.37 (m, 1H), 7.24 (d, *J*=3.0 Hz, 1H), 6.80-6.84 (m, 2 h), 6.35-6.36 (m, 1H). LCMS (ESI, *m*/*z):* 412 [M-H]⁻.

### Example 42. Synthesis of compound 48

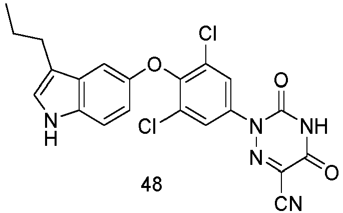

A 50 mL round-bottom was charged with 5-(2,6-dichloro-4-nitrophenoxy)-1*H-*indole (2.00 g, 6.19 mmol), DCM (10.0 mL). SnCl₄ (1.93 g, 7.42 mmol) was added at 0 °C. The reaction mixture stirred at rt for 30 min. Propanoyl chloride (1.14 g, 12.3 mmol) was added, followed by CH₃NO₂ (15 mL). The reaction mixture was stirred overnight at rt and quenched with water (10 mL). The resulting mixture was extracted with EA (3 x 200 mL) and the organic layers were combined, washed with brine (100 mL), dried over anhyd. Na₂SO₄, filtered and concentrated under reduced pressure. The residue was purified on a silica gel column with EA:PE (9:1) to provide 1-[5-(2,6-dichloro-4-nitrophenoxy)-1*H*-indol-3-yl]propan-1-one as a yellow solid (1.6 g, 68.17%).

A 50 mL round-bottom flask was charged with 1-[5-(2,6-dichloro-4-nitrophenoxy)-1*H*-indol-3-yl]propan-1-one (1.90 g, 5.01 mmol), THF (20 mL). NaH (0.802 g, 20.0 mmol, 60% in mineral oil) was added at 0 °C. 4-toluene sulfonyl chloride (1.91 g, 10.0 mmol) was added at 0 °C. The resulting solution was stirred at rt for 2 h and quenched with NaHCO₃ (sat. aq.,30 mL). The resulting mixture was extracted with EA (3 x 80 mL) and the organic layers were combined, washed with brine (2 x 40 mL), dried over anhyd. Na₂SO₄, the solids were removed by filtration and the filtrate was concentrated under reduced pressure. The crude was purified on a silica gel column with EA:PE (1:5) to provide 1-[5-(2,6-dichloro-4-nitrophenoxy)-1-(4-methyl-benzenesulfonyl)-indol-3-yl]propan-1-one as a yellow solid (1.1 g, 41%).

NBH₄ (0.78 g, 20.6 mmol) was added to TFA (10 mL) at 0 °C under N₂. Then a solution of 1-[5-(2,6-dichloro-4-nitrophenoxy)-1-(4-methylbenzene-sulfonyl)indol-3-yl]propan-1-one (1.10 g, 2.06 mmol) in DCM (30 mL) was added to the mixture. The reaction mixture stirred overnight at rt and quenched with water (50 mL), then extracted with DCM (3 x 150 mL) and the organic layers were combined, washed with brine (1 x 100 mL), dried over anhyd. Na₂SO₄, the solids were removed by filtration and the filtrate was concentrated under reduced pressure to provide 1-[5-(2,6-dichloro-4-nitrophenoxy)-1*H*-indol-3-yl]propan-1-one as a yellow solid (1.1 g, 99%).

3,5-dichloro-4-[[1-(4-methylbenzenesulfonyl)-3-propylindol-5-yl]-oxy]aniline, a yellow solid (870 mg, 74%) was prepared similarly as described for 3,5-dichloro-4-[[3-isopropyl-1-(4-methy-lbenzenesulfonyl)-indol-5-yl]oxy]aniline with the exception that the reaction stirred overnight at 50°C.

Ethyl (2-cyano-2-(2-(3,5-dichloro-4-((3-propyl-1-tosyl-1*H*-indol-5-yl)oxy)phenyl)hydrazineylidene)acetyl)carbamate , an orange solid (850 mg, 48%) was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3,5-dichloro-4-[[1-(4-methylbenzenesulfonyl)-3-propylindol-5-yl]oxy]phenyl)-3,5-dioxo-4*H*-1,2,4-triazine-6-carbonitrile, a white solid (400 mg, 48%), was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile with the exception that the reaction duration was 5h instead of 2h.

Subsequent tosyl group deprotection via TBAF afford 2-[3,5-dichloro-4-[(3-propyl-1H-indol-5-yl)oxy]phenyl]-3,5-dioxo-4*H*-1,2,4-triazine-6-carbonitrile as an orange solid (51.7 mg, 20%) was performed similarly as described for 2-[4-[(3-isopropyl-1*H-*pyrrolo[3,2-*b*]pyridin-5-yl)methyl]-3,5-dimethyl-phenyl]-3,5-dioxo-1,2,4-triazine-6-carbonitrile. ¹H NMR (400 MHz, DMSO-*d*₆) δ 10.79 (s, 1H), 7.80 (s, 2H), 7.30 (d, *J*=8.8 Hz, 1H), 7.14 (s, 1H), 6.85 (d, *J*=2.4 Hz, 1H), 6.65-6.68 (m, 1H), 2.52-2.57 (m, 2H), 1.54-1.64 (m, 2H), 0.90 (t, *J*=7.2 Hz, 3H). LCMS (ESI, *m*/*z):* 454 [M-H]⁻.

### Example 43. Synthesis of compound 49

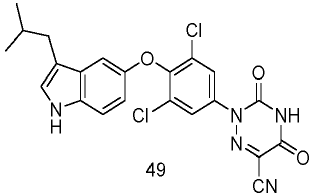

To a stirred solution of 5-(2,6-dichloro-4-nitrophenoxy)-1*H*-indole (2.00 g, 6.19 mmol) in 1,2-dichloroethane (80 mL) was added InBr₃ (0.22 g, 0.619 mmol) and isobutyryl chloride (0.99 g, 9.28 mmol) at 0 °C under N₂. The reaction mixture was stirred for 2 h at 85 °C, then quenched with water (20 mL). The solution was extracted with EA (3 x 200 mL) and the organic layers were combined, dried over MgSO₄, filtered and concentrated under reduced pressure. The residue was purified on a silica gel column with PE:EA (3: 1) to afford 1-[5-(2,6-dichloro-4-nitrophenoxy)-1*H*-indol-3-yl]-2-methylpropan-1-one as a light brown solid (1.5 g, 56%).

1-[5-(2,6-Dichloro-4-nitrophenoxy)-1-(4-methyl-benzenesulfonyl)-indol-3-yl]-2-methylpropan-1-one,a light yellow solid (1.3 g, 70%) was prepared similarly as described for 1-[5-(2,6-dichloro-4-nitrophenoxy)-1-(4-methyl-benzenesulfonyl)-indol-3-yl]propan-1-one.

5-(2,6-Dichloro-4-nitrophenoxy)-1-(4-methylbenzenesulfonyl)-3-(2-methylpropyl)indole,a light brown solid (1.2 g, 85%), was prepared similarly as described for 1-[5-(2,6-dichloro-4-nitrophenoxy)-1*H*-indol-3-yl]propan-1-one with the exception that, after quenching with water, the reaction was neutralized by the addition of NaOH solution at 0 °C.

3,5-Dichloro-4-[[1-(4-methyl-benzenesulfonyl)-3-(2-methylpropyl)indol-5-yl]oxy]aniline as a light yellow solid (1.2 g, 95%) was prepared similarly as described for 3,5-dichloro-4-[[3-isopropyl-1-(4-methy-lbenzenesulfonyl)-indol-5-yl]oxy]aniline.

Ethyl (2-cyano-2-(2-(3,5-dichloro-4-((3-isobutyl-1-tosyl-1*H*-indol-5-yl)oxy)phenyl)hydrazineylidene)acetyl)carbamate, a red solid (860 mg, 48%), was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3,5-Dichloro-4-[[1-(4-methylbenzenesulfonyl)-3-(2-methylpropyl)indol-5-yl]oxy]phenyl)-3,5-dioxo-4*H*-1,2,4-triazine-6-carbonitrile, a red solid (600 mg, 56%) was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

Tosyl group deprotection to afford 2-(3,5-dichloro-4-[[3-(2-methylpropyl)-1*H-*indol-5-yl]oxy]phenyl)-3,5-dioxo-4*H*-1,2,4-triazine-6-carbonitrile as an orange solid (51.2 mg, 22%) was prepared similarly as described for 2-[3,5-dichloro-4-(1*H*-indol-5-yloxy)phenyl]-3,5-dioxo-4*H*-1,2,4-triazine-6-carbonitrile. ¹H NMR (300 MHz, MeOH-*d*₄) δ 7.78 (s, 2H), 7.28 (d, *J*=8.7 Hz, 1H), 7.02 (s, 1H), 6.73-6.83 (m, 2H), 2.51 (d, *J*=7.2 Hz, 2H), 1.83-1.92 (m, 1H), 0.90-1.00 (m, 6H). LC-MS (ESI, *m*/*z):* 468 [M-H]⁻.

### Example 44. Synthesis of compound 50

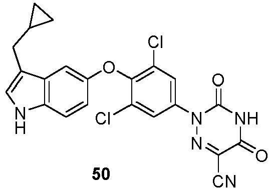

To a solution of 5-(2,6-dichloro-4-nitrophenoxy)-1*H*-indole (2.00 g, 6.19 mmol) in DCM (50 mL) was added SnCl₄ (1.93 g, 7.43 mmol) at 0 °C. The solution warmed to rt and stirred for 30 min, then cyclopropanecarbonyl chloride (0.78 g, 7.43 mmol) was added in small portions to the suspension by syringe, followed by nitromethane (40 mL). The reaction mixture was stirred at rt for 2 h, after which ice water (40 mL) was slowly added. The solids were removed by filtration, and the filtrate was extracted with EA (3 x 50 mL), the organic layers were combined, dried over anhyd. Na₂SO₄, the solids were removed by filtration and the filtrate was concentrated under reduced pressure. The residue was purified on a silica gel column with hexane:EA (3:1) to afford 3-cyclopropanecarbonyl-5-(2,6-dichloro-4-nitrophenoxy)-1*H*-indole as a yellow solid (1.3 g, 48%).

3-Cyclopropanecarbonyl-5-(2,6-dichloro-4-nitrophenoxy)-1-(4-methylbenzenesulfonyl)-indole, a light brown solid (1.8 g, 70%) was prepared similarly as described for 1-[5-(2,6-dichloro-4-nitrophenoxy)-1-(4-methyl-benzenesulfonyl)-indol-3-yl]propan-1-one.

3-(Cyclopropylmethyl)-5-(2,6-dichloro-4-nitrophenoxy)-1-(4-methylbenzenesulfonyl)indole, a light brown solid (1.6 g, 77%) was prepared similarly as described for 1-[5-(2,6-dichloro-4-nitrophenoxy)-1*H*-indol-3-yl]propan-1-one with the exception that, after quenching with water, the reaction was neutralized by the addition of NaOH solution at 0 °C.

3,5-Dichloro-4-[[3-(cyclopropylmethyl)-1-(4-methylbenzenesulfonyl)-indol-5-yl]oxy]aniline, a light yellow solid (1.3 g, 78%) was prepared similarly as described for 3,5-dichloro-4-[[3-isopropyl-1-(4-methy-lbenzenesulfonyl)-indol-5-yl]oxy]aniline.

Ethyl (2-cyano-2-(2-(3,5-dichloro-4-((3-(cyclopropylmethyl)-1-tosyl-1*H*-indol-5-yl)oxy)phenyl)hydrazineylidene)acetyl)carbamate, a red solid (1.18 g, 62%) was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3,5-Dichloro-4-[[3-(cyclopropylmethyl)-1-(4-methylbenzenesulfonyl)indol-5-yl]oxy]phenyl)-3,5-dioxo-4*H*-1,2,4-triazine-6-carbonitrile, a light brown solid (732 mg, 62%), purified via reverse phase chromatography using a C18 column, was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

Tosyl group deprotection to afford 2-(3,5-dichloro-4-[[3-(cyclopropylmethyl)-1H-indol-5-yl]oxy]phenyl)-3,5-dioxo-4*H*-1,2,4-triazine-6-carbonitrile as a orange solid (121 mg, 22%) was prepared similarly as described for 2-[3,5-dichloro-4-(1*H*-indol-5-yloxy)phenyl]-3,5-dioxo-4*H*-1,2,4-triazine-6-carbonitrile. ¹H NMR (300 MHz, MeOH-*d*₄) δ 7.79 (s, 2H), 7.28-7.31 (m, 1H), 7.11 (s, 1H), 6.77-6.85 (m, 2H), 2.57 (d, *J*=6.6 Hz, 2H), 0.95-1.02 (m, 1H), 0.47-0.50 (m, 2H), 0.13-0.16 (m, 2H). LCMS (ESI, *m*/*z):* 466 [M-H]⁻.

### Example 45. Synthesis of compound 51

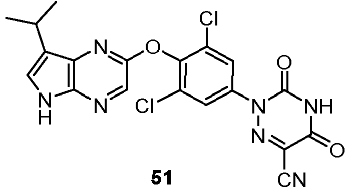

To a stirred solution of 2-bromo-5*H*-pyrrolo[2,3-*b*]pyrazine (14.0 g, 70.7 mmol), Cs₂CO₃ (34.7 g, 106 mmol) in DMF (200 mL) was added [2-(chloro-methoxy)ethyl]trimethylsilane (17.7 g, 106 mmol) dropwise at 0 °C. The mixture was stirred for 2 h at rt and quenched with water (400 mL). The mixture was extracted with EA (3 x 500 mL) and the organic layers were combined, dried over anhyd. Na₂SO₄, the solids were removed by filtration and the filtrate was concentrated under reduced pressure. The residue was purified by silica gel column chromatography with PE:EA (18:1) to afford 2-bromo-5-[[2-(trimethylsilyl) ethoxy]methyl]pyrrolo[2,3-*b*]pyrazine as a yellow solid (21 g, 86%).

To a stirred solution of 2-bromo-5-[[2-(trimethylsilyl)ethoxy]-methyl]pyrrolo[2,3-*b*]pyrazine (20.0 g, 60.9 mmol) and 4-amino-2,6-dichloro-phenol (16.3 g, 91.4 mmol) in DMSO (200 mL) were added K₂CO₃ (25.3 g, 183 mmol) and CuI (4.64 g, 24.4 mmol) at rt. The mixture was stirred for overnight at 90 °C under N₂. The reaction was quenched with sat. NH₄Cl (aq.) and extracted with EA (3 x 800 mL). The organic layers were combined, washed with brine (2 x 600 mL), dried over anhyd. Na₂SO₄, the solids were removed by filtration and the filtrate was concentrated under reduced pressure. The residue was purified by reverse phase column chromatography using a C18 column (ACN:H₂O=10-60% in 50 min) to afford 3,5-dichloro-4-[(5-[[2-(trimethylsilyl)ethoxy]-methyl]pyrrolo[2,3-*b*]pyrazin-2-yl)oxy]aniline as a yellow solid (2.2 g, 8%).

To a stirred solution of 3,5-dichloro-4-[(5-[[2-(trimethylsilyl)ethoxy]-methyl]-pyrrolo[2,3-b]pyrazin-2-yl)oxy]aniline (2.20 g, 5.17 mmol) in CH₃CN (30 mL) was added (dimethoxymethyl)dimethylamine (1.54 g, 12.9 mmol) dropwise at rt. The mixture was stirred for overnight at 80 °C, quenched with water (50 mL), then was extracted with EA (3 x 50 mL) and the organic layers were combined, washed with brine (2 x 40 mL), dried over anhyd. Na₂SO₄, filtered and concentrated under reduced pressure. The residue was purified by silica gel column chromatography with PE:EA (1:1) to afford *N*-[3,5-dichloro-4-[(5-[[2-(trimethyl-silyl)ethoxy]methyl]pyrrolo-[2,3-*b*]pyrazin-2-yl)oxy]phenyl]-*N,N-*dimethylmethanimid-amide as a brown oil (1.875 g, 72%).

To a stirred solution of *N*-[3,5-dichloro-4-[(5-[[2-(trimethylsilyl)ethoxy]-methyl]pyrrolo[2,3-*b*]pyrazin-2-yl)oxy]phenyl]-*N,N*-dimethylmethanimidamide (1.83 g, 3.81 mmol) in DCM (20 mL) was added NIS (1.11 g, 4.95 mmol) and *p*-toluenesulfonic acid (0.20 g, 1.16 mmol) in portions at 0 °C. The reaction mixture was stirred for 4 h at rt and quenched with water (50 mL). The mixture was extracted with DCM (3 x 50 mL) and the organic layers were combined, washed with brine (2 x 40 mL), dried over anhyd. Na₂SO₄, the solids were removed by filtration and the filtrate was concentrated under reduced pressure. The residue was purified by silica gel column chromatography with PE:EA (4: 1) to afford *N-*[3,5-dichloro-4-[(7-iodo-5-[[2-(trimethylsilyl)ethoxy]methyl]-pyrrolo[2,3-*b*]pyrazin-2-yl)oxy]phenyl]-*N,N*-dimethylmethanimidamide as a brown oil (1.33 g, 55%).

To a stirred mixture of *N*-[3,5-dichloro-4-[(7-iodo-5-[[2-(trimethylsilyl)-ethoxy]methyl]pyrrolo[2,3-*b*]pyrazin-2-yl)oxy]phenyl]-*N,N*-dimethyl-methanimidamide (980 mg, 1.62 mmol), PdCl₂(dppf) (106 mg, 0.162 mmol) and K₂PO₃ (515 mg, 2.42 mmol) in dioxane (33 mL) and water (6 mL) were added 4,4,5,5-tetramethyl-2-(prop-1-en-2-yl)-1,3,2-dioxaborolane (1.09 g, 6.47 mmol) at rt. The mixture was stirred for overnight at 55 °C under N₂, then quenched with water (60 mL). The mixture was extracted with EA (3 x 60 mL) and the organic layers were combined, washed with brine (3 x 50 mL), dried over anhyd. Na₂SO₄, filtered and concentrated under reduced pressure. The residue was purified by silica gel column chromatography with PE:EA (7: 1) to afford *N*-(3,5-dichloro-4-[[7-(prop-1-en-2-yl)-5-[[2-(trimethylsilyl)ethoxy] methyl]-pyrrolo[2,3-*b*]pyra-zin-2-yl]oxy]-phenyl)-*N,N-*dimethylmethanimidamide as a yellow solid (688 mg, 81%).

To a stirred mixture of 2-(3,5-dichloro-4-[[3-(prop-1-en-2-yl)-1*H*-pyrrolo[3,2-*b*]pyridin-5-yl]oxy]phenyl)-3,5-dioxo-4*H*-1,2,4-triazine-6-carbonitrile (688 mg, 1.51 mmol) in EA (24.0 mL) was added Pd/C (90.0 mg, 0.846 mmol) at rt under N₂. The mixture was stirred for 1 h at rt under H₂, the solids were removed by filtration through celite, and the filtrate was concentrated under reduced pressure to provide 2-[3,5-dichloro-4-([3-isopropyl-1*H*-pyrrolo[3,2-*b*]pyridin-5-yl]oxy)phenyl]-3,5-dioxo-4*H*-1,2,4-triazine-6-carbonitrile as a yellow oil (670 mg, 93%). The crude product was used in the next step without further purification.

To a stirred solution of (*E*)-*N*-[3,5-dichloro-4-[(7-isopropyl-5-[[2-(trimethylsilyl)ethoxy]methyl]pyrrolo[2,3-*b*]pyrazin-2-yl)oxy]phenyl]-*N,N*-dimethyl-methanimidamide (670 mg, 1.28 mmol) in ethyl alcohol (15 mL) was added ethylenediamine (347 mg, 5.77 mmol) dropwise at rt. The mixture was stirred overnight at 80 °C then concentrated under reduced pressure. The mixture was diluted with EA (40 mL), washed with brine (2 x 30 mL), dried over anhyd. Na₂SO₄, the solids were removed by filtration and the filtrate was concentrated under reduced pressure to afford 3,5-dichloro-4-[(7-isopropyl-5-[[2-(trimethylsilyl)-ethoxy]methyl]-pyrrolo[2,3-*b*]-pyrazin-2-yl)oxy] aniline as a brown solid (700 mg, crude).

Ethyl (Z)-(2-cyano-2-(2-(3,5-dichloro-4-((7-isopropyl-5-((2-(trimethylsilyl)ethoxy)methyl)-5*H*-pyrrolo[2,3-*b*]pyrazin-2-yl)oxy)phenyl)hydrazineylidene)-acetyl)carbamate, a red solid (800 mg, crude) was prepared similarly as described for ethyl (2-cyano-2-(2-(3,5-dichloro-4-((1-tosyl-1*H*-indol-5-yl)oxy)phenyl)-hydrazineyl-idene)-acetyl) carbamate.

2-[3,5-dichloro-4-[(7-isopropyl-5-[[2-(trimethylsilyl)ethoxy]methyl]-pyrrolo[2,3-*b*]pyrazin-2-yl)-oxy]phenyl]-3,5-dioxo-4*H*-1,2,4-triazine-6-carbonitrile,a brown solid (186 mg, 21%) was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

Tosyl group deprotection to afford 2-[3,5-dichloro-4-([7-isopropyl-5*H-*pyrrolo[2,3-*b*]pyrazin-2-yl]oxy)phenyl]-3,5-dioxo-4*H*-1,2,4-triazine-6-carbonitrile as a white solid (25.5 mg, 17%) was prepared similarly as described for 2-[3,5-dichloro-4-(1*H-*indol-5-yloxy)phenyl]-3,5-dioxo-4*H*-1,2,4-triazine-6-carbonitrile. ¹H NMR (300 MHz, DMSO-*d*₆) δ 12.45 (br, 1H), 12.21 (s, 1H), 8.10 (s, 2H), 7.43 (s, 1H), 3.58 (s, 3H), 3.11-2.97 (m, 1H), 1.20 (d, *J*=6.9 Hz, 6H). LCMS (ESI, *m*/*z):* 456 [M-H]⁺.

### Example 46. Synthesis of compound 52

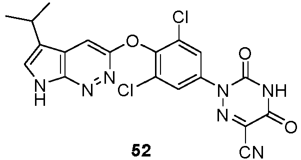

To a stirred solution of 3-chloro-7*H*-pyrrolo[2,3-*c*]pyridazine (4.00 g, 26.1 mmol) and NEt₃ (3.96 g, 39.2 mmol) in DCM (50 mL) was added [2-(chloromethoxy)ethyl]trimethylsilane (13.02 g, 78.4 mmol) dropwise at 0 °C. The mixture was stirred for overnight at rt and quenched with water (100 mL), then was extracted with DCM (3 x 150 mL). The combined organic layers were washed with brine (2 x 100 mL), dried over anhyd. Na₂SO₄, filtrated and concentrated under reduced pressure. The residue was purified by silica gel column chromatography, eluted with PE:EA (10:1) to afford 3-chloro-7-[[2-(trimethylsilyl)ethoxy]methyl]pyrrolo[2,3-c]pyrid-azine as a yellow solid (3.50 g, 47%).

To a stirred solution of 3-chloro-7-[[2-(trimethylsilyl)ethoxy]methyl]-pyrrolo[2,3-*c*]pyridazine (3.20 g, 11.3 mmol) and (*E*)-*N*'-(3,5-dichloro-4-hydroxyphenyl)-*N,N*-dimethylformimidamide (2.63 g, 11.3 mmol) in THF (40 mL) was added Pd₂(dba)₃-chloroform adduct (1.17 g, 1.13 mmol), JosiPhos (625 mg, 1.13 mmol) and Cs₂CO₃ (7.35 g, 22.5 mmol) at rt. The mixture was stirred for 48 h at 95 °C under N₂ and quenched with water (50 mL). The mixture was extracted with EA (3 x 60 mL). The combined organic layers were washed with brine (2 x 50 mL), dried over anhyd. Na₂SO₄, filtrated and concentrated under reduced pressure. The residue was purified by silica gel column chromatography, eluted with PE:EA (1:2) to afford (*E*)-*N'*-(3,5-dichloro-4-((7-((2-(trimethylsilyl)ethoxy)methyl)-7*H-*pyrrolo[2,3-*c*]pyridazin-3-yl)oxy)phenyl)-*N,N*-dimethylformimidamide as a yellow solid (2.2 g crude).

To a stirred mixture of (*E*)-*N*-[3,5-dichloro-4-[(7-[[2-(trimethylsilyl)-ethoxy]methyl]pyrrolo[2,3-*c*]pyridazin-3-yl)oxy]phenyl]-*N,N*-dimethylmethanimidamide (1.12 g, 2.33 mmol) in DCM (12 mL) was added NIS (0.68 g, 3.02 mmol) and *p-*toluenesulfonic acid (0.12 g, 0.699 mmol) in portions at 0 °C. The mixture was stirred for 4 h at rt and quenched with water (30 mL) at rt. The mixture was extracted with DCM (3 x 40 mL). The combined organic layers were washed with brine (2 x 30 mL), dried over anhyd. Na₂SO₄ the solids were removed by filtration and the filtrate was concentrated under reduced pressure. The residue was purified by silica gel column chromatography, eluted with PE:EA (4:1) to afford (*E*)-*N*-[3,5-dichloro-4-[(5-iodo-7-[[2-(trimethylsilyl)ethoxy]-methyl]pyrrolo[2,3-*c*]pyridazin-3-yl)oxy] phenyl]-*N,N*-dimethyl-methanimidamide as a brown solid (600 mg, crude).

To a stirred mixture of (*E*)-*N*-[3,5-dichloro-4-[(5-iodo-7-[[2-(trimethylsilyl)-ethoxy]methyl]pyrrolo[2,3-*c*]pyridazin-3-yl)oxy]phenyl]-*N,N*-dimethylmethanimidamide (650 mg, 1.07 mmol), 1,1'-Bis (di-*t*-butylphosphino)ferrocene palladium dichloride (69.9 mg, 0.107 mmol) and K₂PO₃ (341 mg, 1.61 mmol) in 1,4-dioxane (10 mL) and water (2 mL) was added 4,4,5,5-tetramethyl-2-(prop-1-en-2-yl)-1,3,2-dioxaborolane (721 mg, 4.29 mmol) at rt. The mixture was stirred for 4 h at 50 °C under N₂ and quenched with water (50 mL) at rt, then was extracted with EA (3 x 50 mL). The combined organic layers were washed with brine (2 x 40 mL), dried over anhyd. Na₂SO₄, the solids were removed by filtration and the filtrate was concentrated under reduced pressure. The residue was purified by silica gel column chromatography, eluted with PE:EA (3:1) to afford (*E*)-*N*-(3,5-dichloro-4-[[5-(prop-1-en-2-yl)-7-[[2-(trimethylsilyl)ethoxy]-methyl]pyrrolo[2,3-*c*]pyridazin-3-yl]oxy]phenyl)-*N,N*-dimethylmethanimidamide as a brown solid (675 mg, crude).

To a stirred mixture of (*E*)-*N*-(3,5-dichloro-4-[[5-(prop-1-en-2-yl)-7-[[2-(trimethylsilyl)ethoxy]methyl]pyrrolo[2,3-c]pyridazin-3-yl]oxy]phenyl)-*N,N*-dimethyl-methanimidamide (800 mg, 1.54 mmol) in EA (10 mL) was added Pd:C (300 mg) at rt under hydrogen. The mixture was stirred for 1 h at rt, then was filtered and the filter cake was washed with EA (3 x 20 mL). The combined organic layers were concentrated under reduced pressure to afford (*E*)-*N*-[3,5-dichloro-4-[(5-isopropyl-7-[[2-(trimethylsilyl)ethoxy]methyl]pyrrolo[2,3-*c*]pyridazin-3-yl)oxy]phenyl]-*N,N*-dimethyl-methanimidamide as a yellow oil (1 g, crude).

To a stirred solution of (*E*)-*N*-[3,5-dichloro-4-[(5-isopropyl-7-[[2-(trimethylsilyl)ethoxy]methyl]pyrrolo[2,3-*c*]pyridazin-3-yl)oxy]phenyl]*-N,N*-dimethyl-methanimidamide (1.00 g, 1.92 mmol) in EtOH (9 mL) was added NaOH (9 mL, 9.00 mmol, 1 M) dropwise at rt. The mixture was stirred for 3 h at 80 °C then diluted with water (30 mL) and extracted with EA (3x40 mL). The combined organic layers were washed with brine (2x30 mL), filtrated and concentrated under reduced pressure to afford 3,5-dichloro-4-[(5-isopropyl-7-[[2-(trimethylsilyl)-ethoxy]methyl]-pyrrolo[2,3-*c*]pyridazin-3-yl)oxy]aniline as a brown oil (900 mg, crude).

Ethyl (2-cyano-2-(2-(3,5-dichloro-4-((5-isopropyl-7-((2-(trimethylsilyl)-ethoxy)methyl)-7*H*-pyrrolo[2,3-*c*]pyridazin-3-yl)oxy)phenyl)hydrazineylidene)-acetyl)carbamate, a red solid (920 mg) was prepared similarly as described for ethyl (2-cyano-2-(2-(3,5-dichloro-4-((1-tosyl-1*H*-indol-5-yl)oxy)phenyl)-hydrazineylidene)-acetyl) carbamate with the exception that the reaction stirred for 10min at 0C. The crude product was used in the next step without further purification.

2-[3,5-dichloro-4-[(5-isopropyl-7-[[2-(trimethylsilyl)-ethoxy]methyl]pyrrolo[2,3-*c*]pyridazin-3-yl)oxy] phenyl]-3,5-dioxo-4*H*-1,2,4-triazine-6-carbonitrile as a brown solid (690 mg, 69%) was prepared similarly as described for 2-(4-[[3-isopropyl-1-(4-methylbenzene-sulfonyl)indol-5-yl]oxy]-3,5-dimethylphenyl)-3,5-dioxo-4*H-*1,2,4-triazine-6-carbonitrile.

Tosyl group deprotection to afford 2-[3,5-dichloro-4-([5-isopropyl-7*H-*pyrrolo[2,3-*c*]pyridazin-3-yl]oxy)phenyl]-3,5-dioxo-4*H*-1,2,4-triazine-6-carbonitrile as a yellow solid (24.8 mg, 31%) was prepared similarly as described for 2-[3,5-dichloro-4-(1*H-*indol-5-yloxy)phenyl]-3,5-dioxo-4*H*-1,2,4-triazine-6-carbonitrile. ¹H NMR (400 MHz, DMSO-*d*₆) 13.28 (s, 1H), 12.03-12.05(m, 1H), 7.83 (s, 1H), 7.79 (s, 2H), 7.73-7.75(m, 1H), 3.12-3.19 (m, 1H), 1.32 (d, *J*=6.8 Hz, 6H). LCMS (ESI, *m*/*z*):458[M+H]⁺.

### Example 47. Synthesis of compound 53

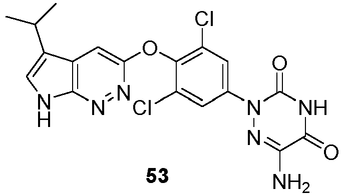

The conversion of compound 52 to 53 was analogous to the method to prepare 6-amino-2-[3,5-dichloro-4-[(5-isopropyl-6-oxo-1*H*-pyridazin-3-yl)oxy]-phenyl]-4*H*-1,2,4-triazine-3,5-dione to afford 2-[3,5-dichloro-4-([5-isopropyl-7*H*-pyrrolo[2,3-*c*]pyridazin-3-yl]oxy)phenyl]-3,5-dioxo-4*H*-1,2,4-triazine-6-carboxylic acid as a yellow solid (190 mg). The crude product was used in the next step without further purification.

To a stirred solution of 2-[3,5-dichloro-4-([5-isopropyl-7*H*-pyrrolo[2,3-c]pyridazin-3-yl]oxy)phenyl]-3,5-dioxo-4*H*-1,2,4-triazine-6-carboxylic acid (150 mg, 0.314 mmol) and DPPA (260 mg, 0.943 mmol) in tBuOH (3 mL) was added NEt₃ (127 mg, 1.26 mmol) dropwise at rt. The resulting mixture was stirred overnight at 85 °C then quenched with water (30 mL) at rt and extracted with EA (3 x 30 mL). The combined organic layers were washed with brine (2 x 20 mL), dried over anhyd. Na₂SO₄, the solids were removed by filtration and the filtrate was concentrated under reduced pressure. The residue was purified by silica gel column chromatography, eluted with PE:EA (3:1) to afford *t*-butyl-*N*-[2-[3,5-dichloro-4-([5-isopropyl-7*H*-pyrrolo[2,3-c]pyridazin-3-yl]-oxy)phenyl]-3,5-dioxo-4*H*-1,2,4-triazin-6-yl]carbamate as a yellow solid (130 mg, crude).

To a stirred solution of *t*-butyl-*N*-[2-[3,5-dichloro-4-([5-isopropyl-7*H-*pyrrolo[2,3-*c*]pyridazin-3-yl]oxy)phenyl]-3,5-dioxo-4*H*-1,2,4-triazin-6-yl]carbamate (120 mg) in DCM (1 mL) was added TFA (2 mL) dropwise at rt. The resulting mixture was stirred for 2 h at rt and concentrated under reduced pressure. The reaction was quenched with sat aq. Na₂CO₃ (20 mL) at rt. The resulting mixture was extracted with DCM (3x30 mL). The combined organic layers were washed with brine (2x20 mL), dried over anhyd. Na₂SO₄, filtrated and concentrated under reduced pressure. The crude product was purified by preparative HPLC to afford 6-amino-2-[3,5-dichloro-4-([5-isopropyl-7*H*-pyrrolo[2,3-*c*]pyridazin-3-yl]oxy)phenyl]-4*H*-1,2,4-triazine-3,5-dione as a white solid (44.8 mg). ¹H NMR (300 MHz, DMSO-*d*₆) δ 11.41-12.07(br, 2H), 7.85-7.92(m, 2H), 7.68-7.79(m, 2H), 6.41(s, 2H), 3.10-3.20(m, 1H), 1.36-1.21 (m, 6H). LCMS (ESI, *m*/*z*): 448[M+H]⁺.

### Example 48. Synthesis of compound 54

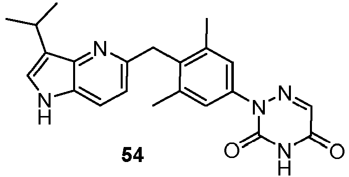

To a mixture of [4-[[3-isopropyl-1-(p-tolylsulfonyl)pyrrolo[3,2-b]pyridin-5-yl]methyl]-3,5-dimethyl-phenyl] trifluoromethanesulfonate (50 mg, 86.1 µmol), 2*H*-1,2,4-triazine-3,5-dione (29.2 mg, 258 µmol), di-*t*-butyl-[2,3,4,5-tetramethyl-6-(2,4,6-triiso-propylphenyl)phenyl]phosphane (8.28 mg, 17.2 µmol) and K₂CO₃ (35.7 mg, 258 µmol) in *t-*BuOH (3 mL) was added Pd₂(dba)₃ (7.89 mg, 8.61 µmol) under N₂ protection. The mixture was stirred at 110 °C for 90 min under microwave irradiation. The reaction was diluted with H₂O (5 mL) and extracted with EA (3x 5 mL). The combined organic layers were washed with brine (5 mL), dried over anhyd. Na₂SO₄, the solids were removed by filtration and the filtrate was concentrated to give a residue, which was purified by silica gel chromatography (0~50% EA in PE) to give 2-[4-[[3-isopropyl-1-(*p*-tolylsulfonyl)-pyrrolo[3,2-*b*]pyridin-5-yl]methyl]-3,5-dimethyl-phenyl]-1,2,4-triazine-3,5-dione as a yellow solid (60 mg, 98%, 37% purity).

Tosyl group deprotection was performed similarly as described for 2-[3,5-dichloro-4-(1*H*-indol-5-yloxy)phenyl]-3,5-dioxo-4*H*-1,2,4-triazine-6-carbonitrile to afford 2-[4-[(3-isopropyl-1*H*-pyrrolo[3,2-*b*]pyridine-5-yl)methyl]-3,5-dimethyl-phenyl]-1,2,4-triazine-3,5-dione as a white solid (5.88 mg, 15%). ¹H NMR (400MHz, DMSO-*d*₆) δ 10.81 (br s, 1H), 8.23 (br s, 1H),7.59 (s, 1H), 7.56 (d, J=8.4 Hz, 1H),7.29 (s,1H), 7.16 (s, 2H),6.69 (d, J=8.4Hz, 1H), 4.23 (s, 2H),3.20 (td, J=6.8, 13.6 Hz, 1H), 2.38(s, 6H), 1.33 (d, J=6.9 Hz, 6H). LCMS (ESI, *m*/*z):* 390.3[M+H]+.

### Example 49. Synthesis of compound 55

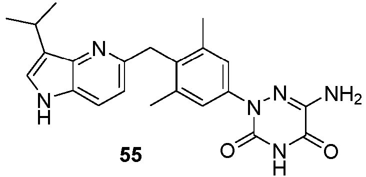

5-[(4-benzyloxy-2,6-dimethyl-phenyl)methyl]-3-isopropyl-1-(*p*-tolyl-sulfonyl)pyrrolo[3,2-*b*]pyridine, a yellow solid (1.36 g, 73%) was prepared similarly as described for 5-[(4-benzyloxy-2,6-dimethyl-phenyl)methyl]-3-isopropyl-1-(*p-*tolylsulfonyl)pyrrolo[3,2-*b*]pyridine.

4-[[3-isopropyl-1-(*p*-tolylsulfon-yl)pyrrolo[3,2-*b*]pyridin-5-yl]methyl]-3,5-dimethyl-phenol, a white solid (1 g, 88%) was prepared similarly as described for 4-[[3-isopropyl-1-(p-tolylsulfonyl)pyrrolo[3,2-*b*]pyridin-5-yl]methyl]-3,5-dimethyl-phenol.

A mixture of [4-[[3-isopropyl-1-(*p*-tolylsulfonyl)pyrrolo[3,2-*b*]pyridin-5-yl]methyl]-3,5-dimethyl-phenyl] trifluoromethanesulfonate (600 mg, 1.03 mmol), bis(pinacolato)diboron (525 mg, 2.07 mmol), Pd(dppf)Cl₂.CH₂Cl₂ (84.4 mg, 103 µmol), KOAc (304 mg, 3.10 mmol) in DMSO (10 mL) was degassed and purged with N₂. The mixture was stirred at 130 °C for 16 h under N₂. The reaction mixture was partitioned between H₂O (30 mL) and EA (50 mL). The organic phase was separated, washed with brine (30 mL), dried over Na₂SO₄, the solids were removed by filtration and the filtrate was concentrated under reduced pressure to give a residue. The residue was purified by silica gel chromatography (0~10% EA in PE) to give 5-[[2,6-dimethyl-4-(4,4,5,5-tetramethyl-1,3,2-dioxa-borolan-2-yl)phenyl]-methyl]-3-isopropyl-1-(*p*-tolylsulfonyl)pyrrolo[3,2-*b*]pyridine as a yellow solid (340 mg, 59%).

To a solution of 5-[[2,6-dimethyl-4-(4,4,5,5-tetramethyl-1,3,2-dioxaborolan-2-yl)phenyl]methyl]-3-isopropyl-1-(*p*-tolylsulfonyl)pyrrolo[3,2-*b*]pyridine (290 mg, 519 µmol) in acetone (12 mL) and H₂O (6 mL) was added NaIO₄ (1.11 g, 5.19 mmol, 287.71 µL) and NH₄OAc (400 mg, 5.19 mmol) at 25 °C. After the addition, the mixture was stirred at 25 °C for 16 h. The reaction mixture was extracted with EA (3 x 10 mL). The organic layers were washed with brine (20 mL), dried over Na₂SO₄, the solids were removed by filtration and the filtrate was concentrated under reduced pressure, then purified by preparatory TLC (SiO₂, PE:EA=1:1) to give [4-[[3-isopropyl-1-(*p*-tolylsulfonyl)pyrrolo[3,2-*b*]pyridin-5-yl] methyl]-3,5-dimethyl-phenyl]-boronic acid as a white solid (190 mg, 76%).

A mixture of 6-amino-4-(benzyloxymethyl)-2*H*-1,2,4-triazine-3,5-dione (252 mg, 1.02 mmol), [4-[[3-isopropyl-1-(*p*-tolylsulfonyl)pyrrolo[3,2-*b*]pyridin-5-yl]methyl]-3,5-dimethyl-phenyl]boronic acid (440 mg, 923 µmol), pyridine (146 mg, 1.85 mmol, 149 µL), 4Å molecular sieves (5 g) and Cu(OAc)₂ (83.9 mg, 462 µmol) in DMF (15 mL) was degassed and purged with O₂, then the mixture was stirred at 60 °C for 16 h under an O₂ atmosphere (via balloon). The reaction mixture was cooled to 25 °C and diluted with EA (30 mL), then the solids were removed by filtration. The filtrate was washed with H₂O (3 x 10 mL), brine (20 mL), dried over Na₂SO₄, the solids were removed by filtration and the filtrate was concentrated under reduced pressure to give a residue. The residue was purified by silica gel chromatography (0~30% EA in PE) to give 6-amino-4-(benzyloxymethyl)-2-[4-[[3-isopropyl-1-(*p*-tolylsulfonyl)-pyrrolo[3,2-*b*]pyrid-ine-5-yl] methyl]-3,5-dimethyl-phenyl]-1,2,4-triazine-3,5-dione as a yellow gum (140 mg, 20%).

A solution of 6-amino-4-(benzyloxymethyl)-2-[4-[[3-isopropyl-1-(*p*-tolyl-sulfonyl)pyrrolo[3,2-*b*]pyridine-5-yl]methyl]-3,5-dimethyl-phenyl]-1,2,4-triazine-3,5-dione (140 mg, 206 µmol) in TFA (5 mL) was stirred at 80 °C for 16 hr. The reaction mixture was diluted with EA (10 mL), and the pH was adjusted to 7 by NaHCO₃ (sat. aq.). The organic layer was separated, and the aq. layer was extracted with EA (3 x 20 mL). The combined organic layers were washed with brine (20 mL), dried over Na₂SO₄, the solids were removed by filtration and the filtrate was concentrated under reduced pressure to give a residue, which was purified by preparatory TLC (SiO₂, PE:EA=1:2) to give 6-amino-2-[4-[[3-isopropyl-1-(*p*-tolylsulfonyl)-pyrrolo[3,2-*b*]pyridin-5-yl]methyl]-3,5-dimethyl-phenyl]-1,2,4-triazine-3,5-dione as a white solid (45 mg, 39%).

Tosyl group deprotection to afford 6-amino-2-[4-[(3-isopropyl-1*H*-pyrrolo[3,2-*b*]pyridin-5-yl)methyl]-3,5-dimethyl-phenyl]-1,2,4-triazine-3,5-dione, a white solid (11.3 mg, 35%) was prepared similarly as described for 2-[3,5-dichloro-4-(1*H*-indol-5-yloxy)phenyl]-3,5-dioxo-4*H*-1,2,4-triazine-6-carbonitrile. ¹H NMR (400MHz, DMSO-*d*₆) δ=12.07 (br s, 1H), 10.80 (br s, 1H),7.55 (d, J=8.4 Hz, 1H), 7.29 (s, 1H),7.19 (s, 2H), 6.63 (d, J=8.4Hz, 1H),6.29 (s, 2H), 4.21 (s, 2H), 3.25 -3.19 (m, 1H), 2.34 (s,6H), 1.34 (d, J=6.9 Hz, 6H). LCMS (ESI, *m*/*z):* 405.6 [M+H]⁺.

### Example 50. Synthesis of compound 56

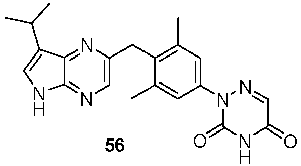

2-(2,6-Dimethyl-4-(4,4,5,5-tetramethyl-1,3,2-dioxaborolan-2-yl)benzyl)-7-isopropyl-5-tosyl-5*H*-pyrrolo[2,3-*b*]pyraz-ine, a yellow solid (520 mg, 73%), was prepared similarly as described for 5-[[2,6-dimethyl-4-(4,4,5,5-tetramethyl-1,3,2-dioxa-borolan-2-yl)phenyl]-methyl]-3-isopropyl-1-(*p*-tolylsulfonyl)pyrrolo[3,2-*b*]pyridine with the exception that the reaction was heated for 12h.

(4-((7-Isopropyl-5-tosyl-5*H*-pyrrolo[2,3-*b*]pyrazin-2-yl)methyl)-3,5-dimethylphenyl)boronic acid, a yellow solid (670 mg, crude) was prepared similarly as described for [4-[[3-isopropyl-1-(*p*-tolylsulfonyl)pyrrolo[3,2-*b*]pyridin-5-yl] methyl]-3,5-dimethyl-phenyl]-boronic acid.

4-((Benzyloxy)methyl)-2-(4-((7-isopropyl-5-tosyl-5*H*-pyrrolo[2,3-*b*]pyrazin-2-yl)-methyl)-3,5-dimethylphenyl)-1,2,4-triazine-3,5(2*H*,4*H*)-dione, a light yellow solid (110 mg, 11%), was prepared similarly as described for 6-amino-4-(benzyloxymethyl)-2-[4-[[3-isopropyl-1-(*p*-tolylsulfonyl)pyrrolo[3,2-*b*]pyrid-ine-5-yl] methyl]-3,5-dimethyl-phenyl]-1,2,4-triazine-3,5-dione.

Tosyl group deprotection to give 2-(4-((7-isopropyl-5*H*-pyrrolo[2,3-*b*]pyrazin-2-yl)methyl)-3,5-dimethylphenyl)-1,2,4-triazine-3,5(2*H*,4*H*)-dione, a white solid (20 mg, 80%), was prepared similarly as described for 2-[3,5-dichloro-4-(1*H*-indol-5-yloxy)phenyl]-3,5-dioxo-4*H*-1,2,4-triazine-6-carbonitrile. ¹H NMR (400MHz, DMSO-*d*₆) δ=12.48 (brs, 1H), 11.54 (br s, 1H), 7.93 (s, 1H), 7.58 (s, 1H), 7.51 (s,1H), 7.16 (s, 2H), 4.26 (s, 2H), 3.09-3.17 (m, 1H), 2.40 (s, 6H), 1.31 (d, J=6.8 Hz, 6H). LCMS (ESI, *m*/*z*): 391.6[M+H]⁺.

### Example 51. Synthesis of compound 57

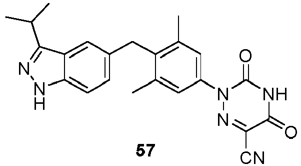

2,2,2-Trifluoro-*N*-(4-(hydroxy(3-isopropyl-1*H*-indazol-5-yl)methyl)-3,5-dimethylphenyl)acetamide,a white solid (294 mg, 36%), was prepared similarly as described for 2,2,2-trifluoro-*N*-(4-(hydroxy(3-pentyl-1*H*-indazol-5-yl)methyl)-3,5-dimethylphenyl)acetamide with the exception that *t*-butyl lithium was used (2.2eq).

2,2,2-Trifluoro-*N*-(4-((3-isopropyl-1*H*-indazol-5-yl)methyl)-3,5-dimethylphenyl)acetamide (209 mg, 81%), a white solid, was prepared similarly as described for 2,2,2-trifluoro-*N*-(4-[[3-isopropyl-1-(4-methylbenzene-sulfonyl)indol-5-yl]methyl]-3,5-dimethylphenyl)acetamide.

Trifluoroacetamide deprotection to afford 4-((3-isopropyl-1*H*-indazol-5-yl)methyl)-3,5-dimethylaniline (141 mg, 96%) was prepared similarly as described for 3,5-dimethyl-4-((3-pentyl-1*H*-indazol-5-yl)methyl)aniline.

2-(4-((3-Isopropyl-1*H*-indazol-5-yl)methyl)-3,5-dimethylphenyl)-3,5-dioxo-2,3,4,5-tetrahydro-1,2,4-triazine-6-carbonitrile,an orange solid (10 mg, 8%) was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile. ¹H NMR (DMSO-*d*₆ 400 MHz): 1.31 (d, *J=* 6.8 Hz, 6H); 2.28 (s, 6H); 3.22-3.27 (m, 1H); 4.15 (s, 2H), 6.96 (d, *J* = 8.3 Hz, 1H); 7.18 (s, 2H); 7.35 (d, *J* = 8.3 Hz, 1H); 7.38 (s, 1H); 12.47 (s, 1H); 12.99 (s, 1H).

### Example 52. Synthesis of compound 58

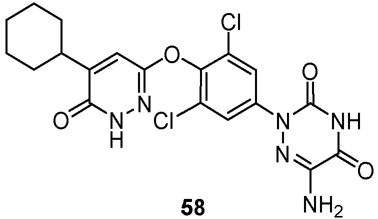

6-Amino-2-[3,5-dichloro-4-[(5-cyclohexyl-6-oxo-1*H*-pyridazin-3-yl)oxy]phenyl]-4*H*-1,2,4-triazine-3,5-dione was prepared similarly as described for 6-amino-2-(3,5-dichloro-4-((5-cyclobutyl-6-oxo-1,6-dihydropyridazin-3-yl)oxy)phenyl)-1,2,4-triazine-3,5(2*H*,4*H*)-dione to afford a white solid (101.1 mg, 40%). ¹H NMR (400 MHz, DMSO-*d*₆) 12.18 (br, 2H), 7.83 (s, 2H), 7.35-7.36 (m, 1H), 6.47 (s, 2H), 2.68-2.72 (m, 1H), 1.21- 1.84 (m, 10H). LCMS (ESI, *m*/*z):* 465 [M+H]⁺.

### Example 53. Synthesis of compound 59

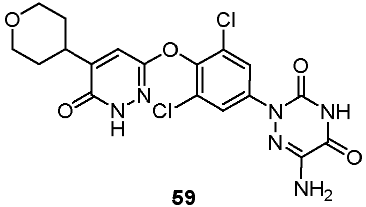

3,6-dichloro-4-(tetrahydro-2*H*-pyran-4-yl)pyridazine was prepared similarly as described for 3,6-dichloro-4-isopropylpyridazine (see also Samaritoni, J. G. Homolytic alkylation of 3,6-dichloropyridazine. Org. Prep. Proced. Int. 1988, 20, 117-121) as a white solid (5.3 g, 68%).

Ethyl (2-cyano-2-(2-(3,5-dichloro-4-hydroxyphenyl)hydrazineylidene)-acetyl)carbamate, a brown solid (42 g, crude) was prepared similarly as described for ethyl (2-cyano-2-(2-(3,5-dichloro-4-((1-tosyl-1*H*-indol-5-yl)oxy)phenyl)-hydrazineyl-idene)-acetyl) carbamate with the exception that the reaction was stirred for 10 min at 0 °C.

To a stirred mixture of ethyl (2-cyano-2-(2-(3,5-dichloro-4-hydroxyphenyl)hydrazineylidene)acetyl)carbamate (41.0 g, 119 mmol) in DMA (400 mL) was added KOAc (46.6 g, 475 mmol) in portions at rt .The resulting mixture was stirred for 2 h at 110 °C and quenched with water (400 mL). The resulting mixture was extracted with EA (3 x 600 mL) and the organic layers were combined, washed with brine (3 x 400 mL), dried over anhyd. Na₂SO₄ the solids were removed by filtration and the filtrate was concentrated under reduced pressure. The residue was purified by silica gel column chromatography with DCM:MeOH (19:1) to afford 2-(3,5-dichloro-4-hydroxyphenyl)-3,5-dioxo-4*H*-1,2,4-triazine-6-carbonitrile as a yellow solid (24.3 g, 62%).

To a stirred solution of 2-(3,5-dichloro-4-hydroxyphenyl)-3,5-dioxo-4*H*-1,2,4-triazine-6-carbonitrile (20 g, 66.9 mmol) in AcOH (200 mL) was added HCl (100 mL) at rt. The resulting mixture was stirred for 5 h at 110 °C and concentrated under reduced pressure. The residue was adjusted to pH=9 with NaOH (1 M). The resulting mixture was extracted with EA (2 x 80 mL). The mixture was adjusted to pH=5 with HCl (1 M), then extracted with EA (3 x 100 mL) and the organic layers were combined, washed with brine (2 x 60 mL), dried over anhyd. Na₂SO₄ and concentrated under reduced pressure to afford 2-(3,5-dichloro-4-hydroxyphenyl)-3,5-dioxo-4*H*-1,2,4-triazine-6-carboxylic acid as a yellow solid (19 g, crude).

To a stirred solution of 2-(3,5-dichloro-4-hydroxyphenyl)-3,5-dioxo-4*H*-1,2,4-triazine-6-carboxylic acid (13.8 g, 43.4 mmol) in *t*BuOH (150 mL) was added DPPA (29.9 g, 108 mmol) and NEt₃ (17.7 g, 174 mmol) at rt. The mixture was stirred for overnight at 85 °C and concentrated under reduced pressure, diluted with water (100 mL) and extracted with EA (3 x 120 mL). The combined organic layers were washed with brine (2 x 80 mL), dried over anhyd. Na₂SO₄, filtrated and concentrated under reduced pressure. The residue was purified by silica gel column chromatography with DCM:MeOH (40:1) to afford *t*-butyl-*N*-(2-[3,5-dichloro-4-[(diphenoxyphosphoryl)-oxy]phenyl]-3,5-dioxo-4*H*-1,2,4-triazin-6-yl)carbamate as a yellow solid (16.0 g, 53%).

To a stirred mixture of *t*-butyl-*N*-(2-[3,5-dichloro-4-[(diphenoxyphosphor-yl)oxy]phenyl]-3,5-dioxo-4*H*-1,2,4-triazin-6-yl)carbamate (11.0 g, 17.7 mmol) in tBuOH (200 mL) was added NaOH (25 mL, 2 M) dropwise at rt. The resulting mixture was stirred for 4 h at rt and concentrated under reduced pressure. The mixture was diluted with water (50 mL), acidified to pH 6 with HCl (1 M), and extracted with EA (3 x 100 mL). The organic layers were combined, washed with brine (2 x 70 mL), dried over anhyd. Na₂SO₄, the solids were removed by filtration and the filtrate was concentrated under reduced pressure. The residue was purified by silica gel column chromatography with PE:EA (1:1) to afford *t*-butyl-*N*-[2-(3,5-dichloro-4-hydroxyphenyl)-3,5-dioxo-4*H*-1,2,4-triazin-6-yl]-carbam-ate as a yellow solid (6.0 g, 78%).

To a stirred mixture of 3,6-dichloro-4-(oxan-4-yl)pyridazine (300 mg, 1.29 mmol) and *t*-butyl-*N*-[2-(3,5-dichloro-4-hydroxyphenyl)-3,5-dioxo-4*H*-1,2,4-triazin-6-yl]carbamate (501 mg, 1.29 mmol) in DMSO (10 mL) was added K₂CO₃ (534 mg, 3.86 mmol) and CuI (123 mg, 0.644 mmol) at rt. The mixture was stirred for 16 h at 110 °C under N₂ and quenched with water (30 mL) at rt. The mixture was extracted with EA (3 x 40 mL) and the organic layers were combined, washed with brine (2 x 30 mL), dried over anhyd. Na₂SO₄ and concentrated under reduced pressure. The residue was purified by silica gel column chromatography DCM:MeOH (3:1) to afford 6-amino-2-(3,5-dichloro-4-[[6-chloro-5-(oxan-4-yl)pyrida-zin-3-yl]oxy]phenyl)-4*H*-1,2,4-triazine-3,5-dione as a brown solid (280 mg, 36%).

To a stirred mixture of 6-amino-2-(3,5-dichloro-4-[[6-chloro-5-(oxan-4-yl)-pyridazin-3-yl]oxy]phenyl)-4*H*-1,2,4-triazine-3,5-dione (250 mg, 0.515 mmol) in AcOH (4 mL) was added NaOAc (169 mg, 2.06 mmol) at rt. The mixture was stirred for overnight at 100 °C then quenched with water (30 mL). The mixture was extracted with EA (3 x 30 mL), the organic layers were combined, washed with brine (2 x 30 mL), dried over anhyd. Na₂SO₄, the solids were removed by filtration and the filtrate was concentrated under reduced pressure. The residue was purified by silica gel column chromatography with DCM:MeOH (20:1) to afford crude product that was purified by preparative HPLC to afford 6-amino-2-(3,5-dichloro-4-[[5-(oxan-4-yl)-6-oxo-1*H*-pyridazin-3-yl]oxy]phenyl)-4*H*-1,2,4-triazine-3,5-dione as a white solid (23.3 mg, 10%). ¹H NMR (300 MHz, DMSO-*d*₆) 12.26-12.28 (m, 2H), 7.86 (s, 2H), 7.44-7.46 (m, 1H), 6.54 (s, 2H), 3.93-3.99 (m, 2H), 3.41-3.49 (m, 2H), 2.95-3.05 (m, 1H), 1.58-1.78 (m, 4H). LCMS (ESI, *m*/*z):* 467 [M+H]⁺.

### Example 54. Synthesis of compounds 60 and 60-A

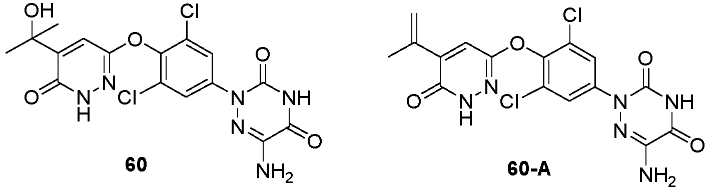

To a mixture of methyl 3,6-dichloropyridazine-4-carboxylate (10.0 g, 48.4 mmol) in THF (100 mL) was added NaOCH₃ (9.57 g, 53.1 mmol, 30% in MeOH) dropwise at 0 °C. The mixture was stirred for 0.5 h at 0 °C then quenched with NH₄Cl (sat., aq., 150 mL). The mixture was extracted with EA (3 x 150 mL), the organic layers were combined, washed with brine (2 x 150 mL), dried over anhyd. Na₂SO₄, the solids were removed by filtration and the filtrate was concentrated under reduced pressure. The sample was purified by column chromatography (EA:PE=0~30% over 30 min) to afford methyl 6-chloro-3-methoxy-pyridazine-4-carboxylate as a yellow solid (7.3 g, 60%).

A mixture of methyl 6-chloro-3-methoxypyridazine-4-carboxylate (9.00 g, 44.4 mmol) in THF (100 mL) was added dropwise CH₃MgBr (59.2 mL, 178 mmol, 3 M in Et₂O) at -50 °C. The mixture was stirred for 2 h at -50 °C to 0 °C, then quenched with sat. aq. NH₄Cl (150 mL) and extracted with EA (3 x 150 mL). The organic layers were combined, washed with brine (2 x 150 mL), dried over anhyd. Na₂SO₄, the solids were removed by filtration and the filtrate was concentrated under reduced pressure to afford crude product that was purified by silica column chromatography (EA:PE=0~30% over 20 min) to afford 2-(6-chloro-3-methoxypyridazin-4-yl)propan-2-ol as an off-white solid (2.35 g, 25%).

To a mixture of 2-(6-chloro-3-methoxypyridazin-4-yl)propan-2-ol (2.90 g, 14.3 mmol), (*E*)-*N*'-(3,5-dichloro-4-hydroxyphenyl)-*N,N*-dimethylmethan-imidamide (3.67 g, 15.7 mmol), Cs₂CO₃ (9.33 g, 28.6 mmol) and JosiPhos (0.85 g, 1.43 mmol) in THF (30 mL) was added Pd₂(dba)₃-CHCl₃ (1.48 g, 1.43 mmol). The mixture was stirred for 48 h at 95 °C under N₂ then quenched with water (50 mL). The mixture was extracted with EA (3 x 80 mL) and the organic layers were combined, washed with brine (2 x 50 mL), dried over anhyd. Na₂SO₄, the solids were removed by filtration and the filtrate was concentrated under reduced pressure. The sample was purified by column chromatography and eluted with EA:PE to afford (*E*)-*N*'-(3,5-dichloro-4-[[5-(2-hydroxypropan-2-yl)-6-methoxypyridazin-3-yl]oxy]phenyl)-*N*,*N*-dimethylmethanimidamide as a yellow solid (690 mg, 11%).

To a mixture of (*E*)-*N*'-(3,5-dichloro-4-[[5-(2-hydroxypropan-2-yl)-6-meth-oxypyridazin-3-yl]oxy]phenyl)-*N,N*-dimethylmethanimidamide (690 mg, 1.73 mmol) and KI (861 mg, 5.18 mmol) in DCM (10 mL) was added TMSCl (563 mg, 5.18 mmol) at 0 °C. The mixture was stirred overnight at rt and quenched with water (20 mL). The mixture was extracted with DCM (3 x 30 mL) and the organic layers were combined, washed with brine (2 x 20 mL), dried over anhyd. Na₂SO₄, the solids were removed by filtration and the filtrate was concentrated under reduced pressure to provide (*E*)-*N*'-(3,5-dichloro-4-[[5-(2-hydroxypropan-2-yl)-6-oxo-1*H*-pyridazin-3-yl]oxy]phenyl)-*N,N*-dimethylmethanimidamide as a yellow solid (670 mg, crude).

A mixture of (*E*)-*N*'-(3,5-dichloro-4-[[5-(2-hydroxypropan-2-yl)-6-oxo-1*H-*pyridazin-3-yl]oxy]phenyl)-*N,N*-dimethylmethanimidamide (670 mg, 1.74 mmol) in ethanol (10 mL) and NaOH (10 mL, 1 M). The mixture was stirred overnight at 70 °C then concentrated under reduced pressure, diluted with water (50 mL) and extracted with EA (3 x 50 mL). The organic layers were combined, washed with brine (2 x 30 mL), dried over anhyd. Na₂SO₄, the solids were removed by filtration and the filtrate was concentrated under reduced pressure to provide 6-(4-amino-2,6-dichlorophenoxy)-4-(2-hydroxypropan-2-yl)-2*H-*pyridazin-3-one as a yellow solid (560 mg, 83%).

Ethyl (2-cyano-2-(2-(3,5-dichloro-4-((5-(2-hydroxypropan-2-yl)-6-oxo-1,6-dihydropyridazin-3-yl)oxy)phenyl)hydrazineylidene)acetyl)carbamate, a yellow solid (500 mg, 55%), was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3,5-dichloro-4-[[5-(2-hydroxypropan-2-yl)-6-oxo-1*H*-pyridazin-3-yl]oxy]-phenyl)-3,5-dioxo-4*H*-1,2,4-triazine-6-carbonitrile as a yellow solid (380 mg, 80%) was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

Further steps in the synthesis of compound 60, 6-amino-2-(3,5-dichloro-4-[[5-(2-hydroxypropan-2-yl)-6-oxo-1*H*-pyridazin-3-yl]oxy]phenyl)-4*H*-1,2,4-triazine-3,5-dione as a white solid (63.9 mg, 35%). ¹H NMR (300 MHz, DMSO- *d*₆) δ ppm: 12.2-12.4 (m, 2H), 7.87 (s, 2H), 7.53 (s, 1H), 6.53 (s, 2H), 5.50 (s, 1H), 1.51 (s, 6H). LCMS (ESI, *m*/*z):* 441 [M+H]⁺.

Compound 60-A, 6-amino-2-(3,5-dichloro-4-[[6-oxo-5-(prop-1-en-2-yl)-1*H-*pyridazin-3-yl]oxy]phenyl)-4*H*-1,2,4-triazine-3,5-dione was obtained as a white solid (13.8 mg, 8%). ¹H NMR (300 MHz, DMSO-*d*₆) δ ppm: 12.0-12.5 (m, 2H), 7.87 (s, 2H), 7.54 (s, 1H), 6.49 (s, 3H), 5.57 (s, 1H), 2.12 (s, 3H). LCMS (ESI, *m*/*z):* 423 [M+H]⁺.

### Example 55. Synthesis of compound 61

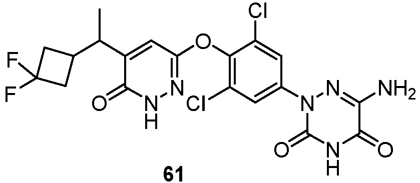

To a solution of diisopropylamine (5.18 g, 51.2 mmol) in THF (60 mL) was added dropwise *n*-buLi (19.5 mL, 2.5 M in hexane, 48.7 mmol) at -78 °C under N₂. The solution was stirred at -78~-50 °C for 0.5 h. Methyl 2-(3,3-difluoro-cyclobutyl)acetate (prepared according to procedures detailed in WO2015005901 and WO20150023913) (4 g, 24.4 mmol) in THF (10 mL) was added dropwise at -78 °C. The solution was stirred at -78 °C for 0.5 h. CH₃I (4.50 g, 31.7 mmol) was added at -78 °C. The reaction mixture was slowly warmed to rt over 3 h, then quenched with water (30 mL) and extracted with EA (3 x 50 mL). The organic layer was dried over anhyd. Na₂SO₄, the solids were removed by filtration and the filtrate was concentrated under reduced pressure to provide methyl 2-(3,3-difluorocyclobutyl)propanoate as a yellow oil (3.9 g, crude).

To a solution of methyl 2-(3,3-difluorocyclobutyl)propanoate (3.8 g, 21.3 mmol) in THF (40 mL) and MeOH (10 mL) was added NaOH solution (10 N, 20 mL). The solution was stirred overnight at rt. The pH was adjusted to 5 with HCl (1 N). The mixture was extracted with EA (3 x 50 mL). The organic phases were combined, washed with brine, dried over anhyd. Na₂SO₄, the solids were removed by filtration and the filtrate was concentrated under reduced pressure to provide 2-(3,3-difluorocyclobutyl)propanoic acid as a yellow oil (1.5 g, crude).

3,6-Dichloro-4-[1-(3,3-difluoro-cyclobutyl)ethyl]-pyridazine was prepared similarly as described for 3,6-dichloro-4-isopropylpyridazine (see also Samaritoni, J. G. Homolytic alkylation of 3,6-dichloropyridazine. Org. Prep. Proced. Int. 1988, 20, 117-121) to afford a yellow oil (390 mg, 21%).

6-Amino-2-[3,5-dichloro-4-([6-chloro-5-[1-(3,3-difluorocyclobutyl)-ethyl]pyrida-zin-3-yl]oxy)-phenyl]-4*H*-1,2,4-triazine-3,5-dione, a yellow oil (280 mg, 33%) was prepared similarly as described for 6-amino-2-(3,5-dichloro-4-[[6-chloro-5-(oxan-4-yl)pyrida-zin-3-yl]oxy]phenyl)-4*H*-1,2,4-triazine-3,5-dione.

To a solution of 6-amino-2-[3,5-dichloro-4-([6-chloro-5-[1-(3,3-difluoro-cyclobutyl)ethyl]pyridazin-3-yl]oxy)phenyl]-4*H*-1,2,4-triazine-3,5-dione (280 mg, 0.539 mmol) in AcOH (8 mL) was added NaOAc (221 mg, 2.69 mmol). The mixture was stirred overnight at 100°C. The mixture reaction was cooled to rt and quenched with water (50 mL). The reaction mixture was stirred for 10 min, the solid was filtered and washed with water (2 x 10 mL) and PE (2 x 5 mL), then dried under reduced pressure to afford the crude product that was purified by preparative HPLC to afford 6-amino-2-[3,5-dichloro-4-([5-[1-(3,3-difluorocyclobutyl)ethyl]-6-oxo-1H-pyridazin-3-yl]oxy)phenyl]-4*H*-1,2,4-tri-azine-3,5-dione as a white solid (34.2 mg, 13%). LCMS (ESI, *m*/*z):* 523 [M+Na]⁺.

The enantiomers were separated by preparative pre-chiral-HPLC using the following gradient conditions: Column: CHIRALPAK IA, 3 x 25cm, 5 µm ; Mobile Phase A:Hex:DCM=3:1 (10mM NH₃-CH3OH)-HPLC, Mobile Phase B:EtOH-HPLC; Flow rate:40 mL/min; Gradient:10 B to 10 B in 50 min; 220/254 nm; Injection Volume: 1 mL; Number Of Runs:14;. Purification resulted in (RT 1: 35.6 min (compound 61-A (RT 1=35.6 min, 43.9 mg) as an off-white solid, and compound 61-B (RT 2=42.1 min, 41.8 mg).

61-A: ¹H NMR (300 MHz, DMSO-*d*₆) δ 12.28 (br, 2H), 7.86 (s, 2H), 7.52 (s, 1H), 6.54 (br, 2H), 2.99 - 3.04 (m, 1H), 2.73 (br, 1H), 2.51 - 2.60 (m, 1H), 2.44 - 2.50 (m, 2H), 2.12 - 2.24 (m, 1H), 1.15 (d, *J* = 6.9 Hz, 3H). LCMS (ESI, *m*/*z):* 501 [M+H]⁺. [α]^{22.1}_{D}= + 18° (MeOH, C=1mg/mL).

And (RT 2: 42.1min (compound 61-B), 41.8 mg) ¹H NMR (300 MHz, DMSO-*d*₆) δ 12.27 (br, 2H), 7.85 (s, 2H), 7.52 (s, 1H), 6.53 (br, 2H), 2.98 - 3.03 (m, 1H), 2.69 - 2.74 (m, 1H), 2.52 - 2.54 (m, 1H), 2.44 - 2.50 (m, 2H), 2.12 - 2.24 (m, 1H), 1.15 (d, *J* = 6.9 Hz, 3H). LCMS (ESI, *m*/*z):* 501 [M+H]⁺. [α]^{22.1}_{D}= -14° (MeOH, C=1mg/mL).

### Example 56. Synthesis of compound 62

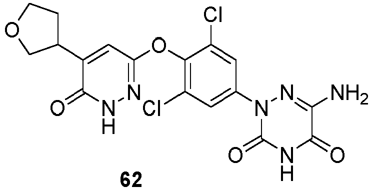

To a solution of 3,6-dichloropyridazine (5.00 g, 33.6 mmol) in water (100 mL) was added silver nitrate (2.85 g, 16.8 mmol) and oxolane-3-carboxylic acid (3.90 g, 33.6 mmol) in portions at rt and then stirred at 50 °C. To the mixture was added sulfuric acid (9.88 g, 101 mmol) in portions at 50 °C and stirred at 60 °C. Then ammonium persulfate (23 g, 101 mmol) in water (50 mL) was added and stirred for 1 h at 70 °C. The mixture was neutralized to pH 9 with NaOH solution (2N). The mixture was extracted with EA (3 x 50 mL). The combined organic layers were washed with brine (50 mL), dried over anhyd. Na₂SO₄, filtered and concentrated under reduced pressure. The residue was purified on a silica gel column with PE:EA (10:1) to provide 3,6-dichloro-4-(oxolan-3-yl)pyridazine as a light yellow oil (2.8 g, 36%).

To a solution of 3,6-dichloro-4-(oxolan-3-yl)pyridazine (330 mg, 1.51 mmol) and *t*-butyl-*N*-[2-(3,5-dichloro-4-hydroxyphenyl)-3,5-dioxo-4*H*-1,2,4-triazin-6-yl]-carbamate (586 mg, 1.51 mmol) in DMSO (10 mL) was added K₂CO₃ (625 mg, 4.52 mmol) and CuI (28.7 mg, 0.15 mmol). The reaction was stirred at 110 °C for 16 h under N₂, then quenched with water (50 mL) The mixture was extracted with EA (3 x 50 mL), washed with brine (30 mL), dried over anhyd. Na₂SO₄, filtered and concentrated under reduced pressure. The residue was purified on a silica gel column with MeOH:DCM (1:20) to afford *t*-butyl-*N-*[2-(3,5-dichloro-4-[[6-chloro-5-(oxolan-3-yl)pyridazin-3-yl]oxy]phenyl)-3,5-dioxo-4*H-*1,2,4-triazin-6-yl]carbamate as a brown solid (600 mg, 49%).

To a solution of 6-amino-2-(3,5-dichloro-4-[[6-chloro-5-(oxolan-3-yl)-pyridazin-3-yl]oxy]phenyl)-4*H*-1,2,4-triazine-3,5-dione (420 mg, 0.89 mmol) in AcOH (10 mL) was added NaOAc (292 mg, 3.56 mmol). The reaction was stirred overnight at 100 °C. The reaction solution was poured into water (100 mL) and filtered. The precipitate was purified by preparative HPLC to afford 6-amino-2-(3,5-dichloro-4-[[6-hydroxy-5-(oxolan-3-yl)pyridazin-3-yl]oxy]phenyl)-4*H*-1,2,4-tri-azine-3,5-dione as a white solid (72.8 mg, 18%). ¹H NMR (300 MHz, DMSO-*d*₆) δ 12.28-12.30 (m, 2H), 7.86 (s, 2H), 7.50 (s, 1H), 6.54 (s, 2H), 3.80-4.01 (m, 2H), 3.75-3.78 (m, 1H), 3.63-3.68 (m, 1H), 3.42-3.54 (m, 1H), 2.18-2.29 (m, 1H), 1.98-2.10 (m, 1H). LCMS (ESI, *m*/*z):* 453 [M+H]⁺.

### Example 57. Synthesis of compound 63

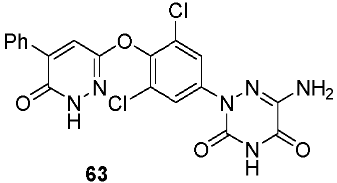

To a solution of 3,6-dichloro-pyridazine (5.00 g, 33.6 mmol) and phenyl boronic acid (8.18 g, 67.1 mmol) in water (180 mL) and 1,2-dichloroethane (180 mL) was added Selectfluor^{™} (23.8 g, 67.1 mmol) and TFA (3.83 g, 33.6 mmol). The reaction was stirred at rt for 2 min and AgNO₃ (1.14 g, 6.71 mmol) in water (20 mL) was added. Then the reaction mixture was stirred 50 °C for 16 h. The solution was extracted with DCM (2 x 200 mL). The organic layers were combined, dried over anhyd. Na₂SO₄, the solids were removed by filtration and the filtrate was concentrated under reduced pressure. The residue was purified on a silica gel column with PE:EA (25:1) to afford 3,6-dichloro-4-phenylpyridazine as a yellow solid (950 mg, 12%).

To a solution of 3,6-dichloro-4-phenylpyridazine (338 mg, 1.50 mmol) and *t-*butyl-*N*-[2-(3,5-dichloro-4-hydroxyphenyl)-3,5-dioxo-4*H*-1,2,4-triazin-6-yl]carbam-ate (585 mg, 1.50 mmol) in DMSO (15 mL) was added K₂CO₃ (623 mg, 4.50 mmol) and CuI (143 mg, 0.75 mmol). The reaction mixture was stirred at 110 °C for 16 h under N₂, then quenched with water (100 mL). The mixture was extracted with EA (3 x 100 mL) and the organic layers were combined, washed with brine (100 mL), dried over anhyd. Na₂SO₄, filtered and concentrated under reduced pressure. The residue was purified on a silica gel column with PE:EA (2:3) to get *t*-butyl-*N*-(2-[3,5-dichloro-4-[(6-chloro-5-phenylpyridazin-3-yl)oxy]phenyl]-3,5-dioxo-4*H*-1,2,4-triazin-6-yl)carbamate as a brown solid (350 mg, 32%).

To a solution of 6-amino-2-[3,5-dichloro-4-[(6-chloro-5-phenylpyridazin-3-yl)oxy]phenyl]-4*H*-1,2,4-triazine-3,5-dione(350 mg, 0.733 mmol) and in AcOH (10 mL) was added NaOAc (240 mg, 2.93 mmol). The reaction was stirred overnight at 100 °C and cooled to rt. The reaction solution was poured into water (100 mL) and filtered. The precipitate was purified by preparative HPLC to afford 6-amino-2-[3,5-dichloro-4-[(6-hydroxy-5-phenylpyridazin-3-yl)oxy]phenyl]-4*H*-1,2,4-triazine-3,5-dione as a white solid (30.4 mg, 9%). ¹H NMR (300 MHz, DMSO-*d*₆) δ 12.48 (s, 1H), 12.29 (s, 1H), 7.95-7.98 (m, 2H), 7.88 (s, 3H), 7.49-7.52 (m, 3H), 6.55 (s, 2H). LCMS (ESI, *m*/*z):* 459 [M+H]⁺.

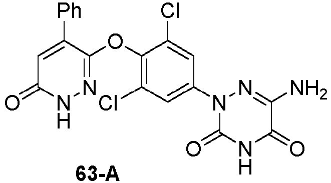

The regioisomeric product 6-amino-2-(3,5-dichloro-4-((6-oxo-4-phenyl-1,6-dihydropyridazin-3-yl)oxy)phenyl)-1,2,4-triazine-3,5(2*H*,4*H*)-dione (63-A) was isolated. ¹H NMR (300 MHz, DMSO-*d*₆) δ 12.40 (s, 1H), 12.28 (s, 1H), 7.87 (s, 1H), 7.79 - 7.82 (m, 2H), 7.55 - 7.59 (m, 3H), 7.13 (s, 1H), 6.53 (s, 2H).

### Example 58. Synthesis of compound 64

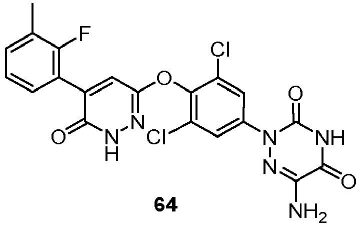

A 250 mL round-bottom flask was charged with Na₂CO₃ (6.00 g, 56.6 mmol), dioxane (45.0 mL), water (15 mL) under N₂. The mixture was stirred for 10 min at rt. 4-Bromo-6-chloropyridazin-3-amine (5.90 g, 28.3 mmol), 2-fluoro-3-methylphenylboronic acid (4.79 g, 31.1 mmol), PdCl₂(dppf)-DCM complex (2.31 g, 2.83 mmol) was added. The reaction was stirred overnight at 110 °C under N₂. The solids were removed by filtration. The organic layers were separated with 400 mL EA:brine (1:1). The organic layer was dried over anhyd. Na₂SO₄, the solids were removed by filtration and the filtrate was concentrated under reduced pressure. The residue was purified on a silica gel column with EA:PE (40:60) to provide 6-chloro-4-(2-fluoro-3-methylphenyl)pyridazin-3-amine as a brown solid (3 g, 37%).

A 100 mL round-bottom flask was charged with *t*-butyl nitrite (3.38 g, 32.8 mmol), cupric chloride (1.77 g, 13.1 mmol) in ACN (30.0 mL). 6-Chloro-4-(2-fluoro-3-methylphenyl)pyridazin-3-amine (2.60 g, 10.9 mmol) was added dropwise at 0 °C. The reaction was stirred overnight at 60 °C and quenched with water (20 mL). The mixture was extracted with EA (3 x 50 mL), washed with brine (50 mL), dried over anhyd. Na₂SO₄, the solids were removed under reduced pressure and the filtrate was concentrated under reduced pressure. The residue was purified on a silica gel with EA:PE (15:85) to provide 3,6-dichloro-4-(2-fluoro-3-methylphenyl)-pyridazine as an off-white solid (1.1 g, 37%).

A 40 mL vial was charged with 3,6-dichloro-4-(2-fluoro-3-methylphenyl)-pyridazine (1.10 g, 4.28 mmol), 4-amino-2,6-dichlorophenyloxidanyl (0.98 g, 5.56 mmol), K₂CO₃ (1.77 g, 12.8 mmol), CuI (0.33 g, 1.71 mmol), DMSO (10 mL) under N₂. The reaction was stirred for 16 h at 90 °C and quenched with water (20 mL). The mixture was extracted with EA (3 x 50 mL), dried over anhyd. Na₂SO₄, the solids were removed by filtration and the filtrate was concentrated under reduced pressure. The residue was purified on a silica gel with EA:PE (1:5) to provide 3,5-dichloro-4-[[6-chloro-5-(2-fluoro-3-methylphenyl)pyridazin-3-yl]-oxy]aniline as a brown solid (1.4 g, 78%).

A 40 mL vial was charged with 3,5-dichloro-4-[[6-chloro-5-(2-fluoro-3-methylphenyl)pyridazin-3-yl]oxy]aniline (1.40 g, 3.51 mmol), NaOAc (1.73 g, 21.1 mmol), AcOH (14 mL) under N₂. The reaction was stirred overnight at 100 °C. The reaction was quenched by water (20 mL), extracted with EA (3 x 50 mL) and the organic layers were washed with brine (50 mL), dried over anhyd. Na₂SO₄, filtered and concentrated under reduced pressure. NaOH (1.40 g, 35.1 mmol)), MeOH (7 mL) and water (7 mL) was added. The reaction was stirred overnight at 120 °C. The reaction was extracted with EA (3 x 50 mL) and the organic layers were combined, washed with brine (50 mL), dried over anhyd. Na₂SO₄, the solids were removed under reduced pressure and the filtrate was concentrated under reduced pressure. The residue was purified on a silica gel column with EA:PE (3:7) to provide 6-(4-amino-2,6-dichlorophenoxy)-4-(2-fluoro-3-methylphenyl)-2*H*-pyridazin-3-one as a brown semi-solid (950 mg, 57%).

Ethyl (2-cyano-2-(2-(3,5-dichloro-4-((5-(2-fluoro-3-methylphenyl)-6-oxo-1,6-dihydropyridazin-3-yl)oxy)phenyl)hydrazineylidene)acetyl)carbamate, a brown solid (800 mg, 55%), was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

2-(3,5-dichloro-4-[[5-(2-fluoro-3-methylphenyl)-6-oxo-1*H*-pyridazin-3-yl]oxy]phenyl)-3,5-dioxo-4*H*-1,2,4-triazine-6-carbonitrile, a brown solid (400 mg, 64%), was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

A 100 mL round-bottom flask was charged with 2-(3,5-dichloro-4-[[5-(2-fluoro-3-methylphenyl)-6-oxo-1*H*-pyridazin-3-yl] oxy]phenyl)-3,5-dioxo-4*H*-1,2,4-tri-azine-6-carbonitrile (250 mg, 0.499 mmol), HCl (2 mL), AcOH (10 mL). The reaction was stirred for 2 h at 120 °C and concentrated reduced pressure. The residue was diluted with sat. NaHCO₃ solution (20 mL), the mixture was extracted with EA (3 x 30 mL) and the organic layers were discarded. The pH of the aq. layer was adjusted to 5~6 with conc. HCl. The solution was extracted with CHCl₃:isopropanol=3:1) (3 x 40 mL), the organic layers were combined, dried over anhyd. Na₂SO₄, the solids were removed by filtration and the filtrate was concentrated under reduced pressure to provide 2-(3,5-dichloro-4-[[5-(2-fluoro-3-methylphenyl)-6-oxo-1*H*-pyridazin-3-yl]oxy]phenyl)-3,5-dioxo-4*H*-1,2,4-triazine-6-carboxylic acid as an off-white solid (110 mg, 41%).

A 25 mL round-bottom flask was charged with 2-(3,5-dichloro-4-[[5-(2-fluoro-3-methylphenyl)-6-oxo-1*H*-pyridazin-3-yl]oxy]phenyl)-3,5-dioxo-4*H*-1,2,4-tri-azine-6-carboxylic acid (110 mg, 0.211 mmol), diphenylphosphoryl azide (174 mg, 0.634 mmol), NEt₃ (85.6 mg, 0.846 mmol), tBuOH (5 mL) under N₂. The reaction was stirred overnight at 85 °C and quenched with water (10 mL). The mixture was extracted with EA (3 x 30 mL), washed with brine (20 mL), dried over anhyd. Na₂SO₄, the solids were removed by filtration and the filtrate was concentrated under reduced pressure. The residue was purified by preparatory TLC with EA:PE (4:1) to provide *t*-butyl-*N*-[2-(3,5-dichloro-4-[[5-(2-fluoro-3-methyl-phenyl)-6-oxo-1*H*-pyridazin-3-yl]oxy]phenyl)-3,5-dioxo-4*H*-1,2,4-triazin-6-yl]carbamate as an off-white solid (80 mg, 46%).

A 50 mL round-bottom flask was charged with *t*-butyl-*N*-[2-(3,5-dichloro-4-[[5-(2-fluoro-3-methylphenyl)-6-oxo-1*H*-pyridazin-3-yl]oxy]phenyl)-3,5-dioxo-4*H*-1,2,4-triazin-6-yl]carbamate (130 mg, 0.220 mmol), DCM (5 mL), TFA (1 mL). The reaction was stirred overnight at rt and concentrated under reduced pressure. The pH of the residue was adjusted to 8 with NaHCO₃ (sat., aq.). The solution was extracted with EA (3 x 30 mL) and the organic layers were combined, washed with brine (30 mL), dried over anhyd. Na₂SO₄, the solids were removed by filtration and the filtrate was concentrated under reduced pressure. The crude product was purified by preparative HPLC to provide 6-amino-2-(3,5-dichloro-4-[[5-(2-fluoro-3-methylphenyl)-6-oxo-1*H*-pyridazin-3-yl]oxy]phenyl)-4*H*-1,2,4-triazine-3,5-dione as a white solid (10.3 mg, 9%).

¹H NMR (300 MHz, DMSO-*d*₆) δ 12.52 (s, 0.3H), 12.29 (s, 0.3H), 7.90 (s, 2H), 7.78 (s, 1H), 7.42-7.44 (m, 2H), 7.21 (t, *J*=7.8 Hz, 1H), 6.48-6.55 (m, 2H), 2.31 (s, 3H).

LCMS (ESI, *m*/*z):* 491 [M+H]⁺.

### Example 59. Synthesis of compound 65

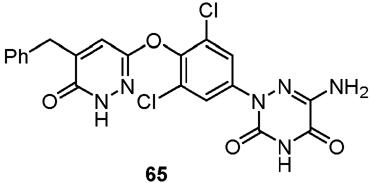

4-Benzyl-3,6-dichloro-pyridazine, a yellow oil (270 mg, 27%), was prepared similarly to 3,6-dichloro-4-isopropylpyridazine.

To a solution of 4-benzyl-3,6-dichloropyridazine (270 mg, 1.13 mmol) and *t-*butyl-*N*-[2-(3,5-dichloro-4-hydroxyphenyl)-3,5-dioxo-4*H*-1,2,4-triazin-6-yl]carbam-ate (483 mg, 1.24 mmol) in DMSO (10 mL) was added K₂CO₃ (468 mg, 3.39 mmol) and CuI (215 mg, 1.13 mmol). The reaction was stirred at 110 °C for 16 h under N₂ and quenched with water (100 mL). The mixture was extracted with EA (3 x 100 mL) and the organic layers were combined, washed with brine (100 mL), dried over anhyd. Na₂SO₄, the solids were removed by filtration and the filtrate was concentrated under reduced pressure. The residue was purified on a silica column with MeOH:DCM (4:96) to afford *t*-butyl-*N*-(2-[4-[(5-benzyl-6-chloropyridazin-3-yl)oxy]-3,5-dichlorophenyl]-3,5-dioxo-4*H*-1,2,4-triazin-6-yl)carbamate as a brown solid (400 mg, 36%).

To a solution of 6-amino-2-[4-[(5-benzyl-6-chloropyridazin-3-yl)oxy]-3,5-dichlorophenyl]-4*H*-1,2,4-triazine-3,5-dione (240 mg, 0.49 mmol) in AcOH (6 mL) was added NaOAc (160 mg, 1.95 mmol). The reaction was stirred overnight at 100 °C and cooled to rt. The reaction solution was poured into water (100 mL) and filtered. The precipitate was purified by preparative HPLC to afford 6-amino-2-[4-[(5-benzyl-6-hydroxypyridazin-3-yl)oxy]-3,5-dichlorophenyl]-4*H*-1,2,4-triazine-3,5-dione as a white solid (56.6 mg, 24%).

¹H NMR (300 MHz, DMSO-*d*₆) δ 12.27-12.30 (m, 2H), 7.85 (s, 2H), 7.24-7.37 (m, 6H), 6.53 (s, 2H), 3.87 (s, 2H).

LCMS (ESI, *m*/*z):* 495 [M+Na]⁺.

### Example 60. Synthesis of compound 66

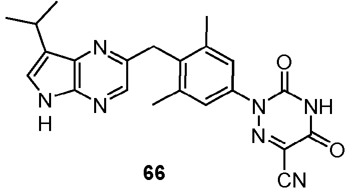

2-[(4-Benzyloxy-2,6-dimethyl-phenyl)methyl]-7-isopropyl-5-(*p-*tolylsulfonyl)pyrrolo [2,3-*b*]pyrazine as a yellow solid (770 mg, 70%) was prepared similarly as described for 5-[(4-benzyloxy-2,6-dimethyl-phenyl)methyl]-3-isopropyl-1-(p-tolylsulfonyl)pyrrolo[3,2-*b*]pyridine.

A mixture of 2-[(4-benzyloxy-2,6-dimethyl-phenyl)methyl]-7-isopropyl-5-(*p-*tolylsulfonyl)pyrrolo[2,3-*b*] pyrazine (610 mg, 1.13 mmol) in DCM (20 mL) and BBr₃ (1.42 g, 5.65 mmol, 544.54 µL) was added. The reaction mixture was degassed and purged with N₂ for 3 times, and then the mixture was stirred at 0 °C for 1 h. The reaction mixture was quenched by addition NaHCO₃ (15 mL) at 0 °C, and then diluted with H₂O (25 mL) and extracted with DCM (3 x 45 mL). The combined organic layers were dried over MgSO₄, the solids were removed by filtration and the filtrate was concentrated under reduced pressure. The crude was purified by flash silica gel chromatography (EA:PE=0~10%) to afford 4-[[7-isopropyl-5-(*p*-tolylsulfonyl)pyrrolo-[2,3-*b*]pyrazin-2-yl]methyl]-3,5-dimethyl-phenol as a yellow solid (400 mg, 79%).

[4-[[7-Isopropyl-5-(*p*-tolylsulfonyl)pyrrolo[2,3-*b*]pyrazine-2-yl]methyl]-3,5-dimethyl-phenyl] trifluoromethanesulfonate, a white solid (430 mg, 83%) was prepared similarly as described for [4-[[3-isopropyl-1-(*p*-tolylsulfonyl)pyrrolo[3,2-*b*]pyridin-5-yl]methyl]-3,5-dimethyl-phenyl] trifluoromethanesulfonate.

A mixture of [4-[[7-isopropyl-5-(*p*-tolylsulfonyl)pyrrolo[2,3-*b*]pyrazin-2-yl]methyl]-3,5-dimethyl- phenyl] trifluoromethanesulfonate (280 mg, 481 µmol), *t*-butyl carbamate (226 mg, 1.93 mmol), XantPhos (68.9 mg, 144 µmol), Pd(OAc)₂ (10.8 mg, 48.1 µmol) and Cs₂CO₃ (627 mg, 1.93 mmol) in dioxane (10 mL) was degassed and purged with N₂, then the mixture was stirred at 100 °C for 4 h under N₂. The reaction mixture was concentrated under reduced pressure, then the residue was diluted with H₂O (20 mL) and extracted with EA (3 x 20 mL). The combined organic layers were washed with brine (20 mL), dried over Na₂SO₄, filtered and concentrated under reduced pressure to give crude product, which was purified by silica gel chromatography (EA:PE=0~15%) to give *t*-butyl-*N*-[4-[[7-isopropyl-5-(*p*-tolylsulfonyl)-pyrrolo[2,3-*b*]-pyrazin-2-yl]methyl]-3,5-dimethylphenyl]carbamate as a yellow gum (290 mg, 76%).

To a solution of *t*-butyl-*N*-[4-[[7-isopropyl-5-(*p*-tolylsulfonyl)-pyrrolo-[2,3-b]pyrazin-2-yl]methyl]-3,5-dimethyl-phenyl]carbamate (356 mg, 454 µmol) in DCM (3 mL) was added TFA (518 mg, 4.54 mmol, 336.27 µL). The mixture was stirred at 20°C for 2 h. The reaction mixture was quenched with sat. aq. NaHCO₃ (5 mL) at 20°C, then diluted with H₂O (5 mL) and extracted with DCM (3 x 20 mL). The combined organic layers were washed with brine (20 mL), dried over MgSO₄, filtered and concentrated under reduced pressure to give a residue, which was purified by flash silica gel chromatography (EA:PE=0~30%) to give 4-[[7-isopropyl-5-(p-tolyl-sulfonyl)pyrrolo[2,3-*b*]pyrazin-2-yl]methyl]-3,5-dimethylaniline as a yellow solid (183 mg, 89%).

Ethyl *N*-[2-cyano-2-[[4-[[7-isopropyl-5-(*p*-tolylsulfonyl)pyrrolo-[2,3-*b*]pyrazin-2-yl]-methyl]-3,5-dimethyl-phenyl]hydrazono]acetyl]carbamate, a yellow solid (90 mg, 47%), was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

To a solution of ethyl *N*-[2-cyano-2-[[4-[[7-isopropyl-5-(p-tolylsulfonyl)pyrrolo[2,3-b]pyrazine-2-yl]methyl]-3,5-dimethyl-phenyl]hydrazono]-acetyl]carbamate (146 mg, 237 µmol) in AcOH (15 mL) was added NaOAc (97.3 mg, 1.19 mmol).The mixture was stirred at 130 °C for 3 h. The reaction mixture was concentrated under reduced pressure, adjusted to pH 7~8 by NaHCO₃ (sat., aq. 5 mL) then extracted with EA (3 x 20 mL). The combined organic layers were washed with brine (20 mL), dried over anhyd. Na₂SO₄, the solids were removed under reduced pressure and the filtrate was concentrated under reduced pressure to give 2-[4-[[7-isopropyl-5-(p-tolylsulfonyl)pyrrolo[2,3-*b*]pyrazin-2-yl]methyl]-3,5-di-methyl-phenyl]-3,5-dioxo-1,2,4-triazine-6-carbonitrile as a yellow solid (100 mg, crude).

Tosyl group deprotection to give 2-[4-[(7-isopropyl-5*H*-pyrrolo[2,3-*b*]pyrazin-2-yl)methyl]-3,5-dimethyl-phenyl]-3,5-dioxo-1,2,4-triazine-6-carbonitrile, a yellow solid (34.8 mg, 48%), was prepared similarly as described for methyl 5-[2,6-dichloro-4-(6-cyano-3,5-dioxo-4*H*-1,2,4-triazin-2-yl)phenoxy]-1*H*-indole-3-carboxylate. ¹H NMR (400MHz, DMSO-*d*₆) δ=12.98 (br s, 1H), 11.57 (br s, 1H),7.96 (s, 1H), 7.53 (s, 1H), 7.16 (s, 2H), 4.29 (s, 2H), 3.19 -3.07 (m, 1H), 2.43 (s, 6H), 1.32 (d, J=6.9 Hz, 6H). LCMS (ESI, *m*/*z):* 416.3[M+H]+.

### Example 61. Synthesis of compound 67 and 67-A

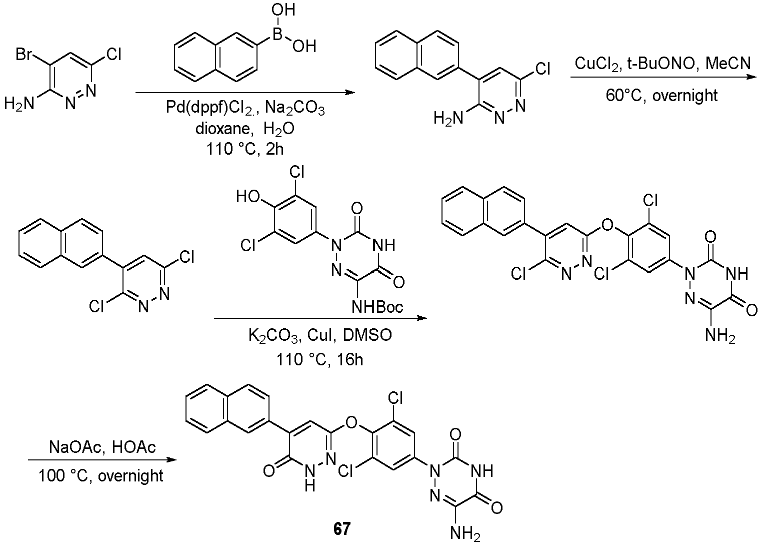

A 100 mL round-bottom flask was charged with sodium carbonate (2.03 g, 19.190 mmol), dioxane (20 mL) and water (4 mL). The mixture was stirred for 10 min at rt. 4-bromo-6-chloropyridazin-3-amine (2 g, 9.595 mmol), 2-naphthalene boronic acid (1.82 g, 10.555 mmol) and PdCl₂(dppf) (783.57 mg, 0.960 mmol) was added. The mixture was stirred for 2 h at 110 °C under N₂. The reaction mixture was diluted with EA (20 mL) and filtered through packed celite, the celite pad was washed with EA (2 x 10 mL), and the filtrate was washed with brine (20 mL). The organic layer was dried over anhydr. sodium sulfate, the solids were removed by filtration and the filtrate was concentrated under reduced pressure. The residue was chromatographed on a silica gel column (PE:EA=10:1) to provide 6-chloro-4-(naphthalen-1-yl)pyridazin-3-amine as a yellow solid (1.75 g, 68%). LCMS (ESI, *m*/*z*): 256 [M+H]⁺.

To a solution of CuCl₂ (1.10 g, 8.212 mmol) in CH₃CN (5 mL) was added *t-*butyl nitrite (2.12 g, 20.531 mmol) at 0°C was added 6-chloro-4-(naphthalen-2-yl)pyridazin-3-amine (1.75 g, 6.844 mmol) in CH₃CN (15 mL) dropwise. The reaction mixture was stirred 18h at 60 °C. The reaction mixture was cooled to rt and filtered through packed celite, the celite pad was washed with EA (3 x 10 mL), and then quenched with water (10 mL) and extracted with EA (3 x 20 mL). The combined organic layers were washed with brine (20 mL), dried over anhydr. sodium sulfate. After filtration, the filtrate was concentrated. The residue was purified by silica gel column chromatography (EA:PE=1:10) to afford 3,6-dichloro-4-(naphthalen-2-yl)pyridazine as a yellow solid (990 mg, 50%).LCMS (ESI, *m*/*z):* 275 [M+H]⁺.

To a solution of 3,6-dichloro-4-(naphthalen-2-yl)pyridazine (400.00 mg, 1.454 mmol), *t*-butyl *N*-[2-(3,5-dichloro-4-hydroxyphenyl)-3,5-dioxo-4*H*-1,2,4-triazin-6-yl]carbamate (565.83 mg, 1.454 mmol) and K₂CO₃ (602.79 mg, 4.362 mmol) in DMSO (8 mL) was added CuI (83.07 mg, 0.436 mmol) under N₂. The solution was stirred overnight at 110 °C then quenched with water (10 mL). The mixture was acidified to pH ~5 with HCl (1 M, aq.), then extracted with EA (3 x 30 mL). The combined organic layers were washed with brine (1 x 20 mL), dried over anhydr. sodium sulfate, the solids were removed by filtration, and the filtrate was concentrated under reduced pressure. The residue was purified by silica gel column chromatography (EA:PE=1:1) to afford 6-amino-2-(3,5-dichloro-4-[[6-chloro-5-(naphthalen-2-yl)pyridazin-3-yl]oxy]phenyl)-4*H*-1,2,4-triazine-3,5-dione as a red solid (300 mg, 37%). LCMS (ESI, *m*/*z):* 527 [M+H]⁺.

To a solution of 6-amino-2-(3,5-dichloro-4-[[6-chloro-5-(naphthalen-2-yl)pyridazin-3-yl]oxy]phenyl)-4*H*-1,2,4-triazine-3,5-dione (300 mg, 0.568 mmol) in acetic acid (6 mL) was added NaOAc (233.16 mg, 2.842 mmol). The mixture was stirred overnight at 100 °C. The reaction mixture was cooled to rt, quenched with water (20 mL) and then stirred for 10 min. The solid was filtered and washed with water (2 x 10 mL) and PE (2 x 5 mL), then dried under reduced pressure then purified by preparative HPLC (XBridge Prep OBD C18 Column, 19 x 250 mm, 5 um; Mobile Phase A:Water (0.05%TFA ), Mobile Phase B: ACN; Flow rate: 25 mL/min; Gradient: 49 %B to 69 %B in 7 min; 220 nm) to afford 6-amino-2-(3,5-dichloro-4-[[5-(naphthalen-2-yl)-6-oxo-1*H*-pyridazin-3-yl]oxy]phenyl)-4*H-*1,2,4-triazine-3,5-dione as a white solid (40.4 mg, 14%). ¹H NMR (300 MHz, DMSO-*d*₆) δ 12.55 (s, 1H), 12.29 (s, 1H), 8.67 (s, 1H), 7.97 - 8.07 (m, 5H), 7.89 (s, 2H), 7.57 - 7.64 (m, 2H), 6.55 (br, 2H). LCMS (ESI, *m*/*z):* 509 [M+H]⁺.

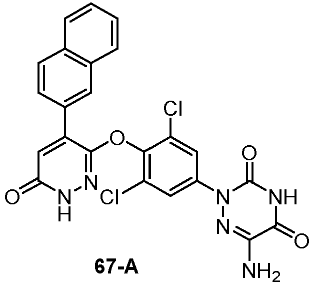

6-amino-2-(3,5-dichloro-4-((4-(naphthalen-2-yl)-6-oxo-1,6-dihydropyridazin-3-yl)oxy)phenyl)-1,2,4-triazine-3,5(2*H*,4*H*)-dione (67-A) was isolated during the synthesis of 6-amino-2-(3,5-dichloro-4-[[5-(naphthalen-2-yl)-6-oxo-1*H-*pyridazin-3-yl]oxy]phenyl)-4*H*-1,2,4-triazine-3,5-dione. ¹H NMR (300 MHz, DMSO-*d*₆) 12.44 (br, 1H), 12.27 (br, 1H), 8.38 (br,1H), 8.03 - 8.11 (m, 3H), 7.88 - 7.93 (m, 3H), 7.60 - 7.66 (m, 2H), 7.26 (s, 1H), 6.52 (br, 2H).

Compounds 68, 69, 70, 71, 72 were prepared similarly as described in Example 61. The regioisomer products 68-A, 69-A, 70-A, 71-A, and 72-A were also isolated.

| Structure | LCMS (ESI, *m*/*z)* | ¹H NMR |
|---|---|---|
| 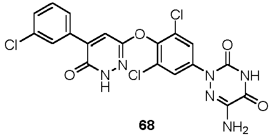 6-Amino-2-(3,5-dichloro-4-((5-(3-chlorophenyl)-6-oxo-1,6-dihydropyridazin-3-yl)oxy)phenyl)-1,2,4-triazine-3,5(2*H*,4*H*)-dione | 493 [M+H]⁺ | ¹H (300 MHz, DMSO-*d*₆) δ 12.55 (br, 1H), 12.28 (br, 1H), 8.09 (s, 1H), 7.99 (s, 1H), 7.88 - 7.94 (m, 3H), 7.53 - 7.57 (m, 2H), 6.53 (s, 2H). |
| 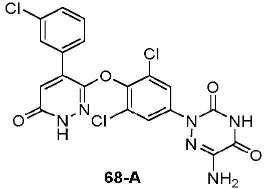 6-amino-2-(3,5-dichloro-4-((4-(3-chlorophenyl)-6-oxo-1,6-dihydropyridazin-3-yl)oxy)phenyl)-1,2,4-triazine-3,5(2*H*,4*H*)-dione | 493 [M+H]⁺ | ¹H NMR (300 MHz, DMSO-*d*₆) δ 12.45 (s, 1H), 12.28 (s, 1H), 7.85 - 7.88(m, 3H), 7.77 (d, J= 6.6Hz, 1H), 7.60 - 7.62 (m, 2H), 7.21 (s, 1H), 6.55 (s, 2H). |
| 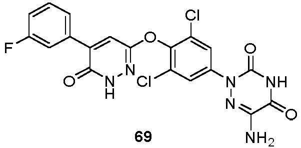 6-Amino-2-(3,5-dichloro-4-((5-(3-fluorophenyl)-6-oxo-1,6-dihydropyridazin-3-yl)oxy)phenyl)-1,2,4-triazine-3,5(2*H*,4*H*)-dione | 477 [M+H]⁺ | ¹H NMR (300 MHz, DMSO-*d₆*) δ 12.46 (br, 1H), 12.28 (s, 1H), 7.88 (s, 2H), 7.61 - 7.68 (m, 3H), 7.34 - 7.42 (m, 1H), 7.21 (s, 1H), 6.54 (br, 2H). |
| 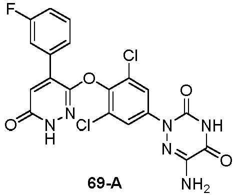 6-amino-2-(3,5-dichloro-4-((4-(3-fluorophenyl)-6-oxo-1,6-dihydropyridazin-3-yl)oxy)phenyl)-1,2,4-triazine-3,5(2*H*,4*H*)-dione | 477 [M+H]⁺ | ¹H NMR (300 MHz, DMSO- *d*₆) δ 12.46 (br, 1H), 12.28 (s, 1H), 7.88 (s, 2H),7.61 - 7.68 (m, 3H), 7.34 - 7.42 (m, 1H), 7.21 (s, 1H), 6.54 (br, 2H). |
| 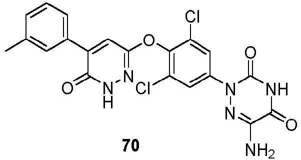 6-Amino-2-(3,5-dichloro-4-((6-oxo-5-(m-tolyl)-1,6-dihydropyridazin-3-yl)oxy)phenyl)-1,2,4-triazine-3,5(2*H*,4*H*)-dione | 473 [M+H]⁺ | ¹H NMR (300MHz, DMSO-*d₆*) δ 12.28 - 12.45 (m, 2H), 7.84 - 7.88 (m, 3H), 7.74 - 7.77 (m,2H), 7.30 - 7.40 (m, 2H), 6.54 (s, 2H), 2.08 (s, 3H). |
| 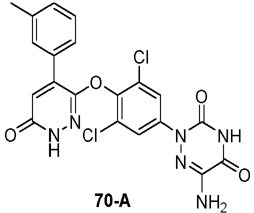 6-amino-2-(3,5-dichloro-4-((6-oxo-4-(m-tolyl)-1,6-dihydropyridazin-3-yl)oxy)phenyl)-1,2,4-triazine-3,5(2H,4H)-dione | 473 [M+H]⁺ | ¹H NMR (300MHz, DMSO-*d*₆) δ 12.28 - 12.38 (m, 2H), 7.87 (s, 2H),7.59 - 7.61 (m, 2H), 7.42 - 7.47 (m, 2H), 7.35 - 7.37 (m, 1H), 7.10 (d, J = 2.1 Hz, 1H), 6.54 (s, 2H), 2.08 (s, 3H). |
| 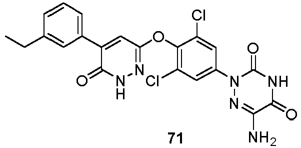 6-Amino-2-(3,5-dichloro-4-((5-(3-ethylphenyl)-6-oxo-1,6-dihydro-pyridazin-3-yl)oxy)phenyl)-1,2,4-triazine-3,5(2*H*,4*H*)-dione | 487 [M+H]⁺ | ¹H NMR (300MHz, DMSO-*d₆*) δ 12.29 - 12.45 (m, 2H), 7.79 - 7.88 (m, 3H), 7.77 - 7.79 (m, 2H), 7.33 - 7.43 (m, 2H), 6.54 (br, 2H), 2.64 - 2.74 (m, 2H), 1.23 (t, *J* = 7.5 Hz, 3H). |
| 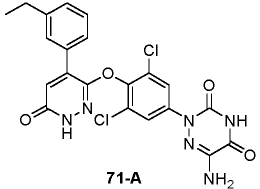 6-amino-2-(3,5-dichloro-4-((4-(3-ethylphenyl)-6-oxo-1,6-dihydropyridazin-3-yl)oxy)phenyl)-1,2,4-triazine-3,5(2H,4H)-dione | 487 [M+H]⁺ | ¹H NMR (300MHz, DMSO- *d₆*) δ 12.28 - 12.38 (m, 2H), 7.87 (s, 2H), 7.61 - 7.65 (m, 2H), 7.45 - 7.50 (m, 1H), 7.38 - 7.41 (m, 1H), 7.11 (d, J = 1.8 Hz, 1H), 6.54 (s, 2H), 2.67 - 2.74 (m, 2H), 1.19 - 1.26 (m, 3H). |
| 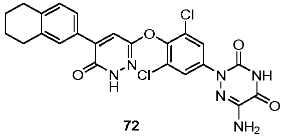 6-Amino-2-(3,5-dichloro-4-((6-oxo-5-(5,6,7,8-tetrahydro-naphthalen-2-yl)-1,6-dihydro-pyridazin-3-yl)oxy)phenyl)-1,2,4-triazine-3,5(2*H*,4*H*)-dione | 513 [M+H]⁺ | ¹H NMR (300 MHz, DMSO-*d₆*) δ 12.23 - 12.41 (m, 2H), 7.87 (s, 2H), 7.80 (s, 1H), 7.67 - 7.69 (m, 2H), 7.14 - 7.17 (m, 1H), 6.53 (br, 2H), 2.77 (br, 4H), 1.76 (br, 4H). |
| 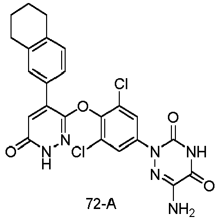 6-amino-2-(3,5-dichloro-4-[[6-oxo-4-(5,6,7,8-tetrahydronaphthalen-2-yl)-1*H-*pyridazin-3-yl]oxy]phenyl)-4*H*-1,2,4-triazine-3,5-dione | 513 [M+H]⁺ | ¹H NMR (300 MHz, DMSO-*d₆*) δ 12.34 - 12.42 (m, 2H), 7.86 (s, 2H), 7.48 - 7.52 (m, 2H), 7.21 - 7.23 (m, 1H), 7.06 (s, 1H), 6.53 (br, 2H), 2.78 (br, 4H), 1.76 (br, 4H). |

### Example 62: Synthesis of compound 73

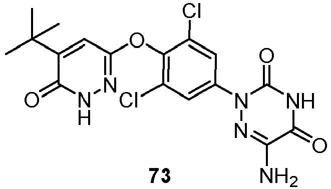

6-Amino-2-(4-((5-(*t*-butyl)-6-oxo-1,6-dihydropyridazin-3-yl)oxy)-3,5-dichlorophenyl)-1,2,4-triazine-3,5(2*H*, 4*H*)-dione was prepared similarly as described for 6-amino-2-(3,5-dichloro-4-[[5-(oxan-4-yl)-6-oxo-1*H*-pyridazin-3-yl]oxy]phenyl)-4*H*-1,2,4-triazine-3,5-dione. ¹H NMR (300 MHz, DMSO-*d*₆) δ 12.29 (s, 1H), 12.13 (s, 1H), 7.86 (s, 2H), 7.33 (s, 1H), 6.54 (s, 2H), 1.35 (s, 9H). LCMS (ESI, *m*/*z):* 439 [M+H]⁺.

### Example 63: Synthesis of compound 74

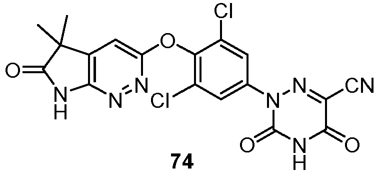

3-Chloro-7-(4-methoxybenzyl)-5,5-dimethyl-5,7-dihydro-6*H*-pyrrolo[2,3-*c*]pyridazin-6-one was prepared according to the literature procedure (Tet. Lett. 2015, 56, 772-774).

A mixture of 3-chloro-7-[(4-methoxyphenyl)methyl]-5,5-dimethyl-pyrrolo[2,3-c]pyridazin-6-one (6 g, 18.88 mmol), *N*'-(3,5-dichloro-4-hydroxy-phenyl)-*N,N-*dimethyl-formamidine (4.40 g, 18.88 mmol), Pd(dbtpf)Cl₂ (1.23 g, 1.89 mmol), Cs₂CO₃ (18.46 g, 56.64 mmol) in dioxane (100 mL) was degassed and purged with N₂ for 3 times. The mixture was stirred at 100 °C for 16 h under N₂. The reaction mixture was diluted with H₂O (200 mL) and extracted with EA (3 x 150 mL). The combined organic layers were washed with brine (200 mL), dried over Na₂SO₄, the solids were removed by filtration and the filtrate was concentrated under reduced pressure. The crude was purified by silica gel chromatography (EA:PE=0~50%) to give *N'*-[3,5-dichloro-4-[7-[(4-methoxyphenyl)methyl]-5,5-dimethyl-6-oxo-pyrrolo[2,3-*c*]pyrida-zin-3-yl]oxy-phenyl]-*N*,*N*-dimethyl-formamidine as a brown solid(6.5 g, 67%).

To a solution of *N'*-[3,5-dichloro-4-[7-[(4-methoxyphenyl)methyl]-5,5-dimethyl-6-oxo-pyrrolo[2,3-*c*]pyridazin-3-yl]oxy-phenyl]-*N,N*-dimethyl-formamidine (4.5 g, 8.75 mmol) in 2-propanol (80 mL) was added NH₂NH₂ hydrate (4.38 g, 87.48 mmol, 4.25 mL) at 20 °C. After the addition, the mixture was stirred at 80 °C for 16 h. The reaction mixture was quenched by H₂O (160 mL) and extracted with EA (3 x 100 mL). The combined organic layers were washed with brine (3 x 50 mL), dried over Na₂SO₄, the solids were removed by filtration and the filtrate was concentrated under reduced pressure. The crude product was purified by flash silica gel chromatography (EA in PE: 0~50%) to afford 3-(4-amino-2,6-dichloro-phenoxy)-7-[(4-methoxyphenyl)methyl]-5,5-dimethyl-pyrrolo[2,3-c]pyridazin-6-one as a white solid (3.2 g, 80%).

Ethyl (2-cyano-2-(2-(3,5-dichloro-4-((7-(4-methoxybenzyl)-5,5-dimethyl-6-oxo-6,7-dihydro-5*H*-pyrrolo[2,3-*c*]pyridazin-3-yl)oxy)phenyl)hydrazine-ylidene)acetyl)carbamate (6.46 g, 5.16 mmol, 74%, 50% purity), an orange solid, was prepared similarly as described for ethyl (2-cyano-2-(2-(3,5-dichloro-4-((1-tosyl-1*H*-indol-5-yl)oxy)phenyl)-hydrazineylidene)-acetyl) carbamate.

To a solution of ethyl *N*-[2-cyano-2-[[3,5-dichloro-4-[7-[(4-methoxyphenyl)methyl]-5,5-dimethyl-6-oxo-pyrrolo[2,3-*c*]pyridazin-3-yl]oxyphenyl]hydrazono]acetyl]carbamate (6.46 g, 5.16 mmol) in DMF (60 mL) was added Et₃N (2.61 g, 25.78 mmol, 3.59 mL) in one portion at 20 °C. The mixture was stirred at 90 °C for 16 h. The reaction mixture was diluted with H₂O (150 mL) and extracted with EA (3 x 150 mL). The combined organic layers were washed with brine (3 x 100 mL), dried over Na₂SO₄, the solids were removed by filtration and the filtrate was concentrated under reduced pressure. The crude product was purified by flash chromatography (MeOH:DCM=0~5%) to afford 2-[3,5-dichloro-4-[7-[(4-methoxyphenyl)methyl]-5,5-dimethyl-6-oxo-pyrrolo[2,3-*c*]pyridazin-3-yl]oxy-phenyl] -3,5-dioxo-1,2,4-triazine-6-carbonitrile as a brown solid (4.1 g, 96%).

To a solution of 2-[3,5-dichloro-4-[7-[(4-methoxyphenyl)methyl]-5,5-dimethyl-6-oxo-pyrrolo[2,3-*c*]pyridazin-3-yl]oxy-phenyl]-3,5-dioxo-1,2,4-triazine-6-carbonitrile (780 mg, 1.34 mmol) in ACN (10 mL) and H₂O (10 mL) was added CAN (2.21 g, 4.03 mmol, 2.01 mL) at 0 °C The mixture was stirred at 25°C for 2 h. The reaction mixture was diluted with H₂O (20 mL) and extracted with EA (3 x 30 mL). The combined organic layers were washed with brine (3 x 20 mL), dried over Na₂SO₄, filtered and concentrated under reduced pressure to give a residue. The residue was purified by preparative HPLC ([Column: Welch Xtimate C18 150 x 30mm x 5um; Mobile phase: from 15% ACN in water (0.225%NH₃.H2O) to 45% ACN in water (0.225%NH₃.H₂O)]) to give 2-[3,5-dichloro-4-[(5,5-dimethyl-6-oxo-7*H*-pyrrolo[2,3-*c*]pyridazin-3-yl)oxy]phenyl]3,5-dioxo-1,2,4-triazine - 6-carbonitrile as a white solid (61.1 mg, 10%). ¹H NMR (400MHz, DMSO-*d*₆) δ =11.55 (br s, 1H), 7.82 (s, 1H), 7.80 (s, 2H), 7.09 (br s, 3H), 1.39 (s, 6H). LCMS (ESI, *m*/*z):* 460.1[M+H]⁺.

### Example 64: Synthesis of compound 75

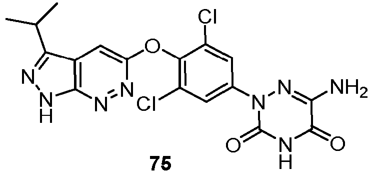

To a solution of 2,2,6,6-tetramethylpiperidine (4.55 g, 32.22 mmol, 5.47 mL) in THF (20 mL) at -30 °C was added dropwise *n*-BuLi (2.5 M, 11.28 mL) over 30 min. After addition, the mixture was stirred at -30 °C for 30 min, and then 3,6-dichloropyridazine (3 g, 20.14 mmol) and 2-methylpropanal (1.60 g, 22.15 mmol, 2.02 mL) in THF (20 mL) was added dropwise at -65 °C. The mixture was stirred at -65 °C for 1 h. The reaction mixture was quenched by NH₄Cl (sat., aq., 10 mL) at -60 °C. The mixture was worked up with a previous batch and then diluted with H₂O (100 mL) and extracted with EA (100 mL x 3). The combined organic layers were washed with brine (80 mL), dried over Na₂SO₄, the solids were removed by filtration and the filtrate was concentrated under reduced pressure. The crude product was purified by silica gel chromatography (EA:PE=0~15%) to afford 1-(3,6-dichloropyridazin-4-yl)-2-methylpropan-1-ol as a yellow liquid (3.34 g, 15%).

To a suspension of NaH (4.11 g, 102.68 mmol, 60% purity) in THF (40 mL) was added 1-(3,6-dichloropyridazin-4-yl)-2-methylpropan-l-ol (2.27 g, 10.27 mmol) and TBSCl (3.10 g, 20.54 mmol, 2.52 mL) in THF (10 mL) at 0°C. The mixture was stirred at 0 °C for 1 hr. The reaction mixture was quenched with H₂O (10 mL), then diluted with H₂O (50 mL) and extracted with EA (3 x 50 mL). The combined organic layers were washed with brine (3 x 50 mL), dried over anhydr. Na₂SO₄, filtered and concentrated under reduced pressure to give a residue, which was purified by flash silica gel chromatography (0~5% EA in PE) to afford 4-(1-((*t*-butyldimethylsilyl)oxy)-2-methylpropyl)-3,6-dichloropyridazine as a yellow liquid (2.49 g, 72%).

To a solution of 4-amino-2,6-dichloro-phenol (3 g, 16.85 mmol) in toluene (30 mL) was added DMF-DMA (2.21 g, 18.54 mmol, 2.46 mL). After the addition, the mixture was stirred at 100 °C for 2 h. The mixture was concentrated to give a residue, which was triturated (PE:EA=6:1) to give *N'*-(3,5-dichloro-4-hydroxyphenyl)-*N,N-*dimethylformimidamide as a brown solid (4.05 g).

*N'*-(4-((5-(1-((*t*-Butyldimethylsilyl)oxy)-2-methylpropyl)-6-chloro-pyridazin-3-yl)oxy)-3,5-dichlorophenyl)-*N*,*N*-dimethylformimidamide (3.86 g, 74%), a yellow solid, was prepared similarly as described for 3,5-dichloro-4-[(6-chloro-5-isopropylpyridazin-3-yl)oxy]aniline with the exception that the reaction was heated for 16 h at 90°C.

*N'*-(4-((5-(1-((*t*-Butyldimethylsilyl)oxy)-2-methylpropyl)-6-chloro-pyridazin-3-yl)oxy)-3,5-dichlorophenyl)-*N*,*N*-dimethylformimidamide (3.85 g, 6.84 mmol) in THF (30 mL) was added TBAF (1 M, 13.68 mL) at 25 °C and the mixture was stirred at 25 °C for 6 h. The mixture was quenched with NH₄Cl (sat., aq., 50 mL) and extracted with EA (3 x 50 mL). The combined organic layers were washed with brine (50 mL), dried over anhydr. Na₂SO₄, filtered and concentrated to give *N'*-(3,5-dichloro-4-((6-chloro-5-(1-hydroxy-2-methylpropyl)pyridazin-3-yl)oxy) phenyl)-N,N-dimethylformimidamide (3.67 g, crude) as a yellow solid.

*N'*-(3,5-Dichloro-4-((6-chloro-5-(1-hydroxy-2-methylpropyl)pyridazin-3-yl)oxy)phenyl)-*N*,*N*-dimethylformimidamide (3.6 g, crude) in DCM (40 mL) was added Dess-Martin reagent (6.84 g, 16.12 mmol, 4.99 mL) at 25 °C in one portion and the mixture was stirred at 25 °C for 2 h. The mixture diluted with NaHCO₃ (sat., aq., 50 mL) and extracted with DCM (2 x 50 mL). The combined organic layers were washed with brine (50 mL), dried over anhydr. Na₂SO₄, the solids were removed by filtration and the filtrate was concentrated under reduced pressure to give a residue, which was purified by combi flash (EA:PE=0-40%) to give *N'*-(3,5-dichloro-4-((6-chloro-5-isobutyrylpyridazin-3-yl)oxy)phenyl)-*N,N-*dimethylformimidamide as a yellow oil (2.23 g, 53%).

To a solution of N'-(3,5-dichloro-4-((6-chloro-5-isobutyrylpyridazin-3-yl)oxy)phenyl)-*N*,*N*-dimethylformimidamide (1.3 g, 3.13 mmol) in 2-propanol (20 mL) was added NH₂NH₂ hydrate (5.4 g, 107.87 mmol, 5.24 mL) and the mixture was stirred at 90°C for 72 h under N₂. The mixture was diluted with H₂O (50 mL) and extracted with EA (3 x 50 mL). The combined organic layers were washed with brine (50 mL), dried over anhydr. Na₂SO₄, the solids were removed by filtration and the filtrate was concentrated under reduced pressure to give a residue, which was purified by silica column chromatography (EA in PE : 0~40%) to afford 3,5-dichloro-4-[(3-isopropyl-1*H*-pyrazolo[3,4-c]pyridazin-5-yl)oxy]aniline as a yellow solid (435 mg, 40%). ¹H NMR (400MHz, DMSO-*d*₆) δ 13.83 (s, 1H), 8.03 (s, 1H), 6.71 (s, 2H), 5.63 (s, 2H), 3.45 - 3.36 (m,1H), 1.37 (d, J=6.9 Hz, 6H). LCMS (ESI, *m*/*z):* 338[M+H]⁺.

Ethyl (2-cyano-2-(2-(3,5-dichloro-4-((3-isopropyl-1*H*-pyrazolo[3,4-c]pyridazin-5-yl)oxy)phenyl)hydrazineylidene)acetyl)carbamate (960 mg), a yellow solid, was prepared similarly as described for ethyl (2-cyano-2-(2-(3,5-dichloro-4-((1-tosyl-1*H-*indol-5-yl)oxy)phenyl)-hydrazineylidene)-acetyl) carbamate, and was used in the subsequent step without further purification.

2-(3,5-Dichloro-4-((3-isopropyl-1*H*-pyrazolo[3,4-*c*]pyridazin-5-yl)oxy)phenyl)-3,5-dioxo-2,3,4,5-tetrahydro-1,2,4-triazine-6-carbonitrile (compound 75A, 910 mg, 76%), a red solid, was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile with the exception that the reaction mixture was heated at 120 °C for 12 h and that NaOAc was used. ¹H NMR (400MHz, DMSO-*d*₆) δ =13.97 (s, 1H), 13.28 (br s, 1H), 8.31(s, 1H), 7.82 (s, 2H), 3.46 - 3.39 (m,1H), 1.40 (d, J=7.0 Hz, 6H). LCMS (ESI, *m*/*z*): 459[M+H]⁺.

2-(3,5-Dichloro-4-((3-isopropyl-1*H*-pyrazolo[3,4-c]pyridazin-5-yl)oxy)phenyl)-3,5-dioxo-2,3,4,5-tetrahydro-1,2,4-triazine-6-carboxylic acid (415 mg, 37%), a yellow solid, was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid, which used in next step without further purification. LCMS (ESI, *m*/*z):* 478[M+H]⁺.

To a solution of 2-(3,5-dichloro-4-((3-isopropyl-1*H*-pyrazolo[3,4-c]pyridazin-5-yl)oxy)phenyl)-3,5-dioxo-2,3,4,5-tetrahydro-1,2,4-triazine-6-carboxylic acid (410 mg, 0.857 mmol) in DMF (8 mL) was added Et₃N (260 mg, 2.57 mmol, 0.357 mL) at 0 °C, followed by slow addition of DPPA (472 mg, 1.71 mmol, 0.371 mL) under N₂. The mixture was stirred at 25 °C for 3 h. Then H₂O (1 mL) was added and the reaction was heated at 100 °C for additional 2 h. The mixture was diluted with H₂O (50 mL) and extracted with EA (3 x 30 mL). The combined organic layers were washed with brine (50 mL), dried over anhydr. Na₂SO₄, the solids were removed by filtration and the filtrate was concentrated under reduced pressure. The crude was purified by preparative HPLC [Phenomenex Luna C18 100 x 30 mm x 5 um; Mobile phase: from 35% ACN in water (0.225%FA) to 65% ACN in water (0.225%FA)] to give 6-amino-2-(3,5-dichloro-4-((3-isopropyl-1*H*-pyrazolo[3,4-*c*]pyridazin-5-yl)oxy)phenyl)-1,2,4-triazine-3,5(2*H*,4*H*)-dione as a white solid (120 mg, 31%). ¹H NMR (400MHz, DMSO-*d*₆) δ =13.92 (s, 1H), 12.28 (br s, 1H), 8.24 (s, 1H), 7.89 (s, 2H), 6.52 (s, 2H), 3.47 - 3.38 (m, 1H), 1.40 (d, J=7.0 Hz, 6H). LCMS (ESI, *m*/*z):* 449.1[M+H]⁺.

### Example 65: Synthesis of compound 76

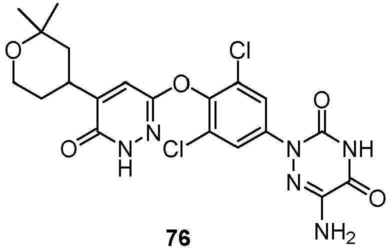

6-Amino-2-(3,5-dichloro-4-((5-(2,2-dimethyltetrahydro-2*H*-pyran-4-yl)-6-oxo-1,6-dihydropyridazin-3-yl)oxy)phenyl)-1,2,4-triazine-3,5(2*H*, 4*H*)-dione, a white solid (95.2 mg, 27%) was prepared similarly as described for 6-amino-2-(3,5-dichloro-4-[[5-(oxan-4-yl)-6-oxo-1*H*-pyridazin-3-yl]oxy]phenyl)-4*H*-1,2,4-triazine-3,5-dione. ¹H NMR (400 MHz, DMSO-*d*₆) δ 12.25 - 12.27 (m, 2H), 7.85 (s, 2H), 7.42 (s, 1H), 6.52 (s, 2H), 3.68 - 3.71 (m, 2H), 3.13 - 3.20 (m, 1H), 1.68 - 1.74 (m, 2H), 1.50 - 1.58 (m,1H), 1.40 (t, *J=* 12.6 Hz, 1H), 1.25 (s, 3H), 1.19 (s, 3H). LCMS (ESI, *m*/*z):* 495 [M+H]⁺.

### Example 66: Synthesis of compound 77

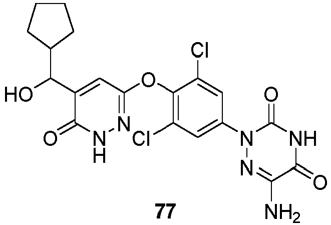

A 500 mL round-bottom flask was charged with 2,2,6,6-tetramethylpiperidine (6.20 g, 43.9 mmol) in THF (60 mL) under N₂. n-BuLi (12.1 mL, 30.2 mmol) was added dropwise at -75 °C. The mixture was warmed to 0 °C and stirred for 30 min. Then the mixture was cooled to -75 °C and 3,6-dichloropyridazine (3.00 g, 20.1 mmol) in THF (60 mL) was added dropwise. The mixture was stirred for 30 min at -75 °C. Cyclopentanecarboxaldehyde (5.93 g, 60.4 mmol) in THF (30 mL) was added dropwise. The reaction was stirred for 90 min at -75 °C, then quenched with NH₄Cl (sat., aq., 100 mL) and extracted with EA (3 x 100 mL), the combined organic layers were washed with brine (2 x 50 mL), dried over anhydr. Na₂SO₄, the solids were removed by filtration and the filtrate was concentrated under reduced pressure. The residue was purified by reverse phase chromatography using a C18 column (MeCN:water+0.05%TFA)=85:15) to provide (6-chloro-3-methoxypyridazin-4-yl)(cyclopentyl)methanol as a yellow oil (1.7 g, 30%). LCMS (ESI, *m*/*z):* 247 [M+H]⁺.

A 100 mL round-bottom flask was charged with cyclopentyl(3,6-dichloropyridazin-4-yl)methanol (1.40 g, 5.66 mmol), imidazole (1.54 g, 22.7 mmol), *t-*butyldimethylsilyl chloride (2.56 g, 17.0 mmol), DMF (15 mL) under N₂. The reaction was stirred overnight at rt. The reaction was quenched with water (10 mL), then extracted with EA (3 x 30 mL), washed with brine (30 mL), dried over anhydr. Na₂SO₄, filtered and concentrated under reduced pressure. The residue was chromatographed on a silica gel column with (EA:PE=3:97) to provide 4-[[(*t*-butyldimethylsilyl)oxy]-(cyclopentyl)methyl]-3,6-dichloropyridazine as a colorless oil (1.3 g, 63%). LCMS (ESI, *m*/*z):* 361 [M+H]⁺.

An 8 mL vial was charged with 4-[[(*t*-butyldimethylsilyl)oxy] (cyclopentyl)methyl]-3,6-dichloropyridazine (1.30 g, 3.60 mmol), *t*-butyl *N*-[2-(3,5-dichloro-4-hydroxyphenyl)-3,5-dioxo-4*H*-1,2,4-triazin-6-yl]carbamate (1.40 g, 3.60 mmol), CuI (342 mg, 1.80 mmol), K₂CO₃ (1.24 g, 9 mmol), DMSO (20 mL) under N₂. The reaction was stirred overnight at 110 °C and quenched with water (80 mL). The solids were removed by filtration. The filtrate was extracted with EA (3 x 100 mL) and the organic layers were combined, washed with brine (2 x 50 mL), dried over anhydr. Na₂SO₄, the solids were removed by filtration and the filtrate was concentrated under reduced pressure. The residue was chromatographed on a silica gel column (EA:PE=15:85) to provide 6-amino-2-[4-[(5-[[(*t-*butyldimethylsilyl)oxy]-(cyclopentyl)methyl]-6-chloropyridazin-3-yl)oxy]-3,5-dichlorophenyl]-4*H*-1,2,4-triazine-3,5-dione as a brown solid (360 mg, 14%). LCMS (ESI, *m*/*z):* 613 [M+H]⁺.

A 40 mL vial was charged with 6-amino-2-[4-[(5-[[(*t*-butyldimethylsilyl)oxy](cyclopentyl)methyl]-6-chloropyridazin-3-yl)oxy]-3,5-dichloro-phenyl]-4*H*-1,2,4-triazine-3,5-dione (170 mg, 0.277 mmol), NaOAc (90.8 mg, 1.11 mmol), CH₃CO₂H (5 mL) under N₂. The reaction was stirred overnight at 110 °C. The pH value of the solution was adjusted to 8.0 with NaHCO₃ (sat., aq.). The solution was extracted with DCM (3 x 20 mL) and the organic layers were combined, washed with brine (2 x 10 mL), dried over anhydr. Na₂SO₄, the solids were removed by filtration and the filtrate was concentrated under reduced pressure. The product was dissolved into CH₃OH (2 mL). NaOH (55.4 mg, 1.38 mmol) in water (2 mL) was added. The reaction was stirred for 2 h at 50 °C. The reaction was quenched with H₂O (10 mL), then extracted with EA (3 x 20 mL). The organic layers were combined, washed with brine (2 x 10 mL), dried over anhydr. Na₂SO₄, the solids were removed by filtration and the filtrate was concentrated under reduced pressure to afford 6-amino-2-[4-[(5-[[(*t*-butyldimethylsilyl)oxy](cyclopentyl)methyl]-6-oxo-1*H*-pyridazin-3-yl)oxy]-3,5-dichlorophenyl]-4*H*-1,2,4-triazine-3,5-dione as a brown solid (150 mg, 57%). LCMS (ESI, *m*/*z):* 595 [M+H]⁺.

6-amino-2-[3,5-dichloro-4-([5-[cyclopentyl(hydroxy)methyl]-6-oxo-1*H-*pyridazin-3-yl]oxy)phenyl]-4*H*-1,2,4-triazine-3,5-dione, a white solid (3.4 mg) was prepared similarly as described for 2-[3,5-dichloro-4-[(3-isopropyl-1*H*-indol-5-yl)oxy]-phenyl]-3,5-dioxo-4*H*-1,2,4-tri-azine-6-carbonitrile except that the reaction with TBAF occurred at rt. ¹H NMR (300 MHz, DMSO-*d*₆) δ 12.27 (br, 1H), 7.88 (s, 2H), 7.40 (s, 1H), 6.34 (br, 2H), 5.41 (d, *J* = 4.8 Hz, 1H), 4.63 (br, 1H), 2.22 (br, 1H), 1.44 - 1.57 (m, 8H). LCMS (ESI, *m*/*z*): 481 [M+H]⁺.

### Example 67: Synthesis of compounds 78 and 79.

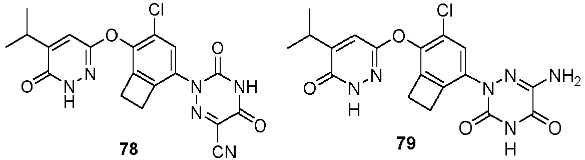

To a solution of 1-(benzyloxy)-2-bromobenzene (10 g, 38.003 mmol) and sodium amide (4.45 g, 114.010 mmol) in THF (150 mL) was added 1,1-diethoxyethene(13.24 g, 114.010 mmol) under N2. The reaction was stirred at 70°C for 16h. The solution was poured into ice water (100 mL), acidified with concentrated hydrochloric acid (25 mL), and extracted with EA (2 x 200 mL). The organic layers were combined, dried over anhydr. Na₂SO₄, the solids were removed by filtration and the filtrate was concentrated under reduced pressure. The residue was chromatographed on a silica gel column (EA:PE=1:50) to afford 5-(benzyloxy)bicyclo[4.2.0]octa-1(6),2,4-trien-7-one as a white solid (3.3 g, 37%). GCMS (ESI, *m*/*z):* 224 [M].

To a solution of 5-(benzyloxy)bicyclo[4.2.0]octa-1(6),2,4-trien-7-one (9 g, 40.132 mmol) in CH₃OH (250 mL) was added NaBH₄ (3.036 g, 80.264 mmol). The reaction was stirred at rt for 4h. The reaction was quenched with water and extracted with EA (2 x 200 mL). The organic layers were combined, dried over anhydr. Na₂SO₄, filtered and concentrated under reduced pressure. The residue was chromatographed on a silica gel column (EA:PE=1:15) to get 5-(benzyloxy)bicyclo[4.2.0]octa-1(6),2,4-trien-7-ol as a white solid. GCMS (ESI, *m*/*z):* 226 [M]. (7.4 g, 77%).

To a solution of iodine (11.41 g, 44.945 mmol) in toluene (90 mL) was added triphenylphosphine (10.22 g, 38.953 mmol). The reaction was stirred at rt for 5 min under N₂ and imidazole (6.12 g, 89.891 mmol) was added. The mixture was stirred at rt for 10 min and 5-(benzyloxy)bicyclo[4.2.0]octa-1(6),2,4-trien-7-ol(3.39 g, 14.982 mmol) in toluene (30 mL) was added. The solution was stirred at rt for 1 h and quenched with sodium sulfite (sat., aq., 100 mL). The mixture was extracted with EA (100 mL x 3), dried over anhydr. Na₂SO₄, the solids were removed by filtration and the filtrate was concentrated under reduced pressure. The residue was chromatographed on a silica gel column (EA:PE=1:50) to get 2-(benzyloxy)-8-iodobicyclo[4.2.0]octa-1(6),2,4-triene as a yellow oil. GCMS (ESI, *m*/*z):* 336 [M]. (4.46g, 84%).

To a solution of LiAlH₄ (4.01 g, 105.601 mmol) in THF (100 mL) was added 2-(benzyloxy)-8-iodobicyclo[4.2.0]octa-1(6),2,4-triene (14.20 g, 42.240 mmol) in THF (150 mL) dropwise. The reaction mixture was stirred at rt for 30 min and quenched with ice water (200 mL) and adjusted to pH=5 with concentrated hydrochloric acid. The mixture was extracted with EA (3 x 200 mL) and the organic layers was combined, washed with brine (100 ml) dried over anhydr. Na₂SO₄, filtered and concentrated under reduced pressure to get 2-(benzyloxy)bicyclo[4.2.0]octa-1(6),2,4-triene as a colorless oil. GCMS (ESI, *m*/*z*): 210 [M]. (8.900 g, 90%).

A solution of 2-(benzyloxy)bicyclo[4.2.0]octa-1(6),2,4-triene (7 g,33.290 mmol) in methanol (175 mL) was added Pd/C (3 g). The reaction was stirred for 16 h at rt under hydrogen and filtered. The filtrate was concentrated under reduced pressure. The residue was chromatographed on a silica gel column (EA:PE=1:20) to get bicyclo[4.2.0]octa-1(6),2,4-trien-2-ol as a colorless oil. LCMS (ESI, *m*/*z):* 119 [M-H]⁻. (1.66 g, 35%).

To a solution of bicyclo[4.2.0]octa-1(6),2,4-trien-2-ol (1 g, 8.323 mmol) and diisopropylamine (84.22 mg, 0.832 mmol) in DCM (37.50 mL) was added sulphone chloride (1123.34 mg, 8.323 mmol) in DCM (10 mL) dropwise at 0 °C. The reaction was stirred at rt for 16 h and quenched with water (100 mL). The mixture was extracted with DCM (3 x 50 mL), washed with brine, dried over anhydr. Na₂SO₄, filtered and concentrated under reduced pressure. The residue was chromatographed on a silica gel column (EA:PE=1:50) to 3-chlorobicyclo[4.2.0]octa-1(6),2,4-trien-2-ol as a yellow oil (760 mg, 51%). LCMS (ESI, *m*/*z*): 153 [M-H]⁻.

To a solution of 3-chlorobicyclo[4.2.0]octa-1(6),2,4-trien-2-ol (800 mg, 5.175 mmol) in acetic acid (22 mL) was added HNO₃ (326.09 mg, 5.175 mmol) in acetic acid (2 mL) at 0 °C. The reaction was stirred at rt overnight and quenched with water (100 mL). The reaction mixture was extracted with EA (3 x 50 mL), washed with brine, dried over anhydr. Na₂SO₄, filtered and concentrated under reduced pressure. The residue was chromatographed on a silica gel column with (EA:PE=1:50) to get 3-chloro-5-nitrobicyclo[4.2.0]octa-1(6),2,4-trien-2-ol (500 mg, 41%) as a yellow solid. LCMS (ESI, *m*/*z):* 198 [M-H]⁻.

To a solution of 3-chloro-5-nitrobicyclo[4.2.0]octa-1(6),2,4-trien-2-ol(500.00 mg, 2.505 mmol) in ethanol (20 mL) and H₂O (10 mL) was added Fe (699.50 mg, 12.526 mmol) and NH₄Cl (1.072g, 20.041 mmol). The mixture was stirred for 5 h at 60 °C. The mixture was filtered, the filter cake was washed with DCM (6 x 50 mL). The filtrate was extracted with dichloromethane (3 x 300 mL). The combined organic layers were dried over anhydr. Na₂SO₄. The solids were removed by filtration, the filtrate was concentrated under reduced pressure to afford 5-amino-3-chlorobicyclo[4.2.0]octa-1(6),2,4-trien-2-ol (450mg, 95%) as a brown solid. LCMS (ESI, *m*/*z):* 170 [M+H]⁺.

4-Chloro-5-[(6-chloro-5-isopropylpyridazin-3-yl)oxy]bicyclo[4.2.0]octa-1(6),2,4-trien-2-amine (640 mg, 72%), a yellow oil, LCMS (ESI, *m*/*z):* 324 [M+H]⁺, was prepared similarly as described for 3,5-dichloro-4-[(6-chloro-5-isopropylpyridazin-3-yl)oxy]aniline.

6-([5-Amino-3-chlorobicyclo[4.2.0]octa-1(6),2,4-trien-2-yl]oxy)-4-isopropyl-2*H*-pyridazin-3-one (300 mg, 52%), a brown solid, LCMS (ESI, *m*/*z):* 306 [M+H]⁺, was prepared similarly as described for 6-(4-amino-2,6-dichlorophenoxy)-4-isopropyl-2*H-*pyridazin-3-one.

Ethyl (2-(2-(4-chloro-5-((5-isopropyl-6-oxo-1,6-dihydropyridazin-3-yl)oxy)bicyclo[4.2.0]octa-1(6),2,4-trien-2-yl)hydrazineylidene)-2-cyanoacetyl)-carbamate (250 mg, 77%), a yellow solid, was prepared similarly as described for ethyl (2-cyano-2-(2-(3,5-dichloro-4-((1-tosyl-1*H*-indol-5-yl)oxy)phenyl)-hydrazineylidene)-acetyl) carbamate. LCMS (ESI, *m*/*z):* 473 [M+H]⁺.

2-[4-Chloro-5-[(5-isopropyl-6-oxo-1*H*-pyridazin-3-yl)oxy]bicyclo[4.2.0]octa-1(6),2,4-trien-2-yl]-3,5-dioxo-4*H*-1,2,4-triazine-6-carbonitrile, compound 78 was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbo-nitrile, and was isolated as a yellow solid. ¹H NMR (300 MHz, DMSO-*d*₆) δ 7.56 (s, 1H), 7.37 (s, 1H), 6.90 - 7.20 (br, 2H), 2.96 - 3.13 (m, 5H), 1.18 (d, *J* = 6.9 Hz, 6H). LCMS (ESI, *m*/*z):* 427 [M+H]⁺), was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile.

2-[4-Chloro-5-[(5-isopropyl-6-oxo-1*H*-pyridazin-3-yl)oxy]bicyclo-[4.2.0]octa-1(6),2,4-trien-2-yl]-3,5-dioxo-4*H*-1,2,4-triazine-6-carboxylic acid (180 mg, 82%), a white solid (LCMS (ESI, *m*/*z):* 446 [M+H]⁺), was prepared similarly as described for 2-[3,5-dichloro-4-[(5-isopropyl-6-oxo-1*H*-pyridazin-3-yl)oxy]phenyl]-3,5-dioxo-4*H*-1,2,4-triazine-6-carboxylic acid.

*N*-(2-[4-Chloro-5-[(5-isopropyl-6-oxo-1*H*-pyridazin-3-yl)oxy]-bicyclo[4.2.0]octa-1(6),2,4-trien-2-yl]-3,5-dioxo-4*H*-1,2,4-triazin-6-yl)carbamate (200 mg, 81%), a yellow oil (LCMS (ESI, *m*/*z):* 517 [M+H]⁺ ) was prepared similarly as described for *t*-butyl *N*-(2-[3,5-dichloro-4-[(5-isopropyl-6-oxo-1*H*-pyridazin-3-yl)oxy]phenyl]-3,5-dioxo-4*H*-1,2,4-triazin-6-yl)-carbamate.

6-Amino-2-[4-chloro-5-[(5-isopropyl-6-oxo-1*H*-pyridazin-3-yl)oxy]-bicyclo[4.2.0]octa-1(6),2,4-trien-2-yl]-4*H*-1,2,4-triazine-3,5-dione (92.5 mg, 56%), a white solid, compound 79 (¹H NMR (300 MHz, DMSO-*d*₆) δ 12.20 - 12.27 (m, 2H), 7.58 (s, 1H), 7.31 (s, 1H), 6.46 (s, 2H), 3.16 (br, 2H), 2.99 - 3.08 (m, 1H), 2.95 (br, 2H), 1.18 (d, J = 6.9 Hz, 6H). LCMS (ESI, *m*/*z):* 417 [M+H]⁺) was prepared similarly as described for 6-amino-2-[3,5-dichloro-4-[(3-isopropyl-1*H*-indol-5-yl)oxy]phenyl]-4*H*-1,2,4-triazine-3,5-dione.

### Example 68: Synthesis of compounds 80 and 81

The synthesis of 6-amino-2-(3-bromo-5-chloro-4-((5-isopropyl-6-oxo-1,6-dihydropyridazin-3-yl)oxy)phenyl)-1,2,4-triazine-3,5(2*H*,4*H*)-dione (white solid, 203.7 mg, 57%), and 6-amino-2-(3,5-dibromo-4-((5-isopropyl-6-oxo-1,6-dihydropyridazin-3-yl)oxy)phenyl)-1,2,4-triazine-3,5(2*H*,4*H*)-dione were prepared similarly as described for 6-amino-2-(3,5-dichloro-4-((5-isopropyl-6-oxo-1,6-dihydropyridazin-3-yl)oxy)phenyl)-1,2,4-triazine-3,5(2*H*,4*H*)-dione with the exception that 4-amino-2-bromo-6-chlorophenol was employed instead of 4-amino-2,6-dichlorophenol in the synthesis of compound 80, and 4-amino-2,6-dibromophenol was employed instead of 4-amino-2,6-dichlorophenol in the synthesis of compound 81.

| Structure | LCMS (ESI, *m*/*z*) | ¹H NMR |
|---|---|---|
| 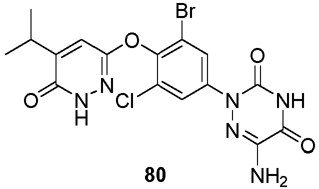 6-Amino-2-(3-bromo-5-chloro-4-((5-isopropyl-6-oxo-1,6-dihydropyridazin-3-yl)oxy)phenyl)-1,2,4-triazine-3,5(2*H*,4*H*)-dione | 469 [M+H]⁺ | ¹H NMR (300 MHz, DMSO-*d*₆) δ 12.20 (br, 1H), 7.97 (s, 1H), 7.88 (s, 1H), 7.42 (s, 1H), 6.51 (br, 2H), 3.01 - 3.10 (m, 1H), 1.20 (d, *J* = 6.6 Hz, 6H). |
| 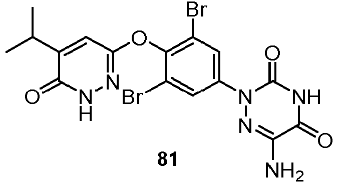 6-Amino-2-(3,5-dibromo-4-((5-isopropyl-6-oxo-1,6-dihydropyridazin-3-yl)oxy)phenyl)-1,2,4-triazine-3,5(2*H*,4*H*)-dione | 513 [M+H]⁺ | ¹H NMR (300 MHz, DMSO-*d*₆) δ 12.20 - 12.26 (m, 2H), 7.99 (s, 2H), 7.41 (s, 1H), 6.52 (br, 2H), 3.03 - 3.08 (m, 1H), 1.20 (d, *J* = 6.6 Hz, 6H). |

### Example 69: Synthesis of compound 82

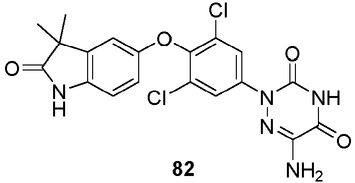

5-Bromo-3,3-dimethyl-1*H*-indol-2-one (3.7 g, 67%), a yellow solid, was prepared similarly as described in the procedures of WO2000066167 and WO2000066556. LCMS (ESI, *m*/*z):* 240 [M+H]⁺.

A 50-mL round-bottom flask was charged with 5-bromo-3,3-dimethyl-1*H-*indol-2-one (1.50 g, 6.247 mmol), 2-(5,5-dimethyl-1,3,2-dioxaborinan-2-yl)-5,5-dimethyl-1,3,2-dioxaborinane (4.23 g, 18.742 mmol), potassium acetate (1.84 g, 18.742 mmol), DMSO (20 mL), PdCl₂(PPh₃)₂ (438.50 mg, 0.625 mmol) under N₂. The mixture was stirred for 2 h at 60 °C under N₂. The solution was purified by C18 reverse phase chromatography to provide 3,3-dimethyl-2-oxo-1*H*-indol-5-ylboronic acid (1.2 g, 89%) as a white solid. LCMS (ESI, *m*/*z):* 206 [M+H]⁺.

A 100-mL 3-necked round-bottom flask was charged with *t*-butyl *N*-[4-[(benzyloxy)methyl]-2-(3,5-dichloro-4-hydroxyphenyl)-3,5-dioxo-1,2,4-triazin-6-yl]carbamate (1.20 g, 2.356 mmol), 3,3-dimethyl-2-oxo-1*H*-indol-5-ylboronic acid (2.42 g, 11.780 mmol), Cu(OAc)₂ (855.86 mg, 4.712 mmol), pyridine (372.72 mg, 4.712 mmol), dichloromethane (100 mL), 4Å molecular sieves (500 mg) under O₂. The reaction was stirred for 3 days at rt. The solids were filtered out. The organic layer was concentrated under reduced pressure. The residue was chromatographed on a C18 column (CH₃CN:H₂O+0.05% TFA=7:3) to provide *t*-butyl *N*-[4-[(benzyloxy)-ethyl]-2-[3,5-dichloro-4-[(3,3-dimethyl-2-oxo-1*H*-indol-5-yl)oxy]phenyl]-3,5-dioxo-1,2,4-triazin-6-yl]carbamate as a yellow solid (830 mg, 45%). LCMS (ESI, *m*/*z):* 668 [M+H]⁺.

A 50-mL round-bottom flask was charged with *t*-butyl *N*-[4-[(benzyloxy)methyl]-2-[3,5-dichloro-4-[(3,3-dimethyl-2-oxo-1*H*-indol-5-yl)oxy]-phenyl]-3,5-dioxo-1,2,4-triazin-6-yl]carbamate (500 mg, 0.748 mmol), DCM (10 mL) under N₂. BBr₃ (1.499g, 5.983 mmol) was added dropwise at 0 °C. The mixture was stirred at 0 °C for 1 h. The reaction mixture was added dropwise to methanol (5 mL) at 0 °C and concentrated under reduced pressure at 0 °C. The crude was dissolved in methanol (5 mL) and purified by preparative HPLC using the following gradient conditions: XBridge Prep OBD C18 Column, 19 x 250 mm, 5 um; Mobile Phase A:Water (10 mmol/L NH₄HCO₃+0.1%NH₃.H₂O), Mobile Phase B:ACN; Flow rate: 25 mL/min; Gradient:20 B to 40 B in 7 min; 220 nm; RT1:6.42; Injection Volume: 6 ml; Purification afforded 6-amino-2-[3,5-dichloro-4-[(3,3-dimethyl-2-oxo-1*H*-indol-5-yl)oxy]phenyl]-4*H*-1,2,4-triazine-3,5-dione as a white solid (59.4 mg, 18%). ¹H NMR (300 MHz, DMSO-*d*₆) δ 12.25 (br, 1H), 10.27 (s, 1H), 7.90 (s, 2H), 7.05 (d, J = 2.4 Hz, 1H), 6.77 (d, J = 8.4 Hz, 1H), 6.54 (br, 2H), 6.45 - 6.49 (m, 1H), 1.25 (s, 6H). LCMS (ESI, *m*/*z):* 448 [M+H]⁺.

### Example 70: Synthesis of compound 83

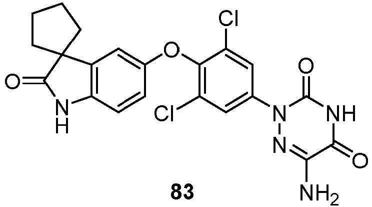

A 50-mL round-bottom flask was charged with 5'-bromo-1'*H-*spiro[cyclopentane-1,3'-indol]-2'-one (prepared according to the procedure in J. Med. Chem. 2008, 51, 1861-1873, 1 g, 3.757 mmol), 2-(5,5-dimethyl-1,3,2-dioxaborinan-2-yl)-5,5-dimethyl-1,3,2-dioxaborinane (2.55 g, 11.289 mmol), potassium acetate (1.11 g, 11.272 mmol), PdCl₂(PPh₃)₂ (263.8 mg, 0.375 mmol), dimethyl sulfoxide (25 mL) under N₂. The resulting solution was stirred for 3 h at 60 °C. The solution was purified by reverse phase column chromatography (C18, H₂O:CH₃CN=13:87) to provide 2'-oxo-1'*H-*spiro[cyclopentane-1,3'-indol]-5'-ylboronic acid as a yellow solid (1.07 g, 74%). LCMS (ESI, *m*/*z):* 232 [M+H]⁺.

The subsequent coupling reaction, deprotection, and purification were prepared similarly as described for 6-amino-2-[3,5-dichloro-4-[(3,3-dimethyl-2-oxo-1*H-*indol-5-yl)oxy]phenyl]-4*H*-1,2,4-triazine-3,5-dione to afford 6-amino-2-(3,5-dichloro-4-[1'*H*-spiro[cyclopentane-1,3'-indol]-2'-oneoxy]phenyl)-4*H*-1,2,4-triazine-3,5-dione as a white solid (11.3 mg, 13%). ¹H NMR (300MHz, DMSO-*d*₆) δ 10.23 (s, 1H), 7.91 (s, 2H), 6.96 (s, 1H), 6.72 - 6.75 (m, 1H), 6.43 - 6.48 (m, 3H), 1.89 - 2.01 (m, 6H), 1.70 - 1.81 (m, 2H). LCMS (ESI, *m*/*z):* 474 [M+H]⁺.

### Example 71: Synthesis of compound 84

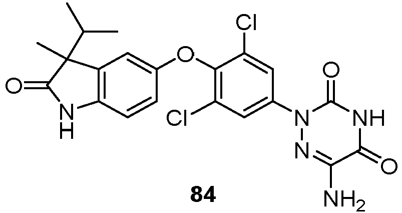

3-Isopropyl-3-methyl-1*H*-indol-2-one was obtained as a yellow solid after following the preparation outlined in Molecules 2017, 22, 1-18. LCMS (ESI, *m*/*z*): 190 [M+H]⁺.

A 40 mL vial was charged with 3-isopropyl-3-methyl-1*H*-indol-2-one (2.80 g, 14.8 mmol), NaOAc (1.23 g, 14.9 mmol), acetic acid (30 mL). Br₂ (0.77 mL, 4.84 mmol) in acetic acid (10 mL) was added dropwise. The reaction was stirred for 50 min at rt and concentrated under reduced pressure. The residue was chromatographed on a silica gel column (EA:PE=25:75) to provide 5-bromo-3-isopropyl-3-methyl-1*H*-indol-2-one as a white solid (4 g, 95%). LCMS (ESI, *m*/*z):* 268 [M+H]⁺.

The subsequent formation of the boronic acid, the coupling reaction, deprotection, and purification were prepared similarly as described for 6-amino-2-[3,5-dichloro-4-[(3,3-dimethyl-2-oxo-1*H*-indol-5-yl)oxy]phenyl]-4*H*-1,2,4-triazine-3,5-dione to afford 6-amino-2-[3,5-dichloro-4-[(3-isopropyl-3-methyl-2-oxo-1*H*-indol-5-yl)oxy]phenyl]-4*H*-1,2,4-triazine-3,5-dione (52.8 mg, 29%). ¹H NMR (400 MHz, DMSO-*d*₆) δ 12.28 (s, 1H), 10.28 (s, 1H), 7.89 (s, 2H), 6.86 (d, *J =*2.8 Hz, 1H), 7.77 (d, *J* = 8.4 Hz, 1H), 6.54 - 6.58 (m, 3H), 1.91- 2.00 (m, 1H), 1.23 (s, 3H), 0.85 (d, *J =* 7.2 Hz, 3H), 0.70 (d, *J =* 6.8 Hz, 3H). LCMS (ESI, *m*/*z):* 476 [M+H]⁺.

### Example 72: Synthesis of compound 85

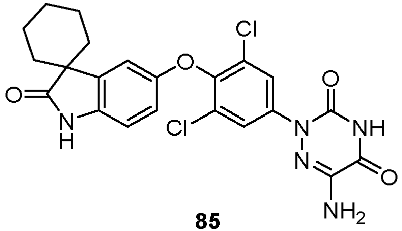

6-Amino-2-(3,5-dichloro-4-[1'*H*-spiro[cyclohexane-1,3'-indol]-2'-oneoxy]phenyl)-4*H*-1,2,4-triazine-3,5-dione, a white solid (¹H NMR (300 MHz, DMSO-*d*₆) δ 10.26 (s, 1H), 7.90 (s, 2H), 7.12 (s, 1H), 6.76 (d, *J* = 4.8 Hz, 1H), 6.49 - 6.53 (m, 3H), 1.86 (br, 2H), 1.53 - 1.62 (m, 8H). LCMS (ESI, *m*/*z):* 488 [M+H]⁺) was prepared similarly as described for 6-amino-2-(3,5-dichloro-4-[1'*H*-spiro[cyclopentane-1,3'-indol]-2'-oneoxy]phenyl)-4*H*-1,2,4-triazine-3,5-dione.

### Example 73: Synthesis of compounds 86 to 89

Compound 86 was prepared similarly as described for 6-amino-2-(3,5-dichloro-4-((5-cyclobutyl-6-oxo-1,6-dihydropyridazin-3-yl)oxy)phenyl)-1,2,4-triazine-3,5(2H,4H)-dione where 3-methylcyclobutane-1-carboxylic acid was employed instead of cyclobutane carboxylic acid. Compound 86 was purified by SFC-HPLC [Column: (R,R)-Whelk-01, 2.12 x 25cm, 5µm; Mobile Phase A: Hex(0.1%FA)-HPLC, Mobile Phase B:EtOH-HPLC; Flow rate: 20 mL/min; Gradient: 20 B to 20 B in 41 min; 254/220 nm] to afford compound 86-A (RT1=31.945, 10.8 mg, 98%pure, SFC analytical Column:(R,R)-Whelk, 0.46 x 5 cm;3.5 µm, Hex (0.1%FA):EtOH=75:25, 1.0 mL/min, Rt: 3.788min), and compound 86-B (RT2=37.638, 7.1 mg, 98%pure, SFC Column:(R,R)-Whelk, 0.46 x 5 cm; 3.5 µm, Hex (0.1%FA): EtOH=75:25, 1.0 mL/min, Rt: 4.378 min), both as a white solids.

Compounds 87-A and 87-B were prepared similarly as described for 6-amino-2-(3,5-dichloro-4-((5-cyclobutyl-6-oxo-1,6-dihydropyridazin-3-yl)oxy)phenyl)-1,2,4-triazine-3,5(2H,4H)-dione with the exception that 3-(benzyloxy)cyclobutane-1-carboxylic acid was employed instead of cyclobutane carboxylic acid. 6-amino-2-(4-((5-(3-(benzyloxy)cyclobutyl)-6-oxo-1,6-dihydro-pyridazin-3-yl)oxy)-3,5-dichlorophenyl)-1,2,4-triazine-3,5(2*H*,4*H*)-dione was isolated by preparative HPLC (Column: Xcelect CSH F-pheny OBD Column, 19 x 250 mm, 5 µm; Mobile Phase A:Water(10 mmol/L NH₄HCO₃), Mobile Phase B:ACN; Flow rate:25 mL/min; Gradient:26 B to 46 B in 9 min; 220 nm; RT:8.85min).. This was followed by chiral preparative HPLC (Column: CHIRALPAK IC, 2 x 25 cm, 5 µm; Mobile Phase A:Hex(0.1%FA)-HPLC, Mobile Phase B:EtOH-HPLC; Flow rate: 20 mL/min; Gradient: 40 B to 40 B in 25 min; 220/254 nm; RT:10.881min; Injection Volume:0.6 ml; Number Of Runs: 25) to afford compound 87-A as a white solid (150 mg, 23%). An additional separation on the racemic mixture was required by SFC prep-HPLC (Column: CHIRALPAK IG, 20 x 250 mm, 5 µm; Mobile Phase A:CO₂, Mobile Phase B:IPA (2 mM NH₃-MEOH); Flow rate: 40 mL/min; Gradient: 50% B; 220 nm; RT1:7.1min; Injection Volume:2 mL; Number Of Runs: 4) to afford 87-B as an off-white solid (13.3 mg).

To compound 87-A (130 mg, 0.239 mmol) in EA (10 mL) was added Pd/C (100 mg). The mixture was stirred for 2 h at rt under H₂. The mixture was filtered through a celite pad and washed with EA (20 mL) and CH₃OH (20 mL). The filtrate was concentrated under reduced pressure. The crude product was purified by preparative HPLC (XBridge Prep OBD C18 Column, 19 x 250 mm, 5 µm; Mobile Phase A:Water (10 mmol/L NH₄HCO₃+0.1%NH₃.H₂O), Mobile Phase B: ACN; Flow rate: 25 mL/min; Gradient:10 B to 30 B in 7 min; 220 nm; RT: 6.13min) to provide 88-A as a white solid (52.5 mg, 48%).

6-amino-2-(3,5-dichloro-4-((5-(3,3-dimethylcyclobutyl)-6-oxo-1,6-dihydropyridazin-3-yl)oxy)phenyl)-1,2,4-triazine-3,5(2*H*,4*H*)-dione was prepared similarly as described for 6-amino-2-(3,5-dichloro-4-((5-cyclobutyl-6-oxo-1,6-dihydropyridazin-3-yl)oxy)phenyl)-1,2,4-triazine-3,5(2*H*,4*H*)-dione, starting from 3,3-dimethylcyclobutane-1-carboxylic acid.

| Structure | LCMS (ESI, *m*/*z*) | ¹H NMR |
|---|---|---|
| 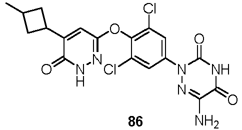 6-Amino-2-(3,5-dichloro-4-[[5-(3-methylcyclobutyl)-6-oxo-1*H-*pyridazin-3-yl]oxy]phenyl)-4*H*-1,2,4-triazine-3,5-dione | 451[M+H]⁺ | ¹H NMR (300 MHz, DMSO-*d*₆) δ 12.36 - 12.03 (m, 2H), 7.84 -7.85 (m, 2H), 7.40 -7.50 (m, 1H), 6.52 (s, 2H), 3.60 -3.65 (m, 1H), 2.24 - 2.46 (m, 3H), 1.84 - 2.00(m, 1H), 1.61 - 1.75 (m, 1H), 1.19 - 1.20 (m, 1H), 1.01 - 1.09 (m, 2H). |
| 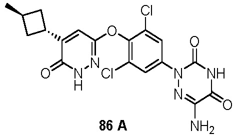 6-Amino-2-(3,5-dichloro-4-((5-((1r,3r)-3-methylcyclobutyl)-6-oxo-1,6-dihydropyridazin-3-yl)oxy)phenyl)-1,2,4-triazine-3,5(2*H*,4*H*)-dione | 1^{st} eluting isomer was labelled 86-A. 451 [M+H]⁺ | ¹H NMR(300 MHz, DMSO-*d*₆) δ 12.13 - 12.27(m, 2H), 7.84 (s, 2H), 7.40 -7.41 (m, 1H), 6.52 (s, 2H), 3.15 - 3.25 (m, 1H), 2.29 - 2.45 (m, 3H), 1.60 - 1.78 (m, 2H), 0.98 (d, *J* = 6.0 Hz, 3H). |
| 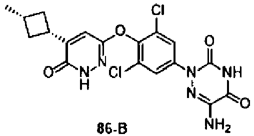 6-Amino-2-(3,5-dichloro-4-((5-((1s,3s)-3-methylcyclobutyl)-6-oxo-1,6-dihydropyridazin-3-yl)oxy)phenyl)-1,2,4-triazine-3,5(2*H*,4*H*)-dione | 2^{nd} eluting isomer was labelled 86-B. 451 [M+H]⁺ | ¹H NMR (300 MHz, DMSO-*d*₆) δ 12.14-12.27 (m, 2H), 7.84 (s, 2H), 7.49 - 7.50 (m, 1H), 6.52 (s, 2H), 3.59 - 3.65 (m, 1H), 2.23 - 2.38 (m, 3H), 1.88 - 1.98 (m, 2H), 1.18 (d, *J* = 6.0 Hz, 3H). |
| 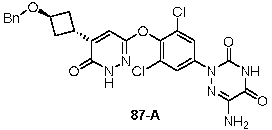 6-Amino-2-(4-((5-((1r,3r)-3-(benzyloxy)cyclobutyl)-6-oxo-1,6-dihydropyridazin-3-yl)oxy)-3,5-dichlorophenyl)-1,2,4-triazine-3,5(2*H*,4*H*)-dione | 543 [M+H]⁺ | ¹HNMR (400MHz, DMSO-*d*₆) δ ppm: 12.28 (s, 1H), 12.19 (s, 1H), 7.84 (s, 2H), 7.44 - 7.47 (m, 1H), 7.24 - 7.37 (m, 5H), 6.53 (s, 2H), 4.42 (s, 2H), 4.03 - 4.11 (m, 1H), 2.99 - 3.06 (m, 1H), 2.53 - 2.63 (m, 2H), 1.93 - 2.05 (m, 2H). |
| 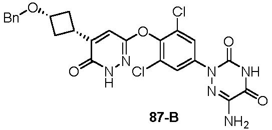 6-Amino-2-(4-((5-((1s,3s)-3-(benzyloxy)cyclobutyl)-6-oxo-1,6-dihydropyridazin-3-yl)oxy)-3,5-dichlorophenyl)-1,2,4-triazine-3,5(2H,4H)-dione | 543 [M+H]⁺ | ¹HNMR (400MHz, DMSO-*d*₆) δ 12.10 - 12.40 (m, 2H), 7.85 (s, 2H), 7.58 (s, 1H), 7.24 - 7.38 (m, 5H), 6.50 (s, 2H), 4.41 (s, 2H), 4.13 - 4.22 (m, 1H), 3.47 - 3.57 (m, 1H), 2.28 - 2.45 (m, 4H). |
| 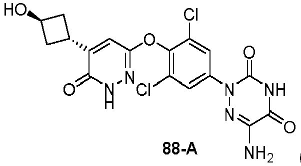 6-Amino-2-[3,5-dichloro-4-([6-oxo-5-[(1r,3r)-3-hydroxycyclobutyl]-1*H-*pyridazin-3-yl]oxy)phenyl]-4*H*-1,2,4-triazine-3,5-dione | 453 [M+H]⁺ | ¹HNMR (400MHz, DMSO-*d*₆) δ 12.10 - 12.42 (m, 2H), 7.85 (s, 2H), 7.38 - 7.41 (m, 1H), 6.51 (s, 2H), 5.09 - 5.15 (m, 1H), 4.06 - 4.13 (m, 1H), 2.88 - 2.95 (m, 1H), 2.50 - 2.60 (m, 2H), 1.85 - 1.93 (m, 2H). |
| 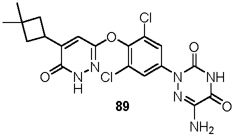 6-Amino-2-(3,5-dichloro-4-((5-(3,3-dimethylcyclobutyl)-6-oxo-1,6-dihydropyridazin-3-yl)oxy)phenyl)-1,2,4-triazine-3,5(2*H*,4*H*)-dione | 465 [M+ H]⁺ | ¹H NMR (400 MHz, DMSO-*d*₆) δ 12.20 (s, 1H), 12.15 (s, 1H), 7.84 (s, 2H), 7.45(s, 1H), 6.54 (s, 2H),3.34 - 3.32 (m, 1H), 2.08 - 2.06 (m, 2H), 2.06 - 1.93 (m, 2H), 1.24 (s, 3H), 1.10 (s, 3H) |
| The following compounds were prepared similarly as described for 6-amino-2-(3,5-dichloro-4-((5-cyclobutyl-6-oxo-1,6-dihydropyridazin-3-yl)oxy)phenyl)-1,2,4-triazine-3,5(2*H*,4*H*)-dione. | | |
| 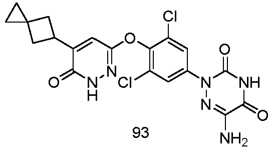 6-amino-2-(3,5-dichloro-4-((6-oxo-5-(spiro[2.3]hexan-5-yl)-1,6-dihydropyridazin-3-yl)oxy)phenyl)-1,2,4-triazine-3,5(2*H*,4*H*)-dione | 463 [M+H]⁺ | ¹H NMR (400 MHz, DMSO-*d*₆) δ 12.16 (s, 1H), 7.86 (s, 2H), 7.54 (s, 1H), 6.46 (s, 2H), 3.72 - 3.67 (m, 1H), 2.34 (d, J = 8.4 Hz, 4H), 0.54 - 0.41 (m, 4H). |
| 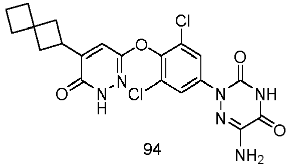 6-amino-2-(3,5-dichloro-4-((6-oxo-5-(spiro[3.3]heptan-2-yl)-1,6-dihydropyridazin-3-yl)oxy)phenyl)-1,2,4-triazine-3,5(2*H*,4*H*)-dione | 477 [M+H]⁺ | ¹H NMR (400 MHz, DMSO-*d*₆) δ 1.78-1.82 (m, 2H), 1.88-1.92 (m, 2H), 2.05-2.12 (m, 4H), 2.32 (td, 2H, J = 9.3 Hz, 2.8 Hz), 3.36-3.38 (m, 1H), 6.51 (br.s, 2H), 7.4 (d, 1H, J = 1.5 Hz), 7.84 (s, 2H), 12.13 (s, 1H), 12.26 (br.s, 1H) |
| 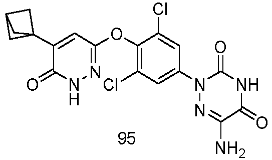 6-amino-2-(4-((5-(bicyclo[1.1.1]pentan-1-yl)-6-oxo-1,6-dihydropyridazin-3-yl)oxy)-3,5-dichlorophenyl)-1,2,4-triazine-3,5(2*H*,4*H*)-dione | 449 [M+H]⁺ | ¹H NMR (400 MHz, DMSO-*d₆*) δ 12.25 (s, 1H), 12.16 (s, 1H), 7.82 (s, 2H), 7.29 (s, 1H), 6.49 (s, 2H), 2.56 (s, 1H), 2.14 (s, 6H) |
| 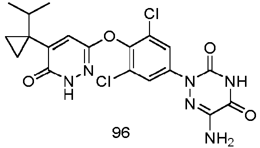 6-amino-2-(3,5-dichloro-4-((5-(1-isopropylcyclopropyl)-6-oxo-1,6-dihydropyridazin-3-yl)oxy)phenyl)-1,2,4-triazine-3,5(2*H*,4*H*)-dione | 463 [M-H]- | ¹H NMR (400 MHz, DMSO-*d*₆) δ 0.64 (t, J= 5.3 Hz, 2H),0.77 (t, J= 5.1 Hz, 2H),0.80 (d, J= 6.9 Hz, 6H), 1.94 (p, J= 6.8 Hz, 1H),6.51 (s, 2H), 7.38 (s, 1H), 7.84 (s, 2H), 12.14 (s, 1H), 12.27 (s, br, 1H) |
| 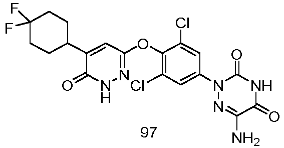 6-amino-2-(3,5-dichloro-4-((5-(4,4-difluorocyclohexyl)-6-oxo-1,6-dihydropyridazin-3-yl)oxy)phenyl)-1,2,4-triazine-3,5(2*H*,4*H*)-dione | 501 [M+H]⁺ | ¹H NMR (400 MHz, DMSO-*d₆*) δ 7.85 (s, 2H), 7.48 (s, 1H), 6.53 (s, 2H), 2.90 (t, J = 11.6 Hz, 1H), 1.89 - 2.11 (m, 6H), 1.62 - 1.68 (m, 2H) |
| 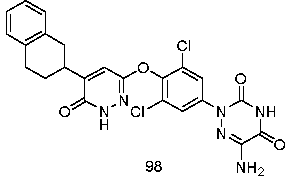 6-amino-2-(3,5-dichloro-4-((6-oxo-5-(1,2,3,4-tetrahydronaphthalen-2-yl)-1,6-dihydropyridazin-3-yl)oxy)phenyl)-1,2,4-triazine-3,5(2*H*,4*H*)-dione | 513 [M+H]⁺ | ¹H NMR (400 MHz, DMSO-*d₆*) δ 1.82-1.91 (m, 1H), 2.03-2.08 (m, 1H), 2.85-2.91 (m, 3H), 2.97-3.04 (m, 1H), 3.10-3.18 (m, 1H), 6.51 (s, 2H), 7.08-7.16 (m, 4H), 7.47 (s, 1H), 7.86 (s, 2H), 12.27 (br s, 1H), 12.29 (s, 1H) |
| 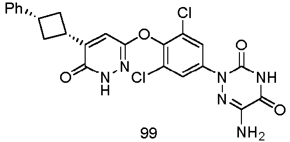 6-amino-2-(3,5-dichloro-4-((6-oxo-5-((1s,3s)-3-phenylcyclobutyl)-1,6-dihydropyridazin-3-yl)oxy)phenyl)-1,2,4-triazine-3,5(2H,4H)-dione | 513 [M+H]⁺ | ¹H NMR (400 MHz, DMSO-*d₆*) δ 12.11 - 12.31 (m, 2H), 7.87 (s, 2H), 7.71 (s, 1H), 7.32 - 7.37 (m, 4H), 7.20 - 7.24 (m, 1H), 6.52 (s, 2H), 3.58 - 3.66 (m, 2H), 3.38 - 3.49 (m, 2H), 2.53 - 2.63 (m, 2H) |
| 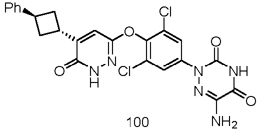 6-amino-2-(3,5-dichloro-4-((6-oxo-5-((1r,3r)-3-phenylcyclobutyl)-1,6-dihydropyridazin-3-yl)oxy)phenyl)-1,2,4-triazine-3,5(2*H*,4*H*)-dione | 513 [M+H]⁺ | ¹H NMR (400 MHz, DMSO-*d₆*) δ 12.14 - 12.34 (m, 2H), 7.85 (s, 2H), 7.53 (s, 1H), 7.29 - 7.33 (m, 4H), 7.17 - 7.23 (m, 1H), 6.54 (s, 2H), 3.46 - 3.58 (m, 2H), 2.63 - 2.70 (m, 2H), 2.20 - 2.29 (m, 2H). |
| 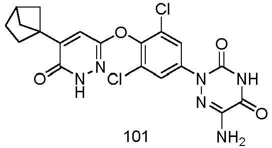 6-amino-2-(4-((5-(bicyclo[2.1.1]hexan-1-yl)-6-oxo-1,6-dihydropyridazin-3-yl)oxy)-3,5-dichlorophenyl)-1,2,4-triazine-3,5(2*H*,4*H*)-dione | 463 [M+H]⁺ | ¹H NMR (400 MHz, DMSO-*d₆*) δ ppm: 12.27 (s, 1H), 12.13 (s, 1H), 7.84 (s, 2H), 7.29 (s, 1H), 6.53 (s, 2H), 2.08 (s, 1H), 1.71 - 1.95 (m, 6H), 1.47 -1.55 (m, 2H) |
| 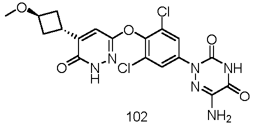 6-amino-2-(3,5-dichloro-4-((5-((1r,3r)-3-methoxycyclobutyl)-6-oxo-1,6-dihydropyridazin-3-yl)oxy)phenyl)-1,2,4-triazine-3,5(2*H*,4*H*)-dione | 467 [M+H]⁺ | ¹H NMR (400 MHz, DMSO-*d₆*) δ 12.18-12.27 (m, 2H), 7.85-7.86 (m, 2H), 7.45-7.46 (m, 1H), 6.53 (s, 2H), 3.85-3.93 (m, 1H), 3.16-3.18 (m, 3H), 2.98-3.10 (m, 1H), 2.58-2.65(m, 2H), 1.90-1.99 (m, 2H) |
| 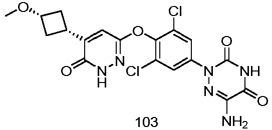 6-amino-2-(3,5-dichloro-4-((5-((1s,3s)-3-methoxycyclobutyl)-6-oxo-1,6-dihydropyridazin-3-yl)oxy)phenyl)-1,2,4-triazine-3,5(2*H*,4*H*)-dione | 467 [M+H]⁺ | ¹H NMR (400 MHz, DMSO-*d₆*) δ 12.19-12.27 (m, 2H), 7.85 -7.86(m, 2H), 7.56-7.57 (m, 1H), 6.52 (s, 2H), 3.96 - 4.00 (m, 1H), 3.49-3.53(m, 1H), 3.17-3.18 (m, 3H), 2.36 -2.42 (m, 2H), 2.24-2.32(m, 2H) |
| 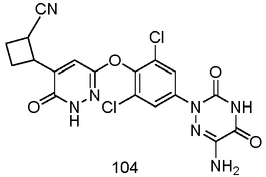 *rac*-6-amino-2-(3,5-dichloro-4-((5-(2-methylcyclobutyl)-6-oxo-1,6-dihydropyridazin-3-yl)oxy)phenyl)-1,2,4-triazine-3,5(2*H*,4*H*)-dione | 462 [M+H]⁺ | ¹H NMR (400 MHz, DMSO-*d₆*) δ 12.29 - 12.31 (m, 1H), 7.96 (s, 2H), 7.59 - 7.64 (m, 1H), 5.85 (s, 2H), 3.78 - 3.97 (m, 2H), 2.56 - 2.66 (m, 1H), 2.10 - 2.32 (m, 3H) |
| 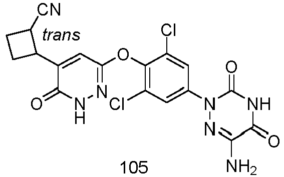 *trans*-2-(6-(4-(6-amino-3,5-dioxo-4,5-dihydro-1,2,4-triazin-2(3H)-yl)-2,6-dichlorophenoxy)-3-oxo-2,3-dihydropyridazin-4-yl)cyclobutane-1-carbonitrile | 462 [M+H]⁺ | ¹H NMR (400 MHz, DMSO-*d₆*) δ 12.27 (s, 2H), 7.85 (s, 2H), 7.59 (d, J = 1.3 Hz, 1H), 6.44 (s, 2H), 3.82 - 3.89 (m, 1H), 3.57 - 3.65 (m, 1H), 2.22 - 2.32 (m, 4H) |
| 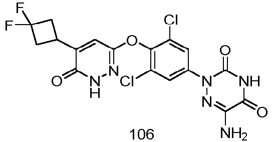 6-amino-2-(3,5-dichloro-4-((5-(3,3-difluorocyclobutyl)-6-oxo-1,6-dihydropyridazin-3-yl)oxy)phenyl)-1,2,4-triazine-3,5(2*H*,4*H*)-dione | 473 [M+H]⁺ | ¹H NMR (400 MHz, DMSO-*d₆*) δ 12.31-12.28 (m, 2H), 7.85 (s, 2H), 7.66 (s, 1H), 6.53 (s, 2H),3.39- 3.31 (m, 1H), 2.94-2.86 (m, 4H) |
| 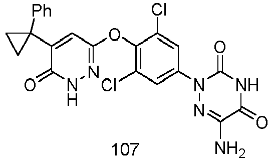 6-amino-2-(3,5-dichloro-4-((6-oxo-5-(1-phenylcyclopropyl)-1,6-dihydropyridazin-3-yl)oxy)phenyl)-1,2,4-triazine-3,5(2*H*,4*H*)-dione | 499 [M+H]⁺ | ¹H NMR (400 MHz, DMSO-*d₆*) δ 1.21-1.25 (m, 2H), 1.37-1.43 (m, 2H), 6.50 (s, 2H), 7.17-7.27 (m, 1H), 7.26-7.34 (m, 4H), 7.58 (s, 1H), 7.84 (s, 2H), 12.17 (s, 1H), 12.26 (br s, 1H) ppm |
| 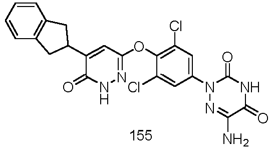 6-amino-2-(3,5-dichloro-4-((5-(2,3-dihydro-1H-inden-2-yl)-6-oxo-1,6-dihydropyridazin-3-yl)oxy)phenyl)-1,2,4-triazine-3,5(2H,4H)-dione | 499 [M+H]⁺ | ¹H NMR (400 MHz, DMSO-*d₆*) δ 3.1 (dd, 2H, J = 15.5 Hz, 8.2 Hz), 3.25 (dd, 2H, J = 15.5 Hz, 7.8 Hz), 3.72 (quint, 1H, J = 8.2 Hz), 6.49 (s, 2H), 7.17 (dd, 2H, *J* = 5.7 Hz, 3.0 Hz), 7.26 (dd, 2H, J = 5.0 Hz, 2.8 Hz), 7.45 (s, 1H), 7.84 (s, 2H), 12.26 (br s, 1H), 12.27 (s, 1H) ppm |
| 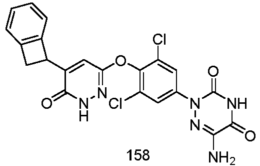 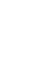 6-amino-2-(4-((5-bicyclo[4.2.0]octa-1(6),2,4-trien-7-yl)-6-oxo-1,6-dixydropyridazin-3-yl)oxy)-3,5-dichlorophenyl)-1,2,4-triazine-3,5(2*H*,4*H*)-dione | 485 [M+H]⁺ | ¹H NMR (400 MHz, DMSO-*d₆*) δ 12.25 -12.33 (m, 2H), 7.82 (s, 2H), 7.20-7.40 (m, 5H), 6.52 (s, 2H), 4.71 (s, 1H), 3.63 - 3.69 (m, 1H), 3.19 - 3.24 (m, 1H) |
| 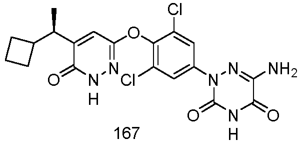 6-amino-2-(3,5-dichloro-4-((5-(1-cyclobutylethyl)-6-oxo-1,6-dihydropyridazin-3-yl)oxy)phenyl)-1,2,4-triazine-3,5(2*H*,4*H*)-dione | 465 [M+H]⁺ | ¹H NMR (300 MHz, DMSO-*d₆*) δ 12.18 -12.28 (m, 2H), 7.85 (s, 2H), 7.38 (s, 1H), 6.53 (s, 2H), 3.03 - 2.90 (m, 1H), 2.55 - 2.67 (m, 1H), 2.02 - 2.13 (m, 1H), 1.68 - 1.93 (m, 4H), 1.53 - 1.66 (m, 1H), 1.08 (d, J=6.9Hz, 3H) |
| 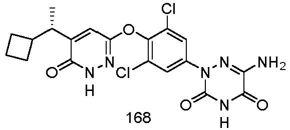 6-amino-2-(3,5-dichloro-4-((5-(1-cyclobutylethyl)-6-oxo-1,6-dihydropyridazin-3-yl)oxy)phenyl)-1,2,4-triazine-3,5(2*H*,4*H*)-dione | 465 [M+H]⁺ | ¹H NMR (300 MHz, DMSO-*d₆*) δ 12.18 -12.28 (m, 2H), 7.85 (s, 2H), 7.38 (s, 1H), 6.53 (s, 2H), 3.03 - 2.90 (m, 1H), 2.55 - 2.67 (m, 1H), 2.02 - 2.13 (m, 1H), 1.68 - 1.93 (m, 4H), 1.53 - 1.66 (m, 1H), 1.08 (d, J=6.9Hz, 3H) |
| 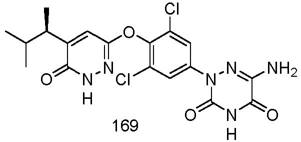 6-amino-2-(3,5-dichloro-4-((5-(3-methylbutan-2-yl)-6-oxo-1,6-dihydropyridazin-3-yl)oxy)phenyl)-1,2,4-triazine-3,5(2*H*,4*H*)-dione | 465 [M+H]⁺ | ¹H NMR (300 MHz, DMSO-*d₆*) δ 12.19 (s, 2H), 7.87 (s, 2H), 7.46 (s, 1H), 6.51 (s, 2H), 2.78 - 2.87 (m, 1H), 1.92 - 2.03 (m, 1H), 1.23 (d, J=7.5Hz, 3H), 0.86 (d, J=6Hz, 6H) |
| 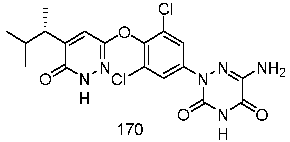 6-amino-2-(3,5-dichloro-4-((5-(3-methylbutan-2-yl)-6-oxo-1,6-dihydropyridazin-3-yl)oxy)phenyl)-1,2,4-triazine-3,5(2*H*,4*H*)-dione | 465 [M+H]⁺ | ¹H NMR (300 MHz, DMSO-*d₆*) δ 12.19 (s, 2H), 7.87 (s, 2H), 7.46 (s, 1H), 6.51 (s, 2H), 2.78 - 2.87 (m, 1H), 1.92 - 2.03 (m, 1H), 1.23 (d, J=7.5Hz, 3H), 0.86 (d, J=6Hz, 6H) |

### Example 74: Synthesis of compound 90

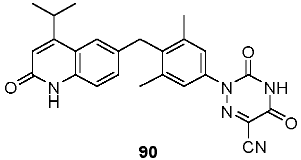

Anhydrous THF (5 mL) was added to a mixture of 6-bromo-4-isopropylquinolin-2(1*H*)-one (was prepared as in Synthesis (6), 934-942; 2011 and International Journal of Chemical Sciences, 7(3), 1784-1792; 2009) (400 mg, 1.50 mmol) and NaH 60% (90 mg) at 0 °C, under N₂. The reaction mixture was stirred at 25 °C for 1 h. Then, the mixture was cooled to -78 °C and t-BuLi (1.94 mL) was added dropwise and the reaction mixture was stirred at -78 °C for 30 min. Then, a solution of 2,2,2-trifluoro-*N*-(4-formyl-3,5-dimethylphenyl)acetamide (368 mg) in anhydr. THF (5 mL) was added dropwise to the reaction mixture at -78 °C. The reaction was further stirred at this temperature for 1.5 h and finally hydrolyzed with sat. aq. NH₄Cl (8 mL) and poured in DCM (50 mL). The organic phase was washed with water (3x), dried over MgSO₄, filtered and evaporated to dryness. The crude mixture was purified by chromatography on silica gel (20 to 85% EA in cyclohexane) to give 2,2,2-trifluoro-*N*-(4-(hydroxy(4-isopropyl-2-oxo-1,2-dihydroquinolin-6-yl)methyl)-3,5-dimethyl-phenyl)acetamide as a white solid (162 mg, 25%). LCMS (ESI, *m*/*z):* 433 [M+H]⁺.

Et₃SiH (1.71 mL) and TMSOTf (0.039 mL) were added dropwise to a solution of 2,2,2-trifluoro-N-(4-(hydroxy(4-isopropyl-2-oxo-1,2-dihydroquinolin-6-yl)methyl)-3,5-dimethylphenyl)acetamide (255 mg, 0.59 mmol) in anhydr. DCM (29 mL) at 0 °C under N₂. The reaction was stirred at 0 °C for 1h, the ice bath was removed and the reaction was stirred at 25 °C for 6 h. Additional Et₃SiH (0.57 mL) and TMSOTf (0.013 mL) were added and the reaction mixture was stirred at rt. After 16 h, 36 h, and 42 h, additional Et₃SiH (0.57 mL) and TMSOTf (0.013 mL) were added and the mixture was stirred for 64 h. The reaction mixture was evaporated to dryness. NaHCO₃ (sat., aq., 50 mL) and EA (3x50 mL) were added. The combined organic layers were washed with brine (150 mL), dried over MgSO₄, the solids were removed by filtration and the filtrate was evaporated to dryness to afford 2,2,2-trifluoro-N-(4-((4-isopropyl-2-oxo-1,2-dihydroquinolin-6-yl)methyl)-3,5-dimethylphenyl)acetamide (306 mg) which was used without purification in the next step. ¹H NMR (DMSO, 400 MHz) δ 1.19 (d, *J* = 6.8 Hz, 6H), 2.22 (s, 6H), 3.20 (hept., *J* = 6.8 Hz, 1H), 4.06 (s, 2H), 6.31 (s, 1H), 7.11 (d, *J* = 8.4 Hz, 1H), 7.22 (d, *J* = 8.4 Hz, 1H), 7.37 (s, 2H), 7.42 (s, 1H), 11.09 (br s, 1H), 11.52 (br s, 1H) ppm. LCMS: C₂₃H₂₃F₃N₂O₂ [M+H]⁺: 417

NaOH (94 mg) was added to a solution of 2,2,2-trifluoro-*N*-(4-((4-isopropyl-2-oxo-1,2-dihydroquinolin-6-yl)methyl)-3,5-dimethylphenyl)acetamide (245 mg, 0.59 mmol) in MeOH (25 mL) and water (2.5 mL) under N₂. The reaction mixture was stirred at 60 °C for 37 h. Additional NaOH (23 mg) was added and heating continued for 24 h. Then, the reaction mixture was quenched with water (150 mL). The solution was extracted with EA (3 x 100 mL). The combined organic layers were washed with brine (100 mL), dried over MgSO₄, the solids were removed by filtration and the filtrate was evaporated to dryness. The crude product was purified by chromatography on silica gel (MeOH:DCM=0-10%) to give 6-(4-amino-2,6-dimethyl-benzyl)-4-isopropylquinolin-2(1*H*)-one as a white solid (133 mg, 71%). ¹H-NMR (DMSO, 400 MHz): 1.19 (d, *J* = 6.8 Hz, 6H), 2.06 (s, 6H), 3.17 (hept., *J* = 6.8 Hz, 1H), 3.89 (s, 2H), 4.76 (s, 2H), 6.29 (s, 2H), 6.30 (s, 1H), 7.13 (d, *J =* 8.4 Hz, 1H), 7.21 (d, *J =* 8.4 Hz, 1H), 7.37 (s, 2H), 11.50 (br s, 1H) ppm. LCMS: C₂₁H₂₄N₂O [M+H]⁺: 321

2-(4-((4-Isopropyl-2-oxo-1,2-dihydroquinolin-6-yl)methyl)-3,5-dimethylphenyl)-3,5-dioxo-2,3,4,5-tetrahydro-1,2,4-triazine-6-carbonitrile, compound 90, was prepared similarly as described for 2-(4-[[3-isopropyl-1-(4-methylbenzene-sulfonyl)indol-5-yl]oxy]-3,5-dimethylphenyl)-3,5-dioxo-4*H*-1,2,4-triazine-6-carbonitrile, starting from 6-(4-amino-2,6-dimethylbenzyl)-4-isopropylquinolin-2(1*H*)-one.

### Example 75: Synthesis of compound 91 and 91-A

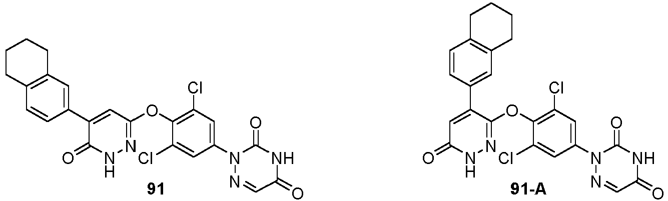

Water (17 mL) was added to a solution of (5,6,7,8-tetrahydronaphthalen-2-yl)boronic acid (1 eq., 0.95 g, 5.4 mmol), 4-bromo-6-chloropyridazin-3-amine (1.12 g, 5.4 mmol), Na₂CO₃ (1.14 g, 10.8 mmol) and Pd(dppf)Cl₂·DCM (0.37 g, 0.46 mmol) in 1,4-dioxane (51 mL). The reaction mixture was evacuated and backfilled with N₂ (3x) and was stirred at 110 °C for 1 h. After cooling to rt, the reaction mixture was diluted in EA and washed with brine. The aqueous phase was extracted with EA (2x). The combined organic phases were dried with Na₂SO₄, filtered and evaporated to dryness. The crude mixture was purified by flash chromatography on silica gel (0% to 10% CH₃OH in DCM) to give 6-chloro-4-(5,6,7,8-tetrahydronaphthalen-2-yl)pyridazin-3-amine (1.12 g, 80%) as a beige solid. ¹H-NMR (300 MHz DMSO-*d*₆,) δ 1.75-1.76 (m, 4H), 2.76-2.77 (m, 4H), 6.32 (br s, 2H), 7.20-7.23 (m, 3H), 7.30 (s, 1H) ppm.

t-Butyl nitrite (1.05 mL, 8.78 mmol) was added to a solution of 6-chloro-4-(5,6,7,8-tetrahydronaphthalen-2-yl)pyridazin-3-amine (1.14 g, 4.39 mmol) and CuCl (0.87 g, 8.78 mmol) in anhydrous CH₃CN (40 mL) under N₂. The reaction mixture was stirred at 60 °C for 24 h. After cooling to rt, the reaction mixture was diluted in EA, washed with sat. aq. NaHCO₃ (2x), washed with brine, dried over MgSO₄, filtered and evaporated to dryness. The crude mixture was purified by flash chromatography on silica gel (0% to 3% CH₃OH in DCM) to give 3,6-dichloro-4-(5,6,7,8-tetrahydronaphthalen-2-yl)pyridazine (0.81 g, 66%) as a yellow solid. ¹H-NMR (300 MHz DMSO-*d*₆,) δ 1.77 (m, 4H), 2.78 (m, 4H), 7.21-7.35 (m, 3H), 8.06 (s, 1H) ppm.

### Preparation of 2-(3,5-dichloro-4-hydroxyphenyl)-1,2,4-triazine-3,5(2H,4H)-dione.

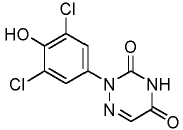

A cooled solution of NaNO₂ (3.88 g, 56.2 mmol) in water (6.5 mL) was added to a solution of 4-amino-2,6-dichlorophenol (10 g, 56.2 mmol) in HCl 37% (23 mL, 281 mmol) and water (63 mL) at 0 °C under N₂. The reaction mixture was stirred at 0 °C for 1 h. This reaction mixture was slowly added to a mixture of ethyl *N*-(2-cyanoacetyl)carbamate (10.5 g, 67.4 mmol), ice (20 g), water (25 mL) and pyridine (225 mL) at 0 °C. The resulting reaction mixture was stirred at 0 °C for 1 h. The precipitate was filtered and washed with water. The precipitate was dissolved in DCM and CH₃OH and evaporated to dryness to give ethyl (2-cyano-2-(2-(3,5-dichloro-4-hydroxyphenyl)hydrazineylidene)acetyl)carbamate (11.94 g, 62%) as a yellow solid, that was used without further purification in the next step.

NaOAc (4 eq., 11.35 g, 0.14 mol) was added to a solution of ethyl (2-cyano-2-(2-(3,5-dichloro-4-hydroxyphenyl)hydrazineylidene)acetyl)carbamate (11.94 g, 35 mmol) in acetic acid (330 mL) under N₂. The reaction mixture was heated to reflux for 3 h and then cooled to 0 °C, water (400 mL) was added and the mixture was stirred for 2 h. Then, the precipitate was filtered, washed with water, dissolved in CH₃OH and evaporated to dryness to give 2-(3,5-dichloro-4-hydroxyphenyl)-3,5-dioxo-2,3,4,5-tetrahydro-1,2,4-triazine-6-carbonitrile (6.48 g, 63%) as a beige solid that was used without further purification in the next step.

A mixture of 2-(3,5-dichloro-4-hydroxyphenyl)-3,5-dioxo-2,3,4,5-tetrahydro-1,2,4-triazine-6-carbonitrile (1 eq., 6.48 g, 21.7 mmol), HCl 37% (16 mL, 195 mmol) and AcOH (33 mL, 580 mmol) under N₂ was stirred at 120 °C for 2.5 h. After cooling to rt, the reaction mixture was diluted in water and the precipitate was filtered and washed with water. The precipitate was dissolved in CH₃OH and evaporated to dryness to give 2-(3,5-dichloro-4-hydroxyphenyl)-3,5-dioxo-2,3,4,5-tetrahydro-1,2,4-triazine-6-carboxylic acid (9.2 g) as a yellow solid, that was used without further purification in the next step. ¹H-NMR (400 MHz DMSO-*d*₆,) δ 7.57 (s, 2H), 10.66 (s, 1H), 12.57 (br s, 1H) ppm.

A solution of 2-(3,5-dichloro-4-hydroxyphenyl)-3,5-dioxo-2,3,4,5-tetrahydro-1,2,4-triazine-6-carboxylic acid (8.88 g, 27.9 mmol) in mercaptoacetic acid (14 mL) under N₂ was stirred at 170 °C for 3 h. After cooling to rt, the reaction mixture was diluted in water and the precipitate was filtered and washed with water. The precipitate was dissolved in CH₃OH and evaporated to dryness to give 2-(3,5-dichloro-4-hydroxyphenyl)-1,2,4-triazine-3,5(2*H*,4*H*)-dione (9.4 g) as a yellow solid, that was used without further purification in the next step. ¹H-NMR (DMSO, 300 MHz): 4.37 (s, 1H), 7.54 (s, 2H), 7.61 (s, 1H) ppm.

A mixture of 3,6-dichloro-4-(5,6,7,8-tetrahydronaphthalen-2-yl)pyridazine (780 mg, 2.79 mmol), 2-(3,5-dichloro-4-hydroxyphenyl)-1,2,4-triazine-3,5(2*H*,4*H*)-dione (766 mg, 2.79 mmol), K₂CO₃ (1.16 mg, 8.38 mmol) and CuI (266 mg, 1.4 mmol) in anhydrous DMSO (13 mL) under N₂ was stirred at 120 °C for 16 h. After cooling to rt, the reaction mixture was diluted in EtOAc and washed with sat. aq. NH₄Cl (2x). The aqueous phase was extracted with EtOAc (2x) and the combined organic layers were dried over Na₂SO₄, filtered and evaporated to dryness. The crude mixture was purified by flash chromatography on silica gel (0% to 3% MeOH in DCM) to give a mixture of 2-(3,5-dichloro-4-((6-chloro-5-(5,6,7,8-tetrahydronaphthalen-2-yl)pyridazin-3-yl)oxy)phenyl)-1,2,4-triazine-3,5(2*H*,4*H*)-dione and 2-(3,5-dichloro-4-((6-chloro-4-(5,6,7,8-tetrahydronaphthalen-2-yl)pyridazin-3-yl)oxy)phenyl)-1,2,4-triazine-3,5(2*H*,4*H*)-dione (65:35, 593 mg, 41%) as a beige solid. LCMS: C₂₃H₁₆Cl₃N₅O₃ [M+H]+: 516/518.

The regioisomeric compound mixture (560 mg, 1.08 mmol) and NaOAc (533 mg, 6.50 mmol) in AcOH (11 mL) under N₂ was stirred at 118 °C for 4 h. After cooling to rt, the reaction mixture was diluted in water, filtered, washed with water (3x) and *n*-pentane. The precipitate was dissolved in EtOAc, dried with Na₂SO₄, filtered and evaporated to dryness. The crude mixture was purified by flash chromatography on silica gel (0% to 5% MeOH in DCM) to give two fractions. The first fraction was purified by trituration with diethylether (3x) to give 2-(3,5-dichloro-4-((6-oxo-5-(5,6,7,8-tetrahydronaphthalen-2-yl)-1,6-dihydropyridazin-3-yl)oxy)phenyl)-1,2,4-triazine-3,5(2*H*,4*H*)-dione (286 mg, 53%) as a beige solid. ¹H-NMR (400 MHz DMSO-*d*₆,) δ 1.76 (br s, 4H), 2.77 (br s, 4H), 7.15-7.17 (m, 1H), 7.68-7.71 (m, 3H), 7.82 (s, 3H), 12.41 (s, 1H), 12.49 (br s, 1H) ppm. LCMS: C₂₃H₁₆Cl₃N₅O₃ [M+H]⁺: 516/518.

The second fraction was purified by trituration with Et₂O (3x) to give 2-(3,5-dichloro-4-((6-oxo-4-(5,6,7,8-tetrahydronaphthalen-2-yl)-1,6-dihydropyridazin-3-yl)oxy)phenyl)-1,2,4-triazine-3,5(2*H*,4*H*)-dione (64 mg, 12%) as a beige solid. ¹H-NMR (400 MHz DMSO-*d*₆,) δ 1.77 (br s, 4H), 2.78 (br s, 4H), 7.06 (d, *J* = 2.4 Hz, 1H), 7.22 (d, *J* = 7.9 Hz, 1H), 7.48-7.52 (m, 2H), 7.70 (s, 1H), 7.81 (s, 2H), 12.34 (d, J= 2.5 Hz, 1H), 12.49 (br s, 1H) ppm. LCMS: C₂₃H₁₆Cl₃N₅O₃ [M+H]⁺: 516/518.

### Example 76: Synthesis of compounds 92 and 92-A

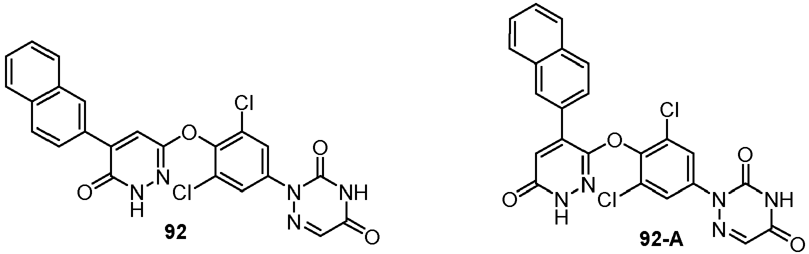

The titled compounds were prepared using a method analogous to that described for 2-(3,5-dichloro-4-((6-oxo-5-(5,6,7,8-tetrahydronaphthalen-2-yl)-1,6-dihydropyridazin-3-yl)oxy)phenyl)-1,2,4-triazine-3,5(2*H*,4*H*)-dione and 2-(3,5-dichloro-4-((6-oxo-4-(5,6,7, 8-tetrahydronaphthalen-2-yl)-1,6-dihydropyridazin-3-yl)oxy)phenyl)-1,2,4-triazine-3,5(2H,4H)-dione with the exception that 2-naphthylboronic acid was employed in the aryl coupling reaction instead of (5,6,7,8-tetrahydronaphthalen-2-yl)boronic acid.

**92:** ¹H-NMR (400 MHz DMSO-*d*₆,) δ 7.56-7.64 (m, 2H), 7.72 (s, 1H), 7.84 (s, 2H), 7.96-8.07 (m, 5H), 8.68 (s, 1H), 12.51 (s, 1H), 12.55 (s, 1H) ppm. LCMS: C₂₃H₁₃Cl₂N₅O₄ [M+H]⁺: 494/496.

**92-A:** ¹H-NMR (400 MHz DMSO-*d*₆,) δ 7.27 (d, *J* = 2.1 Hz, 1H), 7.59-7.67 (m, 2H), 7.70 (s, 1H), 7.82 (s, 2H), 7.92 (dd, *J* = 8.5 Hz, 1.9 Hz, 1H), 7.99-8.06 (m, 2H), 8.09 (d, *J* = 8.7 Hz, 1H), 8.38 (d, *J* = 1.2 Hz, 1H), 12.45 (d, *J* = 1.9 Hz, 1H), 12.49 (s, 1H) ppm. LCMS: C₂₃H₁₃Cl₂N₅O₄ [M+H]⁺: 494/496.

The following compounds were prepared similarly as described for 6-amino-2-(3,5-dichloro-4-[[5-(naphthalen-2-yl)-6-oxo-1*H*-pyridazin-3-yl]oxy]phenyl)-4*H*-1,2,4-triazine-3,5-dione (compound 67).

| Structure | LCMS (ESI, *m*/*z*) | NMR |
|---|---|---|
| 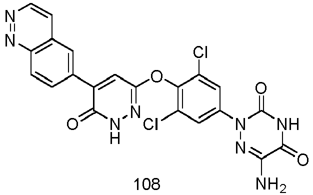 6-amino-2-(3,5-dichloro-4-((5-(cinnolin-6-yl)-6-oxo-1,6-dihydropyridazin-3-yl)oxy)phenyl)-1,2,4-triazine-3,5(2*H*,4*H*)-dione | [M+H]⁺=511 | ¹H NMR (400 MHz, DMSO-*d*₆) δ 12.56 (Br,2H), 9.39(d, J = 3.0 Hz, 1H), 8.71(s, 1H), 8.49-8.46(m, 1H), 8.42-8.38(m, 1H), 8.32-8.21(m,1H),8.07 (s,1H), 7.92(s, 2H), 5.79 (Br,2H) |
| 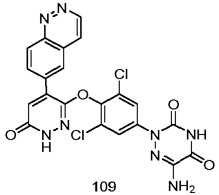 6-amino-2-(3,5-dichloro-4-((4-(cinnolin-6-yl)-6-oxo-1,6-dihydropyridazin-3-yl)oxy)phenyl)-1,2,4-triazine-3,5(2*H*,4*H*)-dione | [M+H]⁺=511 | ¹H NMR (400 MHz, DMSO-*d*₆) δ 12.54 (Br, 1H), 12.30(s, 1H), 9.49(d, J = 3.0 Hz, 1H), 8.64 (d, J = 4.4 Hz, 1H),8.52(s,1H),8.35-8.31(m,2H), 7.91(s, 2H), 7.37(s, 1H), 6.33 (Br,2H) |
| 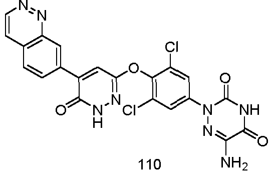 6-amino-2-(3,5-dichloro-4-((5-(cinnolin-7-yl)-6-oxo-1,6-dihydropyridazin-3-yl)oxy)phenyl)-1,2,4-triazine-3,5(2*H*,4*H*)-dione | [M+H]⁺=511 | ¹H NMR (400 MHz, DMSO-*d*₆) δ 12.41 (Br,1H), 9.45(d, J = 3.0 Hz, 1H),9.21(s, 1H), 8.39-8.37(m,1H), 8.37-8.36(m, 1H), 8.28 (s,1H), 8.23-8.17(m, 1H), 7.96(s,2H), 6.10 (Br,2H) |
| 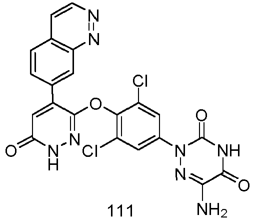 6-amino-2-(3,5-dichloro-4-((4-(cinnolin-7-yl)-6-oxo-1,6-dihydropyridazin-3-yl)oxy)phenyl)-1,2,4-triazine-3,5(2*H*,4*H*)-dione | [M+H]⁺=511 | ¹H NMR (400 MHz, DMSO-*d*₆) δ 12.48 (Br,2H), 9.48(d, J = 3.0 Hz, 1H), 8.91(s, 1H), 8.34-8.32(m,1H), 8.29-8.25(m,2H), 7.94 (s,2H), 7.43(s, 1H), 6.18 (Br,2H) |
| 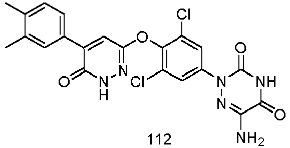 6-amino-2-(3,5-dichloro-4-((5-(3,4-dimethylphenyl)-6-oxo-1,6-dihydropyridazin-3-yl)oxy)phenyl)-1,2,4-triazine-3,5(2*H*,4*H*)-dione | [M+H]⁺=487 | ¹H NMR (400 MHz, DMSO-*d*₆) δ 12.40 (s, 1H), 12.28 (s, 1H), 7.72 - 7.87 (m, 5H), 7.77 (d, J= 7.8Hz, 1H), 6.53 (s, 2H), 2.29 (s, 6H) |
| 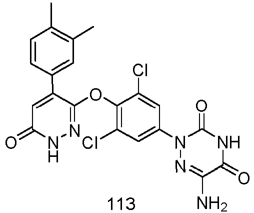 6-amino-2-(3,5-dichloro-4-((4-(3,4-dimethylphenyl)-6-oxo-1,6-dihydropyridazin-3-yl)oxy)phenyl)-1,2,4-triazine-3,5(2*H*,4*H*)-dione | [M+H]⁺=487 | ¹H NMR (400 MHz, DMSO-*d*₆) δ 12.38 (s, 1H), 12.27 (s, 1H), 7.86 (s, 2H), 7.51 - 7.57 (m, 2H), 7.31 (d, J= 7.8Hz, 1H), 7.06 (s, 1H), 6.51 (s, 2H), 2.29 (s, 6H) |
| 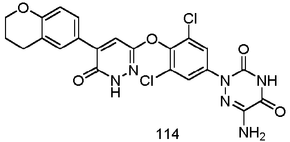 6-amino-2-(3,5-dichloro-4-((5-(chroman-6-yl)-6-oxo-1,6-dihydropyridazin-3-yl)oxy)phenyl)-1,2,4-triazine-3,5(2*H*,4*H*)-dione | [M+H]⁺=515 | ¹H NMR (400 MHz, DMSO-*d*₆) δ 12.37 C 12.29 (m, 2H), 7.86 C 7.78 (m, 5H), 6.83C 6.81 (m, 1H),6.55 (s, 2H), 4.21 C 4.19 (m, 2H), 2.82 C 2.78 (m, 2H), 1.98C 1.95 (m, 6H) |
| 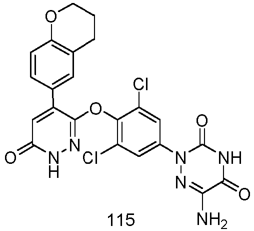 6-amino-2-(3,5-dichloro-4-((4-(chroman-6-yl)-6-oxo-1,6-dihydropyridazin-3-yl)oxy)phenyl)-1,2,4-triazine-3,5(2*H*,4*H*)-dione | [M+H]⁺=515 | ¹H NMR (400 MHz, DMSO-*d*₆) δ 12.92 (s, 2H), 7.86 (s, 2H) , 7.56 C 7.54 (m, 2H) , 7.03 (s, 1H) , 6.90C 6.88 (m, 1H) , 6.54 (s, 2H), 4.21 C 4.19 (m, 2H) , 2.83 C 2.80 (m, 2H), 1.97C 1.94 (m, 2H) |
| 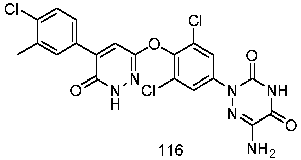 6-amino-2-(3,5-dichloro-4-((5-(4-chloro-3-methylphenyl)-6-oxo-1,6-dihydropyridazin-3-yl)oxy)phenyl)-1,2,4-triazine-3,5(2H,4H)-dione | [M+H]⁺=507 | ¹H NMR (400 MHz, DMSO-*d*₆) δ 12.51 (br, 1H), 12.27 (br, 1H), 7.87 - 8.06 (m, 5H), 7.53 (d, J = 8.1Hz, 1H), 6.53 (s, 2H), 2.40 (s, 3H). |
| 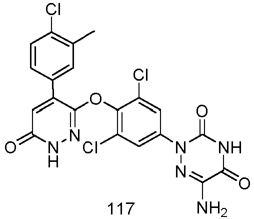 6-amino-2-(3,5-dichloro-4-((4-(4-chloro-3-methylphenyl)-6-oxo-1,6-dihydropyridazin-3-yl)oxy)phenyl)-1,2,4-triazine-3,5(2H,4H)-dione | [M+H]⁺=507 | ¹H NMR (400 MHz, DMSO-*d*₆) δ 12.28 - 12.41 (m, 2H), 7.87 (s, 2H), 7.78 (s, 1H), 7.59 - 7.67 (m, 2H), 7.16 (s, 1H), 6.54 (s, 2H), 2.41 (s, 3H) |
| 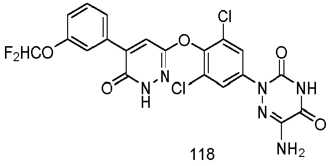 6-amino-2-(3,5-dichloro-4-((5-(3-difluoromethoxyphenyl)-6-oxo-1,6-dihydropyridazin-3-yl)oxy)phenyl)-1,2,4-triazine-3,5(2H,4H)-dione | [M+H]⁺=525 | ¹H NMR (400 MHz, DMSO-*d*₆) δ 12.29-12.57 (m, 2H), 7.99 (s, 1H), 7.82 - 7.90 (m, 4H), 7.53 - 7.60 (m, 1H), 7.23 - 7.40 (m, 2H), 6.55 (s, 2H) |
| 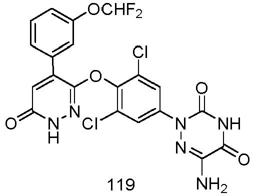 6-amino-2-(3,5-dichloro-4-((4-(3-(difluoromethoxy)phenyl)-6-oxo-1,6-dihydropyridazin-3-yl)oxy)phenyl)-1,2,4-triazine-3,5(2H,4H)-dione | [M+H]⁺=525 | ¹H NMR (400 MHz, DMSO-ds) δ 12.28-12.46 (m, 2H), 7.87 (s, 2H), 7.59 C 7.72 (m, 3H), 7.36-7.39 (m, 2H), 7.21-7.23 (m, 1H), 6.55 (s, 2H) |
| 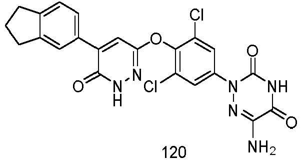 6-amino-2-(3,5-dichloro-4-((5-(2,3-dihydro-1H-inden-5-yl)-6-oxo-1,6-dihydropyridazin-3-yl)oxy)phenyl)-1,2,4-triazine-3,5(2H,4H)-dione | [M+H]⁺=525 | ¹H NMR (400 MHz, DMSO-*d*₆) δ 12.27 - 12.40 (m, 2H), 7.72 - 7.87 (m, 5H), 7.33 (d, J = 8.1 Hz, 1H), 6.53 (s, 2H), 2.50 (t, J = 1.8Hz, 4H), 2.01 - 2.11 (m, 2H) |
| 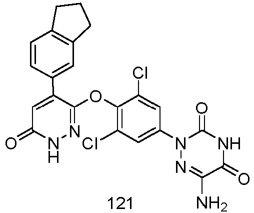 6-amino-2-(3,5-dichloro-4-((4-(2,3-dihydro-1H-inden-5-yl)-6-oxo-1,6-dihydropyridazin-3-yl)oxy)phenyl)-1,2,4-triazine-3,5(2H,4H)-dione | [M+H]⁺=525 | ¹H NMR (400 MHz, DMSO-*d*₆) δ 12.26 - 12.33 (m, 2H), 7.86 (s, 2H), 7.63 (s, 1H), 7.55 (d, J = 7.8 Hz, 1H), 7.39 (d, J = 7.8 Hz, 1H), 7.05 (s, 1H), 6.53 (s, 2H), 2.91 - 2.96 (m, 4H), 2.01 - 2.11 (m, 2H) |
| 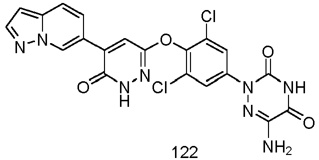 6-amino-2-(3,5-dichloro-4-((6-oxo-5-(pyrazolo[1,5-a]pyridin-6-yl)-1,6-dihydropyridazin-3-yl)oxy)phenyl)-1,2,4-triazine-3,5(2H,4H)-dione | [M+H]⁺=499 | ¹H NMR (400 MHz, DMSO-*d*₆) δ 12.64 (s, 1H), 12.29 (s, 1H), 9.84 (s, 1H), 8.09 - 8.18 (m, 2H), 7.76 - 7.89 (m, 4H), 6.66 - 6.71 (m, 1H), 6.53 (s, 2H) |
| 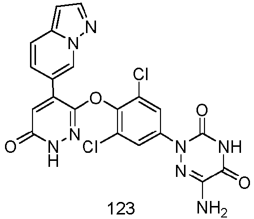 6-amino-2-(3,5-dichloro-4-((6-oxo-4-(pyrazolo[1,5-a]pyridin-6-yl)-1,6-dihydropyridazin-3-yl)oxy)phenyl)-1,2,4-triazine-3,5(2H,4H)-dione | [M+H]⁺=499 | ¹H NMR (400 MHz, DMSO-*d*₆) δ 12.44 (s, 1H), 12.28 (s, 1H), 9.17 (s, 1H), 8.11 - 8.15 (m, 1H), 7.82 - 7.89 (m, 3H), 7.57 - 7.62 (m, 1H), 7.35 (s, 1H), 6.70 - 6.74 (m, 1H), 6.54 (s, 2H) |
| 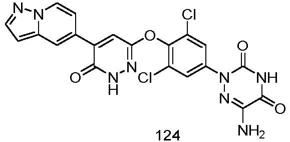 6-amino-2-(3,5-dichloro-4-((6-oxo-5-(pyrazolo[1,5-a]pyridin-5-yl)-1,6-dihydropyridazin-3-yl)oxy)phenyl)-1,2,4-triazine-3,5(2H,4H)-dione | [M+H]⁺=499 | ¹H NMR (300 MHz, DMSO-*d*₆) δ 12.60 (s, 1H), 12.28 (s, 1H), 8.70 - 8.77 (m, 2H), 8.08 - 8.11 (m, 2H), 7.88 (s, 2H), 7.46 - 7.49 (m, 1H), 6.82 (d, J = 1.8 Hz. 1H), 6.54 (br, 2H) |
| 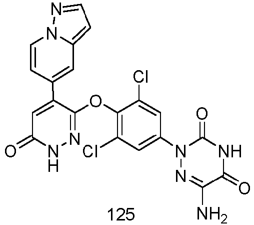 6-amino-2-(3,5-dichloro-4-((6-oxo-4-(pyrazolo[1,5-a]pyridin-5-yl)-1,6-dihydropyridazin-3-yl)oxy)phenyl)-1,2,4-triazine-3,5(2H,4H)-dione | [M+H]⁺=499 | ¹H NMR (300 MHz, DMSO-*d*₆) δ 0.85 (d, J = 7.2 Hz, 1H), 8.11 - 8.18 (m, 2H), 7.98 (s, 2H), 8.27 - 7.28 (m, 2H), 6.82 (d, J = 1.5 Hz. 1H), 5.87 (br, 2H) |
| 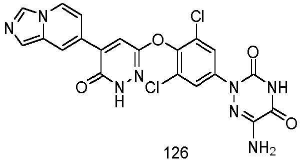 6-amino-2-(3,5-dichloro-4-((5-(imidazo[1,5-a]pyridin-7-yl)-6-oxo-1,6-dihydropyridazin-3-yl)oxy)phenyl)-1,2,4-triazine-3,5(2H,4H)-dione | [M+H]⁺=499 | ¹H NMR (300 MHz, DMSO-*d*₆) δ 12.52 (s, 1H), 8.80 C 8.81 (m, 1H), 8.39 C 8.47 (m, 2H), 8.01 (s, 1H), 7.87 (s, 2H), 7.62 (s, 1H), 7.25 C 7.29 (m, 1H), 6.47 (s, 2H). |
| 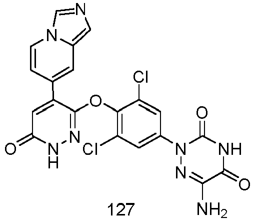 6-amino-2-(3,5-dichloro-4-((4-(imidazo[1,5-a]pyridin-7-yl)-6-oxo-1,6-dihydropyridazin-3-yl)oxy)phenyl)-1,2,4-triazine-3,5(2H,4H)-dione | [M+H]⁺=499 | ¹H NMR (300 MHz, DMSO-*d*₆) δ 12.25 C 12.38 (m, 2H), 8.42 C 8.53 (m, 2H), 8.11 (s, 1H), 7.88 (s, 2H), 7.59 (s, 1H), 7.23 (s, 1H), 7.03 C 7.07 (m, 1H), 6.49 (s, 2H) |
| 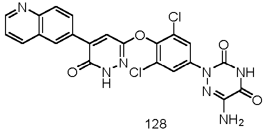 6-amino-2-(3,5-dichloro-4-((6-oxo-5-(quinolin-6-yl)-1,6-dihydropyridazin-3-yl)oxy)phenyl)-1,2,4-triazine-3,5(2H,4H)-dione | [M+H]⁺=510 | ¹H NMR (300 MHz, DMSO-*d*₆) δ 12.52 (br, 1H), 8.97 - 8.94 (m, 1H), 8.72- 8.71 (m, 1H), 8.51-8.47 (m,1H), 8.28 (d,J=1Hz, 1H), 8.05-8.02 (m, 4H), 7.62-7.60(m, 1H),5.65(s, 2H) |
| 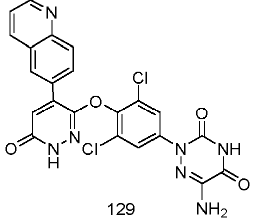 6-amino-2-(3,5-dichloro-4-((6-oxo-4-(quinolin-6-yl)-1,6-dihydropyridazin-3-yl)oxy)phenyl)-1,2,4-triazine-3,5(2H,4H)-dione | [M+H]⁺=510 | ¹H NMR (400 MHz, DMSO-*d*₆) δ 9.01 C 8.99 (m, 1H), 8.49 (d, J = 4.0 Hz, 1H), 8.43 (s, 1H), 8.17 (s,2H), 8.01 (s, 2H), 7.65 - 7.62 (m, 1H), 7.22 (s, 1H), 5.56(s, 2H). |
| 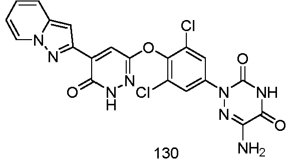 6-amino-2-(3,5-dichloro-4-((6-oxo-5-(pyrazolo[1,5-a]pyridin-2-yl)-1,6-dihydropyridazin-3-yl)oxy)phenyl)-1,2,4-triazine-3,5(2H,4H)-dione | [M+H]⁺=499 | ¹H NMR (400 MHz, DMSO-*d*₆) δ 12.78 - 11.75 (m, 2H), 8.79 C 8.72 (m, 1H), 8.07 (s, 1H), 7.92 C 7.74 (m, 3H), 7.62 (d, J = 0.9 Hz, 1H), 7.27-7.15(m, 1H), 7.03-6.95 (m, 1H), 6.49 (s, 2H) |
| 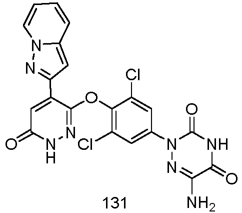 6-amino-2-(3,5-dichloro-4-((6-oxo-4-(pyrazolo[1,5-a]pyridin-2-yl)-1,6-dihydropyridazin-3-yl)oxy)phenyl)-1,2,4-triazine-3,5(2H,4H)-dione | [M+H]⁺=499 | ¹H NMR (400 MHz, DMSO-*d*₆) δ 8.84-8.82 (m, 1H), 7.89 (s, 2H), 7.83 (m, 1H), 7.52 (s, 1H), 7.35 (d, J = 0.9 Hz, 1H), 7.34-7.32 (m, 1H), 7.08-7.03 (m, 1H), 6.49 (s, 2H). |
| 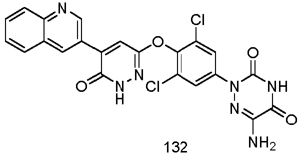 6-amino-2-(3,5-dichloro-4-((6-oxo-5-(quinolin-3-yl)-1,6-dihydropyridazin-3-yl)oxy)phenyl)-1,2,4-triazine-3,5(2H,4H)-dione | [M+H]⁺=510 | ¹H NMR (400 MHz, DMSO-*d*₆) δ 9.38 (s, 1H), 9.04 (d, J = 2.0 Hz, 1h), 8.19 (s, 1H), 8.08 - 8.11 (m, 2H), 7.93 (s, 2H), 7.84 - 7.88 (m, 1H), 7.68 - 7.71 (m, 1H), 6.32 (s, 2H). |
| 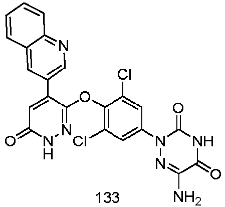 6-amino-2-(3,5-dichloro-4-((6-oxo-4-(quinolin-3-yl)-1,6-dihydropyridazin-3-yl)oxy)phenyl)-1,2,4-triazine-3,5(2H,4H)-dione | [M+H]⁺=510 | ¹H NMR (400 MHz, DMSO-*d*₆) δ 12.25 (br, 2H), 9.28 (d, J = 2.0 Hz, 1H), 8.81 (d, J = 2.0 Hz, 1H), 8.12 (d, J = 8.8 Hz, 2H), 7.89 - 7.92 (m, 3H), 7.71 - 7.75 (m, 1H), 7.43 (s, 1H), 6.51 (s, 2H). |
| 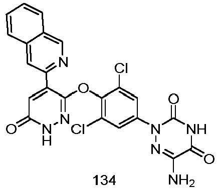 6-amino-2-(3,5-dichloro-4-((4-(isoquinolin-3-yl)-6-oxo-1,6-dihydropyridazin-3-yl)oxy)phenyl)-1,2,4-triazine-3,5(2H,4H)-dione | [M+H]⁺=510 | ¹H NMR (400 MHz, DMSO-*d*₆) δ 9.53 (s, 1H), 8.66 (s, 1H), 8.26 (d, J = 8.1 Hz, 1H), 8.15 (d, J = 8.4 Hz, 1H), 7.80 - 7.93 (m, 4H), 7.62 (s, 1H), 6.33 (br, 2H). |

The following compounds were prepared similarly as described for compounds 91 and 91-A.

| Structure | LCMS (ESI, *m*/*z*) | ¹H NMR |
|---|---|---|
| 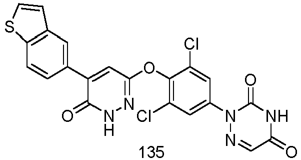 2-(4-((5-(benzo[b]thiophen-5-yl)-6-oxo-1,6-dihydropyridazin-3-yl)oxy)-3,5-dichlorophenyl)-1,2,4-triazine-3,5(2H,4H)-dione | [M+H]⁺=500 | ¹H NMR (400 MHz, DMSO-*d*₆) δ 7.57 (d, 1H, J = 5.4 Hz), 7.71 (s, 1H), 7.83-7.86 (m, 3H), 7.93 (dd, 1H, J = 8.6 Hz, 1.8 Hz), 7.98 (s, 1H), 8.12 (d, 1H, J = 8.5 Hz), 8.59 (d, 1H, J = 1.8 Hz), 12.50-12.52 (m, 2H) |
| 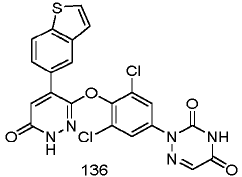 2-(4-((4-(benzo[b]thiophen-5-yl)-6-oxo-1,6-dihydropyridazin-3-yl)oxy)-3,5-dichlorophenyl)-1,2,4-triazine-3,5(2H,4H)-dione | [M+H]⁺=500 | ¹H NMR (400 MHz, DMSO-*d*₆) δ 7.21 (d, 1H, J = 2.1 Hz), 7.58 (d, 1H, J = 5.5 Hz), 7.70 (br.s, 1 H), 7.77 (dd, 1H, J = 8.6 Hz, 1.9 Hz), 7.82 (s, 2H), 7.9 (d, 1H, J = 5.5 Hz), 8.2 (d, 1H, J = 8.7 Hz), 8.31 (d, 1H, J = 1.6 Hz), 12.41 (br.s, 1H), 12.49 (br.s, 1H) |
| 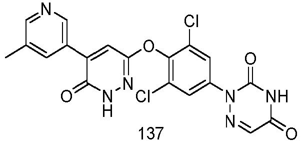 2-(3,5-dichloro-4-((5-(5-methylpyridin-3-yl)-6-oxo-1,6-dihydropyridazin-3-yl)oxy)phenyl)-1,2,4-triazine-3,5(2H,4H)-dione | [M+H]⁺=459 | ¹H NMR (400 MHz, DMSO-*d*₆) δ 2.37 (s, 3H), 7.59 (br s, 1H), 7.82 (s, 2H), 8.02 (s, 1H), 8.17-8.19 (m, 1H), 8.51 (d, J = 1.7 Hz, 1H), 8.91 (d, J = 1.9 Hz, 1H), 12.50 (br s, 1H), 12.57 (s, 1H) |
| 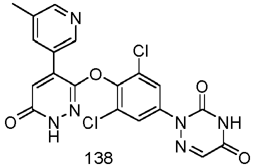 2-(3,5-dichloro-4-((4-(5-methylpyridin-3-yl)-6-oxo-1,6-dihydropyridazin-3-yl)oxy)phenyl)-1,2,4-triazine-3,5(2H,4H)-dione | [M+H]⁺=459 | ¹H NMR (400 MHz, DMSO-*d*₆) δ 2.40 (s, 3H), 7.27 (d, J = 2.0 Hz, 1H), 7.66 (s, 1H), 7.82 (s, 2H), 8.00-8.04 (m, 1H), 8.57 (d, J = 1.5 Hz, 1H), 8.79 (d, J = 1.8 Hz, 1H), 12.46 (d, J = 1.6 Hz, 1H), 12.49 (s, 1H) |
| 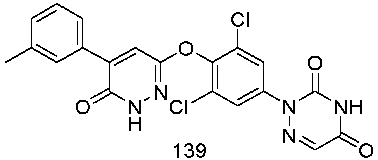 2-(3,5-dichloro-4-((6-oxo-5-(m-tolyl)-1,6-dihydropyridazin-3-yl)oxy)phenyl)-1,2,4-triazine-3,5(2H,4H)-dione | [M+H]⁺=458 | ¹H NMR (400 MHz, DMSO-*d*₆) δ 2.38 (s, 3H), 7.31 (d, 1H, J = 7.6 Hz), 7.38 (t, 1H, J = 7.6 Hz), 7.71 (s, 1H), 7.75- 7.78 (m, 2H), 7.82 (s, 2H), 7.86 (s, 1H), 12.47 (s, 1H), 12.50 (br.s, 1H) |
| 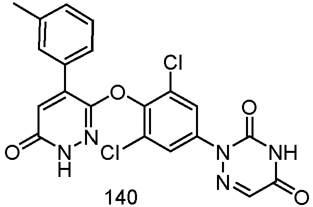 2-(3,5-dichloro-4-((6-oxo-4-(m-tolyl)-1,6-dihydropyridazin-3-yl)oxy)phenyl)-1,2,4-triazine-3,5(2H,4H)-dione | [M+H]⁺=458 | ¹H NMR (400 MHz, DMSO-*d*₆) δ 2.38 (s, 3H), 7.1 (d, 1H, J = 2.2 Hz), 7.36 (d, 1H, J = 7.6 Hz), 7.44 (t, 1H, J = 7.7 Hz), 7.59-7.61 (m, 2H), 7.70 (s, 1H), 7.81 (s, 2H), 12.38 (br.s, 1H), 12.49 (br.s, 1H) |
| 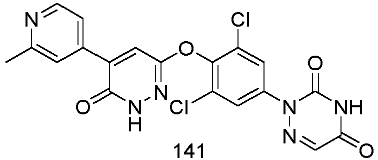 2-(3,5-dichloro-4-((5-(2-methylpyridin-4-yl)-6-oxo-1,6-dihydropyridazin-3-yl)oxy)phenyl)-1,2,4-triazine-3,5(2H,4H)-dione | [M+H]⁺=459 | ¹H NMR (400 MHz, DMSO-*d*₆) δ 2.55 (s, 3H), 7.72 (d, 1H, J = 1.8 Hz), 7.75 (dd, 1H, J = 5.0 Hz, J = 1.8 Hz), 7.82 (br.s, 1H), 7.83 (s, 2H), 8.07 (s, 1H), 8.57 (d, 1H, J = 5.3 Hz), 12.50 (br.s, 1H), 12.63 (s, 1H). |
| 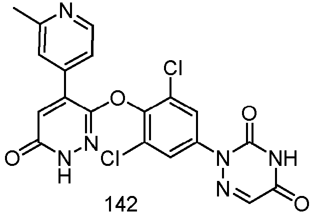 2-(3,5-dichloro-4-((4-(2-methylpyridin-4-yl)-6-oxo-1,6-dihydropyridazin-3-yl)oxy)phenyl)-1,2,4-triazine-3,5(2H,4H)-dione | [M+H]⁺=459 | ¹H NMR (400 MHz, DMSO-*d*₆) δ 2.62 (s, 3H), 7.32 (s, 1H), 7.71 (d, 1H, J = 1.8 Hz), 7.73 (dd, 1 H, J = 5.2 Hz, J = 1.8 Hz), 7.79 (br.s, 1H), 7.82 (s, 2H), 8.72 (d, 1H, J = 5.4 Hz), 12.49 (s, 1H), 12.56 (br.s, 1H). |

### Example 77: Synthesis of compound 143

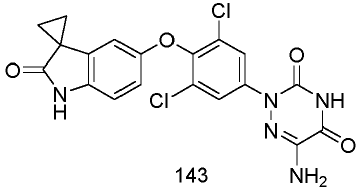

The titled compound was prepared similarly as described for compound 82 with the exception that 5'-bromospiro[cyclopropane-1,3'-indolin]-2'-one was used instead of 5-bromo-3,3-dimethylindolin-2-one. ¹H NMR (400 MHz, DMSO-*d*₆) δ 1.44-1.48 (m, 2H), 1.55-1.60 (m, 2H), 6.43 (dd, J= 8.4 Hz,2.6 Hz,1H), 6.49 (brs, 2H), 6.75 (d, J= 2.6 Hz, 1H), 6.80 (d, J= 8.4 Hz, 1H), 7.89 (s, 2H), 10.47 (s, 1H), 12.26 (brs, 1H).

### Example 78: Synthesis of compound 144

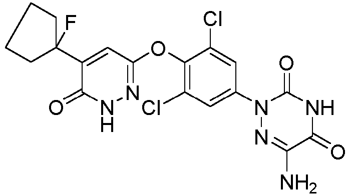

A 500 mL round-bottom flask was charged with 2,2,6,6-tetramethylpiperidine (26 mL) in THF (100 mL) under N₂. *n*-Butyllithium (60.00 mL, 636.953 mmol) was added dropwise at -78°C. The mixture was warm to -45°C and stirred for 30 min. Then the mixture was cooled to -78 °C and 3,6-dichloropyridazine (10.00 g, 67.128 mmol) in THF (100 mL) was added dropwise. The mixture was stirred for 30 min at -78 °C. Cyclopentanone (60 mL) in THF (50 mL) was added dropwise. The reaction was stirred for 60 min at -75 °C. The reaction was quenched by saturated ammonium chloride (100 mL) and extracted with EA (3 x 100 mL), the combined organic layers were washed with brine (2 x 100 mL), dried over anhydrous sodium sulfate, filtered and concentrated under reduced pressure. The residue was purified by silica gel column chromatography with EA/PE (2/5) to afford 1-(3,6-dichloropyridazin-4-yl)cyclopentan-1-ol (5 g, 28.76%) as yellow oil. LCMS (ESI, m/z): 233 [M+H]⁺.

A 100 mL round-bottom flask was charged with 1-(3,6-dichloropyridazin-4-yl)cyclopentan-1-ol (4.50 g, 19.306 mmol) in CH₂Cl₂ (50 mL) under N₂. Bis(2-methoxyethyl)aminosulfur trifluoride (BAST) (12.81 g, 57.918 mmol, 3.00 equiv) was added dropwise at 0 °C. The reaction was stirred for 16h at rt. The reaction mixture was diluted with water (50 mL) and filtered through celite, the celite pad was washed with dichloromethane (2 x 50 mL), the filtrate was washed with brine (2 x 50 mL). The organic layer was dried over anhydrous sodium sulfate, filtered and concentrated under reduced pressure. The residue was chromatographed on a silica gel column with PE /EA = 5/2 to provide the 3,6-dichloro-4-(1-fluorocyclopentyl)pyridazine (2.5 g, 44%) as a yellow oil. LCMS (ESI, *m*/*z):* 235 [M+H]⁺.

To a solution of 3,6-dichloro-4-(1-fluorocyclopentyl) pyridazine (500.00 mg, 2.127 mmol), t-butyl N-[2-(3,5-dichloro-4-hydroxyphenyl)-3,5-dioxo-4H-1,2,4-triazin-6-yl]carbamate (827.78 mg, 2.127 mmol) and K₂CO₃ (881.86 mg, 6.381 mmol, 3.00 equiv) in DMSO (10 mL) was added CuI (81.01 mg, 0.425 mmol) under nitrogen. The mixture was stirred for 16h at 110 °C and then quenched with water (20 mL). The mixture was acidified to pH ~5 with HCl (1M, aq.). The resulting mixture was extracted with ethyl acetate (3 x 30 mL). The combined organic layers were washed with brine (2 x 20 mL), dried over anhydrous sodium sulfate. After filtration, the filtrate was concentrated. The residue was purified by silica gel column chromatography, eluted with EA/ PE (1:1) to afford 6-amino-2-(3,5-dichloro-4-[[6-chloro-5-(1-fluorocyclopentyl)pyridazin-3-yl]oxy]phenyl)-4H-1,2,4-triazine-3,5-dione (250 mg, 24%) as a yellow solid. LCMS (ESI, *m*/*z):* 487 [M+H]⁺.

To a solution of 6-amino-2-(3,5-dichloro-4-[[6-chloro-5-(1-fluorocyclopentyl)pyridazin-3-yl]oxy]phenyl)-4*H*-1,2,4-triazine-3,5-dione (200.00 mg, 0.410 mmol) in acetic acid (5 mL) was added sodium acetate (168.21 mg, 2.050 mmol). The mixture was stirred overnight at 100 °C. The mixture reaction was cooled to rt and then quenched with water(10ml) and then stirred for 10 min, the resulting solid was filtered and washed with water(2 x 10ml) and petroleum ether (2 X 5mL), then dried under high vaccum to afford the crude product. The crude product (100 mg) was purified by preparative HPLC using the following gradient conditions: Column: XBridge Prep OBD C18 Column, 19 x 250 mm, 5um; mobile Phase A:Water (10 MMOL/L NH₄HCO₃+0.1%NH₃.H₂O), Mobile Phase B: CH₃CN ; Flow rate:25 mL/min; Gradient:29 B to 49 B in 7 min; 220 nm; RT1:6.98; Purification resulted in 6-amino-2-(3,5-dichloro-4-[[5-(1-fluorocyclopentyl)-6-oxo-1H-pyridazin-3-yl]oxy]phenyl)-4*H*-1,2,4-triazine-3,5-dione (4 mg, 2.06%) as a white solid. ¹H NMR (400 MHz, DMSO-*d*₆) δ 12.47 (s, 1H), 12.27 (s, 1H), 7.87 (s, 2H), 7.53(s, 1H), 6.50 (s, 2H), 2.50 - 2.49 (m, 2H), 1.87 - 1.85 (m, 6H).

### Example 79: Synthesis of compound 145

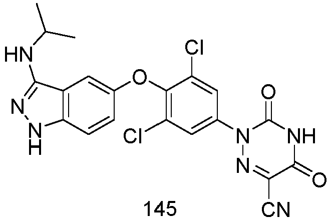

A mixture of 2-bromo-5-hydroxybenzonitrile (5 g, 25.25 mmol), 1,2,3-trichloro-5-nitrobenzene (5.72 g, 25.25 mmol) and K₂CO₃ (5.23 g, 37.88 mmol) in DMF (185 mL) under N₂ was stirred at 60 °C for 1 h. After cooling to rt, the reaction mixture was diluted in water and filtered. The precipitate was washed with water and dissolved in DCM. The solution was dried with MgSO₄, filtered and evaporated to dryness providing 2-bromo-5-(2,6-dichloro-4-nitrophenoxy)benzonitrile (9.65 g, 98%) as a yellow solid which was used without further purification. ¹H NMR (400 MHz, DMSO-*d*₆) δ 7.36 (dd, *J* = 8.9 Hz, 3.1 Hz, 1H), 7.71 (d, *J= 3.2* Hz, 1H), 7.88 (d, *J=* 9.0 Hz, 1H), 8.58 (s, 2H) ppm.

A solution of benzophenone hydrazone (1.89 g, 9.64 mmol), Pd(OAc)₂ (98 mg, 0.44 mmol) and BINAP (300.1 mg, 0.48 mmol) in toluene (17 mL) was evacuated and backfilled with N₂ (3x) and the reaction mixture was heated at 100 °C for 3 min. After cooling to rt, 2-bromo-5-(2,6-dichloro-4-nitrophenoxy)benzonitrile (3.4 g, 8.76 mmol), Cs₂CO₃ (4 g, 12.27 mmol) and toluene (5.1 mL) were added. The reaction vessel was again evacuated and backfilled with N₂ (3x) and the reaction mixture was heated at 100 °C for 5 h. After cooling to rt, the mixture was filtrated through a pad of Celite, the Celite was washed with DCM, and the solution was evaporated to dryness providing 5-(2,6-dichloro-4-nitrophenoxy)-2-(2-(diphenylmethylene)hydrazinyl) benzonitrile as a dark red solid (4.41 g, quant.) which was used without further purification. ¹H NMR (400 MHz, DMSO-*d*₆) δ 6.76 (d, *J* = 2.9 Hz, 1H), 7.15 (dd, *J= 9.3* Hz, 2.9 Hz, 1H), 7.32-7.37 (m, 6H), 7.59-7.63 (m, 4H), 7.73 (d, *J* = 9.3 Hz, 1H), 8.08 (s, 1H), 8.31 (s, 2H) ppm.

*p*-Toluenesulfonic acid monohydrate (3.33 g, 17.5 mmol) was added to a solution of 5-(2,6-dichloro-4-nitrophenoxy)-2-(2-(diphenylmethylene)hydrazineyl)benzonitrile (4.4 g, 8.74 mmol) in MeOH (61 mL) under N₂. The reaction mixture was refluxed for 21 h. After cooling to rt, the reaction mixture was diluted with a sat. aq. Na₂CO₃ and extracted with EA (3x). The combined organic layers were washed with brine (3x) and water, dried over MgSO₄, filtered and evaporated to dryness. The crude was purified by chromatography on silica gel (20% to 70% EA in DCM) providing 5-(2,6-dichloro-4-nitrophenoxy)-1H-indazol-3-amine (1.45 g, 49%) as a yellow solid. ¹H NMR (400 MHz, DMSO-*d*₆) δ 5.20 (s, 2H), 6.99 (d, *J* = 2.4 Hz, 1H), 7.08 (dd, *J* = 9.0 Hz, 2.4 Hz, 1H), 7.26 (d, *J =* 9.0 Hz, 1H), 8.57 (s, 2H), 11.40 (s, 1H) ppm.

Trifluoroacetic anhydride (0.36 mL, 2.57 mmol) was added to a solution of 5-(2,6-dichloro-4-nitrophenoxy)-1H-indazol-3-amine (435 mg, 1.28 mmol) in anhydrous DCM (10 mL) at 0 °C under N₂. The reaction mixture was stirred at rt for 1 h. Afterward, the reaction mixture was diluted in water and extracted with DCM (3x). The combined organic layers were dried over MgSO₄, the solids were removed by filtration and the solvent of the filtrate was evaporated to dryness. The crude mixture was purified by flash chromatography on silica gel (20% to 60% EA in cyclohexane) to give N-(5-(2,6-dichloro-4-nitrophenoxy)-1*H*-indazol-3-yl)-2,2,2-trifluoroacetamide (558 mg, quant.) as a yellow solid. ¹H NMR (400 MHz, DMSO-*d*₆) δ 7.05 (d, *J =* 2.1 Hz, 1H), 7.17 (dd, *J* = 9.1 Hz, 2.2 Hz, 1H), 7.57 (d, *J =* 9.0 Hz, 1H), 8.56 (s, 2H), 11.82 (s, 1H), 13.18 (s, 1H) ppm.

A solution of NH₄Cl (685.9 mg, 12.82 mmol) in water (6.4 mL) was added to a solution of *N*-(5-(2,6-dichloro-4-nitrophenoxy)-1H-indazol-3-yl)-2,2,2-trifluoroacetamide (558 mg, 1.28 mmol) in ethanol (13 mL) under N₂. Fe (358.1 mg, 6.41 mmol) was added and the reaction mixture was stirred at 70 °C for 3 h. After cooling to rt, HCl 37% (1 mL) was added and the reaction mixture was diluted in water. K₂CO₃ was slowly added until basic pH and the resulting solution was extracted with EA (3x). The combined organic layers were dried with Na₂SO₄, filtered and evaporated to dryness providing *N*-(5-(4-amino-2,6-dichlorophenoxy)-1*H*-indazol-3-yl)-2,2,2-trifluoroacetamide (365 mg, 70%) as a green solid which was used without further purification. ¹H NMR (400 MHz, DMSO-*d*₆) δ 5.60 (s, 2H), 6.73 (s, 2H), 6.85 (s, 1H), 7.08 (d, *J=* 9.1 Hz, 1H), 7.50 (d, *J=* 9.0 Hz, 1H), 11.74 (s, 1H), 13.01 (s, 1H) ppm.

A solution of NaNO₂ (94.8 mg, 1.37 mmol) in water (13.7 mL) was added to a solution of *N*-(5-(4-amino-2,6-dichlorophenoxy)-1H-indazol-3-yl)-2,2,2-trifluoroacetamide (265 mg, 0.65 mmol) in HCl 37% (5.7 mL, 69.1 mmol), acetic acid (17.5 mL) and water (13.7 mL) at 0 °C under N₂. The reaction mixture was stirred at 0 °C for 1 h. In parallel, a solution of ethyl *N*-(2-cyanoacetyl)carbamate (153.2 mg, 0.98 mmol) in water (16.5 mL) and pyridine (5.7 mL) was stirred at 0 °C for 15 min. The first reaction mixture was quickly added to the second one. The resulting reaction mixture was stirred at 0 °C for 1.5 h. Afterwards, the reaction mixture was diluted in water and filtered. The precipitate was washed with water providing ethyl (2-cyano-2-(2-(3,5-dichloro-4-((3-(2,2,2-trifluoroacetamido)-1*H*-indazol-5-yl)oxy)phenyl)hydrazineylidene)acetyl)carbamate (170 mg, 45%) as a beige solid which was used without further purification.

Sodium acetate (97.5 mg, 1.19 mmol) was added to a solution of ethyl (2-cyano-2-(2-(3,5-dichloro-4-((3-(2,2,2-trifluoroacetamido)-1*H*-indazol-5-yl)oxy)phenyl)hydrazineylidene)acetyl)carbamate (170 mg, 0.3 mmol) in acetic acid (10 mL) under N₂. The reaction mixture was stirred at 120 °C for 3 h. After cooling to 0 °C, the reaction mixture was diluted in water, stirred for 30 min at 0 °C and filtered. The precipitate was washed with water and pentane. The crude mixture was purified by flash chromatography on silica gel (0% to 10% MeOH in DCM) to give *N*-(5-(2,6-dichloro-4-(6-cyano-3,5-dioxo-4,5-dihydro-1,2,4-triazin-2(3H)-yl)phenoxy)-1*H*-indazol-3-yl)-2,2,2-trifluoroacetamide (45 mg, 29%) as an orange solid. LCMS: C₁₉H₈Cl₂F₃N₇O₄ [M+H]⁺: 525.

A solution of *N*-(5-(2,6-dichloro-4-(6-cyano-3,5-dioxo-4,5-dihydro-1,2,4-triazin-2(3*H*)-yl)phenoxy)-1H-indazol-3-yl)-2,2,2-trifluoroacetamide (158 mg, 0.3 mmol) in ammonia (15 mL, 7 N in MeOH) under N₂ was stirred at 60 °C for 3 h. The reaction mixture was evaporated to dryness to give 2-(4-((3-amino-1*H*-indazol-5-yl)oxy)-3,5-dichlorophenyl)-3,5-dioxo-2,3,4,5-tetrahydro-1,2,4-triazine-6-carbonitrile (127 mg, 98%) as an orange solid which was used without further purification. ¹H NMR (400 MHz, DMSO-*d*₆) δ 5.25 (br s, 2H), 6.96 (d, *J =* 2.6 Hz, 1H), 7.05 (dd, *J =* 9.2 Hz, 2.6 Hz, 1H), 7.09 (br s, 1H), 7.24 (d, *J =* 8.9 Hz, 1H), 7.81 (s, 2H), 11.34 (br s, 1H) ppm.

Acetone (0.109 mL, 1.48 mmol) was added to a solution of 2-(4-((3-amino-1*H*-indazol-5-yl)oxy)-3,5-dichlorophenyl)-3,5-dioxo-2,3,4,5-tetrahydro-1,2,4-triazine-6-carbonitrile (127 mg, 0.3 mmol) in ethanol (1.5 mL), THF (1.5 mL) and acetic acid (0.3 mL) under N₂. After cooling to 0 °C, NaCNBH₃ (2 eq., 37.1 mg, 0.59 mmol) was added and the mixture was stirred at 0 °C for 1.5 h. Then, the reaction mixture was diluted in water and filtered. The precipitate was washed with water. The crude mixture was purified by flash chromatography on silica gel (0% to 10% MeOH in DCM) and the resulting product was dissolved in MTBE and filtered. The solution was evaporated to dryness to give 2-(3,5-dichloro-4-((3-(isopropylamino)-1H-indazol-5-yl)oxy)phenyl)-3,5-dioxo-2,3,4,5-tetrahydro-1,2,4-triazine-6-carbonitrile (12 mg, 9%) as a red solid. ¹H NMR (400 MHz, DMSO-*d*₆) δ 1.17 (d, *J* = 6.4 Hz, 6H), 3.76 (br s, 1H), 5.67 (s, 1H), 6.98 (d, *J* = 2.5 Hz, 1H), 7.09 (dd, *J*= 2.5 Hz, 8.9 Hz, 1H), 7.26 (d, *J= 8.9* Hz, 1H), 7.81 (s, 2H), 11.34 (s, 1H), 13.28 (br s, 1 H).

### Example 80: Synthesis of compound 146 and 147

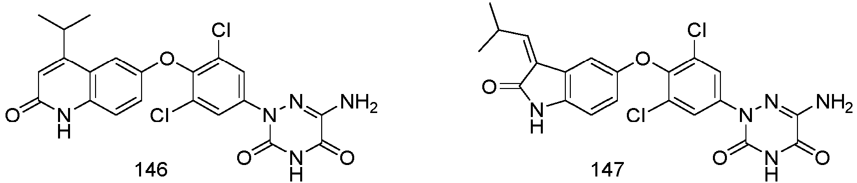

The mixture of 4-amino-2,6-dichloro-phenol (30 g, 168.52 mmol) and Boc₂O (91.95 g, 421.31 mmol, 96.79 mL) in THF (400 mL) was heated to 80°C for 2h. The mixture was concentrated to afford crude. The residue was purified by silica gel chromatography (eluent of 0-15% EA/PE) to afford t-butyl N-(3,5-dichloro-4-hydroxy-phenyl)carbamate (35 g, 125.84 mmol, 75% yield) was obtained as light yellow solid. ¹H NMR (400MHz, CDCl₃) δ 7.19 (s, 1H), 6.27 (br s, 1H), 5.55 (s, 1H), 1.44 (s, 10H).

The mixture of t-butyl N-(3,5-dichloro-4-hydroxy-phenyl)carbamate (33.14 g, 119.15 mmol) and 2-bromo-4-fluoro-1-nitro-benzene (31.46 g, 142.98 mmol) and Cs₂CO₃ (54.35 g, 166.81 mmol) in CH₃CN (500 mL) was heated to 65°C for 18 h. The mixture was concentrated to remove CH₃CN and diluted with EA (500 mL) and the organic layer was washed with H₂O (200 mL x 2). The organic layer was dried over MgSO₄ and concentrated to afford crude, purified by silica gel chromatography (eluent of 0~15% EA/PE) to afford t-butyl N-[4-(3-bromo-4-nitro-phenoxy)-3,5-dichloro-phenyl] carbamate (20 g, 41.83 mmol, 35% yield) obtained as white solid. ¹H NMR (400MHz, CDCl₃) δ 7.97 (d, J=9.0 Hz, 1H), 7.55 (s, 2H), 7.18 (d, J=2.6 Hz, 1H), 6.88 (dd, J=2.6, 9.0 Hz, 1H), 6.60 (s, 1H), 1.56 (s, 9H).

The mixture of t-butyl N-[4-(3-bromo-4-nitro-phenoxy)-3,5-dichlorophenyl]carbamate (21.5 g, 44.97 mmol) and TFA (51.27 g, 449.68 mmol, 33.29 mL) in DCM (200 mL) was stirred at 20°C for 4h. The mixture was concentrated. NaHCO₃ (sat., aq., 500 mL) was added to mixture. The mixture was stirred at 20°C for 4 h. The precipitated solid was filtered and dried. 4-(3-bromo-4-nitro-phenoxy)-3,5-dichloro-aniline (16.1 g, 39.18 mmol, 87% yield, 92% purity) was obtained as yellow solid.

To a solution of 4-(3-bromo-4-nitro-phenoxy)-3,5-dichloro-aniline (5 g, 13.23 mmol) in AcOH (60 mL) was added HCl (12 M, 11.02 mL). The mixture was cooled to 5°C in an ice bath. A solution of NaNO₂ (2.74 g, 39.68 mmol) in H₂O (30 mL) was added to above suspended solution, while maintaining the temperature between 5-10°C. The mixture was stirred at 5°C for 30 min. A solution of ethyl *N*-(2-cyanoacetyl)carbamate (2.48 g, 15.87 mmol) in pyridine (25 mL) was added to above light yellow clear solution slowly. The mixture was stirred at 5°C for 1 hr and then 20°C for 1h. The mixture was filtered and the filtered solid was diluted with ethyl acetate (500 mL) and washed with H₂O (200 mL x 2). The organic layer was dried over MgSO4 and concentrated to afford crude product. The crude product was used for the next step without further purification. ethyl N-[2-[[4-(3-bromo-4-nitro-phenoxy)-3,5-dichloro-phenyl]hydrazono]-2-cyano-acetyl]carbamate (6.7 g, 12.29 mmol, 93% yield) was obtained as red solid. ¹H NMR (400 MHz, DMSO-*d*₆) δ 10.94 (s, 1H), 8.13 - 8.06 (m, 3H),7.51 (d, J=2.6 Hz, 1H), 7.09 (dd, J=2.7, 9.1 Hz, 1H), 4.21 (q, J=7.1 Hz, 2H), 1.28 (t, J=7.1 Hz, 3H).

The mixture of ethyl N-[2-[[4-(3-bromo-4-nitro-phenoxy)-3,5-dichlorophenyl]hydrazono]-2- cyano-acetyl]carbamate (2 g, 3.67 mmol) and Et3N (1.86 g, 18.34 mmol, 2.55 mL) in DMF (30 mL) was heated to 100°C and stirred for 18 h under N₂. The mixture was diluted with EA (200 mL) and washed with H₂O (100 mL x 3) and brine (100mL x 3). The organic layer was dried over MgSO₄, the solids were removed by filtration and the filtrate was concentrated under reduced pressure. The crude was purified by silica gel chromatography (eluent of 0~100% EA/PE then 0~10% MeOH/EA). 2-[4-(3-bromo- 4-nitro-phenoxy)-3,5-dichloro-phenyl]-3,5-dioxo-1,2,4-triazine-6-carbonitrile (1.3 g, 2.60 mmol, 61.74% yield) was obtained as yellow solid. ¹H NMR (400 MHz, DMSO-*d*₆) δ 8.10 (d, J=9.0 Hz, 1H), 7.89 (s, 2H), 7.61 (d, J=2.8 Hz, 1H), 7.10 (dd, J=2.8, 9.1 Hz, 1H).

To a solution of 2-[4-(3-bromo-4-nitro-phenoxy)-3,5-dichloro-phenyl]-3,5-dioxo-1,2,4-triazine-6- carbonitrile (12.1 g, 24.25 mmol) in AcOH (20 mL) was was added HCl (70 mL) and the mixture was stirred at 120°C for 16h. The mixture was concentrated to remove HCl and AcOH. H₂O (80 mL) was added and the mixture was cooled to 0°C. The precipitated solid was isolated by filtration and dried to afford 2-[4-(3-bromo-4-nitro-phenoxy)-3,5-dichloro-phenyl]-3,5-dioxo-1,2,4-triazine-6-carboxylic acid (10.06 g, 19.42 mmol, 80% yield) as yellow solid. ¹H NMR (400 MHz, DMSO-*d*₆) δ 13.19 - 12.22 (m, 1H), 8.09 (d, J=9.0 Hz, 1H), 7.91 (s, 2H), 7.63 (d, J=2.6 Hz, 1H), 7.10 (dd, J=2.7, 9.1 Hz, 1H).

The mixture of 2-[4-(3-bromo-4-nitro-phenoxy)-3,5-dichloro-phenyl]-3,5-dioxo-1,2,4-triazine-6- carboxylic acid (9.06 g, 17.49 mmol) and DPPA (7.22 g, 26.23 mmol) and Et₃N (7.08 g, 69.95 mmol) in t-BuOH (15 mL) was heated to 85°C for 16h under nitrogen atmosphere. The mixture was diluted with EA (30 mL) and washed with H₂O (10mL x 2). The organic layer was dried over MgSO₄ and concentrated to afford crude. The residue was purified by silica gel chromatography (eluent of 0~50% EA/PE). t-Butyl N-[2-[4-(3-bromo-4-nitro-phenoxy)-3,5-dichloro-phenyl]-3,5-dioxo-1,2,4-triazin-6-yl] carbamate (5.2 g, 8.83 mmol, 45.47% yield) was obtained as yellow solid. ¹H NMR (400 MHz, DMSO-*d*₆) δ 12.66 (br s, 1H), 9.18 (s, 1H), 8.10 (d, J=9.1 Hz, 1H), 7.98 (s, 2H), 7.61- 7.55 (m, 1H), 7.08 (dd, J=2.7, 9.1 Hz, 1H), 1.46 (s, 9H).

To a solution of *t*-butyl *N*-[2-[4-(3-bromo-4-nitro-phenoxy)-3,5-dichlorophenyl]-3,5-dioxo-1,2,4-tria-zin-6-yl]carbamate (1 g, 1.70 mmol) in THF (20 mL), MeOH (10 mL) and H₂O (5 mL) was added Fe (473.92 mg, 8.49 mmol) and NH₄Cl (453.95 mg, 8.49 mmol). The mixture was stirred at 80 °C for 2 h. The reaction mixture was diluted with EA (50 mL), filetred and the filtrate was concentrated under reduced pressure to give t-butyl N-[2-[4-(4-amino-3-bromo-phenoxy)-3,5-dichloro-phenyl]-3,5-dioxo-1,2,4-triazin-6-yl]carbamate (700 mg, 1.25 mmol, 74% yield) as a yellow solid. ¹H NMR (400 MHz, DMSO-*d₆*) *δ* 12.30 (br s, 1H), 9.15 (s,1H), 7.88(s, 2H), 6.87 (d, J=2.8 Hz, 1H), 6.79(d, J=8.8 Hz, 1H), 6.71 - 6.64 (m,1H), 5.10 (br s, 2H), 1.45 (s, 9H).

To a solution of *t*-butyl *N*-[2-[4-(4-amino-3-bromo-phenoxy)-3,5-dichlorophenyl]-3,5-dioxo-1,2,4-tr-iazin-6-yl]carbamate (700 mg, 1.25 mmol) in THF (20 mL) was added DIEA (485.35 mg, 3.76 mmol), and 4-methylpent-2-enoyl chloride (199.17 mg, 1.50 mmol, 11.69 uL) at 25 °C. The mixture was stirred at 25 °C for 3 hr. The reaction mixture was diluted with H₂O (50 mL) and extracted with EA (50 mL x 3). The combined organic layers were washed with brine (60 mL), dried over Na₂SO₄, filtered and concentrated under reduced pressure to give a residue. The residue was purified by flash silica gel chromatography (eluent 0~35% THF/PE) to give *t*-butyl*N*-[2-[4-[3-bromo-4-[[4-methylpent-2-enoyl]amino]phenoxy]-3,5-dichloro-phenyl]-3,5-dioxo-1,2,4-triazin-6-yl]carbamate (370 mg, 564.61 umol, 45.10% yield) as a yellow solid. ¹H NMR (400 MHz, DMSO-*d*₆) δ 12.65 (br s, 1H), 9.52 (s, 1H), 9.09(br s, 1H), 7.97 - 7.94 (m, 2H), 7.53(d, *J*=8.9 Hz, 1H), 7.23 (d, *J*=2.9Hz, 1H), 6.87 (s, 1H), 6.80 (dd,*J*=6.3, 15.4 Hz, 1H), 6.67 (s, 1H),6.58 (s, 1H), 6.18 (br d, *J=15.1* Hz,1H), 1.46 (s, 9H), 1.05 (d, *J*=6.8 Hz,6H).

To a solution of *t*-butyl *N*-[2-[4-[3-bromo-4-[[4-methylpent-2-enoyl]amino]phenoxy]-3,5-dichloro-phenyl]-3,5-dioxo-1,2,4-triazin-6-yl]carbamate (320 mg, 488.31 umol) in dioxane (2 mL) was added TEA (148.24 mg, 1.46 mmol, 203.90 uL), and palladium tri-t-butylphosphane (24.96 mg, 48.83 umol) at 25 °C. Then the mixture was stirred at 130 °C for 1.5 h. LCMS showed the starting material was consumed completely and desired mass was detected. The reaction mixture was diluted with H₂O (10 mL) and extracted with EA (10 mL x 3). The combined organic layers were washed with brine (20 mL), dried over Na₂SO₄, filtered and concentrated under reduced pressure to give a residue. The residue was purified by prep-HPLC [Column: Welch Xtimate C18 150 x 30mm x 5um; Mobile phase: from 50% CH₃CN in water (0.225%FA) to 80% CH₃CN in water (0.225%FA)] to give t-butyl -[2-[3,5-dichloro-4-[(4-isopropyl-2-oxo-1H-quinolin-6-yl)oxy]phenyl]-3,5-dioxo-1,2,4-triazin- 6-yl]carbamate (20 mg, 34.82 umol, ¹H NMR (400 MHz, DMSO-*d*₆) δ (br s, 1H), 11.66 (s, 1H), 9.15(br s, 1H), 7.94 (s, 2H), 7.35 (d,*J*=9.0 Hz, 1H), 7.23 (d, *J*=2.9 Hz,1H), 7.11 (dd, *J*=2.6, 9.0 Hz, 1H),6.39 (s, 1H), 3.20 (br d, *J*=6.4 Hz,1H), 1.46 (s, 9H), 1.21 (d, *J*=6.8 Hz, 6H)) as a yellow solid and t-butyl N-[2-[3,5-dichloro-4-[(3Z)-3-(2-methylpropylidene)-2-oxo-indolin-5-yl]oxy-phenyl]-3,5-dioxo-1,2,4-triazin-6-yl]carbamate (26 mg, 45.26 umol, ¹H NMR (400 MHz, DMSO-*d*₆) δ 12.63 (s, 1H), 10.45 (s, 1H), 9.17 (s, 1H), 7.90 (s, 2H), 7.25 (d, *J*=2.3 Hz, 1H), 6.79 (d, *J*=8.5 Hz, 1H), 6.68 (d, *J=*10.0 Hz, 1H), 6.55 (dd, *J*=2.5, 8.5Hz, 1H), 3.14 (qd, *J*=6.7, 16.5 Hz,1H), 1.45 (s, 9H), 1.14 (d, *J*=6.5 Hz, 6H) as a yellow solid.

To a solution of t-butyl N-[2-[3,5-dichloro-4-[(4-isopropyl-2-oxo-1H-quinolin-6-yl)oxy]phenyl]-3,5-dioxo-1,2,4-triazin-6-yl]carbamate (20 mg, 34.40 umol) in MeOH (1 mL) was added HCl/dioxane (4 M, 43.00 uL) at 15 °C. The mixture was stirred at 15 °C for 6 hr. The reaction mixture was concentrated in vacuo. The residue was purified by prep-HPLC [Column: Welch Xtimate C18 150 x 30mm x 5um; mobile phase: from 30% CH₃CN in water (0.04%NH₃H₂O+10mM NH₄HCO₃) to 60% CH₃CN in water(0.04%NH₃H₂O+10mM NH₄HCO₃)] to give 6-amino-2-[3,5-dichloro-4-[(4-isopropyl-2-oxo-1*H*-quinolin-6-yl)oxy]phenyl] -1,2,4-triazine-3,5-dione (1.95 mg, 4.07 umol, 11.82% yield, 99% purity) as a white solid. ¹H NMR (400 MHz, DMSO-*d*₆) δ 12.30 (br s, 1H), 11.65 (s, 1H), 7.95(s, 2H), 7.34 (d, *J*=8.9 Hz, 1H), 7.19(d, *J*=2.6 Hz, 1H), 7.11 (dd, *J*=2.7,8.9 Hz, 1H), 6.56 (br s, 2H), 6.39 (s,1H), 3.23 - 3.14 (m, 1H), 1.20 (d, *J*=6.8 Hz, 6H).

To a solution of *t*-butyl *N*-[2-[3,5-dichloro-4-[(3Z)-3-(2-methylpropylidene)-2-oxo-indolin-5-yl]oxy-phenyl]-3,5-dioxo-1,2,4-triazin-6-yl]carbamate (26 mg, 45.26 umol) in MeOH (1 mL) was added HCl/dioxane (4 M, 0.057 mL) at 15 °C. The mixture was stirred at 15 °C for 16 hr. The reaction mixture was concentrated in vacuo to give crude product, that was purified by prep-HPLC [Column: Welch Xtimate C18 150 x 30mm x 5um; mobile phase: from 10% CH₃CN in water(0.2%FA) to 50% CH₃CN in water (0.2%FA) to give 6-amino-2-[3,5-dichloro-4-[(3Z)-3-(2-methylpropylidene)-2-oxo-indolin-5-yl]oxyphenyl] -1,2,4-triazine-3,5-dione (4.64 mg, 9.73 umol, 99.44% purity) as a yellow solid. ¹H NMR (400 MHz, DMSO-*d*₆) δ 12.30 (br s, 1H), 10.44 (s, 1H), 7.91(s, 2H), 7.24 (d, *J*=2.5 Hz, 1H), 6.78(d, *J*=8.5 Hz, 1H), 6.68 (d, *J*=9.9Hz, 1H), 6.54 (d, *J*=2.6 Hz, 1H), 6.53 - 6.50 (m, 2H), 3.14 (td, *J*=6.5, 9.8 Hz, 1H), 1.14 (d, *J*=6.5 Hz, 6H).

### Example 81: Synthesis of compound 148

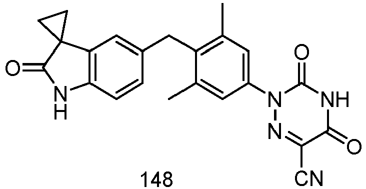

Anhydrous THF (20 mL) was added to a mixture of 5'-bromospiro[cyclopropane-1,3'-indolin]-2'-one (400 mg, 1.68 mmol) and NaH 60% (1.5 eq., 101 mg) at 0 °C, under N₂. The resulting reaction mixture was stirred at 25 °C for 1h. Then, the mixture was cooled to -78 °C and *t*-BuLi (2.2 eq., 2.17 mL) was added dropwise and the reaction mixture was stirred at -78 °C for 30 min. Then, a solution of 2,2,2-trifluoro-N-(4-formyl-3,5-dimethylphenyl)acetamide (1 eq., 412 mg) in anhydrous THF (5 mL) was added dropwise to the reaction mixture at -78 °C. The reaction mixture was then allowed to warm to rt for 2h and finally quenched with NH₄Cl (sat., aq., 4 mL) and poured in CH₂Cl₂ (100 mL). The organic phase was washed with water (3x), dried over MgSO₄, filtered and evaporated to dryness. The crude mixture was purified by flash chromatography on silica gel (0 to 50% EA in cyclohexane) to give 2,2,2-trifluoro-N-(4-(hydroxy(2'-oxospiro[cyclopropane-1,3'-indolin]-5'-yl)methyl)-3,5-dimethylphenyl)acetamide (269 mg, 40%) as a white solid. ¹H NMR (400 MHz, DMSO-*d*₆) δ 1.38-1.52 (m, 4H), 2.20 (s, 6H), 5.75 (d, *J =* 4.0 Hz, 1H), 6.06 (d, *J =* 4.0 Hz, 1H), 6.78 (s, 2H), 6.92 (s, 1H), 7.28 (s, 2H), 10.46 (s, 1H), 11.06 (br s, 1H) ppm.

Et₃SiH (0.64 mL) and TMSOTf (0.027 mL) were added dropwise to a solution of 2,2,2-trifluoro-N-(4-(hydroxy(2'-oxospiro[cyclopropane-1,3'-indolin]-5'-yl)methyl)-3,5-dimethylphenyl)acetamide (268 mg, 0.66 mmol) in anhydrous CH₂Cl₂ (30 mL) at 0 °C under N₂. The reaction was stirred at 0 °C for 1h, at which point the ice bath was removed and the reaction was stirred at 25 °C for 16h. The reaction mixture was quenched with sat. aq. NaHCO₃ (20 mL) and extracted with CH₂Cl₂ (3 x 40 mL). The combined organic layers were washed with brine (100 mL), dried over MgSO₄, filtered and evaporated to dryness to afford N-(3,5-dimethyl-4-((2'-oxospiro[cyclopropane-1,3'-indolin]-5'-yl)methyl)phenyl)-2,2,2-trifluoroacetamide (229 mg, 89%) as a white solid which was used without further purification. ¹H NMR (400 MHz, DMSO-*d*₆) δ 1.41-1.48 (m, 4H), 2.18 (s, 6H), 3.91 (s, 2H), 6.58 (d, *J* = 8.0 Hz, 1H), 6.72 (s, 1H), 6.75 (d, *J* = 8.0 Hz, 1H), 7.35 (s, 2H), 10.43 (s, 1H), 11.06 (br s, 1H) ppm.

NaOH (6 eq., 141 mg) was added to a solution of N-(3,5-dimethyl-4-((2'-oxospiro[cyclopropane-1,3'-indolin]-5'-yl)methyl)phenyl)-2,2,2-trifluoroacetamide (229 mg, 0.59 mmol) in CH₃OH (25 mL) andwater (2.5 mL) under N₂. The mixture was stirred at 60 °C for 23h. Then, the mixture was quenched with water (100 mL). The resulting solution was extracted with CH₂Cl₂ (3 x 100 mL). The combined organic layers were washed with brine (300 mL), dried over MgSO₄, filtered and evaporated to dryness to give 5'-(4-amino-2,6-dimethylbenzyl)spiro[cyclopropane-1,3'-indolin]-2'-one (147 mg, 85%) as a white solid which was used without further purification. ¹H NMR (400 MHz, DMSO-*d*₆) δ 1.40-1.46 (m, 4H), 2.02 (s, 6H), 3.76 (s, 2H), 4.71 (s, 2H), 6.26 (s, 2H), 6.61 (d, *J* = 8.0 Hz, 1H), 6.68 (s, 1H), 6.73 (d, *J* = 8.0 Hz, 1H), 10.39 (s, 1H) ppm.

*t*-Butyl nitrite (0.090 mL) andpyridine (0.12 mL) were added to a solution of 5'-(4-amino-2,6-dimethylbenzyl)spiro[cyclopropane-1,3'-indolin]-2'-one (1 eq., 147 mg, 0.503 mmol) and ethyl *N*-(2-cyanoacetyl)carbamate (118 mg) in anhydrous CH₃CN (15.5 mL) under N₂. The reaction mixture was stirred at 60 °C for 1.5h. After cooling to rt, NaOAc (6 eq., 248 mg) was added and the reaction mixture was heated at 80 °C for 3h. Additional NaOAc (82 mg) was added and heating was pursued for 64h. The reaction mixture was cooled to 0 °C and water (50 mL) was added. The mixture was stirred for 30 min at 0 °C and the resulting solution was extracted with EA (3 x 20 mL). The combined organic layers were dried over MgSO₄, filtered and evaporated to dryness. The crude mixture was purified by flash chromatography on silica gel (0 to 7.5% CH₃OH in CH₂Cl₂) to give 2-(3,5-dimethyl-4-((2'-oxospiro[cyclopropane-1,3'-indolin]-5'-yl)methyl)phenyl)-3,5-dioxo-2,3,4,5-tetrahydro-1,2,4-triazine-6-carbonitrile (143 mg, 69%) as a white solid. ¹H NMR (400 MHz, DMSO-*d*₆) δ 1.42-1.50 (m, 4H), 2.24 (s, 6H), 3.98 (s, 2H), 6.59 (d, *J=* 8.0 Hz, 1H), 6.76 (d, *J=* 8.0 Hz, 1H), 6.78 (s, 1H), 7.16 (s, 2H), 10.45 (s, 1H), 12.98 (br s, 1H).

### Example 82: Synthesis of compound 149

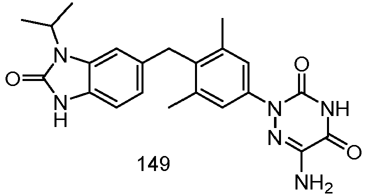

Anhydrous THF (6 mL) was added to a mixture of 6-bromo-1-isopropyl-1,3-dihydro-2H-benzo[d]imidazol-2-one (prepared according to Bioorganic & Medicinal Chemistry Letters 2005, 15(15), pages 3600-3603, 300 mg, 1.18 mmol) and NaH 60% (70.6 mg, 1.76 mmol) at 0 °C, under N₂. The resulting reaction mixture was stirred at rt for 1 h. Then, the mixture was cooled to -78 °C and *t*-BuLi (1.5 mL, 2.59 mmol, 1.7 M in pentane) was added dropwise and the reaction mixture was stirred at -78 °C for 1 h. Then, a solution of 4-bromo-2,6-dimethylbenzaldehyde (250.6 mg, 1.18 mmol) in anhydrous THF (3.8 mL) was added dropwise to the reaction mixture at -78 °C. The reaction mixture warmed to rt for 1h and then quenched with sat. aq. NH₄Cl and extracted with CH₂Cl₂ (3x). The combined organic phases were washed with water (3x), dried over Na₂SO₄, filtered and evaporated to dryness. The crude mixture was purified by flash chromatography on silica gel (0 to 60% AcOEt in cyclohexane) to give 6-((4-bromo-2,6-dimethylphenyl)(hydroxy)methyl)-1-isopropyl-1,3-dihydro-2*H*-benzo[d]imidazol-2-one (277 mg, 61%) as a white solid. ¹H NMR (400 MHz, DMSO-*d*₆) δ 1.36-1.43 (m, 6H), 2.22 (s, 6H), 4.53 (p, *J =* 6.9 Hz, 1H), 5.85 (d, *J* = 3.3 Hz, 1H), 6.12 (d, *J* = 3.0 Hz, 1H), 6.56 (d, *J* = 8.0 Hz, 1H), 6.84 (d, *J* = 8.3 Hz, 1H), 7.19 (s, 1H), 7.21 (s, 2H), 10.66 (s, 1H) ppm.

Et₃SiH (0.69 mL, 4.27 mmol) and TMSOTf (28.8 µL, 0.16 mmol) were added dropwise to a solution of 6-((4-bromo-2,6-dimethylphenyl)(hydroxy)methyl)-1-isopropyl-1,3-dihydro-2*H*-benzo[d]imidazol-2-one (277 mg, 0.71 mmol) in anhydrous CH₂Cl₂ (32.2 mL) at 0 °C under N₂. The reaction was stirred at 0 °C for 30 min, at which point the ice bath was removed and the reaction was stirred at rt for 2 h. The reaction mixture was quenched with sat. aq. NaHCO₃ and extracted with CH₂Cl₂ (3x). The combined organic layers were washed with brine, dried over Na₂SO₄, filtered and evaporated to dryness providing 6-(4-bromo-2,6-dimethylbenzyl)-1-isopropyl-1,3-dihydro-2*H*-_{b}enzo[d]imidazol-2-one (242 mg, 91%) as a white solid which was used in the next step without further purification.

A solution of 6-(4-bromo-2,6-dimethylbenzyl)-1-isopropyl-1,3-dihydro-2H-benzo[d]imidazol-2-one (195 mg, 0.52 mmol), 6-amino-4-[(benzyloxy)methyl]-2,3,4,5-tetrahydro-1,2,4-triazine-3,5-dione (142.6 mg, 0.57 mmol), K₂CO₃ (288.8 mg, 2.09 mmol), CuBr (74.9 mg, 0.52 mmol) and *trans-N,N-*dimethylcyclohexane-1,2-diamine (74.3 mg, 0.52 mmol) in anhydrous DMSO (3.9 mL) under N₂ was stirred at 120 °C for 4 h. After cooling to rt, the reaction mixture was diluted in water and was filtered. The precipitate was washed with water and was dissolved in CH₂Cl₂. The solution was dried with Na₂SO₄, filtered and evaporated to dryness. The crude mixture was purified by flash chromatography on silica gel (0% to 5% CH₃OH in CH₂Cl₂) to give 6-amino-4-((benzyloxy)methyl)-2-(4-((3-isopropyl-2-oxo-2,3-dihydro-1*H*-benzo[d]imidazol-5-yl)methyl)-3,5-dimethylphenyl)-1,2,4-triazine-3,5(2*H*,4*H*)-dione (81 mg, 29%) as a white solid. ¹H NMR (400 MHz, DMSO-*d*₆) δ 1.39 (d, *J =* 6.9 Hz, 6H), 2.25 (s, 6H), 4.06 (s, 2H), 4.51 (p, *J =* 6.9 Hz, 1H), 4.66 (s, 2H), 5.41 (s, 2H), 6.38 (s, 2H), 6.46 (d, *J =* 7.8 Hz, 1H), 6.83 (d, *J =* 7.9 Hz, 1H), 7.00 (s, 1H), 7.19 (s, 2H), 7.26-7.35 (m, 5H), 10.63 (s, 1H) ppm.

BBr₃ (1.2 mL, 1.2 mmol, 1 M in CH₂Cl₂) was added dropwise to a solution of give 6-amino-4-((benzyloxy)methyl)-2-(4-((3-isopropyl-2-oxo-2,3-dihydro-1*H-*benzo[d]imidazol-5-yl)methyl)-3,5-dimethylphenyl)-1,2,4-triazine-3,5(2*H*,4*H*)-dione (81 mg, 0.15 mmol) in CH₂Cl₂ (50 mL) at 0 °C under N₂. The reaction mixture was stirred at 0 °C for 2 h. Afterwards, cold CH₃OH was added dropwise at 0 °C and the reaction mixture slowly warmed to rt, then was evaporated to dryness and the crude mixture was purified by chromatography on silica gel (0% to 10% CH₃OH in CH₂Cl₂). The resulting product was purified by reverse phase flash chromatography (5 to 100% CH₃CN in water (0.1% TFA)) to give 6-amino-2-(4-((3-isopropyl-2-oxo-2,3-dihydro-1H-benzo[d]imidazol-5-yl)methyl)-3,5-dimethylphenyl)-1,2,4-triazine-3,5(2*H*,4*H*)-dione (26 mg, 41%) as a white solid. ¹H NMR (400 MHz, DMSO-*d*₆) δ 1.39 (d, *J* = 6.9 Hz, 6H), 2.23 (s, 6H), 4.04 (s, 2H), 4.51 (hept, *J =* 7.0 Hz, 1H), 6.30 (s, 2H), 6.44 (d, *J* = 8.0 Hz, 1H), 6.83 (d, *J* = 7.9 Hz, 1H), 7.01 (s, 1H), 7.20 (s, 2H), 10.67 (s, 1H), 12.07 (s, 1H) ppm.

### Example 83: Synthesis of compound 150

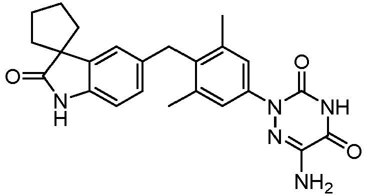

6-Amino-2-(3,5-dimethyl-4-((2'-oxospiro[cyclopentane-1,3'-indolin]-5'-yl)methyl)phenyl)-1,2,4-triazine-3,5(2*H*,4*H*)-dione was prepared using a method analogous to that described for compound 149 where 5'-bromospiro[cyclopentane-1,3'-indolin]-2'-one was employed in the first step instead of 6-bromo-1-isopropyl-1,3-dihydro-2*H-*benzo[d]imidazol-2-one. ¹H NMR (400 MHz, DMSO-*d*₆) δ 1.65-1.73 (m, 2H), 1.84-1.98 (m, 6H), 2.21 (s, 6H), 3.96 (s, 2H), 6.29 (s, 2H), 6.59 (dd *J =* 7.9 Hz, 1H) ; 6.69 (d, *J =* 8.0 Hz, 1H), 7.03 (s, 1H), 7.19 (s, 2H), 10.17 (s, 1H), 12.07 (s, 1H).

### Example 84: Synthesis of compound 151

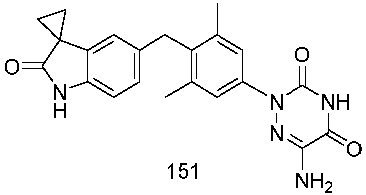

6-Amino-2-(3,5-dimethyl-4-((2'-oxospiro[cyclopropane-1,3'-indolin]-5'-yl)methyl)phenyl)-1,2,4-triazine-3,5(2*H*,4*H*)-dione was prepared using a method analogous to that described for compound 149 where 5'-bromospiro[cyclopropane-1,3'-indolin]-2'-one was employed in the first step instead of 6-bromo-1-isopropyl-1,3-dihydro-2H-benzo[d]imidazol-2-one. ¹H NMR (400 MHz, DMSO-*d*₆) δ 1.42-1.49 (m, 4H), 2.20 (s, 6H), 3.94 (s, 2H), 6.30 (s, 2H), 6.58 (dd, *J =* 8.0 Hz, 1.9 Hz, 1H), 6.76 (dd, *J =* 5.2 Hz, 3.3 Hz, 2H), 7.19 (s, 2H), 10.44 (s, 1H), 12.08 (br s, 1H) ppm.

### Example 85: Synthesis of compound 152

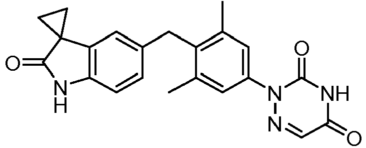

KOH (10 eq., 140 mg, 2.49 mmol) was added to a solution of 2-(3,5-dimethyl-4-((2'-oxospiro[cyclopropane-1,3'-indolin]-5'-yl)methyl)phenyl)-3,5-dioxo-2,3,4,5-tetrahydro-1,2,4-triazine-6-carbonitrile (103 mg, 0.25 mmol) in water (1.3 mL) and EtOH (1.3 mL) under N₂. The reaction mixture was stirred at 80 °C for 2 h. The reaction mixture was evaporated to dryness, water was added and aqueous 1N HCl solution was added until acidic pH. Then, the aqueous layer was extracted with EtOAc (3x). The combined organic layers were dried with Na₂SO₄, filtered and evaporated to dryness providing 2-(3,5-dimethyl-4-((2'-oxospiro[cyclopropane-1,3'-indolin]-5'-yl)methyl)phenyl)-3,5-dioxo-2,3,4,5-tetrahydro-1,2,4-triazine-6-carboxylic acid (102 mg, 95%) as a brown oil which was used without further purification.

A solution of 2-(3,5-dimethyl-4-((2'-oxospiro[cyclopropane-1,3'-indolin]-5'-yl)methyl)phenyl)-3,5-dioxo-2,3,4,5-tetrahydro-1,2,4-triazine-6-carboxylic acid (1 eq., 69 mg, 0.16 mmol) in mercaptoacetic acid (25 eq., 276 µL, 4 mmol) was stirred rapidly and heated to 100 °C for 3 h. After cooling to rt, the reaction mixture was diluted in water, filtered and washed with water. The precipitate was dissolved in MeOH and evaporated to dryness. The crude mixture was purified by flash chromatography on silica gel (0% to 5% MeOH in DCM), triturated with MeCN (2x) and co-evaporated with EtOH (2x) to give 2-(3,5-dimethyl-4-((2'-oxospiro[cyclopropane-1,3'-indolin]-5'-yl)methyl)phenyl)-1,2,4-triazine-3,5(2*H*,4*H*)-dione (11 mg, 18%) as a yellow solid. ¹H-NMR (DMSO-*d*₆, 400 MHz) δ 1.43-1.48 (m, 4H), 2.23 (s, 6H), 3.96 (s, 2H), 6.59 (d, *J* = 7.7 Hz, 1H), 6.77 (d, *J* = 8.0 Hz, 2H), 7.17 (s, 2H), 7.61 (s, 1H), 10.44 (s, 1H), 12.30 (br s, 1H) ppm.

### Example 86: Synthesis of compound 153

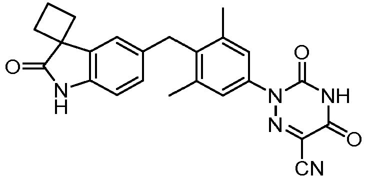

2-(3,5-Dimethyl-4-((2'-oxospiro[cyclobutane-1,3'-indolin]-5'-yl)methyl)phenyl)-3,5-dioxo-2,3,4,5-tetrahydro-1,2,4-triazine-6-carbonitrile was prepared using a method analogous to that described for the preparation of 2-(3,5-dimethyl-4-((2'-oxospiro[cyclopropane-1,3'-indolin]-5'-yl)methyl)phenyl)-3,5-dioxo-2,3,4,5-tetrahydro-1,2,4-triazine-6-carbonitrile, where 5'-bromospiro[cyclobutane-1,3'-indolin]-2'-one was used instead of 5'-bromospiro[cyclopropane-1,3'-indolin]-2'-one. ¹H-NMR (DMSO-*d*₆, 400 MHz) δ 2.11-2.27 (m, 10H), 2.37-2.43 (m, 2H), 4.04 (s, 2H), 6.60 (dd, J = 8.0 Hz, 2.0 Hz, 1H), 6.66 (d, J = 7.9 Hz, 1H), 7.18 (s, 2H), 7.37 (s, 1H), 10.13 (s, 1H), 12.99 (br s, 1H) ppm.

### Example 87: Preparation of compound 154

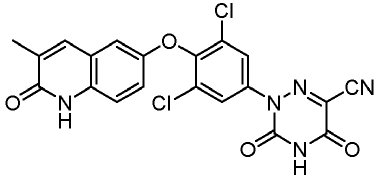

To a solution of 6-bromo-3-methyl-1H-quinolin-2-one (3 g, 12.60 mmol) in THF (50 mL) was added NaH (2.02 g, 50.40 mmol, 60% purity) at 20 °C. The mixture was stirred at 40°C for 0.5 hr. Then SEM-Cl (4.20 g, 25.20 mmol, 4.46 mL) was added The mixture was stirred at 40 °C for 16 h. The reaction mixture was quenched by addition of NH₄Cl (sat., aq., 20 mL) at 10 °C, and then diluted with H₂O (60 mL) and extracted with EA (60 mL x 3). The combined organic layers were washed with brine (100 mL), dried over Na₂SO₄, filtered and concentrated under reduced pressure to give a residue, which was purified by flash silica gel chromatography (EA/PE = 0~10%) to give 6-bromo-3-methyl-1-(2-trimethylsilylethoxymethyl)quinolin-2-one (3.25 g, 8.82 mmol, 70% yield) as a yellow oil. ¹H NMR(400MHz, CDCl₃) δ 7.64 (d,*J*=2.1 Hz, 1H), 7.61 - 7.55 (m, 1H),7.52 - 7.44 (m, 2H), 5.79 (s, 2H), 3.77 - 3.65 (m, 2H), 2.29 (d, *J*=0.9Hz, 3H), 1.03 - 0.89 (m, 2H), 0.04 -0.05 (m, 9H).

A mixture of 6-bromo-3-methyl-1-(2-trimethylsilylethoxymethyl)quinolin-2-one (3.25 g, 8.82 mmol, 1 eq), Pin₂B₂ (3.36 g, 13.24 mmol, 1.5 eq), KOAc (2.60 g, 26.47 mmol), PdCl₂(dppf) (720.55 mg, 882.34 umol) in dioxane (60 mL) was degassed and purged with N₂ for 3 times, and then the mixture was stirred at 100 °C for 16 hr under N₂ atmosphere. The reaction mixture was diluted with H₂O (100 mL) and extracted with EA (100 mL x 3). The combined organic layers were washed with brine (100 mL x 2), dried over Na₂SO₄, the solids were removed by filtration and the filtrate was concentrated under reduced pressure to give a residue. The residue was purified by silica gel chromatography (EA/PE = 0~10%) to give 3-methyl-6-(4,4,5,5-tetramethyl-1,3,2-dioxaborolan-2-yl)-1-(2-trimethylsilylethoxymethyl)quinolin-2-one (4.1 g, crude) as a white solid. ¹H NMR (400MHz, CDCl₃) δ 7.99 (s, 1H),7.93 (dd, *J=*1.1, 8.4 Hz, 1H), 7.64 -7.54 (m, 2H), 5.83 (s, 2H), 3.79 -3.66 (m, 2H), 2.29 (s, 3H), 1.41 (s,12H), 1.02 - 0.89 (m, 2H), 0.00 (s,9H).

To a solution of 3-methyl-6-(4,4,5,5-tetramethyl-1,3,2-dioxaborolan-2-yl)-1-(2-trimethylsilylethoxymethyl)quinolin-2-one (4.1 g, 9.87 mmol) in acetone (60 mL) and H₂O (30 mL) was added NaIO₄ (21.11 g, 98.70 mmol, 5.47 mL) and NH₄OAc (7.61 g, 98.70 mmol) at 20 °C. The mixture was stirred at 20°C for 16 hr. The reaction mixture was diluted with H₂O (60 mL) and extracted with EA (60 mL x 3). The combined organic layers were washed with brine (100 mL), dried over anhydrous Na₂SO₄, the solids were removed under reduced pressure and the filtrate was concentrated under reduced pressure to give [3-methyl-2-oxo-1-(2-trimethylsilylethoxymethyl)-6-quinolyl]boronic acid (2.7 g, 7.38 mmol, 75% yield, 91 % purity) as a pink solid. ¹H NMR (400MHz, DMSO-*d*₆) δ 8.20 (s, 2H), 8.09 (s, 1H), 8.03 -7.95 (m, 1H), 7.90 - 7.81 (m, 1H),7.55 (d, *J*=8.5 Hz, 1H), 5.79 (s, 2H),3.68 (t, *J*=7.9 Hz, 2H), 2.26 - 2.16(m, 3H), 0.94 (t, *J*=7.9 Hz, 2H), 0.05 -0.05 (m, 9H).

To a solution of [3-methyl-2-oxo-1-(2-trimethylsilylethoxymethyl)-6-quinolyl]boronic acid (500 mg, 1.50 mmol) in DMF (20 mL) was added 4Å MS (5 g, 1.50 mmol), *N'*-(3,5-dichloro-4-hydroxy-phenyl)-*N,N*-dimethyl-formamidine (384.69 mg, 1.65 mmol), bis(trifluoromethylsulfonyloxy)copper (542.64 mg, 1.50 mmol) and triethylamine (759.08 mg, 7.50 mmol) at 15 °C. The mixture was stirred at 15 °C for 16 hr under O₂. The reaction mixture was diluted with H₂O (50 mL) and extracted with EA (50 mL x 2). The combined organic layers were washed with brine (50 mL x 2), dried over Na₂SO₄, the solids were removed by filtration and the filtrate was concentrated under reduced pressure to give a residue. The residue was purified by flash silica gel chromatography (0~35% EA/PE = 0~35%) to give *N'*-[3,5-dichloro-4-[[3-methyl-2-oxo-1- (2-trimethylsilylethoxymethyl)-6-quinolyl]oxy]phenyl]-N,N-dimethylformamidine (250 mg, 480.29 umol, 32% yield) as a green solid. ¹H NMR (400MHz, CDCl₃) δ 7.56 (s, 1H),7.53 (d, *J*=9.1 Hz, 1H), 7.43 (s, 1H),7.11 (dd, *J*=2.8, 9.2 Hz, 1H), 7.01(s, 2H), 6.81 (d, *J*=2.9 Hz, 1H), 5.77(s, 2H), 3.73 - 3.66 (m, 2H), 3.07 (br d, *J =*11.0 Hz, 6H), 2.23 (d, *J*=0.9Hz, 3H), 0.98 - 0.92 (m, 2H), -0.01-0.04 (m, 8H), -0.01-0.04 (m, 1H).

To a solution of *N*-[3,5-dichloro-4-[[3-methyl-2-oxo-1-(2-trimethylsilylethoxymethyl)-6-quinolyl]oxy] phenyl]-*N,N*-dimethyl-formamidine (658 mg, 1.26 mmol) in isopropanol (20 mL) was added NH₂NH₂.H₂O (630.76 mg, 12.60 mmol) at 20 °C. The mixture was stirred at 80 °C for 12 hr under N₂. The reaction mixture was diluted with H₂O (50 mL) and extracted with EA (30 mL x 2). The aqueous phase was adjusted to pH=7 with 1M aq. HCl. The combined organic layers were washed with brine (20 mL), dried over Na₂SO₄, filtered and concentrated under reduced pressure to give a residue. The residue was purified by silica gel chromatography (EA/PE = 0~30%) to give 6-(4-amino-2,6-dichloro-phenoxy)-3-methyl-1-(2-trimethylsilylethoxymethyl) quinolin-2-one (387 mg, 831.46 umol, 66% yield) as a white solid. ¹H NMR (400MHz, DMSO-*d*₆) δ 7.77 (s, 1H), 7.48 (d, *J*=9.3 Hz, 1H),7.10 (dd, *J*=2.9, 9.3 Hz, 1H), 6.94(d, *J*=2.9 Hz, 1H), 6.73 (s, 2H), 5.67(s, 4H), 3.60 - 3.54 (m, 2H), 2.08 (d,*J*=0.6 Hz, 3H), 0.89 - 0.81 (m, 2H),-0.04 - -0.15 (m, 9H).

To a mixture of 6-(4-amino-2,6-dichloro-phenoxy)-3-methyl-1-(2-trimethylsilylethoxymethyl) quinolin-2-one (387 mg, 665.17 umol, 80% purity) in AcOH (5 mL) was added HCl (12 M, 0.554 mL). Then dropwise a solution of NaNO₂ (137.69 mg, 2.00 mmol) in H₂O (5 mL) while maintaining the temperature below 0 °C. After the addition was complete, the reaction mixture was stirred for 0.5 h. A mixture of ethyl N-(2-cyanoacetyl)carbamate (207.72 mg, 1.33 mmol) in pyridine (2.5 mL) was added drop-wise to the resulting diazonium salt solution below 0 °C and stirred for an additional 2h. The reaction mixture was diluted with H₂O (50 mL), then extracted with EA (50 mL x 3). The combined organic layers were washed with brine (60 mL), dried over anhydrous Na₂SO₄, the solids were removed by filtration and the filtrate was concentrated under reduced pressure. The residue was purified by silica gel chromatography (EA in PE = 0~40%) to give ethyl N-[2-cyano-2-[[3,5-dichloro-4-[[3-methyl-2-oxo-1-(2-trimethylsilylethoxymethyl)-6-quinolyl]oxy]phenyl]hydrazono]acetyl]carbamate (315 mg, 497.97 umol, 74.8% yield) as a yellow solid. ¹H NMR (400 MHz, DMSO-*d*₆) δ 10.93 (br s, 1H), 10.89 (s, 1H), 8.07(s, 2H), 7.77 (s, 1H), 7.51 (d, *J* = 9.3Hz, 1H), 7.17 (dd, *J=3.0,* 9.3 Hz,1H), 7.02 (d, *J=3.0* Hz, 1H), 5.69 (s,2H), 4.24 - 4.18 (m, 2H), 3.58 (t,*J*=7.9 Hz, 2H), 2.08 (d, *J* = 0.8 Hz,3H), 1.25 - 1.19 (m, 3H), 0.86 (t, *J*=7.9 Hz, 2H), -0.05 - -0.12 (m, 9H).

*N*-[2-cyano-2-[[3,5-dichloro-4-[[3-methyl-2-oxo-1-(2-trimethylsilylethoxymethyl)-6-quinolyl]oxy]phenyl]hydrazono]acetyl]carbamate (315 mg, 497.97 umol) in AcOH (10 mL) was added NaOAc (204.25 mg, 2.49 mmol) . The reaction mixture was concentrated in vacuo, then quenched by addition of sat. aq. NaHCO₃ (15 mL), then diluted with H₂O (25 mL) and extracted with EA (25 mL x 3). The combined organic layers were washed with brine (50 mL), dried over Na₂SO₄, filtered and concentrated under reduced pressure to give 2-[3,5-dichloro-4-[[3-methyl-2-oxo-1-(2-trimethylsilylethoxymethyl)-6-quinolyl]oxy]phenyl]-3,5-dioxo-1,2,4-triazine-6-carbonitrile (300 mg, 406.48 umol, 81.6% yield, 79% purity) as an orange solid.

A mixture of 2-[3,5-dichloro-4-[[3-methyl-2-oxo-1-(2-trimethylsilylethoxymethyl)-6-quinolyl]oxy]phenyl]-3,5-dioxo-1,2,4-triazine-6-carbonitrile (265 mg, 359.06 umol, 79.5% purity) , TBAF (1 M, 10.77 mL) in THF (10 mL) was degassed and purged with N₂ three times, then the mixture was stirred at 90°C for 16 h under N₂. The reaction mixture was quenched by NH₄Cl (sat., aq., 20 mL) at 15 °C, and then diluted with H₂O (10 mL) and extracted with EA (20 mL x 3). The combined organic layers were washed with sat. NH₄Cl (30 mL), dried over Na₂SO₄, the solids were removed by filtration and the filtrate was concentrated under reduced pressure. The residue was purified by prep-HPLC [Column: Welch Xtimate C18 150 x 30mm x 5um; mobile phase: from 48% CH₃CN in water (0.225% FA) to 78% CH₃CN in water (0.225% FA)] to give 2-[3,5-dichloro-4-[(3-methyl-2-oxo-1H-quinolin-6-yl)oxy]phenyl]-3,5-dioxo-1,2,4-triazine-6-carbonitrile (60.05 mg, 128.59 umol, 35.8% yield, 98% purity) as a light yellow solid. ¹H NMR (400 MHz, DMSO-*d*₆) δ 13.28 (br s, 1H), 11.75 (s, 1H), 7.82(s, 2H), 7.76 (s, 1H), 7.31 (d, *J*=9.0Hz, 1H), 7.17 (dd, *J*=2.8, 8.9 Hz,1H), 7.00 (d, *J*=2.8 Hz, 1H), 2.05 (s,3H).

### Example 88: Synthesis of compound 156

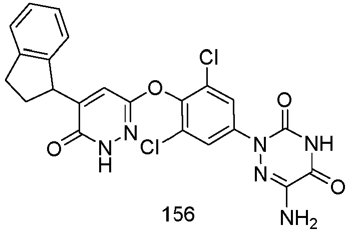

A 40 mL vial was charged with 2,2,6,6-tetramethylpiperidine (10.3 g, 73.2 mmol) in THF (100 mL) under nitrogen. n-butyllithium (26.8 mL, 67.1 mmol) was added dropwise at - 75 °C. The reaction was warmed to 0 °C and stirred for 30 min. 3,6-dichloropyridazine (5 g, 33.6 mmol) in THF (100 mL) was added dropwise at -75 °C. The reaction was stirred for 30 min. 2,3-dihydro-1*H*-inden-1-one (5.32 g, 40.3 mmol) in THF (50 mL) was added dropwise at -75 °C. The reaction was stirred for 90 min at - 75 °C. The reaction was stirred for 90 min at -75 °C. The resulting solution was quenched with NH₄Cl (sat., aq., 100 mL). The resulting solution was extracted with EA (3 x 100 mL) and the organic layers were combined, washed with brine (2 x 100 mL), dried over anhydrous sodium sulfate, the solids were removed by filtration and the filtrate was concentrated under reduced pressure. The residue was chromatographed on a silica gel column with EA/PE (1/4) to provide 2.28 g (yield 12%) of 1-(3,6-dichloropyridazin-4-yl)-2,3-dihydroinden-1-ol as a yellow solid. ¹H NMR (400 MHz, DMSO-*d*₆) δ 8.17 (s, 1H), 7.28 - 7.37 (m, 2H), 7.14 - 7.19 (m, 1H), 6.41 (m, 1H), 3.21 - 3.32 (m, 1H), 3.02 - 3.11 (m, 1H), 2.70 - 2.81 (m, 1H), 2.19 - 2.27 (m, 1H). LCMS (ESI, m/z): 281 [M+H]⁺.

A 40 mL vial was charged with 1-(3,6-dichloropyridazin-4-yl)-2,3-dihydroinden-1-ol (800 mg, 2.85 mmol), 4-methylbenzenesulfonic acid (245 mg, 1.42 mmol), toluene (10 mL). The reaction was stirred overnight at 60 °C. The resulting solution was quenched with water (20 mL). The resulting solution was extracted with EA (3 x 20 mL) and the organic layers were combined, washed with brine (2 x 20 mL), dried over anhydrous sodium sulfate, filtered and concentrated under reduced pressure. The residue was chromatographed on a silica gel column with EA/PE (1/9) to provide 520 mg (yield 50%) of 3,6-dichloro-4-(3H-inden-1-yl)pyridazine as a yellow solid. ¹H NMR (300 MHz, CDCl₃) δ 7.59 - 7.62 (m, 2H), 733 - 7.40 (m, 2H), 723 - 7.26 (m, 1H), 6.92 (t, *J =* 2.1 Hz, 1H), 3.68 (br, 2H). LCMS (ESI, *m*/*z):263* [M+H]⁺.

A 100 mL round-bottom flask was charged with 3,6-dichloro-4-(3H-inden-1-yl)pyridazine (800 mg, 0.380 mmol), platinumoxidehydrate (400 mg), EA (5 mL), ethanol (5 mL). The content of the flask was placed under an atmosphere of hydrogen. The reaction was stirred overnight at rt. The solids were filtered out. The filtrate was concentrated under reduced pressure. The residue was chromatographed on a silica gel column with EA/PE (12/88) to provide 165 mg (yield 19%) of 3,6-dichloro-4-(2,3-dihydro-1H-inden-1-yl)pyridazine as a white solid. ¹H NMR (300 MHz, CDCl₃) δ 7.31 - 7.40 (m, 3H), 7.08 (d, *J* = 7.5 Hz, 1H), 7.01 (s, 1H), 4.74 (t, *J =* 7.5 Hz, 1H), 3.05 (t, *J =* 7.5 Hz, 2H), 2.74 - 2.86 (m, 1H), 1.94 - 2.06 (m, 1H). LCMS (ESI, *m*/*z):* 265 [M+H]⁺.

A 40 mL vial was charged with 3,6-dichloro-4-(2,3-dihydro-1H-inden-1-yl)pyridazine (200 mg, 0.754 mmol), *t*-butyl *N*-[2-(3,5-dichloro-4-hydroxyphenyl)-3,5-dioxo-4*H*-1,2,4-triazin-6-yl]carbamate (382 mg, 0.981 mmol), potassium carbonate (313 mg, 2.26 mmol), CuI (57.5 mg, 0.302 mmol), DMSO (10 mL) under nitrogen. The reaction was stirred for 16 h at 110 °C. The reaction was quenched by water (10 mL). The resulting solution was extracted with ethyl acetate (3 x 20 mL) and the organic layers were combined, washed with brine (2 x 10 mL), dried over anhydrous sodium sulfate, filtered and concentrated under reduced pressure. The residue was chromatographed on a silica gel column with ethyl acetate/petroleum ether (1/1) to provide 120 mg (yield 21%) of 6-amino-2-(3,5-dichloro-4-[[6-chloro-5-(2,3-dihydro-1*H*-inden-1-yl)pyridazin-3-yl]oxy]phenyl)-4*H*-1,2,4-triazine-3,5-dione as a brown solid. LCMS (ESI, *m*/*z*): 517 [M+H]⁺.

A 40 mL vial was charged with 6-amino-2-(3,5-dichloro-4-[[6-chloro-5-(2,3-dihydro-1H-inden-1-yl)pyridazin-3-yl]oxy]phenyl)-4H-1,2,4-triazine-3,5-dione (120 mg, 0.232 mmol, 1.00 equiv), sodium acetate (114 mg, 1.39 mmol), acetic acid (5 mL) under nitrogen. The reaction was stirred overnight at 100 °C. The reaction was poured into water (5 mL). The solids were collected by filtration to provide the crude product. The crude product was purified by preparative HPLC using the following gradient conditions: Column: XBridge Prep OBD C18, 19 x 250 mm, 5um; mobile phase A:Water(0.05%TFA ), mobile phase B: CH₃CN ; Flow rate:25 mL/min; Gradient:48 B to 68 B in 7 min; 220 nm; RT1:5.38; Purification resulted in 26.6 mg (yield 31%) of 6-amino-2-(3,5-dichloro-4-[[5-(2,3-dihydro-1*H*-inden-1-yl)-6-oxo-1H-pyridazin-3-yl]oxy]phenyl)-4H-1,2,4-triazine-3,5-dione as a light yellow solid. ¹H NMR (300 MHz, DMSO-*d*₆) δ 12.27 - 12.36 (m, 2H), 7.84 (s, 2H), 7.33 (d, *J =* 6.3 Hz, 1H), 7.16 - 7.26 (m, 3H), 7.03 (s, 1H), 6.53 (s, 2H), 4.55 (t, *J =* 7.2 Hz, 1H), 2.88 - 3.09 (m, 2H), 2.42 - 2.45 (m, 1H), 2.08 - 2.18 (m, 1H). LCMS (ESI, m/z): 499 [M+H]⁺.

### Example 89: Synthesis of compound 157

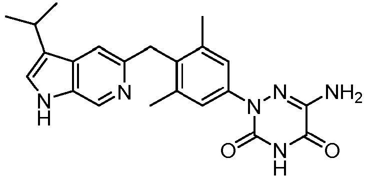

To a solution of 5-bromo-3-isopropyl-1-(*p*-tolylsulfonyl)pyrrolo[2,3-c]pyridine (1.9 g, 4.83 mmol) in MeOH (20 mL) and THF (5 mL) was added Et₃N (1.47 g, 14.49 mmol, 2.02 mL, 3 *eq*) and Pd(dppf)Cl₂ (706.97 mg, 0.966 mmol), and the mixture was stirred at 80 °C for 12 h under CO (50 psi), then diluted with H₂O (100 mL) and extracted with EA (100 mL x 3). The combined organic layers were washed with brine (100 mL), dried over anhydrous Na₂SO₄, the solids were removed by filtration and the filtrate was concentrated under reduced pressure. The crude was purified by silica gel chromatography (EA in PE 0~30%) to give methyl 3-isopropyl-1-(*p*-tolylsulfonyl)pyrrolo[2,3-c] pyridine-5-carboxylate(1.61 g,4.29 mmol, 88.77% yield, 99.2% purity) as a white solid. ¹H NMR (400MHz, CDCl₃) δ 9.34 (d,*J*=0.6 Hz, 1H), 8.38 (d, *J*=0.6 Hz,1H), 7.80 (d, *J*=8.4Hz, 2H), 7.49 (d,*J*=0.8 Hz, 1H), 7.28 - 7.23 (m, 3H),4.02 (s, 3H), 3.14 (spt, *J*=6.8 Hz, 1H), 2.37 (s, 3H), 1.35 (d, *J*=6.9 Hz,6H).

To a solution of methyl 3-isopropyl-1-(p-tolylsulfonyl)pyrrolo[2,3-c]pyridine-5-carboxyl-ate (1.0 g, 2.66 mmol) in THF (30 mL) was added DIBAL-H (1 M, 7.99 mL) dropwise at -65 °C. After the addition, the resulting mixture was stirred at -65 °C for 0.5 hr under N₂. The reaction mixture was quenched with methanol (10 mL) and citric acid (sat., aq., 10 mL). The mixture was diluted with H₂O (100 mL) and extracted with EA (50 mL x 3). The combined organic layers were washed with brine (100 mL), dried over anhydrous Na₂SO₄, filtered and concentrated under reduced pressure to give a residue, which was purified by silica gel chromatography (eluent of 0~15% EA/PE) to give 3-isopropyl-1-(*p*-tolylsulfonyl)pyrrolo[2,3-c]pyridine-5-carbaldehyde (807 mg, 2.28 mmol, 85.61% yield, 96.694% purity) as a white solid. ¹H NMR (400MHz, CDCl₃) δ 10.18 (s,1H),9.42 (d, *J*=0.8 Hz, 1H), 8.23 (d, *J =*0.9 Hz, 1H), 7.85 (d, *J*=8.4 Hz,2H), 7.53 (d, *J=0.8* Hz, 1H), 7.31 (d, *J*=8.3 Hz, 2H), 3.16 (td, *J*=6.8, 13.4Hz, 1H), 2.40 (s, 3H), 1.37 (d, *J*=6.9Hz, 6H).

To a solution of 5-bromo-2-iodo-1,3-dimethyl-benzene (544.88 mg, 1.75 mmol) in THF (20 mL) was added dropwise *n*-BuLi (2.5 M, 0.701 mL) at -70 °C over 30 min. After addition, the mixture was stirred at -70 °C for 1 h, then 3-isopropyl-1-(p-tolylsulfonyl)pyrrolo[2,3-*c*]pyridine-5-carbaldehyde (200 mg, 584.09 umol) in THF (2 mL) was added dropwise at -70 °C. The resulting mixture was stirred at -70 °C for 1 h. Then the mixture was stirred for 12 hr at 20 °C. The reaction mixture was quenched by addition of NH₄Cl (sat., aq., 80 mL) at 15 °C, extracted with EA (40 mL x 3). The combined organic layers were combined with those of previous batches, washed with H₂O (60 mL), dried over Na₂SO₄, the solids were removed by filtration and the filtrate was concentrated under reduced pressure to give a residue. The residue was purified by flash silica gel chromatography (eluent of 0~15% EA/PE) to give (4-bromo-2,6-dimethyl-phenyl)-[3-isopropyl-1-(p-tolylsulfonyl)pyrrolo[2,3-*c*]pyridin-5-yl]methanol (341 mg, 646.48 umol) as a white solid. ¹H NMR (400MHz, CDCl₃) δ 9.15 (d, *J =* 1.0 Hz, 1H), 7.73 (d, *J*=8.4 Hz, 2H), 7.33 (d, *J=1.0* Hz, 1H), 7.21 (s,2H), 7.09 (s, 2H), 6.87(s, 1H), 6.13 (s, 1H), 5.25 (s, 1H), 2.85 (dd, *J*=6.0, 6.9 Hz, 1H), 2.31 (s, 3H),2.10 (s, 6H), 1.13 (dd, *J*=1.9, 6.9Hz, 6H).

(4-Bromo-2,6-dimethyl-phenyl)-[3-isopropyl-1-(*p*-tolylsulfonyl)pyrrolo[2,3-*c*]pyridin-5-yl]methanol (1.11 g, 2.10 mmol), and HI (1.47 g, 6.31 mmol, 0.864 mL, 55% pure) were taken up into a microwave tube in AcOH (24 mL). The sealed tube was heated at 140°C for 1h in the microwave. The reaction mixture was quenched by addition of NaHCO₃ (sat., aq., 50 mL) at 25 °C, then diluted with H₂O (20 mL) and extracted with EA (40 mL x 2). The combined organic layers were washed with brine (30 mL), dried over Na₂SO₄, filtered and concentrated under reduced pressure to give a residue. The residue was purified by silica gel chromatography (eluent of 0~15% EA/PE) to give 5-[(4-bromo-2,6-dimethyl-phenyl)methyl]-3-isopropyl-1-(*p*-tolylsulfonyl)pyrrolo[2,3-c]pyridine (920 mg, 1.80 mmol, 85% yield) as a brown solid. ¹H NMR (400MHz, CDCl₃) δ 9.12 (s, 1H),7.72 (d, *J*=8.4 Hz, 2H), 7.28 (d,*J*=0.9 Hz, 1H), 7.20 (s, 1H), 7.18 (s,1H), 7.15 (s, 2H), 6.75 (s, 1H), 4.16(s, 2H), 2.84 (spt, *J*=6.8 Hz, 1H),2.33 - 2.26 (m, 3H), 2.15 (s, 6H),1.13 (d, *J*=6.9 Hz, 6H).

A mixture of 5-[(4-bromo-2,6-dimethyl-phenyl)methyl]-3-isopropyl-1-(*p-*tolylsulfonyl)pyrrolo[2,3-*c*]pyridine (340 mg, 398.25 umol), Pin₂B₂ (202.26 mg, 796.50 umol), Pd(dppf)Cl₂.CH₂Cl₂ (32.52 mg, 39.82 umol), KOAc (97.71 mg, 995.62 umol) in DMSO (5 mL) was degassed and purged with N₂ for 3 times, and then the mixture was stirred at 130 °C for 16 hr under N₂ atmosphere. The reaction mixture was diluted with H₂O (20 mL) and extracted with EA (20 mL x 3). The combined organic layers were washed with brine (30 mL), dried over Na₂SO₄, filtered and concentrated under reduced pressure to give a residue. The crude product was combined with a previous batch, then purified by chromatography on a silica gel eluted with PE: EA (from 1/0 to 6/1) to afford 5-[[2,6-dimethyl-4-(4,4,5,5-tetramethyl-1,3,2-dioxaborolan-2-yl)phenyl]methyl]-3-isopropyl-1-(*p*-tolylsulfonyl)pyrrolo[2,3-c]pyridine (350 mg, crude) as a white solid. ¹H NMR (400 MHz, CDCl₃) δ 9.12 (d,*J*=0.8 Hz, 1H), 7.71 (d, *J*=8.4 Hz,2H), 7.45 (s, 2H), 7.24 (d, *J =*1.0 Hz,1H), 7.17 (s, 2H), 6.71 (s, 1H), 4.23(s, 2H), 2.79 (td, *J*=6.5, 13.4 Hz,1H), 2.29 (s, 3H), 2.17 (s, 6H), 1.29(s, 12H), 1.10 (d, *J*=6.8 Hz, 6H).

A mixture of 5-[[2,6-dimethyl-4-(4,4,5,5-tetramethyl-1,3,2-dioxaborolan-2-yl)phenyl]methyl]-3-isopropyl-1-(*p*-tolylsulfonyl)pyrrolo[2,3-c]pyridine (680 mg, 1.22 mmol), NaIO₄ (781.21 mg, 3.65 mmol, 202.39 uL), NH₄OAc (281.53 mg, 3.65 mmol) in acetone (20 mL) and H₂O (10 mL) was stirred at 25 °C for 42 hr. The reaction mixture was diluted with H₂O (30 mL) and extracted with EA (30 mL x 3). The combined organic layers were washed with brine (20 mL x 3), dried over Na₂SO₄, the solids were removed by filtration and the filtrate was concentrated under reduced pressure. The crude was purified by prep-TLC (SiO₂, PE: EA = 1:2) to afford 4-[[3-isopropyl-1-(p-tolylsulfonyl)pyrrolo[2,3-*c*]pyridin-5-yl]methyl]-3,5-dimethyl-phenyl]boronic acid (380 mg, 785.31 umol, 64.5% yield, 98% purity) as a white solid.

A mixture of 6-amino-4-(benzyloxymethyl)-2*H*-1,2,4-triazine-3,5-dione (186.22 mg, 750.18 umol), [4-[[3-isopropyl-1-(*p*-tolylsulfonyl)pyrrolo[2,3-c]pyridin-5-yl]methyl]-3,5-dimethyl-phenyl]boronic acid (330 mg, 681.98 umol), pyridine (107.89 mg, 1.36 mmol) , 4A MS (5 g, 681.98 umol) and Cu(OAc)₂ (61.93 mg, 340.99 umol) in DMF (10 mL) was degassed and purged with O₂ for 3 times, and then the mixture was stirred at 60 °C for 16 hr under O₂ atmosphere. EA (60 mL) was added and stirred at 25 °C for 10 min. Then filtered and filtrate was washed with H₂O (20 mL x 3), brine (20 mL), dried over Na₂SO₄, filtered and concentrated under reduced pressure. The crude was purified by prep-TLC (SiO₂, PE: EA = 1:2) to give 6-amino-4-(benzyloxymethyl)-2-[4-[[3-isopropyl-1-(p-tolylsulfonyl)pyrrolo[2,3-*c*]pyridine-5-yl]methyl]-3,5-dimethyl-phenyl]-1,2,4-triazine-3,5-dione (120 mg, 138.10 umol, 20% yield, 78% purity) as a yellow gum. ¹H NMR (400MHz, CDCl₃) δ 9.12 (s, 1H),7.72 (d, *J*=8.3 Hz, 3H), 7.35 - 7.23(m, 7H), 7.18 - 7.13 (m, 4H), 6.83(s, 1H), 5.52 (s, 2H), 4.69 (s, 2H),4.22 (s, 2H), 2.90 - 2.81 (m, 2H),2.30 (s, 4H), 2.22 (s, 6H), 1.13 (d, *J*=6.9 Hz, 6H).

To a solution of 6-amino-4-(benzyloxymethyl)-2-[4-[[3-isopropyl-1-(p-tolylsulfonyl)pyrrolo[2,3-c]pyridine-5-yl]methyl]-3,5-dimethyl-phenyl]-1,2,4-triazine-3,5-dione (120 mg, 138.10 umol) in DCM (5 mL) was added BBr₃ (276.79 mg, 1.10 mmol, 0.106 mL) at 0°C . The mixture was stirred at 0 °C for 2 h. The reaction mixture was added dropwise to methanol (5 mL) at 0°C and concentrated under reduced pressure to give 6-amino-2-[4-[[3-isopropyl-1-(*p*-tolylsulfonyl)pyrrolo[2,3-*c*]pyridin-5-yl]methyl]-3,5-dimethyl-phenyl]-1,2,4-triazine-3,5-dione (160 mg, crude) as a yellow solid.

To a solution of 6-amino-2-[4-[[3-isopropyl-1-(p-tolylsulfonyl)pyrrolo[2,3-c]pyridin-5-yl]methyl]-3,5-dimethyl-phenyl]-1,2,4-triazine-3,5-dione (160 mg, 286.40 umol) in THF (6 mL) was added TBAF (1 M, 5.73 mL) under N₂ .The mixture was stirred at 65 °C for 16 hr . The reaction mixture was quenched by addition of sat. aq. NH₄Cl (10 mL) at 25 °C, then diluted with H₂O (10 mL) and extracted with EA (20 mL x 3). The combined organic layers were washed with brine (20 mL), dried over anhydrous Na₂SO₄, filtered and concentrated under reduced pressure. The residue was purified by prep-HPLC [Column: Welch Xtimate C18 150 x 30mm x 5um; mobile phase: from 15% CH₃CN in water (0.225%FA) to 45% CH₃CN in water (0.225%FA)] to give 6-amino-2-[4-[(3-isopropyl-1H-pyrrolo[2,3-*c*]pyridin-5-yl)methyl]-3,5-dimethyl-phenyl]-1,2,4-triazine-3,5-dione (12.34 mg, 30.30 umol, 10.6% yield, 99% purity) as a white solid. ¹H NMR (400MHz, DMSO-*d*₆) δ 11.15 (br s, 1H), 8.58 (s, 1H), 8.16(s, 1H), 7.27 (d, J=2.0 Hz, 1H), 7.18(s, 2H), 7.13 (s, 1H), 6.31 (s, 2H),4.18 (s, 2H), 3.01 (td, *J*=7.0, 13.7Hz, 1H), 2.31 (s, 6H), 1.23 (d, *J*=6.9Hz, 6H).

### Example 90: Synthesis of compound 159

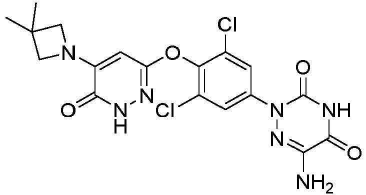

To a stirred mixture of 3,4,6-trichloropyridazine (4.00 g, 21.808 mmol) and 3,3-dimethylazetidine (2.93 g, 23.989 mmol) in DMF (50 mL) was added K₂CO₃ (9.04 g, 65.424 mmol) in portions at rt. The resulting mixture was stirred for 3 h at 40 °C and quenched with water (150 mL) at rt. The resulting mixture was filtered, the filter cake was washed with water (2 x 50 mL) and dried under IR lamp to afford 3,6-dichloro-4-(3,3-dimethylazetidin-1-yl)pyridazine (4.6 g, 88.15%) as a white solid. ¹H NMR (300 MHz, CDCl₃) δ 6.21 (s, 1H), 4.00 (s, 4H), 1.38 (s, 6H). LCMS (ESI, *m*/*z):* 232 [M+H]⁺.

To a stirred mixture of 3,6-dichloro-4-(3,3-dimethylazetidin-1-yl)pyridazine (4.60 g, 19.818 mmol) in methanol (30 mL) was added NaOCH₃ (1.28 g, 23.693 mmol) dropwise at rt. The resulting mixture was stirred overnight at 60 °C and concentrated under reduced pressure. Then water (60 mL) was added. The resulting mixture was filtered, the filter cake was washed with water (2 x 40 mL) and dried under IR lamp to afford 6-chloro-4-(3,3-dimethylazetidin-1-yl)-3-methoxypyridazine (4.22 g, 87%) as a white solid. ¹H NMR (300 MHz, CDCl₃) δ 6.02 (s, 1H), 4.03 (s, 3H), 3.86 - 3.91 (m, 4H), 1.35 (s, 6H). LCMS (ESI, *m*/*z):* 228 [M+H]⁺.

To a stirred mixture of 6-chloro-4-(3,3-dimethylazetidin-1-yl)-3-methoxypyridazine (4.00 g, 17.568 mmol), *N'*-(3,5-dichloro-4-hydroxyphenyl)-*N,N-*dimethylmethanimidamide (4.09 g, 17.568 mmol), Pd₂(dba)₃ (2.73 g, 2.635 mmol) and (R)-1-[(S)-2-(dicyclohexylphosphino)ferrocenyl]ethyldi-t-butylphosphine (1.46 g, 2.631 mmol) in dioxane (80 mL) was added Cs₂CO₃ (11.45 g, 35.135 mmol) in portions at rt. The resulting mixture was stirred overnight at 110 °C under nitrogen. The resulting mixture was concentrated under reduced pressure and diluted with water (60 mL). The resulting mixture was extracted with ethyl acetate (3 x 120 mL) and the organic layers were combined, washed with brine (2 x 80 mL), dried over anhydrous sodium sulfate, filtered and concentrated under reduced pressure. The residue was purified by silica gel column chromatography with CH₂Cl₂/methanol (40/1) to afford *N*-(3,5-dichloro-4-[[5-(3,3-dimethylazetidin-1-yl)-6-methoxypyridazin-3-yl]oxy]phenyl)-*N,N-*dimethylmethanimidamide (6 g, 74.05%) as a brown solid. ¹H NMR (400 MHz, DMSO-*d*₆) δ 7.90 (s, 1H), 7.07 (s, 2H), 6.11 (s, 1H), 3.81 - 3.91 (m, 7H), 2.90 - 3.05 (m, 6H), 1.26 - 1.30 (m, 6H). LCMS (ESI, *m*/*z):* 424 [M+H]⁺.

To a stirred mixture of *N*-(3,5-dichloro-4-[[5-(3,3-dimethylazetidin-1-yl)-6-methoxypyridazin-3-yl]oxy]phenyl)-*N,N*-dimethylmethanimidamide (2.00 g, 4.713 mmol) in CH₃CN (20 mL) was added TMSI (1.89 g, 9.446 mmol) dropwise at rt. The resulting mixture was stirred overnight at 70 °C and quenched by water (50 mL). The resulting solution was extracted with EA (3 x 60 mL) and the organic layers were combined, washed with brine (2 x 50 mL), dried over anhydrous sodium sulfate, the solids were removed by filtration and the filtrate was concentrated under reduced pressure. The residue was purified by silica gel column chromatography with CH₂Cl₂/methanol (16:1) to afford N-(3,5-dichloro-4-[[5-(3,3-dimethylazetidin-1-yl)-6-oxo-1*H*-pyridazin-3-yl]oxy]phenyl)-*N,N-*dimethylmethanimidamide (950 mg, 44.70%) as a yellow solid. ¹H NMR (400 MHz, DMSO-*d*₆) δ 11.62 (s, 1H), 7.85 (s, 1H), 7.02 (s, 2H), 5.80 (s, 1H), 3.59 - 4.13 (m, 4H), 3.01 (s, 3H), 2.89 (s, 3H), 1.24 (s, 6H). LCMS (ESI, *m*/*z):* 410 [M+H]⁺.

To a stirred solution of *N*-(3,5-dichloro-4-[[5-(3,3-dimethylazetidin-1-yl)-6-oxo-1*H*-pyridazin-3-yl]oxy]phenyl)-*N,N*-dimethylmethanimidamide (950.00 mg, 2.315 mmol) in ethanol (10 mL) was added ethylenediamine (626.19 mg, 10.419 mmol) dropwise at rt.The resulting mixture was stirred overnight at 70 °C and concentrated under reduced pressure. The residue was purified by silica gel column chromatography with CH₂Cl₂/methanol (49/1) to afford 6-(4-amino-2,6-dichlorophenoxy)-4-(3,3-dimethylazetidin-1-yl)-2H-pyridazin-3-one (420 mg, 49.53%) as a yellow solid. ¹H NMR (400 MHz, DMSO-*d*₆) δ 11.59 (s, 1H), 6.60 (s, 2H), 5.74 (s, 1H), 5.53 (s, 2H), 3.86 (br, 4H), 1.24 (s, 6H). LCMS (ESI, *m*/*z):* 355 [M+H]⁺.

To a stirred solution of 6-(4-amino-2,6-dichlorophenoxy)-4-(3,3-dimethylazetidin-1-yl)-2*H*-pyridazin-3-one (250.00 mg, 0.706 mmol) in HCl (8.0 mL), acetic acid (22 mL) and water (16 mL) was added sodium nitrite (102.0 mg, 1.483 mmol) in water (2mL), dropwise at 0 °C. The resulting mixture was stirred for 45 min at 0 °C. Ethyl N-(2-cyanoacetyl)carbamate (165.0 mg, 1.059 mmol) in water (52 mL) and pyridine (8 mL) was stirred for 10 min at 0 °C. Put the above solution to this mixture and the resulting mixture was stirred for 1h at 0 °C. The resulting mixture was filtered , the filter cake was washed with water (2 x 10 mL) and dried under IR lamp to afford ethyl (2-cyano-2-(2-(3,5-dichloro-4-((5-(3,3-dimethylazetidin-1-yl)-6-oxo-1,6-dihydropyridazin-3-yl)oxy)phenyl)hydrazineylidene)acetyl)carbamate (300 mg, crude) as a yellow solid. ¹H NMR (400 MHz, DMSO-*d*₆) δ 12.07 - 12.10 (m, 1H), 11.66 (s, 1H), 10.90 (s, 1H), 7.93 (s, 2H), 5.83 (s, 1H), 4.15 - 4.22 (m, 2H), 3.61 - 4.01 (m, 4H), 1.26 (s, 9H). LCMS (ESI, *m*/*z):* 522 [M+H]⁺.

To a stirred mixture of ethyl (2-cyano-2-(2-(3,5-dichloro-4-((5-(3,3-dimethylazetidin-1-yl)-6-oxo-1,6-dihydropyridazin-3-yl)oxy)phenyl)hydrazineylidene)acetyl)carbamate (300 mg, 0.574 mmol) in DMA (8 mL) was added KOAc (225.47 mg, 2.297 mmol) in portions at rt .The resulting mixture was stirred for 6 h at 110 °C and quenched by water (15 mL) at rt. The mixture was extracted with ethyl acetate (3 x 40 mL) and the organic layers were combined, washed with brine (2 x 40 mL), dried over anhydrous sodium sulfate, filtered and concentrated under reduced pressure to afford 2-(3,5-dichloro-4-[[5-(3,3-dimethylazetidin-1-yl)-6-oxo-1*H*-pyridazin-3-yl]oxy]phenyl)-3,5-dioxo-4H-1,2,4-triazine-6-carbonitrile (300 mg, crude) as a yellow solid. ¹H NMR (400 MHz, DMSO-*d*₆) δ 13.27 (s, 1H), 11.70 (s, 1H), 7.73 (s, 2H), 5.91 (s, 1H), 3.92 (br, 4H), 1.27 (s, 6H). LCMS (ESI, *m*/*z):* 476 [M+H]⁺.

To a stirred mixture of 2-(3,5-dichloro-4-[[5-(3,3-dimethylazetidin-1-yl)-6-oxo-1H-pyridazin-3-yl]oxy]phenyl)-3,5-dioxo-4H-1,2,4-triazine-6-carbonitrile (180.00 mg, 0.378 mmol) in methanol (4 mL) was added NaOH (8 mL, 2M) dropwise at rt .The resulting mixture was stirred for 30 min at 55 °C and diluted with water (30 mL) at rt. The resulting mixture was extracted with EA (3 x 50 mL) and the organic layers were combined, were washed with brine (2 x 40 mL), dried over anhydrous sodium sulfate, filtered and concentrated under reduced pressure to afford 2-(3,5-dichloro-4-[[5-(3,3-dimethylazetidin-1-yl)-6-oxo-1H-pyridazin-3-yl]oxy]phenyl)-3,5-dioxo-4*H*-1,2,4-triazine-6-carboxylic acid (180 mg, crude) as a yellow solid. LCMS (ESI, *m*/*z):* 495 [M+H]⁺.

To a stirred mixture of 2-(3,5-dichloro-4-[[5-(3,3-dimethylazetidin-1-yl)-6-oxo-1H-pyridazin-3-yl]oxy]phenyl)-3,5-dioxo-4*H*-1,2,4-triazine-6-carboxylic acid (180.00 mg, 0.363 mmol) and triethylamine (147.11 mg, 1.454 mmol) in t-butanol (5 mL) was added diphenylphosphoryl azide (300.05 mg, 1.090 mmol) dropwise at rt .The resulting mixture was stirred for overnight at 85 °C .The resulting mixture was concentrated under reduced pressure. The residue was extracted with EA (3 x 40 mL) and the organic layers were combined, washed with brine (2 x 30 mL), dried over anhydrous sodium sulfate, the solids were removed by filtration and the filtrate was concentrated under reduced pressure. The residue was purified by silica gel column chromatography, eluted with dichloromethane/methanol (24/1) to afford *t*-butyl *N*-[2-(3,5-dichloro-4-[[5-(3,3-dimethylazetidin-1-yl)-6-oxo-1H*-*pyridazin-3-yl]oxy]phenyl)-3,5-dioxo-4*H*-1,2,4-triazin-6-yl]carbamate (48 mg, 18%) as a yellow solid. ¹H NMR (400 MHz, CDCl₃) δ 12.79 (s, 1H), 11.13 (s, 1H), 7.59 (s, 1H), 7.55 (s, 2H), 5.72 (s, 1H), 4.02 (s, 4H), 1.51(s, 9H), 1.25 (s, 6H). LCMS (ESI, *m*/*z):* 566 [M+H]⁺.

To a stirred solution of t-butyl *N*-[2-(3,5-dichloro-4-[[5-(3,3-dimethylazetidin-1-yl)-6-oxo-1*H*-pyridazin-3-yl]oxy]phenyl)-3,5-dioxo-4H-1,2,4-triazin-6-yl]carbamate (38.00 mg, 0.067 mmol) in CH₂Cl₂ (5 mL) was added TFA (1 mL) dropwise at rt. The resulting mixture was stirred for 2 h at rt and concentrated under reduced pressure. The residue was purified by prep-HPLC with the following conditions (Column: XBridge Prep Phenyl OBD, 19×150mm 5um 13nm ; Mobile Phase A: water (10 MMOL/L NH₄CO₃+0.1% NH₄OH), Mobile Phase B: Acetonitrile; Flow rate:25 mL/min; Gradient:20 B to 39 B in 8 min; 254 nm; RT1:6.52) to afford 6-amino-2-(3,5-dichloro-4-[[5-(3,3-dimethylazetidin-1-yl)-6-oxo-1*H*-pyridazin-3-yl]oxy]phenyl)-4*H*-1,2,4-triazine-3,5-dione (8.1 mg, 26%) as a white solid. ¹H NMR (300 MHz, DMSO-*d*₆) δ 11.67 (br, 2H), 7.79 (s, 2H), 6.44 (s, 2H), 5.87 (s, 1H), 3.77 (br, 4H), 1.27 (s, 6H). LCMS (ESI, *m*/*z):* 466 [M+H]⁺.

### Example 91: Synthesis of compound 160

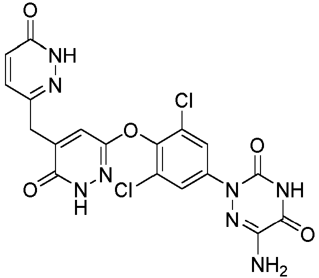

A 500mL autoclave was charged with 3-chloro-6-methoxypyridazine (50.00 g, 345.877 mmol), Pd(dppf)Cl₂ (15.18 g, 20.753 mmol), triethylamine (12.25 g, 121.057 mmol) and methanol (150 mL), and CO (30 atm). The reaction was stirred at 60 °C for 3 days. The resulting solution was concentrated under reduced pressure to get the residue. The residue was chromatographed on a silica gel column with PE/EA (3/1) to get methyl 6-methoxypyridazine-3-carboxylate (16.4g, 27%) as a white solid. ¹H NMR (300MHz, CDCl₃) δ 8.10 (d, J = 9.0Hz, 1H), 7.07 (d, J = 9.0Hz, 1H), 4.25 (s, 3H), 4.06 (s, 3H). LCMS (ESI, *m*/*z):* 169 [M+H]⁺.

To a solution of methyl 6-methoxypyridazine-3-carboxylate (16.40 g, 97.531 mmol) in methanol (55 mL) and THF (275 mL) was added NaBH₄ (11.07 g, 292.592 mmol) and CaCl₂ (10.82 g, 97.531 mmol) in portions. The reaction was stirred at rt for 1h and quenched with water and concentrated under reduced pressure. The residue was chromatographed on a silica gel column with methanol/ CH₂Cl₂ (1/17) to get (6-methoxypyridazin-3-yl)methanol (10.5g, 73%) as a brown oil. ¹H NMR (300 MHz, methanol-*d*₄) δ 7.24 (d, J = 9.0Hz, 1H), 7.22 (d, J = 9.0Hz, 1H), 4.79 (s, 2H), 4.10 (s, 3H). LCMS (ESI, *m*/*z):* 141 [M+H]⁺.

To a solution of (6-methoxypyridazin-3-yl)methanol (10.20 g, 72.783 mmol) in dichloromethane (500 mL) was added Dess-Martin periodinane (43.22 g, 101.897 mmol). The reaction was stirred at rt for 16h and quenched with water (200 mL). The resulting solution was extracted with CH₂Cl₂ (3 x 200 mL) and the organic layers were combined, washed with brine (2 x 100 mL), dried over anhydrous sodium sulfate, the solids were removed by filtration and the filtrate was concentrated under reduced pressure. The residue was chromatographed on a silica gel column with EA/PE (1/3) to provide 6-methoxypyridazine-3-carbaldehyde (7.5g, 67%) as a yellow solid. ¹H NMR (300MHz, CDCl₃) δ 10.27 (s, 1H), 7.97 (d, J = 9.0Hz, 1H), 7.12 (d, J = 9.0Hz, 1H), 4.29 (s, 3H). LCMS (ESI, m/z): 139 [M+H]⁺.

To a solution of 2,2,6,6-tetramethylpiperidine(6.83 g, 48.332 mmol) in THF (100 mL) was added *n*-butyllithium (17.7 mL, 2.5M in hexane, 44.304 mmol) at -85 °C under nitrogen and then the solution was allowed to warm to 0 °C and stirred for 30 min. Then the solution was cooled to -85 °C and a solution of 3,6-dichloropyridazine (6 g, 40.277 mmol) in THF (25 mL) was added. The reaction was stirred at -85 °C for 30 min and a solution of 6-methoxypyridazine-3-carbaldehyde (6.12 g, 44.304 mmol) in THF (25 mL) was added. The resulting solution was stirred at -85 °C for 1h and quenched with NH₄Cl (aq., 100 mL). The resulting solution was extracted with EA (3 x 100 mL) and the organic layers were combined, washed with brine (1 x 100 mL), dried over anhydrous sodium sulfate, filtered and concentrated under reduced pressure. The residue was chromatographed on a silica gel column with EA/ PE (2/3) to get the crude product. The crude product was purified by C18 column (CH₃CN/water=1/3) to provide (3,6-dichloropyridazin-4-yl)(6-methoxypyridazin-3-yl)methanol (2.38 g, 18.52%) as a brown solid. ¹H NMR (300 MHz, CDCl₃) δ 7.94 (s, 1H), 7.52 (d, J = 9.0Hz, 1H), 7.13 (d, J = 9.0Hz, 1H), 6.27 (s, 1H), 4.76 (b, 1H), 4.29 (s, 3H). LCMS (ESI, *m*/*z):* 287 [M+H]⁺.

To a solution of (3,6-dichloropyridazin-4-yl)(6-methoxypyridazin-3-yl)methanol(4 g, 13.93 mmol) in CH₂Cl₂ (160 mL) was added 2,6-lutidine (5.9718 g, 55.73 mmol) and sulfurous dichloride (3.3151 g, 27.86 mmol). The reaction was stirred at rt for 16h and quenched with water (100 mL). The resulting solution was extracted with CH₂Cl₂ (3 x 100 mL) and the organic layers were combined, washed with brine (2 x 100 mL), dried over anhydrous sodium sulfate, the solids were removed by filtration and the filtrate was concentrated under reduced pressure. The residue was chromatographed on a silica gel column with EA/PE (1/3) to get 3,6-dichloro-4-[chloro(6-methoxypyridazin-3-yl)methyl]pyridazine (2.6 g, 61.2%) as a yellow oil. ¹H NMR (300 MHz, CDCl₃) δ 8.07 (s, 1H), 7.65 (d, J = 9.0Hz, 1H), 7.12 (d, J = 9.0 Hz, 1H), 6.43 (s, 1H), 4.16 (s, 3H). LCMS (ESI, *m*/*z):* 305 [M+H]⁺.

To a solution of 3,6-dichloro-4-[chloro(6-methoxypyridazin-3-yl)methyl]pyridazine(2.600 g, 8.510 mmol) in toluene (104 mL) was added tributyltin hydride (4954 mg, 17.019 mmol) and 2,2'-azobis(2-methylpropionitrile) (279.47 mg, 1.702 mmol). The reaction was stirred at 100 °C for 16h and quenched with water (100 mL). The mixture was extracted with ethyl acetate (3 x 100 mL) and the organic layers were combined, washed with brine (100 mL), dried over anhydrous sodium sulfate, the solids were removed by filtration and the filtrate was concentrated under reduced pressure. The residue was chromatographed on a silica gel column with EA/PE (1/2) to get 3,6-dichloro-4-[(6-methoxypyridazin-3-yl)methyl]pyridazine (1.9 g, 76%) as a yellow oil. ¹H NMR (300MHz, CDCl₃) δ 7.55 (s, 1H), 7.41 (d, J = 6.0Hz, 1H), 7.05 (d, J = 6.0Hz, 1H), 4.35 (s, 2H), 4.16 (s, 3H). LCMS (ESI, m/z): 271 [M+H]⁺.

To a solution of 3,6-dichloro-4-[(6-methoxypyridazin-3-yl)methyl]pyridazine(1.100 g, 4.058 mmol) and 4-amino-2,6-dichlorophenol (866.74 mg, 4.869 mmol) in DMSO (22 mL) was added K₂CO₃ (1401.94 mg, 10.144 mmol). The reaction was stirred at 90 °C for 8h and quenched with water (100 mL). The mixture was extracted with EA (3 x 100 mL) and the organic layers were combined, washed with brine (1 x 50 mL), dried over anhydrous sodium sulfate, the solids were removed by filtration and the filtrate was concentrated under reduced pressure. The residue was chromatographed on a silica gel column with EA/ PE (1/2) to afford 3,5-dichloro-4-([6-chloro-5-[(6-methoxypyridazin-3-yl)methyl]pyridazin-3-yl]oxy)aniline (430mg, 25%) as brown solid. ¹H NMR (300MHz, DMSO-*d*₆) δ 7.68 - 7.71 (m, 2H), 7.26 (d, J = 9.0Hz, 1H), 6.72 (s, 2H), 4.97 (b, 2H), 4.40 (s, 2H), 3.99 (s, 3H). LCMS (ESI, m/z): 412 [M+H]⁺.

To a solution of 3,5-dichloro-4-([6-chloro-5-[(6-methoxypyridazin-3-yl)methyl]pyridazin-3-yl]oxy)aniline(435.00 mg, 1.054 mmol) in water (32 mL), conc. HCl (12 mL) and acetic acid (36 mL) was added sodium nitrite (152.73 mg, 2.214 mmol) in water (5 mL) dropwise at 0 °C. After the addition, the reaction was stirred at 0 °C for 45 min. Then the reaction mixture was added to a solution of ethyl N-(2-cyanoacetyl)carbamate (246.89 mg, 1.581 mmol) in water (32 mL) and pyridine(14 mL) at 0 °C quickly. The resulting mixture was stirred at 0 °C for 1 h and filtered. The filter cake was dried under IR lamp to get ethyl (2-cyano-2-(2-(3,5-dichloro-4-((6-chloro-5-((6-methoxypyridazin-3-yl)methyl)pyridazin-3-yl)oxy)phenyl)hydrazineylidene) acetyl)carbamate (410 mg, 62.01%) as a brown yellow solid. LCMS (ESI, *m*/*z):* 579 [M+H]⁺.

To a solution of ethyl (2-cyano-2-(2-(3,5-dichloro-4-((6-chloro-5-((6-methoxypyridazin-3-yl)methyl)pyridazin-3-yl)oxy)phenyl)hydrazineylidene)acetyl)carbamate (410.00 mg, 0.707 mmol) in acetic acid (10 mL) was added sodium acetate (290.3 mg, 3.54 mmol). The reaction was stirred at 105 °C for 4h and cooled to rt. The reaction was poured into water (100 mL) and filtered. The filter cake was dissolved in DMF and purified by C18 column (CH₃CN/water=2/3) to get 2-[3,5-dichloro-4-([5-[(6-methoxypyridazin-3-yl)methyl]-6-oxo-1H-pyridazin-3-yl]oxy)phenyl]-3,5-dioxo-4H-1,2,4-triazine-6-carbonitrile (270 mg, 67%) as a brown solid. ¹H NMR (300MHz, methanol-*d*₄) δ 7.83 - 7.87 (m, 2H), 7.78 (s, 2H), 7.56 (s, 1H), 4.15 (s, 3H), 2.05 (s, 2H). LCMS (ESI, *m*/*z*): 515 [M+H]⁺.

A solution of 2-[3,5-dichloro-4-([5-[(6-methoxypyridazin-3-yl)methyl]-6-oxo-1*H*-pyridazin-3-yl]oxy)phenyl]-3,5-dioxo-4H-1,2,4-triazine-6-carbonitrile (160.00 mg, 0.311 mmol) in acetic acid (5 mL) and conc. HCl (aq. 2.5 mL) was stirred at 105 °C for 3h. The pH value of the reaction mixture was adjusted to 10 with NaHCO3 (sat., aq.) and extracted with EA (2 x 20 mL) and the organic layers were discarded. The pH value of the aqueous layers was adjusted to 4 with conc. HCl and extracted with isopropanol/CHCl₃ (1/3) (2 x 50 ml). The organic layers were combined, dried over anhydrous sodium sulfate, filtered and concentrated under reduced pressure to get 2-[3,5-dichloro-4-([5-[(6-hydroxypyridazin-3-yl)methyl]-6-oxo-1*H*-pyridazin-3-yl]oxy)phenyl]-3,5-dioxo-4H-1,2,4-triazine-6-carboxylic acid (120mg, 66.8%) as a brown solid. LCMS (ESI, *m*/*z):* 520 [M+H]⁺.

To a solution of 2-[3,5-dichloro-4-([6-oxo-5-[(6-oxo-1*H*-pyridazin-3-yl)methyl]-1*H*-pyridazin-3-yl]oxy)phenyl]-3,5-dioxo-4H-1,2,4-triazine-6-carboxylic acid (110 mg, 0.211 mmol) in t-butanol (4 mL) was added DPPA (174.57 mg, 0.634 mmol) and triethylamine (85.58 mg, 0.846 mmol). The reaction was stirred at 80 °C for 16h and concentrated under reduced pressure. The residue was dissolved in EA (50 mL). The resulting mixture was washed with sodium bicarbonate solution (3 x 30 mL) and brine. The combined organic layers were dried over anhydrous sodium sulfate. After filtration, the filtrate was concentrated under reduced pressure to afford *t*-butyl N-[2-[3,5-dichloro-4-([6-oxo-5-[(6-oxo-1*H*-pyridazin-3-yl)methyl]-1*H*-pyridazin-3-yl]oxy)phenyl]-3,5-dioxo-4*H*-1,2,4-triazin-6-yl]carbamate (60 mg, 38%) as a white solid. LCMS (ESI, *m*/*z):* 591 [M+H]⁺.

To a solution of *t*-butyl N-[2-[3,5-dichloro-4-([6-oxo-5-[(6-oxo-1*H-*pyridazin-3-yl)methyl]-1*H*-pyridazin-3-yl]oxy)phenyl]-3,5-dioxo-4H-1,2,4-triazin-6-yl]carbamate (60 mg, 0.101 mmol) in CH₂Cl₂ (5 mL) was added TFA (2 mL). The reaction was stirred at rt for 2h and concentrated under reduced pressure. The residue was purified by Pre-HPLC(Column: XBridge Shield RP18 OBD Column, 19 x 250mm,10 µm; Mobile Phase A:Water (10 MMOL/L NH₄HCO₃ + 0.1%NH₃.H₂O), Mobile Phase B: CH₃CN ; Flow rate:25 mL/min; Gradient:10 B to 35 B in 7 min; 220 nm; RT1:6.35) to get 6-amino-2-[3,5-dichloro-4-([6-oxo-5-[(6-oxo-1*H*-pyridazin-3-yl)methyl]-1H-pyridazin-3-yl]oxy)phenyl]-4H-1,2,4-triazine-3,5-dione (5.7 mg, 11.32%) as a white solid. ¹H NMR (300 MHz, DMSO-*d*₆) δ 7.86 (s, 2H), 7.56 (s, 1H), 7.47 (d, *J =* 9.6Hz, 1H), 6.88 (d, *J =* 9.6Hz, 1H), 6.48 (s, 2H), 3.83 (s, 2H). LCMS (ESI, m/z): 491 [M+H]⁺.

### Example 92: Synthesis of compound 161

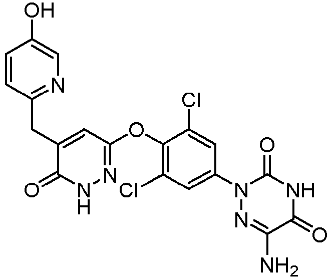

Potassium t-butoxide (43.19 g, 384.869 mmol) was added to 6-methylpyridin-3-ol (40.00 g, 366.542 mmol) in THF (1.6 L) at 0 °C. The mixture was stirred at rt for 30 min. CH₃I (54.63 g, 384.869 mmol) was added dropwise at 0 °C, and stirring was continued at rt for overnight. Water (500 mL) was added and the mixture was evaporated to half its volume and extracted with ethyl acetate (3 x 500 mL). The combined organic layers were washed with brine, dried over anhydrous sodium sulfate, and filtered. The filtrate was concentrated under reduced pressure. The residue was chromatographed on a silica gel column with EA/PE (1/3) to give 5-methoxy-2-methylpyridine (17.5 g, 36.8%). ¹H NMR (300 MHz, Methanol-*d*₄) δ 8.09 (d, J = 3.0 Hz, 1H), 7.33 (dd, J = 8.6, 3.0 Hz, 1H), 7.22 (d, J = 8.6 Hz, 1H), 3.86 (s, 3H), 2.46 (s, 3H). LCMS (ESI, *m*/*z):* 124 [M+H]⁺.

To a solution of 2,2,6,6-tetramethylpiperidine (13.76 g, 97.438 mmol) in THF (75 mL) was added *n*-butyllithium (39 mL, 2.5M in hexane, 97.438 mmol) at -75°C under nitrogen and then the solution was allowed to warm to 0 °C and stirred for 30 min. Then the solution was cooled to -75°C and a solution of 5-methoxy-2-methylpyridine (10.00 g, 81.198 mmol) in THF (75 mL) was added. The reaction was stirred at -75°C for 30 min and a solution of 3,4,6-trichloropyridazine (17.87 g, 97.438 mmol) in THF (50 mL) was added. The resulting solution was stirred at -75°C for 1h. The reaction was quenched with NH₄Cl (aq., 100 mL). The resulting solution was extracted with EA (3 x 300 mL) and the organic layers were combined, washed with brine (1 x 100 mL), dried over anhydrous sodium sulfate, filtered and concentrated under reduced pressure. The residue was chromatographed on a silica gel column with EA/PE (1/3) to get 3,6-dichloro-4-[(5-methoxypyridin-2-yl)methyl]pyridazine (3.2 g, 13.8%) as a light brown solid. ¹H NMR (300 MHz, methanol-*d₄*) δ 8.19 - 8.20 (m, 1H), 7.67 (s, 1H), 7.33 - 7.48 (m, 2H), 4.26 (s, 2H), 3.89 (s, 3H). LCMS (ESI, m/z): 270 [M+H]⁺.

To a solution of 3,6-dichloro-4-[(5-methoxypyridin-2-yl)methyl]pyridazine (3.20 g, 11.847 mmol) in DMSO (90 mL) was added 4-amino-2,6-dichlorophenol (2.53 g, 14.216 mmol) and potassium carbonate (4.91 g, 35.541 mmol). The mixture was stirred at 90 °C for 2h and quenched with water (100 mL). The resulting solution was extracted with EA (3 x 200 mL) and the organic layers were combined, washed with brine, dired over anhyrous sodium sulfate, filtered and concentrated under reduced pressure. The residue was chromatographed on a C18 column with acetonitrile/water (2/3) to get 3,5-dichloro-4-([6-chloro-5-[(5-methoxypyridin-2-yl)methyl]pyridazin-3-yl]oxy)aniline (1.51 g, 165.13%) as a brown solid. ¹H NMR (300 MHz, DMSO-*d*₆) δ 8.27 (s, 1H), 7.55 (s, 1H), 7.39 - 7.52 (m, 2H), 6.70 (s, 2H), 4.80 (br, 2H), 4.26 (s, 2H), 3.83 (s, 3H). LCMS (ESI, m/z): 411 [M+H] ⁺.

To a solution of 3,5-dichloro-4-([6-chloro-5-[(5-methoxypyridin-2-yl)methyl]pyridazin-3-yl]oxy)aniline (1.50 g, 3.635 mmol) in acetic acid (130 mL), concentrated HCl (45 mL) and water (100 mL) was added sodium nitrite solution (0.53 g, 7.633 mmol) in water (10 mL) dropwise at 0 °C. Then the reaction was stirred at 0 °C for 45 min. Then the reaction mixture was added to the mixture of ethyl *N*-(2-cyanoacetyl)carbamate (0.85 g, 5.452 mmol) in pyridine (50 mL) and water (100 mL) at 0 °C quickly. The resulting mixture was stirred for 30 min at 0 °C. The precipitated solids were collected by filtration and washed with water and PE (50 mL) to afford ethyl (2-cyano-2-(2-(3,5-dichloro-4-((6-chloro-5-((5-methoxypyridin-2-yl)methyl)pyridazin-3-yl)oxy)phenyl)hydrazineylidene)acetyl)carbamate (1.03 g, 45.5%) as a yellow solid. LCMS (ESI, *m*/*z):* 578 [M+H]⁺.

To a solution of ethyl (2-cyano-2-(2-(3,5-dichloro-4-((6-chloro-5-((5-methoxypyridin-2-yl)methyl)pyridazin-3-yl)oxy)phenyl)hydrazineylidene)acetyl)carbamate (1.00 g, 1.785 mmol) in acetic acid (30 mL) was added sodium acetate (0.73 g, 8.923 mmol).The mixture was stirred at 105 °C for 3h and quenched with water (100 mL). The precipitated solids were collected by filtration and washed with water and PE (20 mL). The residue was chromatographed on a C18 column with CH₃CN/water (2/3) to get 2-[3,5-dichloro-4-([5-[(5-methoxypyridin-2-yl)methyl]-6-oxo-1*H*-pyridazin-3-yl]oxy)phenyl]-3,5-dioxo-4*H*-1,2,4-triazine-6-carbonitrile (200 mg, 20.27%) as a brown solid. LCMS (ESI, *m*/*z*): 514 [M+H]+.

To a solution of 2-[3,5-dichloro-4-([5-[(5-methoxypyridin-2-yl)methyl]-6-oxo-1*H*-pyridazin-3-yl]oxy)phenyl]-3,5-dioxo-4H-1,2,4-triazine-6-carbonitrile (200.00 mg, 0.390 mmol) in acetic acid (4 mL) was added concentrated hydrochloric acid (2 mL).The mixture was stirred at 105°C for 3h. The pH of the reaction mixture was adjusted to 10 with NaHCO₃ (sat., aq.) and extracted with EA (2 x 20 mL) and the organic layers were discarded. The pH value of the aqueous layers was adjusted to 4 with concentrated hydrochloride acid and extracted with isopropanol/chloroform (1/3) (2 x 50 ml). The organic layers were combined, dried over anhydrous sodium sulfate, the solids were removed by filtration and the filtrate was concentrated under reduced pressure to afford 2-[3,5-dichloro-4-([5-[(5-methoxypyridin-2-yl)methyl]-6-oxo-1H-pyridazin-3-yl]oxy)phenyl]-3,5-dioxo-4H-1,2,4-triazine-6-carboxylic acid (200 mg, 77.15%) as a yellow solid. LCMS (ESI, m/z): 533 [M+H]⁺.

To a solution of 2-[3,5-dichloro-4-([5-[(5-methoxypyridin-2-yl)methyl]-6-oxo-1H-pyridazin-3-yl]oxy)phenyl]-3,5-dioxo-4H-1,2,4-triazine-6-carboxylic acid (200 mg, 0.375 mmol) in t-butyl alcohol (8 mL) was added DPPA (309.63 mg, 1.125 mmol), triethylamine (151.80 mg, 1.500 mmol). The mixture was stirred at 85 °C overnight and concentrated under reduced pressure. The residue was dissolved in EA (50 mL). The resulting mixture was washed with sodium bicarbonate solution (3 x 30 mL) and brine. The combined organic layers were dried over anhydrous sodium sulfate. The solids were removed by filtration, and the filtrate was concentrated under reduced pressure. The residue was chromatographed on a C18 column with CH₃CN/water (1/2) to afford *t*-butyl *N*-[2-[3,5-dichloro-4-([5-[(5-methoxypyridin-2-yl)methyl]-6-oxo-1*H*-pyridazin-3-yl]oxy)phenyl]-3,5-dioxo-4*H*-1,2,4-triazin-6-yl]carbamate (112 mg, 47.38%) as a brown solid. LCMS (ESI, m/z): 604 [M+H]⁺.

To a solution of t-butyl N-[2-[3,5-dichloro-4-([5-[(5-methoxypyridin-2-yl)methyl]-6-oxo-1*H*-pyridazin-3-yl]oxy)phenyl]-3,5-dioxo-4H-1,2,4-triazin-6-yl]carbamate (50.00 mg, 0.083 mmol) in CH₂Cl₂ (3 mL) was added BBr₃ (414.50 mg, 1.655 mmol) at 0 °C. The mixture was stirred at rt for 2 days. The reaction mixture was quenched with methanol and concentrated under reduced pressure. The residue was purified by preparatory HPLC(Column: XSelect CSH Prep C18 OBD, 19 x 250 mm, 5 µm; Mobile Phase A: Water(10 MMOL/L NH₄CO₃ +0.1% NH₄OH), Mobile Phase B: CH₃CN ; Flow rate:25 mL/min; Gradient:10 B to 30 B in 8 min; 254 nm; RT1:6.65) to get 6-amino-2-[3,5-dichloro-4-([5-[(5-hydroxypyridin-2-yl)methyl]-6-oxo-1H-pyridazin-3-yl]oxy)phenyl]-4H-1,2,4-triazine-3,5-dione (8 mg, 19.41%) as a white solid. ¹H NMR (400 MHz, DMSO-*d*₆) δ 12.26 (br, 2H), 9.82 (br, 1H), 8.07 (d, J = 2.8 Hz, 1H), 7.85 (s, 2H), 7.13 - 7.28 (m, 3H), 6.49 (br, 2H), 3.89 (s, 2H). LCMS (ESI, m/z): 490 [M+H]+.

### Example 93: Synthesis of compound 162

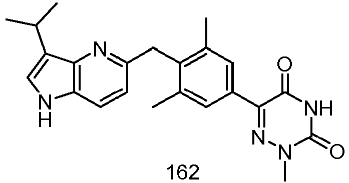

A mixture of 5-[[2,6-dimethyl-4-(4,4,5,5-tetramethyl-1,3,2-dioxaborolan-2-yl)phenyl]methyl] -3-isopropyl-1-(*p*-tolylsulfonyl)pyrrolo[3,2-b]pyridine (200 mg, 322.27 umol, 90% purity), 6-bromo-2-methyl-1,2,4-triazine-3,5-dione (69.71 mg, 338.38 umol), K₂CO₃ (89.08 mg, 644.54 umol) and palladium;triphenylphosphane (74.48 mg, 64.45 umol) in dioxane (8 mL) and H₂O (1.6 mL) was stirred at 110 °C for 12 hr under N₂. The reaction mixture was cooled down, diluted with H₂O (30 mL) and extracted with EA (30 mL x 2). The combined organic layers were washed with brine (30 mL), dried over anhydrous Na₂SO₄, filtered and concentrated under reduced pressure to give a residue. The residue was purified by silica gel chromatography (Eluent of 0~50% EA/PE) to give 6-[4-[[3-isopropyl-1-(p-tolylsulfonyl)pyrrolo[3,2-b]pyridin-5-yl]methyl]-3,5-dimethyl-phenyl]-2-methyl -1,2,4-triazine-3,5-dione (96 mg, 106.73 umol, 33.12% yield, 62% purity) as a yellow solid.

To a solution of 6-[4-[[3-isopropyl-1-(p-tolylsulfonyl)pyrrolo[3,2-b]pyridin-5-yl]methyl]-3,5-dimethyl- phenyl]-2-methyl-1,2,4-triazine-3,5-dione (96 mg, 172.15 umol) in THF (8 mL) was added TBAF (1 M, 1.38 mL) at 20 °C. After the addition, the mixture was stirred at 65 °C for 12 h. LCMS showed the starting material was consumed completely and 29% desired mass was detected. The reaction mixture was diluted with sat. aq. NH₄Cl (120 mL) and extracted with EA (30 mL x 2). The combined organic layers were washed with brine (30 mL), dried over anhydrous Na₂SO₄, filtered and concentrated under reduced pressure to give a residue. The residue was purified by prep-HPLC [Column: Phenomenex luna C18 80 x 40 mm x 3 um; mobile phase: from 15% CH₃CN in water (0.05% HCl) to 45% CH₃CN in water (0.05%HCl)] to give 6-[4-[(3-isopropyl-1*H*-pyrrolo[3,2-b] pyridin-5-yl)methyl]-3,5-dimethyl-phenyl]-2-methyl-1,2,4-triazine-3,5-dione ].6-[4-[(3-isopropyl-1H-pyrrolo[3,2-*b*]pyridin-5-yl)methyl]-3,5-dimethyl-phenyl]-2-methyl-1,2,4-triazine-3,5-dione (14.07 mg, 33.87 umol, 19.68% yield, 97.13% purity) as a white solid.

### Example 94: Synthesis of compounds 163 and 164

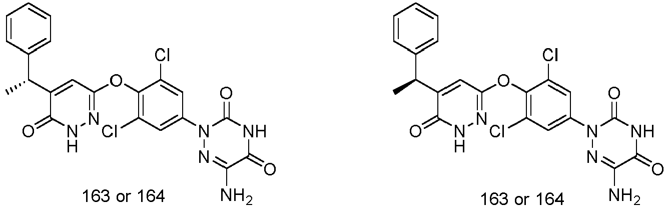

6-Amino-2-(3,5-dichloro-4-((6-oxo-5-(1-phenylethyl)-1,6-dihydropyridazin-3-yl)oxy)phenyl)-1,2,4-triazine-3,5(2*H*,4*H*)-dioneSynthesis of 6-bromo-4-isopropyl-1,5-naphthyridin-2(1H)-one was synthesized using a method analogous to that described for the synthesis of 6-amino-2-(3,5-dichloro-4-((5-(2-fluoro-3-methylphenyl)-6-oxo-1,6-dihydropyridazin-3-yl)oxy)phenyl)-1,2,4-triazine-3,5(2H,4H)-dione where (1-phenylvinyl)boronic acid was employed instead of (2-fluoro-3-methylphenyl)boronic acid.

The crude product (200 mg) was purified by preparative HPLC (Column: CHIRALPAK IE-3, 4.6 x 50mm, 3µm, Hexane (0.1%TFA ): EtOH=55:45, flow rate:1 mL/min) to provide the first eluting enantiomer (Rt: 2.340 min), compound 163 (37.9 mg, 19.69%), as a white solid. And the second eluting isomer (Rt: 2.903 min), compound 164 (41.5 mg, 21.65%) as a white solid. ¹H NMR (400 MHz, DMSO-*d*₆) δ 12.24 - 12.27 (m, 2H), 7.85 (s, 2H), 7.51 (s, 1H), 7.31 - 7.35 (m, 4H), 7.21 - 7.26 (m, 1H), 6.52 (s, 2H), 4.27 - 4.33 (m, 1H), 1.55 (d, *J* = 7.2 Hz, 3H). LCMS (ESI, *m*/*z):* 487 [M+H]⁺.

### Example 95: Synthesis of compounds 165 and 166

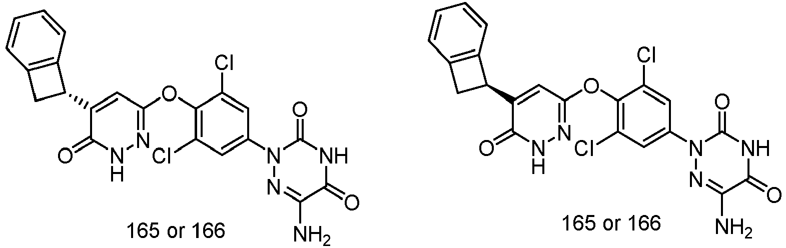

A chiral separation was performed on 6-amino-2-(4-((5-(bicyclo[4.2.0]octa-1(6),2,4-trien-7-yl)-6-oxo-1,6-dihydropyridazin-3-yl)oxy)-3,5-dichlorophenyl)-1,2,4-triazine-3,5(2*H*,4*H*)-dione to afford compounds 165 (Rt: 2.845min on chiral HPLC) and 166 (Rt: 3.697 min on chiral HPLC) (Chiral HPLC conditions: column name: (R,R)-WHELK-O1 4.6 x 50 mm 3.5um, mobile phase:Hex(0.1%DEA):ethanol=70:30, temperature: 25°C, flow 1.000 mL/min, instrument: Agilent 1260). ¹H NMR (400 MHz, DMSO-*d*₆) δ 12.33 (br, 2H), 7.84 (s, 2H), 7.19 - 7.39 (m, 5H), 6.50 (s, 2H), 4.71 (s, 1H), 3.63 - 3.69 (m, 1H), 3.19 - 3.24 (m, 1H).

### Example 96: Synthesis of compound 171

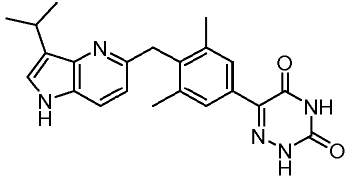

A mixture of 5-[[2,6-dimethyl-4-(4,4,5,5-tetramethyl-1,3,2-dioxaborolan-2-yl)phenyl]methyl] -3-isopropyl-1-(*p*-tolylsulfonyl)pyrrolo[3,2-*b*]pyridine (320 mg, 0.516 mmol, 90% purity), 6-bromo-2*H*-1,2,4-triazine-3,5-dione (148.48 mg, 0.773 mmol), K₂CO₃ (142.53 mg, 1.03 mmol) and palladium;triphenylphosphane (119.17 mg, 0.103 mmol) in dioxane (10 mL) and H₂O (1 mL) was stirred at 100°C for 12h under N₂. The reaction mixture was diluted with H₂O (20 mL) and extracted with EA (20 mL x 2). The combined organic layers were washed with brine (20 mL), dried over anhydrous Na₂SO₄, filtered and concentrated to give crude product, which was purified by flash silica gel chromatography (Eluent of 0~50% EA/PE) to give 6-[4-[[3-isopropyl-1-(*p*-tolylsulfonyl)pyrrolo[3,2-*b]pyridin-5-yl]methyl]-3,5-dimethyl-phenyl]* -2*H*-1,2,4-triazine-3,5-dione (246 mg, 0.453 mmol, 88% yield) as a colorless oil. LCMS (ESI, *m*/*z):* 544 [M+H]⁺.

To a solution of 6-[4-[[3-isopropyl-1-(*p*-tolylsulfonyl)pyrrolo[3,2-b]pyridin-5-yl]methyl]-3,5-dimethyl -phenyl]-2*H*-1,2,4-triazine-3,5-dione (200 mg, 0.368 mmol) in THF (8 mL) was added TBAF (1 M, 1.84 mL) at 20 °C. The mixture was stirred at 65°C for 12h. The reaction mixture was diluted with H₂O (50 mL), extracted with EA (100 mL). The organic layer was washed with brine (50 mL), dried over anhydrous Na₂SO₄, filtered and concentrated to give a residue. The residue was purified by prep-HPLC [Column: Phenomenex luna C18 80 x 40mm x 3 um; mobile phase: from 10% CH₃CN in water (0.05% HCl) to 40% CH₃CN in water (0.05% HCl)] then dissolved in EA (20 mL), washed with NH₄Cl (sat., aq., 20 mL), brine (20 mL) and concentrated to afford 6-[4-[(3-isopropyl-1H-pyrrolo[3,2-b]pyridin-5-yl)methyl]-3,5-dimethyl-phenyl]-2-methyl-1,2,4-triazine -3,5-dione (5 mg, 12.84 umol, 3.5% yield, 100% purity) as a white solid. ¹H NMR (400 MHz, DMSO-*d*₆) δ 12.43 (d, *J =* 1.8 Hz, 1H), 12.04 (s, 1H), 10.80 (br s, 1H), 7.58 - 7.52(m, 3H), 7.29 (d, *J =* 1.6 Hz, 1H), 6.65 (d, *J =* 8.4 Hz, 1H), 4.23 (s, 2H), 3.24 - 3.16 (m, 1H), 2.37 (s, 6H), 1.33 (d, *J* = 6.9 Hz, 6H).

### Example 97: Synthesis of 6-bromo-4-isopropyl-1,5-naphthyridin-2(1H)-one

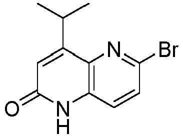

To a solution of 2,6-dibromopyridin-3-amine (30 g, 119.09 mmol) in DCM (500 mL) was added Et₃N (36.15 g, 357.27 mmol, 49.73 mL) and 2,2-dimethylpropanoyl chloride (28.72 g, 238.18 mmol, 29.31 mL) at 0 °C, and the mixture was stirred at 15 °C for 12 h. The mixture was quenched by sat. aq. NH₄Cl (sat., aq., 100 mL), extracted with DCM (200 mL x 3). The combined organic layers were washed with brine (100 mL x2), dried over MgSO₄, filtered and concentrated to give a residue, which was purified by silica gel chromatography (Eluent of 0~20% EA/PE) to give N-(2,6-dibromopyridin-3-yl)pivalamide (41.4 g, crude) as a yellow solid. ¹H NMR (400 MHz, DMSO-*d*₆) δ 9.18 (s, 1H), 7.83 (d, J=8.3 Hz, 1H), 7.71 (d, J=8.3 Hz, 1H), 1.24 (s, 9H).

To a solution of N-(2,6-dibromopyridin-3-yl)pivalamide (21 g, 62.50 mmol) in toluene (300 mL) was added dropwise *n*-BuLi (2.5 M, 62.50 mL) at -70 °C under N₂, the mixture was stirred at -70 °C for 2 h. Then to the mixture was added 2-methylpropanal (9.01 g, 124.99 mmol, 11.41 mL), the resulting mixture was stirred at -70 °C for 2 h. The mixture was quenched by sat. aq. NH₄Cl (60 mL) at -70 °C, then warmed to 10 °C, diluted with H₂O (200 mL) and extracted with EA (200 mLx3). The combined organic layers were washed with brine (300 mL), dried over anhydrous Na₂SO₄, filtered and concentrated to give a residue, which was purified by flash silica gel chromatography (0~10% EA in PE) to afford N-(6-bromo-2-(1-hydroxy -2-methylpropyl)pyridin-3-yl)pivalamide (13 g, 37.71 mmol, 30.17% yield, 95.5% purity) as a yellow solid. ¹H NMR (400 MHz, DMSO-*d*₆) δ 10.27 (s, 1H), 8.50 (d, J=8.6 Hz, 1H), 7.49 (d, J=8.8 Hz, 1H), 6.77 (d, J=4.5 Hz, 1H), 4.45 (dd, J=4.6, 6.8 Hz, 1H), 2.06 - 1.93 (m, 1H), 1.21 (s, 9H), 0.93 (d, J=6.6 Hz, 3H), 0.77 (d, J=6.8 Hz, 3H).

To a solution of N-(6-bromo-2-(1-hydroxy-2-methylpropyl)pyridin-3-yl)pivalamide (13 g, 39.49 mmol) in DCM (350 mL) was added Dess-Martin (50.24 g, 118.46 mmol, 36.67 mL) in portions at 20 °C. After the addition, the mixture was stirred at 20 °C for 3 h. LCMS showed the starting material was consumed completely and 47.3% of desired product formed under 220 nm. The mixture was quenched by addition of sat. aq. Na₂SO₃ (100 mL) and sat. aq. NaHCO₃ (200 mL), extracted with DCM (100 mLx3). The combined organic layers were washed with brine (200 mL), dried over anhydrous MgSO₄, filtered and concentrated to give a residue, which was purified by flash silica gel chromatography (EA in PE = 0~10%) to give *N*-(6-bromo-2-isobutyrylpyridin-3-yl)pivalamide (11 g, 33.48 mmol, 84.80% yield, 99.6% purity) as a colorless oil. ¹H NMR (400 MHz, DMSO-*d*₆) δ 11.59 (s, 1H), 8.94 (d, J=9.0 Hz, 1H), 7.89 (d, J=9.0 Hz, 1H), 4.02 (spt, J=6.9 Hz, 1H), 1.26 (s, 9H), 1.13 (d, J=6.9 Hz, 6H).

To a solution of LDA (2 M, 35.30 mL) in THF (250 mL) was added t-butyl acetate (8.20 g, 70.60 mmol, 9.47 mL) at -70 °C, the mixture was stirred at -70 °C for 30 min. Then to the mixture was added N-(6-bromo-2-isobutyrylpyridin-3-yl)pivalamide (11 g, 33.62 mmol) in THF (50 mL) at -70 °C, the resulting mixture was stirred at -70 °C for 1 h. The mixture was quenched with sat. aq. NH₄Cl (100 mL) at -70 °C. Then warmed to 20 °C, diluted with H₂O (100 mL) and extracted with EA (100 mLx3). The combined organic layers were washed with brine (200 mL), dried over anhydrous MgSO₄, filtered and concentrated to give a residue, which was purified by flash silica gel chromatography (0~10% EA in PE) to afford t-butyl 3-(6-bromo-3-pivalamidopyridin-2-yl)-3-hydroxy-4-methylpentanoate (14.92 g, 26.55 mmol, 78.98% yield, 78.9% purity) as a yellow oil.

The reaction was set up in 4 batches. t-Butyl 3-(6-bromo-3-pivalamidopyridin-2-yl)-3-hydroxy-4-methylpentanoate (2 g, 3.56 mmol, 78.9% pure) and HBr (18.42 mmol, 5 mL, 20%) were taken up into a microwave tube in dioxane (1 mL). The sealed tube was heated at 160°C for 25 min under microwave irradition. The combined reaction mixture was diluted with H₂O (100 mL) and EA (100 mL). The suspension was filtered, and the filtrate was extracted with EA (100 mL x 3). The combined organic layers were washed with brine (100 mL), dried over anhydrous MgSO₄, filtered and concentrated to give a residue, which was purified by flash silica gel chromatography (0~70% EA in PE) to give 6-bromo-4-isopropyl-1,5-naphthyridin-2(1H)-one (4.36 g, 15.64 mmol, 60.60% yield, 95.8% purity) as a yellow solid. ¹H NMR (400 MHz, DMSO-*d*₆) δ 11.89 (br s, 1H), 7.75 - 7.66 (m, 1H), 7.60 (d, J=8.6 Hz, 1H), 6.60 (s, 1H), 3.70 - 3.56 (m, 1H), 1.23 (d, J=7.0 Hz, 6H).

### Example 98: Methodology for LCMS

### 1. Description of LCMS Conditions

Several different conditions were used to assess the purity, retention time and [M+H]⁺ or [M-H]⁻ of the compounds disclosed herein using LCMS methods. The conditions are listed in Table 1, below. All data were obtained using a Shimadzu LCMS-2020 instrument except when noted differently. For all conditions, column temperature was 40 °C and run time was 3 min, except when noted differently. Flow is reported in units of mL/min. "C" is the number indicator for the listed LCMS condition, which is then referenced in Table 2, below.

**Table 1**

| C | Column | Mobile phase | Gradient | Flow |
|---|---|---|---|---|
| 1 | Ascentis Express C18 (2.7 µm, 3.0x50 mm) | A: H₂O +0.05% TFA | From 95% A to 5% A in 1.99 min, held for 0.6 min, to 95% A in 0.15 min, held for 0.25 min | 1.5 |
| | column temp: 40°C | B: CH₃CN +0.05% TFA | | |
| 2 | ACE Excel 3 Super (3.0 µm, 3.0x50 mm) | A: H₂O + 5 mM NH₄HCO₃ | From 90% A to 5% A in 2.09 min, held for 0.6 min, to 90% A in 0.05 min, held for 0.25 min | 1.2 |
| | column temp: 40°C | B: CH₃CN | | |
| 3 | YMC-Triart C18 (3.0 µm, 3.0x50 mm) | A: H₂O +0.04% NH₄OH | From 90% A to 5% A in 1.89 min, held for 0.8 min, to 90% A in 0.05 min, held for 0.25 min | 1.5 |
| | | B: CH₃CN | | |
| 4 | Poroshell HPH-C18 (2.7 µm, 3.0x50 mm) | A: 6.5 mM NH₄HCO₃ + NH₃H₂O | From 90% A to 5% A in 1.99 min, held for 0.6 min, to 90% A in 0.15 min, held for 0.25 min | 1.2 |
| | column temp: 40°C | B: CH₃CN | | |
| 5 | HALO C18 (2.0 µm, 3.0x30 mm) | A: H₂O +0.05% TFA B: CH ₃CN +0.05% TFA | From 95% A to 50% A in 1.79 min, to 5% A in 0.7 min, held for 0.3 min, to 95% A in 0.01 min, held for 0.19 min. Runtime 3min | 1.5 |
| | column temp: 40°C | | | |
| 5A | HALO C18 (2.7µm, 3.0 x 50mm), | | From 95% A to 0% A in 1.99min, held for 0.7min, to 95% A in 0.05 min, held for 0.15min. Runtime 2.9min | |
| | column temp.: 40°C | | | |
| 5B | (2.7µm, 3.0 x50mm), column temp.: 45°C | | From 70% A to 5% A in 3.99min, held for 0.8min, to95% A in 0.2 min, held for 0.2min. Runtime 5.2min | 1.5 |
| 5C | | | From 95% A to 45% A in 2.99min, to 0% A in 1min, held for 0.6min, to 95% A in 0.1 min, held for 0.5min. Runtime 5.2min | |
| 6 | Shim-pack XR-ODS (2.2 µm, 3.0x50 mm) | | From 95% A to 0% A in 1.99 min, held for 0.7 min, to 95% A in 0.05 min, held for 0.25 min | 1.2 |
| | column temp.: 40°C | | | |
| 7 | Xselect CSH C18 (2.5 µm, 3.0x50 mm) | A: H₂O /0.1% FA | From 95% A to 5% A in 2.09 min, held for 0.6 min, to 95% A in 0.05 min, held for 0.25 min | 1.2 |
| | column temp.: 40°C | B: CH₃CN/0.1% FA | | |
| 8 | CORTECS C18 (2.7 µm, 2.1x50 mm) | A: H₂O +0.05% TFA | From 95% A to 0% A in 1.99 min, held for 0.8 min, to 95% A in 0.1 min, held for 0.1 min | 1.0 |
| | column temp.: 40°C | B: CH₃CN +0.05% TFA | | |
| 9 | Ascentis Express C18 (2.7 µm, 3.0x50 mm) | A: H₂O +0.05% TFA | From 95% A to 0% A in 1.99min, held for 0.8min, to 95% A in 0.1 min, held for 0.1min | 1.5 |
| 10 | column temp.: 40°C | B: CH₃CN +0.05% TFA | From 95% A to 5% A in 1.99min, held for 0.7min, to 95% A in 0.1 min, held for 0.2min | 1.0 |
| 11 | Ascentis Express C18 (2.7µm,3.0x50 mm), column temp.: 45°C | A: H₂O +0.05% TFA | From 95% A to 60% A in 3.19min, to 0% A in 0.8min, held for 0.6min, to 95% A in 0.1 min, held for 0.5min. Runtime: 5.2 min | 1.5 |
| | | B: CH₃CN +0.05% TFA | | |
| 12 | Kinetex 2.6µm EVO C18 100A (2.6µm, 3.0x50 mm), column temp.: 40°C | A: H₂O + 5 mM NH₄HCO₃ | From 90% A to 5% A in 1.99 min, held for 0.6 min, to 90% A in 0.15 min, held for 0.10 min. Runtime: 2.85 min | 1.2 |
| 12A | | B: CH₃CN | From 90% A to 5% A in 1.99min, held for 0.7min, to 90% A in 0.05 min, held for 0.10min. Runtime: 2.85 min | |
| 13 | Ascentis Express C18 (2.7µm,3.0x50 mm), column temp.: 40°C | A: H₂O +0.05% TFA | From 95% A to 40% A in 3.19 min, to 5% A in 0.9 min, held for 0.9 min, to 95% A in 0.1 min, held for 0.2 min. Runtime: 5.3 min | 1.5 |
| 13B | | B: CH₃CN +0.05% TFA | From 95% A to 30% A in 3.59min, to 5% A in 0.6min, held for 0.8min, to 95% A in 0.1 min, held for 0.2min | |
| 14A /B/F | Xtimate C18 2.1x30mm, 3 µm column temp.: 50°C | A: H₂O (4L) +TFA(1.5mL) B: CH₃CN (4L)+TFA(0.75mL) | From 90% A to 20% A in 3 minutes and holding at 20% for 0.5 minutes to 90% A in 0.01 min held for 0.49 min. Runtime: A/B/F: 4 min | 14A:0.8 |
| | | | | 14B:1.2 |
| | | | | 14F:1.0 |
| 14C | | | From 95% A to 5% A in 0.9 minutes and holding at 5% for 0.2 minutes, to 95% A in 0.01min held for 0.49min. Runtime 2min | 1.2 |
| 14D | | | From 95% A to 5% A in 0.9 minutes and holding at 5% for 0.2 minutes, to 95% A in 0.01min held for 0.49min. Runtime 2min | |
| 14E | | | From 10% B to 80% B in 0.9 minutes and holding at 80% for 0.6 minutes ,to 10% B in 0.01min held for 0.49min. Runtime 2min | |
| 15 | Agilent Poroshell 120, EC-C18, 4.6x100 mm - 4µm column temp.: 30°C | A: 0.1% FA in H₂O | 2.0 min 98% A, from 98% A to 0% A in 10 min, hold for 3.4 min. Runtime: 15.4 min | 1 |
| | Agilent 1260 Infinity II | B: 0.05% FA in CH₃CN | | |
| 16A | HALO 90A C18 (2.0µm, 2.1x30mm) column temp.: 40°C | A: H₂O +0.05%TFA | rom 95% A to 0% A in 2.19min, held for 0.5min, to 95% A in 0.05 min, held for 0.25min. Runtime: 3 min | 1 |
| 16B | | B: CH₃CN +0.05%TFA | From 95% A to 50% A in 2.79min, to 0% A in 1 min, held for 0.6min, to 95% A in 0.1 min, held for 0.5min. Runtime: 5 min | 1 |
| 16C | | | From 95% A to 40% A in 2.79min, to 0% A in 1 min, held for 0.6min, to 95% A in 0.1 min, held for 0.5min. Runtime: 5 min | 1 |
| 17 | Ascentis Express C18 (2.7µm, 3.0x50mm) | A: H₂O /0.05%TFA | From 95% A to 50% A in 2.99min, to 0% A in 1.0min, held for 0.6min, to 95% A in 0.1 min, held for 0.5min | 1.5 |
| | | B: CH₃CN /0.05%TFA | | |
| | Column temp.: 45°C | Runtime:5.2 min | | |
| 18 | Kinetex EVO C18 100A (2.6 µm, 3.0x50 mm) column temp.: 40°C | A: H₂O/5mM NH₄HCO₃ | From 90% A to 5% A in 2.99min, held for 0.6min, to 90% A in 0.10 min, held for 0.30min. Runtime:4 min | 1.2 |
| 18B | | B: CH₃CN | From 90% A to 60% A in 3.19min, to 5% in 0.8 min, held for 0.9min, to 90% A in 0.2 min, held for 0.20min. Runtime:5.3 min | |
| 19 | Ascentis Express C18 (2.7µm, 3.0x50mm), column temp.: 40°C | A: H₂O/0.05%TFA | From 95% A to 5% A in 1.99min, held for 0.7min, to 95% A in 0.05 min, held for 0.25min | 1.5 |
| 19A | | | From 95% A to 0% A in 1.99min, held for 0.7min, to 95% A in 0.05 min, held for 0.25min. Runtime 3min | |
| 19B | | B: CH₃CN/0.05%TFA | From 95% A to 0% A in 1.09min, held for 0.6min, to 95% A in 0.05 min, held for 0.1min. Runtime 1.85min | |
| 20 | HALO 90A C18 (2.0µm, 3.0 x 30mm), column temp.: 40°C | A: H₂O/0.05%TFA | From 95% A to 50% A in 3.09min, to 5% A in 1.0min, held for 0.9min, to 95% A in 0.1 min, held for 0.2min. Runtime:5.3 min | 1.5 |
| 20A | | | From 95% A to 0% A in 1.99min, held for 0.7min, to 95% A in 0.05 min, held for 0.25min. Runtime:3min | |
| 20B | | B: CH₃CN/0.05%TFA | From 95% A to 0% A in 1.99min, held for 0.7min, to 95% A in 0.05 min, held for 0.45min. Runtime 3.2min | |
| 20C | | | From 95% A to 60% A in 3.09min, to 10% A in 1.0min, held for 0.9min, to 95% A in 0.1 min, held for 0.2min. Runtime:5.3 min | |
| 21 | | | From 90% A to 5% A in 1.99min, held for 0.7min, to 90% A in 0.05 min, held for 0.25min | 1.2 |
| 21A | Poroshell HPH-C18(2.7 µm, 3.0x50 mm), | A:6.5 mM | From 90% A to 55% A in 2.99min, to 5% in 1min, held for 0.7min, to 90% A in 0.2 min, held for 0.1min (Run time 5min) | |
| | Column temp:40°C | NH₄HCO₃+NH₃H₂O | | |
| 21B | | B: CH₃CN | From 90% A to 60% A in 2.99min, to 5% in 1min, held for 0.7min, to 90% A in 0.2 min, held for 0.1min. Runtime 5min | |
| 22 | Ascentis Express C18 (2.7µm,4.6x100mm) | | From 90% A to 5% A in 7.99min, held for 2.0min, to 90% A in 0.5 min, held for 1.5min. Runtime: 12min | 1.5 |
| | Column temp:40°C | A: Water/0.05%TFA | | |
| 23 | HALO C18 (2.7µm, 3.0x50mm) | B: CH₃CN/0.05%TFA | From 95% A to 0% A in 1.99min, held for 0.7min, to 95% A in 0.05 min, held for 0.25min. Runtime:3min | 1.5 |
| | Column temp:40°C | | | |
| 24 | HPH-C18 (2.7µm, 3.0*50mm) | A:6.5 mM | From 90% A to 50% A in 2.99min, to 5% A in 0.3min held for 0.45min, to 90% A in 0.05 min, held for 0.2min. Runtime 4min | 1.2 |
| | Column temp:35°C | NH₄HCO₃+NH₃H₂O | | |
| | | B: CH₃CN | | |

### 2. LCMS Results

The results of LCMS characterizations and the particular set of conditions used for the compounds disclosed here are presented in Table 2.

**Table 2**

| Cmp'd No. | Rt (min) | [M+H]⁺ or [M-H]⁻ | LCMS method |
|---|---|---|---|
| 1 | 1.660 | [M-H]⁻=454 | 2 |
| 2 | 1.035 | [M-H]⁻=414 | 3 |
| 3 | 1.394 | [M+H]⁺=414 | 4 |
| 4 | 1.217 | [M-H]⁻=470 | 4 |
| 5 | 1.461 | [M-H]⁻=480 | 4 |
| 6 | 1.428 | [M-H]⁻=516 | 4 |
| 7 | 1.429 | [M-H]⁻= 488 | 4 |
| 8 | 1.375 | [M+H]⁺=454 | 5 |
| 9 | 0.734 | [M+H]⁺=453 | 3 |
| 10 | 1.392 | [M+H]⁺=425 | 6 |
| 11 | 1.379 | [M+H]⁺=434 | 1 |
| 12 | 1.304 | [M+H]⁺=449 | 8 |
| 13 | 0.808 | [M+H]⁺=468 | 4 |
| 14 | 1.310 | [M+H]⁺=439 | 1 |
| 15 | 1.489 | [M+H]⁺=453 | 1 |
| 16 | 1.363 | [M+H]⁺=424 | 8 |
| 17 | 1.131 | [M-H]⁻= 437 | 4 |
| 18 | 1.592 | [M+H]⁺=457 | 1 |
| 19 | 1.572 | [M+H]⁺=457 | 1 |
| 20 | 2.692 | [M+H]⁺=499 | 13 |
| 21 | 1.331 | [M+H]⁺=485 | 1 |
| 22 | 1.536 | [M+H]⁺=573 | 1 |
| 23 | 1.504 | [M+H]⁺=438 | 1 |
| 24 | 2.246 | [M+H]⁺=453 | 11 |
| 25 | 1.141 | [M+H]⁺=440 | 9 |
| 26 | 1.234 | [M+H]⁺=467 | 10 |
| 27 | 1.004 | [M+H]⁺=490 | 9 |
| 28 | 1.133 | [M+H]⁺=460 | 12 |
| 29 | 1.293 | [M+H]⁺=454 | 9 |
| 30 | 0.950 | [M+H]⁺=439 | 9 |
| 31 | 1.862 | [M+H]+=415 | 14A |
| 32 | 10.91 | [M+H]+=443 | 15 |
| 33 | 1.427 | [M+H]⁺=470 | 9 |
| 34 | 1.036 | [M+H]⁺=502 | 4 |
| 35 | 1.237 | [M+H]⁺=439 | 1 |
| 36 | 1.107 | [M+H]⁺=423 | 16A |
| 37 | 1.099 | [M+H]⁺=405 | 16A |
| 38 | 1.075 | [M+H]⁺=385 | 9 |
| 39-A | 2.295 | [M+H]⁺=439 | 16B |
| 39-B | 2.298 | [M+H]+=439 | 16B |
| 40-A | 2.614 | [M+H]+=447 | 16B |
| 40 | 1.229 | [M+Na]⁺=459 | 9 |
| 41 | 1.441 | [M+H]⁺=446 | 16A |
| 42 | 1.323 | [M+H]⁺=451 | 9 |
| 43 | 0.865 | [M+H]⁺=416 | 4 |
| 44 | 1.193 | [M+H]⁺=471 | 16A |
| 45 | 1.348 | [M-H]⁻= 508 | 12 |
| 46 | 1.348 | [M-H]⁻= 502 | 4 |
| 47 | 1.199 | [M-H]⁻= 412 | 4 |
| 48 | 1.361 | [M-H]⁻= 454 | 4 |
| 49 | 1.394 | [M-H]⁻= 468 | 4 |
| 50 | 1.324 | [M-H]⁻= 466 | 4 |
| 51 | 1.182 | [M-H]⁻=456 | 4 |
| 52 | 2.541 | [M+H]⁺=458 | 16C |
| 53 | 1.338 | [M+H]⁺=448 | 9 |
| 54 | 0.992 | [M+H]⁺=390 | 14C |
| 55 | 1.144 | [M+H]⁺=405 | 14C |
| 56 | 2.557 | [M+H]⁺=391 | 14B |
| 57 | 10.149 | [M+H]⁺=415 | 15 |
| 58 | 1.576 | [M+H]⁺=465 | 18 |
| 59 | 0.796 | [M+H]⁺=467 | 4 |
| 60 | 1.020 | [M+H]⁺=441 | 1 |
| 60-A | 0.933 | [M+H]⁺=423 | 4 |
| 61 | 1.191 | [M+Na]⁺=523 | 9 |
| 62 | 1.042 | [M+H]⁺=453 | 1 |
| 63 | 1.013 | [M+H]⁺=459 | 4 |
| 63-A | 2.290 | [M+H]⁺=459 | 11 |
| 64 | 1.410 | [M+H]⁺=491 | 1 |
| 65 | 2.657 | [M+Na]⁺=495 | 17 |
| 66 | 1.195 | [M+H]⁺=416 | 14C |
| 67 | 1.525 | [M+H]⁺=509 | 19 |
| 67-A | 1.105 | [M+H]⁺=509 | 21 |
| 68 | 1.452 | [M+H]⁺=493 | 19 |
| 68-A | 1.340 | [M+H]⁺=493 | 19 |
| 69 | 1.084 | [M+H]⁺=477 | 21 |
| 69-A | 0.988 | [M+H]⁺=477 | 21 |
| 70 | 1.428 | [M+H]⁺=473 | 19 |
| 70-A | 1.313 | [M+H]⁺=473 | 19 |
| 71 | 1.518 | [M+H]⁺=487 | 19 |
| 71-A | 1.392 | [M+H]⁺=487 | 19 |
| 72 | 1.286 | [M+H]⁺=513 | 21 |
| 72-A | 2.349 | [M+H]⁺=513 | 21 |
| 73 | 1.393 | [M+H]⁺=439 | 1 |
| 74 | 0.849 | [M+H]⁺=460 | 14D |
| 75 | 2.167 | [M+H]⁺=449 | 14B |
| 75A | 0.928 | [M+H]⁺=459 | 14D |
| 76 | 1.209 | [M+H]⁺=495 | 19 |
| 77 | 2.348 | [M+H]⁺=481 | 17 |
| 78 | 1.238 | [M+H]⁺=427 | 9 |
| 79 | 0.977 | [M+H]⁺=417 | 21 |
| 80 | 1.019 | [M+H]⁺=469 | 21 |
| 81 | 1.017 | [M+H]⁺=513 | 21 |
| 82 | 1.223 | [M+H]⁺=448 | 19 |
| 83 | 1.079 | [M+H]⁺=474 | 21 |
| 84 | 2.612 | [M+H]⁺=476 | 17 |
| 85 | 2.864 | [M+H]⁺=488 | 20 |
| 86 | 1.270 | [M+H]⁺=451 | 19 |
| 86-A | 1.268 | [M+H]⁺=451 | 19 |
| 86-B | 1.219 | [M+H]⁺=451 | 19 |
| 87-A | 1.462 | [M+H]⁺=543 | 20 |
| 87-B | 1.453 | [M+H]⁺=543 | 20 |
| 88-A | 0.910 | [M+H]⁺=453 | 20 |
| 89 | 2.872 | [M+H]⁺=465 | 13 |
| 90 | 9.502 | [M+H]⁺=442 | 15 |
| 91 | 11.305 | [M+H]⁺=498 | 15 |
| 92 | 10.622 | [M+H]⁺=494 | 15 |
| 93 | 2.276 | [M+H]+=463 | 21A |
| 94 | 10.309 | [M+H]⁺=477 | 15 |
| 95 | 1.231 | [M+H]⁺=449 | 12A |
| 96 | 9.653 | [M+H]⁻=463 | 15 |
| 97 | 1.352 | [M+H]⁺=501 | 19 |
| 98 | 10.265 | [M+H]⁺=513 | 15 |
| 99 | 1.195 | [M+H]⁺=513 | 21 |
| 100 | 1.188 | [M+H]⁺=513 | 21 |
| 101 | 1.448 | [M+H]⁺=463 | 19 |
| 102 | 0.973 | [M+H]⁺=467 | 20B |
| 103 | 2.502 | [M+H]⁺=467 | 20C |
| 104 | 1.647 | [M+H]⁺=462 | 24 |
| 105 | 1.712 | [M+H]⁺=462 | 24 |
| 106 | 1.416 | [M+H]⁺=473 | 19A |
| 107 | 9.809 | [M+H]⁺=499 | 15 |
| 108 | 0.842 | [M+H]⁺=511 | 21 |
| 109 | 0.727 | [M+H]⁺=511 | 21 |
| 110 | 0.86 | [M+H]⁺=511 | 21 |
| 111 | 0.751 | [M+H]⁺=511 | 21 |
| 112 | 1.341 | [M+H]⁺=487 | 5A |
| 113 | 1.34 | [M+H]⁺=487 | 19A |
| 114 | 1.146 | [M+H]⁺=515 | 5B |
| 115 | 1.023 | [M+H]⁺=515 | 21 |
| 116 | 2.964 | [M+H]⁺=507 | 13B |
| 117 | 2.709 | [M+H]⁺=507 | 5C |
| 118 | 2.733 | [M+H]⁺=525 | 13 |
| 119 | 1.257 | [M+H]⁺=525 | 19A |
| 120 | 2.733 | [M+H]⁺=525 | 13 |
| 121 | 1.257 | [M+H]⁺=525 | 19A |
| 122 | 1.208 | [M+H]⁺=499 | 19 |
| 123 | 1.098 | [M+H]⁺=499 | 19 |
| 124 | 0.94 | [M+H]⁺=499 | 21 |
| 125 | 1.941 | [M+H]⁺=499 | 18B |
| 126 | 2.381 | [M+H]⁺=499 | 22 |
| 127 | 0.731 | [M+H]⁺=499 | 21 |
| 128 | 0.941 | [M+H]⁺=510 | 21 |
| 129 | 0.844 | [M+H]⁺=510 | 21 |
| 130 | 2.283 | [M+H]⁺=499 | 21B |
| 131 | 1.842 | [M+H]⁺=499 | 21B |
| 132 | 1.003 | [M+H]⁺=510 | 21 |
| 133 | 0.906 | [M+H]⁺=510 | 21 |
| 134 | 1.196 | [M+H]⁺=510 | 19A |
| 135 | 10.362 | [M+H]⁺=500 | 15 |
| 136 | 9.674 | [M+H]⁺=500 | 15 |
| 137 | 7.539 | [M+H]⁺=459 | 15 |
| 138 | 7.52 | [M+H]⁺=459 | 15 |
| 139 | 10.135 | [M+H]⁺=458 | 15 |
| 140 | 9.48 | [M+H]⁺=458 | 15 |
| 141 | 6.874 | [M+H]⁺=459 | 15 |
| 142 | 6.669 | [M+H]⁺=459 | 15 |
| 143 | 8.458 | [M+H]⁺=446 | 15 |
| 144 | 1.124 | [M+H]⁺=469 | 21 |
| 145 | 9.305 | [M+H]⁺=472 | 15 |
| 146 | 0.831 | [M+H]⁺=474 | 14D |
| 147 | 0.870 | [M+H]⁺=474 | 14A |
| 148 | 9.338 | [M+H]⁺=414 | 15 |
| 149 | 8.28 | [M+H]⁺=421 | 15 |
| 150 | 8.915 | [M+H]⁺=432 | 15 |
| 151 | 8.195 | [M+H]⁺=404 | 15 |
| 152 | 8.695 | [M+H]⁺=389 | 15 |
| 153 | 9.801 | [M+H]⁺=428 | 15 |
| 154 | 1.963 | [M+H]⁺=456 | 14A |
| 155 | 9.865 | [M+H]⁺=499 | 15 |
| 156 | 1.418 | [M+H]⁺=499 | 19A |
| 157 | 0.740 | [M+H]⁺=405 | 14D |
| 158 | 1.239 | [M+H]⁺=485 | 23 |
| 159 | 1.533 | [M+H]⁺=466 | 19A |
| 160 | 0.696 | [M+H]⁺=491 | 21 |
| 161 | 0.705 | [M+H]⁺=490 | 20A |
| 162 | 0.681 | [M+H]⁺=404 | 14E |
| 163 | 1.125 | [M+H]⁺=487 | 21 |
| 164 | 1.122 | [M+H]⁺=487 | 21 |
| 165 | 0.945 | [M+H]⁺=485 | 19B |
| 166 | 1.373 | [M+H]⁺=485 | 19A |
| 167 | 2.345 | [M+H]⁺=465 | 21A |
| 168 | 1.092 | [M+H]⁺=465 | 21 |
| 169 | 1.041 | [M+H]⁺=453 | 21 |
| 170 | 1.046 | [M+H]⁺=453 | 21 |
| 171 | 1.036 | [M+H]⁺=390 | 14F |

### Example 99: Biological Assays

### THR biochemical assay

The TR-FRET thyroid receptor beta coactivator assay was used with slight, optimized modifications of the manufacturer's protocol (Invitrogen). The assay uses a terbium-labeled anti-GST antibody, a glutathione-S-transferase (GST) tagged human thyroid receptor, beta or alpha, ligand-binding domain (LBD), and a fluorescein labeled SRC2-2 coactivator peptide. The antibody interacts with the LBD, where the agonist also binds, resulting in increased affinity for the SRC2-2 coactivator peptide causing energy transfer of the acceptor fluorophore and a FRET emission shift from 495 to 520 nm. The energy transfer is detected as an increase in the fluorescence emission of the fluorescein acceptor, and a decrease in the fluorescence emission of the terbium donor. The assay is performed in a 384-well black plate in a final volume of 20 µL. Serial dilution of various test agonists was performed in DMSO (1% final DMSO concentration) and added to the test plate. Thyroid receptor beta LBD is added to the plate at a final concentration of 1 nM, followed by the mixture of the fluorescein labeled SRC2-2 coactivator peptide, and the terbium-labeled anti-GST antibody at final concentrations of 200 nM and 2 nM respectively. The assay is incubated for 1 hr at rt protected from light. The TR-FRET is then measured on a Victor multilabel reader (Perkin Elmer) using an excitation wavelength of 340 nm with emission filters of 495 nm and 520 nm. The assay is quantified by expressing a ratio (520:495) of the intensities, and the resulting activation curves; EC₅₀ values were generated using a sigmoidal dose response (variable slope) equation in GraphPad^{™} Prism 8.0.

### HEK293T reporter THRalpha/beta/RXR reporter assay (assay 2)

The purpose of this assay is to evaluate the effect of compounds on the thyroid hormone nuclear receptor pathway in HEK293T cells. To this end, HEK293T cells are transiently transfected with a luciferase reporter under the control of the thyroid response element (TRE), an RXR expression plasmid and either a THR alpha or THR beta expression plasmid. Transfected cells are stimulated with test compounds for 18-24 hours before activation of the thyroid hormone pathway is measured via a luciferase read-out.

Procedure. 24 hours prior to transfection, approximately 7 x10⁵ HEK293T (ATCC, catalog # CRL-3216) are plated in one well of a 6-well-plate using DMEM (Hyclone, catalog # SH30022) supplemented with 10% FBS (Gibco, catalog # 16000-044) and incubated overnight. Transfection complexes are prepared by mixing 12 µL of Lipofectamine 2000 (Invitrogen catalog # 11668019) with 4 µg of a plasmid mixture at a ratio of 1:1:4 (TRalpha or TRbeta: RXR: TRE-Luc) in 200 µL OptiMem (Invitrogen catalog # 11058-021) and added to the cells. After overnight incubation, transfected cells are re-seeded at (1×10⁴cells/well, 30µL/well) in a 384 microplate and incubated for an additional 5-6 hours. Ten five-fold dilutions of test compounds are prepared in DMSO and 30 nL are dispensed to the cells. Pure DMSO serves as negative control while T3 (MCE catalog # HY-A0070) and GC-1 (MCE catalog # HY-14823) are used as positive controls. Approximately 18-24 h after compound addition, 384 well plates are allowed to adjust to rt, 30 µL One-Glo (Promega catalog # E6120) is added to each well and luminescence is measured on a Perkin Elmer Enspire plate reader. Percent agonism is calculated using the following equation: 100x (sample-negative control)/ (positive control-negative control).

### Huh-7 Differential Gene Expression (assay 3)

### Serum Stripping

AG^{®} 1-X8 Anion Exchange Resin (analytical grade, 200-400 mesh, chloride form; 1401451, Bio-Rad) was pre-washed with distilled water three times; water was separated from resin via centrifugation. Fetal bovine serum (FBS) was incubated with washed resin (50 mg resin/mL FBS; resin weight is dry weight of resin prior to washing) for 5 hr at room temperature on a rotor. The FBS was separated from the resin via centrifugation and incubated with new, washed resin for 18h at room temperature on a rotor. The resin-treated FBS (rFBS) was separated via centrifugation and then sterilized via filtration (0.22 µM PES membrane).

### Cell culture and drug treatment

Huh-7 cells were cultured in DMEM (10-013-CM, Corning) supplemented with 10% FBS and 1% Pen-Strep at 37 °C under 5% CO2. When 70-80% confluence was reached, the cells were removed by trypsinization. The medium was aspirated from the cell culture dish, the cell monolayer was washed with 1X PBS, and 0.05% trypsin, 0.53 mM EDTA (25-052-CV, Corning) solution was added to the dish. After 3 min incubation, the cells were detached completely by repeatedly pipetting solution onto the monolayer. Equal volume of DMEM supplemented with 10% rFBS and 1% Pen-Strep (TH-depleted DMEM) was added to the dish to inactivate the trypsin. The cell suspension was centrifuged at 350 x g at room temperature for 3 min. The supernatant was aspirated out and the cell pellet was resuspended in TH-depleted DMEM. Cell density was quantified with a Vi-CELL XR Cell Viability Analyzer (Beckman Coulter) and cells were seeded onto Collagen I 96-well plates (356407, Corning) at 50,000 cells/well in 150 µL TH-depleted DMEM; the outer, perimeter wells were not used to avoid edge effect. After 24 hr incubation, the media was replaced with treatment media. All compounds were serially diluted in DMSO and final concentrations were reached by dilution in TH-depleted DMEM (0.1% DMSO). The cells were incubated in treatment media for 24 hr. Treatments were performed in biological duplicates.

### Cell lysis and RT-qPCR

After 24 hr in treatment media, the cells were lysed directly on the culture plates and cDNA was produced using the TaqMan^{™} Fast Advanced Cells-to-CT^{™} Kit (A35374, Invitrogen) and following the manufacturer's protocol. RT-qPCR for CPT1A (Hs00912671_m1) and two housekeeping genes, ACTB (Hs01060665_g1) and TFG (Hs02832013_g1), was performed using TaqMan^{™} Fast Advanced Master Mix. RT-qPCR reactions were run on the qTOWER³ 84 G (Analytik Jena) in technical duplicates.

### Data Analysis

ΔRn values were obtained from the qPCRsoft384 1.0 software and CPT1A gene expression was quantified via the 2-ΔΔCt method. Dose-response curves were generated using GraphPad Prism 8 using four parameter logistic equation without top constraint to derive EC₅₀ and Eₘₐₓ..

Compounds of Formula (I) are active as THR-beta agonists as shown in Table 3, where: for Assay 1: 'A' indicates an EC₅₀ < 50 nM, 'B' indicates an EC₅₀ of ≥50 nM and < 250 nM, 'C' indicates an EC₅₀ ≥ 250 nM and < 1000 nM, 'D' indicates an EC₅₀ ≥ 1000 nM and < 25000 nM, and 'E' indicates an EC₅₀ > 25000 nM. For Assay 2, 'A' indicates an EC₅₀ < 50 nM, 'B' indicates an EC₅₀ of ≥ 50 nM and < 250 nM, 'C' indicates an EC₅₀ ≥ 250 nM and < 1000 nM, 'D' indicates an EC₅₀ ≥ 1000 nM and < 10000 nM, and 'E' indicates an EC₅₀ > 10000 nM.

**Table 3**

| Compound number | Activity category | |
|---|---|---|
| | Assay 1 | Assay 2 |
| 1 | A | B |
| 2 | A | B |
| 3 | A | B |
| 4 | A | D |
| 5 | A | C |
| 6 | E | |
| 7 | E | |
| 8 | E | |
| 9 | D | |
| 10 | B | B |
| 11 | A | B |
| 12 | C | D |
| 13 | E | E |
| 14 | E | |
| 15 | E | E |
| 16 | B | B |
| 17 | A | D |
| 18 | A | D |
| 19 | A | D |
| 20 | E | E |
| 21 | B | E |
| 22 | E | |
| 23 | D | |
| 24 | E | |
| 25 | D | |
| 26 | E | |
| 27 | E | |
| 28 | B | C |
| 29 | C | C |
| 30 | E | |
| 31 | A | C |
| 32 | A | B |
| 33 | C | |
| 34 | D | |
| 35 | D | D |
| 35-A | B | E |
| 36 | A | A |
| 37 | B | B |
| 38 | C | C |
| 39-A | A | B |
| 39-B | A | A |
| 40-A | C | D |
| 40 | A | B |
| 41 | A | A |
| 42 | A | B |
| 43 | A | |
| 44 | B | E |
| 45 | A | B |
| 46 | A | D |
| 47 | A | D |
| 48 | A | B |
| 49 | A | C |
| 50 | A | B |
| 51 | D | E |
| 52 | B | E |
| 53 | A | C |
| 54 | A | A |
| 55 | A | A |
| 56 | A | B |
| 57 | A | B |
| 58 | A | B |
| 59 | D | D |
| 60 | C | D |
| 60-A | A | C |
| 61 | A | B |
| 61-A | A | A |
| 61-B | B | C |
| 62 | D | E |
| 63 | B | B |
| 63-A | E | E |
| 64 | A | B |
| 65 | A | B |
| 66 | B | D |
| 67 | B | B |
| 67-A | A | |
| 68 | A | A |
| 68-A | E | |
| 69 | B | B |
| 69-A | E | |
| 70 | A | A |
| 70-A | E | |
| 71 | A | A |
| 71-A | E | |
| 72 | B | B |
| 72-A | A | B |
| 73 | B | B |
| 74 | D | E |
| 75 | A | A |
| 75A | A | |
| 76 | D | C |
| 77 | B | B |
| 78 | C | |
| 79 | D | E |
| 80 | A | A |
| 81 | A | A |
| 82 | A | A |
| 83 | A | A |
| 84 | A | B |
| 85 | A | |
| 86-A | B | B |
| 86-B | B | C |
| 87-A | C | E |
| 87-B | E | |
| 88-A | D | E |
| 89 | A | |
| 90 | A | E |
| 91 | A | B |
| 91-A | A | B |
| 92 | B | B |
| 92-A | A | C |
| 93 | B | C |
| 94 | B | B |
| 95 | B | B |
| 96 | A | A |
| 97 | C | D |
| 98 | B | B |
| 99 | B | E |
| 100 | B | B |
| 101 | B | B |
| 102 | D | D |
| 103 | D | D |
| 104 | C | D |
| 105 | D | |
| 106 | C | D |
| 107 | A | B |
| 108 | E | |
| 109 | E | |
| 110 | E | |
| 111 | E | |
| 112 | B | B |
| 113 | B | C |
| 114 | B | C |
| 115 | B | C |
| 116 | B | B |
| 117 | B | E |
| 118 | A | A |
| 119 | D | E |
| 120 | A | B |
| 121 | B | B |
| 122 | D | |
| 123 | E | |
| 124 | D | E |
| 125 | D | |
| 126 | E | |
| 127 | E | |
| 128 | E | E |
| 129 | C | E |
| 130 | E | |
| 131 | D | |
| 132 | E | |
| 133 | C | |
| 134 | A | C |
| 135 | E | |
| 136 | D | |
| 137 | D | |
| 138 | E | |
| 139 | B | A |
| 140 | E | |
| 141 | D | |
| 142 | E | |
| 143 | A | A |
| 144 | B | C |
| 145 | A | D |
| 146 | A | A |
| 147 | A | A |
| 148 | A | D |
| 149 | A | A |
| 150 | A | A |
| 151 | A | A |
| 152 | A | A |
| 153 | A | |
| 154 | B | E |
| 155 | B | C |
| 156 | A | B |
| 157 | A | A |
| 158 | A | B |
| 159 | B | B |
| 160 | D | E |
| 161 | C | D |
| 162 | A | |
| 163 | E | |
| 164 | A | |
| 165 | A | |
| 166 | B | |
| 167 | B | |
| 168 | A | |
| 169 | A | |
| 170 | B | |

Compounds of Formula (I) may have activity as THR-alpha agonists as shown in Table 4, where: for Assay 1: 'A' indicates an EC₅₀ < 50 nM, 'B' indicates an EC₅₀ of ≥ 50 nM and < 250 nM, 'C' indicates an EC₅₀ ≥ 250 nM and < 1000 nM, 'D' indicates an EC₅₀ ≥ 1000 nM and < 25000 nM, and 'E' indicates an EC₅₀ ≥ 25000 nM. For Assay 2, 'A' indicates an EC₅₀ < 50 nM, 'B' indicates an EC₅₀ of ≥50 nM and < 250 nM, 'C' indicates an EC₅₀ ≥ 250 nM and < 1000 nM, 'D' indicates an EC₅₀ ≥ 1000 nM and < 10000 nM, and 'E' indicates an EC₅₀ > 10000 nM.

**Table 4**

| Compound number | Activity category | |
|---|---|---|
| | Assay 1 | Assay 2 |
| 1 | A | C |
| 2 | A | C |
| 3 | E | C |
| 4 | E | E |
| 5 | E | D |
| 6 | E | |
| 7 | E | |
| 8 | D | |
| 9 | E | |
| 10 | C | B |
| 11 | B | C |
| 12 | D | D |
| 13 | C | D |
| 14 | E | |
| 15 | E | E |
| 16 | C | C |
| 17 | E | E |
| 18 | E | D |
| 19 | E | D |
| 20 | E | E |
| 21 | E | E |
| 22 | E | |
| 23 | D | |
| 24 | E | |
| 25 | D | |
| 26 | E | |
| 27 | E | |
| 28 | C | D |
| 29 | D | C |
| 30 | E | |
| 31 | A | D |
| 32 | A | B |
| 33 | C | |
| 34 | E | |
| 35 | D | D |
| 35-A | C | B |
| 36 | B | A |
| 37 | C | B |
| 38 | D | E |
| 39-A | C | B |
| 39-B | B | A |
| 40-A | C | D |
| 40 | B | C |
| 41 | A | A |
| 42 | C | C |
| 43 | A | A |
| 44 | E | E |
| 45 | A | C |
| 46 | E | E |
| 47 | A | D |
| 48 | A | C |
| 49 | A | C |
| 50 | A | C |
| 51 | E | E |
| 52 | D | E |
| 53 | C | D |
| 54 | A | A |
| 55 | A | B |
| 56 | C | B |
| 57 | A | C |
| 58 | C | C |
| 59 | D | E |
| 60 | D | D |
| 60-A | B | C |
| 61 | C | C |
| 61-A | B | B |
| 61-B | D | C |
| 62 | D | E |
| 63 | C | C |
| 63-A | E | E |
| 64 | C | C |
| 65 | C | D |
| 66 | C | D |
| 67 | D | E |
| 67-A | E | D |
| 68 | B | B |
| 68-A | E | |
| 69 | C | B |
| 69-A | E | |
| 70 | B | B |
| 70-A | E | |
| 71 | B | B |
| 71-A | E | |
| 72 | E | E |
| 72-A | B | E |
| 73 | C | C |
| 74 | D | E |
| 75 | B | C |
| 75-A | B | E |
| 76 | E | E |
| 77 | C | C |
| 78 | D | |
| 79 | D | D |
| 80 | B | B |
| 81 | A | A |
| 82 | A | A |
| 83 | A | A |
| 84 | A | B |
| 85 | A | A |
| 86-A | C | C |
| 86-B | D | D |
| 87-A | C | E |
| 87-B | E | |
| 88-A | E | E |
| 89 | C | D |
| 90 | B | E |
| 91 | D | E |
| 91-A | D | E |
| 92 | D | E |
| 92-A | D | E |
| 93 | C | D |
| 94 | C | D |
| 95 | C | C |
| 96 | B | B |
| 97 | D | E |
| 98 | E | E |
| 99 | E | E |
| 100 | E | E |
| 101 | C | B |
| 102 | D | E |
| 103 | E | E |
| 104 | D | E |
| 105 | D | |
| 106 | D | D |
| 107 | D | C |
| 108 | E | |
| 109 | E | |
| 110 | E | |
| 111 | E | |
| 112 | C | D |
| 113 | C | E |
| 114 | E | E |
| 115 | E | E |
| 116 | E | E |
| 117 | E | E |
| 118 | B | B |
| 119 | E | E |
| 120 | E | E |
| 121 | C | E |
| 122 | D | |
| 123 | E | |
| 124 | D | E |
| 125 | E | |
| 126 | E | |
| 127 | E | |
| 128 | E | E |
| 129 | E | E |
| 130 | D | |
| 131 | D | |
| 132 | E | |
| 133 | E | |
| 134 | C | E |
| 135 | E | |
| 136 | D | |
| 137 | E | |
| 138 | E | |
| 139 | B | B |
| 140 | E | |
| 141 | E | |
| 142 | E | |
| 143 | A | A |
| 144 | C | D |
| 145 | A | D |
| 146 | B | B |
| 147 | A | A |
| 148 | A | D |
| 149 | A | A |
| 150 | A | A |
| 151 | A | A |
| 152 | A | A |
| 153 | A | |
| 154 | E | |
| 155 | E | E |
| 156 | B | D |
| 157 | C | B |
| 158 | C | D |
| 159 | C | D |
| 160 | D | E |
| 161 | C | E |
| 162 | B | |
| 163 | E | |
| 164 | B | C |
| 165 | C | |
| 166 | E | |
| 167 | D | |
| 168 | B | |
| 169 | C | |
| 170 | E | |

Compounds of Formula (I) have activity as THR-alpha/beta agonists as shown in Table 5, where: for Assays 1 and 2: 'C' indicates an Eₘₐₓ < 50%, 'B' indicates an Eₘₐₓ ≥50%, and < 75%, 'A' indicates an Eₘₐₓ ≥ 75%.

**Table 5**

| Compound number | Activity category | | | |
|---|---|---|---|---|
| | Assay 1 | | Assay 2 | |
| | THRα | THRβ | THRα | THRβ |
| 1 | C | B | A | A |
| 2 | C | A | A | A |
| 3 | C | B | A | A |
| 4 | C | C | B | A |
| 5 | C | C | B | A |
| 6 | C | C | | |
| 7 | C | C | | |
| 8 | C | C | | |
| 9 | C | C | | |
| 10 | B | B | A | A |
| 11 | C | C | A | A |
| 12 | B | A | A | A |
| 13 | C | C | A | A |
| 14 | C | C | | |
| 15 | C | C | C | C |
| 16 | C | B | A | A |
| 17 | C | C | C | A |
| 18 | C | C | B | A |
| 19 | C | C | C | A |
| 20 | C | C | C | C |
| 21 | C | C | C | C |
| 22 | C | C | | |
| 23 | C | C | | |
| 24 | C | C | | |
| 25 | B | B | | |
| 26 | C | C | | |
| 27 | C | C | | |
| 28 | B | C | B | A |
| 29 | B | B | A | A |
| 30 | A | B | | |
| 31 | C | B | A | A |
| 32 | B | A | A | A |
| 33 | C | A | | |
| 34 | C | C | | |
| 35 | B | B | C | B |
| 35-A | B | C | C | A |
| 36 | B | B | C | B |
| 37 | B | B | B | A |
| 38 | A | B | C | A |
| 39-A | B | C | A | A |
| 39-B | B | B | A | A |
| 40-A | C | C | C | A |
| 40 | B | B | A | A |
| 41 | B | B | B | A |
| 42 | C | B | A | A |
| 43 | A | A | A | A |
| 44 | C | C | C | C |
| 45 | C | C | A | A |
| 46 | C | C | C | B |
| 47 | C | C | A | A |
| 48 | B | B | A | A |
| 49 | C | C | C | A |
| 50 | B | B | A | A |
| 51 | A | C | C | C |
| 52 | C | C | B | A |
| 53 | C | B | A | A |
| 54 | C | B | B | A |
| 55 | B | B | A | A |
| 56 | C | C | B | A |
| 57 | C | C | A | A |
| 58 | C | C | B | A |
| 59 | C | C | B | A |
| 60 | C | C | A | A |
| 60-A | C | C | C | B |
| 61 | B | C | A | A |
| 61-A | B | B | B | A |
| 61-B | C | C | C | A |
| 62 | C | C | B | A |
| 63 | C | C | B | A |
| 63-A | C | C | C | C |
| 64 | C | C | C | B |
| 65 | C | C | C | A |
| 66 | C | B | B | A |
| 67 | C | C | C | B |
| 67-A | C | C | C | C |
| 68 | C | B | B | A |
| 68-A | C | C | | |
| 69 | C | C | B | B |
| 69-A | C | C | | |
| 70 | C | C | B | A |
| 70-A | C | C | | |
| 71 | C | C | B | A |
| 71-A | C | C | | |
| 72 | C | C | C | B |
| 72-A | C | C | C | B |
| 73 | B | C | A | A |
| 74 | C | B | C | C |
| 75 | A | A | A | A |
| 75-A | C | B | C | B |
| 76 | C | C | C | B |
| 77 | B | C | A | A |
| 78 | C | C | | |
| 79 | C | B | B | A |
| 80 | B | B | A | A |
| 81 | B | B | A | A |
| 82 | A | A | A | A |
| 83 | B | B | A | B |
| 84 | B | C | A | A |
| 85 | C | C | B | A |
| 86-A | C | C | B | A |
| 86-B | C | C | C | A |
| 87-A | C | C | C | C |
| 87-B | C | C | | |
| 88-A | C | C | A | C |
| 89 | C | C | C | B |
| 90 | C | C | C | B |
| 91 | B | C | C | B |
| 91-A | C | C | C | B |
| 92 | C | C | C | C |
| 92-A | B | C | C | C |
| 93 | C | C | C | B |
| 94 | C | C | C | B |
| 95 | C | C | C | A |
| 96 | C | C | B | A |
| 97 | C | C | C | C |
| 98 | C | C | C | C |
| 99 | A | C | C | C |
| 100 | C | C | C | C |
| 101 | C | C | C | B |
| 102 | C | C | C | C |
| 103 | C | C | C | C |
| 104 | B | C | C | B |
| 105 | C | C | | |
| 106 | C | C | C | B |
| 107 | C | C | C | B |
| 108 | C | C | | |
| 109 | C | C | | |
| 110 | C | C | | |
| 111 | C | C | | |
| 112 | C | C | C | C |
| 113 | C | C | C | C |
| 114 | C | C | C | C |
| 115 | C | C | C | C |
| 116 | C | C | C | C |
| 117 | C | C | C | C |
| 118 | C | B | B | B |
| 119 | C | C | C | C |
| 120 | C | C | C | B |
| 121 | C | C | C | B |
| 122 | C | C | | |
| 123 | C | C | | |
| 124 | C | C | C | C |
| 125 | C | C | | |
| 126 | C | C | | |
| 127 | C | C | | |
| 128 | C | C | C | C |
| 129 | C | C | C | C |
| 130 | C | C | | |
| 131 | C | C | | |
| 132 | C | C | | |
| 133 | C | C | | |
| 134 | C | C | C | B |
| 135 | C | C | | |
| 136 | C | C | | |
| 137 | C | C | | |
| 138 | C | C | | |
| 139 | C | C | C | B |
| 140 | C | C | | |
| 141 | C | C | | |
| 142 | C | C | | |
| 143 | A | B | A | A |
| 144 | C | C | B | B |
| 145 | B | C | B | B |
| 146 | C | C | C | B |
| 147 | C | B | A | A |
| 148 | B | C | C | B |
| 149 | C | C | A | A |
| 150 | C | C | B | B |
| 151 | A | B | B | B |
| 152 | B | C | B | B |
| 153 | C | C | | |
| 154 | C | C | | C |
| 155 | C | C | C | C |
| 156 | C | C | C | B |
| 157 | B | A | B | A |
| 158 | C | C | C | B |
| 159 | C | C | C | B |
| 160 | C | C | C | C |
| 161 | C | C | C | C |
| 162 | C | B | | |
| 163 | C | C | | |
| 164 | C | C | B | A |
| 165 | C | C | | |
| 166 | C | C | | |
| 167 | C | C | | |
| 168 | B | C | | |
| 169 | C | C | | |
| 170 | C | C | | |

Compounds of Formula (I) have activity as THR agonists as shown in Table 6, where: for Assay 3 in the EC₅₀ column: 'A' indicates an EC₅₀ < 100 nM, 'B' indicates an EC₅₀ of ≥100 nM and < 1000 nM, 'C' indicates an EC₅₀ ≥ 1000nM. In the Eₘₐₓ column, 'C' indicates an Eₘₐₓ < 50%, 'B' indicates an Eₘₐₓ ≥ 50%, and < 75%, 'A' indicates an Eₘₐₓ ≥ 75%.

**Table 6**

| Compound | Activity category | |
|---|---|---|
| | Assay 3 | |
| | EC₅₀ | Eₘₐₓ |
| Reference (T3) | A | A |
| 1 | B | A |
| 10 | A | B |
| 11 | A | A |
| 13 | C | A |
| 16 | B | C |
| 18 | C | A |
| 31 | B | A |
| 39-A | A | B |
| 39-B | A | A |
| 40 | A | A |
| 42 | A | B |
| 43 | A | A |
| 52 | C | A |
| 54 | A | A |
| 55 | A | A |
| 61-A | A | B |
| 61-B | C | A |
| 67 | C | A |
| 67-A | B | C |
| 70 | A | B |
| 72 | B | A |
| 72-A | B | A |
| 75-A | C | A |
| 83 | A | A |
| 86-A | A | B |
| 89 | B | A |
| 90 | C | B |
| 93 | A | B |
| 94 | B | B |
| 101 | A | C |
| 112 | B | A |
| 114 | B | B |
| 115 | C | A |
| 120 | B | A |
| 121 | B | B |
| 145 | B | A |
| 146 | B | A |
| 148 | B | A |
| 151 | A | A |
| 157 | A | A |
| 158 | C | C |
| 164 | A | B |

### Example 100: X-Ray Diffraction Data

The chemical structures of compounds 67 and 67-A were confirmed by x-ray crystallography as described below.

**Experimental.** Single colorless chunk crystals of compound 67 recrystallized from a mixture of THF and acetonitrile by slow evaporation. A suitable crystal with dimensions 0.39 × 0.20 × 0.10 mm3 was selected and mounted on a nylon loop with paratone oil on a Bruker APEX-II CCD diffractometer. The crystal was kept at a steady T = 173(2) K during data collection. The structure was solved with the XT (Sheldrick, 2015) solution program using dual methods and by using OLEX2 as the graphical interface. The model was refined with ShelXL 2018/3 (Sheldrick, 2015) using full matrix least squares minimization on*F*2.

**Crystal Data.** C₂₉H₂₅Cl₂N₇O₅, *Mᵣ* = 622.46, triclinic, *P*-1 (No. 2), a = 8.91530(10) Å, b = 10.93240(10) Å, c = 16.2174(2) Å, α = 75.7870(10)°, β = 83.5020(10)°, γ = 67.6090(10)°, *V* = 1416.39(3) Å³, *T =* 173(2) K, *Z* = 2, Z' = 1, µ(CuK_{α}) = 2.518, 22070 reflections measured, 5231 unique (*Rint =* 0.0434) which were used in all calculations. The final wR2 was 0.1228 (all data) and *R1* was 0.0445 (I > 2(I)).

**Experimental.** Single colorless block crystals of compound 67-A recrystallized from THF by slow evaporation. A suitable crystal with dimensions 0.14 × 0.07 × 0.04 mm3 was selected and mounted on a nylon loop with paratone oil on a Bruker APEX-II CCD diffractometer. The crystal was kept at a steady *T* = 173(1) K during data collection. The structure was solved with the ShelXT (Sheldrick, G.M. (2015). Acta Cryst. A71, 3-8) solution program using dual methods and by using OLEX2 as the graphical interface. The model was refined with ShelXL (Sheldrick, Acta Cryst. A64 2008, 112-122) using full matrix least squares minimization on F2.

**Crystal Data.** C₂₇H₂₂Cl₂N₆O₅, *Mᵣ* = 581.40, monoclinic, *C*2/c (No. 15), a = 38.956(17) Å, b = 8.001(3) Å, c = 23.093(13) Å, β = 125.580(12)°, α = γ = 90°, *V =* 5854(5) Å³, *T=* 173(1) K, Z = 8, Z' = 1, µ(CuK_{α}) = 2.389, 44732 reflections measured, 5360 unique (*Rint* = 0.1596) which were used in all calculations. The final wR2 was 0.3362 (all data) and *R1* was 0.1018 (I > 2(I)).

### Example 101: Comparison of Compounds 42, 40, and 10 with -CN counterparts.

Compounds 42, 40, and 10 have R₉ = NH₂. These compounds were compared to direct analogs wherein R₉ = CN, in the HEK293T reporter THRalpha/beta/RXR reporter assay.

**Table 7. HEK293T reporter THRalpha/beta/RXR reporter assay data**

| | Compd 42 | CN analog 1 | Compd 40 | CN analog 2 | Compd 10 | CN analog 3 |
|---|---|---|---|---|---|---|
| EC₅₀ α (µM) | 0.834 (87%) | >10 | 0.564 (95%) | 9.65 (35%) | 0.191 (79%) | 16.9 (62%) |
| EC₅₀ β (µM) | 0.077 (94%) | >10 | 0.058 (92%) | 3.78 (81%) | 0.050 (91%) | 2.37 (93%) |
| Ratio (α/β) | 11 (n = 5) | -- | 10 (n = 3) | 2.5 (n = 1) | 4 (n = 7) | >4 (n =4) |

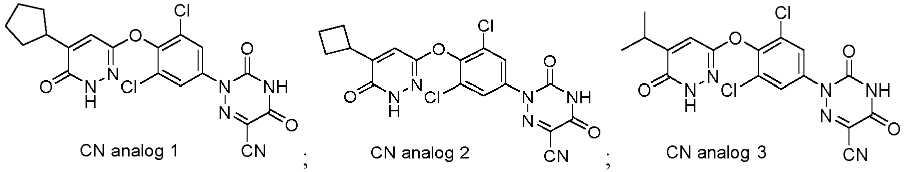

References:
1. Younossi, Z.M., Koenig, A.B., Abdelatif, D., Fazel, Y., Henry, L., Wymer, M., 2016. Global epidemiology of nonalcoholic fatty liver disease-Meta-analytic assessment of prevalence, incidence, and outcomes. Hepatology 64(1): 73e84.
2. Gastroenterology. 2012 Jun;142(7): 1592-609. doi: 10.1053/j.gastro.2012.04.001. Epub 2012 May 15.
3. Serfaty, L., Lemoine, M., 2008. Definition and natural history of metabolic steatosis: clinical aspects of NAFLD, NASH and cirrhosis. Diabetes and Metabolism 34 (6 Pt 2): 634e637.
4. Hepatology. 2012 Oct; 56(4): 1580-1584. doi: 10.1002/hep.26031
5. Dulai, P.S., Singh, S., Patel, J., Soni, M., Prokop, L.J., Younossi, Z., et al., 2017. Increased risk of mortality by fibrosis stage in nonalcoholic fatty liver disease: systematic review and meta-analysis. Hepatology 65(5): 1557e1565.
6. Younossi, Z.M., Loomba, R., Rinella, M.E., Bugianesi, E., Marchesini, G., Neuschwander-Tetri, B.A., et al., 2018. Current and future therapeutic regimens for non-alcoholic fatty liver disease (NAFLD) and non-alcoholic steatohepatitis (NASH). Hepatology 68(1): 349e360.
7. Thyroid. 2002 Jun;12(6): 441-6. Mechanism of thyroid hormone action. Harvey CB, Williams GR.
8. A.L. Bookout, Y. Jeong, M. Downes, R.T. Yu, R.M. Evans, D.J. Mangelsdorf Anatomical profiling of nuclear receptor expression reveals a hierarchical transcriptional network. Cell, 126 (2006), pp. 789-799
9. F. Flamant, J.D. Baxter, D. Forrest, S. Refetoff, H.H. Samuels, T.S. Scanlan, B. Vennstrom, J. Samarut International union of pharmacology. LIX. The pharmacology and classification of the nuclear receptor superfamily: thyroid hormone receptors. Pharmacol. Rev., 58 (2006), pp. 705-711
10. Bioorg Med Chem Lett. 2005 Apr 1;15(7): 1835-40. Novel heterocyclic thyromimetics. Haning H1, Woltering M, Mueller U, Schmidt G, Schmeck C, Voehringer V, Kretschmer A, Pernerstorfer J.
11. Expert Opin Ther Pat. 2010 Feb;20(2): 213-28. doi: 10.1517/13543770903567069. Thyromimetics: a review of recent reports and patents (2004 - 2009). Hirano T1, Kagechika H.
12. Front Endocrinol (Lausanne). 2018 Jul 10;9: 382. doi: 10.3389/fendo.2018.00382. eCollection 2018. Thyroid Hormones, Thyromimetics and Their Metabolites in the Treatment of Liver Disease. Kowalik MA, Columbano A, Perra A
13. Proc Natl Acad Sci U S A. 2007 Sep 25;104(39): 15490-5. Epub 2007 Sep 18.Targeting thyroid hormone receptor-beta agonists to the liver reduces cholesterol and triglycerides and improves the therapeutic index. Erion MD1, Cable EE, Ito BR, Jiang H, Fujitaki JM, Finn PD, Zhang BH, Hou J, Boyer SH, van Poelje PD, Linemeyer DL.

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of" will be understood to include those elements specifically recited and those additional elements that do not materially affect the basic and novel characteristics of the claimed technology. The phrase "consisting of' excludes any element not specified.

Where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

## Claims

1. A compound of Formula I':
(I') TL-Lₐ-CE-HD
or a stereoisomer or a tautomer thereof, or a pharmaceutically acceptable salt thereof, wherein:
i) TL is a moiety of Formula IIIb: 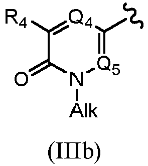 wherein:
Q₄ is -CH- and Q₅ is nitrogen;
R₄ is an optionally substituted alkyl, an optionally substituted alkenyl, an optionally substituted non-aromatic carbocyclic group, an optionally substituted aryl group, an optionally substituted heterocyclic group, an optionally substituted heteroaryl group, an optionally substituted (carbocyclic)alkyl group, an optionally substituted aralkyl group, an optionally substituted (heterocycloalkyl)alkyl group, an optionally substituted (heteroaryl)alkyl group, an optionally substituted amino group, an optionally substituted sulfamoyl group, an optionally substituted carbamoyl group, or an optionally substituted carbamoyl alkyl group, where the nitrogen of the carbamoyl or carbamoyl alkyl group is optionally a heteroatom in a ring structure; and
Alk is hydrogen or an optionally substituted alkyl;
ii) CE is a moiety of Formula IV 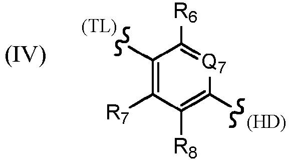 wherein:
each of R₆ and R₇ is independently selected from halogen, -CN, optionally substituted lower alkyl, optionally substituted lower alkoxy, optionally substituted lower alkenyl, or cyclopropyl;
R₈ is selected from hydrogen, optionally substituted lower alkyl, optionally substituted lower alkoxy, cyano, or halogen;
optionally R₇ and R₈ taken together, along with the carbon atoms to which they are attached, form a 4-, 5- or 6-membered non-aromatic carbocyclic, heterocycloalkyl, aryl, or heteroaryl ring;
Q₇ is nitrogen or -CR_{c}-, wherein R_{c} is hydrogen, halogen, or lower alkyl;
(TL) denotes the point where the moiety of Formula IV connects to TL-Lₐ-; and
(HD) denotes the point where the moiety of Formula IV connects to -HD;
iii) HD is a moiety of Formula V: 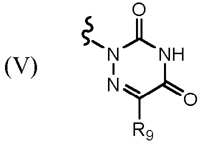 wherein:
R₉ is -NH₂; wherein
each Rₐ is independently hydrogen or optionally substituted lower alkyl; and
each n is independently selected from 0, 1, 2, 3, 4, or 5;
Lₐ is independently a bond; -(C(Rₐ)₂)_{z}-; oxygen; sulfur; or -NRₐ-; wherein:
each Rₐ is independently a hydrogen or lower alkyl; and
z is 0, 1, 2, 3, 4 or 5.

2. The compound of claim 1, or a stereoisomer or a tautomer thereof, or a pharmaceutically acceptable salt thereof, wherein R₄ is C₁-C₆ alkyl; C₂-C₁₀ alkenyl; a non-aromatic C₃-C₁₂ carbocyclic ring; a C₆-C₁₀ aryl group; a 3- to 6-membered heterocycloalkyl ring containing one to four ring heteroatoms independently selected from oxygen, sulfur, or nitrogen; a five- to ten-membered heteroaryl ring containing one to four ring heteroatoms independently selected from oxygen, sulfur, or nitrogen; a (carbocyclic)alkyl group; an aralkyl group; or a (heterocycloalkyl)alkyl group; and R₄ is optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

3. The compound of claim 1 or claim 2, or a stereoisomer or a tautomer thereof, or a pharmaceutically acceptable salt thereof, wherein R₄ is C₁-C₆ alkyl, C₂-C₁₀ alkenyl, cyclopropyl, cyclobutyl, cyclopentyl, cyclohexyl, tetrahydrofuranyl, tetrahydropyranyl, azetidinyl, pyridazin-3(2H)-one, phenyl, naphthyl, pyridinyl, cinnolinyl, isoquinolinyl, quinolinyl, pyrazolo[1,5-a]pyridinyl, imidazo[1,5-a]pyridinyl, benzo[b]thiophenyl, a (cyclobutyl)alkyl group, a (cyclopentyl)alkyl group, a benzyl group, a (tetrahydrofuranyl)alkyl group, or a (tetrahydropyranyl)alkyl group; and R₄ is optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

4. The compound of claim 1, or a stereoisomer or a tautomer thereof, or a pharmaceutically acceptable salt thereof, wherein R₄ is C₁-C₆ alkyl or a non-aromatic C₃-C₁₂ carbocyclic ring; and R₄ is optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy.

5. The compound of claim 1, or a stereoisomer or a tautomer thereof, or a pharmaceutically acceptable salt thereof, wherein R₄ is C₁-C₆ alkyl optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy.

6. The compound of claim 1, or a stereoisomer or a tautomer thereof, or a pharmaceutically acceptable salt thereof, wherein R₄ is a non-aromatic C₃-C₁₂ carbocyclic ring optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy.

7. The compound of claim 1, or a stereoisomer or a tautomer thereof, or a pharmaceutically acceptable salt thereof, wherein R₄ is a C₆-C₁₀ aryl group or a five- to ten-membered heteroaryl ring containing one to four ring heteroatoms independently selected from oxygen, sulfur, or nitrogen; and R₄ is optionally substituted with one to five R_{g} independently selected from the group consisting of hydroxy, halogen, CN, C₁-C₆ alkyl, C₆-C₁₀ aryl, C₁-C₆ alkoxy, C₁-C₆ haloalkoxy, and C₆-C₁₀ aralkoxy, or two R_{g} together with the atoms to which they are attached form an aromatic or non-aromatic 3- to 6-membered ring, optionally containing one or two ring heteroatoms independently selected from oxygen, sulfur, or nitrogen.

8. The compound of claim 1, or a stereoisomer or a tautomer thereof, or a pharmaceutically acceptable salt thereof, wherein R₄ is benzene optionally substituted with one to five R_{g} independently selected from the group consisting of hydroxy, halogen, CN, C₁-C₆ alkyl, C₁-C₆ alkoxy, and C₁-C₆ haloalkoxy, or two R_{g} together with the atoms to which they are attached form a ring, or two R_{g} together with the atoms to which they are attached form an aromatic or non-aromatic 3- to 6-membered ring, optionally containing one or two ring heteroatoms independently selected from oxygen, sulfur, or nitrogen.

9. The compound of any one of claims 1-8, or a stereoisomer or a tautomer thereof, or a pharmaceutically acceptable salt thereof, wherein Lₐ is oxygen or -CH₂-.

10. The compound of any one of claims 1-9, or a stereoisomer or a tautomer thereof, or a pharmaceutically acceptable salt thereof, wherein Q₇ is -CH-.

11. The compound of any one of claims 1-10, or a stereoisomer or a tautomer thereof, or a pharmaceutically acceptable salt thereof, wherein
each of R₆ and R₇ is independently halogen or C₁-C₅ alkyl optionally substituted with one to five substituents independently selected from hydroxy, halogen, and C₁-C₆ alkoxy; and R₈ is hydrogen; or
R₆ is halogen or C₁-C₅ alkyl optionally substituted with one to five substituents independently selected from hydroxy, halogen, and C₁-C₆ alkoxy; and R₇ and R₈ taken together, along with the carbon atoms to which they are attached, form a 4-, 5- or 6-membered carbocyclic ring.

12. A pharmaceutical composition comprising the compound of any one of claims 1-11, or the stereoisomer or the tautomer thereof, or the pharmaceutically acceptable salt thereof, and at least one pharmaceutically acceptable excipient.

13. A compound of any one of claims 1-11, or a stereoisomer or a tautomer thereof, or a pharmaceutically acceptable salt thereof, for use in treating a disorder or disease selected from non-alcoholic steatohepatitis (NASH), obesity, hyperlipidemia, hypercholesterolemia, diabetes, liver steatosis, atherosclerosis, cardiovascular diseases, hypothyroidism, and thyroid cancer.

14. A composition of claim 12 for use in treating a disorder or disease selected from non-alcoholic steatohepatitis (NASH), obesity, hyperlipidemia, hypercholesterolemia, diabetes, liver steatosis, atherosclerosis, cardiovascular diseases, hypothyroidism, and thyroid cancer.

15. A compound of any one of claims 1-11, or a stereoisomer or a tautomer thereof, or a pharmaceutically acceptable salt thereof, for use in selectively modulating the activity of a thyroid hormone receptor beta (THR-β).

16. A composition of claim 12 for use in selectively modulating the activity of a thyroid hormone receptor beta (THR-β).

## Patentansprüche

1. Verbindung der Formel I ':
(I') TL-Lₐ₋CE-HD
oder ein Stereoisomer oder ein Tautomer davon oder ein pharmazeutisch akzeptables Salz davon, wobei:
i) TL eine Einheit der Formel Illb ist: wobei: 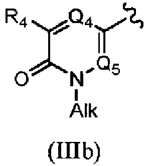
Q₄ -CH- ist und Q₅ Stickstoff ist;
R₄ ist ein optional substituiertes Alkyl, ein optional substituiertes Alkenyl, eine optional substituierte nicht-aromatische carbocyclische Gruppe, eine optional substituierte Aryl-Gruppe, eine optional substituierte heterocyclische Gruppe, eine optional substituierte Heteroaryl-Gruppe, eine optional substituierte (carbocyclische)Alkyl-Gruppe, eine optional substituierte Aralkyl-Gruppe, eine optional substituierte (Heterocycloalkyl)alkyl-Gruppe, eine optional substituierte (Heteroaryl)alkyl-Gruppe, eine optional substituierte Aminogruppe, eine optional substituierte Sulfamoyl-Gruppe, eine optional substituierte Carbamoyl-Gruppe oder eine optional substituierte Carbamoylalkyl-Gruppe, wobei der Stickstoff der Carbamoyl- oder Carbamoylalkyl-Gruppe optional ein Heteroatom in einer Ringstruktur ist; und
Alk Wasserstoff oder ein optional substituiertes Alkyl ist;
ii) CE eine Einheit der Formel IV ist 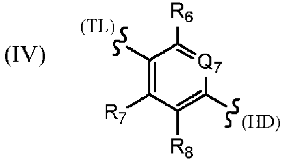 wobei:
jedes von R₆ und R₇ unabhängig ausgewählt ist aus Halogen, -CN, optional substituiertem Niederalkyl, optional substituiertem Niederalkoxy, optional substituiertem Niederalkenyl oder Cyclopropyl;
R₈ ausgewählt ist aus Wasserstoff, optional substituiertem Niederalkyl, optional substituiertem Niederalkoxy, Cyano oder Halogen;
optional bilden R₇ und R₈ zusammen, zusammen mit den Kohlenstoffatomen, an die sie gebunden sind, einen 4-, 5- oder 6-gliedrigen nicht-aromatischen carbocyclischen, Heterocycloalkyl-, Aryl- oder Heteroarylring;
Q₇ Stickstoff oder -CR_{c}- ist, wobei R_{c} Wasserstoff, Halogen oder Niederalkyl ist;
(TL) den Punkt bezeichnet, an dem die Einheit der Formel IV an TL-Lₐ₋ gebunden ist; und
(HD) den Punkt bezeichnet, an dem die Einheit der Formel IV an -HD gebunden ist;
iii) HD eine Einheit der Formel V ist: 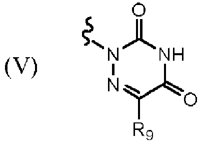 wobei:
R₉ ist -NH₂; wobei
jedes R_{d} unabhängig Wasserstoff oder optional substituiertes Niederalkyl ist; und
jedes n unabhängig ausgewählt ist aus 0, 1, 2, 3, 4 oder 5;
Lₐ unabhängig eine Bindung; -(C(Rₐ)₂)_{z}-; Sauerstoff; Schwefel; oder -NRₐ₋ ist; wobei:
jedes Rₐ unabhängig ein Wasserstoff oder Niederalkyl ist; und
z 0, 1, 2, 3, 4 oder 5 ist.

2. Verbindung nach Anspruch 1, oder ein Stereoisomer oder ein Tautomer davon oder ein pharmazeutisch akzeptables Salz davon, wobei R₄ C₁-C₆-Alkyl; C₂-C₁₀-Alkenyl; ein nicht-aromatischer C₃-C₁₂-carbocyclischer Ring ist; eine C₆-C₁₀-Arylgruppe; ein 3- bis 6-gliedriger Heterocycloalkylring, der ein bis vier Ringheteroatome enthält, die unabhängig ausgewählt sind aus Sauerstoff, Schwefel oder Stickstoff; ein fünf- bis zehngliedriger Heteroarylring, der ein bis vier Ringheteroatome enthält, die unabhängig ausgewählt sind aus Sauerstoff, Schwefel oder Stickstoff; eine (carbocyclische)Alkylgruppe; eine Aralkylgruppe; oder eine (Heterocycloalkyl)Alkylgruppe; und R₄ optional substituiert ist mit einem bis fünf R_{g}, die unabhängig ausgewählt sind aus der Gruppe bestehend aus Hydroxy, Halogen, CN, C₁-C₆-Alkyl, C₆-C₁₀-Aryl, C₁-C₆-Alkoxy, C₁-C₆-Haloalkoxy und C₆-C₁₀-Aralkoxy, oder zwei R_{g} zusammen mit den Atomen, an die sie gebunden sind, einen aromatischen oder nicht-aromatischen 3- bis 6-gliedrigen Ring bilden, der optional ein oder zwei Ringheteroatome enthält, die unabhängig ausgewählt sind aus Sauerstoff, Schwefel oder Stickstoff.

3. Verbindung nach Anspruch 1 oder Anspruch 2, oder ein Stereoisomer oder ein Tautomer davon oder ein pharmazeutisch akzeptables Salz davon, wobei R₄ C₁-C₆-Alkyl, C₂-C₁₀-Alkenyl, Cyclopropyl, Cyclobutyl, Cyclopentyl, Cyclohexyl, Tetrahydrofuranyl, Tetrahydropyranyl, Azetidinyl, Pyridazin- 3(2H)-on, Phenyl, Naphthyl, Pyridinyl, Cinnolinyl, Isoquinolinyl, Quinolinyl, Pyrazolo[1,5-a]pyridinyl, Imidazo[1,5-a] pyridinyl, Benzo[b]thiophenyl, eine (Cyclobutyl)Alkylgruppe, eine (Cyclopentyl)Alkylgruppe, eine Benzylgruppe, eine (Tetrahydrofuranyl)Alkylgruppe oder eine (Tetrahydropyranyl)Alkylgruppe ist; und R₄ optional substituiert ist mit einem bis fünf R_{g}, die unabhängig ausgewählt sind aus der Gruppe bestehend aus Hydroxy, Halogen, CN, C₁-C₆-Alkyl, C₆-C₁₀-Aryl, C₁-C₆-Alkoxy, C₁-C₆-Haloalkoxy und C₆-C₁₀-Aralkoxy, oder zwei R_{g} zusammen mit den Atomen, an die sie gebunden sind, einen aromatischen oder nicht-aromatischen 3- bis 6-gliedrigen Ring bilden, der optional ein oder zwei Ringheteroatome enthält, die unabhängig ausgewählt sind aus Sauerstoff, Schwefel oder Stickstoff.

4. Verbindung nach Anspruch 1, oder ein Stereoisomer oder ein Tautomer davon oder ein pharmazeutisch akzeptables Salz davon, wobei R₄ C₁-C₆-Alkyl oder ein nicht-aromatischer C₃-C₁₂-carbocyclischer Ring ist; und R₄ optional substituiert ist mit einem bis fünf R_{g}, die unabhängig ausgewählt sind aus der Gruppe bestehend aus Hydroxy, Halogen, CN, C₁-C₆-Alkyl, C₆-C₁₀-Aryl, C₁-C₆-Alkoxy, C₁-C₆-Haloalkoxy und C₆-C₁₀-Aralkoxy.

5. Verbindung nach Anspruch 1, oder ein Stereoisomer oder ein Tautomer davon oder ein pharmazeutisch akzeptables Salz davon, wobei R₄ ein C₁-C₆-Alkyl ist, das optional mit einem bis fünf R_{g} substituiert ist, die unabhängig ausgewählt sind aus der Gruppe bestehend aus Hydroxy, Halogen, CN, C₁-C₆-Alkyl, C₆-C₁₀-Aryl, C₁-C₆-Alkoxy, C₁-C₆-Haloalkoxy und C₆-C₁₀-Aralkoxy.

6. Verbindung nach Anspruch 1, oder ein Stereoisomer oder ein Tautomer davon oder ein pharmazeutisch akzeptables Salz davon, wobei R₄ ein nicht-aromatischer C₃-C₁₂ - carbocyclischer Ring ist, der optional mit einem bis fünf R_{g} substituiert ist, die unabhängig ausgewählt sind aus der Gruppe bestehend aus Hydroxy, Halogen, CN, C₁-C₆-Alkyl, C₁-C₁₀-Aryl, C₁-C₆-Alkoxy, C₁-C₆-Haloalkoxy und C₆-C₁₀-Aralkoxy.

7. Verbindung nach Anspruch 1, oder ein Stereoisomer oder ein Tautomer davon oder ein pharmazeutisch akzeptables Salz davon, wobei R₄ eine C₆-C₁₀-Arylgruppe oder ein fünfbis zehngliedriger Heteroarylring ist, der ein bis vier Ringheteroatome enthält, die unabhängig ausgewählt sind aus Sauerstoff, Schwefel oder Stickstoff; und R4 optional substituiert ist mit einem bis fünf R_{g}, die unabhängig ausgewählt sind aus der Gruppe bestehend aus Hydroxy, Halogen, CN, C₁-C₆-Alkyl, C₆-C₁₀-Aryl, C₁-C₆-Alkoxy, C₁-C₆-Haloalkoxy und C₆-C₁₀-Aralkoxy, oder zwei R_{g} zusammen mit den Atomen, an die sie gebunden sind, einen aromatischen oder nicht-aromatischen 3- bis 6-gliedrigen Ring bilden, der optional ein oder zwei Ringheteroatome enthält, die unabhängig ausgewählt sind aus Sauerstoff, Schwefel oder Stickstoff.

8. Verbindung nach Anspruch 1, oder ein Stereoisomer oder ein Tautomer davon oder ein pharmazeutisch akzeptables Salz davon, wobei R₄ Benzol ist, das optional mit einem bis fünf R_{g} substituiert ist, die unabhängig ausgewählt sind aus der Gruppe bestehend aus Hydroxy, Halogen, CN, C₁-C₆-Alkyl, C₁-C₆-Alkoxy und C₁-C₆-Haloalkoxy, oder zwei R_{g} zusammen mit den Atomen, an die sie gebunden sind, einen Ring bilden, oder zwei R_{g} zusammen mit den Atomen, an die sie gebunden sind, einen aromatischen oder nicht-aromatischen 3- bis 6-gliedrigen Ring bilden, der optional ein oder zwei Ringheteroatome enthält, die unabhängig ausgewählt sind aus Sauerstoff, Schwefel oder Stickstoff.

9. Verbindung nach einem der Ansprüche 1-8, oder ein Stereoisomer oder ein Tautomer davon oder ein pharmazeutisch akzeptables Salz davon, wobei Lₐ Sauerstoff oder -CH₂- ist.

10. Verbindung nach einem der Ansprüche 1-9, oder ein Stereoisomer oder ein Tautomer davon oder ein pharmazeutisch akzeptables Salz davon, wobei Q₇ -CH- ist.

11. Verbindung nach einem der Ansprüche 1-10, oder ein Stereoisomer oder ein Tautomer davon oder ein pharmazeutisch akzeptables Salz davon, wobei
jedes von R₆ und R₇ unabhängig Halogen oder C₁-C₅-Alkyl ist, das optional mit einem bis fünf Substituenten substituiert ist, die unabhängig ausgewählt sind aus Hydroxy, Halogen und C₁-C₆-Alkoxy; und R₈ Wasserstoff ist; oder
R₆ Halogen oder C₁-C₅-Alkyl ist, das optional mit einem bis fünf Substituenten substituiert ist, die unabhängig ausgewählt sind aus Hydroxy, Halogen und C₁-C₆-Alkoxy; und R₇ und R₈ zusammen, zusammen mit den Kohlenstoffatomen, an die sie gebunden sind, einen 4-, 5- oder 6-gliedrigen carbocyclischen Ring bilden.

12. Pharmazeutische Zusammensetzung, die die Verbindung nach einem der Ansprüche 1 bis 11 umfasst, oder das Stereoisomer oder das Tautomer davon oder das pharmazeutisch verträgliche Salz davon und mindestens einen pharmazeutisch verträglichen Hilfsstoff.

13. Verbindung nach einem der Ansprüche 1-11, oder ein Stereoisomer oder ein Tautomer davon oder ein pharmazeutisch akzeptables Salz davon, zur Verwendung bei der Behandlung einer Störung oder Erkrankung, ausgewählt aus nichtalkoholischer Steatohepatitis (NASH), Fettleibigkeit, Hyperlipidämie, Hypercholesterinämie, Diabetes, Lebersteatose, Atherosklerose, kardiovaskulären Erkrankungen, Hypothyreose und Schilddrüsenkrebs.

14. Zusammensetzung nach Anspruch 12 zur Verwendung bei der Behandlung einer Störung oder Erkrankung, ausgewählt aus nichtalkoholischer Steatohepatitis (NASH), Fettleibigkeit, Hyperlipidämie, Hypercholesterinämie, Diabetes, Lebersteatose, Atherosklerose, kardiovaskulären Erkrankungen, Hypothyreose und Schilddrüsenkrebs.

15. Verbindung nach einem der Ansprüche 1-11, oder ein Stereoisomer oder ein Tautomer davon oder ein pharmazeutisch akzeptables Salz davon, zur Verwendung bei der selektiven Modulation der Aktivität eines Thyroidhormonrezeptors beta (THR-β).

16. Zusammensetzung nach Anspruch 12 zur Verwendung bei der selektiven Modulation der Aktivität eines Thyroidhormonrezeptors beta (THR-β).

## Revendications

1. Composé de formule I' :
(I') TL-Lₐ-CE-HD
ou stéréoisomère ou tautomère de celui-ci, ou sel pharmaceutiquement acceptable de celui-ci, dans lequel :
i) TL est un fragment de formule IIIb : dans lequel : 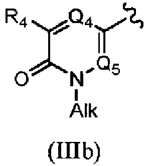
Q₄ est -CH- et Q₅ est l'azote ;
R₄ est un alkyle facultativement substitué, un alcényle facultativement substitué, un groupe carbocyclique non aromatique facultativement substitué, un groupe aryle facultativement substitué, un groupe hétérocyclique facultativement substitué, un groupe hétéroaryle facultativement substitué, un groupe (carbocyclique)alkyle facultativement substitué, un groupe aralkyle facultativement substitué, un groupe (hétérocycloalkyl)alkyle facultativement substitué, un groupe (hétéroaryl)alkyle facultativement substitué, un groupe amino facultativement substitué, un groupe sulfamoyle facultativement substitué, un groupe carbamoyle facultativement substitué, ou un groupe carbamoyle-alkyle facultativement substitué, où l'azote du groupe carbamoyle ou carbamoyle-alkyle est facultativement un hétéroatome sous forme de structure cyclique ; et
Alk est l'hydrogène ou un alkyle facultativement substitué ;
ii) CE est un fragment de formule IV 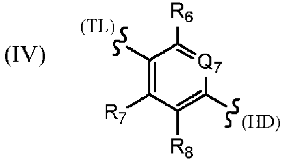 dans lequel :
chacun parmi R₆ et R₇ est indépendamment sélectionné parmi halogène, -CN, alkyle inférieur facultativement substitué, alcoxy inférieur facultativement substitué, alcényle inférieur facultativement substitué, ou cyclopropyle ;
R₈ est sélectionné parmi hydrogène, alkyle inférieur facultativement substitué, alcoxy inférieur facultativement substitué, cyano, ou halogène ;
facultativement R₇ et R₈ pris ensemble, avec les atomes de carbone auxquels ils sont fixés, forment un noyau carbocyclique, hétérocycloalkyle, aryle ou hétéroaryle non aromatique de 4, 5 ou 6 chaînons ;
Q₇ est l'azote ou -CR_{c}-, dans lequel R_{c} est l'hydrogène, un halogène, ou un alkyle inférieur ;
(TL) désigne le point où le fragment de formule IV se lie à TL-Lₐ₋ ; et
(HD) désigne le point où le fragment de formule IV se lie à -HD ;
iii)HD est un fragment de formule V : 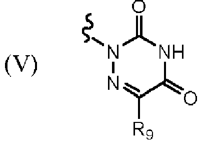 dans lequel :
R₉ est -NH₂; dans lequel
chaque R_{d} est indépendamment hydrogène ou alkyle inférieur facultativement substitué ; et
chaque n est indépendamment sélectionné parmi 0, 1, 2, 3, 4, ou 5 ;
Lₐ est indépendamment une liaison ; -(C(Rₐ)₂)_{z}- ; oxygène ; soufre ; ou -NRₐ₋ ; dans lequel :
chaque Rₐ est indépendamment un hydrogène ou alkyle inférieur ; et
z est 0, 1, 2, 3, 4 ou 5.

2. Composé selon la revendication 1, ou stéréoisomère ou tautomère de celui-ci, ou sel pharmaceutiquement acceptable de celui-ci, dans lequel R₄ est un alkyle en C₁-C₆ ; un alcényle en C₂-C₁₀ ; un noyau carbocyclique en C₃-C₁₂ non aromatique ; un groupe aryle en C₆-C₁₀ ; un noyau hétérocycloalkyle de 3 à 6 chaînons contenant un à quatre hétéroatomes du noyau sélectionnés indépendamment parmi oxygène, soufre, ou azote ; un noyau hétéroaryle à cinq à dix chaînons contenant un à quatre hétéroatomes cycliques sélectionnés indépendamment parmi oxygène, soufre, ou azote ; un groupe (carbocyclique)alkyle ; un groupe aralkyle ; ou un groupe (hétérocycloalkyl)alkyle ; et R₄ est facultativement substitué par un à cinq R_{g} sélectionnés indépendamment parmi le groupe constitué de hydroxy, halogène, CN, alkyle en C₁-C₆, aryle en C₆-C₁₀, alcoxy en C₁-C₆, haloalcoxy en C₁-C₆, et aralcoxy en C₆-C₁₀, ou deux R_{g} avec les atomes auxquels ils sont fixés forment un noyau de 3 à 6 chaînons aromatique ou non aromatique, contenant facultativement un ou deux hétéroatomes cycliques sélectionnés indépendamment parmi oxygène, soufre, ou azote.

3. Composé selon la revendication 1 ou la revendication 2, ou stéréoisomère ou tautomère de celui-ci, ou sel pharmaceutiquement acceptable de celui-ci, dans lequel R₄ est un alkyle en C₁-C₆, un alcényle en C₂-C₁₀, cyclopropyle, cyclobutyle, cyclopentyle, cyclohexyle, tétrahydrofuranyle, tétrahydropyranyle, azétidinyle, pyridazin-3(2H)-one, phényle, naphtyle, pyridinyle, cinnolinyle, isoquinolinyle, quinolinyle, pyrazolo[1,5-a]pyridinyle, imidazo[1,5-a]pyridinyle, benzo[b]thiophényle, un groupe (cyclobutyl)alkyle, un groupe (cyclopentyl)alkyle, un groupe benzyle, un groupe (tétrahydrofuranyl)alkyle, ou un groupe (tétrahydropyranyl)alkyle ; et R₄ est facultativement substitué par un à cinq R_{g} sélectionnés indépendamment parmi le groupe constitué de hydroxy, halogène, CN, alkyle en C₁-C₆, aryle en C₆-C₁₀, alcoxy en C₁-C₆, haloalcoxy en C₁-C₆, et aralcoxy en C₆-C₁₀, ou deux R_{g} avec les atomes auxquels ils sont fixés forment un noyau de 3 à 6 chaînons aromatique ou non aromatique, contenant facultativement un ou deux hétéroatomes cycliques sélectionnés indépendamment parmi oxygène, soufre, ou azote.

4. Composé selon la revendication 1, ou stéréoisomère ou tautomère de celui-ci, ou sel pharmaceutiquement acceptable de celui-ci, dans lequel R₄ est un alkyle en C₁-C₆ ou un noyau carbocyclique en C₃-C₁₂ non aromatique ; et R₄ est facultativement substitué par un à cinq R_{g} sélectionnés indépendamment parmi le groupe constitué de hydroxy, halogène, CN, alkyle en C₁-C₆, aryle en C₆-C₁₀, alcoxy en C₁-C₆, haloalcoxy en C₁-C₆, et aralcoxy en C₆-C₁₀.

5. Composé selon la revendication 1, ou stéréoisomère ou tautomère de celui-ci, ou sel pharmaceutiquement acceptable de celui-ci, dans lequel R₄ est un alkyle en C₁-C₆ facultativement substitué par un à cinq R_{g} sélectionnés indépendamment parmi le groupe constitué de hydroxy, halogène, CN, alkyle en C₁-C₆, aryle en C₆-C₁₀, alcoxy en C₁-C₆, haloalcoxy en C₁-C₆, et aralcoxy en C₆-C₁₀.

6. Composé selon la revendication 1, ou stéréoisomère ou tautomère de celui-ci, ou sel pharmaceutiquement acceptable de celui-ci, dans lequel R₄ est un noyau carbocyclique en C₃-C₁₂ non aromatique facultativement substitué par un à cinq R_{g} sélectionnés indépendamment parmi le groupe constitué de hydroxy, halogène, CN, alkyle en C₁-C₆, aryle en C₁-C₁₀, alcoxy en C₁-C₆, haloalcoxy en C₁-C₆, et aralcoxy en C₆-C₁₀.

7. Composé selon la revendication 1, ou stéréoisomère ou tautomère de celui-ci, ou sel pharmaceutiquement acceptable de celui-ci, dans lequel R₄ est un groupe aryle en C₆-C₁₀ ou un noyau hétéroaryle de cinq à dix chaînons contenant un à quatre hétéroatomes cycliques sélectionnés indépendamment parmi oxygène, soufre, ou azote ; et R₄ est facultativement substitué par un à cinq R_{g} sélectionnés indépendamment parmi le groupe constitué de hydroxy, halogène, CN, alkyle en C₁-C₆, aryle en C₆-C₁₀, alcoxy en C₁-C₆, haloalcoxy en C₁-C₆, et aralcoxy en C₆-C₁₀, ou deux R_{g} avec les atomes auxquels ils sont fixés forment un noyau de 3 à 6 chaînons aromatique ou non aromatique, contenant facultativement un ou deux hétéroatomes cycliques sélectionnés indépendamment parmi oxygène, soufre, ou azote.

8. Composé selon la revendication 1, ou stéréoisomère ou tautomère de celui-ci, ou sel pharmaceutiquement acceptable de celui-ci, dans lequel R₄ est un benzène facultativement substitué par un à cinq R_{g} sélectionnés indépendamment parmi le groupe constitué de hydroxy, halogène, CN, alkyle en C₁-C₆, alcoxy en C₁-C₆, et haloalcoxy en C₁-C₆, ou deux R_{g} avec les atomes auxquels ils sont fixés forment un noyau, ou deux R_{g} avec les atomes auxquels ils sont fixés forment un noyau de 3 à 6 chaînons aromatique ou non aromatique, contenant facultativement un ou deux hétéroatomes cycliques sélectionnés indépendamment parmi oxygène, soufre, ou azote.

9. Composé selon l'une quelconque des revendications 1 à 8, ou stéréoisomère ou tautomère de celui-ci, ou sel pharmaceutiquement acceptable de celui-ci, dans lequel Lₐ est oxygène ou -CH₂-.

10. Composé selon l'une quelconque des revendications 1 à 9, ou stéréoisomère ou tautomère de celui-ci, ou sel pharmaceutiquement acceptable de celui-ci, dans lequel Q₇ est -CH-.

11. Composé selon l'une quelconque des revendications 1 à 10, ou stéréoisomère ou tautomère de celui-ci, ou sel pharmaceutiquement acceptable de celui-ci, dans lequel
chacun de R₆ et R₇ est indépendamment halogène ou alkyle en C₁-C₅ facultativement substitué par un à cinq substituants sélectionnés indépendamment parmi hydroxy, halogène, et alcoxy en C₁-C₆ ; et R₈ est hydrogène ; ou
R₆ est halogène ou alkyle en C₁-C₅ facultativement substitué par un à cinq substituants sélectionnés indépendamment parmi hydroxy, halogène, et alcoxy en C₁-C₆ ; et R₇ et R₈ pris ensemble, avec les atomes de carbone auxquels ils sont fixés, forment un noyau carbocyclique de 4, 5 ou 6 chaînons.

12. Composition pharmaceutique comprenant le composé selon l'une quelconque des revendications 1 à 11, ou stéréoisomère ou tautomère de celui-ci, ou sel pharmaceutiquement acceptable de celui-ci, et au moins un excipient pharmaceutiquement acceptable.

13. Composé selon l'une quelconque des revendications 1 à 11, ou stéréoisomère ou tautomère de celui-ci, ou sel pharmaceutiquement acceptable de celui-ci, pour utilisation dans le traitement d'un trouble ou d'une maladie sélectionné parmi la stéatohépatite non alcoolique (NASH), l'obésité, l'hyperlipidémie, l'hypercholestérolémie, le diabète, la stéatose hépatique, l'athérosclérose, les maladies cardiovasculaires, l'hypothyroïdie, et le cancer de la thyroïde.

14. Composition selon la revendication 12 pour utilisation dans le traitement d'un trouble ou d'une maladie sélectionné parmi la stéatohépatite non alcoolique (NASH), l'obésité, l'hyperlipidémie, l'hypercholestérolémie, le diabète, la stéatose hépatique, l'athérosclérose, les maladies cardiovasculaires, l'hypothyroïdie, et le cancer de la thyroïde.

15. Composé selon l'une quelconque des revendications 1 à 11, ou stéréoisomère ou tautomère de celui-ci, ou sel pharmaceutiquement acceptable de celui-ci, pour utilisation dans la modulation sélective de l'activité d'un récepteur bêta de l'hormone thyroïdienne (THR-β).

16. Composition selon la revendication 12 pour utilisation dans la modulation sélective de l'activité d'un récepteur bêta de l'hormone thyroïdienne (THR-β).
